# EUROPÄISCHE PATENTSCHRIFT

(11) **EP 2 598 646 B1**
(45) Veröffentlichungstag und Bekanntmachung des Hinweises auf die Patenterteilung: **07.11.2018**
(21) Anmeldenummer: 11734101.6
(22) Anmeldetag: 20.07.2011
(51) Int. Cl.: C12P 19/44, A01N 43/16, C12R 1/385

(54) **ZELLEN UND VERFAHREN ZUR HERSTELLUNG VON RHAMNOLIPIDEN**
CELLS AND METHOD FOR PRODUCING RHAMNOLIPIDS
CELLULES ET PROCÉDÉ DE PRODUCTION DE RHAMNOLIPIDES

(30) Priorität: 28.07.2010 DE 102010032484
(43) Veröffentlichungstag der Anmeldung: 05.06.2013
(60) Teilanmeldung: 18184367.3
(73) Patentinhaber: Evonik Degussa GmbH, 45128 Essen (DE)
(72) Erfinder: SCHAFFER, Steffen, 45699 Herten (DE); WESSEL, Mirja, 44799 Bochum (DE); THIESSENHUSEN, Anja, 48147 Münster (DE); STEIN, Nadine, 45657 Recklinghausen (DE)
(86) Internationale Anmeldenummer: PCT/EP2011/062441
(87) Internationale Veröffentlichungsnummer: WO 2012/013554

(56) Entgegenhaltungen:
- WO-A1-2004/050882
- WO-A2-2004/083385
- US-A1- 2007 020 624
- OCHSNER: "Production of Pseudomonas aeruginosa rhamnolipid biosurfactants in heterologous hosts", APPLIED AND ENVIRONMENTAL MICROBIOLOGY, Bd. 61, Nr. 9, 1. Januar 1995 (1995-01-01) , Seite 3503, XP055005925, ISSN: 0099-2240 in der Anmeldung erwähnt
- Xiangdong Fang et al.: "Final Report: Bio-Engineering High Performance Microbial Strains for MEOR by Directed Protein Evolution Technology", Oil & Natural Gas Technology Projects , Nr. DOE No. DE-FC26-06NT15525 2. Juli 2008 (2008-07-02), Seiten II-V,1-90, XP002657933, Gefunden im Internet: URL:http://www.netl.doe.gov/technologies/o il-gas/publications/EP/NT15525_FinalReport .PDF
- DATABASE EMBL [Online] 6. Mai 1994 (1994-05-06), "Pseudomonas aeruginosa rhamnosyl transferase genes and regulatory protein gene, complete cds.", XP002657937, gefunden im EBI accession no. EM_PRO:L28170 Database accession no. L28170
- CHA ET AL: "Heterologous production of Pseudomonas aeruginosa EMS1 biosurfactant in Pseudomonas putida", BIORESOURCE TECHNOLOGY, ELSEVIER BV, GB, vol. 99, no. 7, 8 February 2008 (2008-02-08), pages 2192-2199, XP022472736, ISSN: 0960-8524, DOI: 10.1016/J.BIORTECH.2007.05.035
- I. C. Boels ET AL: "Identification and Functional Characterization of the Lactococcus lactis rfb Operon, Required for dTDP-Rhamnose Biosynthesis", Journal of Bacteriology, vol. 186, no. 5, 1 March 2004 (2004-03-01) , pages 1239-1248, XP55213648, ISSN: 0021-9193, DOI: 10.1128/JB.186.5.1239-1248.2004

### SEQUENCE LISTING

<110> Evonik Degussa GmbH
<120> Zellen und Verfahren zur Herstellung von Rhamnolipide
<130> 201000205
<160> 92
<170> PatentIn version 3.5
<210> 1
   <211> 888
   <212> DNA
   <213> Pseudomonas aeruginosa
<220>
   <221> CDS
   <222> (1)..(888)
<400> 1
   atg cgg cgc gaa agt ctg ttg gta tcg gtt tgc aag ggc ctg cgg gta        48
   Met Arg Arg Glu Ser Leu Leu Val Ser Val Cys Lys Gly Leu Arg Val
   1               5                   10                  15
   cat gtc gag cgc gtt ggg cag gat ccc ggg cgc agc acg gtg atg ctg        96
   His Val Glu Arg Val Gly Gln Asp Pro Gly Arg Ser Thr Val Met Leu
               20                  25                  30
   gtc aac ggc gcg atg gcg acc acc gcc tcg ttc gcc cgg acc tgc aag       144
   Val Asn Gly Ala Met Ala Thr Thr Ala Ser Phe Ala Arg Thr Cys Lys
           35                  40                  45
   tgc ctg gcc gaa cat ttc aac gtg gtg ctg ttc gac ctg ccc ttc gcc       192
   Cys Leu Ala Glu His Phe Asn Val Val Leu Phe Asp Leu Pro Phe Ala
       50                  55                  60
   ggg cag tcg cgt cag cac aac ccg cag cgg ggg ttg atc acc aag gac       240
   Gly Gln Ser Arg Gln His Asn Pro Gln Arg Gly Leu Ile Thr Lys Asp
   65                  70                  75                  80
   gac gag gtg gaa atc ctc ctg gcg ctg atc gag cgc ttc gag gtc aat       288
   Asp Glu Val Glu Ile Leu Leu Ala Leu Ile Glu Arg Phe Glu Val Asn
                   85                  90                  95
   cac ctg gtc tcc gcg tcc tgg ggc ggt atc tcc acg ctg ctg gcg ctg       336
   His Leu Val Ser Ala Ser Trp Gly Gly Ile Ser Thr Leu Leu Ala Leu
               100                 105                 110
   tcg cgc aat ccg cgc ggc atc cgc agc tcg gtg gtg atg gca ttc gcc       384
   Ser Arg Asn Pro Arg Gly Ile Arg Ser Ser Val Val Met Ala Phe Ala
           115                 120                 125
   cct gga ctg aac cag gcg atg ctc gac tac gtc ggg cgg gcg cag gcg       432
   Pro Gly Leu Asn Gln Ala Met Leu Asp Tyr Val Gly Arg Ala Gln Ala
       130                 135                 140
   ctg atc gag ctg gac gac aag tcg gcg atc ggc cat ctg ctc aac gag       480
   Leu Ile Glu Leu Asp Asp Lys Ser Ala Ile Gly His Leu Leu Asn Glu
   145                 150                 155                 160
   acc gtc ggc aaa tac ctg ccg ccg cgc ctg aaa gcc agc aac cat cag       528
   Thr Val Gly Lys Tyr Leu Pro Pro Arg Leu Lys Ala Ser Asn His Gln
                   165                 170                 175
   cac atg gct tcg ctg gcc acc ggc gaa tac gag cag gcg cgc ttt cac        576
   His Met Ala Ser Leu Ala Thr Gly Glu Tyr Glu Gln Ala Arg Phe His
               180                 185                 190
   atc gac cag gtg ctg gcg ctc aac gat cgg ggc tac ctg gct tgc ctg        624
   Ile Asp Gln Val Leu Ala Leu Asn Asp Arg Gly Tyr Leu Ala Cys Leu
           195                 200                 205
   gag cgg atc cag agc cac gtg cat ttc atc aac ggc agc tgg gac gaa        672
   Glu Arg Ile Gln Ser His Val His Phe Ile Asn Gly Ser Trp Asp Glu
       210                 215                 220
   tac acc acc gcc gag gac gcc cgc cag ttc cgc gac tac ctg ccg cac        720
   Tyr Thr Thr Ala Glu Asp Ala Arg Gln Phe Arg Asp Tyr Leu Pro His
   225                 230                 235                 240
   tgc agt ttc tcg cgg gtg gag ggc acc ggg cat ttc ctc gac ctg gag        768
   Cys Ser Phe Ser Arg Val Glu Gly Thr Gly His Phe Leu Asp Leu Glu
                   245                 250                 255
   tcc aag ctg gcc gcg gta cgc gtg cac cgc gcc ctg ctc gag cac ctg        816
   Ser Lys Leu Ala Ala Val Arg Val His Arg Ala Leu Leu Glu His Leu
               260                 265                 270
   ctg aag caa ccg gag ccg cag cgg gcg gaa cgc gcg gcg gga ttc cac        864
   Leu Lys Gln Pro Glu Pro Gln Arg Ala Glu Arg Ala Ala Gly Phe His
           275                 280                 285
   gag atg gcc atc ggc tac gcc tga                                        888
   Glu Met Ala Ile Gly Tyr Ala
       290                 295
<210> 2
   <211> 295
   <212> PRT
   <213> Pseudomonas aeruginosa
<400> 2
   Met Arg Arg Glu Ser Leu Leu Val Ser Val Cys Lys Gly Leu Arg Val
   1               5                   10                  15
   His Val Glu Arg Val Gly Gln Asp Pro Gly Arg Ser Thr Val Met Leu
               20                  25                  30
   Val Asn Gly Ala Met Ala Thr Thr Ala Ser Phe Ala Arg Thr Cys Lys
           35                  40                  45
   Cys Leu Ala Glu His Phe Asn Val Val Leu Phe Asp Leu Pro Phe Ala
       50                  55                  60
   Gly Gln Ser Arg Gln His Asn Pro Gln Arg Gly Leu Ile Thr Lys Asp
   65                  70                  75                  80
   Asp Glu Val Glu Ile Leu Leu Ala Leu Ile Glu Arg Phe Glu Val Asn
                   85                  90                  95
   His Leu Val Ser Ala Ser Trp Gly Gly Ile Ser Thr Leu Leu Ala Leu
                   100                 105                 110
   Ser Arg Asn Pro Arg Gly Ile Arg Ser Ser Val Val Met Ala Phe Ala
               115                 120                 125
   Pro Gly Leu Asn Gln Ala Met Leu Asp Tyr Val Gly Arg Ala Gln Ala
       130                 135                 140
   Leu Ile Glu Leu Asp Asp Lys Ser Ala Ile Gly His Leu Leu Asn Glu
   145                 150                 155                 160
   Thr Val Gly Lys Tyr Leu Pro Pro Arg Leu Lys Ala Ser Asn His Gln
                   165                 170                 175
   His Met Ala Ser Leu Ala Thr Gly Glu Tyr Glu Gln Ala Arg Phe His
               180                 185                 190
   Ile Asp Gln Val Leu Ala Leu Asn Asp Arg Gly Tyr Leu Ala Cys Leu
           195                 200                 205
   Glu Arg Ile Gln Ser His Val His Phe Ile Asn Gly Ser Trp Asp Glu
       210                 215                 220
   Tyr Thr Thr Ala Glu Asp Ala Arg Gln Phe Arg Asp Tyr Leu Pro His
   225                 230                 235                 240
   Cys Ser Phe Ser Arg Val Glu Gly Thr Gly His Phe Leu Asp Leu Glu
                   245                 250                 255
   Ser Lys Leu Ala Ala Val Arg Val His Arg Ala Leu Leu Glu His Leu
               260                 265                 270
   Leu Lys Gln Pro Glu Pro Gln Arg Ala Glu Arg Ala Ala Gly Phe His
           275                 280                 285
   Glu Met Ala Ile Gly Tyr Ala
       290                 295
<210> 3
   <211> 1281
   <212> DNA
   <213> Pseudomonas aeruginosa
<220>
   <221> CDS
   <222> (1)..(1281)
<400> 3
   atg cac gcc atc ctc atc gcc atc ggc tcg gcc ggc gac gta ttt ccc 48
   Met His Ala Ile Leu Ile Ala Ile Gly Ser Ala Gly Asp Val Phe Pro
   1               5                   10                  15
   ttc atc ggc ctg gcc cgg acc ctg aaa ttg cgc ggg cac cgc gtg agc       96
   Phe Ile Gly Leu Ala Arg Thr Leu Lys Leu Arg Gly His Arg Val Ser
               20                  25                  30
   ctc tgc acc atc ccg gtg ttt cgc gac gcg gtg gag cag cac ggc atc      144
   Leu Cys Thr Ile Pro Val Phe Arg Asp Ala Val Glu Gln His Gly Ile
           35                  40                  45
   gcg ttc gtc ccg ctg agc gac gaa ctg acc tac cgc cgg acc atg ggc      192
   Ala Phe Val Pro Leu Ser Asp Glu Leu Thr Tyr Arg Arg Thr Met Gly
       50                  55                  60
   gat ccg cgc ctg tgg gac ccc aag acg tcc ttc ggc gtg ctc tgg caa      240
   Asp Pro Arg Leu Trp Asp Pro Lys Thr Ser Phe Gly Val Leu Trp Gln
   65                  70                  75                  80
   acc atc gcc ggg atg atc gag ccg gtc tac gag tac gtc tcg gcg cag      288
   Thr Ile Ala Gly Met Ile Glu Pro Val Tyr Glu Tyr Val Ser Ala Gln
                   85                  90                  95
   cgc cat gac gac atc gtg gtg gtc ggc tcg ctc tgg gcg ctg ggc gca      336
   Arg His Asp Asp Ile Val Val Val Gly Ser Leu Trp Ala Leu Gly Ala
               100                 105                 110
   cgc atc gct cac gag aag tac ggg att ccc tac ctg tcc gcg cag gtc      384
   Arg Ile Ala His Glu Lys Tyr Gly Ile Pro Tyr Leu Ser Ala Gln Val
           115                 120                 125
   tcg cca tcg acc ttg ttg tcg gcg cac ctg ccg ccg gta cac ccc aag      432
   Ser Pro Ser Thr Leu Leu Ser Ala His Leu Pro Pro Val His Pro Lys
       130                 135                 140
   ttc aac gtg ccc gag cag atg ccg ctg gcg atg cgc aag ctg ctc tgg      480
   Phe Asn Val Pro Glu Gln Met Pro Leu Ala Met Arg Lys Leu Leu Trp
   145                 150                 155                 160
   cgc tgc atc gag cgc ttc aag ctg gat cgc acc tgc gcg ccg gat atc      528
   Arg Cys Ile Glu Arg Phe Lys Leu Asp Arg Thr Cys Ala Pro Asp Ile
                   165                 170                 175
   aac gcg gtg cgg cgc aag gtc ggc ctg gag acg ccg gtg aag cgc atc      576
   Asn Ala Val Arg Arg Lys Val Gly Leu Glu Thr Pro Val Lys Arg Ile
               180                 185                 190
   ttc acc caa tgg atg cat tcg ccg cag ggc gtg gtc tgc ctg ttc ccg      624
   Phe Thr Gln Trp Met His Ser Pro Gln Gly Val Val Cys Leu Phe Pro
           195                 200                 205
   gcc tgg ttc gcg ccg ccc cag cag gat tgg ccg caa ccc ctg cac atg      672
   Ala Trp Phe Ala Pro Pro Gln Gln Asp Trp Pro Gln Pro Leu His Met
       210                 215                 220
   acc ggc ttc ccg ctg ttc gac ggc agt atc ccg ggg acc ccg ctc gac      720
   Thr Gly Phe Pro Leu Phe Asp Gly Ser Ile Pro Gly Thr Pro Leu Asp
   225                 230                 235                 240
   gac gaa ctg caa cgc ttt ctc gat cag ggc agc cgg ccg ctg gtg ttc      768
   Asp Glu Leu Gln Arg Phe Leu Asp Gln Gly Ser Arg Pro Leu Val Phe
                   245                 250                 255
   acc cag ggc tcg acc gaa cac ctg cag ggc gac ttc tac gcc atg gcc      816
   Thr Gln Gly Ser Thr Glu His Leu Gln Gly Asp Phe Tyr Ala Met Ala
               260                 265                 270
   ctg cgc gcg ctg gaa cgc ctc ggc gcg cgt ggg atc ttc ctc acc ggc       864
   Leu Arg Ala Leu Glu Arg Leu Gly Ala Arg Gly Ile Phe Leu Thr Gly
           275                 280                 285
   gcc ggc cag gaa ccg ctg cgc ggc ttg ccg aac cac gtg ctg cag cgc       912
   Ala Gly Gln Glu Pro Leu Arg Gly Leu Pro Asn His Val Leu Gln Arg
       290                 295                 300
   gcc tac gcg cca ctg gga gcc ttg ctg cca tcg tgc gcc ggg ctg gtc       960
   Ala Tyr Ala Pro Leu Gly Ala Leu Leu Pro Ser Cys Ala Gly Leu Val
   305                 310                 315                 320
   cat ccg ggc ggt atc ggc gcc atg agc ctg gcc ttg gcg gcg ggg gtg      1008
   His Pro Gly Gly Ile Gly Ala Met Ser Leu Ala Leu Ala Ala Gly Val
                   325                 330                 335
   ccg cag gtg ctg ctg ccc tgc gcc cac gac cag ttc gac aat gcc gaa      1056
   Pro Gln Val Leu Leu Pro Cys Ala His Asp Gln Phe Asp Asn Ala Glu
               340                 345                 350
   cgg ctg gtc cgg ctc ggc tgc ggg atg cgc ctg ggc gtg cca ttg cgc      1104
   Arg Leu Val Arg Leu Gly Cys Gly Met Arg Leu Gly Val Pro Leu Arg
           355                 360                 365
   gag cag gag ttg cgc ggg gcg ctg tgg cgc ttg ctc gag gac ccg gcc      1152
   Glu Gln Glu Leu Arg Gly Ala Leu Trp Arg Leu Leu Glu Asp Pro Ala
       370                 375                 380
   atg gcg gcg gcc tgt cgg cgt ttc atg gaa ttg tca caa ccg cac agt      1200
   Met Ala Ala Ala Cys Arg Arg Phe Met Glu Leu Ser Gln Pro His Ser
   385                 390                 395                 400
   atc gct tgc ggt aaa gcg gcc cag gtg gtc gaa cgt tgt cat agg gag      1248
   Ile Ala Cys Gly Lys Ala Ala Gln Val Val Glu Arg Cys His Arg Glu
                   405                 410                 415
   ggg gat gcg cga tgg ctg aag gct gcg tcc tga                          1281
   Gly Asp Ala Arg Trp Leu Lys Ala Ala Ser
               420                 425
<210> 4
   <211> 426
   <212> PRT
   <213> Pseudomonas aeruginosa
<400> 4
   Met His Ala Ile Leu Ile Ala Ile Gly Ser Ala Gly Asp Val Phe Pro
   1               5                   10                  15
   Phe Ile Gly Leu Ala Arg Thr Leu Lys Leu Arg Gly His Arg Val Ser
               20                  25                  30
   Leu Cys Thr Ile Pro Val Phe Arg Asp Ala Val Glu Gln His Gly Ile
           35                  40                  45
   Ala Phe Val Pro Leu Ser Asp Glu Leu Thr Tyr Arg Arg Thr Met Gly
       50                  55                  60
   Asp Pro Arg Leu Trp Asp Pro Lys Thr Ser Phe Gly Val Leu Trp Gln
   65                  70                  75                  80
   Thr Ile Ala Gly Met Ile Glu Pro Val Tyr Glu Tyr Val Ser Ala Gln
                   85                  90                  95
   Arg His Asp Asp Ile Val Val Val Gly Ser Leu Trp Ala Leu Gly Ala
               100                 105                 110
   Arg Ile Ala His Glu Lys Tyr Gly Ile Pro Tyr Leu Ser Ala Gln Val
           115                 120                 125
   Ser Pro Ser Thr Leu Leu Ser Ala His Leu Pro Pro Val His Pro Lys
       130                 135                 140
   Phe Asn Val Pro Glu Gln Met Pro Leu Ala Met Arg Lys Leu Leu Trp
   145                 150                 155                 160
   Arg Cys Ile Glu Arg Phe Lys Leu Asp Arg Thr Cys Ala Pro Asp Ile
                   165                 170                 175
   Asn Ala Val Arg Arg Lys Val Gly Leu Glu Thr Pro Val Lys Arg Ile
               180                 185                 190
   Phe Thr Gln Trp Met His Ser Pro Gln Gly Val Val Cys Leu Phe Pro
           195                 200                 205
   Ala Trp Phe Ala Pro Pro Gln Gln Asp Trp Pro Gln Pro Leu His Met
       210                 215                 220
   Thr Gly Phe Pro Leu Phe Asp Gly Ser Ile Pro Gly Thr Pro Leu Asp
   225                 230                 235                 240
   Asp Glu Leu Gln Arg Phe Leu Asp Gln Gly Ser Arg Pro Leu Val Phe
                   245                 250                 255
   Thr Gln Gly Ser Thr Glu His Leu Gln Gly Asp Phe Tyr Ala Met Ala
               260                 265                 270
   Leu Arg Ala Leu Glu Arg Leu Gly Ala Arg Gly Ile Phe Leu Thr Gly
           275                 280                 285
   Ala Gly Gln Glu Pro Leu Arg Gly Leu Pro Asn His Val Leu Gln Arg
       290                 295                 300
   Ala Tyr Ala Pro Leu Gly Ala Leu Leu Pro Ser Cys Ala Gly Leu Val
   305                 310                 315                 320
   His Pro Gly Gly Ile Gly Ala Met Ser Leu Ala Leu Ala Ala Gly Val
   325 330 335
   Pro Gln Val Leu Leu Pro Cys Ala His Asp Gln Phe Asp Asn Ala Glu
   340 345 350
   Arg Leu Val Arg Leu Gly Cys Gly Met Arg Leu Gly Val Pro Leu Arg
   355 360 365
   Glu Gln Glu Leu Arg Gly Ala Leu Trp Arg Leu Leu Glu Asp Pro Ala
   370 375 380
   Met Ala Ala Ala Cys Arg Arg Phe Met Glu Leu Ser Gln Pro His Ser
   385 390 395 400
   Ile Ala Cys Gly Lys Ala Ala Gln Val Val Glu Arg Cys His Arg Glu
   405 410 415
   Gly Asp Ala Arg Trp Leu Lys Ala Ala Ser
   420 425
<210> 5
   <211> 978
   <212> DNA
   <213> Pseudomonas aeruginosa
<220>
   <221> CDS
   <222> (1)..(978)
<400> 5
   atg gac cgg ata gac atg ggc gtg ctg gtg gta ctg ttc aat cct ggc       48
   Met Asp Arg Ile Asp Met Gly Val Leu Val Val Leu Phe Asn Pro Gly
   1               5                   10                  15
   gac gac gac ctg gaa cac ctt ggc gaa ctg gcg gcg gcg ttt ccg caa       96
   Asp Asp Asp Leu Glu His Leu Gly Glu Leu Ala Ala Ala Phe Pro Gln
               20                  25                  30
   ctg cgc ttc ctt gcc gtc gac aac tca ccg cac agc gat ccg cag cgc      144
   Leu Arg Phe Leu Ala Val Asp Asn Ser Pro His Ser Asp Pro Gln Arg
           35                  40                  45
   aat gcc cgg ctg cgc ggg caa ggc atc gcc gtg ctg cac cac ggc aac      192
   Asn Ala Arg Leu Arg Gly Gln Gly Ile Ala Val Leu His His Gly Asn
       50                  55                  60
   cgg cag ggc atc gcc ggc gcc ttc aac cag gga ctc gac gcg cta ttc      240
   Arg Gln Gly Ile Ala Gly Ala Phe Asn Gln Gly Leu Asp Ala Leu Phe
   65                  70                  75                  80
   cgg cgt ggc gtg cag ggt gtg ctg ctg ctc gac cag gac tcc cgt ccc      288
   Arg Arg Gly Val Gln Gly Val Leu Leu Leu Asp Gln Asp Ser Arg Pro
                   85                  90                  95
   ggc ggc gcc ttc ctc gcc gcc cag tgg cgc aac ctg cag gcg cgc aac      336
   Gly Gly Ala Phe Leu Ala Ala Gln Trp Arg Asn Leu Gln Ala Arg Asn
               100                 105                 110
   ggt cag gcc tgc ctg ctc ggc cca cgg atc ttc gac cgg ggt gac cgg       384
   Gly Gln Ala Cys Leu Leu Gly Pro Arg Ile Phe Asp Arg Gly Asp Arg
           115                 120                 125
   cgc ttc ctg ccg gcc atc cat ctc gac gga ctg acg ctc agg caa ttg       432
   Arg Phe Leu Pro Ala Ile His Leu Asp Gly Leu Thr Leu Arg Gln Leu
       130                 135                 140
   tct ctg gac ggc ctg acg acc ccg cag cgc acc tcg ttc ctg atc tcc       480
   Ser Leu Asp Gly Leu Thr Thr Pro Gln Arg Thr Ser Phe Leu Ile Ser
   145                 150                 155                 160
   tcc ggc tgc ctg ctg acc cgc gag gcc tac cag cgc ctc ggc cac ttc       528
   Ser Gly Cys Leu Leu Thr Arg Glu Ala Tyr Gln Arg Leu Gly His Phe
                   165                 170                 175
   gac gag gaa ctg ttc atc gac cac gtg gac acc gaa tac agc ctg cgc       576
   Asp Glu Glu Leu Phe Ile Asp His Val Asp Thr Glu Tyr Ser Leu Arg
               180                 185                 190
   gcc cag gcg ctg gac gtg ccc ctg tac gtc gac ccg cgg ctg gtc ctc       624
   Ala Gln Ala Leu Asp Val Pro Leu Tyr Val Asp Pro Arg Leu Val Leu
           195                 200                 205
   gag cac cgc atc ggc acg cgc aag acc cgc cgc ctc ggc ggt ctc agc       672
   Glu His Arg Ile Gly Thr Arg Lys Thr Arg Arg Leu Gly Gly Leu Ser
       210                 215                 220
   ctc agc gcg atg aac cac gcc ccg ctg cgc cgc tac tac ctg gcg cgc       720
   Leu Ser Ala Met Asn His Ala Pro Leu Arg Arg Tyr Tyr Leu Ala Arg
   225                 230                 235                 240
   aac ggc ctg ctg gtc ctg cgc cgc tac gcc cgg tcc tcg ccg ctg gcc       768
   Asn Gly Leu Leu Val Leu Arg Arg Tyr Ala Arg Ser Ser Pro Leu Ala
                   245                 250                 255
   ctg ctg gcg aac ctg ccg acc ctg acc cag ggc ctc gcg gtg ctc ctg       816
   Leu Leu Ala Asn Leu Pro Thr Leu Thr Gln Gly Leu Ala Val Leu Leu
               260                 265                 270
   ctc gaa cgc gac aag ctg ctc aag ctg cgc tgc ctg ggc tgg ggc ctg       864
   Leu Glu Arg Asp Lys Leu Leu Lys Leu Arg Cys Leu Gly Trp Gly Leu
           275                 280                 285
   tgg gac ggc ctg cgg gga cgc ggc ggc gcg ctg gag acc aac cgc ccg       912
   Trp Asp Gly Leu Arg Gly Arg Gly Gly Ala Leu Glu Thr Asn Arg Pro
       290                 295                 300
   cgc ctg ctg aag cgc ctc gcc ggc ccg gcc gtg gcg tcc gta gct tcc       960
   Arg Leu Leu Lys Arg Leu Ala Gly Pro Ala Val Ala Ser Val Ala Ser
   305                 310                 315                 320
   ggc aag gcc aag gcc tag                                               978
   Gly Lys Ala Lys Ala
                   325
<210> 6
   <211> 325
   <212> PRT
   <213> Pseudomonas aeruginosa
<400> 6
   Met Asp Arg Ile Asp Met Gly Val Leu Val Val Leu Phe Asn Pro Gly
   1               5                   10                  15
   Asp Asp Asp Leu Glu His Leu Gly Glu Leu Ala Ala Ala Phe Pro Gln
               20                  25                  30
   Leu Arg Phe Leu Ala Val Asp Asn Ser Pro His Ser Asp Pro Gln Arg
           35                  40                  45
   Asn Ala Arg Leu Arg Gly Gln Gly Ile Ala Val Leu His His Gly Asn
       50                  55                  60
   Arg Gln Gly Ile Ala Gly Ala Phe Asn Gln Gly Leu Asp Ala Leu Phe
   65                  70                  75                  80
   Arg Arg Gly Val Gln Gly Val Leu Leu Leu Asp Gln Asp Ser Arg Pro
                   85                  90                  95
   Gly Gly Ala Phe Leu Ala Ala Gln Trp Arg Asn Leu Gln Ala Arg Asn
               100                 105                 110
   Gly Gln Ala Cys Leu Leu Gly Pro Arg Ile Phe Asp Arg Gly Asp Arg
           115                 120                 125
   Arg Phe Leu Pro Ala Ile His Leu Asp Gly Leu Thr Leu Arg Gln Leu
       130                 135                 140
   Ser Leu Asp Gly Leu Thr Thr Pro Gln Arg Thr Ser Phe Leu Ile Ser
   145                 150                 155                 160
   Ser Gly Cys Leu Leu Thr Arg Glu Ala Tyr Gln Arg Leu Gly His Phe
                   165                 170                 175
   Asp Glu Glu Leu Phe Ile Asp His Val Asp Thr Glu Tyr Ser Leu Arg
               180                 185                 190
   Ala Gln Ala Leu Asp Val Pro Leu Tyr Val Asp Pro Arg Leu Val Leu
           195                 200                 205
   Glu His Arg Ile Gly Thr Arg Lys Thr Arg Arg Leu Gly Gly Leu Ser
       210                 215                 220
   Leu Ser Ala Met Asn His Ala Pro Leu Arg Arg Tyr Tyr Leu Ala Arg
   225                 230                 235                 240
   Asn Gly Leu Leu Val Leu Arg Arg Tyr Ala Arg Ser Ser Pro Leu Ala
                   245                 250                 255
   Leu Leu Ala Asn Leu Pro Thr Leu Thr Gln Gly Leu Ala Val Leu Leu
               260                 265                 270
   Leu Glu Arg Asp Lys Leu Leu Lys Leu Arg Cys Leu Gly Trp Gly Leu
           275                 280                 285
   Trp Asp Gly Leu Arg Gly Arg Gly Gly Ala Leu Glu Thr Asn Arg Pro
       290                 295                 300
   Arg Leu Leu Lys Arg Leu Ala Gly Pro Ala Val Ala Ser Val Ala Ser
   305                 310                 315                 320
   Gly Lys Ala Lys Ala
                   325
<210> 7
   <211> 1269
   <212> DNA
   <213> Pseudomonas aeruginosa
<220>
   <221> CDS
   <222> (1)..(1269)
<400> 7
   gtg tcc acg acc agc ctc tgc ccc tcc gcc acg cgg gaa cac ggt ccc          48
   Val Ser Thr Thr Ser Leu Cys Pro Ser Ala Thr Arg Glu His Gly Pro
   1               5                   10                  15
   ggc gcg aaa cgc gtc ctg cct ctg ctg ttc ctc acc tgc ctg ctg gat          96
   Gly Ala Lys Arg Val Leu Pro Leu Leu Phe Leu Thr Cys Leu Leu Asp
               20                  25                  30
   gcc gct ggc gtc ggc ctg atc gtg ccc ctg ctg ccg acg ctg atc ggc         144
   Ala Ala Gly Val Gly Leu Ile Val Pro Leu Leu Pro Thr Leu Ile Gly
           35                  40                  45
   agc gtg gcg ccg ctg gcg gtc cgc gac gcg gcc acc tgg ggc gcc gcc         192
   Ser Val Ala Pro Leu Ala Val Arg Asp Ala Ala Thr Trp Gly Ala Ala
       50                  55                  60
   ctg gtg atg acc ttc gcg ctg ctg caa ttg ttc ttt tcg ccg gtc ctc         240
   Leu Val Met Thr Phe Ala Leu Leu Gln Leu Phe Phe Ser Pro Val Leu
   65                  70                  75                  80
   ggc agc ctc agc gac cgc ttc gga cgc cgc ccc gtc ctg gtc ctg gcg         288
   Gly Ser Leu Ser Asp Arg Phe Gly Arg Arg Pro Val Leu Val Leu Ala
                   85                  90                  95
   atg ctc ggc ttc gcc ctc agc tat ctg ctg ctg gcg ctg gcc gac agc         336
   Met Leu Gly Phe Ala Leu Ser Tyr Leu Leu Leu Ala Leu Ala Asp Ser
               100                 105                 110
   ctc tgg atg ctg ttc ctc ggt cgc gcg ctg gcc ggg ctc acc ggc gcc         384
   Leu Trp Met Leu Phe Leu Gly Arg Ala Leu Ala Gly Leu Thr Gly Ala
           115                 120                 125
   agc gtg gcc acc gcg atg gcc tgc gcg gct gac ctc ggc acg cac ggg         432
   Ser Val Ala Thr Ala Met Ala Cys Ala Ala Asp Leu Gly Thr His Gly
       130                 135                 140
   cag cgc acc cgg cac ttc ggc tgg ctg tac gcc ggc ctc gcc ctg ggc        480
   Gln Arg Thr Arg His Phe Gly Trp Leu Tyr Ala Gly Leu Ala Leu Gly
   145                 150                 155                 160
   atg atc ctc ggc ccc gcc ctc ggt ggg ctg ctg gcg gtg cac ggc acg        528
   Met Ile Leu Gly Pro Ala Leu Gly Gly Leu Leu Ala Val His Gly Thr
                   165                 170                 175
   acg ctg ccg ctg ttg ctg gcc gcc ggc ctg tgc ctg ctc aac gcc ctg        576
   Thr Leu Pro Leu Leu Leu Ala Ala Gly Leu Cys Leu Leu Asn Ala Leu
               180                 185                 190
   ctc gcc ggc ctg ttc ctc gag gaa acc ctg ccc ccg acg cga cgc cgc        624
   Leu Ala Gly Leu Phe Leu Glu Glu Thr Leu Pro Pro Thr Arg Arg Arg
           195                 200                 205
   cgc ctg gac ccg agg cgg atg aat gcc ttg cgc tcg atc agc ggc ctg        672
   Arg Leu Asp Pro Arg Arg Met Asn Ala Leu Arg Ser Ile Ser Gly Leu
       210                 215                 220
   gct cgg caa ccg ggg gtc gga cgc ctg ctg gcg gtg ctt gcc ctg gta        720
   Ala Arg Gln Pro Gly Val Gly Arg Leu Leu Ala Val Leu Ala Leu Val
   225                 230                 235                 240
   ttc ctc ggc ttg cag gcg gtg atg gtg gtc tgg ccg ttc ttc gtg atc        768
   Phe Leu Gly Leu Gln Ala Val Met Val Val Trp Pro Phe Phe Val Ile
                   245                 250                 255
   gag aag ttt cac tgg agc agc gcc tgg atc ggc tac tcg ctg gcc ctc        816
   Glu Lys Phe His Trp Ser Ser Ala Trp Ile Gly Tyr Ser Leu Ala Leu
               260                 265                 270
   tac ggc gtg ctc gcg gtg ctc gcc cag acc ctc ggc gtg aac ctc tgc        864
   Tyr Gly Val Leu Ala Val Leu Ala Gln Thr Leu Gly Val Asn Leu Cys
           275                 280                 285
   aag cgg cgc ctg gac gac gcc cgc ctg ctg cgc ctg ggc ctc gcc ctg        912
   Lys Arg Arg Leu Asp Asp Ala Arg Leu Leu Arg Leu Gly Leu Ala Leu
       290                 295                 300
   caa ggc tgc ggc ctg ctg ctg ttc gcc ctg gtc gac tcg tca ttc tgg        960
   Gln Gly Cys Gly Leu Leu Leu Phe Ala Leu Val Asp Ser Ser Phe Trp
   305                 310                 315                 320
   ctg gtc tgc gcg ctg ctg ccc ttc gcg ctc ggc agc ctc gcc acc ccg       1008
   Leu Val Cys Ala Leu Leu Pro Phe Ala Leu Gly Ser Leu Ala Thr Pro
                   325                 330                 335
   gcc atg cag ggg ctg ctc tcg gcc cgc gtg ccg gtc gac cgc cag ggc       1056
   Ala Met Gln Gly Leu Leu Ser Ala Arg Val Pro Val Asp Arg Gln Gly
               340                 345                 350
   gag ttg cag ggc gtg ctg agc agc ctg atg agc ctc gcc gcg atc gtc       1104
   Glu Leu Gln Gly Val Leu Ser Ser Leu Met Ser Leu Ala Ala Ile Val
           355                 360                 365
   ggt ccg ccg ctg atg agc ggc ctg ttc cac tgg ggc agc ggt ccg ctc       1152
   Gly Pro Pro Leu Met Ser Gly Leu Phe His Trp Gly Ser Gly Pro Leu
       370                 375                 380
   gcg ccg ctg ccc ctg gcc ggc gcg cca ttc ctc gcc ggc gcc ctt ctc       1200
   Ala Pro Leu Pro Leu Ala Gly Ala Pro Phe Leu Ala Gly Ala Leu Leu
   385                 390                 395                 400
   gtt ctg gcc ggg ctg gtc ctg gcc tgg caa ctt cga cct acg gga gaa       1248
   Val Leu Ala Gly Leu Val Leu Ala Trp Gln Leu Arg Pro Thr Gly Glu
                   405                 410                 415
   gaa cga tca tgg acc gga tag                                           1269
   Glu Arg Ser Trp Thr Gly
               420
<210> 8
   <211> 422
   <212> PRT
   <213> Pseudomonas aeruginosa
<400> 8
   Val Ser Thr Thr Ser Leu Cys Pro Ser Ala Thr Arg Glu His Gly Pro
   1               5                   10                  15
   Gly Ala Lys Arg Val Leu Pro Leu Leu Phe Leu Thr Cys Leu Leu Asp
               20                  25                  30
   Ala Ala Gly Val Gly Leu Ile Val Pro Leu Leu Pro Thr Leu Ile Gly
           35                  40                  45
   Ser Val Ala Pro Leu Ala Val Arg Asp Ala Ala Thr Trp Gly Ala Ala
       50                  55                  60
   Leu Val Met Thr Phe Ala Leu Leu Gln Leu Phe Phe Ser Pro Val Leu
   65                  70                  75                  80
   Gly Ser Leu Ser Asp Arg Phe Gly Arg Arg Pro Val Leu Val Leu Ala
                   85                  90                  95
   Met Leu Gly Phe Ala Leu Ser Tyr Leu Leu Leu Ala Leu Ala Asp Ser
               100                 105                 110
   Leu Trp Met Leu Phe Leu Gly Arg Ala Leu Ala Gly Leu Thr Gly Ala
           115                 120                 125
   Ser Val Ala Thr Ala Met Ala Cys Ala Ala Asp Leu Gly Thr His Gly
       130                 135                 140
   Gln Arg Thr Arg His Phe Gly Trp Leu Tyr Ala Gly Leu Ala Leu Gly
   145                 150                 155                 160
   Met Ile Leu Gly Pro Ala Leu Gly Gly Leu Leu Ala Val His Gly Thr
                   165                 170                 175
   Thr Leu Pro Leu Leu Leu Ala Ala Gly Leu Cys Leu Leu Asn Ala Leu
               180                 185                 190
   Leu Ala Gly Leu Phe Leu Glu Glu Thr Leu Pro Pro Thr Arg Arg Arg
           195                 200                 205
   Arg Leu Asp Pro Arg Arg Met Asn Ala Leu Arg Ser Ile Ser Gly Leu
       210                 215                 220
   Ala Arg Gln Pro Gly Val Gly Arg Leu Leu Ala Val Leu Ala Leu Val
   225                 230                 235                 240
   Phe Leu Gly Leu Gln Ala Val Met Val Val Trp Pro Phe Phe Val Ile
                   245                 250                 255
   Glu Lys Phe His Trp Ser Ser Ala Trp Ile Gly Tyr Ser Leu Ala Leu
               260                 265                 270
   Tyr Gly Val Leu Ala Val Leu Ala Gln Thr Leu Gly Val Asn Leu Cys
           275                 280                 285
   Lys Arg Arg Leu Asp Asp Ala Arg Leu Leu Arg Leu Gly Leu Ala Leu
       290                 295                 300
   Gln Gly Cys Gly Leu Leu Leu Phe Ala Leu Val Asp Ser Ser Phe Trp
   305                 310                 315                 320
   Leu Val Cys Ala Leu Leu Pro Phe Ala Leu Gly Ser Leu Ala Thr Pro
                   325                 330                 335
   Ala Met Gln Gly Leu Leu Ser Ala Arg Val Pro Val Asp Arg Gln Gly
               340                 345                 350
   Glu Leu Gln Gly Val Leu Ser Ser Leu Met Ser Leu Ala Ala Ile Val
           355                 360                 365
   Gly Pro Pro Leu Met Ser Gly Leu Phe His Trp Gly Ser Gly Pro Leu
       370                 375                 380
   Ala Pro Leu Pro Leu Ala Gly Ala Pro Phe Leu Ala Gly Ala Leu Leu
   385                 390                 395                 400
   Val Leu Ala Gly Leu Val Leu Ala Trp Gln Leu Arg Pro Thr Gly Glu
                   405                 410                 415
   Glu Arg Ser Trp Thr Gly
               420
<210> 9
   <211> 882
   <212> DNA
   <213> Pseudomonas putida
<220>
   <221> CDS
   <222> (1)..(882)
<400> 9
   atg gct cgt aaa gga att att ctg gcc ggc ggt tcg ggt aca cgc ctg      48
   Met Ala Arg Lys Gly Ile Ile Leu Ala Gly Gly Ser Gly Thr Arg Leu
   1               5                   10                  15
   cat ccg gcc aca ctt tcg gtt tcg aag cag ctg ctg ccg gtg tat gac      96
   His Pro Ala Thr Leu Ser Val Ser Lys Gln Leu Leu Pro Val Tyr Asp
               20                  25                  30
   aaa ccg atg atc tac tac ccg ctg agc acc ctg ctg ctc gct ggt atc     144
   Lys Pro Met Ile Tyr Tyr Pro Leu Ser Thr Leu Leu Leu Ala Gly Ile
           35                  40                  45
   cgg gac atc ctg atc att tcc acc ccg cag gac acc ccg cgc ttc gaa     192
   Arg Asp Ile Leu Ile Ile Ser Thr Pro Gln Asp Thr Pro Arg Phe Glu
       50                  55                  60
   cag ctg ctg ggc gat ggc agc cag tgg ggc ctg aac ctg tca tac gca     240
   Gln Leu Leu Gly Asp Gly Ser Gln Trp Gly Leu Asn Leu Ser Tyr Ala
   65                  70                  75                  80
   ata caa cca agc ccg gat ggc ttg gcg caa gcg ttc acc atc ggc gct     288
   Ile Gln Pro Ser Pro Asp Gly Leu Ala Gln Ala Phe Thr Ile Gly Ala
                   85                  90                  95
   gac ttc atc ggt aac gac cct tct gcg ttg gtt ctc ggt gac aat att     336
   Asp Phe Ile Gly Asn Asp Pro Ser Ala Leu Val Leu Gly Asp Asn Ile
               100                 105                 110
   ttc tac ggc cat gac ttc cag gca ctg cta ttg aac gca gat aaa cgt     384
   Phe Tyr Gly His Asp Phe Gln Ala Leu Leu Leu Asn Ala Asp Lys Arg
           115                 120                 125
   gaa tcc ggt gct tca gta ttc gct tat cat gtt cat gac cca gaa cgc     432
   Glu Ser Gly Ala Ser Val Phe Ala Tyr His Val His Asp Pro Glu Arg
       130                 135                 140
   tat ggc gta gcg gag ttt gac gat agc ggt cgc gta ttg tcg ctg gaa     480
   Tyr Gly Val Ala Glu Phe Asp Asp Ser Gly Arg Val Leu Ser Leu Glu
   145                 150                 155                 160
   gaa aaa ccg gca gtt cca aag tct agc tat gcg gtc acc ggc ctg tat     528
   Glu Lys Pro Ala Val Pro Lys Ser Ser Tyr Ala Val Thr Gly Leu Tyr
                   165                 170                 175
   ttc tat gac aat cag gta gtc aat ctg gct cgc gag ctg aag cct tcc     576
   Phe Tyr Asp Asn Gln Val Val Asn Leu Ala Arg Glu Leu Lys Pro Ser
               180                 185                 190
   cca cgt ggc gag ctg gaa atc acc gac ctc aac aac ctt tac ttg cag     624
   Pro Arg Gly Glu Leu Glu Ile Thr Asp Leu Asn Asn Leu Tyr Leu Gln
           195                 200                 205
   cag cag cag ttg cag gtc gaa atc atg ggc cgt ggc tat gcg tgg ctc     672
   Gln Gln Gln Leu Gln Val Glu Ile Met Gly Arg Gly Tyr Ala Trp Leu
       210                 215                 220
   gac acc ggc acg cac gac agt ctg ctg gag gct agc cag tac atc gca     720
   Asp Thr Gly Thr His Asp Ser Leu Leu Glu Ala Ser Gln Tyr Ile Ala
   225                 230                 235                 240
   acc atg gag cgc cgt cag ggc ttg aaa gtc gcc tgc cct gag gaa att        768
   Thr Met Glu Arg Arg Gln Gly Leu Lys Val Ala Cys Pro Glu Glu Ile
                   245                     250                 255
   tgc tac cgc gct ggc tgg atc aac gct gag caa ctc gag tgc ctg gct        816
   Cys Tyr Arg Ala Gly Trp Ile Asn Ala Glu Gln Leu Glu Cys Leu Ala
               260                     265                 270
   caa cca ctg ctg aaa aac ggt tat ggc aag tat ctg cag aac ttg ctg        864
   Gln Pro Leu Leu Lys Asn Gly Tyr Gly Lys Tyr Leu Gln Asn Leu Leu
           275                     280                 285
   aaa gag aag gtg ttc tga                                                882
   Lys Glu Lys Val Phe
       290
<210> 10
   <211> 293
   <212> PRT
   <213> Pseudomonas putida
<400> 10
   Met Ala Arg Lys Gly Ile Ile Leu Ala Gly Gly Ser Gly Thr Arg Leu
   1               5                   10                  15
   His Pro Ala Thr Leu Ser Val Ser Lys Gln Leu Leu Pro Val Tyr Asp
               20                  25                  30
   Lys Pro Met Ile Tyr Tyr Pro Leu Ser Thr Leu Leu Leu Ala Gly Ile
           35                  40                  45
   Arg Asp Ile Leu Ile Ile Ser Thr Pro Gln Asp Thr Pro Arg Phe Glu
       50                  55                  60
   Gln Leu Leu Gly Asp Gly Ser Gln Trp Gly Leu Asn Leu Ser Tyr Ala
   65                  70                  75                  80
   Ile Gln Pro Ser Pro Asp Gly Leu Ala Gln Ala Phe Thr Ile Gly Ala
                   85                  90                  95
   Asp Phe Ile Gly Asn Asp Pro Ser Ala Leu Val Leu Gly Asp Asn Ile
               100                 105                 110
   Phe Tyr Gly His Asp Phe Gln Ala Leu Leu Leu Asn Ala Asp Lys Arg
           115                 120                 125
   Glu Ser Gly Ala Ser Val Phe Ala Tyr His Val His Asp Pro Glu Arg
       130                 135                 140
   Tyr Gly Val Ala Glu Phe Asp Asp Ser Gly Arg Val Leu Ser Leu Glu
   145                 150                 155                 160
   Glu Lys Pro Ala Val Pro Lys Ser Ser Tyr Ala Val Thr Gly Leu Tyr
                   165                 170                 175
   Phe Tyr Asp Asn Gln Val Val Asn Leu Ala Arg Glu Leu Lys Pro Ser
               180                 185                 190
   Pro Arg Gly Glu Leu Glu Ile Thr Asp Leu Asn Asn Leu Tyr Leu Gln
           195                 200                 205
   Gln Gln Gln Leu Gln Val Glu Ile Met Gly Arg Gly Tyr Ala Trp Leu
       210                 215                 220
   Asp Thr Gly Thr His Asp Ser Leu Leu Glu Ala Ser Gln Tyr Ile Ala
   225                 230                 235                 240
   Thr Met Glu Arg Arg Gln Gly Leu Lys Val Ala Cys Pro Glu Glu Ile
                   245                 250                 255
   Cys Tyr Arg Ala Gly Trp Ile Asn Ala Glu Gln Leu Glu Cys Leu Ala
               260                 265                 270
   Gln Pro Leu Leu Lys Asn Gly Tyr Gly Lys Tyr Leu Gln Asn Leu Leu
           275                 280                 285
   Lys Glu Lys Val Phe
       290
<210> 11
   <211> 1101
   <212> DNA
   <213> Pseudomonas putida
<220>
   <221> CDS
   <222> (1)..(1101)
<400> 11
   atg att cta gta aca ggc gga gcc ggc ttc atc ggc tca aat ttc gta          48
   Met Ile Leu Val Thr Gly Gly Ala Gly Phe Ile Gly Ser Asn Phe Val
   1               5                   10                  15
   ctg caa tgg tgt gcg cac aat gag gaa ccc gtc ctc aac ctc gac gcc          96
   Leu Gln Trp Cys Ala His Asn Glu Glu Pro Val Leu Asn Leu Asp Ala
               20                  25                  30
   ctg acc tac gca ggc aac ctg gcc aac ctg cag ccg ctg gaa ggc aac         144
   Leu Thr Tyr Ala Gly Asn Leu Ala Asn Leu Gln Pro Leu Glu Gly Asn
           35                  40                  45
   cct cag cat cgc ttt gtg caa ggc aat att tgc gat gct gcg ctt ctg         192
   Pro Gln His Arg Phe Val Gln Gly Asn Ile Cys Asp Ala Ala Leu Leu
       50                  55                  60
   acc aag ctg ttc gca gag cac cgc ccg cgc gcc gtg gtt cac ttc gcg         240
   Thr Lys Leu Phe Ala Glu His Arg Pro Arg Ala Val Val His Phe Ala
   65                  70                  75                  80
   gcg gaa tcc cat gta gac cgc tca atc acc ggc ccc gaa gcg ttt gtc       288
   Ala Glu Ser His Val Asp Arg Ser Ile Thr Gly Pro Glu Ala Phe Val
                   85                  90                  95
   gaa acc aac gtg atg ggc acg ttt cgc ttg ctt gaa gcc gcc cgg gcg       336
   Glu Thr Asn Val Met Gly Thr Phe Arg Leu Leu Glu Ala Ala Arg Ala
               100                 105                 110
   cat tgg aat agt ttg gaa ggt gca gag aag gag gcc ttc cgt ttc ctc       384
   His Trp Asn Ser Leu Glu Gly Ala Glu Lys Glu Ala Phe Arg Phe Leu
           115                 120                 125
   cat gtc tct acc gac gaa gtc tac ggc aca cta ggg cca aac gac ccg       432
   His Val Ser Thr Asp Glu Val Tyr Gly Thr Leu Gly Pro Asn Asp Pro
       130                 135                 140
   gcg ttc acc gaa acc acg ccg tac gcg ccg aac agc cca tac tcc gcc       480
   Ala Phe Thr Glu Thr Thr Pro Tyr Ala Pro Asn Ser Pro Tyr Ser Ala
   145                 150                 155                 160
   agc aag gca gcc agc gac cat ctg gta cgc tcg tat ttc cat acc tac       528
   Ser Lys Ala Ala Ser Asp His Leu Val Arg Ser Tyr Phe His Thr Tyr
                   165                 170                 175
   ggc atg ccg gta ctc act acc aac tgc tcc aac aat tac ggg ccg ctc       576
   Gly Met Pro Val Leu Thr Thr Asn Cys Ser Asn Asn Tyr Gly Pro Leu
               180                 185                 190
   cac ttc ccg gaa aaa ctg atc ccg ctg atg atc gtc aac gca ctc gcc       624
   His Phe Pro Glu Lys Leu Ile Pro Leu Met Ile Val Asn Ala Leu Ala
           195                 200                 205
   ggt aag gcg ctg cct gtc tat ggc gac ggc cag caa atc cgc gac tgg       672
   Gly Lys Ala Leu Pro Val Tyr Gly Asp Gly Gln Gln Ile Arg Asp Trp
       210                 215                 220
   ctg tat gtc gaa gat cac tgc tcg ggc atc cgt cgc gta ctg gaa gcc       720
   Leu Tyr Val Glu Asp His Cys Ser Gly Ile Arg Arg Val Leu Glu Ala
   225                 230                 235                 240
   ggt gcg ttc ggc gag acg tac aat att ggc ggc tgg aat gaa aaa gcc       768
   Gly Ala Phe Gly Glu Thr Tyr Asn Ile Gly Gly Trp Asn Glu Lys Ala
                   245                 250                 255
   aac att gac att gtg cgt aca ctc tgc agc ctt ctc gac gag atg gca       816
   Asn Ile Asp Ile Val Arg Thr Leu Cys Ser Leu Leu Asp Glu Met Ala
               260                 265                 270
   cct gcg gca tcg cgc cag gta atc aat cag aag acc ggc gag cct gtc       864
   Pro Ala Ala Ser Arg Gln Val Ile Asn Gln Lys Thr Gly Glu Pro Val
           275                 280                 285
   gaa cag tat gca gaa ctc atc gcc tac gta acc gac cgc cca ggc cat       912
   Glu Gln Tyr Ala Glu Leu Ile Ala Tyr Val Thr Asp Arg Pro Gly His
       290                 295                 300
   gac cgc cgt tat gcc atc gat gca cgc aag atc gag cgg gag ctc ggc       960
   Asp Arg Arg Tyr Ala Ile Asp Ala Arg Lys Ile Glu Arg Glu Leu Gly
   305                 310                 315                 320
   tgg aaa cct gcc gaa acc ttc gag acg ggc att cga aag aca gtc gct      1008
   Trp Lys Pro Ala Glu Thr Phe Glu Thr Gly Ile Arg Lys Thr Val Ala
                   325                 330                 335
   tgg tac ttg gcc aac cag aaa tgg gta aaa ggt gtc atg gac ggc agc       1056
   Trp Tyr Leu Ala Asn Gln Lys Trp Val Lys Gly Val Met Asp Gly Ser
               340                 345                 350
   tac cgt gac tgg gtg gca caa caa tac ggg gca aat aaa gcg tga           1101
   Tyr Arg Asp Trp Val Ala Gln Gln Tyr Gly Ala Asn Lys Ala
           355                 360                 365
<210> 12
   <211> 366
   <212> PRT
   <213> Pseudomonas putida
<400> 12
   Met Ile Leu Val Thr Gly Gly Ala Gly Phe Ile Gly Ser Asn Phe Val
   1               5                   10                  15
   Leu Gln Trp Cys Ala His Asn Glu Glu Pro Val Leu Asn Leu Asp Ala
               20                  25                  30
   Leu Thr Tyr Ala Gly Asn Leu Ala Asn Leu Gln Pro Leu Glu Gly Asn
           35                  40                  45
   Pro Gln His Arg Phe Val Gln Gly Asn Ile Cys Asp Ala Ala Leu Leu
       50                  55                  60
   Thr Lys Leu Phe Ala Glu His Arg Pro Arg Ala Val Val His Phe Ala
   65                  70                  75                  80
   Ala Glu Ser His Val Asp Arg Ser Ile Thr Gly Pro Glu Ala Phe Val
                   85                  90                  95
   Glu Thr Asn Val Met Gly Thr Phe Arg Leu Leu Glu Ala Ala Arg Ala
               100                 105                 110
   His Trp Asn Ser Leu Glu Gly Ala Glu Lys Glu Ala Phe Arg Phe Leu
           115                 120                 125
   His Val Ser Thr Asp Glu Val Tyr Gly Thr Leu Gly Pro Asn Asp Pro
       130                 135                 140
   Ala Phe Thr Glu Thr Thr Pro Tyr Ala Pro Asn Ser Pro Tyr Ser Ala
   145                 150                 155                 160
   Ser Lys Ala Ala Ser Asp His Leu Val Arg Ser Tyr Phe His Thr Tyr
                   165                 170                 175
   Gly Met Pro Val Leu Thr Thr Asn Cys Ser Asn Asn Tyr Gly Pro Leu
               180                 185                 190
   His Phe Pro Glu Lys Leu Ile Pro Leu Met Ile Val Asn Ala Leu Ala
           195                 200                 205
   Gly Lys Ala Leu Pro Val Tyr Gly Asp Gly Gln Gln Ile Arg Asp Trp
       210                 215                 220
   Leu Tyr Val Glu Asp His Cys Ser Gly Ile Arg Arg Val Leu Glu Ala
   225                 230                 235                 240
   Gly Ala Phe Gly Glu Thr Tyr Asn Ile Gly Gly Trp Asn Glu Lys Ala
                   245                 250                 255
   Asn Ile Asp Ile Val Arg Thr Leu Cys Ser Leu Leu Asp Glu Met Ala
               260                 265                 270
   Pro Ala Ala Ser Arg Gln Val Ile Asn Gln Lys Thr Gly Glu Pro Val
           275                 280                 285
   Glu Gln Tyr Ala Glu Leu Ile Ala Tyr Val Thr Asp Arg Pro Gly His
       290                 295                 300
   Asp Arg Arg Tyr Ala Ile Asp Ala Arg Lys Ile Glu Arg Glu Leu Gly
   305                 310                 315                 320
   Trp Lys Pro Ala Glu Thr Phe Glu Thr Gly Ile Arg Lys Thr Val Ala
                   325                 330                 335
   Trp Tyr Leu Ala Asn Gln Lys Trp Val Lys Gly Val Met Asp Gly Ser
               340                 345                 350
   Tyr Arg Asp Trp Val Ala Gln Gln Tyr Gly Ala Asn Lys Ala
           355                 360                 365
<210> 13
   <211> 549
   <212> DNA
   <213> Pseudomonas putida
<220>
   <221> CDS
   <222> (1)..(549)
<400> 13
   atg caa gcc att ccg ctg gat atc ccc gaa gtc gtg ctg ttt acc ccc         48
   Met Gln Ala Ile Pro Leu Asp Ile Pro Glu Val Val Leu Phe Thr Pro
   1               5                   10                  15
   aag gtt ttt ggc gac gaa cgt ggt ttc ttc tac gag agc ttc aac gcc         96
   Lys Val Phe Gly Asp Glu Arg Gly Phe Phe Tyr Glu Ser Phe Asn Ala
               20                  25                  30
   cgt gtt ttc agc gaa gtg acc ggc ctg cag ccc gac ttc gta caa gac        144
   Arg Val Phe Ser Glu Val Thr Gly Leu Gln Pro Asp Phe Val Gln Asp
           35                  40                  45
   aac cac tcg cgc tcg gta aaa ggc gtg ctc cgt ggc ctg cac tat cag        192
   Asn His Ser Arg Ser Val Lys Gly Val Leu Arg Gly Leu His Tyr Gln
       50                  55                  60
   ctg gca cct cac gcc cag ggc aag ctg gtg cgt gtg gtg caa ggc gaa        240
   Leu Ala Pro His Ala Gln Gly Lys Leu Val Arg Val Val Gln Gly Glu
   65                  70                  75                  80
   gtc ttc gat gtt gcg gtg gat atc cgt cgc tcg tcc aca acc ttc ggt        288
   Val Phe Asp Val Ala Val Asp Ile Arg Arg Ser Ser Thr Thr Phe Gly
                   85                  90                  95
   aaa tgg gta ggt gcg gtg ttg tcg gcc gag aac aag aac cag ctg tgg        336
   Lys Trp Val Gly Ala Val Leu Ser Ala Glu Asn Lys Asn Gln Leu Trp
               100                 105                 110
   atc ccg cca ggg ttc gca cac ggg ttc gtc acg ttg agt gaa acc gca        384
   Ile Pro Pro Gly Phe Ala His Gly Phe Val Thr Leu Ser Glu Thr Ala
           115                 120                 125
   gag ttc ctc tac aag acc acc gac ttc tac tcg ccg cag tgc gag cgc        432
   Glu Phe Leu Tyr Lys Thr Thr Asp Phe Tyr Ser Pro Gln Cys Glu Arg
       130                 135                 140
   tgc att gcc tgg aat gat ccg gca gtg ggt atc gaa tgg ccc atc gac        480
   Cys Ile Ala Trp Asn Asp Pro Ala Val Gly Ile Glu Trp Pro Ile Asp
   145                 150                 155                 160
   tcc gta cca agc ttg tct ggc aag gac cag ctt ggg gtc gca ttg gct        528
   Ser Val Pro Ser Leu Ser Gly Lys Asp Gln Leu Gly Val Ala Leu Ala
                   165                 170                 175
   gac gcc gaa ctg ttc gac taa                                            549
   Asp Ala Glu Leu Phe Asp
               180
<210> 14
   <211> 182
   <212> PRT
   <213> Pseudomonas putida
<400> 14
   Met Gln Ala Ile Pro Leu Asp Ile Pro Glu Val Val Leu Phe Thr Pro
   1               5                   10                  15
   Lys Val Phe Gly Asp Glu Arg Gly Phe Phe Tyr Glu Ser Phe Asn Ala
               20                  25                  30
   Arg Val Phe Ser Glu Val Thr Gly Leu Gln Pro Asp Phe Val Gln Asp
           35                  40                  45
   Asn His Ser Arg Ser Val Lys Gly Val Leu Arg Gly Leu His Tyr Gln
       50                  55                  60
   Leu Ala Pro His Ala Gln Gly Lys Leu Val Arg Val Val Gln Gly Glu
   65                  70                  75                  80
   Val Phe Asp Val Ala Val Asp Ile Arg Arg Ser Ser Thr Thr Phe Gly
                   85                  90                  95
   Lys Trp Val Gly Ala Val Leu Ser Ala Glu Asn Lys Asn Gln Leu Trp
               100                 105                 110
   Ile Pro Pro Gly Phe Ala His Gly Phe Val Thr Leu Ser Glu Thr Ala
           115                 120                 125
   Glu Phe Leu Tyr Lys Thr Thr Asp Phe Tyr Ser Pro Gln Cys Glu Arg
       130                 135                 140
   Cys Ile Ala Trp Asn Asp Pro Ala Val Gly Ile Glu Trp Pro Ile Asp
   145                 150                 155                 160
   Ser Val Pro Ser Leu Ser Gly Lys Asp Gln Leu Gly Val Ala Leu Ala
                   165                 170                 175
   Asp Ala Glu Leu Phe Asp
               180
<210> 15
   <211> 903
   <212> DNA
   <213> Pseudomonas putida
<220>
   <221> CDS
   <222> (1)..(903)
<400> 15
   gtg aaa atc ctg ctg ttg ggg aaa aac ggg caa gta ggc tgg gag cta        48
   Val Lys Ile Leu Leu Leu Gly Lys Asn Gly Gln Val Gly Trp Glu Leu
   1               5                   10                  15
   cag cgc gcc ttg gcg ccg ctg ggt gag gtc att gcg ctg gat cgt cag        96
   Gln Arg Ala Leu Ala Pro Leu Gly Glu Val Ile Ala Leu Asp Arg Gln
               20                  25                  30
   ggg gcc gag ggc tta tgt ggc gac ttg tcc aac ctg gac ggc ttg gcc       144
   Gly Ala Glu Gly Leu Cys Gly Asp Leu Ser Asn Leu Asp Gly Leu Ala
           35                  40                  45
   gct acg att cgt cag ctg gcg ccg gac gtg atc gtc aac gct gct gcc       192
   Ala Thr Ile Arg Gln Leu Ala Pro Asp Val Ile Val Asn Ala Ala Ala
       50                  55                  60
   tac act gca gtg gat aaa gct gag agc gat cag gca ctg gct gca atg       240
   Tyr Thr Ala Val Asp Lys Ala Glu Ser Asp Gln Ala Leu Ala Ala Met
   65                  70                  75                  80
   atc aat gcc gcg gct cct gct gta tta gca cgt gaa aca gca gct ttg       288
   Ile Asn Ala Ala Ala Pro Ala Val Leu Ala Arg Glu Thr Ala Ala Leu
                   85                  90                  95
   ggc gcc tgg ttg att cac tat tcc acc gat tat gta ttt gac ggc agc       336
   Gly Ala Trp Leu Ile His Tyr Ser Thr Asp Tyr Val Phe Asp Gly Ser
               100                 105                 110
   ggc agt cag cgc tgg gag gaa act gcg cct acc ggc ccc ctt tcg gtc        384
   Gly Ser Gln Arg Trp Glu Glu Thr Ala Pro Thr Gly Pro Leu Ser Val
           115                 120                 125
   tac ggc cgg acc aag ctg gaa ggc gag cat gcc att ctc gcc agc ggc        432
   Tyr Gly Arg Thr Lys Leu Glu Gly Glu His Ala Ile Leu Ala Ser Gly
       130                 135                 140
   gcc aag gcc gtg gta ctg cgc acc agc tgg gtg tat gct gcg cgc ggg        480
   Ala Lys Ala Val Val Leu Arg Thr Ser Trp Val Tyr Ala Ala Arg Gly
   145                 150                 155                 160
   cac aat ttt gcc aag acc atg ctg cgc ctg gcg gcg gag cgt gag acg        528
   His Asn Phe Ala Lys Thr Met Leu Arg Leu Ala Ala Glu Arg Glu Thr
                   165                 170                 175
   ttg agc gtg gta gca gac caa ttt ggc gca ccc acg ggc gct gac ctg        576
   Leu Ser Val Val Ala Asp Gln Phe Gly Ala Pro Thr Gly Ala Asp Leu
               180                 185                 190
   atc gcc gac gtt act gca cac atc ctg cgg caa atc ttc aat ggg caa        624
   Ile Ala Asp Val Thr Ala His Ile Leu Arg Gln Ile Phe Asn Gly Gln
           195                 200                 205
   gac aac cgt cac ctg gca ggg att tac cac ttg gct gcg tcc ggt gaa        672
   Asp Asn Arg His Leu Ala Gly Ile Tyr His Leu Ala Ala Ser Gly Glu
       210                 215                 220
   acc tct tgg cat ggt ttt gct cag ttc gtg ctg gcg cat gct caa cgc        720
   Thr Ser Trp His Gly Phe Ala Gln Phe Val Leu Ala His Ala Gln Arg
   225                 230                 235                 240
   act ggc gta gcg ctg aaa gtg aca gct gat aag gtt gcc gca atc agc        768
   Thr Gly Val Ala Leu Lys Val Thr Ala Asp Lys Val Ala Ala Ile Ser
                   245                 250                 255
   acc gaa gct tat cca gta cct gca cca cgt ccg cgc aac tcg cgc ctg        816
   Thr Glu Ala Tyr Pro Val Pro Ala Pro Arg Pro Arg Asn Ser Arg Leu
               260                 265                 270
   gca ctg ggc aaa ctg gaa aac acg ttc aat ttc aaa atg ccg ctt tgg        864
   Ala Leu Gly Lys Leu Glu Asn Thr Phe Asn Phe Lys Met Pro Leu Trp
           275                 280                 285
   gag caa ggc gtg caa cgt atg ctg gac gaa atc cag taa                    903
   Glu Gln Gly Val Gln Arg Met Leu Asp Glu Ile Gln
       290                 295                 300
<210> 16
   <211> 300
   <212> PRT
   <213> Pseudomonas putida
<400> 16
   Val Lys Ile Leu Leu Leu Gly Lys Asn Gly Gln Val Gly Trp Glu Leu
   1               5                   10                  15
   Gln Arg Ala Leu Ala Pro Leu Gly Glu Val Ile Ala Leu Asp Arg Gln
               20                  25                  30
   Gly Ala Glu Gly Leu Cys Gly Asp Leu Ser Asn Leu Asp Gly Leu Ala
           35                  40                  45
   Ala Thr Ile Arg Gln Leu Ala Pro Asp Val Ile Val Asn Ala Ala Ala
       50                  55                  60
   Tyr Thr Ala Val Asp Lys Ala Glu Ser Asp Gln Ala Leu Ala Ala Met
   65                  70                  75                  80
   Ile Asn Ala Ala Ala Pro Ala Val Leu Ala Arg Glu Thr Ala Ala Leu
                   85                  90                  95
   Gly Ala Trp Leu Ile His Tyr Ser Thr Asp Tyr Val Phe Asp Gly Ser
               100                 105                 110
   Gly Ser Gln Arg Trp Glu Glu Thr Ala Pro Thr Gly Pro Leu Ser Val
           115                 120                 125
   Tyr Gly Arg Thr Lys Leu Glu Gly Glu His Ala Ile Leu Ala Ser Gly
       130                 135                 140
   Ala Lys Ala Val Val Leu Arg Thr Ser Trp Val Tyr Ala Ala Arg Gly
   145                 150                 155                 160
   His Asn Phe Ala Lys Thr Met Leu Arg Leu Ala Ala Glu Arg Glu Thr
                   165                 170                 175
   Leu Ser Val Val Ala Asp Gln Phe Gly Ala Pro Thr Gly Ala Asp Leu
               180                 185                 190
   Ile Ala Asp Val Thr Ala His Ile Leu Arg Gln Ile Phe Asn Gly Gln
           195                 200                 205
   Asp Asn Arg His Leu Ala Gly Ile Tyr His Leu Ala Ala Ser Gly Glu
       210                 215                 220
   Thr Ser Trp His Gly Phe Ala Gln Phe Val Leu Ala His Ala Gln Arg
   225                 230                 235                 240
   Thr Gly Val Ala Leu Lys Val Thr Ala Asp Lys Val Ala Ala Ile Ser
                   245                 250                 255
   Thr Glu Ala Tyr Pro Val Pro Ala Pro Arg Pro Arg Asn Ser Arg Leu
               260                 265                 270
   Ala Leu Gly Lys Leu Glu Asn Thr Phe Asn Phe Lys Met Pro Leu Trp
           275                 280                 285
   Glu Gln Gly Val Gln Arg Met Leu Asp Glu Ile Gln
       290                 295                 300
<210> 17
   <211> 1041
   <212> DNA
   <213> Burkholderia thailandensis
<220>
   <221> CDS
   <222> (1)..(1041)
<400> 17
   atg cgc ggt tcc ggc gag tgg gta gcc gct gcg gcg cgc gtg agg cag        48
   Met Arg Gly Ser Gly Glu Trp Val Ala Ala Ala Ala Arg Val Arg Gln
   1               5                   10                  15
   ggc ggg cag atc gcg cgg gag ggc ggc tac gtg gag gcg tcc atc aag        96
   Gly Gly Gln Ile Ala Arg Glu Gly Gly Tyr Val Glu Ala Ser Ile Lys
               20                  25                  30
   ggc gcg gga tca gct cat ttg ccg agc aga tgc ggg aga tac gcc atg       144
   Gly Ala Gly Ser Ala His Leu Pro Ser Arg Cys Gly Arg Tyr Ala Met
           35                  40                  45
   cct atc gag aaa cag gtg gta gcg ctg ccg agc gga ctg aag gtc cac       192
   Pro Ile Glu Lys Gln Val Val Ala Leu Pro Ser Gly Leu Lys Val His
       50                  55                  60
   gtc gag cgt cat gtg ttc gat ccg gcc ttc gag acg gtc atc ctc gtg       240
   Val Glu Arg His Val Phe Asp Pro Ala Phe Glu Thr Val Ile Leu Val
   65                  70                  75                  80
   aac ggc gcg ctg gcg acg acc gcg tcg ttc ggc cag acg att cgc tac       288
   Asn Gly Ala Leu Ala Thr Thr Ala Ser Phe Gly Gln Thr Ile Arg Tyr
                   85                  90                  95
   ctg ggc gaa cgc gtg aac gcg gtg tgc ttc gac ttg ccg tac gcg ggc       336
   Leu Gly Glu Arg Val Asn Ala Val Cys Phe Asp Leu Pro Tyr Ala Gly
               100                 105                 110
   cag tcg cgc cag cac aat ccg ggc gag tac att ctg acg aag gac gac       384
   Gln Ser Arg Gln His Asn Pro Gly Glu Tyr Ile Leu Thr Lys Asp Asp
           115                 120                 125
   gag gtg gag att ctg ctg cac ctg gcc gag cgg ttc gag ccg agt ttc       432
   Glu Val Glu Ile Leu Leu His Leu Ala Glu Arg Phe Glu Pro Ser Phe
       130                 135                 140
   ctg ctg tcg gtg tcg tgg ggc ggg gtg gcg tcg ctg ttc gcg ctg gcg       480
   Leu Leu Ser Val Ser Trp Gly Gly Val Ala Ser Leu Phe Ala Leu Ala
   145                 150                 155                 160
   cgg ggg tgc gcg agc gtg cgg cgg gcg gtg atc gcg tcg ttc tcg ccg       528
   Arg Gly Cys Ala Ser Val Arg Arg Ala Val Ile Ala Ser Phe Ser Pro
                   165                 170                 175
   ttc ctg aac gac gcg atg acg gat tac gtg acg cgc gcg cgc gat cac       576
   Phe Leu Asn Asp Ala Met Thr Asp Tyr Val Thr Arg Ala Arg Asp His
               180                 185                 190
   atc gcg gcg ggg gag aac ctg aag gcg gcg cag ttg ctc aac gac acg       624
   Ile Ala Ala Gly Glu Asn Leu Lys Ala Ala Gln Leu Leu Asn Asp Thr
           195                 200                 205
   gtg ggg cgc tac ctg ccg cgg atc atg aag ctg tac aac tac cgg tat        672
   Val Gly Arg Tyr Leu Pro Arg Ile Met Lys Leu Tyr Asn Tyr Arg Tyr
       210                 215                 220
   ctg acg aag ctg ccg cgc acc gag cag gac cag gtg gcg ttc cac gtc        720
   Leu Thr Lys Leu Pro Arg Thr Glu Gln Asp Gln Val Ala Phe His Val
   225                 230                 235                 240
   gac cag atc ctg tcg atg cgg ccg gag cag tac ctg ccg gaa ttc cgc        768
   Asp Gln Ile Leu Ser Met Arg Pro Glu Gln Tyr Leu Pro Glu Phe Arg
                   245                 250                 255
   cag atc ggc tgc gcg gtg aag ttc atc aac ggc gag ctg gac gag tac        816
   Gln Ile Gly Cys Ala Val Lys Phe Ile Asn Gly Glu Leu Asp Glu Tyr
               260                 265                 270
   acg acg gcg tcg gac gtg cgg cgg ctg gcg gcc tac gtg cgg cgc gcg        864
   Thr Thr Ala Ser Asp Val Arg Arg Leu Ala Ala Tyr Val Arg Arg Ala
           275                 280                 285
   gag ttc gcg acg atc cgg cag gcg ggg cac ttc ctg gac ctc gag ggg        912
   Glu Phe Ala Thr Ile Arg Gln Ala Gly His Phe Leu Asp Leu Glu Gly
       290                 295                 300
   cgt cag cag cag gag cag ctt cgc gcg gcg atc ctg ggc ttc ttc ggc        960
   Arg Gln Gln Gln Glu Gln Leu Arg Ala Ala Ile Leu Gly Phe Phe Gly
   305                 310                 315                 320
   gac gag cgg gcg agc gcg gcg cgc gac gac gcg cag gac gag acg ctc       1008
   Asp Glu Arg Ala Ser Ala Ala Arg Asp Asp Ala Gln Asp Glu Thr Leu
                   325                 330                 335
   gcg ccg ctc ggt cag ttg ccg gcg ctg tcg tag                           1041
   Ala Pro Leu Gly Gln Leu Pro Ala Leu Ser
               340                 345
<210> 18
   <211> 346
   <212> PRT
   <213> Burkholderia thailandensis
<400> 18
   Met Arg Gly Ser Gly Glu Trp Val Ala Ala Ala Ala Arg Val Arg Gln
   1               5                   10                  15
   Gly Gly Gln Ile Ala Arg Glu Gly Gly Tyr Val Glu Ala Ser Ile Lys
               20                  25                  30
   Gly Ala Gly Ser Ala His Leu Pro Ser Arg Cys Gly Arg Tyr Ala Met
           35                  40                  45
   Pro Ile Glu Lys Gln Val Val Ala Leu Pro Ser Gly Leu Lys Val His
       50                  55                  60
   Val Glu Arg His Val Phe Asp Pro Ala Phe Glu Thr Val Ile Leu Val
   65                  70                  75                  80
   Asn Gly Ala Leu Ala Thr Thr Ala Ser Phe Gly Gln Thr Ile Arg Tyr
   85 90 95
   Leu Gly Glu Arg Val Asn Ala Val Cys Phe Asp Leu Pro Tyr Ala Gly
   100 105 110
   Gln Ser Arg Gln His Asn Pro Gly Glu Tyr Ile Leu Thr Lys Asp Asp
   115 120 125
   Glu Val Glu Ile Leu Leu His Leu Ala Glu Arg Phe Glu Pro Ser Phe
   130 135 140
   Leu Leu Ser Val Ser Trp Gly Gly Val Ala Ser Leu Phe Ala Leu Ala
   145 150 155 160
   Arg Gly Cys Ala Ser Val Arg Arg Ala Val Ile Ala Ser Phe Ser Pro
   165 170 175
   Phe Leu Asn Asp Ala Met Thr Asp Tyr Val Thr Arg Ala Arg Asp His
   180 185 190
   Ile Ala Ala Gly Glu Asn Leu Lys Ala Ala Gln Leu Leu Asn Asp Thr
   195 200 205
   Val Gly Arg Tyr Leu Pro Arg Ile Met Lys Leu Tyr Asn Tyr Arg Tyr
   210 215 220
   Leu Thr Lys Leu Pro Arg Thr Glu Gln Asp Gln Val Ala Phe His Val
   225 230 235 240
   Asp Gln Ile Leu Ser Met Arg Pro Glu Gln Tyr Leu Pro Glu Phe Arg
   245 250 255
   Gln Ile Gly Cys Ala Val Lys Phe Ile Asn Gly Glu Leu Asp Glu Tyr
   260 265 270
   Thr Thr Ala Ser Asp Val Arg Arg Leu Ala Ala Tyr Val Arg Arg Ala
   275 280 285
   Glu Phe Ala Thr Ile Arg Gln Ala Gly His Phe Leu Asp Leu Glu Gly
   290 295 300
   Arg Gln Gln Gln Glu Gln Leu Arg Ala Ala Ile Leu Gly Phe Phe Gly
   305 310 315 320
   Asp Glu Arg Ala Ser Ala Ala Arg Asp Asp Ala Gln Asp Glu Thr Leu
   325 330 335
   Ala Pro Leu Gly Gln Leu Pro Ala Leu Ser
   340 345
<210> 19
   <211> 1482
   <212> DNA
   <213> Burkholderia thailandensis
<220>
   <221> CDS
   <222> (1)..(1482)
<400> 19
   atg gat gcg ggc cgg atc ggg ctg cat gac gcg gcg gcg gcg ggg cgc      48
   Met Asp Ala Gly Arg Ile Gly Leu His Asp Ala Ala Ala Ala Gly Arg
   1               5                   10                  15
   atc ggc atg acg gaa gcg ttc gcg tca cgg gcc cgt tgt tcg gcg gcg      96
   Ile Gly Met Thr Glu Ala Phe Ala Ser Arg Ala Arg Cys Ser Ala Ala
               20                  25                  30
   gcc ctg gcg gca ggc gga cga gcc ccg gcc ggc gat gga cga tcc ggt     144
   Ala Leu Ala Ala Gly Gly Arg Ala Pro Ala Gly Asp Gly Arg Ser Gly
           35                  40                  45
   tcg aac agg gct gcg gcg aac ggt tcc gtc gac tgc cgc gcg gga tgg     192
   Ser Asn Arg Ala Ala Ala Asn Gly Ser Val Asp Cys Arg Ala Gly Trp
       50                  55                  60
   aat gac gag gcg atg gct aaa gta atc gtg acg gcg atc ggg tcg gcg     240
   Asn Asp Glu Ala Met Ala Lys Val Ile Val Thr Ala Ile Gly Ser Ala
   65                  70                  75                  80
   ggc gac gtg cac ccg ttg ctg ggg gtg agc cgg gcg ctg tcg gcg cgg     288
   Gly Asp Val His Pro Leu Leu Gly Val Ser Arg Ala Leu Ser Ala Arg
                   85                  90                  95
   ggc cac gag gtg gtg ttc tgc acg cat gcg ccg ttc gag gcg gcg gtg     336
   Gly His Glu Val Val Phe Cys Thr His Ala Pro Phe Glu Ala Ala Val
               100                 105                 110
   cgc gcg agc ggc ttc gcg ttc gtg ccg gtg ggc acg gcc gag gac tac     384
   Arg Ala Ser Gly Phe Ala Phe Val Pro Val Gly Thr Ala Glu Asp Tyr
           115                 120                 125
   gtg cgg gcg atg gcg gac ccg gcg ctg tgg gat ccg cgc acg tcg ttc     432
   Val Arg Ala Met Ala Asp Pro Ala Leu Trp Asp Pro Arg Thr Ser Phe
       130                 135                 140
   aag acg ctg tgg cgg gtg atc gcg ccg gtg gtg agg ccg cac ttc gag     480
   Lys Thr Leu Trp Arg Val Ile Ala Pro Val Val Arg Pro His Phe Glu
   145                 150                 155                 160
   gtg ctg cgc gcg ctg agc gac gcg gac acg gtg ctg gtg ggc acg ctg     528
   Val Leu Arg Ala Leu Ser Asp Ala Asp Thr Val Leu Val Gly Thr Leu
                   165                 170                 175
   tgg gcg ttc tcg gcg cgg ctg atg cag gag cgc ttc ggc acg cgg tac     576
   Trp Ala Phe Ser Ala Arg Leu Met Gln Glu Arg Phe Gly Thr Arg Tyr
               180                 185                 190
   gtg tcg gtg cag gtg tcg ccg tcg acg ctg ctg tcg gcg cat gcg ccg     624
   Val Ser Val Gln Val Ser Pro Ser Thr Leu Leu Ser Ala His Ala Pro
           195                 200                 205
   ccg acg cac aag cgg ctg acg atc ccg aag ggc ctg ccg ctg gcg gtg       672
   Pro Thr His Lys Arg Leu Thr Ile Pro Lys Gly Leu Pro Leu Ala Val
       210                 215                 220
   aag gcg ggg ctg atg acg ctg atc gag cgg cag gtg ctg gac cgg gtg       720
   Lys Ala Gly Leu Met Thr Leu Ile Glu Arg Gln Val Leu Asp Arg Val
   225                 230                 235                 240
   tgc ggc ccg gag ctg aac gcg gcg cgg cag gcg ctg ggc ctg gcg ccg       768
   Cys Gly Pro Glu Leu Asn Ala Ala Arg Gln Ala Leu Gly Leu Ala Pro
                   245                 250                 255
   gcg aag cgg atc ctg ggc cgg tgg ctg cat tcg acg gac ggg gtg ctg       816
   Ala Lys Arg Ile Leu Gly Arg Trp Leu His Ser Thr Asp Gly Val Leu
               260                 265                 270
   tgc ctg ttt ccg tcg tgg ttc gcg ccg gcg cag ccg gac tgg ccg gcg       864
   Cys Leu Phe Pro Ser Trp Phe Ala Pro Ala Gln Pro Asp Trp Pro Ala
           275                 280                 285
   aac cac ctg caa agc ggg ttt ccg ctg ttc aac gac gcg ggt ccg gcg       912
   Asn His Leu Gln Ser Gly Phe Pro Leu Phe Asn Asp Ala Gly Pro Ala
       290                 295                 300
   cag gcg gat gcg gag ctg gag gcg ttc gtc gcg tcg ggc gag gcg ccg       960
   Gln Ala Asp Ala Glu Leu Glu Ala Phe Val Ala Ser Gly Glu Ala Pro
   305                 310                 315                 320
   gtg gtg ttc acg gcg ggc tcg acg ctg gtg gac ggc cgc acg tat gag      1008
   Val Val Phe Thr Ala Gly Ser Thr Leu Val Asp Gly Arg Thr Tyr Glu
                   325                 330                 335
   cac gcg gtg acg cag gtg ctg cag gcc acg ggg gtg cgg ggg att ctg      1056
   His Ala Val Thr Gln Val Leu Gln Ala Thr Gly Val Arg Gly Ile Leu
               340                 345                 350
   ctc gcg ccg gat gcg ccg gat gcg ccg gcg gca tcg gac ggg gcg gcg      1104
   Leu Ala Pro Asp Ala Pro Asp Ala Pro Ala Ala Ser Asp Gly Ala Ala
           355                 360                 365
   ctg ctc aag cgc cgc tac gtg ccg ctc gcg gcg ttg ctg ccg cgc tgc      1152
   Leu Leu Lys Arg Arg Tyr Val Pro Leu Ala Ala Leu Leu Pro Arg Cys
       370                 375                 380
   cgg gcg ctg gtg cac cac ggg ggg atc ggg acg gcg tcg ctc gcg tac      1200
   Arg Ala Leu Val His His Gly Gly Ile Gly Thr Ala Ser Leu Ala Tyr
   385                 390                 395                 400
   gcg gcg ggg gtg ccg cag gtg gtg acg ccg ttc gcg cac gac cag ttc      1248
   Ala Ala Gly Val Pro Gln Val Val Thr Pro Phe Ala His Asp Gln Phe
                   405                 410                 415
   gac aac gcg cag cgg gtg gcg gcg agc ggc tgc ggg gtg cgg ctg gac      1296
   Asp Asn Ala Gln Arg Val Ala Ala Ser Gly Cys Gly Val Arg Leu Asp
               420                 425                 430
   gcg ccg gtg cgc ggc gag ccg ctc gcg cgg gcg ctg gcg cag gtg ctg      1344
   Ala Pro Val Arg Gly Glu Pro Leu Ala Arg Ala Leu Ala Gln Val Leu
           435                 440                 445
   ggc gac gcg gcg atg gcg gcg cgc tgc gcg cag gtg cgc gcg cgg atg      1392
   Gly Asp Ala Ala Met Ala Ala Arg Cys Ala Gln Val Arg Ala Arg Met
       450                 455                 460
   gcg gcg gag ccg aac ggc tgc gac gcg gcg gcg cgc ttc atc gag cgc        1440
   Ala Ala Glu Pro Asn Gly Cys Asp Ala Ala Ala Arg Phe Ile Glu Arg
   465                 470                 475                 480
   ttc gcg ccg ggc gtc gcg gcg cgg cgg gcg cag ccg gca tga                1482
   Phe Ala Pro Gly Val Ala Ala Arg Arg Ala Gln Pro Ala
                   485                 490
<210> 20
   <211> 493
   <212> PRT
   <213> Burkholderia thailandensis
<400> 20
   Met Asp Ala Gly Arg Ile Gly Leu His Asp Ala Ala Ala Ala Gly Arg
   1               5                   10                  15
   Ile Gly Met Thr Glu Ala Phe Ala Ser Arg Ala Arg Cys Ser Ala Ala
               20                  25                  30
   Ala Leu Ala Ala Gly Gly Arg Ala Pro Ala Gly Asp Gly Arg Ser Gly
           35                  40                  45
   Ser Asn Arg Ala Ala Ala Asn Gly Ser Val Asp Cys Arg Ala Gly Trp
       50                  55                  60
   Asn Asp Glu Ala Met Ala Lys Val Ile Val Thr Ala Ile Gly Ser Ala
   65                  70                  75                  80
   Gly Asp Val His Pro Leu Leu Gly Val Ser Arg Ala Leu Ser Ala Arg
                   85                  90                  95
   Gly His Glu Val Val Phe Cys Thr His Ala Pro Phe Glu Ala Ala Val
               100                 105                 110
   Arg Ala Ser Gly Phe Ala Phe Val Pro Val Gly Thr Ala Glu Asp Tyr
           115                 120                 125
   Val Arg Ala Met Ala Asp Pro Ala Leu Trp Asp Pro Arg Thr Ser Phe
       130                 135                 140
   Lys Thr Leu Trp Arg Val Ile Ala Pro Val Val Arg Pro His Phe Glu
   145                 150                 155                 160
   Val Leu Arg Ala Leu Ser Asp Ala Asp Thr Val Leu Val Gly Thr Leu
                   165                 170                 175
   Trp Ala Phe Ser Ala Arg Leu Met Gln Glu Arg Phe Gly Thr Arg Tyr
               180                 185                 190
   Val Ser Val Gln Val Ser Pro Ser Thr Leu Leu Ser Ala His Ala Pro
           195                 200                 205
   Pro Thr His Lys Arg Leu Thr Ile Pro Lys Gly Leu Pro Leu Ala Val
       210                 215                 220
   Lys Ala Gly Leu Met Thr Leu Ile Glu Arg Gln Val Leu Asp Arg Val
   225                 230                 235                 240
   Cys Gly Pro Glu Leu Asn Ala Ala Arg Gln Ala Leu Gly Leu Ala Pro
                   245                 250                 255
   Ala Lys Arg Ile Leu Gly Arg Trp Leu His Ser Thr Asp Gly Val Leu
               260                 265                 270
   Cys Leu Phe Pro Ser Trp Phe Ala Pro Ala Gln Pro Asp Trp Pro Ala
           275                 280                 285
   Asn His Leu Gln Ser Gly Phe Pro Leu Phe Asn Asp Ala Gly Pro Ala
       290                 295                 300
   Gln Ala Asp Ala Glu Leu Glu Ala Phe Val Ala Ser Gly Glu Ala Pro
   305                 310                 315                 320
   Val Val Phe Thr Ala Gly Ser Thr Leu Val Asp Gly Arg Thr Tyr Glu
                   325                 330                 335
   His Ala Val Thr Gln Val Leu Gln Ala Thr Gly Val Arg Gly Ile Leu
               340                 345                 350
   Leu Ala Pro Asp Ala Pro Asp Ala Pro Ala Ala Ser Asp Gly Ala Ala
           355                 360                 365
   Leu Leu Lys Arg Arg Tyr Val Pro Leu Ala Ala Leu Leu Pro Arg Cys
       370                 375                 380
   Arg Ala Leu Val His His Gly Gly Ile Gly Thr Ala Ser Leu Ala Tyr
   385                 390                 395                 400
   Ala Ala Gly Val Pro Gln Val Val Thr Pro Phe Ala His Asp Gln Phe
                   405                 410                 415
   Asp Asn Ala Gln Arg Val Ala Ala Ser Gly Cys Gly Val Arg Leu Asp
               420                 425                 430
   Ala Pro Val Arg Gly Glu Pro Leu Ala Arg Ala Leu Ala Gln Val Leu
           435                 440                 445
   Gly Asp Ala Ala Met Ala Ala Arg Cys Ala Gln Val Arg Ala Arg Met
       450                 455                 460
   Ala Ala Glu Pro Asn Gly Cys Asp Ala Ala Ala Arg Phe Ile Glu Arg
   465                 470                 475                 480
   Phe Ala Pro Gly Val Ala Ala Arg Arg Ala Gln Pro Ala
                   485                 490
<210> 21
   <211> 966
   <212> DNA
   <213> Burkholderia thailandensis
<220>
   <221> CDS
   <222> (1)..(966)
<400> 21
   atg acg atc ctg ggg gcg ctg gtg att ctg tac gac ccg acg gac gag        48
   Met Thr Ile Leu Gly Ala Leu Val Ile Leu Tyr Asp Pro Thr Asp Glu
   1               5                   10                  15
   cag ttg tcg ggg ctg gag gcg ctc gcg cgc gac agc gac gcg ctc gtg        96
   Gln Leu Ser Gly Leu Glu Ala Leu Ala Arg Asp Ser Asp Ala Leu Val
               20                  25                  30
   gtc gtg gac aac acg ccg cac gag cac gcg gcg gcg cgc gag cgg gtg        144
   Val Val Asp Asn Thr Pro His Glu His Ala Ala Ala Arg Glu Arg Val
           35                  40                  45
   cgt gcg ctg tcg gcg cgg acg aac acg gtg tgg cga cac cac ggc aac        192
   Arg Ala Leu Ser Ala Arg Thr Asn Thr Val Trp Arg His His Gly Asn
       50                  55                  60
   cgg ggc ggg gtc gcg ggc ggg tac aac gcg ggg ctg tcg gtg ctg ttc        240
   Arg Gly Gly Val Ala Gly Gly Tyr Asn Ala Gly Leu Ser Val Leu Phe
   65                  70                  75                  80
   gcg cag ggc gtc gag gcg gtc gcg ctg ttc gac cag gac tcg acg gtg        288
   Ala Gln Gly Val Glu Ala Val Ala Leu Phe Asp Gln Asp Ser Thr Val
                   85                  90                  95
   ccg gcc ggg tac ttc gag cgg atg cgc gag gcg tgc gcg caa ctg ggt        336
   Pro Ala Gly Tyr Phe Glu Arg Met Arg Glu Ala Cys Ala Gln Leu Gly
               100                 105                 110
   gag caa ccg ggc gcg cac gcg ggc gcg ttc atc gcg ggc ccg cgg atc        384
   Glu Gln Pro Gly Ala His Ala Gly Ala Phe Ile Ala Gly Pro Arg Ile
           115                 120                 125
   tac gac gcg aac gag cag cgc ttc ctg ccg gag ctg atg acg agc ggg        432
   Tyr Asp Ala Asn Glu Gln Arg Phe Leu Pro Glu Leu Met Thr Ser Gly
       130                 135                 140
   gtg acg gtg cgc cgc gtg cgg gtg gag ggc gag acg gcg ccg cag cgc        480
   Val Thr Val Arg Arg Val Arg Val Glu Gly Glu Thr Ala Pro Gln Arg
   145                 150                 155                 160
   tgc gcg ttc ctg atc tcg tcg ggc agc gtg att tcg cgg gcc gcg tac        528
   Cys Ala Phe Leu Ile Ser Ser Gly Ser Val Ile Ser Arg Ala Ala Tyr
                   165                 170                 175
   gcg cgg ctc ggt cga ttc gac gag gcg ctg ttc atc gat cac gtc gac        576
   Ala Arg Leu Gly Arg Phe Asp Glu Ala Leu Phe Ile Asp His Val Asp
               180                 185                 190
   acc gag tat tgc ctg cgc gcg ctc gcg cac aac gtg ccg ctg tac gtg        624
   Thr Glu Tyr Cys Leu Arg Ala Leu Ala His Asn Val Pro Leu Tyr Val
           195                 200                 205
   gtg ccg ccg ctc gtg ctg acg cac cgg atc ggc gcg cgg cgc cgg cac        672
   Val Pro Pro Leu Val Leu Thr His Arg Ile Gly Ala Arg Arg Arg His
       210                 215                 220
   aag gtg ggg ccg ttc gag ctg acg gcg atg cat cac ggg tgg ttg cgc        720
   Lys Val Gly Pro Phe Glu Leu Thr Ala Met His His Gly Trp Leu Arg
   225                 230                 235                 240
   cga tac tac ggc gcg cgc aac gcg atg caa ctg ggg ctg cag tac ggc        768
   Arg Tyr Tyr Gly Ala Arg Asn Ala Met Gln Leu Gly Leu Gln Tyr Gly
                   245                 250                 255
   ttg cgg ttt ccg gtg gcg ctg gtg ccg aat ctg ctg acg ata tgg cag        816
   Leu Arg Phe Pro Val Ala Leu Val Pro Asn Leu Leu Thr Ile Trp Gln
               260                 265                 270
   gtg atc cag gtg gtg ctg tgc gag cgg gag aag ggc gcg aag ctg cgc        864
   Val Ile Gln Val Val Leu Cys Glu Arg Glu Lys Gly Ala Lys Leu Arg
           275                 280                 285
   ggg atc gcg ctg ggc gtg ctc gac ggc ctg ttc ggg cgg ctg gga tcg        912
   Gly Ile Ala Leu Gly Val Leu Asp Gly Leu Phe Gly Arg Leu Gly Ser
       290                 295                 300
   ttc gac gat gcg cgc gcg ggc gcg gcg gcg cgc gag ccg gtg cgg cag        960
   Phe Asp Asp Ala Arg Ala Gly Ala Ala Ala Arg Glu Pro Val Arg Gln
   305                 310                 315                 320
   gaa tga                                                                966
   Glu
<210> 22
   <211> 321
   <212> PRT
   <213> Burkholderia thailandensis
<400> 22
   Met Thr Ile Leu Gly Ala Leu Val Ile Leu Tyr Asp Pro Thr Asp Glu
   1               5                   10                  15
   Gln Leu Ser Gly Leu Glu Ala Leu Ala Arg Asp Ser Asp Ala Leu Val
               20                  25                  30
   Val Val Asp Asn Thr Pro His Glu His Ala Ala Ala Arg Glu Arg Val
           35                  40                  45
   Arg Ala Leu Ser Ala Arg Thr Asn Thr Val Trp Arg His His Gly Asn
       50                  55                  60
   Arg Gly Gly Val Ala Gly Gly Tyr Asn Ala Gly Leu Ser Val Leu Phe
   65                  70                  75                  80
   Ala Gln Gly Val Glu Ala Val Ala Leu Phe Asp Gln Asp Ser Thr Val
                   85                  90                  95
   Pro Ala Gly Tyr Phe Glu Arg Met Arg Glu Ala Cys Ala Gln Leu Gly
               100                 105                 110
   Glu Gln Pro Gly Ala His Ala Gly Ala Phe Ile Ala Gly Pro Arg Ile
           115                 120                 125
   Tyr Asp Ala Asn Glu Gln Arg Phe Leu Pro Glu Leu Met Thr Ser Gly
       130                 135                 140
   Val Thr Val Arg Arg Val Arg Val Glu Gly Glu Thr Ala Pro Gln Arg
   145                 150                 155                 160
   Cys Ala Phe Leu Ile Ser Ser Gly Ser Val Ile Ser Arg Ala Ala Tyr
                   165                 170                 175
   Ala Arg Leu Gly Arg Phe Asp Glu Ala Leu Phe Ile Asp His Val Asp
               180                 185                 190
   Thr Glu Tyr Cys Leu Arg Ala Leu Ala His Asn Val Pro Leu Tyr Val
           195                 200                 205
   Val Pro Pro Leu Val Leu Thr His Arg Ile Gly Ala Arg Arg Arg His
       210                 215                 220
   Lys Val Gly Pro Phe Glu Leu Thr Ala Met His His Gly Trp Leu Arg
   225                 230                 235                 240
   Arg Tyr Tyr Gly Ala Arg Asn Ala Met Gln Leu Gly Leu Gln Tyr Gly
                   245                 250                 255
   Leu Arg Phe Pro Val Ala Leu Val Pro Asn Leu Leu Thr Ile Trp Gln
               260                 265                 270
   Val Ile Gln Val Val Leu Cys Glu Arg Glu Lys Gly Ala Lys Leu Arg
           275                 280                 285
   Gly Ile Ala Leu Gly Val Leu Asp Gly Leu Phe Gly Arg Leu Gly Ser
       290                 295                 300
   Phe Asp Asp Ala Arg Ala Gly Ala Ala Ala Arg Glu Pro Val Arg Gln
   305                 310                 315                 320
   Glu
<210> 23
   <211> 1557
   <212> DNA
   <213> Burkholderia thailandensis
<220>
   <221> CDS
   <222> (1)..(1557)
<400> 23
   atg tcg gcg gat cag gcg ggc gtt gcg ccg ccg gcg gcc gcc ccg ctg        48
   Met Ser Ala Asp Gln Ala Gly Val Ala Pro Pro Ala Ala Ala Pro Leu
   1               5                   10                  15
   cgc ggc gcg aag ctc gcg ctg ctg acg ttc gcg ctg tcg ctc gcg acg        96
   Arg Gly Ala Lys Leu Ala Leu Leu Thr Phe Ala Leu Ser Leu Ala Thr
               20                  25                  30
   ttc atc gaa gtg ctg gat tcg acg gtg gcg aac gtg gcg gtg ccg gcg       144
   Phe Ile Glu Val Leu Asp Ser Thr Val Ala Asn Val Ala Val Pro Ala
           35                  40                  45
   atc tcg ggc agc ctc ggg gtg tcg aac agc cag ggc acg tgg gtg atc       192
   Ile Ser Gly Ser Leu Gly Val Ser Asn Ser Gln Gly Thr Trp Val Ile
       50                  55                  60
   agc tcg tac tcg gtg gcc gcg gcg atc gcg gtg ccg ctg acg ggg tgg       240
   Ser Ser Tyr Ser Val Ala Ala Ala Ile Ala Val Pro Leu Thr Gly Trp
   65                  70                  75                  80
   ctt gcg cgg cgc gtg ggc gag ctg agg ctg ttc gtg gcg tcg gtg atc       288
   Leu Ala Arg Arg Val Gly Glu Leu Arg Leu Phe Val Ala Ser Val Ile
                   85                  90                  95
   ctg ttc acg ctg acg tcg ctg ctg tgc ggg ctc gcg cgg gac ctg gag       336
   Leu Phe Thr Leu Thr Ser Leu Leu Cys Gly Leu Ala Arg Asp Leu Glu
               100                 105                 110
   gtg ctg gtt gcg tgc cgg gcg ctg cag ggg ctg ttc tcg ggg ccg atg       384
   Val Leu Val Ala Cys Arg Ala Leu Gln Gly Leu Phe Ser Gly Pro Met
           115                 120                 125
   gtg ccg ctg tcg cag acg atc ctg atg cgc gcg ttc ccg ccg gcg cgg       432
   Val Pro Leu Ser Gln Thr Ile Leu Met Arg Ala Phe Pro Pro Ala Arg
       130                 135                 140
   cgc acg ctg gcg ctg gcg ctg tgg ggg atg acg gtg ctg ctc gcg ccg       480
   Arg Thr Leu Ala Leu Ala Leu Trp Gly Met Thr Val Leu Leu Ala Pro
   145                 150                 155                 160
   atc ttc ggg ccg gtg gtg ggc ggc tgg ctg atc gac aac ttc tcg tgg       528
   Ile Phe Gly Pro Val Val Gly Gly Trp Leu Ile Asp Asn Phe Ser Trp
                   165                 170                 175
   ccg tgg atc ttc ctg atc aac ctg ccg atc ggg ctg ttc tcg ttc gcg       576
   Pro Trp Ile Phe Leu Ile Asn Leu Pro Ile Gly Leu Phe Ser Phe Ala
               180                 185                 190
   gtg tgc acg ctg atg ctg cgc ccg cag gcg cag cgc ggc gag gcg agc       624
   Val Cys Thr Leu Met Leu Arg Pro Gln Ala Gln Arg Gly Glu Ala Ser
           195                 200                 205
   ccg atc gac gcg ccg ggg atc gtg ctg ctg gtg atc ggg gtg ggc tcg      672
   Pro Ile Asp Ala Pro Gly Ile Val Leu Leu Val Ile Gly Val Gly Ser
       210                 215                 220
   ctg cag gcg atg ctg gac ctg ggg cac gac cgg ggc tgg ttc gat tcg      720
   Leu Gln Ala Met Leu Asp Leu Gly His Asp Arg Gly Trp Phe Asp Ser
   225                 230                 235                 240
   ccg ctg atc acg gcg ctg gcg atc gcg gcg ggg gtg tcg ctc gtg tcg      768
   Pro Leu Ile Thr Ala Leu Ala Ile Ala Ala Gly Val Ser Leu Val Ser
                   245                 250                 255
   ctg ctg atc tgg gag ctg ggc gag gcg cat ccg gtg gtg gat ctg agc      816
   Leu Leu Ile Trp Glu Leu Gly Glu Ala His Pro Val Val Asp Leu Ser
               260                 265                 270
   ctg ttc cgg gag cgg acc ttc acg ttc tgc gtg gtg atc atc tcg ctg      864
   Leu Phe Arg Glu Arg Thr Phe Thr Phe Cys Val Val Ile Ile Ser Leu
           275                 280                 285
   ggg atg atg agc ttc tcg gtg gtg ggg gtg gtg ttt ccg ctg tgg ctg      912
   Gly Met Met Ser Phe Ser Val Val Gly Val Val Phe Pro Leu Trp Leu
       290                 295                 300
   cag gcg gtg atg gga tac acg gcg tac cag gcg ggg ctg gcg acg gcg      960
   Gln Ala Val Met Gly Tyr Thr Ala Tyr Gln Ala Gly Leu Ala Thr Ala
   305                 310                 315                 320
   tcg atg ggg gtg ctg gcg ctg gtg ttc tcg atc ctg gtg ggg ctg tac     1008
   Ser Met Gly Val Leu Ala Leu Val Phe Ser Ile Leu Val Gly Leu Tyr
                   325                 330                 335
   gcg agc cgg gtg gac gcg cgg gtg ctg gtg acg ttc ggg ttc ggg gtg     1056
   Ala Ser Arg Val Asp Ala Arg Val Leu Val Thr Phe Gly Phe Gly Val
               340                 345                 350
   ttt gcg gcg gtg atg tgg tgg agc acg cac ttc acg ctg tcg atg acg     1104
   Phe Ala Ala Val Met Trp Trp Ser Thr His Phe Thr Leu Ser Met Thr
           355                 360                 365
   ttc gcg cag gtg gtg acg ccg cgg ctg att cag ggg atg ggg ctg ccg     1152
   Phe Ala Gln Val Val Thr Pro Arg Leu Ile Gln Gly Met Gly Leu Pro
       370                 375                 380
   tgc ttc ttc ata ccg ctg acg gcg gcg acg ctg tcg cgg gtg ccg gac     1200
   Cys Phe Phe Ile Pro Leu Thr Ala Ala Thr Leu Ser Arg Val Pro Asp
   385                 390                 395                 400
   gag aag ctg gcg gcg gcg tcg agc ctg tcg aac ttc ctg cgg acg ctg     1248
   Glu Lys Leu Ala Ala Ala Ser Ser Leu Ser Asn Phe Leu Arg Thr Leu
                   405                 410                 415
   tcg gcg gcg ttc ggc acg gcg ctg agc gtg acg tgg tgg gac aac cgg     1296
   Ser Ala Ala Phe Gly Thr Ala Leu Ser Val Thr Trp Trp Asp Asn Arg
               420                 425                 430
   gcg acg tac cac tac gcg gtg gtg tcg caa tcg gtg acg cgc gcc tcg     1344
   Ala Thr Tyr His Tyr Ala Val Val Ser Gln Ser Val Thr Arg Ala Ser
           435                 440                 445
   gag aac acg cag cgg tac gtg gac gcg ctg cac gcg atg ggg ctg cac     1392
   Glu Asn Thr Gln Arg Tyr Val Asp Ala Leu His Ala Met Gly Leu His
       450                 455                 460
   ggc gcg cgg gag ctg agc tcg ctg cac cag gtg gtg cgg cag cag gcg        1440
   Gly Ala Arg Glu Leu Ser Ser Leu His Gln Val Val Arg Gln Gln Ala
   465                 470                 475                 480
   tac atg atg gcg acg aac gac atg ttc tac atg gcg agc gcg acg tgc        1488
   Tyr Met Met Ala Thr Asn Asp Met Phe Tyr Met Ala Ser Ala Thr Cys
                   485                 490                 495
   ctg ctg ctg gcg ggg ctg atg tgg ctg acg cgg ccg aag cgg ggc gcg        1536
   Leu Leu Leu Ala Gly Leu Met Trp Leu Thr Arg Pro Lys Arg Gly Ala
               500                 505                 510
   gcg gcg gcg ctc ggg cac tga                                            1557
   Ala Ala Ala Leu Gly His
           515
<210> 24
   <211> 518
   <212> PRT
   <213> Burkholderia thailandensis
<400> 24
   Met Ser Ala Asp Gln Ala Gly Val Ala Pro Pro Ala Ala Ala Pro Leu
   1               5                   10                  15
   Arg Gly Ala Lys Leu Ala Leu Leu Thr Phe Ala Leu Ser Leu Ala Thr
               20                  25                  30
   Phe Ile Glu Val Leu Asp Ser Thr Val Ala Asn Val Ala Val Pro Ala
           35                  40                  45
   Ile Ser Gly Ser Leu Gly Val Ser Asn Ser Gln Gly Thr Trp Val Ile
       50                  55                  60
   Ser Ser Tyr Ser Val Ala Ala Ala Ile Ala Val Pro Leu Thr Gly Trp
   65                  70                  75                  80
   Leu Ala Arg Arg Val Gly Glu Leu Arg Leu Phe Val Ala Ser Val Ile
                   85                  90                  95
   Leu Phe Thr Leu Thr Ser Leu Leu Cys Gly Leu Ala Arg Asp Leu Glu
               100                 105                 110
   Val Leu Val Ala Cys Arg Ala Leu Gln Gly Leu Phe Ser Gly Pro Met
           115                 120                 125
   Val Pro Leu Ser Gln Thr Ile Leu Met Arg Ala Phe Pro Pro Ala Arg
       130                 135                 140
   Arg Thr Leu Ala Leu Ala Leu Trp Gly Met Thr Val Leu Leu Ala Pro
   145                 150                 155                 160
   Ile Phe Gly Pro Val Val Gly Gly Trp Leu Ile Asp Asn Phe Ser Trp
                   165                 170                 175
   Pro Trp Ile Phe Leu Ile Asn Leu Pro Ile Gly Leu Phe Ser Phe Ala
               180                 185                 190
   Val Cys Thr Leu Met Leu Arg Pro Gln Ala Gln Arg Gly Glu Ala Ser
           195                 200                 205
   Pro Ile Asp Ala Pro Gly Ile Val Leu Leu Val Ile Gly Val Gly Ser
       210                 215                 220
   Leu Gln Ala Met Leu Asp Leu Gly His Asp Arg Gly Trp Phe Asp Ser
   225                 230                 235                 240
   Pro Leu Ile Thr Ala Leu Ala Ile Ala Ala Gly Val Ser Leu Val Ser
                   245                 250                 255
   Leu Leu Ile Trp Glu Leu Gly Glu Ala His Pro Val Val Asp Leu Ser
               260                 265                 270
   Leu Phe Arg Glu Arg Thr Phe Thr Phe Cys Val Val Ile Ile Ser Leu
           275                 280                 285
   Gly Met Met Ser Phe Ser Val Val Gly Val Val Phe Pro Leu Trp Leu
       290                 295                 300
   Gln Ala Val Met Gly Tyr Thr Ala Tyr Gln Ala Gly Leu Ala Thr Ala
   305                 310                 315                 320
   Ser Met Gly Val Leu Ala Leu Val Phe Ser Ile Leu Val Gly Leu Tyr
                   325                 330                 335
   Ala Ser Arg Val Asp Ala Arg Val Leu Val Thr Phe Gly Phe Gly Val
               340                 345                 350
   Phe Ala Ala Val Met Trp Trp Ser Thr His Phe Thr Leu Ser Met Thr
           355                 360                 365
   Phe Ala Gln Val Val Thr Pro Arg Leu Ile Gln Gly Met Gly Leu Pro
       370                 375                 380
   Cys Phe Phe Ile Pro Leu Thr Ala Ala Thr Leu Ser Arg Val Pro Asp
   385                 390                 395                 400
   Glu Lys Leu Ala Ala Ala Ser Ser Leu Ser Asn Phe Leu Arg Thr Leu
                   405                 410                 415
   Ser Ala Ala Phe Gly Thr Ala Leu Ser Val Thr Trp Trp Asp Asn Arg
                   420                 425                 430
   Ala Thr Tyr His Tyr Ala Val Val Ser Gln Ser Val Thr Arg Ala Ser
           435                 440                 445
   Glu Asn Thr Gln Arg Tyr Val Asp Ala Leu His Ala Met Gly Leu His
       450                 455                 460
   Gly Ala Arg Glu Leu Ser Ser Leu His Gln Val Val Arg Gln Gln Ala
   465                 470                 475                 480
   Tyr Met Met Ala Thr Asn Asp Met Phe Tyr Met Ala Ser Ala Thr Cys
                   485                 490                 495
   Leu Leu Leu Ala Gly Leu Met Trp Leu Thr Arg Pro Lys Arg Gly Ala
               500                 505                 510
   Ala Ala Ala Leu Gly His
           515
<210> 25
   <211> 1662
   <212> DNA
   <213> Burkholderia thailandensis
<220>
   <221> CDS
   <222> (1)..(1662)
<400> 25
   atg cgc gcg cgg gcg cgg cgg cgc gcg agc cgg tgc ggc agg aat gaa        48
   Met Arg Ala Arg Ala Arg Arg Arg Ala Ser Arg Cys Gly Arg Asn Glu
   1               5                   10                  15
   cgg aac ggg ccg cag cgg gat acc gga aag caa gaa gga cgc atc ata        96
   Arg Asn Gly Pro Gln Arg Asp Thr Gly Lys Gln Glu Gly Arg Ile Ile
               20                  25                  30
   cga atg acg cag aca gca acg caa gca gcc act cgc gcg atg atc gcg       144
   Arg Met Thr Gln Thr Ala Thr Gln Ala Ala Thr Arg Ala Met Ile Ala
           35                  40                  45
   aca gga agc cgc gcg gcg cgc cgg ctc gcg gca gcc gcg ctc gcg tgg       192
   Thr Gly Ser Arg Ala Ala Arg Arg Leu Ala Ala Ala Ala Leu Ala Trp
       50                  55                  60
   gcg ctc gcc ggc tgc gtg ccg tcg ggc ttc gag ccg gcg ctc gcg ccg       240
   Ala Leu Ala Gly Cys Val Pro Ser Gly Phe Glu Pro Ala Leu Ala Pro
   65                  70                  75                  80
   cgc acg ccg ggc gac gac gcg ctc gcg cac acg gcg ggg ggc gcc gcg       288
   Arg Thr Pro Gly Asp Asp Ala Leu Ala His Thr Ala Gly Gly Ala Ala
                   85                  90                  95
   cac ggc gca tgg ccg agc ccc gac tgg gtc cgg cag ctc ggc gat ccg       336
   His Gly Ala Trp Pro Ser Pro Asp Trp Val Arg Gln Leu Gly Asp Pro
               100                 105                 110
   caa ctc gac gcg ctc gtc gac gag gcg ctg cgg cag aac ccg acg ctg        384
   Gln Leu Asp Ala Leu Val Asp Glu Ala Leu Arg Gln Asn Pro Thr Leu
           115                 120                 125
   cag gcc gcg cag gcg cgc atc ggc gtc gcg cag tcg cag ctg cag cag        432
   Gln Ala Ala Gln Ala Arg Ile Gly Val Ala Gln Ser Gln Leu Gln Gln
       130                 135                 140
   ttc gaa tcg ctg acg ggg ctc acc gcg acg gcg ggc gcg tcg ctc tcc        480
   Phe Glu Ser Leu Thr Gly Leu Thr Ala Thr Ala Gly Ala Ser Leu Ser
   145                 150                 155                 160
   aag gcg cac gtg ccg cgc tcg ggc ggc acc atc aat acg acg ttc aac        528
   Lys Ala His Val Pro Arg Ser Gly Gly Thr Ile Asn Thr Thr Phe Asn
                   165                 170                 175
   ggc ttg ccg gtg tcg gtg ccg ctc gtc ggc gaa tcg gtg gtg tcg tcg        576
   Gly Leu Pro Val Ser Val Pro Leu Val Gly Glu Ser Val Val Ser Ser
               180                 185                 190
   tcg tcg ctg ttc gtc ggg ctg aac tat cag ctg gac ctg tgg ggc aag        624
   Ser Ser Leu Phe Val Gly Leu Asn Tyr Gln Leu Asp Leu Trp Gly Lys
           195                 200                 205
   aac gcg gcg gcc acg cgc ggg ctg ctg tcg atg cgc gat gcg gcg cgc        672
   Asn Ala Ala Ala Thr Arg Gly Leu Leu Ser Met Arg Asp Ala Ala Arg
       210                 215                 220
   gtg gag gcc gag cag gcg cgg ctc gcg ctg tcg gtg gcg atc gtg acg        720
   Val Glu Ala Glu Gln Ala Arg Leu Ala Leu Ser Val Ala Ile Val Thr
   225                 230                 235                 240
   ctg tac ggc gag ctg gac cgc gcg tat gcg ctg cgc gag ctg ctg cag        768
   Leu Tyr Gly Glu Leu Asp Arg Ala Tyr Ala Leu Arg Glu Leu Leu Gln
                   245                 250                 255
   cag aag cgc cgc gcg agc gag cag gtg gag acg gtg ctg cgc gag cgc        816
   Gln Lys Arg Arg Ala Ser Glu Gln Val Glu Thr Val Leu Arg Glu Arg
               260                 265                 270
   gcg gcg cgc ggg atc gac aac ggc tac gat gcg gac gac gcg gcg ctc        864
   Ala Ala Arg Gly Ile Asp Asn Gly Tyr Asp Ala Asp Asp Ala Ala Leu
           275                 280                 285
   aag cgg ggc aag ctg ctc gag cag ctc gcg ctg acc gac gag cag atc        912
   Lys Arg Gly Lys Leu Leu Glu Gln Leu Ala Leu Thr Asp Glu Gln Ile
       290                 295                 300
   cag ttg cag aag ctg caa ctg ggg gtg ctg agc ggg cgg ggg ccg gag        960
   Gln Leu Gln Lys Leu Gln Leu Gly Val Leu Ser Gly Arg Gly Pro Glu
   305                 310                 315                 320
   cgc ggg ctg tcg ctc gcg cgg ccg aag ctc gcg ccg ctc gcg gac gcg       1008
   Arg Gly Leu Ser Leu Ala Arg Pro Lys Leu Ala Pro Leu Ala Asp Ala
                   325                 330                 335
   ccg ctg ccg gcg cgg ctg ccg gcc ggg ctg ctg ggg cgg cgg ccg gac       1056
   Pro Leu Pro Ala Arg Leu Pro Ala Gly Leu Leu Gly Arg Arg Pro Asp
               340                 345                 350
   atc gtc gcg gcg cgg ctg cgg gtg gag gcg gcg tac gcg gcg atc gac       1104
   Ile Val Ala Ala Arg Leu Arg Val Glu Ala Ala Tyr Ala Ala Ile Asp
           355                 360                 365
   ggc acg cgc gcg tcg ttc tac ccg gac gtg aac ctg gcg gcg ctg ggc      1152
   Gly Thr Arg Ala Ser Phe Tyr Pro Asp Val Asn Leu Ala Ala Leu Gly
       370                 375                 380
   ggg ctg ttc gcg ctc acg ccg gcg tcg ctg ttc aag cac gat gcg ctg      1200
   Gly Leu Phe Ala Leu Thr Pro Ala Ser Leu Phe Lys His Asp Ala Leu
   385                 390                 395                 400
   ggg ggc tcg atc ggt ccg gcg ctg tcg ctg ccg atc ttc gat cgc ggc      1248
   Gly Gly Ser Ile Gly Pro Ala Leu Ser Leu Pro Ile Phe Asp Arg Gly
                   405                 410                 415
   cgg ctg aag gcg aag ctg ggg ggc gac gtg gcg aac gcg gac gtg gcg      1296
   Arg Leu Lys Ala Lys Leu Gly Gly Asp Val Ala Asn Ala Asp Val Ala
               420                 425                 430
   ctg gcg ctg tac aac cag acg gtg gat gcg gcg ctg ggc gag gtg gcg      1344
   Leu Ala Leu Tyr Asn Gln Thr Val Asp Ala Ala Leu Gly Glu Val Ala
           435                 440                 445
   cgg cag ttg acg tcg ctg tcg acg gtg gat gcg ctg ctc gag gcg cag      1392
   Arg Gln Leu Thr Ser Leu Ser Thr Val Asp Ala Leu Leu Glu Ala Gln
       450                 455                 460
   cag cag gcg gtg cgc tcg gcg cag cgg atg gtg gcg ctg gcg cag gac      1440
   Gln Gln Ala Val Arg Ser Ala Gln Arg Met Val Ala Leu Ala Gln Asp
   465                 470                 475                 480
   cgg cac cgg cgg ggg atg ggg atg cgc aag gac gtg aac gtg gcg aag      1488
   Arg His Arg Arg Gly Met Gly Met Arg Lys Asp Val Asn Val Ala Lys
                   485                 490                 495
   ctg acg ctg ctg gac gag cgt gcg cac gtg atc gag ctg cag gcg cgg      1536
   Leu Thr Leu Leu Asp Glu Arg Ala His Val Ile Glu Leu Gln Ala Arg
               500                 505                 510
   cgg cgg acg ctg cgg gtg ggg ctg atc ggg gcg ctg ggc ggc ggc ttc      1584
   Arg Arg Thr Leu Arg Val Gly Leu Ile Gly Ala Leu Gly Gly Gly Phe
           515                 520                 525
   gac gcg cgg ccg gcg ggc ggc gcg ccg ctc gcg cag ggc aag ccg ttc      1632
   Asp Ala Arg Pro Ala Gly Gly Ala Pro Leu Ala Gln Gly Lys Pro Phe
       530                 535                 540
   gcg gcg gcg agc gac agg ccg ccc gat tga                              1662
   Ala Ala Ala Ser Asp Arg Pro Pro Asp
   545                 550
<210> 26
   <211> 553
   <212> PRT
   <213> Burkholderia thailandensis
<400> 26
   Met Arg Ala Arg Ala Arg Arg Arg Ala Ser Arg Cys Gly Arg Asn Glu
   1               5                   10                  15
   Arg Asn Gly Pro Gln Arg Asp Thr Gly Lys Gln Glu Gly Arg Ile Ile
               20                  25                  30
   Arg Met Thr Gln Thr Ala Thr Gln Ala Ala Thr Arg Ala Met Ile Ala
           35                  40                  45
   Thr Gly Ser Arg Ala Ala Arg Arg Leu Ala Ala Ala Ala Leu Ala Trp
       50                  55                  60
   Ala Leu Ala Gly Cys Val Pro Ser Gly Phe Glu Pro Ala Leu Ala Pro
   65                  70                  75                  80
   Arg Thr Pro Gly Asp Asp Ala Leu Ala His Thr Ala Gly Gly Ala Ala
                   85                  90                  95
   His Gly Ala Trp Pro Ser Pro Asp Trp Val Arg Gln Leu Gly Asp Pro
               100                 105                 110
   Gln Leu Asp Ala Leu Val Asp Glu Ala Leu Arg Gln Asn Pro Thr Leu
           115                 120                 125
   Gln Ala Ala Gln Ala Arg Ile Gly Val Ala Gln Ser Gln Leu Gln Gln
       130                 135                 140
   Phe Glu Ser Leu Thr Gly Leu Thr Ala Thr Ala Gly Ala Ser Leu Ser
   145                 150                 155                 160
   Lys Ala His Val Pro Arg Ser Gly Gly Thr Ile Asn Thr Thr Phe Asn
                   165                 170                 175
   Gly Leu Pro Val Ser Val Pro Leu Val Gly Glu Ser Val Val Ser Ser
               180                 185                 190
   Ser Ser Leu Phe Val Gly Leu Asn Tyr Gln Leu Asp Leu Trp Gly Lys
           195                 200                 205
   Asn Ala Ala Ala Thr Arg Gly Leu Leu Ser Met Arg Asp Ala Ala Arg
       210                 215                 220
   Val Glu Ala Glu Gln Ala Arg Leu Ala Leu Ser Val Ala Ile Val Thr
   225                 230                 235                 240
   Leu Tyr Gly Glu Leu Asp Arg Ala Tyr Ala Leu Arg Glu Leu Leu Gln
                   245                 250                 255
   Gln Lys Arg Arg Ala Ser Glu Gln Val Glu Thr Val Leu Arg Glu Arg
               260                 265                 270
   Ala Ala Arg Gly Ile Asp Asn Gly Tyr Asp Ala Asp Asp Ala Ala Leu
           275                 280                 285
   Lys Arg Gly Lys Leu Leu Glu Gln Leu Ala Leu Thr Asp Glu Gln Ile
       290                 295                 300
   Gln Leu Gln Lys Leu Gln Leu Gly Val Leu Ser Gly Arg Gly Pro Glu
   305                 310                 315                 320
   Arg Gly Leu Ser Leu Ala Arg Pro Lys Leu Ala Pro Leu Ala Asp Ala
                   325                 330                 335
   Pro Leu Pro Ala Arg Leu Pro Ala Gly Leu Leu Gly Arg Arg Pro Asp
               340                 345                 350
   Ile Val Ala Ala Arg Leu Arg Val Glu Ala Ala Tyr Ala Ala Ile Asp
           355                 360                 365
   Gly Thr Arg Ala Ser Phe Tyr Pro Asp Val Asn Leu Ala Ala Leu Gly
       370                 375                 380
   Gly Leu Phe Ala Leu Thr Pro Ala Ser Leu Phe Lys His Asp Ala Leu
   385                 390                 395                 400
   Gly Gly Ser Ile Gly Pro Ala Leu Ser Leu Pro Ile Phe Asp Arg Gly
                   405                 410                 415
   Arg Leu Lys Ala Lys Leu Gly Gly Asp Val Ala Asn Ala Asp Val Ala
               420                 425                 430
   Leu Ala Leu Tyr Asn Gln Thr Val Asp Ala Ala Leu Gly Glu Val Ala
           435                 440                 445
   Arg Gln Leu Thr Ser Leu Ser Thr Val Asp Ala Leu Leu Glu Ala Gln
       450                 455                 460
   Gln Gln Ala Val Arg Ser Ala Gln Arg Met Val Ala Leu Ala Gln Asp
   465                 470                 475                 480
   Arg His Arg Arg Gly Met Gly Met Arg Lys Asp Val Asn Val Ala Lys
                   485                 490                 495
   Leu Thr Leu Leu Asp Glu Arg Ala His Val Ile Glu Leu Gln Ala Arg
               500                 505                 510
   Arg Arg Thr Leu Arg Val Gly Leu Ile Gly Ala Leu Gly Gly Gly Phe
           515                 520                 525
   Asp Ala Arg Pro Ala Gly Gly Ala Pro Leu Ala Gln Gly Lys Pro Phe
       530                 535                 540
   Ala Ala Ala Ser Asp Arg Pro Pro Asp
   545                 550
<210> 27
   <211> 1401
   <212> DNA
   <213> Burkholderia thailandensis
<220>
   <221> CDS
   <222> (1)..(1401)
<400> 27
   atg cgg ccc gaa gcc acc gac acc cga aga cac cga cac caa cgc cac      48
   Met Arg Pro Glu Ala Thr Asp Thr Arg Arg His Arg His Gln Arg His
   1               5                   10                  15
   ctt cac cgt gta cac gag cga ttc aac cga cac cgc ccc cga gca tcg      96
   Leu His Arg Val His Glu Arg Phe Asn Arg His Arg Pro Arg Ala Ser
               20                  25                  30
   aag ccc gtc ggg ccg atc cgc gac ggc ttg cgg gcc ggc ccg gcc gtt     144
   Lys Pro Val Gly Pro Ile Arg Asp Gly Leu Arg Ala Gly Pro Ala Val
           35                  40                  45
   gcc ggc cgg cgc cac cga cat cac gca cgc gaa gac ctt gaa cga tac     192
   Ala Gly Arg Arg His Arg His His Ala Arg Glu Asp Leu Glu Arg Tyr
       50                  55                  60
   cgc cac cga tac ccc gcg cgc gaa ggc gcc cac cga tcc ggc cgc cct     240
   Arg His Arg Tyr Pro Ala Arg Glu Gly Ala His Arg Ser Gly Arg Pro
   65                  70                  75                  80
   cga cgg cgc gca cgc gca gcc cgt gcc ggc gca cga gcg cgg atc gcc     288
   Arg Arg Arg Ala Arg Ala Ala Arg Ala Gly Ala Arg Ala Arg Ile Ala
                   85                  90                  95
   tcc gcc gcc gga agc cgc ggc gac gct cgc cgc gcg ccg cgc gac gcg     336
   Ser Ala Ala Gly Ser Arg Gly Asp Ala Arg Arg Ala Pro Arg Asp Ala
               100                 105                 110
   ccg ccg gcg ctt cgc gct gtt ctt cgg gct gct ggc gct ggc cgc gct     384
   Pro Pro Ala Leu Arg Ala Val Leu Arg Ala Ala Gly Ala Gly Arg Ala
           115                 120                 125
   gac cgc ggg gct cta ctg gtt cgt cgc cgg gcg ctt cag cga gga gac     432
   Asp Arg Gly Ala Leu Leu Val Arg Arg Arg Ala Leu Gln Arg Gly Asp
       130                 135                 140
   gga cga cgc gta cgt ggc cgg caa cgt ggt gca gat cgc cgc gca gat     480
   Gly Arg Arg Val Arg Gly Arg Gln Arg Gly Ala Asp Arg Arg Ala Asp
   145                 150                 155                 160
   cca ggg gac ggt gac cga cgt gct ggt ggc gga cac gca gca ggt gaa     528
   Pro Gly Asp Gly Asp Arg Arg Ala Gly Gly Gly His Ala Ala Gly Glu
                   165                 170                 175
   ggc ggg gca ggc gct ggt gaa gct cga cga cgc gga cgc gtc ggc ggc     576
   Gly Gly Ala Gly Ala Gly Glu Ala Arg Arg Arg Gly Arg Val Gly Gly
               180                 185                 190
   gtt cgc gca ggc gcg ggc gca gct cgc gca ggc ggt gcg gca ggt ggc     624
   Val Arg Ala Gly Ala Gly Ala Ala Arg Ala Gly Gly Ala Ala Gly Gly
           195                 200                 205
   gaa cac gcg gct ctc gat ggg gat gta cga gga gac ggt gaa ggc gcg     672
   Glu His Ala Ala Leu Asp Gly Asp Val Arg Gly Asp Gly Glu Gly Ala
       210                 215                 220
   cga ggc gga cct gaa gct tgc gca gca ggc gta tcc gga gga act ggc      720
   Arg Gly Gly Pro Glu Ala Cys Ala Ala Gly Val Ser Gly Gly Thr Gly
   225                 230                 235                 240
   gcg gcg aaa gtc gtc gct ggc gaa cgc gca ggc ggc gct ggc ggg ggc      768
   Ala Ala Lys Val Val Ala Gly Glu Arg Ala Gly Gly Ala Gly Gly Gly
                   245                 250                 255
   gca ggc gca gct gga ggc ggc gcg cgc gct ggg cag cga gcg gcc ggt      816
   Ala Gly Ala Ala Gly Gly Gly Ala Arg Ala Gly Gln Arg Ala Ala Gly
               260                 265                 270
   cga gca gaa ccc ggc ggt gca gca ggc ggc cgc gca gtt caa gct ggc      864
   Arg Ala Glu Pro Gly Gly Ala Ala Gly Gly Arg Ala Val Gln Ala Gly
           275                 280                 285
   gta ccg gaa cct gag gcg cac gac gat cgt gtc gcc ggt gga cgg cac      912
   Val Pro Glu Pro Glu Ala His Asp Asp Arg Val Ala Gly Gly Arg His
       290                 295                 300
   ggt cgg tca gcg gtc ggt gca gat cgg tca gca ggt ggg gcc ggg ggt      960
   Gly Arg Ser Ala Val Gly Ala Asp Arg Ser Ala Gly Gly Ala Gly Gly
   305                 310                 315                 320
   gcc gct gat gtc ggt ggt gca gtt gcg gca ggt gtg ggt gga ggc gaa     1008
   Ala Ala Asp Val Gly Gly Ala Val Ala Ala Gly Val Gly Gly Gly Glu
                   325                 330                 335
   ctt caa gga agg gca gat ccg gca cat gcg ggt ggg cca gcc ggt gcg     1056
   Leu Gln Gly Arg Ala Asp Pro Ala His Ala Gly Gly Pro Ala Gly Ala
               340                 345                 350
   gct cga atc gga cct gta cgg cgc gcg ggt gac gta cca cgg ccg ggt     1104
   Ala Arg Ile Gly Pro Val Arg Arg Ala Gly Asp Val Pro Arg Pro Gly
           355                 360                 365
   gga ggg ggt ctc ggc ggg cac ggg cag cgc gtt ctc gat gct gcc gtc     1152
   Gly Gly Gly Leu Gly Gly His Gly Gln Arg Val Leu Asp Ala Ala Val
       370                 375                 380
   gca gaa cgc ggc ggg gaa ctg gat caa ggt ggt gca gcg cct gcc ggt     1200
   Ala Glu Arg Gly Gly Glu Leu Asp Gln Gly Gly Ala Ala Pro Ala Gly
   385                 390                 395                 400
   ggt gat ctc gct gga gcc gtc gga gct ggc ggc gca ccc gct gcg ggt     1248
   Gly Asp Leu Ala Gly Ala Val Gly Ala Gly Gly Ala Pro Ala Ala Gly
                   405                 410                 415
   ggg gct gtc gat gcg cgc gac ggt gga gac gaa ggt gcg tgg cgg ccg     1296
   Gly Ala Val Asp Ala Arg Asp Gly Gly Asp Glu Gly Ala Trp Arg Pro
               420                 425                 430
   cct gct cga cgg cga cgc gcc gct gcc ggg gct gcg cac gcg ggt gca     1344
   Pro Ala Arg Arg Arg Arg Ala Ala Ala Gly Ala Ala His Ala Gly Ala
           435                 440                 445
   cga agc gca ggc ggg cga ggc cga ggc cgc ggc ttc ggc agt gat tcg     1392
   Arg Ser Ala Gly Gly Arg Gly Arg Gly Arg Gly Phe Gly Ser Asp Ser
       450                 455                 460
   gga gaa tga                                                         1401
   Gly Glu
   465
<210> 28
   <211> 466
   <212> PRT
   <213> Burkholderia thailandensis
<400> 28
   Met Arg Pro Glu Ala Thr Asp Thr Arg Arg His Arg His Gln Arg His
   1               5                   10                  15
   Leu His Arg Val His Glu Arg Phe Asn Arg His Arg Pro Arg Ala Ser
               20                  25                  30
   Lys Pro Val Gly Pro Ile Arg Asp Gly Leu Arg Ala Gly Pro Ala Val
           35                  40                  45
   Ala Gly Arg Arg His Arg His His Ala Arg Glu Asp Leu Glu Arg Tyr
       50                  55                  60
   Arg His Arg Tyr Pro Ala Arg Glu Gly Ala His Arg Ser Gly Arg Pro
   65                  70                  75                  80
   Arg Arg Arg Ala Arg Ala Ala Arg Ala Gly Ala Arg Ala Arg Ile Ala
                   85                  90                  95
   Ser Ala Ala Gly Ser Arg Gly Asp Ala Arg Arg Ala Pro Arg Asp Ala
               100                 105                 110
   Pro Pro Ala Leu Arg Ala Val Leu Arg Ala Ala Gly Ala Gly Arg Ala
           115                 120                 125
   Asp Arg Gly Ala Leu Leu Val Arg Arg Arg Ala Leu Gln Arg Gly Asp
       130                 135                 140
   Gly Arg Arg Val Arg Gly Arg Gln Arg Gly Ala Asp Arg Arg Ala Asp
   145                 150                 155                 160
   Pro Gly Asp Gly Asp Arg Arg Ala Gly Gly Gly His Ala Ala Gly Glu
                   165                 170                 175
   Gly Gly Ala Gly Ala Gly Glu Ala Arg Arg Arg Gly Arg Val Gly Gly
               180                 185                 190
   Val Arg Ala Gly Ala Gly Ala Ala Arg Ala Gly Gly Ala Ala Gly Gly
           195                 200                 205
   Glu His Ala Ala Leu Asp Gly Asp Val Arg Gly Asp Gly Glu Gly Ala
       210                 215                 220
   Arg Gly Gly Pro Glu Ala Cys Ala Ala Gly Val Ser Gly Gly Thr Gly
   225                 230                 235                 240
   Ala Ala Lys Val Val Ala Gly Glu Arg Ala Gly Gly Ala Gly Gly Gly
                   245                 250                 255
   Ala Gly Ala Ala Gly Gly Gly Ala Arg Ala Gly Gln Arg Ala Ala Gly
               260                 265                 270
   Arg Ala Glu Pro Gly Gly Ala Ala Gly Gly Arg Ala Val Gln Ala Gly
           275                 280                 285
   Val Pro Glu Pro Glu Ala His Asp Asp Arg Val Ala Gly Gly Arg His
       290                 295                 300
   Gly Arg Ser Ala Val Gly Ala Asp Arg Ser Ala Gly Gly Ala Gly Gly
   305                 310                 315                 320
   Ala Ala Asp Val Gly Gly Ala Val Ala Ala Gly Val Gly Gly Gly Glu
                   325                 330                 335
   Leu Gln Gly Arg Ala Asp Pro Ala His Ala Gly Gly Pro Ala Gly Ala
               340                 345                 350
   Ala Arg Ile Gly Pro Val Arg Arg Ala Gly Asp Val Pro Arg Pro Gly
           355                 360                 365
   Gly Gly Gly Leu Gly Gly His Gly Gln Arg Val Leu Asp Ala Ala Val
       370                 375                 380
   Ala Glu Arg Gly Gly Glu Leu Asp Gln Gly Gly Ala Ala Pro Ala Gly
   385                 390                 395                 400
   Gly Asp Leu Ala Gly Ala Val Gly Ala Gly Gly Ala Pro Ala Ala Gly
                   405                 410                 415
   Gly Ala Val Asp Ala Arg Asp Gly Gly Asp Glu Gly Ala Trp Arg Pro
               420                 425                 430
   Pro Ala Arg Arg Arg Arg Ala Ala Ala Gly Ala Ala His Ala Gly Ala
           435                 440                 445
   Arg Ser Ala Gly Gly Arg Gly Arg Gly Arg Gly Phe Gly Ser Asp Ser
       450                 455                 460
   Gly Glu
   465
<210> 29
   <211> 1680
   <212> DNA
   <213> Pseudomonas putida
<220>
   <221> CDS
   <222> (1)..(1680)
<400> 29
   atg agt aac aag aac aac gat gag cta cag cgg cag gcc tcg gaa aac       48
   Met Ser Asn Lys Asn Asn Asp Glu Leu Gln Arg Gln Ala Ser Glu Asn
   1               5                   10                  15
   acc atg ggg ctg aac ccg gtc atc ggc atc cgc cgc aag gac ctg ttg       96
   Thr Met Gly Leu Asn Pro Val Ile Gly Ile Arg Arg Lys Asp Leu Leu
               20                  25                  30
   agc tcg gca cgc acc gtg ctg cgc cag gcc gtg cgc caa ccg ctg cac      144
   Ser Ser Ala Arg Thr Val Leu Arg Gln Ala Val Arg Gln Pro Leu His
           35                  40                  45
   agc gcc aag cat gtg gct cac ttt ggc ctg gag ctg aag aac gtg ttg      192
   Ser Ala Lys His Val Ala His Phe Gly Leu Glu Leu Lys Asn Val Leu
       50                  55                  60
   ctg ggc aaa tcc agc ctg gcc ccg gac agc gac gac cgt cgc ttc aat      240
   Leu Gly Lys Ser Ser Leu Ala Pro Asp Ser Asp Asp Arg Arg Phe Asn
   65                  70                  75                  80
   gac ccg gcc tgg agc aac aac ccg ctg tac cgc cgc tac ctg caa acc      288
   Asp Pro Ala Trp Ser Asn Asn Pro Leu Tyr Arg Arg Tyr Leu Gln Thr
                   85                  90                  95
   tac ctg gcc tgg cgc aag gag ctg cag gac tgg gtg agc agc agc gac      336
   Tyr Leu Ala Trp Arg Lys Glu Leu Gln Asp Trp Val Ser Ser Ser Asp
               100                 105                 110
   ctg tcc ccc cag gac atc agc cgc ggc cag ttc gtc atc aac ctg atg      384
   Leu Ser Pro Gln Asp Ile Ser Arg Gly Gln Phe Val Ile Asn Leu Met
           115                 120                 125
   acc gag gcc atg gcg ccg acc aat acc ctg tcc aac ccg gct gcg gtc      432
   Thr Glu Ala Met Ala Pro Thr Asn Thr Leu Ser Asn Pro Ala Ala Val
       130                 135                 140
   aaa cgc ttc ttc gaa acc ggc ggc aag agc ctg ctc gat ggc ctg tcc      480
   Lys Arg Phe Phe Glu Thr Gly Gly Lys Ser Leu Leu Asp Gly Leu Ser
   145                 150                 155                 160
   aac ctg gcc aag gac atg gtc aac aac ggc ggc atg ccc agc cag gtg      528
   Asn Leu Ala Lys Asp Met Val Asn Asn Gly Gly Met Pro Ser Gln Val
                   165                 170                 175
   aac atg gat gcc ttc gaa gtg ggc aag aac ctg ggc acc agc gaa ggc      576
   Asn Met Asp Ala Phe Glu Val Gly Lys Asn Leu Gly Thr Ser Glu Gly
               180                 185                 190
   gcg gtg gtg tac cgc aac gat gtg ctg gaa ctg atc cag tac agc ccc      624
   Ala Val Val Tyr Arg Asn Asp Val Leu Glu Leu Ile Gln Tyr Ser Pro
           195                 200                 205
   atc acc gag cag gtg cat gcc cgt ccg ctg ctg gtg gtg cca ccg cag      672
   Ile Thr Glu Gln Val His Ala Arg Pro Leu Leu Val Val Pro Pro Gln
       210                 215                 220
   atc aac aag ttc tac gtg ttc gac ctc agc ccg gaa aag agc ctg gcg        720
   Ile Asn Lys Phe Tyr Val Phe Asp Leu Ser Pro Glu Lys Ser Leu Ala
   225                 230                 235                 240
   cgc ttc tgc ctg cgc tcg cag cag cag acc ttc atc atc agc tgg cgc        768
   Arg Phe Cys Leu Arg Ser Gln Gln Gln Thr Phe Ile Ile Ser Trp Arg
                   245                 250                 255
   aac ccg acc aag gcc cag cgt gaa tgg ggc ctg tcc acc tac atc gat        816
   Asn Pro Thr Lys Ala Gln Arg Glu Trp Gly Leu Ser Thr Tyr Ile Asp
               260                 265                 270
   gcg ctg aaa gaa gcc gtc gac gcg gtg ctg tcg att acc ggc agc aag        864
   Ala Leu Lys Glu Ala Val Asp Ala Val Leu Ser Ile Thr Gly Ser Lys
           275                 280                 285
   gac ctg aac atg ctc ggc gcc tgc tcc ggt ggc atc act tgt acc gca        912
   Asp Leu Asn Met Leu Gly Ala Cys Ser Gly Gly Ile Thr Cys Thr Ala
       290                 295                 300
   ctg gtc ggg cac tat gcc gca ttg ggc gag aac aag gtc aac gcc ctg        960
   Leu Val Gly His Tyr Ala Ala Leu Gly Glu Asn Lys Val Asn Ala Leu
   305                 310                 315                 320
   acc gtg ctg gtc agc gtg ctg gac acc acc atg gac aac cag gtt gct       1008
   Thr Val Leu Val Ser Val Leu Asp Thr Thr Met Asp Asn Gln Val Ala
                   325                 330                 335
   ttg ttt gtc gac gag cag acc ttg gag gcc gcc aag cgc cac tcc tat       1056
   Leu Phe Val Asp Glu Gln Thr Leu Glu Ala Ala Lys Arg His Ser Tyr
               340                 345                 350
   cag gcg ggc gtg ctg gaa ggc agc gaa atg gcc aag gtg ttc gcc tgg       1104
   Gln Ala Gly Val Leu Glu Gly Ser Glu Met Ala Lys Val Phe Ala Trp
           355                 360                 365
   atg cgc ccc aac gac ctg atc tgg aac tac tgg gta aac aac tac ctg       1152
   Met Arg Pro Asn Asp Leu Ile Trp Asn Tyr Trp Val Asn Asn Tyr Leu
       370                 375                 380
   ctc ggc aat gag ccc ccc gtg ttc gac atc ctg ttc tgg aac aac gac       1200
   Leu Gly Asn Glu Pro Pro Val Phe Asp Ile Leu Phe Trp Asn Asn Asp
   385                 390                 395                 400
   acc acg cgc ctg ccg gcc gcc ttc cac ggc gac ctg atc gaa atg ttc       1248
   Thr Thr Arg Leu Pro Ala Ala Phe His Gly Asp Leu Ile Glu Met Phe
                   405                 410                 415
   aag agc aac ccg ctg acc cgc ccc gac gcc ctg aaa gtg tgc ggc acc       1296
   Lys Ser Asn Pro Leu Thr Arg Pro Asp Ala Leu Lys Val Cys Gly Thr
               420                 425                 430
   gcg atc gac ctg aaa cag gtc aaa tgc gac atc tac agc ctc gcc ggc       1344
   Ala Ile Asp Leu Lys Gln Val Lys Cys Asp Ile Tyr Ser Leu Ala Gly
           435                 440                 445
   acc aac gac cac atc acc ccc tgg ccg tca tgc tac cgc tcg gca cat       1392
   Thr Asn Asp His Ile Thr Pro Trp Pro Ser Cys Tyr Arg Ser Ala His
       450                 455                 460
   ctg ttc ggc ggc aag atc gaa ttc gta ctg tcc aac agc ggg cat atc       1440
   Leu Phe Gly Gly Lys Ile Glu Phe Val Leu Ser Asn Ser Gly His Ile
   465                 470                 475                 480
   cag agc atc ctc aac ccg ccg ggc aac ccg aag gca cgt ttc atg acc       1488
   Gln Ser Ile Leu Asn Pro Pro Gly Asn Pro Lys Ala Arg Phe Met Thr
                   485                 490                 495
   ggt gcc gat cgc ccg ggt gac ccg gtg gcc tgg cag gaa aat gcc atc       1536
   Gly Ala Asp Arg Pro Gly Asp Pro Val Ala Trp Gln Glu Asn Ala Ile
               500                 505                 510
   aag cat gca gac tcc tgg tgg ctg cac tgg cag agt tgg ctg ggc gag       1584
   Lys His Ala Asp Ser Trp Trp Leu His Trp Gln Ser Trp Leu Gly Glu
           515                 520                 525
   cgt gcc ggc gcg ctg aaa aag gca ccg acc cgc ctg ggc aac cgt acc       1632
   Arg Ala Gly Ala Leu Lys Lys Ala Pro Thr Arg Leu Gly Asn Arg Thr
       530                 535                 540
   tat gcc gcc ggc gaa gcc tcc cca ggc acc tac gtt cac gag cgt tga       1680
   Tyr Ala Ala Gly Glu Ala Ser Pro Gly Thr Tyr Val His Glu Arg
   545                 550                 555
<210> 30
   <211> 559
   <212> PRT
   <213> Pseudomonas putida
<400> 30
   Met Ser Asn Lys Asn Asn Asp Glu Leu Gln Arg Gln Ala Ser Glu Asn
   1               5                   10                  15
   Thr Met Gly Leu Asn Pro Val Ile Gly Ile Arg Arg Lys Asp Leu Leu
               20                  25                  30
   Ser Ser Ala Arg Thr Val Leu Arg Gln Ala Val Arg Gln Pro Leu His
           35                  40                  45
   Ser Ala Lys His Val Ala His Phe Gly Leu Glu Leu Lys Asn Val Leu
       50                  55                  60
   Leu Gly Lys Ser Ser Leu Ala Pro Asp Ser Asp Asp Arg Arg Phe Asn
   65                  70                  75                  80
   Asp Pro Ala Trp Ser Asn Asn Pro Leu Tyr Arg Arg Tyr Leu Gln Thr
                   85                  90                  95
   Tyr Leu Ala Trp Arg Lys Glu Leu Gln Asp Trp Val Ser Ser Ser Asp
               100                 105                 110
   Leu Ser Pro Gln Asp Ile Ser Arg Gly Gln Phe Val Ile Asn Leu Met
           115                 120                 125
   Thr Glu Ala Met Ala Pro Thr Asn Thr Leu Ser Asn Pro Ala Ala Val
       130                 135                 140
   Lys Arg Phe Phe Glu Thr Gly Gly Lys Ser Leu Leu Asp Gly Leu Ser
   145 150 155 160
   Asn Leu Ala Lys Asp Met Val Asn Asn Gly Gly Met Pro Ser Gln Val
   165 170 175
   Asn Met Asp Ala Phe Glu Val Gly Lys Asn Leu Gly Thr Ser Glu Gly
   180 185 190
   Ala Val Val Tyr Arg Asn Asp Val Leu Glu Leu Ile Gln Tyr Ser Pro
   195 200 205
   Ile Thr Glu Gln Val His Ala Arg Pro Leu Leu Val Val Pro Pro Gln
   210 215 220
   Ile Asn Lys Phe Tyr Val Phe Asp Leu Ser Pro Glu Lys Ser Leu Ala
   225 230 235 240
   Arg Phe Cys Leu Arg Ser Gln Gln Gln Thr Phe Ile Ile Ser Trp Arg
   245 250 255
   Asn Pro Thr Lys Ala Gln Arg Glu Trp Gly Leu Ser Thr Tyr Ile Asp
   260 265 270
   Ala Leu Lys Glu Ala Val Asp Ala Val Leu Ser Ile Thr Gly Ser Lys
   275 280 285
   Asp Leu Asn Met Leu Gly Ala Cys Ser Gly Gly Ile Thr Cys Thr Ala
   290 295 300
   Leu Val Gly His Tyr Ala Ala Leu Gly Glu Asn Lys Val Asn Ala Leu
   305 310 315 320
   Thr Val Leu Val Ser Val Leu Asp Thr Thr Met Asp Asn Gln Val Ala
   325 330 335
   Leu Phe Val Asp Glu Gln Thr Leu Glu Ala Ala Lys Arg His Ser Tyr
   340 345 350
   Gln Ala Gly Val Leu Glu Gly Ser Glu Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Met Phe
                   405                 410                 415
   Lys Ser Asn Pro Leu Thr Arg Pro Asp Ala Leu Lys Val Cys Gly Thr
               420                 425                 430
   Ala Ile Asp Leu Lys Gln Val Lys Cys Asp Ile Tyr Ser Leu Ala Gly
           435                 440                 445
   Thr Asn Asp His Ile Thr Pro Trp Pro Ser Cys Tyr Arg Ser Ala His
       450                 455                 460
   Leu Phe Gly Gly Lys Ile Glu Phe Val Leu Ser Asn Ser Gly His Ile
   465                 470                 475                 480
   Gln Ser Ile Leu Asn Pro Pro Gly Asn Pro Lys Ala Arg Phe Met Thr
                   485                 490                 495
   Gly Ala Asp Arg Pro Gly Asp Pro Val Ala Trp Gln Glu Asn Ala Ile
               500                 505                 510
   Lys His Ala Asp Ser Trp Trp Leu His Trp Gln Ser Trp Leu Gly Glu
           515                 520                 525
   Arg Ala Gly Ala Leu Lys Lys Ala Pro Thr Arg Leu Gly Asn Arg Thr
       530                 535                 540
   Tyr Ala Ala Gly Glu Ala Ser Pro Gly Thr Tyr Val His Glu Arg
   545                 550                 555
<210> 31
   <211> 1683
   <212> DNA
   <213> Pseudomonas putida
<220>
   <221> CDS
   <222> (1)..(1683)
<400> 31
   atg aca gac aaa ccg gcc aaa gga tcg aca acg ctc ccc gcc acc cgc        48
   Met Thr Asp Lys Pro Ala Lys Gly Ser Thr Thr Leu Pro Ala Thr Arg
   1               5                   10                  15
   atg aac gtg cag aac gcc atc ctc ggc ctg cgc ggc cgc gac ctg ctt        96
   Met Asn Val Gln Asn Ala Ile Leu Gly Leu Arg Gly Arg Asp Leu Leu
               20                  25                  30
   tcc acg ctg cgc aac gtc ggc cgc cac ggc ctg cgc cac ccg ctg cat       144
   Ser Thr Leu Arg Asn Val Gly Arg His Gly Leu Arg His Pro Leu His
           35                  40                  45
   acc gcg cat cat ctg ctg gcg ctt ggc ggg cag ttg ggg cgg gtg atg       192
   Thr Ala His His Leu Leu Ala Leu Gly Gly Gln Leu Gly Arg Val Met
       50                  55                  60
   ctg ggg gac acg ccc tac cag ccg aac ccg cgt gac gca cgc ttc agt      240
   Leu Gly Asp Thr Pro Tyr Gln Pro Asn Pro Arg Asp Ala Arg Phe Ser
   65                  70                  75                  80
   gac ccg acc tgg agc cag aac ccg ttc tac cgc cgc ggc ctg caa gcc      288
   Asp Pro Thr Trp Ser Gln Asn Pro Phe Tyr Arg Arg Gly Leu Gln Ala
                   85                  90                  95
   tat ctg gcc tgg cag aag cag aca cgc cag tgg atc gat gaa agc cat      336
   Tyr Leu Ala Trp Gln Lys Gln Thr Arg Gln Trp Ile Asp Glu Ser His
               100                 105                 110
   ttg aac gac gat gat cga gcc cgc gcc cac ttc ctg ttc aac ctg atc      384
   Leu Asn Asp Asp Asp Arg Ala Arg Ala His Phe Leu Phe Asn Leu Ile
           115                 120                 125
   aac gat gcg ctg gcg ccc agc aac tca ctg ctc aat ccg cag gcg gtc      432
   Asn Asp Ala Leu Ala Pro Ser Asn Ser Leu Leu Asn Pro Gln Ala Val
       130                 135                 140
   aag ggg ctg ttc aac acc ggc ggc cag agc ctg gtg cgc ggc gtg gct      480
   Lys Gly Leu Phe Asn Thr Gly Gly Gln Ser Leu Val Arg Gly Val Ala
   145                 150                 155                 160
   cac ctg ctc gac gac ctg cgt cac aac gat ggg ctg cct cgt cag gtg      528
   His Leu Leu Asp Asp Leu Arg His Asn Asp Gly Leu Pro Arg Gln Val
                   165                 170                 175
   gac gag cgc gcc ttc gaa gtg ggc gtt aac ctg gcc gca acc cct ggc      576
   Asp Glu Arg Ala Phe Glu Val Gly Val Asn Leu Ala Ala Thr Pro Gly
               180                 185                 190
   gca gtg gta ttt cgc aac gag ctg ctg gag ctg atc cag tac tcg ccg      624
   Ala Val Val Phe Arg Asn Glu Leu Leu Glu Leu Ile Gln Tyr Ser Pro
           195                 200                 205
   atg agc gaa aag cag cac gca cgc cca ctg ctg gtc gtg ccg cct cag      672
   Met Ser Glu Lys Gln His Ala Arg Pro Leu Leu Val Val Pro Pro Gln
       210                 215                 220
   atc aac agg ttc tac atc ttc gac ctc agc gcg acc aac agc ttc gtc      720
   Ile Asn Arg Phe Tyr Ile Phe Asp Leu Ser Ala Thr Asn Ser Phe Val
   225                 230                 235                 240
   cag tac atg ctc aaa agc ggc ttg cag gtg ttc atg gtc agc tgg agc      768
   Gln Tyr Met Leu Lys Ser Gly Leu Gln Val Phe Met Val Ser Trp Ser
                   245                 250                 255
   aac ccc gac cca cgc cac cgt gaa tgg ggc ctt tcc agc tat gtg caa      816
   Asn Pro Asp Pro Arg His Arg Glu Trp Gly Leu Ser Ser Tyr Val Gln
               260                 265                 270
   gcc ctg gag gaa gcg ctc aat gcc tgc cgc agt atc agc ggc aac cgc      864
   Ala Leu Glu Glu Ala Leu Asn Ala Cys Arg Ser Ile Ser Gly Asn Arg
           275                 280                 285
   gac ccc aac ctg atg ggt gcc tgt gcc ggc ggc ctg acc atg gcc gca      912
   Asp Pro Asn Leu Met Gly Ala Cys Ala Gly Gly Leu Thr Met Ala Ala
       290                 295                 300
   ctg caa ggc cat ctg caa gcc aag aag caa ttg cgc cgg gtg cgc agt      960
   Leu Gln Gly His Leu Gln Ala Lys Lys Gln Leu Arg Arg Val Arg Ser
   305                 310                 315                 320
   gcc acg tat ctg gtc agc ttg ctg gac agc aag ttc gaa agc ccg gcc      1008
   Ala Thr Tyr Leu Val Ser Leu Leu Asp Ser Lys Phe Glu Ser Pro Ala
                   325                 330                 335
   agc ctg ttc gcc gat gag cag acc atc gaa gcg gcc aag cga cgc tcc      1056
   Ser Leu Phe Ala Asp Glu Gln Thr Ile Glu Ala Ala Lys Arg Arg Ser
               340                 345                 350
   tat cag cgt ggc gtg ctg gac ggt ggt gaa gtg gcg cgg atc ttc gcc      1104
   Tyr Gln Arg Gly Val Leu Asp Gly Gly Glu Val Ala Arg Ile Phe Ala
           355                 360                 365
   tgg atg cgg ccc aac gac ctg atc tgg aac tac tgg gta aac aac tac      1152
   Trp Met Arg Pro Asn Asp Leu Ile Trp Asn Tyr Trp Val Asn Asn Tyr
       370                 375                 380
   ctg ctc ggc aag aca ccg cct gcg ttc gac atc ctg tac tgg aat gcc      1200
   Leu Leu Gly Lys Thr Pro Pro Ala Phe Asp Ile Leu Tyr Trp Asn Ala
   385                 390                 395                 400
   gac agc acg cgc ctg ccc gcc gcg ctg cat ggc gac ctg ctg gag ttt      1248
   Asp Ser Thr Arg Leu Pro Ala Ala Leu His Gly Asp Leu Leu Glu Phe
                   405                 410                 415
   ttc aag ctc aac ccc ttg acc tac gcg tcc ggg ctg gag gtg tgc ggt      1296
   Phe Lys Leu Asn Pro Leu Thr Tyr Ala Ser Gly Leu Glu Val Cys Gly
               420                 425                 430
   acg ccg atc gac ctg cag cag gtc aat atc gac agc ttt acc gtg gcc      1344
   Thr Pro Ile Asp Leu Gln Gln Val Asn Ile Asp Ser Phe Thr Val Ala
           435                 440                 445
   ggc agc aac gac cac atc aca cca tgg gat gcg gtg tac cgc tcg gcc      1392
   Gly Ser Asn Asp His Ile Thr Pro Trp Asp Ala Val Tyr Arg Ser Ala
       450                 455                 460
   ttg ctg ctg ggt ggc gag cgg cgc ttc gtg ctg gcc aac agc ggg cat      1440
   Leu Leu Leu Gly Gly Glu Arg Arg Phe Val Leu Ala Asn Ser Gly His
   465                 470                 475                 480
   atc cag agc atc atc aac ccg cca ggc aac ccc aag gcc tac tac ctg      1488
   Ile Gln Ser Ile Ile Asn Pro Pro Gly Asn Pro Lys Ala Tyr Tyr Leu
                   485                 490                 495
   gcc aac ccc aag ctg agc agc gac cca cgc gcc tgg ttc cac gac gcc      1536
   Ala Asn Pro Lys Leu Ser Ser Asp Pro Arg Ala Trp Phe His Asp Ala
               500                 505                 510
   aag cgc agt gaa ggc agc tgg tgg ccg ttg tgg ctg gag tgg atc acc      1584
   Lys Arg Ser Glu Gly Ser Trp Trp Pro Leu Trp Leu Glu Trp Ile Thr
           515                 520                 525
   gca cgc tcc ggc ctg ctc aag gca ccg cgt act gaa ctg ggc aac gcc      1632
   Ala Arg Ser Gly Leu Leu Lys Ala Pro Arg Thr Glu Leu Gly Asn Ala
       530                 535                 540
   act tac cca ctg cta ggc ccc gcg cca ggc acc tac gtg ctg acc cga      1680
   Thr Tyr Pro Leu Leu Gly Pro Ala Pro Gly Thr Tyr Val Leu Thr Arg
   545                 550                 555                 560
   tga                                                                  1683
<210> 32
   <211> 560
   <212> PRT
   <213> Pseudomonas putida
<400> 32
   Met Thr Asp Lys Pro Ala Lys Gly Ser Thr Thr Leu Pro Ala Thr Arg
   1               5                   10                  15
   Met Asn Val Gln Asn Ala Ile Leu Gly Leu Arg Gly Arg Asp Leu Leu
               20                  25                  30
   Ser Thr Leu Arg Asn Val Gly Arg His Gly Leu Arg His Pro Leu His
           35                  40                  45
   Thr Ala His His Leu Leu Ala Leu Gly Gly Gln Leu Gly Arg Val Met
       50                  55                  60
   Leu Gly Asp Thr Pro Tyr Gln Pro Asn Pro Arg Asp Ala Arg Phe Ser
   65                  70                  75                  80
   Asp Pro Thr Trp Ser Gln Asn Pro Phe Tyr Arg Arg Gly Leu Gln Ala
                   85                  90                  95
   Tyr Leu Ala Trp Gln Lys Gln Thr Arg Gln Trp Ile Asp Glu Ser His
               100                 105                 110
   Leu Asn Asp Asp Asp Arg Ala Arg Ala His Phe Leu Phe Asn Leu Ile
           115                 120                 125
   Asn Asp Ala Leu Ala Pro Ser Asn Ser Leu Leu Asn Pro Gln Ala Val
       130                 135                 140
   Lys Gly Leu Phe Asn Thr Gly Gly Gln Ser Leu Val Arg Gly Val Ala
   145                 150                 155                 160
   His Leu Leu Asp Asp Leu Arg His Asn Asp Gly Leu Pro Arg Gln Val
                   165                 170                 175
   Asp Glu Arg Ala Phe Glu Val Gly Val Asn Leu Ala Ala Thr Pro Gly
               180                 185                 190
   Ala Val Val Phe Arg Asn Glu Leu Leu Glu Leu Ile Gln Tyr Ser Pro
           195                 200                 205
   Met Ser Glu Lys Gln His Ala Arg Pro Leu Leu Val Val Pro Pro Gln
       210                 215                 220
   Ile Asn Arg Phe Tyr Ile Phe Asp Leu Ser Ala Thr Asn Ser Phe Val
   225                 230                 235                 240
   Gln Tyr Met Leu Lys Ser Gly Leu Gln Val Phe Met Val Ser Trp Ser
                   245                 250                 255
   Asn Pro Asp Pro Arg His Arg Glu Trp Gly Leu Ser Ser Tyr Val Gln
               260                 265                 270
   Ala Leu Glu Glu Ala Leu Asn Ala Cys Arg Ser Ile Ser Gly Asn Arg
           275                 280                 285
   Asp Pro Asn Leu Met Gly Ala Cys Ala Gly Gly Leu Thr Met Ala Ala
       290                 295                 300
   Leu Gln Gly His Leu Gln Ala Lys Lys Gln Leu Arg Arg Val Arg Ser
   305                 310                 315                 320
   Ala Thr Tyr Leu Val Ser Leu Leu Asp Ser Lys Phe Glu Ser Pro Ala
                   325                 330                 335
   Ser Leu Phe Ala Asp Glu Gln Thr Ile Glu Ala Ala Lys Arg Arg Ser
               340                 345                 350
   Tyr Gln Arg Gly Val Leu Asp Gly Gly Glu Val Ala Arg Ile Phe Ala
           355                 360                 365
   Trp Met Arg Pro Asn Asp Leu Ile Trp Asn Tyr Trp Val Asn Asn Tyr
       370                 375                 380
   Leu Leu Gly Lys Thr Pro Pro Ala Phe Asp Ile Leu Tyr Trp Asn Ala
   385                 390                 395                 400
   Asp Ser Thr Arg Leu Pro Ala Ala Leu His Gly Asp Leu Leu Glu Phe
                   405                 410                 415
   Phe Lys Leu Asn Pro Leu Thr Tyr Ala Ser Gly Leu Glu Val Cys Gly
               420                 425                 430
   Thr Pro Ile Asp Leu Gln Gln Val Asn Ile Asp Ser Phe Thr Val Ala
           435                 440                 445
   Gly Ser Asn Asp His Ile Thr Pro Trp Asp Ala Val Tyr Arg Ser Ala
       450                 455                 460
   Leu Leu Leu Gly Gly Glu Arg Arg Phe Val Leu Ala Asn Ser Gly His
   465                 470                 475                 480
   Ile Gln Ser Ile Ile Asn Pro Pro Gly Asn Pro Lys Ala Tyr Tyr Leu
                   485                 490                 495
   Ala Asn Pro Lys Leu Ser Ser Asp Pro Arg Ala Trp Phe His Asp Ala
   500 505 510
   Lys Arg Ser Glu Gly Ser Trp Trp Pro Leu Trp Leu Glu Trp Ile Thr
   515 520 525
   Ala Arg Ser Gly Leu Leu Lys Ala Pro Arg Thr Glu Leu Gly Asn Ala
   530 535 540
   Thr Tyr Pro Leu Leu Gly Pro Ala Pro Gly Thr Tyr Val Leu Thr Arg
   545 550 555 560
<210> 33
   <211> 888
   <212> DNA
   <213> Pseudomonas putida
<220>
   <221> CDS
   <222> (1)..(888)
<400> 33
   atg agg cca gaa atc gct gta ctt gat atc caa ggt cag tat cgg gtt          48
   Met Arg Pro Glu Ile Ala Val Leu Asp Ile Gln Gly Gln Tyr Arg Val
   1               5                   10                  15
   tac acg gag ttc tat cgc gcg gat gcg gcc gaa aac acg atc atc ctg          96
   Tyr Thr Glu Phe Tyr Arg Ala Asp Ala Ala Glu Asn Thr Ile Ile Leu
               20                  25                  30
   atc aac ggc tcg ctg gcc acc acg gcc tcg ttc gcc cag acg gta cgt         144
   Ile Asn Gly Ser Leu Ala Thr Thr Ala Ser Phe Ala Gln Thr Val Arg
           35                  40                  45
   aac ctg cac cca cag ttc aac gtg gtt ctg ttc gac cag ccg tat tca         192
   Asn Leu His Pro Gln Phe Asn Val Val Leu Phe Asp Gln Pro Tyr Ser
       50                  55                  60
   ggc aag tcc aag ccg cac aac cgt cag gaa cgg ctg atc agc aag gag         240
   Gly Lys Ser Lys Pro His Asn Arg Gln Glu Arg Leu Ile Ser Lys Glu
   65                  70                  75                  80
   acc gag gcg cat atc ctc ctt gag ctg atc gag cac ttc cag gca gac         288
   Thr Glu Ala His Ile Leu Leu Glu Leu Ile Glu His Phe Gln Ala Asp
                   85                  90                  95
   cac gtg atg tct ttt tcg tgg ggt ggc gca agc acg ctg ctg gcg ctg         336
   His Val Met Ser Phe Ser Trp Gly Gly Ala Ser Thr Leu Leu Ala Leu
               100                 105                 110
   gcg cac cag ccg cgg tac gtg aag aag gca gtg gtg agt tcg ttc tcg         384
   Ala His Gln Pro Arg Tyr Val Lys Lys Ala Val Val Ser Ser Phe Ser
           115                 120                 125
   cca gtg atc aac gag cca atg cgc gac tat ctg gac cgt ggc tgc cag         432
   Pro Val Ile Asn Glu Pro Met Arg Asp Tyr Leu Asp Arg Gly Cys Gln
       130                 135                 140
   tac ctg gcc gcc tgc gac cgt tat cag gtc ggc aac ctg gtc aat gac         480
   Tyr Leu Ala Ala Cys Asp Arg Tyr Gln Val Gly Asn Leu Val Asn Asp
   145                 150                 155                 160
   acc atc ggc aag cac ttg ccg tcg ctg ctc aaa cgc ttc aac tac cgc        528
   Thr Ile Gly Lys His Leu Pro Ser Leu Leu Lys Arg Phe Asn Tyr Arg
                   165                 170                 175
   cat gtg agc agc ctg gac agc cac gag tac gca cag atg cac ttc cac        576
   His Val Ser Ser Leu Asp Ser His Glu Tyr Ala Gln Met His Phe His
               180                 185                 190
   atc aac caa gtg ctg gag cac gac ctg gaa cgt gcg ctg caa ggc gcg        624
   Ile Asn Gln Val Leu Glu His Asp Leu Glu Arg Ala Leu Gln Gly Ala
           195                 200                 205
   cgc aat atc aac atc ccg gtg ttg ttc atc aac ggc gaa cgc gac gag        672
   Arg Asn Ile Asn Ile Pro Val Leu Phe Ile Asn Gly Glu Arg Asp Glu
       210                 215                 220
   tac acc acg gtc gaa gat gcg cgg cag ttc agc aag cat gtg ggc aga        720
   Tyr Thr Thr Val Glu Asp Ala Arg Gln Phe Ser Lys His Val Gly Arg
   225                 230                 235                 240
   agc cag ttc agc gtg atc cgc gat gcg ggc cac ttc ctg gac atg gag        768
   Ser Gln Phe Ser Val Ile Arg Asp Ala Gly His Phe Leu Asp Met Glu
                   245                 250                 255
   aac aag acc gcc tgc gag aac acc cgc agt gtc atg ctg ggg ttc ctc        816
   Asn Lys Thr Ala Cys Glu Asn Thr Arg Ser Val Met Leu Gly Phe Leu
               260                 265                 270
   aag cca acc gtg cgt gaa ccc cgc caa cgt tac caa ccc gtg caa cag        864
   Lys Pro Thr Val Arg Glu Pro Arg Gln Arg Tyr Gln Pro Val Gln Gln
           275                 280                 285
   ggg cag cat gca ttg gcc atc tga                                        888
   Gly Gln His Ala Leu Ala Ile
       290                 295
<210> 34
   <211> 295
   <212> PRT
   <213> Pseudomonas putida
<400> 34
   Met Arg Pro Glu Ile Ala Val Leu Asp Ile Gln Gly Gln Tyr Arg Val
   1               5                   10                  15
   Tyr Thr Glu Phe Tyr Arg Ala Asp Ala Ala Glu Asn Thr Ile Ile Leu
               20                  25                  30
   Ile Asn Gly Ser Leu Ala Thr Thr Ala Ser Phe Ala Gln Thr Val Arg
           35                  40                  45
   Asn Leu His Pro Gln Phe Asn Val Val Leu Phe Asp Gln Pro Tyr Ser
       50                  55                  60
   Gly Lys Ser Lys Pro His Asn Arg Gln Glu Arg Leu Ile Ser Lys Glu
   65                  70                  75                  80
   Thr Glu Ala His Ile Leu Leu Glu Leu Ile Glu His Phe Gln Ala Asp
                   85                  90                  95
   His Val Met Ser Phe Ser Trp Gly Gly Ala Ser Thr Leu Leu Ala Leu
               100                 105                 110
   Ala His Gln Pro Arg Tyr Val Lys Lys Ala Val Val Ser Ser Phe Ser
           115                 120                 125
   Pro Val Ile Asn Glu Pro Met Arg Asp Tyr Leu Asp Arg Gly Cys Gln
       130                 135                 140
   Tyr Leu Ala Ala Cys Asp Arg Tyr Gln Val Gly Asn Leu Val Asn Asp
   145                 150                 155                 160
   Thr Ile Gly Lys His Leu Pro Ser Leu Leu Lys Arg Phe Asn Tyr Arg
                   165                 170                 175
   His Val Ser Ser Leu Asp Ser His Glu Tyr Ala Gln Met His Phe His
               180                 185                 190
   Ile Asn Gln Val Leu Glu His Asp Leu Glu Arg Ala Leu Gln Gly Ala
           195                 200                 205
   Arg Asn Ile Asn Ile Pro Val Leu Phe Ile Asn Gly Glu Arg Asp Glu
       210                 215                 220
   Tyr Thr Thr Val Glu Asp Ala Arg Gln Phe Ser Lys His Val Gly Arg
   225                 230                 235                 240
   Ser Gln Phe Ser Val Ile Arg Asp Ala Gly His Phe Leu Asp Met Glu
                   245                 250                 255
   Asn Lys Thr Ala Cys Glu Asn Thr Arg Ser Val Met Leu Gly Phe Leu
               260                 265                 270
   Lys Pro Thr Val Arg Glu Pro Arg Gln Arg Tyr Gln Pro Val Gln Gln
           275                 280                 285
   Gly Gln His Ala Leu Ala Ile
       290                 295
<210> 35
   <211> 903
   <212> DNA
   <213> Pseudomonas aeruginosa
<220>
   <221> CDS
   <222> (1)..(903)
<400> 35
   atg agg ccg gaa aca gcc atc atc gag atc cac ggg caa tac agg att         48
   Met Arg Pro Glu Thr Ala Ile Ile Glu Ile His Gly Gln Tyr Arg Ile
   1               5                   10                  15
   cac acc gag ttc tac ggc aac ccc gcg gcg cag caa acc atc atc ctg         96
   His Thr Glu Phe Tyr Gly Asn Pro Ala Ala Gln Gln Thr Ile Ile Leu
               20                  25                  30
   gtc aac ggc tcg ctg tcg acc aca gcg tcc ttc gcc cag acc gtg aag        144
   Val Asn Gly Ser Leu Ser Thr Thr Ala Ser Phe Ala Gln Thr Val Lys
           35                  40                  45
   tac ctg cag ccg cat tac aac gtg gtg ctc tac gac cag ccg tat gcc        192
   Tyr Leu Gln Pro His Tyr Asn Val Val Leu Tyr Asp Gln Pro Tyr Ala
       50                  55                  60
   ggc cag tcc aaa ccc cat aac gaa aac cac acg ccg atc agc aag gaa        240
   Gly Gln Ser Lys Pro His Asn Glu Asn His Thr Pro Ile Ser Lys Glu
   65                  70                  75                  80
   tgc gag gcc agg atc ctg ctg gaa ctg atc gaa cgc ttc cgt gcc gag        288
   Cys Glu Ala Arg Ile Leu Leu Glu Leu Ile Glu Arg Phe Arg Ala Glu
                   85                  90                  95
   gta gtg atg tcg ttc tcg tgg ggc ggc gtc gcc acc ctg ctg gcc ctg        336
   Val Val Met Ser Phe Ser Trp Gly Gly Val Ala Thr Leu Leu Ala Leu
               100                 105                 110
   gcg caa cgt ccc gga cgg atc cgc agg gcg gtg gtc aac tca ttc tcg        384
   Ala Gln Arg Pro Gly Arg Ile Arg Arg Ala Val Val Asn Ser Phe Ser
           115                 120                 125
   cct cag ctc aac ccg gcc atg ctc gac tac ctg cat cgc ggc ctc gac        432
   Pro Gln Leu Asn Pro Ala Met Leu Asp Tyr Leu His Arg Gly Leu Asp
       130                 135                 140
   tac ctc gcc gcc tgc gat cgc acc cag atc ggc aac ctg gtc aac gaa        480
   Tyr Leu Ala Ala Cys Asp Arg Thr Gln Ile Gly Asn Leu Val Asn Glu
   145                 150                 155                 160
   acc atc ggc cgc tac ctg cca cag ttg ttc aag cgc tac aac ttc cgc        528
   Thr Ile Gly Arg Tyr Leu Pro Gln Leu Phe Lys Arg Tyr Asn Phe Arg
                   165                 170                 175
   cac gtc agc agc ctg gac gag cac gaa tac cac cag atg cac ttc cat        576
   His Val Ser Ser Leu Asp Glu His Glu Tyr His Gln Met His Phe His
               180                 185                 190
   atc cgc gaa gtg ctg cgc ctg aac gcc gat agc tat acc gag agc ttc        624
   Ile Arg Glu Val Leu Arg Leu Asn Ala Asp Ser Tyr Thr Glu Ser Phe
           195                 200                 205
   gcc ggc atc gag atc ccg atg ctg ttc atg aac ggc gag ctg gac atc        672
   Ala Gly Ile Glu Ile Pro Met Leu Phe Met Asn Gly Glu Leu Asp Ile
       210                 215                 220
   tac acc acg ccc cac gaa gcc cgc cag ttc ggc caa ctg atc cgc ggc        720
   Tyr Thr Thr Pro His Glu Ala Arg Gln Phe Gly Gln Leu Ile Arg Gly
   225                 230                 235                 240
   gcg gaa ttc cac acc atc cgc aat gcc ggc cac ttc atc gac gtc gag        768
   Ala Glu Phe His Thr Ile Arg Asn Ala Gly His Phe Ile Asp Val Glu
                   245                 250                 255
   cac aag gcc gcc tgg cag cag acc cag gac gcc ctg ctg gcc ttc ctc         816
   His Lys Ala Ala Trp Gln Gln Thr Gln Asp Ala Leu Leu Ala Phe Leu
               260                 265                 270
   cgc ccg cag cgc acg cag ccg ctc aac ccg atc tac cgc ccc cag ccc         864
   Arg Pro Gln Arg Thr Gln Pro Leu Asn Pro Ile Tyr Arg Pro Gln Pro
           275                 280                 285
   aac ggc gcc agc gtc ccc ctc gcc gcc ctc gcc agc taa                     903
   Asn Gly Ala Ser Val Pro Leu Ala Ala Leu Ala Ser
       290                 295                 300
<210> 36
   <211> 300
   <212> PRT
   <213> Pseudomonas aeruginosa
<400> 36
   Met Arg Pro Glu Thr Ala Ile Ile Glu Ile His Gly Gln Tyr Arg Ile
   1               5                   10                  15
   His Thr Glu Phe Tyr Gly Asn Pro Ala Ala Gln Gln Thr Ile Ile Leu
               20                  25                  30
   Val Asn Gly Ser Leu Ser Thr Thr Ala Ser Phe Ala Gln Thr Val Lys
           35                  40                  45
   Tyr Leu Gln Pro His Tyr Asn Val Val Leu Tyr Asp Gln Pro Tyr Ala
       50                  55                  60
   Gly Gln Ser Lys Pro His Asn Glu Asn His Thr Pro Ile Ser Lys Glu
   65                  70                  75                  80
   Cys Glu Ala Arg Ile Leu Leu Glu Leu Ile Glu Arg Phe Arg Ala Glu
                   85                  90                  95
   Val Val Met Ser Phe Ser Trp Gly Gly Val Ala Thr Leu Leu Ala Leu
               100                 105                 110
   Ala Gln Arg Pro Gly Arg Ile Arg Arg Ala Val Val Asn Ser Phe Ser
           115                 120                 125
   Pro Gln Leu Asn Pro Ala Met Leu Asp Tyr Leu His Arg Gly Leu Asp
       130                 135                 140
   Tyr Leu Ala Ala Cys Asp Arg Thr Gln Ile Gly Asn Leu Val Asn Glu
   145                 150                 155                 160
   Thr Ile Gly Arg Tyr Leu Pro Gln Leu Phe Lys Arg Tyr Asn Phe Arg
                   165                 170                 175
   His Val Ser Ser Leu Asp Glu His Glu Tyr His Gln Met His Phe His
               180                 185                 190
   Ile Arg Glu Val Leu Arg Leu Asn Ala Asp Ser Tyr Thr Glu Ser Phe
           195                 200                 205
   Ala Gly Ile Glu Ile Pro Met Leu Phe Met Asn Gly Glu Leu Asp Ile
       210                 215                 220
   Tyr Thr Thr Pro His Glu Ala Arg Gln Phe Gly Gln Leu Ile Arg Gly
   225                 230                 235                 240
   Ala Glu Phe His Thr Ile Arg Asn Ala Gly His Phe Ile Asp Val Glu
                   245                 250                 255
   His Lys Ala Ala Trp Gln Gln Thr Gln Asp Ala Leu Leu Ala Phe Leu
               260                 265                 270
   Arg Pro Gln Arg Thr Gln Pro Leu Asn Pro Ile Tyr Arg Pro Gln Pro
           275                 280                 285
   Asn Gly Ala Ser Val Pro Leu Ala Ala Leu Ala Ser
       290                 295                 300
<210> 37
   <211> 2310
   <212> DNA
   <213> Pseudomonas aeruginosa
<400> 37
   aggtaccaga tctggcattt ttgggaggtg tgaaatgcgg cgcgaaagtc tgttggtatc        60
   ggtttgcaag ggcctgcggg tacatgtcga gcgcgttggg caggatcccg ggcgcagcac       120
   ggtgatgctg gtcaacggcg cgatggcgac caccgcctcg ttcgcccgga cctgcaagtg       180
   cctggccgaa catttcaacg tggtgctgtt cgacctgccc ttcgccgggc agtcgcgtca       240
   gcacaacccg cagcgggggt tgatcaccaa ggacgacgag gtggaaatcc tcctggcgct       300
   gatcgagcgc ttcgaggtca atcacctggt ctccgcgtcc tggggcggta tctccacgct       360
   gctggcgctg tcgcgcaatc cgcgcggcat ccgcagctcg gtggtgatgg cattcgcccc       420
   tggactgaac caggcgatgc tcgactacgt cgggcgggcg caggcgctga tcgagctgga       480
   cgacaagtcg gcgatcggcc atctgctcaa cgagaccgtc ggcaaatacc tgccgccgcg       540
   cctgaaagcc agcaaccatc agcacatggc ttcgctggcc accggcgaat acgagcaggc       600
   gcgctttcac atcgaccagg tgctggcgct caacgatcgg ggctacctgg cttgcctgga       660
   gcggatccag agccacgtgc atttcatcaa cggcagctgg gacgaataca ccaccgccga       720
   ggacgcccgc cagttccgcg actacctgcc gcactgcagt ttctcgcggg tggagggcac       780
   cgggcatttc ctcgacctgg agtccaagct ggccgcggta cgcgtgcacc gcgccctgct       840
   cgagcacctg ctgaagcaac cggagccgca gcgggcggaa cgcgcggcgg gattccacga 900
   gatggccatc ggctacgcct gaacccttga cctgcgaaga cccggcctgg ccgggctttg 960
   cggttgcata acgcacggag tagcaccatg cacgccatcc tcatcgccat cggctcggcc 1020
   ggcgacgtat ttcccttcat cggcctggcc cggaccctga aattgcgcgg gcaccgcgtg 1080
   agcctctgca ccatcccggt gtttcgcgac gcggtggagc agcacggcat cgcgttcgtc 1140
   ccgctgagcg acgaactgac ctaccgccgg accatgggcg atccgcgcct gtgggacccc 1200
   aagacgtcct tcggcgtgct ctggcaaacc atcgccggga tgatcgagcc ggtctacgag 1260
   tacgtctcgg cgcagcgcca tgacgacatc gtggtggtcg gctcgctctg ggcgctgggc 1320
   gcacgcatcg ctcacgagaa gtacgggatt ccctacctgt ccgcgcaggt ctcgccatcg 1380
   accttgttgt cggcgcacct gccgccggta caccccaagt tcaacgtgcc cgagcagatg 1440
   ccgctggcga tgcgcaagct gctctggcgc tgcatcgagc gcttcaagct ggatcgcacc 1500
   tgcgcgccgg atatcaacgc ggtgcggcgc aaggtcggcc tggagacgcc ggtgaagcgc 1560
   atcttcaccc aatggatgca ttcgccgcag ggcgtggtct gcctgttccc ggcctggttc 1620
   gcgccgcccc agcaggattg gccgcaaccc ctgcacatga ccggcttccc gctgttcgac 1680
   ggcagtatcc cggggacccc gctcgacgac gaactgcaac gctttctcga tcagggcagc 1740
   cggccgctgg tgttcaccca gggctcgacc gaacacctgc agggcgactt ctacgccatg 1800
   gccctgcgcg cgctggaacg cctcggcgcg cgtgggatct tcctcaccgg cgccggccag 1860
   gaaccgctgc gcggcttgcc gaaccacgtg ctgcagcgcg cctacgcgcc actgggagcc 1920
   ttgctgccat cgtgcgccgg gctggtccat ccgggcggta tcggcgccat gagcctggcc 1980
   ttggcggcgg gggtgccgca ggtgctgctg ccctgcgccc acgaccagtt cgacaatgcc 2040
   gaacggctgg tccggctcgg ctgcgggatg cgcctgggcg tgccattgcg cgagcaggag 2100
   ttgcgcgggg cgctgtggcg cttgctcgag gacccggcca tggcggcggc ctgtcggcgt 2160
   ttcatggaat tgtcacaacc gcacagtatc gcttgcggta aagcggccca ggtggtcgaa 2220
   cgttgtcata gggaggggga tgcgcgatgg ctgaaggctg cgtcctgaac ggtgctggca 2280
   taacagatag ggttgcctct agagagctca 2310
<210> 38
   <211> 7422
   <212> DNA
   <213> Artificial
<220>
   <223> Vector
<400> 38
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagaggcaac cctatctgtt atgccagcac cgttcaggac gcagccttca gccatcgcgc 3300
   atccccctcc ctatgacaac gttcgaccac ctgggccgct ttaccgcaag cgatactgtg 3360
   cggttgtgac aattccatga aacgccgaca ggccgccgcc atggccgggt cctcgagcaa 3420
   gcgccacagc gccccgcgca actcctgctc gcgcaatggc acgcccaggc gcatcccgca 3480
   gccgagccgg accagccgtt cggcattgtc gaactggtcg tgggcgcagg gcagcagcac 3540
   ctgcggcacc cccgccgcca aggccaggct catggcgccg ataccgcccg gatggaccag 3600
   cccggcgcac gatggcagca aggctcccag tggcgcgtag gcgcgctgca gcacgtggtt 3660
   cggcaagccg cgcagcggtt cctggccggc gccggtgagg aagatcccac gcgcgccgag 3720
   gcgttccagc gcgcgcaggg ccatggcgta gaagtcgccc tgcaggtgtt cggtcgagcc 3780
   ctgggtgaac accagcggcc ggctgccctg atcgagaaag cgttgcagtt cgtcgtcgag 3840
   cggggtcccc gggatactgc cgtcgaacag cgggaagccg gtcatgtgca ggggttgcgg 3900
   ccaatcctgc tggggcggcg cgaaccaggc cgggaacagg cagaccacgc cctgcggcga 3960
   atgcatccat tgggtgaaga tgcgcttcac cggcgtctcc aggccgacct tgcgccgcac 4020
   cgcgttgata tccggcgcgc aggtgcgatc cagcttgaag cgctcgatgc agcgccagag 4080
   cagcttgcgc atcgccagcg gcatctgctc gggcacgttg aacttggggt gtaccggcgg 4140
   caggtgcgcc gacaacaagg tcgatggcga gacctgcgcg gacaggtagg gaatcccgta 4200
   cttctcgtga gcgatgcgtg cgcccagcgc ccagagcgag ccgaccacca cgatgtcgtc 4260
   atggcgctgc gccgagacgt actcgtagac cggctcgatc atcccggcga tggtttgcca 4320
   gagcacgccg aaggacgtct tggggtccca caggcgcgga tcgcccatgg tccggcggta 4380
   ggtcagttcg tcgctcagcg ggacgaacgc gatgccgtgc tgctccaccg cgtcgcgaaa 4440
   caccgggatg gtgcagaggc tcacgcggtg cccgcgcaat ttcagggtcc gggccaggcc 4500
   gatgaaggga aatacgtcgc cggccgagcc gatggcgatg aggatggcgt gcatggtgct 4560
   actccgtgcg ttatgcaacc gcaaagcccg gccaggccgg gtcttcgcag gtcaagggtt 4620
   caggcgtagc cgatggccat ctcgtggaat cccgccgcgc gttccgcccg ctgcggctcc 4680
   ggttgcttca gcaggtgctc gagcagggcg cggtgcacgc gtaccgcggc cagcttggac 4740
   tccaggtcga ggaaatgccc ggtgccctcc acccgcgaga aactgcagtg cggcaggtag 4800
   tcgcggaact ggcgggcgtc ctcggcggtg gtgtattcgt cccagctgcc gttgatgaaa 4860
   tgcacgtggc tctggatccg ctccaggcaa gccaggtagc cccgatcgtt gagcgccagc 4920
   acctggtcga tgtgaaagcg cgcctgctcg tattcgccgg tggccagcga agccatgtgc 4980
   tgatggttgc tggctttcag gcgcggcggc aggtatttgc cgacggtctc gttgagcaga 5040
   tggccgatcg ccgacttgtc gtccagctcg atcagcgcct gcgcccgccc gacgtagtcg 5100
   agcatcgcct ggttcagtcc aggggcgaat gccatcacca ccgagctgcg gatgccgcgc 5160
   ggattgcgcg acagcgccag cagcgtggag ataccgcccc aggacgcgga gaccaggtga 5220
   ttgacctcga agcgctcgat cagcgccagg aggatttcca cctcgtcgtc cttggtgatc 5280
   aacccccgct gcgggttgtg ctgacgcgac tgcccggcga agggcaggtc gaacagcacc 5340
   acgttgaaat gttcggccag gcacttgcag gtccgggcga acgaggcggt ggtcgccatc 5400
   gcgccgttga ccagcatcac cgtgctgcgc ccgggatcct gcccaacgcg ctcgacatgt 5460
   acccgcaggc ccttgcaaac cgataccaac agactttcgc gccgcatttc acacctccca 5520
   aaaatgccag atcccccggg ctgcaggaat tcgatatcaa gcttatcgat accgtcgacc 5580
   tcgagggggg gcccggtacc cagcttttgt tccctttagt gagggttaat tgcgcgcttg 5640
   gcgtaatcat ggtcatagct gtttcctgtg tgaaattgtt atccgctcac aattccacac 5700
   aacatacgag ccggaagcat aaagtgtaaa gcctggggtg cctaatgagt gagctaactc 5760
   acattaattg cgttgcgctc actgcccgct ttccagtcgg gaaacctgtc gtgccagctg 5820
   cattaatgaa tcggccaacg cgcggggaga ggcggtttgc gtattgggcg catgcataaa 5880
   aactgttgta attcattaag cattctgccg acatggaagc catcacaaac ggcatgatga 5940
   acctgaatcg ccagcggcat cagcaccttg tcgccttgcg tataatattt gcccatgggg 6000
   gtgggcgaag aactccagca tgagatcccc gcgctggagg atcatccagc cggcgtcccg 6060
   gaaaacgatt ccgaagccca acctttcata gaaggcggcg gtggaatcga aatctcgtga 6120
   tggcaggttg ggcgtcgctt ggtcggtcat ttcgaacccc agagtcccgc tcagaagaac 6180
   tcgtcaagaa ggcgatagaa ggcgatgcgc tgcgaatcgg gagcggcgat accgtaaagc 6240
   acgaggaagc ggtcagccca ttcgccgcca agctcttcag caatatcacg ggtagccaac 6300
   gctatgtcct gatagcggtc cgccacaccc agccggccac agtcgatgaa tccagaaaag 6360
   cggccatttt ccaccatgat attcggcaag caggcatcgc catgggtcac gacgagatcc 6420
   tcgccgtcgg gcatgcgcgc cttgagcctg gcgaacagtt cggctggcgc gagcccctga 6480
   tgctcttcgt ccagatcatc ctgatcgaca agaccggctt ccatccgagt acgtgctcgc 6540
   tcgatgcgat gtttcgcttg gtggtcgaat gggcaggtag ccggatcaag cgtatgcagc 6600
   cgccgcattg catcagccat gatggatact ttctcggcag gagcaaggtg agatgacagg 6660
   agatcctgcc ccggcacttc gcccaatagc agccagtccc ttcccgcttc agtgacaacg 6720
   tcgagcacag ctgcgcaagg aacgcccgtc gtggccagcc acgatagccg cgctgcctcg 6780
   tcctgcagtt cattcagggc accggacagg tcggtcttga caaaaagaac cgggcgcccc 6840
   tgcgctgaca gccggaacac ggcggcatca gagcagccga ttgtctgttg tgcccagtca 6900
   tagccgaata gcctctccac ccaagcggcc ggagaacctg cgtgcaatcc atcttgttca 6960
   atcatgcgaa acgatcctca tcctgtctct tgatcagatc ttgatcccct gcgccatcag 7020
   atccttggcg gcaagaaagc catccagttt actttgcagg gcttcccaac cttaccagag 7080
   ggcgccccag ctggcaattc cggttcgctt gctgtccata aaaccgccca gtctagctat 7140
   cgccatgtaa gcccactgca agctacctgc tttctctttg cgcttgcgtt ttcccttgtc 7200
   cagatagccc agtagctgac attcatccca ggtggcactt ttcggggaaa tgtgcgcgcc 7260
   cgcgttcctg ctggcgctgg gcctgtttct ggcgctggac ttcccgctgt tccgtcagca 7320
   gcttttcgcc cacggccttg atgatcgcgg cggccttggc ctgcatatcc cgattcaacg 7380
   gccccagggc gtccagaacg ggcttcaggc gctcccgaag gt 7422
<210> 39
   <211> 3294
   <212> DNA
   <213> Pseudomonas aeruginosa
<400> 39
   aggtaccaga tctggcattt ttgggaggtg tgaaatgcgg cgcgaaagtc tgttggtatc      60
   ggtttgcaag ggcctgcggg tacatgtcga gcgcgttggg caggatcccg ggcgcagcac     120
   ggtgatgctg gtcaacggcg cgatggcgac caccgcctcg ttcgcccgga cctgcaagtg     180
   cctggccgaa catttcaacg tggtgctgtt cgacctgccc ttcgccgggc agtcgcgtca     240
   gcacaacccg cagcgggggt tgatcaccaa ggacgacgag gtggaaatcc tcctggcgct     300
   gatcgagcgc ttcgaggtca atcacctggt ctccgcgtcc tggggcggta tctccacgct 360
   gctggcgctg tcgcgcaatc cgcgcggcat ccgcagctcg gtggtgatgg cattcgcccc 420
   tggactgaac caggcgatgc tcgactacgt cgggcgggcg caggcgctga tcgagctgga 480
   cgacaagtcg gcgatcggcc atctgctcaa cgagaccgtc ggcaaatacc tgccgccgcg 540
   cctgaaagcc agcaaccatc agcacatggc ttcgctggcc accggcgaat acgagcaggc 600
   gcgctttcac atcgaccagg tgctggcgct caacgatcgg ggctacctgg cttgcctgga 660
   gcggatccag agccacgtgc atttcatcaa cggcagctgg gacgaataca ccaccgccga 720
   ggacgcccgc cagttccgcg actacctgcc gcactgcagt ttctcgcggg tggagggcac 780
   cgggcatttc ctcgacctgg agtccaagct ggccgcggta cgcgtgcacc gcgccctgct 840
   cgagcacctg ctgaagcaac cggagccgca gcgggcggaa cgcgcggcgg gattccacga 900
   gatggccatc ggctacgcct gaacccttga cctgcgaaga cccggcctgg ccgggctttg 960
   cggttgcata acgcacggag tagcaccatg cacgccatcc tcatcgccat cggctcggcc 1020
   ggcgacgtat ttcccttcat cggcctggcc cggaccctga aattgcgcgg gcaccgcgtg 1080
   agcctctgca ccatcccggt gtttcgcgac gcggtggagc agcacggcat cgcgttcgtc 1140
   ccgctgagcg acgaactgac ctaccgccgg accatgggcg atccgcgcct gtgggacccc 1200
   aagacgtcct tcggcgtgct ctggcaaacc atcgccggga tgatcgagcc ggtctacgag 1260
   tacgtctcgg cgcagcgcca tgacgacatc gtggtggtcg gctcgctctg ggcgctgggc 1320
   gcacgcatcg ctcacgagaa gtacgggatt ccctacctgt ccgcgcaggt ctcgccatcg 1380
   accttgttgt cggcgcacct gccgccggta caccccaagt tcaacgtgcc cgagcagatg 1440
   ccgctggcga tgcgcaagct gctctggcgc tgcatcgagc gcttcaagct ggatcgcacc 1500
   tgcgcgccgg atatcaacgc ggtgcggcgc aaggtcggcc tggagacgcc ggtgaagcgc 1560
   atcttcaccc aatggatgca ttcgccgcag ggcgtggtct gcctgttccc ggcctggttc 1620
   gcgccgcccc agcaggattg gccgcaaccc ctgcacatga ccggcttccc gctgttcgac 1680
   ggcagtatcc cggggacccc gctcgacgac gaactgcaac gctttctcga tcagggcagc 1740
   cggccgctgg tgttcaccca gggctcgacc gaacacctgc agggcgactt ctacgccatg 1800
   gccctgcgcg cgctggaacg cctcggcgcg cgtgggatct tcctcaccgg cgccggccag 1860
   gaaccgctgc gcggcttgcc gaaccacgtg ctgcagcgcg cctacgcgcc actgggagcc 1920
   ttgctgccat cgtgcgccgg gctggtccat ccgggcggta tcggcgccat gagcctggcc 1980
   ttggcggcgg gggtgccgca ggtgctgctg ccctgcgccc acgaccagtt cgacaatgcc 2040
   gaacggctgg tccggctcgg ctgcgggatg cgcctgggcg tgccattgcg cgagcaggag 2100
   ttgcgcgggg cgctgtggcg cttgctcgag gacccggcca tggcggcggc ctgtcggcgt 2160
   ttcatggaat tgtcacaacc gcacagtatc gcttgcggta aagcggccca ggtggtcgaa 2220
   cgttgtcata gggaggggga tgcgcgatgg ctgaaggctg cgtcctgacc tacgggagaa      2280
   gaacgatcat ggaccggata gacatgggcg tgctggtggt actgttcaat cctggcgacg      2340
   acgacctgga acaccttggc gaactggcgg cggcgtttcc gcaactgcgc ttccttgccg      2400
   tcgacaactc accgcacagc gatccgcagc gcaatgcccg gctgcgcggg caaggcatcg      2460
   ccgtgctgca ccacggcaac cggcagggca tcgccggcgc cttcaaccag ggactcgacg      2520
   cgctattccg gcgtggcgtg cagggtgtgc tgctgctcga ccaggactcc cgtcccggcg      2580
   gcgccttcct cgccgcccag tggcgcaacc tgcaggcgcg caacggtcag gcctgcctgc      2640
   tcggcccacg gatcttcgac cggggtgacc ggcgcttcct gccggccatc catctcgacg      2700
   gactgacgct caggcaattg tctctggacg gcctgacgac cccgcagcgc acctcgttcc      2760
   tgatctcctc cggctgcctg ctgacccgcg aggcctacca gcgcctcggc cacttcgacg      2820
   aggaactgtt catcgaccac gtggacaccg aatacagcct gcgcgcccag gcgctggacg      2880
   tgcccctgta cgtcgacccg cggctggtcc tcgagcaccg catcggcacg cgcaagaccc      2940
   gccgcctcgg cggtctcagc ctcagcgcga tgaaccacgc cccgctgcgc cgctactacc      3000
   tggcgcgcaa cggcctgctg gtcctgcgcc gctacgcccg gtcctcgccg ctggccctgc      3060
   tggcgaacct gccgaccctg acccagggcc tcgcggtgct cctgctcgaa cgcgacaagc      3120
   tgctcaagct gcgctgcctg ggctggggcc tgtgggacgg cctgcgggga cgcggcggcg      3180
   cgctggagac caaccgcccg cgcctgctga agcgcctcgc cggcccggcc gtggcgtccg      3240
   tagcttccgg caaggccaag gcctagtcgg cgaaacgcat tccctctaga gagc            3294
<210> 40
   <211> 8409
   <212> DNA
   <213> Artificial
<220>
   <223> Vector
<400> 40
   accttcggga gcgcctgaag cccgttctgg acgccctggg gccgttgaat cgggatatgc      60
   aggccaaggc cgccgcgatc atcaaggccg tgggcgaaaa gctgctgacg gaacagcggg     120
   aagtccagcg ccagaaacag gcccagcgcc agcaggaacg cgggcgcgca catttccccg     180
   aaaagtgcca cctgggatga atgtcagcta ctgggctatc tggacaaggg aaaacgcaag     240
   cgcaaagaga aagcaggtag cttgcagtgg gcttacatgg cgatagctag actgggcggt     300
   tttatggaca gcaagcgaac cggaattgcc agctggggcg ccctctggta aggttgggaa     360
   gccctgcaaa gtaaactgga tggctttctt gccgccaagg atctgatggc gcaggggatc     420
   aagatctgat caagagacag gatgaggatc gtttcgcatg attgaacaag atggattgca     480
   cgcaggttct ccggccgctt gggtggagag gctattcggc tatgactggg cacaacagac     540
   aatcggctgc tctgatgccg ccgtgttccg gctgtcagcg caggggcgcc cggttctttt     600
   tgtcaagacc gacctgtccg gtgccctgaa tgaactgcag gacgaggcag cgcggctatc 660
   gtggctggcc acgacgggcg ttccttgcgc agctgtgctc gacgttgtca ctgaagcggg 720
   aagggactgg ctgctattgg gcgaagtgcc ggggcaggat ctcctgtcat ctcaccttgc 780
   tcctgccgag aaagtatcca tcatggctga tgcaatgcgg cggctgcata cgcttgatcc 840
   ggctacctgc ccattcgacc accaagcgaa acatcgcatc gagcgagcac gtactcggat 900
   ggaagccggt cttgtcgatc aggatgatct ggacgaagag catcaggggc tcgcgccagc 960
   cgaactgttc gccaggctca aggcgcgcat gcccgacggc gaggatctcg tcgtgaccca 1020
   tggcgatgcc tgcttgccga atatcatggt ggaaaatggc cgcttttctg gattcatcga 1080
   ctgtggccgg ctgggtgtgg cggaccgcta tcaggacata gcgttggcta cccgtgatat 1140
   tgctgaagag cttggcggcg aatgggctga ccgcttcctc gtgctttacg gtatcgccgc 1200
   tcccgattcg cagcgcatcg ccttctatcg ccttcttgac gagttcttct gagcgggact 1260
   ctggggttcg aaatgaccga ccaagcgacg cccaacctgc catcacgaga tttcgattcc 1320
   accgccgcct tctatgaaag gttgggcttc ggaatcgttt tccgggacgc cggctggatg 1380
   atcctccagc gcggggatct catgctggag ttcttcgccc acccccatgg gcaaatatta 1440
   tacgcaaggc gacaaggtgc tgatgccgct ggcgattcag gttcatcatg ccgtttgtga 1500
   tggcttccat gtcggcagaa tgcttaatga attacaacag tttttatgca tgcgcccaat 1560
   acgcaaaccg cctctccccg cgcgttggcc gattcattaa tgcagctggc acgacaggtt 1620
   tcccgactgg aaagcgggca gtgagcgcaa cgcaattaat gtgagttagc tcactcatta 1680
   ggcaccccag gctttacact ttatgcttcc ggctcgtatg ttgtgtggaa ttgtgagcgg 1740
   ataacaattt cacacaggaa acagctatga ccatgattac gccaagcgcg caattaaccc 1800
   tcactaaagg gaacaaaagc tgggtaccgg gccccccctc gaggtcgacg gtatcgataa 1860
   gcttgatatc gaattcctgc agcccggggg atctggcatt tttgggaggt gtgaaatgcg 1920
   gcgcgaaagt ctgttggtat cggtttgcaa gggcctgcgg gtacatgtcg agcgcgttgg 1980
   gcaggatccc gggcgcagca cggtgatgct ggtcaacggc gcgatggcga ccaccgcctc 2040
   gttcgcccgg acctgcaagt gcctggccga acatttcaac gtggtgctgt tcgacctgcc 2100
   cttcgccggg cagtcgcgtc agcacaaccc gcagcggggg ttgatcacca aggacgacga 2160
   ggtggaaatc ctcctggcgc tgatcgagcg cttcgaggtc aatcacctgg tctccgcgtc 2220
   ctggggcggt atctccacgc tgctggcgct gtcgcgcaat ccgcgcggca tccgcagctc 2280
   ggtggtgatg gcattcgccc ctggactgaa ccaggcgatg ctcgactacg tcgggcgggc 2340
   gcaggcgctg atcgagctgg acgacaagtc ggcgatcggc catctgctca acgagaccgt 2400
   cggcaaatac ctgccgccgc gcctgaaagc cagcaaccat cagcacatgg cttcgctggc 2460
   caccggcgaa tacgagcagg cgcgctttca catcgaccag gtgctggcgc tcaacgatcg 2520
   gggctacctg gcttgcctgg agcggatcca gagccacgtg catttcatca acggcagctg      2580
   ggacgaatac accaccgccg aggacgcccg ccagttccgc gactacctgc cgcactgcag      2640
   tttctcgcgg gtggagggca ccgggcattt cctcgacctg gagtccaagc tggccgcggt      2700
   acgcgtgcac cgcgccctgc tcgagcacct gctgaagcaa ccggagccgc agcgggcgga      2760
   acgcgcggcg ggattccacg agatggccat cggctacgcc tgaacccttg acctgcgaag      2820
   acccggcctg gccgggcttt gcggttgcat aacgcacgga gtagcaccat gcacgccatc      2880
   ctcatcgcca tcggctcggc cggcgacgta tttcccttca tcggcctggc ccggaccctg      2940
   aaattgcgcg ggcaccgcgt gagcctctgc accatcccgg tgtttcgcga cgcggtggag      3000
   cagcacggca tcgcgttcgt cccgctgagc gacgaactga cctaccgccg gaccatgggc      3060
   gatccgcgcc tgtgggaccc caagacgtcc ttcggcgtgc tctggcaaac catcgccggg      3120
   atgatcgagc cggtctacga gtacgtctcg gcgcagcgcc atgacgacat cgtggtggtc      3180
   ggctcgctct gggcgctggg cgcacgcatc gctcacgaga agtacgggat tccctacctg      3240
   tccgcgcagg tctcgccatc gaccttgttg tcggcgcacc tgccgccggt acaccccaag      3300
   ttcaacgtgc ccgagcagat gccgctggcg atgcgcaagc tgctctggcg ctgcatcgag      3360
   cgcttcaagc tggatcgcac ctgcgcgccg gatatcaacg cggtgcggcg caaggtcggc      3420
   ctggagacgc cggtgaagcg catcttcacc caatggatgc attcgccgca gggcgtggtc      3480
   tgcctgttcc cggcctggtt cgcgccgccc cagcaggatt ggccgcaacc cctgcacatg      3540
   accggcttcc cgctgttcga cggcagtatc ccggggaccc cgctcgacga cgaactgcaa      3600
   cgctttctcg atcagggcag ccggccgctg gtgttcaccc agggctcgac cgaacacctg      3660
   cagggcgact tctacgccat ggccctgcgc gcgctggaac gcctcggcgc gcgtgggatc      3720
   ttcctcaccg gcgccggcca ggaaccgctg cgcggcttgc cgaaccacgt gctgcagcgc      3780
   gcctacgcgc cactgggagc cttgctgcca tcgtgcgccg ggctggtcca tccgggcggt      3840
   atcggcgcca tgagcctggc cttggcggcg ggggtgccgc aggtgctgct gccctgcgcc     3900
   cacgaccagt tcgacaatgc cgaacggctg gtccggctcg gctgcgggat gcgcctgggc      3960
   gtgccattgc gcgagcagga gttgcgcggg gcgctgtggc gcttgctcga ggacccggcc      4020
   atggcggcgg cctgtcggcg tttcatggaa ttgtcacaac cgcacagtat cgcttgcggt      4080
   aaagcggccc aggtggtcga acgttgtcat agggaggggg atgcgcgatg gctgaaggct      4140
   gcgtcctgac ctacgggaga agaacgatca tggaccggat agacatgggc gtgctggtgg      4200
   tactgttcaa tcctggcgac gacgacctgg aacaccttgg cgaactggcg gcggcgtttc      4260
   cgcaactgcg cttccttgcc gtcgacaact caccgcacag cgatccgcag cgcaatgccc      4320
   ggctgcgcgg gcaaggcatc gccgtgctgc accacggcaa ccggcagggc atcgccggcg      4380
   ccttcaacca gggactcgac gcgctattcc ggcgtggcgt gcagggtgtg ctgctgctcg      4440
   accaggactc ccgtcccggc ggcgccttcc tcgccgccca gtggcgcaac ctgcaggcgc      4500
   gcaacggtca ggcctgcctg ctcggcccac ggatcttcga ccggggtgac cggcgcttcc 4560
   tgccggccat ccatctcgac ggactgacgc tcaggcaatt gtctctggac ggcctgacga 4620
   ccccgcagcg cacctcgttc ctgatctcct ccggctgcct gctgacccgc gaggcctacc 4680
   agcgcctcgg ccacttcgac gaggaactgt tcatcgacca cgtggacacc gaatacagcc 4740
   tgcgcgccca ggcgctggac gtgcccctgt acgtcgaccc gcggctggtc ctcgagcacc 4800
   gcatcggcac gcgcaagacc cgccgcctcg gcggtctcag cctcagcgcg atgaaccacg 4860
   ccccgctgcg ccgctactac ctggcgcgca acggcctgct ggtcctgcgc cgctacgccc 4920
   ggtcctcgcc gctggccctg ctggcgaacc tgccgaccct gacccagggc ctcgcggtgc 4980
   tcctgctcga acgcgacaag ctgctcaagc tgcgctgcct gggctggggc ctgtgggacg 5040
   gcctgcgggg acgcggcggc gcgctggaga ccaaccgccc gcgcctgctg aagcgcctcg 5100
   ccggcccggc cgtggcgtcc gtagcttccg gcaaggccaa ggcctagtcg gcgaaacgca 5160
   ttccctctag agcggccgcc accgcggtgg agctccaatt cgccctatag tgagtcgtat 5220
   tacgcgcgct cactggccgt cgttttacaa cgtcgtgact gggaaaaccc tggcgttacc 5280
   caacttaatc gccttgcagc acatccccct ttcgccagct ggcgtaatag cgaagaggcc 5340
   cgcaccgatc gcccttccca acagttgcgc agcctgaatg gcgaatggaa attgtaagcg 5400
   ttaatatttt gttaaaattc gcgttaaatt tttgttaaat cagctcattt tttaaccaat 5460
   aggccgactg cgatgagtgg cagggcgggg cgtaattttt ttaaggcagt tattggtgcc 5520
   cttaaacgcc tggtgctacg cctgaataag tgataataag cggatgaatg gcagaaattc 5580
   gaaagcaaat tcgacccggt cgtcggttca gggcagggtc gttaaatagc cgcttatgtc 5640
   tattgctggt ttaccggttt attgactacc ggaagcagtg tgaccgtgtg cttctcaaat 5700
   gcctgaggcc agtttgctca ggctctcccc gtggaggtaa taattgacga tatgatcatt 5760
   tattctgcct cccagagcct gataaaaacg gtgaatccgt tagcgaggtg ccgccggctt 5820
   ccattcaggt cgaggtggcc cggctccatg caccgcgacg caacgcgggg aggcagacaa 5880
   ggtatagggc ggcgaggcgg ctacagccga tagtctggaa cagcgcactt acgggttgct 5940
   gcgcaaccca agtgctaccg gcgcggcagc gtgacccgtg tcggcggctc caacggctcg 6000
   ccatcgtcca gaaaacacgg ctcatcgggc atcggcaggc gctgctgccc gcgccgttcc 6060
   cattcctccg tttcggtcaa ggctggcagg tctggttcca tgcccggaat gccgggctgg 6120
   ctgggcggct cctcgccggg gccggtcggt agttgctgct cgcccggata cagggtcggg 6180
   atgcggcgca ggtcgccatg ccccaacagc gattcgtcct ggtcgtcgtg atcaaccacc 6240
   acggcggcac tgaacaccga caggcgcaac tggtcgcggg gctggcccca cgccacgcgg 6300
   tcattgacca cgtaggccga cacggtgccg gggccgttga gcttcacgac ggagatccag 6360
   cgctcggcca ccaagtcctt gactgcgtat tggaccgtcc gcaaagaacg tccgatgagc 6420
   ttggaaagtg tcttctggct gaccaccacg gcgttctggt ggcccatctg cgccacgagg 6480
   tgatgcagca gcattgccgc cgtgggtttc ctcgcaataa gcccggccca cgcctcatgc 6540
   gctttgcgtt ccgtttgcac ccagtgaccg ggcttgttct tggcttgaat gccgatttct 6600
   ctggactgcg tggccatgct tatctccatg cggtagggtg ccgcacggtt gcggcaccat 6660
   gcgcaatcag ctgcaacttt tcggcagcgc gacaacaatt atgcgttgcg taaaagtggc 6720
   agtcaattac agattttctt taacctacgc aatgagctat tgcggggggt gccgcaatga 6780
   gctgttgcgt accccccttt tttaagttgt tgatttttaa gtctttcgca tttcgcccta 6840
   tatctagttc tttggtgccc aaagaagggc acccctgcgg ggttccccca cgccttcggc 6900
   gcggctcccc ctccggcaaa aagtggcccc tccggggctt gttgatcgac tgcgcggcct 6960
   tcggccttgc ccaaggtggc gctgccccct tggaaccccc gcactcgccg ccgtgaggct 7020
   cggggggcag gcgggcgggc ttcgccttcg actgccccca ctcgcatagg cttgggtcgt 7080
   tccaggcgcg tcaaggccaa gccgctgcgc ggtcgctgcg cgagccttga cccgccttcc 7140
   acttggtgtc caaccggcaa gcgaagcgcg caggccgcag gccggaggct tttccccaga 7200
   gaaaattaaa aaaattgatg gggcaaggcc gcaggccgcg cagttggagc cggtgggtat 7260
   gtggtcgaag gctgggtagc cggtgggcaa tccctgtggt caagctcgtg ggcaggcgca 7320
   gcctgtccat cagcttgtcc agcagggttg tccacgggcc gagcgaagcg agccagccgg 7380
   tggccgctcg cggccatcgt ccacatatcc acgggctggc aagggagcgc agcgaccgcg 7440
   cagggcgaag cccggagagc aagcccgtag ggcgccgcag ccgccgtagg cggtcacgac 7500
   tttgcgaagc aaagtctagt gagtatactc aagcattgag tggcccgccg gaggcaccgc 7560
   cttgcgctgc ccccgtcgag ccggttggac accaaaaggg aggggcaggc atggcggcat 7620
   acgcgatcat gcgatgcaag aagctggcga aaatgggcaa cgtggcggcc agtctcaagc 7680
   acgcctaccg cgagcgcgag acgcccaacg ctgacgccag caggacgcca gagaacgagc 7740
   actgggcggc cagcagcacc gatgaagcga tgggccgact gcgcgagttg ctgccagaga 7800
   agcggcgcaa ggacgctgtg ttggcggtcg agtacgtcat gacggccagc ccggaatggt 7860
   ggaagtcggc cagccaagaa cagcaggcgg cgttcttcga gaaggcgcac aagtggctgg 7920
   cggacaagta cggggcggat cgcatcgtga cggccagcat ccaccgtgac gaaaccagcc 7980
   cgcacatgac cgcgttcgtg gtgccgctga cgcaggacgg caggctgtcg gccaaggagt 8040
   tcatcggcaa caaagcgcag atgacccgcg accagaccac gtttgcggcc gctgtggccg 8100
   atctagggct gcaacggggc atcgagggca gcaaggcacg tcacacgcgc attcaggcgt 8160
   tctacgaggc cctggagcgg ccaccagtgg gccacgtcac catcagcccg caagcggtcg 8220
   agccacgcgc ctatgcaccg cagggattgg ccgaaaagct gggaatctca aagcgcgttg 8280
   agacgccgga agccgtggcc gaccggctga caaaagcggt tcggcagggg tatgagcctg 8340
   ccctacaggc cgccgcagga gcgcgtgaga tgcgcaagaa ggccgatcaa gcccaagaga 8400
   cggcccgag 8409
<210> 41
   <211> 3590
   <212> DNA
   <213> Pseudomonas aeruginosa
<400> 41
   aggtaccaga tctggcattt ttgggaggtg tgaaatgcgg cgcgaaagtc tgttggtatc 60
   ggtttgcaag ggcctgcggg tacatgtcga gcgcgttggg caggatcccg ggcgcagcac 120
   ggtgatgctg gtcaacggcg cgatggcgac caccgcctcg ttcgcccgga cctgcaagtg 180
   cctggccgaa catttcaacg tggtgctgtt cgacctgccc ttcgccgggc agtcgcgtca 240
   gcacaacccg cagcgggggt tgatcaccaa ggacgacgag gtggaaatcc tcctggcgct 300
   gatcgagcgc ttcgaggtca atcacctggt ctccgcgtcc tggggcggta tctccacgct 360
   gctggcgctg tcgcgcaatc cgcgcggcat ccgcagctcg gtggtgatgg cattcgcccc 420
   tggactgaac caggcgatgc tcgactacgt cgggcgggcg caggcgctga tcgagctgga 480
   cgacaagtcg gcgatcggcc atctgctcaa cgagaccgtc ggcaaatacc tgccgccgcg 540
   cctgaaagcc agcaaccatc agcacatggc ttcgctggcc accggcgaat acgagcaggc 600
   gcgctttcac atcgaccagg tgctggcgct caacgatcgg ggctacctgg cttgcctgga 660
   gcggatccag agccacgtgc atttcatcaa cggcagctgg gacgaataca ccaccgccga 720
   ggacgcccgc cagttccgcg actacctgcc gcactgcagt ttctcgcggg tggagggcac 780
   cgggcatttc ctcgacctgg agtccaagct ggccgcggta cgcgtgcacc gcgccctgct 840
   cgagcacctg ctgaagcaac cggagccgca gcgggcggaa cgcgcggcgg gattccacga 900
   gatggccatc ggctacgcct gaacccttga cctgcgaaga cccggcctgg ccgggctttg 960
   cggttgcata acgcacggag tagcaccatg cacgccatcc tcatcgccat cggctcggcc 1020
   ggcgacgtat ttcccttcat cggcctggcc cggaccctga aattgcgcgg gcaccgcgtg 1080
   agcctctgca ccatcccggt gtttcgcgac gcggtggagc agcacggcat cgcgttcgtc 1140
   ccgctgagcg acgaactgac ctaccgccgg accatgggcg atccgcgcct gtgggacccc 1200
   aagacgtcct tcggcgtgct ctggcaaacc atcgccggga tgatcgagcc ggtctacgag 1260
   tacgtctcgg cgcagcgcca tgacgacatc gtggtggtcg gctcgctctg ggcgctgggc 1320
   gcacgcatcg ctcacgagaa gtacgggatt ccctacctgt ccgcgcaggt ctcgccatcg 1380
   accttgttgt cggcgcacct gccgccggta caccccaagt tcaacgtgcc cgagcagatg 1440
   ccgctggcga tgcgcaagct gctctggcgc tgcatcgagc gcttcaagct ggatcgcacc 1500
   tgcgcgccgg atatcaacgc ggtgcggcgc aaggtcggcc tggagacgcc ggtgaagcgc 1560
   atcttcaccc aatggatgca ttcgccgcag ggcgtggtct gcctgttccc ggcctggttc 1620
   gcgccgcccc agcaggattg gccgcaaccc ctgcacatga ccggcttccc gctgttcgac 1680
   ggcagtatcc cggggacccc gctcgacgac gaactgcaac gctttctcga tcagggcagc 1740
   cggccgctgg tgttcaccca gggctcgacc gaacacctgc agggcgactt ctacgccatg 1800
   gccctgcgcg cgctggaacg cctcggcgcg cgtgggatct tcctcaccgg cgccggccag 1860
   gaaccgctgc gcggcttgcc gaaccacgtg ctgcagcgcg cctacgcgcc actgggagcc 1920
   ttgctgccat cgtgcgccgg gctggtccat ccgggcggta tcggcgccat gagcctggcc 1980
   ttggcggcgg gggtgccgca ggtgctgctg ccctgcgccc acgaccagtt cgacaatgcc 2040
   gaacggctgg tccggctcgg ctgcgggatg cgcctgggcg tgccattgcg cgagcaggag 2100
   ttgcgcgggg cgctgtggcg cttgctcgag gacccggcca tggcggcggc ctgtcggcgt 2160
   ttcatggaat tgtcacaacc gcacagtatc gcttgcggta aagcggccca ggtggtcgaa 2220
   cgttgtcata gggaggggga tgcgcgatgg ctgaaggctg cgtcctgacg ccgggaggat 2280
   cctggcgtgt ccacgaccag cctctgcccc tccgccacgc gggaacacgg tcccggcgcg 2340
   aaacgcgtcc tgcctctgct gttcctcacc tgcctgctgg atgccgctgg cgtcggcctg 2400
   atcgtgcccc tgctgccgac gctgatcggc agcgtggcgc cgctggcggt ccgcgacgcg 2460
   gccacctggg gcgccgccct ggtgatgacc ttcgcgctgc tgcaattgtt cttttcgccg 2520
   gtcctcggca gcctcagcga ccgcttcgga cgccgccccg tcctggtcct ggcgatgctc 2580
   ggcttcgccc tcagctatct gctgctggcg ctggccgaca gcctctggat gctgttcctc 2640
   ggtcgcgcgc tggccgggct caccggcgcc agcgtggcca ccgcgatggc ctgcgcggct 2700
   gacctcggca cgcacgggca gcgcacccgg cacttcggct ggctgtacgc cggcctcgcc 2760
   ctgggcatga tcctcggccc cgccctcggt gggctgctgg cggtgcacgg cacgacgctg 2820
   ccgctgttgc tggccgccgg cctgtgcctg ctcaacgccc tgctcgccgg cctgttcctc 2880
   gaggaaaccc tgcccccgac gcgacgccgc cgcctggacc cgaggcggat gaatgccttg 2940
   cgctcgatca gcggcctggc tcggcaaccg ggggtcggac gcctgctggc ggtgcttgcc 3000
   ctggtattcc tcggcttgca ggcggtgatg gtggtctggc cgttcttcgt gatcgagaag 3060
   tttcactgga gcagcgcctg gatcggctac tcgctggccc tctacggcgt gctcgcggtg 3120
   ctcgcccaga ccctcggcgt gaacctctgc aagcggcgcc tggacgacgc ccgcctgctg 3180
   cgcctgggcc tcgccctgca aggctgcggc ctgctgctgt tcgccctggt cgactcgtca 3240
   ttctggctgg tctgcgcgct gctgcccttc gcgctcggca gcctcgccac cccggccatg 3300
   caggggctgc tctcggcccg cgtgccggtc gaccgccagg gcgagttgca gggcgtgctg 3360
   agcagcctga tgagcctcgc cgcgatcgtc ggtccgccgc tgatgagcgg cctgttccac 3420
   tggggcagcg gtccgctcgc gccgctgccc ctggccggcg cgccattcct cgccggcgcc 3480
   cttctcgttc tggccgggct ggtcctggcc tggcaacttc gacctacggg agaagaacga 3540
   tcatggaccg gatagacatg ggcgtgctgg tggtacttct agagagctca 3590
<210> 42
   <211> 8702
   <212> DNA
   <213> Artificial
<220>
   <223> Vector
<400> 42
   accttcggga gcgcctgaag cccgttctgg acgccctggg gccgttgaat cgggatatgc 60
   aggccaaggc cgccgcgatc atcaaggccg tgggcgaaaa gctgctgacg gaacagcggg 120
   aagtccagcg ccagaaacag gcccagcgcc agcaggaacg cgggcgcgca catttccccg 180
   aaaagtgcca cctgggatga atgtcagcta ctgggctatc tggacaaggg aaaacgcaag 240
   cgcaaagaga aagcaggtag cttgcagtgg gcttacatgg cgatagctag actgggcggt 300
   tttatggaca gcaagcgaac cggaattgcc agctggggcg ccctctggta aggttgggaa 360
   gccctgcaaa gtaaactgga tggctttctt gccgccaagg atctgatggc gcaggggatc 420
   aagatctgat caagagacag gatgaggatc gtttcgcatg attgaacaag atggattgca 480
   cgcaggttct ccggccgctt gggtggagag gctattcggc tatgactggg cacaacagac 540
   aatcggctgc tctgatgccg ccgtgttccg gctgtcagcg caggggcgcc cggttctttt 600
   tgtcaagacc gacctgtccg gtgccctgaa tgaactgcag gacgaggcag cgcggctatc 660
   gtggctggcc acgacgggcg ttccttgcgc agctgtgctc gacgttgtca ctgaagcggg 720
   aagggactgg ctgctattgg gcgaagtgcc ggggcaggat ctcctgtcat ctcaccttgc 780
   tcctgccgag aaagtatcca tcatggctga tgcaatgcgg cggctgcata cgcttgatcc 840
   ggctacctgc ccattcgacc accaagcgaa acatcgcatc gagcgagcac gtactcggat 900
   ggaagccggt cttgtcgatc aggatgatct ggacgaagag catcaggggc tcgcgccagc 960
   cgaactgttc gccaggctca aggcgcgcat gcccgacggc gaggatctcg tcgtgaccca 1020
   tggcgatgcc tgcttgccga atatcatggt ggaaaatggc cgcttttctg gattcatcga 1080
   ctgtggccgg ctgggtgtgg cggaccgcta tcaggacata gcgttggcta cccgtgatat 1140
   tgctgaagag cttggcggcg aatgggctga ccgcttcctc gtgctttacg gtatcgccgc 1200
   tcccgattcg cagcgcatcg ccttctatcg ccttcttgac gagttcttct gagcgggact 1260
   ctggggttcg aaatgaccga ccaagcgacg cccaacctgc catcacgaga tttcgattcc 1320
   accgccgcct tctatgaaag gttgggcttc ggaatcgttt tccgggacgc cggctggatg 1380
   atcctccagc gcggggatct catgctggag ttcttcgccc acccccatgg gcaaatatta 1440
   tacgcaaggc gacaaggtgc tgatgccgct ggcgattcag gttcatcatg ccgtttgtga 1500
   tggcttccat gtcggcagaa tgcttaatga attacaacag tttttatgca tgcgcccaat 1560
   acgcaaaccg cctctccccg cgcgttggcc gattcattaa tgcagctggc acgacaggtt 1620
   tcccgactgg aaagcgggca gtgagcgcaa cgcaattaat gtgagttagc tcactcatta 1680
   ggcaccccag gctttacact ttatgcttcc ggctcgtatg ttgtgtggaa ttgtgagcgg 1740
   ataacaattt cacacaggaa acagctatga ccatgattac gccaagcgcg caattaaccc 1800
   tcactaaagg gaacaaaagc tgggtaccgg gccccccctc gaggtcgacg gtatcgataa 1860
   gcttgatatc gaattcctgc agcccggggg atctggcatt tttgggaggt gtgaaatgcg 1920
   gcgcgaaagt ctgttggtat cggtttgcaa gggcctgcgg gtacatgtcg agcgcgttgg 1980
   gcaggatccc gggcgcagca cggtgatgct ggtcaacggc gcgatggcga ccaccgcctc 2040
   gttcgcccgg acctgcaagt gcctggccga acatttcaac gtggtgctgt tcgacctgcc 2100
   cttcgccggg cagtcgcgtc agcacaaccc gcagcggggg ttgatcacca aggacgacga 2160
   ggtggaaatc ctcctggcgc tgatcgagcg cttcgaggtc aatcacctgg tctccgcgtc 2220
   ctggggcggt atctccacgc tgctggcgct gtcgcgcaat ccgcgcggca tccgcagctc 2280
   ggtggtgatg gcattcgccc ctggactgaa ccaggcgatg ctcgactacg tcgggcgggc 2340
   gcaggcgctg atcgagctgg acgacaagtc ggcgatcggc catctgctca acgagaccgt 2400
   cggcaaatac ctgccgccgc gcctgaaagc cagcaaccat cagcacatgg cttcgctggc 2460
   caccggcgaa tacgagcagg cgcgctttca catcgaccag gtgctggcgc tcaacgatcg 2520
   gggctacctg gcttgcctgg agcggatcca gagccacgtg catttcatca acggcagctg 2580
   ggacgaatac accaccgccg aggacgcccg ccagttccgc gactacctgc cgcactgcag 2640
   tttctcgcgg gtggagggca ccgggcattt cctcgacctg gagtccaagc tggccgcggt 2700
   acgcgtgcac cgcgccctgc tcgagcacct gctgaagcaa ccggagccgc agcgggcgga 2760
   acgcgcggcg ggattccacg agatggccat cggctacgcc tgaacccttg acctgcgaag 2820
   acccggcctg gccgggcttt gcggttgcat aacgcacgga gtagcaccat gcacgccatc 2880
   ctcatcgcca tcggctcggc cggcgacgta tttcccttca tcggcctggc ccggaccctg 2940
   aaattgcgcg ggcaccgcgt gagcctctgc accatcccgg tgtttcgcga cgcggtggag 3000
   cagcacggca tcgcgttcgt cccgctgagc gacgaactga cctaccgccg gaccatgggc 3060
   gatccgcgcc tgtgggaccc caagacgtcc ttcggcgtgc tctggcaaac catcgccggg 3120
   atgatcgagc cggtctacga gtacgtctcg gcgcagcgcc atgacgacat cgtggtggtc 3180
   ggctcgctct gggcgctggg cgcacgcatc gctcacgaga agtacgggat tccctacctg 3240
   tccgcgcagg tctcgccatc gaccttgttg tcggcgcacc tgccgccggt acaccccaag 3300
   ttcaacgtgc ccgagcagat gccgctggcg atgcgcaagc tgctctggcg ctgcatcgag 3360
   cgcttcaagc tggatcgcac ctgcgcgccg gatatcaacg cggtgcggcg caaggtcggc 3420
   ctggagacgc cggtgaagcg catcttcacc caatggatgc attcgccgca gggcgtggtc 3480
   tgcctgttcc cggcctggtt cgcgccgccc cagcaggatt ggccgcaacc cctgcacatg 3540
   accggcttcc cgctgttcga cggcagtatc ccggggaccc cgctcgacga cgaactgcaa 3600
   cgctttctcg atcagggcag ccggccgctg gtgttcaccc agggctcgac cgaacacctg 3660
   cagggcgact tctacgccat ggccctgcgc gcgctggaac gcctcggcgc gcgtgggatc 3720
   ttcctcaccg gcgccggcca ggaaccgctg cgcggcttgc cgaaccacgt gctgcagcgc 3780
   gcctacgcgc cactgggagc cttgctgcca tcgtgcgccg ggctggtcca tccgggcggt 3840
   atcggcgcca tgagcctggc cttggcggcg ggggtgccgc aggtgctgct gccctgcgcc 3900
   cacgaccagt tcgacaatgc cgaacggctg gtccggctcg gctgcgggat gcgcctgggc 3960
   gtgccattgc gcgagcagga gttgcgcggg gcgctgtggc gcttgctcga ggacccggcc 4020
   atggcggcgg cctgtcggcg tttcatggaa ttgtcacaac cgcacagtat cgcttgcggt 4080
   aaagcggccc aggtggtcga acgttgtcat agggaggggg atgcgcgatg gctgaaggct 4140
   gcgtcctgac gccgggagga tcctggcgtg tccacgacca gcctctgccc ctccgccacg 4200
   cgggaacacg gtcccggcgc gaaacgcgtc ctgcctctgc tgttcctcac ctgcctgctg 4260
   gatgccgctg gcgtcggcct gatcgtgccc ctgctgccga cgctgatcgg cagcgtggcg 4320
   ccgctggcgg tccgcgacgc ggccacctgg ggcgccgccc tggtgatgac cttcgcgctg 4380
   ctgcaattgt tcttttcgcc ggtcctcggc agcctcagcg accgcttcgg acgccgcccc 4440
   gtcctggtcc tggcgatgct cggcttcgcc ctcagctatc tgctgctggc gctggccgac 4500
   agcctctgga tgctgttcct cggtcgcgcg ctggccgggc tcaccggcgc cagcgtggcc 4560
   accgcgatgg cctgcgcggc tgacctcggc acgcacgggc agcgcacccg gcacttcggc 4620
   tggctgtacg ccggcctcgc cctgggcatg atcctcggcc ccgccctcgg tgggctgctg 4680
   gcggtgcacg gcacgacgct gccgctgttg ctggccgccg gcctgtgcct gctcaacgcc 4740
   ctgctcgccg gcctgttcct cgaggaaacc ctgcccccga cgcgacgccg ccgcctggac 4800
   ccgaggcgga tgaatgcctt gcgctcgatc agcggcctgg ctcggcaacc gggggtcgga 4860
   cgcctgctgg cggtgcttgc cctggtattc ctcggcttgc aggcggtgat ggtggtctgg 4920
   ccgttcttcg tgatcgagaa gtttcactgg agcagcgcct ggatcggcta ctcgctggcc 4980
   ctctacggcg tgctcgcggt gctcgcccag accctcggcg tgaacctctg caagcggcgc 5040
   ctggacgacg cccgcctgct gcgcctgggc ctcgccctgc aaggctgcgg cctgctgctg 5100
   ttcgccctgg tcgactcgtc attctggctg gtctgcgcgc tgctgccctt cgcgctcggc 5160
   agcctcgcca ccccggccat gcaggggctg ctctcggccc gcgtgccggt cgaccgccag 5220
   ggcgagttgc agggcgtgct gagcagcctg atgagcctcg ccgcgatcgt cggtccgccg 5280
   ctgatgagcg gcctgttcca ctggggcagc ggtccgctcg cgccgctgcc cctggccggc 5340
   gcgccattcc tcgccggcgc ccttctcgtt ctggccgggc tggtcctggc ctggcaactt 5400
   cgacctacgg gagaagaacg atcatggacc ggatagacat gggcgtgctg gtggtacttc 5460
   tagagcggcc gccaccgcgg tggagctcca attcgcccta tagtgagtcg tattacgcgc 5520
   gctcactggc cgtcgtttta caacgtcgtg actgggaaaa ccctggcgtt acccaactta 5580
   atcgccttgc agcacatccc cctttcgcca gctggcgtaa tagcgaagag gcccgcaccg 5640
   atcgcccttc ccaacagttg cgcagcctga atggcgaatg gaaattgtaa gcgttaatat 5700
   tttgttaaaa ttcgcgttaa atttttgtta aatcagctca ttttttaacc aataggccga 5760
   ctgcgatgag tggcagggcg gggcgtaatt tttttaaggc agttattggt gcccttaaac 5820
   gcctggtgct acgcctgaat aagtgataat aagcggatga atggcagaaa ttcgaaagca 5880
   aattcgaccc ggtcgtcggt tcagggcagg gtcgttaaat agccgcttat gtctattgct 5940
   ggtttaccgg tttattgact accggaagca gtgtgaccgt gtgcttctca aatgcctgag 6000
   gccagtttgc tcaggctctc cccgtggagg taataattga cgatatgatc atttattctg 6060
   cctcccagag cctgataaaa acggtgaatc cgttagcgag gtgccgccgg cttccattca 6120
   ggtcgaggtg gcccggctcc atgcaccgcg acgcaacgcg gggaggcaga caaggtatag 6180
   ggcggcgagg cggctacagc cgatagtctg gaacagcgca cttacgggtt gctgcgcaac 6240
   ccaagtgcta ccggcgcggc agcgtgaccc gtgtcggcgg ctccaacggc tcgccatcgt 6300
   ccagaaaaca cggctcatcg ggcatcggca ggcgctgctg cccgcgccgt tcccattcct 6360
   ccgtttcggt caaggctggc aggtctggtt ccatgcccgg aatgccgggc tggctgggcg 6420
   gctcctcgcc ggggccggtc ggtagttgct gctcgcccgg atacagggtc gggatgcggc 6480
   gcaggtcgcc atgccccaac agcgattcgt cctggtcgtc gtgatcaacc accacggcgg 6540
   cactgaacac cgacaggcgc aactggtcgc ggggctggcc ccacgccacg cggtcattga 6600
   ccacgtaggc cgacacggtg ccggggccgt tgagcttcac gacggagatc cagcgctcgg 6660
   ccaccaagtc cttgactgcg tattggaccg tccgcaaaga acgtccgatg agcttggaaa 6720
   gtgtcttctg gctgaccacc acggcgttct ggtggcccat ctgcgccacg aggtgatgca 6780
   gcagcattgc cgccgtgggt ttcctcgcaa taagcccggc ccacgcctca tgcgctttgc 6840
   gttccgtttg cacccagtga ccgggcttgt tcttggcttg aatgccgatt tctctggact 6900
   gcgtggccat gcttatctcc atgcggtagg gtgccgcacg gttgcggcac catgcgcaat 6960
   cagctgcaac ttttcggcag cgcgacaaca attatgcgtt gcgtaaaagt ggcagtcaat 7020
   tacagatttt ctttaaccta cgcaatgagc tattgcgggg ggtgccgcaa tgagctgttg 7080
   cgtacccccc ttttttaagt tgttgatttt taagtctttc gcatttcgcc ctatatctag 7140
   ttctttggtg cccaaagaag ggcacccctg cggggttccc ccacgccttc ggcgcggctc 7200
   cccctccggc aaaaagtggc ccctccgggg cttgttgatc gactgcgcgg ccttcggcct 7260
   tgcccaaggt ggcgctgccc ccttggaacc cccgcactcg ccgccgtgag gctcgggggg 7320
   caggcgggcg ggcttcgcct tcgactgccc ccactcgcat aggcttgggt cgttccaggc 7380
   gcgtcaaggc caagccgctg cgcggtcgct gcgcgagcct tgacccgcct tccacttggt 7440
   gtccaaccgg caagcgaagc gcgcaggccg caggccggag gcttttcccc agagaaaatt 7500
   aaaaaaattg atggggcaag gccgcaggcc gcgcagttgg agccggtggg tatgtggtcg 7560
   aaggctgggt agccggtggg caatccctgt ggtcaagctc gtgggcaggc gcagcctgtc 7620
   catcagcttg tccagcaggg ttgtccacgg gccgagcgaa gcgagccagc cggtggccgc 7680
   tcgcggccat cgtccacata tccacgggct ggcaagggag cgcagcgacc gcgcagggcg 7740
   aagcccggag agcaagcccg tagggcgccg cagccgccgt aggcggtcac gactttgcga 7800
   agcaaagtct agtgagtata ctcaagcatt gagtggcccg ccggaggcac cgccttgcgc 7860
   tgcccccgtc gagccggttg gacaccaaaa gggaggggca ggcatggcgg catacgcgat 7920
   catgcgatgc aagaagctgg cgaaaatggg caacgtggcg gccagtctca agcacgccta 7980
   ccgcgagcgc gagacgccca acgctgacgc cagcaggacg ccagagaacg agcactgggc 8040
   ggccagcagc accgatgaag cgatgggccg actgcgcgag ttgctgccag agaagcggcg 8100
   caaggacgct gtgttggcgg tcgagtacgt catgacggcc agcccggaat ggtggaagtc 8160
   ggccagccaa gaacagcagg cggcgttctt cgagaaggcg cacaagtggc tggcggacaa 8220
   gtacggggcg gatcgcatcg tgacggccag catccaccgt gacgaaacca gcccgcacat 8280
   gaccgcgttc gtggtgccgc tgacgcagga cggcaggctg tcggccaagg agttcatcgg 8340
   caacaaagcg cagatgaccc gcgaccagac cacgtttgcg gccgctgtgg ccgatctagg 8400
   gctgcaacgg ggcatcgagg gcagcaaggc acgtcacacg cgcattcagg cgttctacga 8460
   ggccctggag cggccaccag tgggccacgt caccatcagc ccgcaagcgg tcgagccacg 8520
   cgcctatgca ccgcagggat tggccgaaaa gctgggaatc tcaaagcgcg ttgagacgcc 8580
   ggaagccgtg gccgaccggc tgacaaaagc ggttcggcag gggtatgagc ctgccctaca 8640
   ggccgccgca ggagcgcgtg agatgcgcaa gaaggccgat caagcccaag agacggcccg 8700
   ag 8702
<210> 43
   <211> 36
   <212> DNA
   <213> Artificial
<220>
   <223> Primer
<400> 43
   tatatataga attcggctgc gctaccgcag cccttc 36
<210> 44
   <211> 34
   <212> DNA
   <213> Artificial
<220>
   <223> Primer
<400> 44
   tatatatatc tagaattaat gcagctggca cgac 34
<210> 45
   <211> 8672
   <212> DNA
   <213> Artificial
<220>
   <223> Vector
<400> 45
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagaactagt ggatcccccg ggctgcagga attcggctgc gctaccgcag cccttctccg 3300
   ctaaaaccgt tagtcgaaca gttcggcgtc agccaatgcg accccaagct ggtccttgcc 3360
   agacaagctt ggtacggagt cgatgggcca ttcgataccc actgccggat cattccaggc 3420
   aatgcagcgc tcgcactgcg gcgagtagaa gtcggtggtc ttgtagagga actctgcggt 3480
   ttcactcaac gtgacgaacc cgtgtgcgaa ccctggcggg atccacagct ggttcttgtt 3540
   ctcggccgac aacaccgcac ctacccattt accgaaggtt gtggacgagc gacggatatc 3600
   caccgcaaca tcgaagactt cgccttgcac cacacgcacc agcttgccct gggcgtgagg 3660
   tgccagctga tagtgcaggc cacggagcac gccttttacc gagcgcgagt ggttgtcttg 3720
   tacgaagtcg ggctgcaggc cggtcacttc gctgaaaaca cgggcgttga agctctcgta 3780
   gaagaaacca cgttcgtcgc caaaaacctt gggggtaaac agcacgactt cggggatatc 3840
   cagcggaatg gcttgcatca gaacaccttc tctttcagca agttctgcag atacttgcca 3900
   taaccgtttt tcagcagtgg ttgagccagg cactcgagtt gctcagcgtt gatccagcca 3960
   gcgcggtagc aaatttcctc agggcaggcg actttcaagc cctgacggcg ctccatggtt 4020
   gcgatgtact ggctagcctc cagcagactg tcgtgcgtgc cggtgtcgag ccacgcatag 4080
   ccacggccca tgatttcgac ctgcaactgc tgctgctgca agtaaaggtt gttgaggtcg 4140
   gtgatttcca gctcgccacg tggggaaggc ttcagctcgc gagccagatt gactacctga 4200
   ttgtcataga aatacaggcc ggtgaccgca tagctagact ttggaactgc cggtttttct 4260
   tccagcgaca atacgcgacc gctatcgtca aactccgcta cgccatagcg ttctgggtca 4320
   tgaacatgat aagcgaatac tgaagcaccg gattcacgtt tatctgcgtt caatagcagt 4380
   gcctggaagt catggccgta gaaaatattg tcaccgagaa ccaacgcaga agggtcgtta 4440
   ccgatgaagt cagcgccgat ggtgaacgct tgcgccaagc catccgggct tggttgtatt 4500
   gcgtatgaca ggttcaggcc ccactggctg ccatcgccca gcagctgttc gaagcgcggg 4560
   gtgtcctgcg gggtggaaat gatcaggatg tcccggatac cagcgagcag cagggtgctc 4620
   agcgggtagt agatcatcgg tttgtcatac accggcagca gctgcttcga aaccgaaagt 4680
   gtggccggat gcaggcgtgt acccgaaccg ccggccagaa taattccttt acgagccatg 4740
   agagtcccta ttactggatt tcgtccagca tacgttgcac gccttgctcc caaagcggca 4800
   ttttgaaatt gaacgtgttt tccagtttgc ccagtgccag gcgcgagttg cgcggacgtg 4860
   gtgcaggtac tggataagct tcggtgctga ttgcggcaac cttatcagct gtcactttca 4920
   gcgctacgcc agtgcgttga gcatgcgcca gcacgaactg agcaaaacca tgccaagagg 4980
   tttcaccgga cgcagccaag tggtaaatcc ctgccaggtg acggttgtct tgcccattga 5040
   agatttgccg caggatgtgt gcagtaacgt cggcgatcag gtcagcgccc gtgggtgcgc 5100
   caaattggtc tgctaccacg ctcaacgtct cacgctccgc cgccaggcgc agcatggtct 5160
   tggcaaaatt gtgcccgcgc gcagcataca cccagctggt gcgcagtacc acggccttgg 5220
   cgccgctggc gagaatggca tgctcgcctt ccagcttggt ccggccgtag accgaaaggg 5280
   ggccggtagg cgcagtttcc tcccagcgct gactgccgct gccgtcaaat acataatcgg 5340
   tggaatagtg aatcaaccag gcgcccaaag ctgctgtttc acgtgctaat acagcaggag 5400
   ccgcggcatt gatcattgca gccagtgcct gatcgctctc agctttatcc actgcagtgt 5460
   aggcagcagc gttgacgatc acgtccggcg ccagctgacg aatcgtagcg gccaagccgt 5520
   ccaggttgga caagtcgcca cataagccct cggccccctg acgatccagc gcaatgacct 5580
   cacccagcgg cgccaaggcg cgctgtagct cccagcctac ttgcccgttt ttccccaaca 5640
   gcaggatttt cacgctttat ttgccccgta ttgttgtgcc acccagtcac ggtagctgcc 5700
   gtccatgaca ccttttaccc atttctggtt ggccaagtac caagcgactg tctttcgaat 5760
   gcccgtctcg aaggtttcgg cagtttccag ccgagctccc gctcgatctt gcgtgcatcg 5820
   atggcataac ggcggtcatg gcctgggcgg tcggttacgt aggcgatgag ttctgcatac 5880
   tgttcgacag gctcgccggt cttctgattg attacctggc gcgatgccgc aggtgccatc 5940
   tcgtcgagaa ggctgcagag tgtacgcaca atgtcaatgt tggctttttc attccagccg 6000
   ccaatattgt acgtctcgcc gaacgcaccg gcttccagta cgcgacggat gcccgagcag 6060
   tgatcttcga catacagcca gtcgcggatt tgctggccgt cgccatagac aggcagcgcc 6120
   ttaccggcga gtgcgttgac gatcatcagc gggatcagtt tttccgggaa gtggagcggc 6180
   ccgtaattgt tggagcagtt ggtagtgagt accggcatgc cgtaggtatg gaaatacgag 6240
   cgtaccagat ggtcgctggc tgccttgctg gcggagtatg ggctgttcgg cgcgtacggc 6300
   gtggtttcgg tgaacgccgg gtcgtttggc cctagtgtgc cgtagacttc gtcggtagag 6360
   acatggagga aacggaaggc ctccttctct gcaccttcca aactattcca atgcgcccgg 6420
   gcggcttcaa gcaagcgaaa cgtgcccatc acgttggttt cgacaaacgc ttcggggccg 6480
   gtgattgagc ggtctacatg ggattccgcc gcgaagtgaa ccacggcgcg cgggcggtgc 6540
   tctgcgaaca gcttggtcag aagcgcagca tcgcaaatat tgccttgcac aaagcgatgc 6600
   tgagggttgc cttccagcgg ctgcaggttg gccaggttgc ctgcgtaggt cagggcgtcg 6660
   aggttgagga cgggttcctc attgtgcgca caccattgca gtacgaaatt tgagccgatg 6720
   aagccggctc cgcctgttac tagaatcata atttggctct aattggacaa aaggtgttgt 6780
   cgtagacaga tgacgcgaat tcgatatcaa gcttatcgat accgtcgacc tcgagggggg 6840
   gcccggtacc cagcttttgt tccctttagt gagggttaat tgcgcgcttg gcgtaatcat 6900
   ggtcatagct gtttcctgtg tgaaattgtt atccgctcac aattccacac aacatacgag 6960
   ccggaagcat aaagtgtaaa gcctggggtg cctaatgagt gagctaactc acattaattg 7020
   cgttgcgctc actgcccgct ttccagtcgg gaaacctgtc gtgccagctg cattaatgaa 7080
   tcggccaacg cgcggggaga ggcggtttgc gtattgggcg catgcataaa aactgttgta 7140
   attcattaag cattctgccg acatggaagc catcacaaac ggcatgatga acctgaatcg 7200
   ccagcggcat cagcaccttg tcgccttgcg tataatattt gcccatgggg gtgggcgaag 7260
   aactccagca tgagatcccc gcgctggagg atcatccagc cggcgtcccg gaaaacgatt 7320
   ccgaagccca acctttcata gaaggcggcg gtggaatcga aatctcgtga tggcaggttg 7380
   ggcgtcgctt ggtcggtcat ttcgaacccc agagtcccgc tcagaagaac tcgtcaagaa 7440
   ggcgatagaa ggcgatgcgc tgcgaatcgg gagcggcgat accgtaaagc acgaggaagc 7500
   ggtcagccca ttcgccgcca agctcttcag caatatcacg ggtagccaac gctatgtcct      7560
   gatagcggtc cgccacaccc agccggccac agtcgatgaa tccagaaaag cggccatttt      7620
   ccaccatgat attcggcaag caggcatcgc catgggtcac gacgagatcc tcgccgtcgg      7680
   gcatgcgcgc cttgagcctg gcgaacagtt cggctggcgc gagcccctga tgctcttcgt      7740
   ccagatcatc ctgatcgaca agaccggctt ccatccgagt acgtgctcgc tcgatgcgat      7800
   gtttcgcttg gtggtcgaat gggcaggtag ccggatcaag cgtatgcagc cgccgcattg      7860
   catcagccat gatggatact ttctcggcag gagcaaggtg agatgacagg agatcctgcc      7920
   ccggcacttc gcccaatagc agccagtccc ttcccgcttc agtgacaacg tcgagcacag      7980
   ctgcgcaagg aacgcccgtc gtggccagcc acgatagccg cgctgcctcg tcctgcagtt      8040
   cattcagggc accggacagg tcggtcttga caaaaagaac cgggcgcccc tgcgctgaca      8100
   gccggaacac ggcggcatca gagcagccga ttgtctgttg tgcccagtca tagccgaata      8160
   gcctctccac ccaagcggcc ggagaacctg cgtgcaatcc atcttgttca atcatgcgaa      8220
   acgatcctca tcctgtctct tgatcagatc ttgatcccct gcgccatcag atccttggcg      8280
   gcaagaaagc catccagttt actttgcagg gcttcccaac cttaccagag ggcgccccag      8340
   ctggcaattc cggttcgctt gctgtccata aaaccgccca gtctagctat cgccatgtaa      8400
   gcccactgca agctacctgc tttctctttg cgcttgcgtt ttcccttgtc cagatagccc      8460
   agtagctgac attcatccca ggtggcactt ttcggggaaa tgtgcgcgcc cgcgttcctg      8520
   ctggcgctgg gcctgtttct ggcgctggac ttcccgctgt tccgtcagca gcttttcgcc      8580
   cacggccttg atgatcgcgg cggccttggc ctgcatatcc cgattcaacg gccccagggc      8640
   gtccagaacg ggcttcaggc gctcccgaag gt                                    8672
<210> 46
   <211> 21
   <212> DNA
   <213> Artificial
<220>
   <223> Primer
<400> 46
   ggccgctcta gaactagtgg a 21
<210> 47
   <211> 12249
   <212> DNA
   <213> Artificial
<220>
   <223> Vector
<400> 47
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg ggagagccct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagaactagt ggatcccccg ggctgcagga attcggctgc gctaccgcag cccttctccg 3300
   ctaaaaccgt tagtcgaaca gttcggcgtc agccaatgcg accccaagct ggtccttgcc 3360
   agacaagctt ggtacggagt cgatgggcca ttcgataccc actgccggat cattccaggc 3420
   aatgcagcgc tcgcactgcg gcgagtagaa gtcggtggtc ttgtagagga actctgcggt 3480
   ttcactcaac gtgacgaacc cgtgtgcgaa ccctggcggg atccacagct ggttcttgtt 3540
   ctcggccgac aacaccgcac ctacccattt accgaaggtt gtggacgagc gacggatatc 3600
   caccgcaaca tcgaagactt cgccttgcac cacacgcacc agcttgccct gggcgtgagg 3660
   tgccagctga tagtgcaggc cacggagcac gccttttacc gagcgcgagt ggttgtcttg 3720
   tacgaagtcg ggctgcaggc cggtcacttc gctgaaaaca cgggcgttga agctctcgta 3780
   gaagaaacca cgttcgtcgc caaaaacctt gggggtaaac agcacgactt cggggatatc 3840
   cagcggaatg gcttgcatca gaacaccttc tctttcagca agttctgcag atacttgcca 3900
   taaccgtttt tcagcagtgg ttgagccagg cactcgagtt gctcagcgtt gatccagcca 3960
   gcgcggtagc aaatttcctc agggcaggcg actttcaagc cctgacggcg ctccatggtt 4020
   gcgatgtact ggctagcctc cagcagactg tcgtgcgtgc cggtgtcgag ccacgcatag 4080
   ccacggccca tgatttcgac ctgcaactgc tgctgctgca agtaaaggtt gttgaggtcg 4140
   gtgatttcca gctcgccacg tggggaaggc ttcagctcgc gagccagatt gactacctga 4200
   ttgtcataga aatacaggcc ggtgaccgca tagctagact ttggaactgc cggtttttct 4260
   tccagcgaca atacgcgacc gctatcgtca aactccgcta cgccatagcg ttctgggtca 4320
   tgaacatgat aagcgaatac tgaagcaccg gattcacgtt tatctgcgtt caatagcagt 4380
   gcctggaagt catggccgta gaaaatattg tcaccgagaa ccaacgcaga agggtcgtta 4440
   ccgatgaagt cagcgccgat ggtgaacgct tgcgccaagc catccgggct tggttgtatt 4500
   gcgtatgaca ggttcaggcc ccactggctg ccatcgccca gcagctgttc gaagcgcggg 4560
   gtgtcctgcg gggtggaaat gatcaggatg tcccggatac cagcgagcag cagggtgctc 4620
   agcgggtagt agatcatcgg tttgtcatac accggcagca gctgcttcga aaccgaaagt 4680
   gtggccggat gcaggcgtgt acccgaaccg ccggccagaa taattccttt acgagccatg 4740
   agagtcccta ttactggatt tcgtccagca tacgttgcac gccttgctcc caaagcggca 4800
   ttttgaaatt gaacgtgttt tccagtttgc ccagtgccag gcgcgagttg cgcggacgtg 4860
   gtgcaggtac tggataagct tcggtgctga ttgcggcaac cttatcagct gtcactttca 4920
   gcgctacgcc agtgcgttga gcatgcgcca gcacgaactg agcaaaacca tgccaagagg 4980
   tttcaccgga cgcagccaag tggtaaatcc ctgccaggtg acggttgtct tgcccattga 5040
   agatttgccg caggatgtgt gcagtaacgt cggcgatcag gtcagcgccc gtgggtgcgc 5100
   caaattggtc tgctaccacg ctcaacgtct cacgctccgc cgccaggcgc agcatggtct 5160
   tggcaaaatt gtgcccgcgc gcagcataca cccagctggt gcgcagtacc acggccttgg 5220
   cgccgctggc gagaatggca tgctcgcctt ccagcttggt ccggccgtag accgaaaggg 5280
   ggccggtagg cgcagtttcc tcccagcgct gactgccgct gccgtcaaat acataatcgg 5340
   tggaatagtg aatcaaccag gcgcccaaag ctgctgtttc acgtgctaat acagcaggag 5400
   ccgcggcatt gatcattgca gccagtgcct gatcgctctc agctttatcc actgcagtgt 5460
   aggcagcagc gttgacgatc acgtccggcg ccagctgacg aatcgtagcg gccaagccgt 5520
   ccaggttgga caagtcgcca cataagccct cggccccctg acgatccagc gcaatgacct 5580
   cacccagcgg cgccaaggcg cgctgtagct cccagcctac ttgcccgttt ttccccaaca 5640
   gcaggatttt cacgctttat ttgccccgta ttgttgtgcc acccagtcac ggtagctgcc 5700
   gtccatgaca ccttttaccc atttctggtt ggccaagtac caagcgactg tctttcgaat 5760
   gcccgtctcg aaggtttcgg caggtttcca gccgagctcc cgctcgatct tgcgtgcatc 5820
   gatggcataa cggcggtcat ggcctgggcg gtcggttacg taggcgatga gttctgcata 5880
   ctgttcgaca ggctcgccgg tcttctgatt gattacctgg cgcgatgccg caggtgccat 5940
   ctcgtcgaga aggctgcaga gtgtacgcac aatgtcaatg ttggcttttt cattccagcc 6000
   gccaatattg tacgtctcgc cgaacgcacc ggcttccagt acgcgacgga tgcccgagca 6060
   gtgatcttcg acatacagcc agtcgcggat ttgctggccg tcgccataga caggcagcgc 6120
   cttaccggcg agtgcgttga cgatcatcag cgggatcagt ttttccggga agtggagcgg 6180
   cccgtaattg ttggagcagt tggtagtgag taccggcatg ccgtaggtat ggaaatacga 6240
   gcgtaccaga tggtcgctgg ctgccttgct ggcggagtat gggctgttcg gcgcgtacgg 6300
   cgtggtttcg gtgaacgccg ggtcgtttgg ccctagtgtg ccgtagactt cgtcggtaga 6360
   gacatggagg aaacggaagg cctccttctc tgcaccttcc aaactattcc aatgcgcccg 6420
   ggcggcttca agcaagcgaa acgtgcccat cacgttggtt tcgacaaacg cttcggggcc 6480
   ggtgattgag cggtctacat gggattccgc cgcgaagtga accacggcgc gcgggcggtg 6540
   ctctgcgaac agcttggtca gaagcgcagc atcgcaaata ttgccttgca caaagcgatg 6600
   ctgagggttg ccttccagcg gctgcaggtt ggccaggttg cctgcgtagg tcagggcgtc 6660
   gaggttgagg acgggttcct cattgtgcgc acaccattgc agtacgaaat ttgagccgat 6720
   gaagccggct ccgcctgtta ctagaatcat aatttggctc taattggaca aaaggtgttg 6780
   tcgtagacag atgacgcgaa ttcgatatca agcttatcga taccgtcgac ctcgaggggg 6840
   ggcccggtac ccagcttttg ttccctttag tgagggttaa ttgcgcgctt ggcgtaatca 6900
   tggtcatagc tgtttcctgt gtgaaattgt tatccgctca caattccaca caacatacga 6960
   gccggaagca taaagtgtaa agcctggggt gcctaatgag tgagctaact cacattaatt 7020
   gcgttgcgct cactgcccgc tttccagtcg ggaaacctgt cgtgccagct gcattaattc 7080
   tagagggaat gcgtttcgcc gactaggcct tggccttgcc ggaagctacg gacgccacgg 7140
   ccgggccggc gaggcgcttc agcaggcgcg ggcggttggt ctccagcgcg ccgccgcgtc 7200
   cccgcaggcc gtcccacagg ccccagccca ggcagcgcag cttgagcagc ttgtcgcgtt 7260
   cgagcaggag caccgcgagg ccctgggtca gggtcggcag gttcgccagc agggccagcg 7320
   gcgaggaccg ggcgtagcgg cgcaggacca gcaggccgtt gcgcgccagg tagtagcggc 7380
   gcagcggggc gtggttcatc gcgctgaggc tgagaccgcc gaggcggcgg gtcttgcgcg 7440
   tgccgatgcg gtgctcgagg accagccgcg ggtcgacgta caggggcacg tccagcgcct 7500
   gggcgcgcag gctgtattcg gtgtccacgt ggtcgatgaa cagttcctcg tcgaagtggc 7560
   cgaggcgctg gtaggcctcg cgggtcagca ggcagccgga ggagatcagg aacgaggtgc 7620
   gctgcggggt cgtcaggccg tccagagaca attgcctgag cgtcagtccg tcgagatgga 7680
   tggccggcag gaagcgccgg tcaccccggt cgaagatccg tgggccgagc aggcaggcct 7740
   gaccgttgcg cgcctgcagg ttgcgccact gggcggcgag gaaggcgccg ccgggacggg 7800
   agtcctggtc gagcagcagc acaccctgca cgccacgccg gaatagcgcg tcgagtccct 7860
   ggttgaaggc gccggcgatg ccctgccggt tgccgtggtg cagcacggcg atgccttgcc 7920
   cgcgcagccg ggcattgcgc tgcggatcgc tgtgcggtga gttgtcgacg gcaaggaagc 7980
   gcagttgcgg aaacgccgcc gccagttcgc caaggtgttc caggtcgtcg tcgccaggat 8040
   tgaacagtac caccagcacg cccatgtcta tccggtccat gatcgttctt ctcccgtagg 8100
   tcaggacgca gccttcagcc atcgcgcatc cccctcccta tgacaacgtt cgaccacctg 8160
   ggccgcttta ccgcaagcga tactgtgcgg ttgtgacaat tccatgaaac gccgacaggc 8220
   cgccgccatg gccgggtcct cgagcaagcg ccacagcgcc ccgcgcaact cctgctcgcg 8280
   caatggcacg cccaggcgca tcccgcagcc gagccggacc agccgttcgg cattgtcgaa 8340
   ctggtcgtgg gcgcagggca gcagcacctg cggcaccccc gccgccaagg ccaggctcat 8400
   ggcgccgata ccgcccggat ggaccagccc ggcgcacgat ggcagcaagg ctcccagtgg 8460
   cgcgtaggcg cgctgcagca cgtggttcgg caagccgcgc agcggttcct ggccggcgcc 8520
   ggtgaggaag atcccacgcg cgccgaggcg ttccagcgcg cgcagggcca tggcgtagaa 8580
   gtcgccctgc aggtgttcgg tcgagccctg ggtgaacacc agcggccggc tgccctgatc 8640
   gagaaagcgt tgcagttcgt cgtcgagcgg ggtccccggg atactgccgt cgaacagcgg 8700
   gaagccggtc atgtgcaggg gttgcggcca atcctgctgg ggcggcgcga accaggccgg 8760
   gaacaggcag accacgccct gcggcgaatg catccattgg gtgaagatgc gcttcaccgg 8820
   cgtctccagg ccgaccttgc gccgcaccgc gttgatatcc ggcgcgcagg tgcgatccag 8880
   cttgaagcgc tcgatgcagc gccagagcag cttgcgcatc gccagcggca tctgctcggg 8940
   cacgttgaac ttggggtgta ccggcggcag gtgcgccgac aacaaggtcg atggcgagac 9000
   ctgcgcggac aggtagggaa tcccgtactt ctcgtgagcg atgcgtgcgc ccagcgccca 9060
   gagcgagccg accaccacga tgtcgtcatg gcgctgcgcc gagacgtact cgtagaccgg 9120
   ctcgatcatc ccggcgatgg tttgccagag cacgccgaag gacgtcttgg ggtcccacag 9180
   gcgcggatcg cccatggtcc ggcggtaggt cagttcgtcg ctcagcggga cgaacgcgat 9240
   gccgtgctgc tccaccgcgt cgcgaaacac cgggatggtg cagaggctca cgcggtgccc 9300
   gcgcaatttc agggtccggg ccaggccgat gaagggaaat acgtcgccgg ccgagccgat 9360
   ggcgatgagg atggcgtgca tggtgctact ccgtgcgtta tgcaaccgca aagcccggcc 9420
   aggccgggtc ttcgcaggtc aagggttcag gcgtagccga tggccatctc gtggaatccc 9480
   gccgcgcgtt ccgcccgctg cggctccggt tgcttcagca ggtgctcgag cagggcgcgg 9540
   tgcacgcgta ccgcggccag cttggactcc aggtcgagga aatgcccggt gccctccacc 9600
   cgcgagaaac tgcagtgcgg caggtagtcg cggaactggc gggcgtcctc ggcggtggtg 9660
   tattcgtccc agctgccgtt gatgaaatgc acgtggctct ggatccgctc caggcaagcc 9720
   aggtagcccc gatcgttgag cgccagcacc tggtcgatgt gaaagcgcgc ctgctcgtat 9780
   tcgccggtgg ccagcgaagc catgtgctga tggttgctgg ctttcaggcg cggcggcagg 9840
   tatttgccga cggtctcgtt gagcagatgg ccgatcgccg acttgtcgtc cagctcgatc 9900
   agcgcctgcg cccgcccgac gtagtcgagc atcgcctggt tcagtccagg ggcgaatgcc 9960
   atcaccaccg agctgcggat gccgcgcgga ttgcgcgaca gcgccagcag cgtggagata 10020
   ccgccccagg acgcggagac caggtgattg acctcgaagc gctcgatcag cgccaggagg 10080
   atttccacct cgtcgtcctt ggtgatcaac ccccgctgcg ggttgtgctg acgcgactgc 10140
   ccggcgaagg gcaggtcgaa cagcaccacg ttgaaatgtt cggccaggca cttgcaggtc 10200
   cgggcgaacg aggcggtggt cgccatcgcg ccgttgacca gcatcaccgt gctgcgcccg 10260
   ggatcctgcc caacgcgctc gacatgtacc cgcaggccct tgcaaaccga taccaacaga 10320
   ctttcgcgcc gcatttcaca cctcccaaaa atgccagatc ccccgggctg caggaattcg 10380
   atatcaagct tatcgatacc gtcgacctcg aggggggccc cggtacccag cttttgttcc 10440
   ctttagtgag ggttaattgc gcgcttggcg taatcatggt catagctgtt tcctgtgtga 10500
   aattgttatc cgctcacaat tccacacaac atacgagccg gaagcataaa gtgtaaagcc 10560
   tggggtgcct aatgagtgag ctaactcaca ttaattgcgt tgcgctcact gcccgctttc 10620
   cagtcgggaa acctgtcgtg ccagctgcat taatgaatcg gccaacgcgc ggggagaggc 10680
   ggtttgcgta ttgggcgcat gcataaaaac tgttgtaatt cattaagcat tctgccgaca 10740
   tggaagccat cacaaacggc atgatgaacc tgaatcgcca gcggcatcag caccttgtcg 10800
   ccttgcgtat aatatttgcc catgggggtg ggcgaagaac tccagcatga gatccccgcg 10860
   ctggaggatc atccagccgg cgtcccggaa aacgattccg aagcccaacc tttcatagaa 10920
   ggcggcggtg gaatcgaaat ctcgtgatgg caggttgggc gtcgcttggt cggtcatttc 10980
   gaaccccaga gtcccgctca gaagaactcg tcaagaaggc gatagaaggc gatgcgctgc 11040
   gaatcgggag cggcgatacc gtaaagcacg aggaagcggt cagcccattc gccgccaagc 11100
   tcttcagcaa tatcacgggt agccaacgct atgtcctgat agcggtccgc cacacccagc 11160
   cggccacagt cgatgaatcc agaaaagcgg ccattttcca ccatgatatt cggcaagcag 11220
   gcatcgccat gggtcacgac gagatcctcg ccgtcgggca tgcgcgcctt gagcctggcg 11280
   aacagttcgg ctggcgcgag cccctgatgc tcttcgtcca gatcatcctg atcgacaaga 11340
   ccggcttcca tccgagtacg tgctcgctcg atgcgatgtt tcgcttggtg gtcgaatggg 11400
   caggtagccg gatcaagcgt atgcagccgc cgcattgcat cagccatgat ggatactttc 11460
   tcggcaggag caaggtgaga tgacaggaga tcctgccccg gcacttcgcc caatagcagc 11520
   cagtcccttc ccgcttcagt gacaacgtcg agcacagctg cgcaaggaac gcccgtcgtg 11580
   gccagccacg atagccgcgc tgcctcgtcc tgcagttcat tcagggcacc ggacaggtcg 11640
   gtcttgacaa aaagaaccgg gcgcccctgc gctgacagcc ggaacacggc ggcatcagag 11700
   cagccgattg tctgttgtgc ccagtcatag ccgaatagcc tctccaccca agcggccgga 11760
   gaacctgcgt gcaatccatc ttgttcaatc atgcgaaacg atcctcatcc tgtctcttga 11820
   tcagatcttg atcccctgcg ccatcagatc cttggcggca agaaagccat ccagtttact 11880
   ttgcagggct tcccaacctt accagagggc gccccagctg gcaattccgg ttcgcttgct 11940
   gtccataaaa ccgcccagtc tagctatcgc catgtaagcc cactgcaagc tacctgcttt 12000
   ctctttgcgc ttgcgttttc ccttgtccag atagcccagt agctgacatt catcccaggt 12060
   ggcacttttc ggggaaatgt gcgcgcccgc gttcctgctg gcgctgggcc tgtttctggc 12120
   gctggacttc ccgctgttcc gtcagcagct tttcgcccac ggccttgatg atcgcggcgg 12180
   ccttggcctg catatcccga ttcaacggcc ccagggcgtc cagaacgggc ttcaggcgct 12240
   cccgaaggt 12249
<210> 48
   <211> 37
   <212> DNA
   <213> Artificial
<220>
   <223> Primer
<400> 48
   tatatataga attcgcgtca tctgtctacg acaacac 37
<210> 49
   <211> 5144
   <212> DNA
   <213> Artificial
<220>
   <223> Vector
<400> 49
   ctcgggccgt ctcttgggct tgatcggcct tcttgcgcat ctcacgcgct cctgcggcgg     60
   cctgtagggc aggctcatac ccctgccgaa ccgcttttgt cagccggtcg gccacggctt    120
   ccggcgtctc aacgcgcttt gagattccca gcttttcggc caatccctgc ggtgcatagg    180
   cgcgtggctc gaccgcttgc gggctgatgg tgacgtggcc cactggtggc cgctccaggg    240
   cctcgtagaa cgcctgaatg cgcgtgtgac gtgccttgct gccctcgatg ccccgttgca    300
   gccctagatc ggccacagcg gccgcaaacg tggtctggtc gcgggtcatc tgcgctttgt    360
   tgccgatgaa ctccttggcc gacagcctgc cgtcctgcgt cagcggcacc acgaacgcgg    420
   tcatgtgcgg gctggtttcg tcacggtgga tgctggccgt cacgatgcga tccgccccgt    480
   acttgtccgc cagccacttg tgcgccttct cgaagaacgc cgcctgctgt tcttggctgg    540
   ccgacttcca ccattccggg ctggccgtca tgacgtactc gaccgccaac acagcgtcct    600
   tgcgccgctt ctctggcagc aactcgcgca gtcggcccat cgcttcatcg gtgctgctgg    660
   ccgcccagtg ctcgttctct ggcgtcctgc tggcgtcagc gttgggcgtc tcgcgctcgc    720
   ggtaggcgtg cttgagactg gccgccacgt tgcccatttt cgccagcttc ttgcatcgca    780
   tgatcgcgta tgccgccatg cctgcccctc ccttttggtg tccaaccggc tcgacggggg      840
   cagcgcaagg cggtgcctcc ggcgggccac tcaatgcttg agtatactca ctagactttg      900
   cttcgcaaag tcgtgaccgc ctacggcggc tgcggcgccc tacgggcttg ctctccgggc      960
   ttcgccctgc gcggtcgctg cgctcccttg ccagcccgtg gatatgtgga cgatggccgc     1020
   gagcggccac cggctggctc gcttcgctcg gcccgtggac aaccctgctg gacaagctga     1080
   tggacaggct gcgcctgccc acgagcttga ccacagggat tgcccaccgg ctacccagcc     1140
   ttcgaccaca tacccaccgg ctccaactgc gcggcctgcg gccttgcccc atcaattttt     1200
   ttaattttct ctggggaaaa gcctccggcc tgcggcctgc gcgcttcgct tgccggttgg     1260
   acaccaagtg gaaggcgggt caaggctcgc gcagcgaccg cgcagcggct tggccttgac     1320
   gcgcctggaa cgacccaagc ctatgcgagt gggggcagtc gaaggcgaag cccgcccgcc     1380
   tgccccccga gcctcacggc ggcgagtgcg ggggttccaa gggggcagcg ccaccttggg      1440
   caaggccgaa ggccgcgcag tcgatcaaca agccccggag gggccacttt ttgccggagg     1500
   gggagccgcg ccgaaggcgt gggggaaccc cgcaggggtg cccttctttg ggcaccaaag     1560
   aactagatat agggcgaaat gcgaaagact taaaaatcaa caacttaaaa aaggggggta      1620
   cgcaacagct cattgcggca ccccccgcaa tagctcattg cgtaggttaa agaaaatctg     1680
   taattgactg ccacttttac gcaacgcata attgttgtcg cgctgccgaa aagttgcagc     1740
   tgattgcgca tggtgccgca accgtgcggc accctaccgc atggagataa gcatggccac     1800
   gcagtccaga gaaatcggca ttcaagccaa gaacaagccc ggtcactggg tgcaaacgga     1860
   acgcaaagcg catgaggcgt gggccgggct tattgcgagg aaacccacgg cggcaatgct     1920
   gctgcatcac ctcgtggcgc agatgggcca ccagaacgcc gtggtggtca gccagaagac     1980
   actttccaag ctcatcggac gttctttgcg gacggtccaa tacgcagtca aggacttggt     2040
   ggccgagcgc tggatctccg tcgtgaagct caacggcccc ggcaccgtgt cggcctacgt     2100
   ggtcaatgac cgcgtggcgt ggggccagcc ccgcgaccag ttgcgcctgt cggtgttcag     2160
   tgccgccgtg gtggttgatc acgacgacca ggacgaatcg ctgttggggc atggcgacct     2220
   gcgccgcatc ccgaccctgt atccgggcga gcagcaacta ccgaccggcc ccggcgagga     2280
   gccgcccagc cagcccggca ttccgggcat ggaaccagac ctgccagcct tgaccgaaac     2340
   ggaggaatgg gaacggcgcg ggcagcagcg cctgccgatg cccgatgagc cgtgttttct     2400
   ggacgatggc gagccgttgg agccgccgac acgggtcacg ctgccgcgcc ggtagcactt     2460
   gggttgcgca gcaacccgta agtgcgctgt tccagactat cggctgtagc cgcctcgccg     2520
   ccctatacct tgtctgcctc cccgcgttgc gtcgcggtgc atggagccgg gccacctcga     2580
   cctgaatgga agccggcggc acctcgctaa cggattcacc gtttttatca ggctctggga     2640
   ggcagaataa atgatcatat cgtcaattat tacctccacg gggagagcct gagcaaactg      2700
   gcctcaggca tttgagaagc acacggtcac actgcttccg gtagtcaata aaccggtaaa     2760
   ccagcaatag acataagcgg ctatttaacg accctgccct gaaccgacga ccgggtcgaa     2820
   tttgctttcg aatttctgcc attcatccgc ttattatcac ttattcaggc gtagcaccag     2880
   gcgtttaagg gcaccaataa ctgccttaaa aaaattacgc cccgccctgc cactcatcgc     2940
   agtcggccta ttggttaaaa aatgagctga tttaacaaaa atttaacgcg aattttaaca     3000
   aaatattaac gcttacaatt tccattcgcc attcaggctg cgcaactgtt gggaagggcg     3060
   atcggtgcgg gcctcttcgc tattacgcca gctggcgaaa gggggatgtg ctgcaaggcg     3120
   attaagttgg gtaacgccag ggttttccca gtcacgacgt tgtaaaacga cggccagtga     3180
   gcgcgcgtaa tacgactcac tatagggcga attggagctc caccgcggtg gcggccgctc     3240
   tagaactagt ggatcccccg ggctgcagga attcgatatc aagcttatcg ataccgtcga     3300
   cctcgagggg gggcccggta cccagctttt gttcccttta gtgagggtta attgcgcgct     3360
   tggcgtaatc atggtcatag ctgtttcctg tgtgaaattg ttatccgctc acaattccac     3420
   acaacatacg agccggaagc ataaagtgta aagcctgggg tgcctaatga gtgagctaac     3480
   tcacattaat tgcgttgcgc tcactgcccg ctttccagtc gggaaacctg tcgtgccagc     3540
   tgcattaatg aatcggccaa cgcgcgggga gaggcggttt gcgtattggg cgcatgcata     3600
   aaaactgttg taattcatta agcattctgc cgacatggaa gccatcacaa acggcatgat     3660
   gaacctgaat cgccagcggc atcagcacct tgtcgccttg cgtataatat ttgcccatgg     3720
   gggtgggcga agaactccag catgagatcc ccgcgctgga ggatcatcca gccggcgtcc     3780
   cggaaaacga ttccgaagcc caacctttca tagaaggcgg cggtggaatc gaaatctcgt     3840
   gatggcaggt tgggcgtcgc ttggtcggtc atttcgaacc ccagagtccc gctcagaaga     3900
   actcgtcaag aaggcgatag aaggcgatgc gctgcgaatc gggagcggcg ataccgtaaa     3960
   gcacgaggaa gcggtcagcc cattcgccgc caagctcttc agcaatatca cgggtagcca     4020
   acgctatgtc ctgatagcgg tccgccacac ccagccggcc acagtcgatg aatccagaaa     4080
   agcggccatt ttccaccatg atattcggca agcaggcatc gccatgggtc acgacgagat     4140
   cctcgccgtc gggcatgcgc gccttgagcc tggcgaacag ttcggctggc gcgagcccct     4200
   gatgctcttc gtccagatca tcctgatcga caagaccggc ttccatccga gtacgtgctc     4260
   gctcgatgcg atgtttcgct tggtggtcga atgggcaggt agccggatca agcgtatgca     4320
   gccgccgcat tgcatcagcc atgatggata ctttctcggc aggagcaagg tgagatgaca     4380
   ggagatcctg ccccggcact tcgcccaata gcagccagtc ccttcccgct tcagtgacaa     4440
   cgtcgagcac agctgcgcaa ggaacgcccg tcgtggccag ccacgatagc cgcgctgcct     4500
   cgtcctgcag ttcattcagg gcaccggaca ggtcggtctt gacaaaaaga accgggcgcc     4560
   cctgcgctga cagccggaac acggcggcat cagagcagcc gattgtctgt tgtgcccagt     4620
   catagccgaa tagcctctcc acccaagcgg ccggagaacc tgcgtgcaat ccatcttgtt     4680
   caatcatgcg aaacgatcct catcctgtct cttgatcaga tcttgatccc ctgcgccatc 4740
   agatccttgg cggcaagaaa gccatccagt ttactttgca gggcttccca accttaccag 4800
   agggcgcccc agctggcaat tccggttcgc ttgctgtcca taaaaccgcc cagtctagct 4860
   atcgccatgt aagcccactg caagctacct gctttctctt tgcgcttgcg ttttcccttg 4920
   tccagatagc ccagtagctg acattcatcc caggtggcac ttttcgggga aatgtgcgcg 4980
   cccgcgttcc tgctggcgct gggcctgttt ctggcgctgg acttcccgct gttccgtcag 5040
   cagcttttcg cccacggcct tgatgatcgc ggcggccttg gcctgcatat cccgattcaa 5100
   cggccccagg gcgtccagaa cgggcttcag gcgctcccga aggt 5144
<210> 50
   <211> 4549
   <212> DNA
   <213> Pseudomonas aeruginosa
<400> 50
   ggtaccagat ctggcatttt tgggaggtgt gaaatgcggc gcgaaagtct gttggtatcg 60
   gtttgcaagg gcctgcgggt acatgtcgag cgcgttgggc aggatcccgg gcgcagcacg 120
   gtgatgctgg tcaacggcgc gatggcgacc accgcctcgt tcgcccggac ctgcaagtgc 180
   ctggccgaac atttcaacgt ggtgctgttc gacctgccct tcgccgggca gtcgcgtcag 240
   cacaacccgc agcgggggtt gatcaccaag gacgacgagg tggaaatcct cctggcgctg 300
   atcgagcgct tcgaggtcaa tcacctggtc tccgcgtcct ggggcggtat ctccacgctg 360
   ctggcgctgt cgcgcaatcc gcgcggcatc cgcagctcgg tggtgatggc attcgcccct 420
   ggactgaacc aggcgatgct cgactacgtc gggcgggcgc aggcgctgat cgagctggac 480
   gacaagtcgg cgatcggcca tctgctcaac gagaccgtcg gcaaatacct gccgccgcgc 540
   ctgaaagcca gcaaccatca gcacatggct tcgctggcca ccggcgaata cgagcaggcg 600
   cgctttcaca tcgaccaggt gctggcgctc aacgatcggg gctacctggc ttgcctggag 660
   cggatccaga gccacgtgca tttcatcaac ggcagctggg acgaatacac caccgccgag 720
   gacgcccgcc agttccgcga ctacctgccg cactgcagtt tctcgcgggt ggagggcacc 780
   gggcatttcc tcgacctgga gtccaagctg gccgcggtac gcgtgcaccg cgccctgctc 840
   gagcacctgc tgaagcaacc ggagccgcag cgggcggaac gcgcggcggg attccacgag 900
   atggccatcg gctacgcctg aacccttgac ctgcgaagac ccggcctggc cgggctttgc 960
   ggttgcataa cgcacggagt agcaccatgc acgccatcct catcgccatc ggctcggccg 1020
   gcgacgtatt tcccttcatc ggcctggccc ggaccctgaa attgcgcggg caccgcgtga 1080
   gcctctgcac catcccggtg tttcgcgacg cggtggagca gcacggcatc gcgttcgtcc 1140
   cgctgagcga cgaactgacc taccgccgga ccatgggcga tccgcgcctg tgggacccca 1200
   agacgtcctt cggcgtgctc tggcaaacca tcgccgggat gatcgagccg gtctacgagt 1260
   acgtctcggc gcagcgccat gacgacatcg tggtggtcgg ctcgctctgg gcgctgggcg 1320
   cacgcatcgc tcacgagaag tacgggattc cctacctgtc cgcgcaggtc tcgccatcga 1380
   ccttgttgtc ggcgcacctg ccgccggtac accccaagtt caacgtgccc gagcagatgc 1440
   cgctggcgat gcgcaagctg ctctggcgct gcatcgagcg cttcaagctg gatcgcacct 1500
   gcgcgccgga tatcaacgcg gtgcggcgca aggtcggcct ggagacgccg gtgaagcgca 1560
   tcttcaccca atggatgcat tcgccgcagg gcgtggtctg cctgttcccg gcctggttcg 1620
   cgccgcccca gcaggattgg ccgcaacccc tgcacatgac cggcttcccg ctgttcgacg 1680
   gcagtatccc ggggaccccg ctcgacgacg aactgcaacg ctttctcgat cagggcagcc 1740
   ggccgctggt gttcacccag ggctcgaccg aacacctgca gggcgacttc tacgccatgg 1800
   ccctgcgcgc gctggaacgc ctcggcgcgc gtgggatctt cctcaccggc gccggccagg 1860
   aaccgctgcg cggcttgccg aaccacgtgc tgcagcgcgc ctacgcgcca ctgggagcct 1920
   tgctgccatc gtgcgccggg ctggtccatc cgggcggtat cggcgccatg agcctggcct 1980
   tggcggcggg ggtgccgcag gtgctgctgc cctgcgccca cgaccagttc gacaatgccg 2040
   aacggctggt ccggctcggc tgcgggatgc gcctgggcgt gccattgcgc gagcaggagt 2100
   tgcgcggggc gctgtggcgc ttgctcgagg acccggccat ggcggcggcc tgtcggcgtt 2160
   tcatggaatt gtcacaaccg cacagtatcg cttgcggtaa agcggcccag gtggtcgaac 2220
   gttgtcatag ggagggggat gcgcgatggc tgaaggctgc gtcctgacgc cgggaggatc 2280
   ctggcgtgtc cacgaccagc ctctgcccct ccgccacgcg ggaacacggt cccggcgcga 2340
   aacgcgtcct gcctctgctg ttcctcacct gcctgctgga tgccgctggc gtcggcctga 2400
   tcgtgcccct gctgccgacg ctgatcggca gcgtggcgcc gctggcggtc cgcgacgcgg 2460
   ccacctgggg cgccgccctg gtgatgacct tcgcgctgct gcaattgttc ttttcgccgg 2520
   tcctcggcag cctcagcgac cgcttcggac gccgccccgt cctggtcctg gcgatgctcg 2580
   gcttcgccct cagctatctg ctgctggcgc tggccgacag cctctggatg ctgttcctcg 2640
   gtcgcgcgct ggccgggctc accggcgcca gcgtggccac cgcgatggcc tgcgcggctg 2700
   acctcggcac gcacgggcag cgcacccggc acttcggctg gctgtacgcc ggcctcgccc 2760
   tgggcatgat cctcggcccc gccctcggtg ggctgctggc ggtgcacggc acgacgctgc 2820
   cgctgttgct ggccgccggc ctgtgcctgc tcaacgccct gctcgccggc ctgttcctcg 2880
   aggaaaccct gcccccgacg cgacgccgcc gcctggaccc gaggcggatg aatgccttgc 2940
   gctcgatcag cggcctggct cggcaaccgg gggtcggacg cctgctggcg gtgcttgccc 3000
   tggtattcct cggcttgcag gcggtgatgg tggtctggcc gttcttcgtg atcgagaagt 3060
   ttcactggag cagcgcctgg atcggctact cgctggccct ctacggcgtg ctcgcggtgc 3120
   tcgcccagac cctcggcgtg aacctctgca agcggcgcct ggacgacgcc cgcctgctgc 3180
   gcctgggcct cgccctgcaa ggctgcggcc tgctgctgtt cgccctggtc gactcgtcat 3240
   tctggctggt ctgcgcgctg ctgcccttcg cgctcggcag cctcgccacc ccggccatgc 3300
   aggggctgct ctcggcccgc gtgccggtcg accgccaggg cgagttgcag ggcgtgctga 3360
   gcagcctgat gagcctcgcc gcgatcgtcg gtccgccgct gatgagcggc ctgttccact 3420
   ggggcagcgg tccgctcgcg ccgctgcccc tggccggcgc gccattcctc gccggcgccc 3480
   ttctcgttct ggccgggctg gtcctggcct ggcaacttcg acctacggga gaagaacgat 3540
   catggaccgg atagacatgg gcgtgctggt ggtactgttc aatcctggcg acgacgacct 3600
   ggaacacctt ggcgaactgg cggcggcgtt tccgcaactg cgcttccttg ccgtcgacaa 3660
   ctcaccgcac agcgatccgc agcgcaatgc ccggctgcgc gggcaaggca tcgccgtgct 3720
   gcaccacggc aaccggcagg gcatcgccgg cgccttcaac cagggactcg acgcgctatt 3780
   ccggcgtggc gtgcagggtg tgctgctgct cgaccaggac tcccgtcccg gcggcgcctt 3840
   cctcgccgcc cagtggcgca acctgcaggc gcgcaacggt caggcctgcc tgctcggccc 3900
   acggatcttc gaccggggtg accggcgctt cctgccggcc atccatctcg acggactgac 3960
   gctcaggcaa ttgtctctgg acggcctgac gaccccgcag cgcacctcgt tcctgatctc 4020
   ctccggctgc ctgctgaccc gcgaggccta ccagcgcctc ggccacttcg acgaggaact 4080
   gttcatcgac cacgtggaca ccgaatacag cctgcgcgcc caggcgctgg acgtgcccct 4140
   gtacgtcgac ccgcggctgg tcctcgagca ccgcatcggc acgcgcaaga cccgccgcct 4200
   cggcggtctc agcctcagcg cgatgaacca cgccccgctg cgccgctact acctggcgcg 4260
   caacggcctg ctggtcctgc gccgctacgc ccggtcctcg ccgctggccc tgctggcgaa 4320
   cctgccgacc ctgacccagg gcctcgcggt gctcctgctc gaacgcgaca agctgctcaa 4380
   gctgcgctgc ctgggctggg gcctgtggga cggcctgcgg ggacgcggcg gcgcgctgga 4440
   gaccaaccgc ccgcgcctgc tgaagcgcct cgccggcccg gccgtggcgt ccgtagcttc 4500
   cggcaaggcc aaggcctagt cggcgaaacg cattccctct agagagctc 4549
<210> 51
   <211> 9663
   <212> DNA
   <213> Artificial Sequence
<220>
   <223> vector
<400> 51
   ctcgggccgt ctcttgggct tgatcggcct tcttgcgcat ctcacgcgct cctgcggcgg      60
   cctgtagggc aggctcatac ccctgccgaa ccgcttttgt cagccggtcg gccacggctt     120
   ccggcgtctc aacgcgcttt gagattccca gcttttcggc caatccctgc ggtgcatagg     180
   cgcgtggctc gaccgcttgc gggctgatgg tgacgtggcc cactggtggc cgctccaggg     240
   cctcgtagaa cgcctgaatg cgcgtgtgac gtgccttgct gccctcgatg ccccgttgca     300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagagggaat gcgtttcgcc gactaggcct tggccttgcc ggaagctacg gacgccacgg 3300
   ccgggccggc gaggcgcttc agcaggcgcg ggcggttggt ctccagcgcg ccgccgcgtc 3360
   cccgcaggcc gtcccacagg ccccagccca ggcagcgcag cttgagcagc ttgtcgcgtt 3420
   cgagcaggag caccgcgagg ccctgggtca gggtcggcag gttcgccagc agggccagcg 3480
   gcgaggaccg ggcgtagcgg cgcaggacca gcaggccgtt gcgcgccagg tagtagcggc 3540
   gcagcggggc gtggttcatc gcgctgaggc tgagaccgcc gaggcggcgg gtcttgcgcg 3600
   tgccgatgcg gtgctcgagg accagccgcg ggtcgacgta caggggcacg tccagcgcct 3660
   gggcgcgcag gctgtattcg gtgtccacgt ggtcgatgaa cagttcctcg tcgaagtggc 3720
   cgaggcgctg gtaggcctcg cgggtcagca ggcagccgga ggagatcagg aacgaggtgc 3780
   gctgcggggt cgtcaggccg tccagagaca attgcctgag cgtcagtccg tcgagatgga 3840
   tggccggcag gaagcgccgg tcaccccggt cgaagatccg tgggccgagc aggcaggcct 3900
   gaccgttgcg cgcctgcagg ttgcgccact gggcggcgag gaaggcgccg ccgggacggg 3960
   agtcctggtc gagcagcagc acaccctgca cgccacgccg gaatagcgcg tcgagtccct 4020
   ggttgaaggc gccggcgatg ccctgccggt tgccgtggtg cagcacggcg atgccttgcc 4080
   cgcgcagccg ggcattgcgc tgcggatcgc tgtgcggtga gttgtcgacg gcaaggaagc 4140
   gcagttgcgg aaacgccgcc gccagttcgc caaggtgttc caggtcgtcg tcgccaggat 4200
   tgaacagtac caccagcacg cccatgtcta tccggtccat gatcgttctt ctcccgtagg 4260
   tcgaagttgc caggccagga ccagcccggc cagaacgaga agggcgccgg cgaggaatgg 4320
   cgcgccggcc aggggcagcg gcgcgagcgg accgctgccc cagtggaaca ggccgctcat 4380
   cagcggcgga ccgacgatcg cggcgaggct catcaggctg ctcagcacgc cctgcaactc 4440
   gccctggcgg tcgaccggca cgcgggccga gagcagcccc tgcatggccg gggtggcgag 4500
   gctgccgagc gcgaagggca gcagcgcgca gaccagccag aatgacgagt cgaccagggc 4560
   gaacagcagc aggccgcagc cttgcagggc gaggcccagg cgcagcaggc gggcgtcgtc 4620
   caggcgccgc ttgcagaggt tcacgccgag ggtctgggcg agcaccgcga gcacgccgta 4680
   gagggccagc gagtagccga tccaggcgct gctccagtga aacttctcga tcacgaagaa 4740
   cggccagacc accatcaccg cctgcaagcc gaggaatacc agggcaagca ccgccagcag 4800
   gcgtccgacc cccggttgcc gagccaggcc gctgatcgag cgcaaggcat tcatccgcct 4860
   cgggtccagg cggcggcgtc gcgtcggggg cagggtttcc tcgaggaaca ggccggcgag 4920
   cagggcgttg agcaggcaca ggccggcggc cagcaacagc ggcagcgtcg tgccgtgcac 4980
   cgccagcagc ccaccgaggg cggggccgag gatcatgccc agggcgaggc cggcgtacag 5040
   ccagccgaag tgccgggtgc gctgcccgtg cgtgccgagg tcagccgcgc aggccatcgc 5100
   ggtggccacg ctggcgccgg tgagcccggc cagcgcgcga ccgaggaaca gcatccagag 5160
   gctgtcggcc agcgccagca gcagatagct gagggcgaag ccgagcatcg ccaggaccag 5220
   gacggggcgg cgtccgaagc ggtcgctgag gctgccgagg accggcgaaa agaacaattg 5280
   cagcagcgcg aaggtcatca ccagggcggc gccccaggtg gccgcgtcgc ggaccgccag 5340
   cggcgccacg ctgccgatca gcgtcggcag caggggcacg atcaggccga cgccagcggc 5400
   atccagcagg caggtgagga acagcagagg caggacgcgt ttcgcgccgg gaccgtgttc 5460
   ccgcgtggcg gaggggcaga ggctggtcgt ggacacgcca ggatcctccc ggcgtcagga 5520
   cgcagccttc agccatcgcg catccccctc cctatgacaa cgttcgacca cctgggccgc 5580
   tttaccgcaa gcgatactgt gcggttgtga caattccatg aaacgccgac aggccgccgc 5640
   catggccggg tcctcgagca agcgccacag cgccccgcgc aactcctgct cgcgcaatgg 5700
   cacgcccagg cgcatcccgc agccgagccg gaccagccgt tcggcattgt cgaactggtc 5760
   gtgggcgcag ggcagcagca cctgcggcac ccccgccgcc aaggccaggc tcatggcgcc 5820
   gataccgccc ggatggacca gcccggcgca cgatggcagc aaggctccca gtggcgcgta 5880
   ggcgcgctgc agcacgtggt tcggcaagcc gcgcagcggt tcctggccgg cgccggtgag 5940
   gaagatccca cgcgcgccga ggcgttccag cgcgcgcagg gccatggcgt agaagtcgcc 6000
   ctgcaggtgt tcggtcgagc cctgggtgaa caccagcggc cggctgccct gatcgagaaa 6060
   gcgttgcagt tcgtcgtcga gcggggtccc cgggatactg ccgtcgaaca gcgggaagcc 6120
   ggtcatgtgc aggggttgcg gccaatcctg ctggggcggc gcgaaccagg ccgggaacag 6180
   gcagaccacg ccctgcggcg aatgcatcca ttgggtgaag atgcgcttca ccggcgtctc 6240
   caggccgacc ttgcgccgca ccgcgttgat atccggcgcg caggtgcgat ccagcttgaa 6300
   gcgctcgatg cagcgccaga gcagcttgcg catcgccagc ggcatctgct cgggcacgtt 6360
   gaacttgggg tgtaccggcg gcaggtgcgc cgacaacaag gtcgatggcg agacctgcgc 6420
   ggacaggtag ggaatcccgt acttctcgtg agcgatgcgt gcgcccagcg cccagagcga 6480
   gccgaccacc acgatgtcgt catggcgctg cgccgagacg tactcgtaga ccggctcgat 6540
   catcccggcg atggtttgcc agagcacgcc gaaggacgtc ttggggtccc acaggcgcgg 6600
   atcgcccatg gtccggcggt aggtcagttc gtcgctcagc gggacgaacg cgatgccgtg 6660
   ctgctccacc gcgtcgcgaa acaccgggat ggtgcagagg ctcacgcggt gcccgcgcaa 6720
   tttcagggtc cgggccaggc cgatgaaggg aaatacgtcg ccggccgagc cgatggcgat 6780
   gaggatggcg tgcatggtgc tactccgtgc gttatgcaac cgcaaagccc ggccaggccg 6840
   ggtcttcgca ggtcaagggt tcaggcgtag ccgatggcca tctcgtggaa tcccgccgcg 6900
   cgttccgccc gctgcggctc cggttgcttc agcaggtgct cgagcagggc gcggtgcacg 6960
   cgtaccgcgg ccagcttgga ctccaggtcg aggaaatgcc cggtgccctc cacccgcgag 7020
   aaactgcagt gcggcaggta gtcgcggaac tggcgggcgt cctcggcggt ggtgtattcg 7080
   tcccagctgc cgttgatgaa atgcacgtgg ctctggatcc gctccaggca agccaggtag 7140
   ccccgatcgt tgagcgccag cacctggtcg atgtgaaagc gcgcctgctc gtattcgccg 7200
   gtggccagcg aagccatgtg ctgatggttg ctggctttca ggcgcggcgg caggtatttg 7260
   ccgacggtct cgttgagcag atggccgatc gccgacttgt cgtccagctc gatcagcgcc 7320
   tgcgcccgcc cgacgtagtc gagcatcgcc tggttcagtc caggggcgaa tgccatcacc 7380
   accgagctgc ggatgccgcg cggattgcgc gacagcgcca gcagcgtgga gataccgccc 7440
   caggacgcgg agaccaggtg attgacctcg aagcgctcga tcagcgccag gaggatttcc 7500
   acctcgtcgt ccttggtgat caacccccgc tgcgggttgt gctgacgcga ctgcccggcg 7560
   aagggcaggt cgaacagcac cacgttgaaa tgttcggcca ggcacttgca ggtccgggcg 7620
   aacgaggcgg tggtcgccat cgcgccgttg accagcatca ccgtgctgcg cccgggatcc 7680
   tgcccaacgc gctcgacatg tacccgcagg cccttgcaaa ccgataccaa cagactttcg 7740
   cgccgcattt cacacctccc aaaaatgcca gatcccccgg gctgcaggaa ttcgatatca 7800
   agcttatcga taccgtcgac ctcgaggggg ggcccggtac ccagcttttg ttccctttag 7860
   tgagggttaa ttgcgcgctt ggcgtaatca tggtcatagc tgtttcctgt gtgaaattgt 7920
   tatccgctca caattccaca caacatacga gccggaagca taaagtgtaa agcctggggt 7980
   gcctaatgag tgagctaact cacattaatt gcgttgcgct cactgcccgc tttccagtcg 8040
   ggaaacctgt cgtgccagct gcattaatga atcggccaac gcgcggggag aggcggtttg 8100
   cgtattgggc gcatgcataa aaactgttgt aattcattaa gcattctgcc gacatggaag 8160
   ccatcacaaa cggcatgatg aacctgaatc gccagcggca tcagcacctt gtcgccttgc 8220
   gtataatatt tgcccatggg ggtgggcgaa gaactccagc atgagatccc cgcgctggag 8280
   gatcatccag ccggcgtccc ggaaaacgat tccgaagccc aacctttcat agaaggcggc 8340
   ggtggaatcg aaatctcgtg atggcaggtt gggcgtcgct tggtcggtca tttcgaaccc 8400
   cagagtcccg ctcagaagaa ctcgtcaaga aggcgataga aggcgatgcg ctgcgaatcg 8460
   ggagcggcga taccgtaaag cacgaggaag cggtcagccc attcgccgcc aagctcttca 8520
   gcaatatcac gggtagccaa cgctatgtcc tgatagcggt ccgccacacc cagccggcca 8580
   cagtcgatga atccagaaaa gcggccattt tccaccatga tattcggcaa gcaggcatcg 8640
   ccatgggtca cgacgagatc ctcgccgtcg ggcatgcgcg ccttgagcct ggcgaacagt 8700
   tcggctggcg cgagcccctg atgctcttcg tccagatcat cctgatcgac aagaccggct 8760
   tccatccgag tacgtgctcg ctcgatgcga tgtttcgctt ggtggtcgaa tgggcaggta 8820
   gccggatcaa gcgtatgcag ccgccgcatt gcatcagcca tgatggatac tttctcggca 8880
   ggagcaaggt gagatgacag gagatcctgc cccggcactt cgcccaatag cagccagtcc 8940
   cttcccgctt cagtgacaac gtcgagcaca gctgcgcaag gaacgcccgt cgtggccagc 9000
   cacgatagcc gcgctgcctc gtcctgcagt tcattcaggg caccggacag gtcggtcttg 9060
   acaaaaagaa ccgggcgccc ctgcgctgac agccggaaca cggcggcatc agagcagccg 9120
   attgtctgtt gtgcccagtc atagccgaat agcctctcca cccaagcggc cggagaacct 9180
   gcgtgcaatc catcttgttc aatcatgcga aacgatcctc atcctgtctc ttgatcagat 9240
   cttgatcccc tgcgccatca gatccttggc ggcaagaaag ccatccagtt tactttgcag 9300
   ggcttcccaa ccttaccaga gggcgcccca gctggcaatt ccggttcgct tgctgtccat 9360
   aaaaccgccc agtctagcta tcgccatgta agcccactgc aagctacctg ctttctcttt 9420
   gcgcttgcgt tttcccttgt ccagatagcc cagtagctga cattcatccc aggtggcact 9480
   tttcggggaa atgtgcgcgc ccgcgttcct gctggcgctg ggcctgtttc tggcgctgga 9540
   cttcccgctg ttccgtcagc agcttttcgc ccacggcctt gatgatcgcg gcggccttgg 9600
   cctgcatatc ccgattcaac ggccccaggg cgtccagaac gggcttcagg cgctcccgaa 9660
   ggt 9663
<210> 52
   <211> 9793
   <212> DNA
   <213> Artificial Sequence
<220>
   <223> vector
<400> 52
   gatctggcat ttttgggagg tgtgaaatgc ggcgcgaaag tctgttggta tcggtttgca      60
   agggcctgcg ggtacatgtc gagcgcgttg ggcaggatcc cgggcgcagc acggtgatgc     120
   tggtcaacgg cgcgatggcg accaccgcct cgttcgcccg gacctgcaag tgcctggccg     180
   aacatttcaa cgtggtgctg ttcgacctgc ccttcgccgg gcagtcgcgt cagcacaacc     240
   cgcagcgggg gttgatcacc aaggacgacg aggtggaaat cctcctggcg ctgatcgagc     300
   gcttcgaggt caatcacctg gtctccgcgt cctggggcgg tatctccacg ctgctggcgc     360
   tgtcgcgcaa tccgcgcggc atccgcagct cggtggtgat ggcattcgcc cctggactga     420
   accaggcgat gctcgactac gtcgggcggg cgcaggcgct gatcgagctg gacgacaagt     480
   cggcgatcgg ccatctgctc aacgagaccg tcggcaaata cctgccgccg cgcctgaaag     540
   ccagcaacca tcagcacatg gcttcgctgg ccaccggcga atacgagcag gcgcgctttc     600
   acatcgacca ggtgctggcg ctcaacgatc ggggctacct ggcttgcctg gagcggatcc     660
   agagccacgt gcatttcatc aacggcagct gggacgaata caccaccgcc gaggacgccc     720
   gccagttccg cgactacctg ccgcactgca gtttctcgcg ggtggagggc accgggcatt     780
   tcctcgacct ggagtccaag ctggccgcgg tacgcgtgca ccgcgccctg ctcgagcacc     840
   tgctgaagca accggagccg cagcgggcgg aacgcgcggc gggattccac gagatggcca     900
   tcggctacgc ctgaaccctt gacctgcgaa gacccggcct ggccgggctt tgcggttgca     960
   taacgcacgg agtagcacca tgcacgccat cctcatcgcc atcggctcgg ccggcgacgt    1020
   atttcccttc atcggcctgg cccggaccct gaaattgcgc gggcaccgcg tgagcctctg    1080
   caccatcccg gtgtttcgcg acgcggtgga gcagcacggc atcgcgttcg tcccgctgag    1140
   cgacgaactg acctaccgcc ggaccatggg cgatccgcgc ctgtgggacc ccaagacgtc    1200
   cttcggcgtg ctctggcaaa ccatcgccgg gatgatcgag ccggtctacg agtacgtctc    1260
   ggcgcagcgc catgacgaca tcgtggtggt cggctcgctc tgggcgctgg gcgcacgcat    1320
   cgctcacgag aagtacggga ttccctacct gtccgcgcag gtctcgccat cgaccttgtt    1380
   gtcggcgcac ctgccgccgg tacaccccaa gttcaacgtg cccgagcaga tgccgctggc    1440
   gatgcgcaag ctgctctggc gctgcatcga gcgcttcaag ctggatcgca cctgcgcgcc    1500
   ggatatcaac gcggtgcggc gcaaggtcgg cctggagacg ccggtgaagc gcatcttcac    1560
   ccaatggatg cattcgccgc agggcgtggt ctgcctgttc ccggcctggt tcgcgccgcc    1620
   ccagcaggat tggccgcaac ccctgcacat gaccggcttc ccgctgttcg acggcagtat    1680
   cccggggacc ccgctcgacg acgaactgca acgctttctc gatcagggca gccggccgct    1740
   ggtgttcacc cagggctcga ccgaacacct gcagggcgac ttctacgcca tggccctgcg    1800
   cgcgctggaa cgcctcggcg cgcgtgggat cttcctcacc ggcgccggcc aggaaccgct    1860
   gcgcggcttg ccgaaccacg tgctgcagcg cgcctacgcg ccactgggag ccttgctgcc    1920
   atcgtgcgcc gggctggtcc atccgggcgg tatcggcgcc atgagcctgg ccttggcggc    1980
   gggggtgccg caggtgctgc tgccctgcgc ccacgaccag ttcgacaatg ccgaacggct 2040
   ggtccggctc ggctgcggga tgcgcctggg cgtgccattg cgcgagcagg agttgcgcgg 2100
   ggcgctgtgg cgcttgctcg aggacccggc catggcggcg gcctgtcggc gtttcatgga 2160
   attgtcacaa ccgcacagta tcgcttgcgg taaagcggcc caggtggtcg aacgttgtca 2220
   tagggagggg gatgcgcgat ggctgaaggc tgcgtcctga acggtgctgg cataacagat 2280
   agggttgcct ctagagtcga cctgcaggca tgcaagcttg gctgttttgg cggatgagag 2340
   aagattttca gcctgataca gattaaatca gaacgcagaa gcggtctgat aaaacagaat 2400
   ttgcctggcg gcagtagcgc ggtggtccca cctgacccca tgccgaactc agaagtgaaa 2460
   cgccgtagcg ccgatggtag tgtggggtct ccccatgcga gagtagggaa ctgccaggca 2520
   tcaaataaaa cgaaaggctc agtcgaaaga ctgggccttt cgttttatct gttgtttgtc 2580
   ggtgaacgct ctcctgagta ggacaaatcc gccgggagcg gatttgaacg ttgcgaagca 2640
   acggcccgga gggtggcggg caggacgccc gccataaact gccaggcatc aaattaagca 2700
   gaaggccatc ctgacggatg gcctttttgc gtttctacaa actctttttg tttatttttc 2760
   taaatacatt caaatatgta tccgctcatg ctccttcgtc ggtgtcgtcg ccggatggtc 2820
   tgcggtggtg ctcagcgtgg agacgcgcac cgtcacggac ccccatcaat cctgcctatt 2880
   tgccacgttt aacaaggtag ttaagcgttc atttacgaag aaaacacgat aagctgcaca 2940
   aatacctgaa aaagttgaac gccccgtgag cgggaactca cagggcgtcg gctaaccccc 3000
   agtcatcagc tgggagaaag cactcaagac atgactctag ccgatccgca ggacacagtc 3060
   acagctagcg cgtggaaatt gtccgccgat ctgttcgaca cccaccccga agctatgcgc 3120
   tgcggctcac gcggctggac ggcagaagat cgccgcgaac tgctcgctca cctgggacgc 3180
   gaaagcttcc agggcagcaa gacaagagat ttcgcgagcg cctggattaa aaacccggat 3240
   accggcgaaa cccaaccaaa gctctaccgg gctggctcaa aagcgctgac gcggtgccag 3300
   tacgttgcgc tgacgcacgc gcaacatgcc gcggtgatcg tgcttgacat cgatgtgccc 3360
   agccaccagg ccggcgggaa gattgagcac gtaaacccgc aggtctacgc gattttagag 3420
   aaatgggcac gcctagaaaa agcgccggct tggatcggcg tgaatccgct gagcgggaaa 3480
   tgccagctca tctggctcat tgacccggtg tatgccgcag caggtaaaac cagcccaaat 3540
   atgcgcctgc tggctgcaac gacggaagaa atgactcgtg ttttcggcgc tgaccaggct 3600
   ttttcgcata ggctgagccg gtggccgctg cacgtctcag acgatccgac agcctataaa 3660
   tggcactgcc agcatgatcg tgtggatcgg ctggccgacc taatggagat tgctcgaacg 3720
   atgaccggat cacagaagcc gaaaaagtac attgagcagg acttttccag cggacgcgcc 3780
   cgcattgaag cggcacaacg cgccaccgca gaagccaagg cgctagcgat tttggacgcg 3840
   agcctgccga gcgccctgga cgcgtccggc gacctgatcg acggcgtgcg agtgctctgg 3900
   acaaatccag agcgagcgcg cgacgagacc gcgtttcgcc acgcgttgac cgtgggatac 3960
   cagctcaaag ctgctggtga gcgcctaaaa gatgccaaga tcatcgacgc gtatgaagtg 4020
   gcgtacaacg ttgcccaggc ggtcggtgca gacggccggg agccggatct tcccgccatg 4080
   cgtgatcgcc tgacgatggc gcgtcgtgtg cgcggctacg tggctaaagg ccagccagtc 4140
   gtccctgctc gtcgggtgga aacgcagagc agccgagggc ggaaagctct agcgacgatg 4200
   gggcgacggg gcgcagctac atcgaatgca cgcagatggg ctgacccaga aagtaagtat 4260
   gcgcaggaga cgcgacagcg attagcggaa gcaaacaaac gccgagaaat gacaggcgag 4320
   ttgctcgaac ttcgcgtcaa aactgcgatc ctggatgccc gttctcaatc ggttgctgat 4380
   ccctcgactc gtgagcttgc aggcgaacta ggtgtcagtg aaaggcgcat ccaacaagtc 4440
   agaaaggcac ttggaatgga agctaaacgc ggccgtccac gggctgaaaa ctaataaacg 4500
   aaacaccgtc agcagaaaac ggttcccccc tttaggggtc ccgtccttgc tctggctctc 4560
   acttgccctc accctccgct atccacgggc tgaaaactaa taaacgaaac accgtcagca 4620
   gaaaacggtt cccccccttt agggtgtctc gctcctagct ctgatccctc cccggttcct 4680
   ccccggcctg atttttaagg ggggctcacg ctgtcggcag agaacggttc cccgccttct 4740
   gctctggctc ttcctcgact ccctccccct caaaaatctc ctcgagatcc tggagacctt 4800
   tttggagcta gcgcgttgct gcttcgcacc aacttgctca tgatgatttt catttttgct 4860
   tgtgtgcttt tttgggttga accctccaaa gaggggaaac caggggcaca cctcatgcac 4920
   taaagtgccg cttcgctggt cagggtgaaa tcacctggaa aaaaagtgcg gtaaccgctg 4980
   cgcttggcgt tttttctggg caagaagtct cgcaggtttt cgcaggagtg ccggaagaaa 5040
   ttatcagaat tggggctaga atttttaacg aacgttcgtt ataatggtgt catgaccttc 5100
   acgacgaagt accaaaactg gcctgaagca tcagcggtgg atctctccga tgtcgcgctg 5160
   gagtccgacg cactcgatgc cgccgtcgat ttaaaaacgg tgatcggatt tttccgcgcc 5220
   ctcgatacga cagacgcgcc agcatcacgc gactgggcaa gtgccgcgag cgacctagaa 5280
   acgcttgtgg ccgaccttga agagctggcc gacgagctgc gtgctcggca gcgccaggag 5340
   gacgcgcagt agtggaggat cgcatcagct gcgcctactg cggtggcctg atcccacccc 5400
   ggcctgaccc acgaggacgg cgcgcaaaat actgctcaga cgcgtgtcgt gccgcagcca 5460
   gccgcgagcg cgccaacaag cgccacgccc aggaggtcga agccgcacgt cgaccgcgtg 5520
   tagtgcgtgg cggaaacttc ttgcgtttcg caagagaaat gcgtcccatt tctcgtcgga 5580
   ctcggggaag gaagcgtgat gctctcggtc aagcacgtcg ctcgccagcg ctgcgaggag 5640
   ttcggccttc gtgcggaagt gccagtagag gccgggctgc tgtacctgta agtgagccgc 5700
   cagcgcgcga gtggtgaagc catcgagccc agtctcgtcg agcacctgcc gggccccgag 5760
   caacacggac gtgcggtcga gacgcttccg gtggtgagtc atagttgcac tttatcatcg 5820
   ataactttat cttagataaa gtgactgctc gctactctca tctgactgct cgctactctc 5880
   atcgtggaat cctgacagcc gtgctcatca cggcgaccct cgatgctgca gggctgggcc 5940
   tcgtgatgcc gatcttgcct acccttctcg accaggtcgg tgcccccgac gacatgatcc 6000
   cactgcacgt cggactactg acagcgctct atgcgatcat gcagtttctt tgcgccccga 6060
   tccttggccg actctctgac cgtttcggac gccgccgcgt gcttgtcgcc tccctcgcag 6120
   gcgcgacgat cgactacctc gtgctcgcac tgacggacac gctgtgggtc ttttacctcg 6180
   cccgcgcggt tgcaggcatt accggcgcca cgaacgccgt caccgcgacg gtgatcgccg 6240
   acattactcc gccggatcag cgcgcaaaac gctacgggtg gctcggcgca tgctacggcg 6300
   gtggcatgat cgcgggtccc gccattggcg gtcttttcgg cggggtctca ccgcatctgc 6360
   cattcctcgt cgccgccgcg ctcgccggaa tcaccctcgt actcagcgcg agtcttctgc 6420
   gtgagacgcg gccaccgggc agcaacggct cgcacgcaca gcaacccggt acggcgaagc 6480
   gaaccgcagt gccggggatg cttatccttc tcgcagtctt cggcatcgtg cagttcatcg 6540
   gccaagcacc aggctccacc tgggtgctct tcacgcagca gcgcctcgac tggaaccccg 6600
   tcgaagtcgg cgtttcgcta tccatcttcg gaatggtgca agtattcgtg caggcggcac 6660
   tgaccggacg catcgtgtcc cggatcggcg agacccgggc gatcctcgtc ggtatcgccg 6720
   cagacgccat tgggctcatc ggccttgccc tcatcgccag cacatgggcg atgctaccga 6780
   tcctcgcagc gctcggactc ggcagcatca cgttgcccgc actgcagacg ctgctctcga 6840
   gacgcgcgcc cgagcagcag cagggacgcc tgcagggaac acttgcaagc ctgaacagcc 6900
   tcacctcgat catcggcccg gtcaccttca ccggcatttt cgcactcacc cgaacgaatg 6960
   cagacggcac cctctggatc tgcgccgcag cgctctacgt tctctgcgcc ctcctgatga 7020
   tccgtgagac atgcgcctca cggcgatctc gataaccgcg ctaaggtgcc atcccgatgc 7080
   gacgggatcg ctctgccacc agtcaagtct cccgtagccg gtatgagcat gaccaaaatc 7140
   ccttaacgtg agttttcgtt ccactgagcg tcagaccccg tagaaaagat caaaggatct 7200
   tcttgagatc ctttttttct gcgcgtaatc tgctgcttgc aaacaaaaaa accaccgcta 7260
   ccagcggtgg tttgtttgcc ggatcaagag ctaccaactc tttttccgaa ggtaactggc 7320
   ttcagcagag cgcagatacc aaatactgtc cttctagtgt agccgtagtt aggccaccac 7380
   ttcaagaact ctgtagcacc gcctacatac ctcgctctgc taatcctgtt accagtggct 7440
   gctgccagtg gcgataagtc gtgtcttacc gggttggact caagacgata gttaccggat 7500
   aaggcgcagc ggtcgggctg aacggggggt tcgtgcacac agcccagctt ggagcgaacg 7560
   acctacaccg aactgagata cctacagcgt gagctatgag aaagcgccac gcttcccgaa 7620
   gggagaaagg cggacaggta tccggtaagc ggcagggtcg gaacaggaga gcgcacgagg 7680
   gagcttccag ggggaaacgc ctggtatctt tatagtcctg tcgggtttcg ccacctctga 7740
   cttgagcgtc gatttttgtg atgctcgtca ggggggcgga gcctatggaa aaacgccagc 7800
   aacgcggcct ttttacggtt cctggccttt tgctggcctt ttgctcacat gttctttcct 7860
   gcgttatccc ctgattctgt ggataaccgt attaccgcct ttgagtgagc tgataccgct 7920
   cgccgcagcc gaacgaccga gcgcagcgag tcagtgagcg aggaagcgga agagcgcctg 7980
   atgcggtatt ttctccttac gcatctgtgc ggtatttcac accgcatatg gtgcactctc 8040
   agtacaatct gctctgatgc cgcatagtta agccagtata cactccgcta tcgctacgtg 8100
   actgggtcat ggctgcgccc cgacacccgc caacacccgc tgacgcgccc tgacgggctt 8160
   gtctgctccc ggcatccgct tacagacaag ctgtgaccgt ctccgggagc tgcatgtgtc 8220
   agaggttttc accgtcatca ccgaaacgcg cgaggcagca gatcaattcg cgcgcgaagg 8280
   cgaagcggca tgcatttacg ttgacaccat cgaatggtgc aaaacctttc gcggtatggc 8340
   atgatagcgc ccggaagaga gtcaattcag ggtggtgaat gtgaaaccag taacgttata 8400
   cgatgtcgca gagtatgccg gtgtctctta tcagaccgtt tcccgcgtgg tgaaccaggc 8460
   cagccacgtt tctgcgaaaa cgcgggaaaa agtggaagcg gcgatggcgg agctgaatta 8520
   cattcccaac cgcgtggcac aacaactggc gggcaaacag tcgttgctga ttggcgttgc 8580
   cacctccagt ctggccctgc acgcgccgtc gcaaattgtc gcggcgatta aatctcgcgc 8640
   cgatcaactg ggtgccagcg tggtggtgtc gatggtagaa cgaagcggcg tcgaagcctg 8700
   taaagcggcg gtgcacaatc ttctcgcgca acgcgtcagt gggctgatca ttaactatcc 8760
   gctggatgac caggatgcca ttgctgtgga agctgcctgc actaatgttc cggcgttatt 8820
   tcttgatgtc tctgaccaga cacccatcaa cagtattatt ttctcccatg aagacggtac 8880
   gcgactgggc gtggagcatc tggtcgcatt gggtcaccag caaatcgcgc tgttagcggg 8940
   cccattaagt tctgtctcgg cgcgtctgcg tctggctggc tggcataaat atctcactcg 9000
   caatcaaatt cagccgatag cggaacggga aggcgactgg agtgccatgt ccggttttca 9060
   acaaaccatg caaatgctga atgagggcat cgttcccact gcgatgctgg ttgccaacga 9120
   tcagatggcg ctgggcgcaa tgcgcgccat taccgagtcc gggctgcgcg ttggtgcgga 9180
   tatctcggta gtgggatacg acgataccga agacagctca tgttatatcc cgccgtcaac 9240
   caccatcaaa caggattttc gcctgctggg gcaaaccagc gtggaccgct tgctgcaact 9300
   ctctcagggc caggcggtga agggcaatca gctgttgccc gtctcactgg tgaaaagaaa 9360
   aaccaccctg gcgcccaata cgcaaaccgc ctctccccgc gcgttggccg attcattaat 9420
   gcagctggca cgacaggttt cccgactgga aagcgggcag tgagcgcaac gcaattaatg 9480
   tgagttagcg cgaattgatc tggtttgaca gcttatcatc gactgcacgg tgcaccaatg 9540
   cttctggcgt caggcagcca tcggaagctg tggtatggct gtgcaggtcg taaatcactg 9600
   cataattcgt gtcgctcaag gcgcactccc gttctggata atgttttttg cgccgacatc 9660
   ataacggttc tggcaaatat tctgaaatga gctgttgaca attaatcatc cggctcgtat 9720
   aatgtgtgga attgtgagcg gataacaatt tcacacagga aacagaccat ggaattcgag 9780
   ctcggtaccc ggg 9793
<210> 53
   <211> 10780
   <212> DNA
   <213> Artificial Sequence
<220>
   <223> vector
<400> 53
<210> 54
   <211> 11073
   <212> DNA
   <213> Artificial Sequence
<220>
   <223> vector
<400> 54
   gatctggcat ttttgggagg tgtgaaatgc ggcgcgaaag tctgttggta tcggtttgca      60
   agggcctgcg ggtacatgtc gagcgcgttg ggcaggatcc cgggcgcagc acggtgatgc     120
   tggtcaacgg cgcgatggcg accaccgcct cgttcgcccg gacctgcaag tgcctggccg     180
   aacatttcaa cgtggtgctg ttcgacctgc ccttcgccgg gcagtcgcgt cagcacaacc     240
   cgcagcgggg gttgatcacc aaggacgacg aggtggaaat cctcctggcg ctgatcgagc 300
   gcttcgaggt caatcacctg gtctccgcgt cctggggcgg tatctccacg ctgctggcgc 360
   tgtcgcgcaa tccgcgcggc atccgcagct cggtggtgat ggcattcgcc cctggactga 420
   accaggcgat gctcgactac gtcgggcggg cgcaggcgct gatcgagctg gacgacaagt 480
   cggcgatcgg ccatctgctc aacgagaccg tcggcaaata cctgccgccg cgcctgaaag 540
   ccagcaacca tcagcacatg gcttcgctgg ccaccggcga atacgagcag gcgcgctttc 600
   acatcgacca ggtgctggcg ctcaacgatc ggggctacct ggcttgcctg gagcggatcc 660
   agagccacgt gcatttcatc aacggcagct gggacgaata caccaccgcc gaggacgccc 720
   gccagttccg cgactacctg ccgcactgca gtttctcgcg ggtggagggc accgggcatt 780
   tcctcgacct ggagtccaag ctggccgcgg tacgcgtgca ccgcgccctg ctcgagcacc 840
   tgctgaagca accggagccg cagcgggcgg aacgcgcggc gggattccac gagatggcca 900
   tcggctacgc ctgaaccctt gacctgcgaa gacccggcct ggccgggctt tgcggttgca 960
   taacgcacgg agtagcacca tgcacgccat cctcatcgcc atcggctcgg ccggcgacgt 1020
   atttcccttc atcggcctgg cccggaccct gaaattgcgc gggcaccgcg tgagcctctg 1080
   caccatcccg gtgtttcgcg acgcggtgga gcagcacggc atcgcgttcg tcccgctgag 1140
   cgacgaactg acctaccgcc ggaccatggg cgatccgcgc ctgtgggacc ccaagacgtc 1200
   cttcggcgtg ctctggcaaa ccatcgccgg gatgatcgag ccggtctacg agtacgtctc 1260
   ggcgcagcgc catgacgaca tcgtggtggt cggctcgctc tgggcgctgg gcgcacgcat 1320
   cgctcacgag aagtacggga ttccctacct gtccgcgcag gtctcgccat cgaccttgtt 1380
   gtcggcgcac ctgccgccgg tacaccccaa gttcaacgtg cccgagcaga tgccgctggc 1440
   gatgcgcaag ctgctctggc gctgcatcga gcgcttcaag ctggatcgca cctgcgcgcc 1500
   ggatatcaac gcggtgcggc gcaaggtcgg cctggagacg ccggtgaagc gcatcttcac 1560
   ccaatggatg cattcgccgc agggcgtggt ctgcctgttc ccggcctggt tcgcgccgcc 1620
   ccagcaggat tggccgcaac ccctgcacat gaccggcttc ccgctgttcg acggcagtat 1680
   cccggggacc ccgctcgacg acgaactgca acgctttctc gatcagggca gccggccgct 1740
   ggtgttcacc cagggctcga ccgaacacct gcagggcgac ttctacgcca tggccctgcg 1800
   cgcgctggaa cgcctcggcg cgcgtgggat cttcctcacc ggcgccggcc aggaaccgct 1860
   gcgcggcttg ccgaaccacg tgctgcagcg cgcctacgcg ccactgggag ccttgctgcc 1920
   atcgtgcgcc gggctggtcc atccgggcgg tatcggcgcc atgagcctgg ccttggcggc 1980
   gggggtgccg caggtgctgc tgccctgcgc ccacgaccag ttcgacaatg ccgaacggct 2040
   ggtccggctc ggctgcggga tgcgcctggg cgtgccattg cgcgagcagg agttgcgcgg 2100
   ggcgctgtgg cgcttgctcg aggacccggc catggcggcg gcctgtcggc gtttcatgga 2160
   attgtcacaa ccgcacagta tcgcttgcgg taaagcggcc caggtggtcg aacgttgtca 2220
   tagggagggg gatgcgcgat ggctgaaggc tgcgtcctga cgccgggagg atcctggcgt 2280
   gtccacgacc agcctctgcc cctccgccac gcgggaacac ggtcccggcg cgaaacgcgt 2340
   cctgcctctg ctgttcctca cctgcctgct ggatgccgct ggcgtcggcc tgatcgtgcc 2400
   cctgctgccg acgctgatcg gcagcgtggc gccgctggcg gtccgcgacg cggccacctg 2460
   gggcgccgcc ctggtgatga ccttcgcgct gctgcaattg ttcttttcgc cggtcctcgg 2520
   cagcctcagc gaccgcttcg gacgccgccc cgtcctggtc ctggcgatgc tcggcttcgc 2580
   cctcagctat ctgctgctgg cgctggccga cagcctctgg atgctgttcc tcggtcgcgc 2640
   gctggccggg ctcaccggcg ccagcgtggc caccgcgatg gcctgcgcgg ctgacctcgg 2700
   cacgcacggg cagcgcaccc ggcacttcgg ctggctgtac gccggcctcg ccctgggcat 2760
   gatcctcggc cccgccctcg gtgggctgct ggcggtgcac ggcacgacgc tgccgctgtt 2820
   gctggccgcc ggcctgtgcc tgctcaacgc cctgctcgcc ggcctgttcc tcgaggaaac 2880
   cctgcccccg acgcgacgcc gccgcctgga cccgaggcgg atgaatgcct tgcgctcgat 2940
   cagcggcctg gctcggcaac cgggggtcgg acgcctgctg gcggtgcttg ccctggtatt 3000
   cctcggcttg caggcggtga tggtggtctg gccgttcttc gtgatcgaga agtttcactg 3060
   gagcagcgcc tggatcggct actcgctggc cctctacggc gtgctcgcgg tgctcgccca 3120
   gaccctcggc gtgaacctct gcaagcggcg cctggacgac gcccgcctgc tgcgcctggg 3180
   cctcgccctg caaggctgcg gcctgctgct gttcgccctg gtcgactcgt cattctggct 3240
   ggtctgcgcg ctgctgccct tcgcgctcgg cagcctcgcc accccggcca tgcaggggct 3300
   gctctcggcc cgcgtgccgg tcgaccgcca gggcgagttg cagggcgtgc tgagcagcct 3360
   gatgagcctc gccgcgatcg tcggtccgcc gctgatgagc ggcctgttcc actggggcag 3420
   cggtccgctc gcgccgctgc ccctggccgg cgcgccattc ctcgccggcg cccttctcgt 3480
   tctggccggg ctggtcctgg cctggcaact tcgacctacg ggagaagaac gatcatggac 3540
   cggatagaca tgggcgtgct ggtggtactt ctagagtcga cctgcaggca tgcaagcttg 3600
   gctgttttgg cggatgagag aagattttca gcctgataca gattaaatca gaacgcagaa 3660
   gcggtctgat aaaacagaat ttgcctggcg gcagtagcgc ggtggtccca cctgacccca 3720
   tgccgaactc agaagtgaaa cgccgtagcg ccgatggtag tgtggggtct ccccatgcga 3780
   gagtagggaa ctgccaggca tcaaataaaa cgaaaggctc agtcgaaaga ctgggccttt 3840
   cgttttatct gttgtttgtc ggtgaacgct ctcctgagta ggacaaatcc gccgggagcg 3900
   gatttgaacg ttgcgaagca acggcccgga gggtggcggg caggacgccc gccataaact 3960
   gccaggcatc aaattaagca gaaggccatc ctgacggatg gcctttttgc gtttctacaa 4020
   actctttttg tttatttttc taaatacatt caaatatgta tccgctcatg ctccttcgtc 4080
   ggtgtcgtcg ccggatggtc tgcggtggtg ctcagcgtgg agacgcgcac cgtcacggac 4140
   ccccatcaat cctgcctatt tgccacgttt aacaaggtag ttaagcgttc atttacgaag 4200
   aaaacacgat aagctgcaca aatacctgaa aaagttgaac gccccgtgag cgggaactca 4260
   cagggcgtcg gctaaccccc agtcatcagc tgggagaaag cactcaagac atgactctag 4320
   ccgatccgca ggacacagtc acagctagcg cgtggaaatt gtccgccgat ctgttcgaca 4380
   cccaccccga agctatgcgc tgcggctcac gcggctggac ggcagaagat cgccgcgaac 4440
   tgctcgctca cctgggacgc gaaagcttcc agggcagcaa gacaagagat ttcgcgagcg 4500
   cctggattaa aaacccggat accggcgaaa cccaaccaaa gctctaccgg gctggctcaa 4560
   aagcgctgac gcggtgccag tacgttgcgc tgacgcacgc gcaacatgcc gcggtgatcg 4620
   tgcttgacat cgatgtgccc agccaccagg ccggcgggaa gattgagcac gtaaacccgc 4680
   aggtctacgc gattttagag aaatgggcac gcctagaaaa agcgccggct tggatcggcg 4740
   tgaatccgct gagcgggaaa tgccagctca tctggctcat tgacccggtg tatgccgcag 4800
   caggtaaaac cagcccaaat atgcgcctgc tggctgcaac gacggaagaa atgactcgtg 4860
   ttttcggcgc tgaccaggct ttttcgcata ggctgagccg gtggccgctg cacgtctcag 4920
   acgatccgac agcctataaa tggcactgcc agcatgatcg tgtggatcgg ctggccgacc 4980
   taatggagat tgctcgaacg atgaccggat cacagaagcc gaaaaagtac attgagcagg 5040
   acttttccag cggacgcgcc cgcattgaag cggcacaacg cgccaccgca gaagccaagg 5100
   cgctagcgat tttggacgcg agcctgccga gcgccctgga cgcgtccggc gacctgatcg 5160
   acggcgtgcg agtgctctgg acaaatccag agcgagcgcg cgacgagacc gcgtttcgcc 5220
   acgcgttgac cgtgggatac cagctcaaag ctgctggtga gcgcctaaaa gatgccaaga 5280
   tcatcgacgc gtatgaagtg gcgtacaacg ttgcccaggc ggtcggtgca gacggccggg 5340
   agccggatct tcccgccatg cgtgatcgcc tgacgatggc gcgtcgtgtg cgcggctacg 5400
   tggctaaagg ccagccagtc gtccctgctc gtcgggtgga aacgcagagc agccgagggc 5460
   ggaaagctct agcgacgatg ggcgacgggg gcgcagctac atcgaatgca cgcagatggg 5520
   ctgacccaga aagtaagtat gcgcaggaga cgcgacagcg attagcggaa gcaaacaaac 5580
   gccgagaaat gacaggcgag ttgctcgaac ttcgcgtcaa aactgcgatc ctggatgccc 5640
   gttctcaatc ggttgctgat ccctcgactc gtgagcttgc aggcgaacta ggtgtcagtg 5700
   aaaggcgcat ccaacaagtc agaaaggcac ttggaatgga agctaaacgc ggccgtccac 5760
   gggctgaaaa ctaataaacg aaacaccgtc agcagaaaac ggttcccccc tttaggggtc 5820
   ccgtccttgc tctggctctc acttgccctc accctccgct atccacgggc tgaaaactaa 5880
   taaacgaaac accgtcagca gaaaacggtt cccccccttt agggtgtctc gctcctagct 5940
   ctgatccctc cccggttcct ccccggcctg atttttaagg ggggctcacg ctgtcggcag 6000
   agaacggttc cccgccttct gctctggctc ttcctcgact ccctccccct caaaaatctc 6060
   ctcgagatcc tggagacctt tttggagcta gcgcgttgct gcttcgcacc aacttgctca 6120
   tgatgatttt catttttgct tgtgtgcttt tttgggttga accctccaaa gaggggaaac 6180
   caggggcaca cctcatgcac taaagtgccg cttcgctggt cagggtgaaa tcacctggaa 6240
   aaaaagtgcg gtaaccgctg cgcttggcgt tttttctggg caagaagtct cgcaggtttt 6300
   cgcaggagtg ccggaagaaa ttatcagaat tggggctaga atttttaacg aacgttcgtt 6360
   ataatggtgt catgaccttc acgacgaagt accaaaactg gcctgaagca tcagcggtgg 6420
   atctctccga tgtcgcgctg gagtccgacg cactcgatgc cgccgtcgat ttaaaaacgg 6480
   tgatcggatt tttccgcgcc ctcgatacga cagacgcgcc agcatcacgc gactgggcaa 6540
   gtgccgcgag cgacctagaa acgcttgtgg ccgaccttga agagctggcc gacgagctgc 6600
   gtgctcggca gcgccaggag gacgcgcagt agtggaggat cgcatcagct gcgcctactg 6660
   cggtggcctg atcccacccc ggcctgaccc acgaggacgg cgcgcaaaat actgctcaga 6720
   cgcgtgtcgt gccgcagcca gccgcgagcg cgccaacaag cgccacgccc aggaggtcga 6780
   agccgcacgt cgaccgcgtg tagtgcgtgg cggaaacttc ttgcgtttcg caagagaaat 6840
   gcgtcccatt tctcgtcgga ctcggggaag gaagcgtgat gctctcggtc aagcacgtcg 6900
   ctcgccagcg ctgcgaggag ttcggccttc gtgcggaagt gccagtagag gccgggctgc 6960
   tgtacctgta agtgagccgc cagcgcgcga gtggtgaagc catcgagccc agtctcgtcg 7020
   agcacctgcc gggccccgag caacacggac gtgcggtcga gacgcttccg gtggtgagtc 7080
   atagttgcac tttatcatcg ataactttat cttagataaa gtgactgctc gctactctca 7140
   tctgactgct cgctactctc atcgtggaat cctgacagcc gtgctcatca cggcgaccct 7200
   cgatgctgca gggctgggcc tcgtgatgcc gatcttgcct acccttctcg accaggtcgg 7260
   tgcccccgac gacatgatcc cactgcacgt cggactactg acagcgctct atgcgatcat 7320
   gcagtttctt tgcgccccga tccttggccg actctctgac cgtttcggac gccgccgcgt 7380
   gcttgtcgcc tccctcgcag gcgcgacgat cgactacctc gtgctcgcac tgacggacac 7440
   gctgtgggtc ttttacctcg cccgcgcggt tgcaggcatt accggcgcca cgaacgccgt 7500
   caccgcgacg gtgatcgccg acattactcc gccggatcag cgcgcaaaac gctacgggtg 7560
   gctcggcgca tgctacggcg gtggcatgat cgcgggtccc gccattggcg gtcttttcgg 7620
   cggggtctca ccgcatctgc cattcctcgt cgccgccgcg ctcgccggaa tcaccctcgt 7680
   actcagcgcg agtcttctgc gtgagacgcg gccaccgggc agcaacggct cgcacgcaca 7740
   gcaacccggt acggcgaagc gaaccgcagt gccggggatg cttatccttc tcgcagtctt 7800
   cggcatcgtg cagttcatcg gccaagcacc aggctccacc tgggtgctct tcacgcagca 7860
   gcgcctcgac tggaaccccg tcgaagtcgg cgtttcgcta tccatcttcg gaatggtgca 7920
   agtattcgtg caggcggcac tgaccggacg catcgtgtcc cggatcggcg agacccgggc 7980
   gatcctcgtc ggtatcgccg cagacgccat tgggctcatc ggccttgccc tcatcgccag 8040
   cacatgggcg atgctaccga tcctcgcagc gctcggactc ggcagcatca cgttgcccgc 8100
   actgcagacg ctgctctcga gacgcgcgcc cgagcagcag cagggacgcc tgcagggaac 8160
   acttgcaagc ctgaacagcc tcacctcgat catcggcccg gtcaccttca ccggcatttt 8220
   cgcactcacc cgaacgaatg cagacggcac cctctggatc tgcgccgcag cgctctacgt 8280
   tctctgcgcc ctcctgatga tccgtgagac atgcgcctca cggcgatctc gataaccgcg 8340
   ctaaggtgcc atcccgatgc gacgggatcg ctctgccacc agtcaagtct cccgtagccg 8400
   gtatgagcat gaccaaaatc ccttaacgtg agttttcgtt ccactgagcg tcagaccccg 8460
   tagaaaagat caaaggatct tcttgagatc ctttttttct gcgcgtaatc tgctgcttgc 8520
   aaacaaaaaa accaccgcta ccagcggtgg tttgtttgcc ggatcaagag ctaccaactc 8580
   tttttccgaa ggtaactggc ttcagcagag cgcagatacc aaatactgtc cttctagtgt 8640
   agccgtagtt aggccaccac ttcaagaact ctgtagcacc gcctacatac ctcgctctgc 8700
   taatcctgtt accagtggct gctgccagtg gcgataagtc gtgtcttacc gggttggact 8760
   caagacgata gttaccggat aaggcgcagc ggtcgggctg aacggggggt tcgtgcacac 8820
   agcccagctt ggagcgaacg acctacaccg aactgagata cctacagcgt gagctatgag 8880
   aaagcgccac gcttcccgaa gggagaaagg cggacaggta tccggtaagc ggcagggtcg 8940
   gaacaggaga gcgcacgagg gagcttccag ggggaaacgc ctggtatctt tatagtcctg 9000
   tcgggtttcg ccacctctga cttgagcgtc gatttttgtg atgctcgtca ggggggcgga 9060
   gcctatggaa aaacgccagc aacgcggcct ttttacggtt cctggccttt tgctggcctt 9120
   ttgctcacat gttctttcct gcgttatccc ctgattctgt ggataaccgt attaccgcct 9180
   ttgagtgagc tgataccgct cgccgcagcc gaacgaccga gcgcagcgag tcagtgagcg 9240
   aggaagcgga agagcgcctg atgcggtatt ttctccttac gcatctgtgc ggtatttcac 9300
   accgcatatg gtgcactctc agtacaatct gctctgatgc cgcatagtta agccagtata 9360
   cactccgcta tcgctacgtg actgggtcat ggctgcgccc cgacacccgc caacacccgc 9420
   tgacgcgccc tgacgggctt gtctgctccc ggcatccgct tacagacaag ctgtgaccgt 9480
   ctccgggagc tgcatgtgtc agaggttttc accgtcatca ccgaaacgcg cgaggcagca 9540
   gatcaattcg cgcgcgaagg cgaagcggca tgcatttacg ttgacaccat cgaatggtgc 9600
   aaaacctttc gcggtatggc atgatagcgc ccggaagaga gtcaattcag ggtggtgaat 9660
   gtgaaaccag taacgttata cgatgtcgca gagtatgccg gtgtctctta tcagaccgtt 9720
   tcccgcgtgg tgaaccaggc cagccacgtt tctgcgaaaa cgcgggaaaa agtggaagcg 9780
   gcgatggcgg agctgaatta cattcccaac cgcgtggcac aacaactggc gggcaaacag 9840
   tcgttgctga ttggcgttgc cacctccagt ctggccctgc acgcgccgtc gcaaattgtc 9900
   gcggcgatta aatctcgcgc cgatcaactg ggtgccagcg tggtggtgtc gatggtagaa 9960
   cgaagcggcg tcgaagcctg taaagcggcg gtgcacaatc ttctcgcgca acgcgtcagt 10020
   gggctgatca ttaactatcc gctggatgac caggatgcca ttgctgtgga agctgcctgc 10080
   actaatgttc cggcgttatt tcttgatgtc tctgaccaga cacccatcaa cagtattatt 10140
   ttctcccatg aagacggtac gcgactgggc gtggagcatc tggtcgcatt gggtcaccag 10200
   caaatcgcgc tgttagcggg cccattaagt tctgtctcgg cgcgtctgcg tctggctggc 10260
   tggcataaat atctcactcg caatcaaatt cagccgatag cggaacggga aggcgactgg 10320
   agtgccatgt ccggttttca acaaaccatg caaatgctga atgagggcat cgttcccact 10380
   gcgatgctgg ttgccaacga tcagatggcg ctgggcgcaa tgcgcgccat taccgagtcc 10440
   gggctgcgcg ttggtgcgga tatctcggta gtgggatacg acgataccga agacagctca 10500
   tgttatatcc cgccgtcaac caccatcaaa caggattttc gcctgctggg gcaaaccagc 10560
   gtggaccgct tgctgcaact ctctcagggc caggcggtga agggcaatca gctgttgccc 10620
   gtctcactgg tgaaaagaaa aaccaccctg gcgcccaata cgcaaaccgc ctctccccgc 10680
   gcgttggccg attcattaat gcagctggca cgacaggttt cccgactgga aagcgggcag 10740
   tgagcgcaac gcaattaatg tgagttagcg cgaattgatc tggtttgaca gcttatcatc 10800
   gactgcacgg tgcaccaatg cttctggcgt caggcagcca tcggaagctg tggtatggct 10860
   gtgcaggtcg taaatcactg cataattcgt gtcgctcaag gcgcactccc gttctggata 10920
   atgttttttg cgccgacatc ataacggttc tggcaaatat tctgaaatga gctgttgaca 10980
   attaatcatc cggctcgtat aatgtgtgga attgtgagcg gataacaatt tcacacagga 11040
   aacagaccat ggaattcgag ctcggtaccc ggg 11073
<210> 55
   <211> 12263
   <212> DNA
   <213> Artificial Sequence
<220>
   <223> vector
<400> 55
   gatctggcat ttttgggagg tgtgaaatgc ggcgcgaaag tctgttggta tcggtttgca      60
   agggcctgcg ggtacatgtc gagcgcgttg ggcaggatcc cgggcgcagc acggtgatgc     120
   tggtcaacgg cgcgatggcg accaccgcct cgttcgcccg gacctgcaag tgcctggccg     180
   aacatttcaa cgtggtgctg ttcgacctgc ccttcgccgg gcagtcgcgt cagcacaacc     240
   cgcagcgggg gttgatcacc aaggacgacg aggtggaaat cctcctggcg ctgatcgagc     300
   gcttcgaggt caatcacctg gtctccgcgt cctggggcgg tatctccacg ctgctggcgc     360
   tgtcgcgcaa tccgcgcggc atccgcagct cggtggtgat ggcattcgcc cctggactga     420
   accaggcgat gctcgactac gtcgggcggg cgcaggcgct gatcgagctg gacgacaagt     480
   cggcgatcgg ccatctgctc aacgagaccg tcggcaaata cctgccgccg cgcctgaaag     540
   ccagcaacca tcagcacatg gcttcgctgg ccaccggcga atacgagcag gcgcgctttc 600
   acatcgacca ggtgctggcg ctcaacgatc ggggctacct ggcttgcctg gagcggatcc 660
   agagccacgt gcatttcatc aacggcagct gggacgaata caccaccgcc gaggacgccc 720
   gccagttccg cgactacctg ccgcactgca gtttctcgcg ggtggagggc accgggcatt 780
   tcctcgacct ggagtccaag ctggccgcgg tacgcgtgca ccgcgccctg ctcgagcacc 840
   tgctgaagca accggagccg cagcgggcgg aacgcgcggc gggattccac gagatggcca 900
   tcggctacgc ctgaaccctt gacctgcgaa gacccggcct ggccgggctt tgcggttgca 960
   taacgcacgg agtagcacca tgcacgccat cctcatcgcc atcggctcgg ccggcgacgt 1020
   atttcccttc atcggcctgg cccggaccct gaaattgcgc gggcaccgcg tgagcctctg 1080
   caccatcccg gtgtttcgcg acgcggtgga gcagcacggc atcgcgttcg tcccgctgag 1140
   cgacgaactg acctaccgcc ggaccatggg cgatccgcgc ctgtgggacc ccaagacgtc 1200
   cttcggcgtg ctctggcaaa ccatcgccgg gatgatcgag ccggtctacg agtacgtctc 1260
   ggcgcagcgc catgacgaca tcgtggtggt cggctcgctc tgggcgctgg gcgcacgcat 1320
   cgctcacgag aagtacggga ttccctacct gtccgcgcag gtctcgccat cgaccttgtt 1380
   gtcggcgcac ctgccgccgg tacaccccaa gttcaacgtg cccgagcaga tgccgctggc 1440
   gatgcgcaag ctgctctggc gctgcatcga gcgcttcaag ctggatcgca cctgcgcgcc 1500
   ggatatcaac gcggtgcggc gcaaggtcgg cctggagacg ccggtgaagc gcatcttcac 1560
   ccaatggatg cattcgccgc agggcgtggt ctgcctgttc ccggcctggt tcgcgccgcc 1620
   ccagcaggat tggccgcaac ccctgcacat gaccggcttc ccgctgttcg acggcagtat 1680
   cccggggacc ccgctcgacg acgaactgca acgctttctc gatcagggca gccggccgct 1740
   ggtgttcacc cagggctcga ccgaacacct gcagggcgac ttctacgcca tggccctgcg 1800
   cgcgctggaa cgcctcggcg cgcgtgggat cttcctcacc ggcgccggcc aggaaccgct 1860
   gcgcggcttg ccgaaccacg tgctgcagcg cgcctacgcg ccactgggag ccttgctgcc 1920
   atcgtgcgcc gggctggtcc atccgggcgg tatcggcgcc atgagcctgg ccttggcggc 1980
   gggggtgccg caggtgctgc tgccctgcgc ccacgaccag ttcgacaatg ccgaacggct 2040
   ggtccggctc ggctgcggga tgcgcctggg cgtgccattg cgcgagcagg agttgcgcgg 2100
   ggcgctgtgg cgcttgctcg aggacccggc catggcggcg gcctgtcggc gtttcatgga 2160
   attgtcacaa ccgcacagta tcgcttgcgg taaagcggcc caggtggtcg aacgttgtca 2220
   tagggagggg gatgcgcgat ggctgaaggc tgcgtcctga cctacgggag aagaacgatc 2280
   atggaccgga tagacatggg cgtgctggtg gtactgttca atcctggcga cgacgacctg 2340
   gaacaccttg gcgaactggc ggcggcgttt ccgcaactgc gcttccttgc cgtcgacaac 2400
   tcaccgcaca gcgatccgca gcgcaatgcc cggctgcgcg ggcaaggcat cgccgtgctg 2460
   caccacggca accggcaggg catcgccggc gccttcaacc agggactcga cgcgctattc 2520
   cggcgtggcg tgcagggtgt gctgctgctc gaccaggact cccgtcccgg cggcgccttc 2580
   ctcgccgccc agtggcgcaa cctgcaggcg cgcaacggtc aggcctgcct gctcggccca 2640
   cggatcttcg accggggtga ccggcgcttc ctgccggcca tccatctcga cggactgacg 2700
   ctcaggcaat tgtctctgga cggcctgacg accccgcagc gcacctcgtt cctgatctcc 2760
   tccggctgcc tgctgacccg cgaggcctac cagcgcctcg gccacttcga cgaggaactg 2820
   ttcatcgacc acgtggacac cgaatacagc ctgcgcgccc aggcgctgga cgtgcccctg 2880
   tacgtcgacc cgcggctggt cctcgagcac cgcatcggca cgcgcaagac ccgccgcctc 2940
   ggcggtctca gcctcagcgc gatgaaccac gccccgctgc gccgctacta cctggcgcgc 3000
   aacggcctgc tggtcctgcg ccgctacgcc cggtcctcgc cgctggccct gctggcgaac 3060
   ctgccgaccc tgacccaggg cctcgcggtg ctcctgctcg aacgcgacaa gctgctcaag 3120
   ctgcgctgcc tgggctgggg cctgtgggac ggcctgcggg gacgcggcgg cgcgctggag 3180
   accaaccgcc cgcgcctgct gaagcgcctc gccggcccgg ccgtggcgtc cgtagcttcc 3240
   ggcaaggcca aggcctagtc ggcgaaacgc attccctcta gatgagaggc cggcaaggat 3300
   acccgactgg cgcacgggtc gcatcattat gacatcacgc cgcccgccgg cgttgccgcg 3360
   accgttcgtc gaacctgtga attccggtag tttcccttgc cctcgctggc gtcccaagat 3420
   caggatttcc tgtgttcgcc gggaggatcc tggcgtgtcc acgaccagcc tctgcccctc 3480
   cgccacgcgg gaacacggtc ccggcgcgaa acgcgtcctg cctctgctgt tcctcacctg 3540
   cctgctggat gccgctggcg tcggcctgat cgtgcccctg ctgccgacgc tgatcggcag 3600
   cgtggcgccg ctggcggtcc gcgacgcggc cacctggggc gccgccctgg tgatgacctt 3660
   cgcgctgctg caattgttct tttcgccggt cctcggcagc ctcagcgacc gcttcggacg 3720
   ccgccccgtc ctggtcctgg cgatgctcgg cttcgccctc agctatctgc tgctggcgct 3780
   ggccgacagc ctctggatgc tgttcctcgg tcgcgcgctg gccgggctca ccggcgccag 3840
   cgtggccacc gcgatggcct gcgcggctga cctcggcacg cacgggcagc gcacccggca 3900
   cttcggctgg ctgtacgccg gcctcgccct gggcatgatc ctcggccccg ccctcggtgg 3960
   gctgctggcg gtgcacggca cgacgctgcc gctgttgctg gccgccggcc tgtgcctgct 4020
   caacgccctg ctcgccggcc tgttcctcga ggaaaccctg cccccgacgc gacgccgccg 4080
   cctggacccg aggcggatga atgccttgcg ctcgatcagc ggcctggctc ggcaaccggg 4140
   ggtcggacgc ctgctggcgg tgcttgccct ggtattcctc ggcttgcagg cggtgatggt 4200
   ggtctggccg ttcttcgtga tcgagaagtt tcactggagc agcgcctgga tcggctactc 4260
   gctggccctc tacggcgtgc tcgcggtgct cgcccagacc ctcggcgtga acctctgcaa 4320
   gcggcgcctg gacgacgccc gcctgctgcg cctgggcctc gccctgcaag gctgcggcct 4380
   gctgctgttc gccctggtcg actcgtcatt ctggctggtc tgcgcgctgc tgcccttcgc 4440
   gctcggcagc ctcgccaccc cggccatgca ggggctgctc tcggcccgcg tgccggtcga 4500
   ccgccagggc gagttgcagg gcgtgctgag cagcctgatg agcctcgccg cgatcgtcgg 4560
   tccgccgctg atgagcggcc tgttccactg gggcagcggt ccgctcgcgc cgctgcccct 4620
   ggccggcgcg ccattcctcg ccggcgccct tctcgttctg gccgggctgg tcctggcctg 4680
   gcaacttcga cctacgggag aagaacgatc atggaccgga tagacatggg cgtgctggtg 4740
   gtactgttca atcctggcgt ctagagtcga cctgcaggca tgcaagcttg gctgttttgg 4800
   cggatgagag aagattttca gcctgataca gattaaatca gaacgcagaa gcggtctgat 4860
   aaaacagaat ttgcctggcg gcagtagcgc ggtggtccca cctgacccca tgccgaactc 4920
   agaagtgaaa cgccgtagcg ccgatggtag tgtggggtct ccccatgcga gagtagggaa 4980
   ctgccaggca tcaaataaaa cgaaaggctc agtcgaaaga ctgggccttt cgttttatct 5040
   gttgtttgtc ggtgaacgct ctcctgagta ggacaaatcc gccgggagcg gatttgaacg 5100
   ttgcgaagca acggcccgga gggtggcggg caggacgccc gccataaact gccaggcatc 5160
   aaattaagca gaaggccatc ctgacggatg gcctttttgc gtttctacaa actctttttg 5220
   tttatttttc taaatacatt caaatatgta tccgctcatg ctccttcgtc ggtgtcgtcg 5280
   ccggatggtc tgcggtggtg ctcagcgtgg agacgcgcac cgtcacggac ccccatcaat 5340
   cctgcctatt tgccacgttt aacaaggtag ttaagcgttc atttacgaag aaaacacgat 5400
   aagctgcaca aatacctgaa aaagttgaac gccccgtgag cgggaactca cagggcgtcg 5460
   gctaaccccc agtcatcagc tgggagaaag cactcaagac atgactctag ccgatccgca 5520
   ggacacagtc acagctagcg cgtggaaatt gtccgccgat ctgttcgaca cccaccccga 5580
   agctatgcgc tgcggctcac gcggctggac ggcagaagat cgccgcgaac tgctcgctca 5640
   cctgggacgc gaaagcttcc agggcagcaa gacaagagat ttcgcgagcg cctggattaa 5700
   aaacccggat accggcgaaa cccaaccaaa gctctaccgg gctggctcaa aagcgctgac 5760
   gcggtgccag tacgttgcgc tgacgcacgc gcaacatgcc gcggtgatcg tgcttgacat 5820
   cgatgtgccc agccaccagg ccggcgggaa gattgagcac gtaaacccgc aggtctacgc 5880
   gattttagag aaatgggcac gcctagaaaa agcgccggct tggatcggcg tgaatccgct 5940
   gagcgggaaa tgccagctca tctggctcat tgacccggtg tatgccgcag caggtaaaac 6000
   cagcccaaat atgcgcctgc tggctgcaac gacggaagaa atgactcgtg ttttcggcgc 6060
   tgaccaggct ttttcgcata ggctgagccg gtggccgctg cacgtctcag acgatccgac 6120
   agcctataaa tggcactgcc agcatgatcg tgtggatcgg ctggccgacc taatggagat 6180
   tgctcgaacg atgaccggat cacagaagcc gaaaaagtac attgagcagg acttttccag 6240
   cggacgcgcc cgcattgaag cggcacaacg cgccaccgca gaagccaagg cgctagcgat 6300
   tttggacgcg agcctgccga gcgccctgga cgcgtccggc gacctgatcg acggcgtgcg 6360
   agtgctctgg acaaatccag agcgagcgcg cgacgagacc gcgtttcgcc acgcgttgac 6420
   cgtgggatac cagctcaaag ctgctggtga gcgcctaaaa gatgccaaga tcatcgacgc 6480
   gtatgaagtg gcgtacaacg ttgcccaggc ggtcggtgca gacggccggg agccggatct 6540
   tcccgccatg cgtgatcgcc tgacgatggc gcgtcgtgtg cgcggctacg tggctaaagg 6600
   ccagccagtc gtccctgctc gtcgggtgga aacgcagagc agccgagggc ggaaagctct 6660
   agcgacgatg ggcgacgggg gcgcagctac atcgaatgca cgcagatggg ctgacccaga 6720
   aagtaagtat gcgcaggaga cgcgacagcg attagcggaa gcaaacaaac gccgagaaat 6780
   gacaggcgag ttgctcgaac ttcgcgtcaa aactgcgatc ctggatgccc gttctcaatc 6840
   ggttgctgat ccctcgactc gtgagcttgc aggcgaacta ggtgtcagtg aaaggcgcat 6900
   ccaacaagtc agaaaggcac ttggaatgga agctaaacgc ggccgtccac gggctgaaaa 6960
   ctaataaacg aaacaccgtc agcagaaaac ggttcccccc tttaggggtc ccgtccttgc 7020
   tctggctctc acttgccctc accctccgct atccacgggc tgaaaactaa taaacgaaac 7080
   accgtcagca gaaaacggtt cccccccttt agggtgtctc gctcctagct ctgatccctc 7140
   cccggttcct ccccggcctg atttttaagg ggggctcacg ctgtcggcag agaacggttc 7200
   cccgccttct gctctggctc ttcctcgact ccctccccct caaaaatctc ctcgagatcc 7260
   tggagacctt tttggagcta gcgcgttgct gcttcgcacc aacttgctca tgatgatttt 7320
   catttttgct tgtgtgcttt tttgggttga accctccaaa gaggggaaac caggggcaca 7380
   cctcatgcac taaagtgccg cttcgctggt cagggtgaaa tcacctggaa aaaaagtgcg 7440
   gtaaccgctg cgcttggcgt tttttctggg caagaagtct cgcaggtttt cgcaggagtg 7500
   ccggaagaaa ttatcagaat tggggctaga atttttaacg aacgttcgtt ataatggtgt 7560
   catgaccttc acgacgaagt accaaaactg gcctgaagca tcagcggtgg atctctccga 7620
   tgtcgcgctg gagtccgacg cactcgatgc cgccgtcgat ttaaaaacgg tgatcggatt 7680
   tttccgcgcc ctcgatacga cagacgcgcc agcatcacgc gactgggcaa gtgccgcgag 7740
   cgacctagaa acgcttgtgg ccgaccttga agagctggcc gacgagctgc gtgctcggca 7800
   gcgccaggag gacgcgcagt agtggaggat cgcatcagct gcgcctactg cggtggcctg 7860
   atcccacccc ggcctgaccc acgaggacgg cgcgcaaaat actgctcaga cgcgtgtcgt 7920
   gccgcagcca gccgcgagcg cgccaacaag cgccacgccc aggaggtcga agccgcacgt 7980
   cgaccgcgtg tagtgcgtgg cggaaacttc ttgcgtttcg caagagaaat gcgtcccatt 8040
   tctcgtcgga ctcggggaag gaagcgtgat gctctcggtc aagcacgtcg ctcgccagcg 8100
   ctgcgaggag ttcggccttc gtgcggaagt gccagtagag gccgggctgc tgtacctgta 8160
   agtgagccgc cagcgcgcga gtggtgaagc catcgagccc agtctcgtcg agcacctgcc 8220
   gggccccgag caacacggac gtgcggtcga gacgcttccg gtggtgagtc atagttgcac 8280
   tttatcatcg ataactttat cttagataaa gtgactgctc gctactctca tctgactgct 8340
   cgctactctc atcgtggaat cctgacagcc gtgctcatca cggcgaccct cgatgctgca 8400
   gggctgggcc tcgtgatgcc gatcttgcct acccttctcg accaggtcgg tgcccccgac 8460
   gacatgatcc cactgcacgt cggactactg acagcgctct atgcgatcat gcagtttctt 8520
   tgcgccccga tccttggccg actctctgac cgtttcggac gccgccgcgt gcttgtcgcc 8580
   tccctcgcag gcgcgacgat cgactacctc gtgctcgcac tgacggacac gctgtgggtc 8640
   ttttacctcg cccgcgcggt tgcaggcatt accggcgcca cgaacgccgt caccgcgacg 8700
   gtgatcgccg acattactcc gccggatcag cgcgcaaaac gctacgggtg gctcggcgca 8760
   tgctacggcg gtggcatgat cgcgggtccc gccattggcg gtcttttcgg cggggtctca 8820
   ccgcatctgc cattcctcgt cgccgccgcg ctcgccggaa tcaccctcgt actcagcgcg 8880
   agtcttctgc gtgagacgcg gccaccgggc agcaacggct cgcacgcaca gcaacccggt 8940
   acggcgaagc gaaccgcagt gccggggatg cttatccttc tcgcagtctt cggcatcgtg 9000
   cagttcatcg gccaagcacc aggctccacc tgggtgctct tcacgcagca gcgcctcgac 9060
   tggaaccccg tcgaagtcgg cgtttcgcta tccatcttcg gaatggtgca agtattcgtg 9120
   caggcggcac tgaccggacg catcgtgtcc cggatcggcg agacccgggc gatcctcgtc 9180
   ggtatcgccg cagacgccat tgggctcatc ggccttgccc tcatcgccag cacatgggcg 9240
   atgctaccga tcctcgcagc gctcggactc ggcagcatca cgttgcccgc actgcagacg 9300
   ctgctctcga gacgcgcgcc cgagcagcag cagggacgcc tgcagggaac acttgcaagc 9360
   ctgaacagcc tcacctcgat catcggcccg gtcaccttca ccggcatttt cgcactcacc 9420
   cgaacgaatg cagacggcac cctctggatc tgcgccgcag cgctctacgt tctctgcgcc 9480
   ctcctgatga tccgtgagac atgcgcctca cggcgatctc gataaccgcg ctaaggtgcc 9540
   atcccgatgc gacgggatcg ctctgccacc agtcaagtct cccgtagccg gtatgagcat 9600
   gaccaaaatc ccttaacgtg agttttcgtt ccactgagcg tcagaccccg tagaaaagat 9660
   caaaggatct tcttgagatc ctttttttct gcgcgtaatc tgctgcttgc aaacaaaaaa 9720
   accaccgcta ccagcggtgg tttgtttgcc ggatcaagag ctaccaactc tttttccgaa 9780
   ggtaactggc ttcagcagag cgcagatacc aaatactgtc cttctagtgt agccgtagtt 9840
   aggccaccac ttcaagaact ctgtagcacc gcctacatac ctcgctctgc taatcctgtt 9900
   accagtggct gctgccagtg gcgataagtc gtgtcttacc gggttggact caagacgata 9960
   gttaccggat aaggcgcagc ggtcgggctg aacggggggt tcgtgcacac agcccagctt 10020
   ggagcgaacg acctacaccg aactgagata cctacagcgt gagctatgag aaagcgccac 10080
   gcttcccgaa gggagaaagg cggacaggta tccggtaagc ggcagggtcg gaacaggaga 10140
   gcgcacgagg gagcttccag ggggaaacgc ctggtatctt tatagtcctg tcgggtttcg 10200
   ccacctctga cttgagcgtc gatttttgtg atgctcgtca ggggggcgga gcctatggaa 10260
   aaacgccagc aacgcggcct ttttacggtt cctggccttt tgctggcctt ttgctcacat 10320
   gttctttcct gcgttatccc ctgattctgt ggataaccgt attaccgcct ttgagtgagc 10380
   tgataccgct cgccgcagcc gaacgaccga gcgcagcgag tcagtgagcg aggaagcgga 10440
   agagcgcctg atgcggtatt ttctccttac gcatctgtgc ggtatttcac accgcatatg 10500
   gtgcactctc agtacaatct gctctgatgc cgcatagtta agccagtata cactccgcta 10560
   tcgctacgtg actgggtcat ggctgcgccc cgacacccgc caacacccgc tgacgcgccc 10620
   tgacgggctt gtctgctccc ggcatccgct tacagacaag ctgtgaccgt ctccgggagc 10680
   tgcatgtgtc agaggttttc accgtcatca ccgaaacgcg cgaggcagca gatcaattcg 10740
   cgcgcgaagg cgaagcggca tgcatttacg ttgacaccat cgaatggtgc aaaacctttc 10800
   gcggtatggc atgatagcgc ccggaagaga gtcaattcag ggtggtgaat gtgaaaccag 10860
   taacgttata cgatgtcgca gagtatgccg gtgtctctta tcagaccgtt tcccgcgtgg 10920
   tgaaccaggc cagccacgtt tctgcgaaaa cgcgggaaaa agtggaagcg gcgatggcgg 10980
   agctgaatta cattcccaac cgcgtggcac aacaactggc gggcaaacag tcgttgctga 11040
   ttggcgttgc cacctccagt ctggccctgc acgcgccgtc gcaaattgtc gcggcgatta 11100
   aatctcgcgc cgatcaactg ggtgccagcg tggtggtgtc gatggtagaa cgaagcggcg 11160
   tcgaagcctg taaagcggcg gtgcacaatc ttctcgcgca acgcgtcagt gggctgatca 11220
   ttaactatcc gctggatgac caggatgcca ttgctgtgga agctgcctgc actaatgttc 11280
   cggcgttatt tcttgatgtc tctgaccaga cacccatcaa cagtattatt ttctcccatg 11340
   aagacggtac gcgactgggc gtggagcatc tggtcgcatt gggtcaccag caaatcgcgc 11400
   tgttagcggg cccattaagt tctgtctcgg cgcgtctgcg tctggctggc tggcataaat 11460
   atctcactcg caatcaaatt cagccgatag cggaacggga aggcgactgg agtgccatgt 11520
   ccggttttca acaaaccatg caaatgctga atgagggcat cgttcccact gcgatgctgg 11580
   ttgccaacga tcagatggcg ctgggcgcaa tgcgcgccat taccgagtcc gggctgcgcg 11640
   ttggtgcgga tatctcggta gtgggatacg acgataccga agacagctca tgttatatcc 11700
   cgccgtcaac caccatcaaa caggattttc gcctgctggg gcaaaccagc gtggaccgct 11760
   tgctgcaact ctctcagggc caggcggtga agggcaatca gctgttgccc gtctcactgg 11820
   tgaaaagaaa aaccaccctg gcgcccaata cgcaaaccgc ctctccccgc gcgttggccg 11880
   attcattaat gcagctggca cgacaggttt cccgactgga aagcgggcag tgagcgcaac 11940
   gcaattaatg tgagttagcg cgaattgatc tggtttgaca gcttatcatc gactgcacgg 12000
   tgcaccaatg cttctggcgt caggcagcca tcggaagctg tggtatggct gtgcaggtcg 12060
   taaatcactg cataattcgt gtcgctcaag gcgcactccc gttctggata atgttttttg 12120
   cgccgacatc ataacggttc tggcaaatat tctgaaatga gctgttgaca attaatcatc 12180
   cggctcgtat aatgtgtgga attgtgagcg gataacaatt tcacacagga aacagaccat 12240
   ggaattcgag ctcggtaccc ggg 12263
<210> 56
   <211> 8471
   <212> DNA
   <213> Artificial Sequence
<220>
   <223> vector
<400> 56
   aagcttgcat gcctgcaggt cgactctaga ggatccccgg gtaccgagct cgaattcact 60
   ggccgtcgtt ttacagccaa gcttggctgt tttggcggat gagagaagat tttcagcctg 120
   atacagatta aatcagaacg cagaagcggt ctgataaaac agaatttgcc tggcggcagt 180
   agcgcggtgg tcccacctga ccccatgccg aactcagaag tgaaacgccg tagcgccgat 240
   ggtagtgtgg ggtctcccca tgcgagagta gggaactgcc aggcatcaaa taaaacgaaa 300
   ggctcagtcg aaagactggg cctttcgttt tatctgttgt ttgtcggtga acgctctcct 360
   gagtaggaca aatccgccgg gagcggattt gaacgttgcg aagcaacggc ccggagggtg 420
   gcgggcagga cgcccgccat aaactgccag gcatcaaatt aagcagaagg ccatcctgac 480
   ggatggcctt tttgcgtttc tacaaactct tttgtttatt tttctaaata cattcaaata 540
   tgtatccgct catgagacaa taaccctgat aaatgcttca ataatattga aaaaggaaga 600
   gtatgagtat tcaacatttc cgtgtcgccc ttattccctt ttttgcggca ttttgccttc 660
   ctgtttttgc tcacccagaa acgctggtga aagtaaaaga tgctgaagat cagttgggtg 720
   cacgagtggg ttacatcgaa ctggatctca acagcggtaa gatccttgag agttttcgcc 780
   ccgaagaacg ttttccaatg atgagcactt ttgatccccc tgcggcgtcg ctgatcgccc 840
   tcgcgacgtt gtgcgggtgg cttgtccctg agggcgctgc gacagatagc taaaaatctg 900
   cgtcaggatc gccgtagagc gcgcgtcgcg tcgattggag gcttcccctt tggttgacgg 960
   tcttcaatcg ctctacggcg atcctgacgc ttttttgttg cgtaccgtcg atcgttttat 1020
   ttctgtcgat cccgaaaaag tttttgcctt ttgtaaaaaa cttctcggtc gccccgcaaa 1080
   ttttcgattc cagatttttt aaaaaccaag ccagaaatac gacacaccgt ttgcagataa 1140
   tctgtctttc ggaaaaatca agtgcgatac aaaattttta gcacccctga gctgcgcaaa 1200
   gtcccgcttc gtgaaaattt tcgtgccgcg tgattttccg ccaaaaactt taacgaacgt 1260
   tcgttataat ggtgtcatga ccttcacgac gaagtaccaa aattggcccg aatcatcagc 1320
   tatggatctc tctgatgtcg cgctggagtc cgacgcgctc gatgctgccg tcgatttaaa 1380
   aacggtgatc ggatttttcc gagctctcga tacgacggac gcgccagcat cacgagactg 1440
   ggccagtgcc gcgagcgacc tagaaactct cgtggcggat cttgaggagc tggctgacga 1500
   gctgcgtgct cggcagcgcc aggaggacgc acagtagtgg aggatcgaat cagttgcgcc 1560
   tactgcggtg gcctgattcc tccccggcct gacccgcgag gacggcgcgc aaaatattgc 1620
   tcagatgcgt gtcgtgccgc agccagccgc gagcgcgcca acaaacgcca cgccgaggag 1680
   ctggaggcgg ctaggtcgca aatggcgctg gaagtgcgtc ccccgagcga aattttggcc 1740
   atggtcgtca cagagctgga agcggcagcg agaattatcc gcgatcgtgg cgcggtgccc 1800
   gcaggcatga caaacatcgt aaatgccgcg tttcgtgtgg ccgtggccgc ccaggacgtg 1860
   tcagcgccgc caccacctgc accgaatcgg cagcagcgtc gcgcgtcgaa aaagcgcaca 1920
   ggcggcaaga agcgataagc tgcacgaata cctgaaaaat gttgaacgcc ccgtgagcgg 1980
   taactcacag ggcgtcggct aacccccagt ccaaacctgg gagaaagcgc tcaaaaatga 2040
   ctctagcgga ttcacgagac attgacacac cggcctggaa attttccgct gatctgttcg 2100
   acacccatcc cgagctcgcg ctgcgatcac gtggctggac gagcgaagac cgccgcgaat 2160
   tcctcgctca cctgggcaga gaaaatttcc agggcagcaa gacccgcgac ttcgccagcg 2220
   cttggatcaa agacccggac acgggagaaa cacagccgaa gttataccga gttggttcaa 2280
   aatcgcttgc ccggtgccag tatgttgctc tgacgcacgc gcagcacgca gccgtgcttg 2340
   tcctggacat tgatgtgccg agccaccagg ccggcgggaa aatcgagcac gtaaaccccg 2400
   aggtctacgc gattttggag cgctgggcac gcctggaaaa agcgccagct tggatcggcg 2460
   tgaatccact gagcgggaaa tgccagctca tctggctcat tgatccggtg tatgccgcag 2520
   caggcatgag cagcccgaat atgcgcctgc tggctgcaac gaccgaggaa atgacccgcg 2580
   ttttcggcgc tgaccaggct ttttcacata ggctgagccg gtggccactg cacgtctccg 2640
   acgatcccac cgcgtaccgc tggcatgccc agcacaatcg cgtggatcgc ctagctgatc 2700
   ttatggaggt tgctcgcatg atctcaggca cagaaaaacc taaaaaacgc tatgagcagg 2760
   agttttctag cggacgggca cgtatcgaag cggcaagaaa agccactgcg gaagcaaaag 2820
   cacttgccac gcttgaagca agcctgccga gcgccgctga agcgtctgga gagctgatcg 2880
   acggcgtccg tgtcctctgg actgctccag ggcgtgccgc ccgtgatgag acggcttttc 2940
   gccacgcttt gactgtggga taccagttaa aagcggctgg tgagcgccta aaagacacca 3000
   agatcatcga cgcctacgag cgtgcctaca ccgtcgctca ggcggtcgga gcagacggcc 3060
   gtgagcctga tctgccgccg atgcgtgacc gccagacgat ggcgcgacgt gtgcgcggct 3120
   acgtcgctaa aggccagcca gtcgtccctg ctcgtcagac agagacgcag agcagccgag 3180
   ggcgaaaagc tctggccact atgggaagac gtggcggtaa aaaggccgca gaacgctgga 3240
   aagacccaaa cagtgagtac gcccgagcac agcgagaaaa actagctaag tccagtcaac 3300
   gacaagctag gaaagctaaa ggaaatcgct tgaccattgc aggttggttt atgactgttg 3360
   agggagagac tggctcgtgg ccgacaatca atgaagctat gtctgaattt agcgtgtcac 3420
   gtcagaccgt gaatagagca cttaagtctg cgggcattga acttccacga ggacgccgta 3480
   aagcttccca gtaaatgtgc catctcgtag gcagaaaacg gttccccccg taggggtctc 3540
   tctcttggcc tcctttctag gtcgggctga ttgctcttga agctctctag gggggctcac 3600
   accataggca gataacggtt ccccaccggc tcacctcgta agcgcacaag gactgctccc 3660
   aaagatcttc aaagccactg ccgcgactcc gcttcgcgaa gccttgcccc gcggaaattt 3720
   cctccaccga gttcgtgcac acccctatgc caagcttctt tcaccctaaa ttcgagagat 3780
   tggattctta ccgtggaaat tcttcgcaaa aatcgtcccc tgatcgccct tgcgacgttg 3840
   ctcgcggcgg tgccgctggt tgcgcttggc ttgaccgact tgatcctccg gcgttcagcc 3900
   tgtgccacag ccgacaggat ggtgaccacc atttgcccca tatcaccgtc ggtactgatc 3960
   ccgtcgtcaa taaaccgaac cgctacaccc tgagcatcaa actcttttat cagttggatc 4020
   atgtcggcgg tgtcgcggcc aagacggtcg agcttcttca ccagaatgac atcaccttcc 4080
   tccaccttca tcctcagcaa atccagccct tcccgatctg ttgaactgcc ggatgccttg 4140
   tcggtaaaga tgcggttagc ttttacccct gcatctttga gcgctgaggt ctgcctcgtg 4200
   aagaaggtgt tgctgactca taccaggcct gaatcgcccc atcatccagc cagaaagtga 4260
   gggagccacg gttgatgaga gctttgttgt aggtggacca gttggtgatt ttgaactttt 4320
   gctttgccac ggaacggtct gcgttgtcgg gaagatgcgt gatctgatcc ttcaactcag 4380
   caaaagttcg atttattcaa caaagccgcc gtcccgtcaa gtcagcgtaa tgctctgcca 4440
   gtgttacaac caattaacca attctgatta gaaaaactca tcgagcatca aatgaaactg 4500
   caatttattc atatcaggat tatcaatacc atatttttga aaaagccgtt tctgtaatga 4560
   aggagaaaac tcaccgaggc agttccatag gatggcaaga tcctggtatc ggtctgcgat 4620
   tccgactcgt ccaacatcaa tacaacctat taatttcccc tcgtcaaaaa taaggttatc 4680
   aagtgagaaa tcaccatgag tgacgactga atccggtgag aatggcaaaa gcttatgcat 4740
   ttctttccag acttgttcaa caggccagcc attacgctcg tcatcaaaat cactcgcatc 4800
   aaccaaaccg ttattcattc gtgattgcgc ctgagcgaga cgaaatacgc gatcgctgtt 4860
   aaaaggacaa ttacaaacag gaatcgaatg caaccggcgc aggaacactg ccagcgcatc 4920
   aacaatattt tcacctgaat caggatattc ttctaatacc tggaatgctg ttttcccggg 4980
   gatcgcagtg gtgagtaacc atgcatcatc aggagtacgg ataaaatgct tgatggtcgg 5040
   aagaggcata aattccgtca gccagtttag tctgaccatc tcatctgtaa catcattggc 5100
   aacgctacct ttgccatgtt tcagaaacaa ctctggcgca tcgggcttcc catacaatcg 5160
   atagattgtc gcacctgatt gcccgacatt atcgcgagcc catttatacc catataaatc 5220
   agcatccatg ttggaattta atcgcggcct cgagcaagac gtttcccgtt gaatatggct 5280
   cataacaccc cttgtattac tgtttatgta agcagacagt tttattgttc atgatgatat 5340
   atttttatct tgtgcaatgt aacatcagag attttgagac acaacgtggc tttgttgaat 5400
   aaatcgaact tttgctgagt tgaaggatca gatcacgcat cttcccgaca acgcagaccg 5460
   ttccgtggca aagcaaaagt tcaaaatcac caactggtcc acctacaaca aagctctcat 5520
   caaccgtggc tccctcactt tctggctgga tgatggggcg attcaggcct ggtatgagtc 5580
   agcaacacct tcttcacgag gcagacctca gcgctagcgg agtgtatact ggcttactat 5640
   gttggcactg atgagggtgt cagtgaagtg cttcatgtgg caggagaaaa aaggctgcac 5700
   cggtgcgtca gcagaatatg tgatacagga tatattccgc ttcctcgctc actgactcgc 5760
   tacgctcggt cgttcgactg cggcgagcgg aaatggctta cgaacggggc ggagatttcc 5820
   tggaagatgc caggaagata cttaacaggg aagtgagagg gccgcggcaa agccgttttt 5880
   ccataggctc cgcccccctg acaagcatca cgaaatctga cgctcaaatc agtggtggcg 5940
   aaacccgaca ggactataaa gataccaggc gtttccccct ggcggctccc tcgtgcgctc 6000
   tcctgttcct gcctttcggt ttaccggtgt cattccgctg ttatggccgc gtttgtctca 6060
   ttccacgcct gacactcagt tccgggtagg cagttcgctc caagctggac tgtatgcacg 6120
   aaccccccgt tcagtccgac cgctgcgcct tatccggtaa ctatcgtctt gagtccaacc 6180
   cggaaagaca tgcaaaagca ccactggcag cagccactgg taattgattt agaggagtta 6240
   gtcttgaagt catgcgccgg ttaaggctaa actgaaagga caagttttgg tgactgcgct 6300
   cctccaagcc agttacctcg gttcaaagag ttggtagctc agagaacctt cgaaaaaccg 6360
   ccctgcaagg cggttttttc gttttcagag caagagatta cgcgcagacc aaaacgatct 6420
   caagaagatc atcttattaa ggggtctgac gctcagtgga acgaaaactc acgttaaggg 6480
   attttggtca tgagattatc aaaaaggatc ttcacctaga tccttttaaa ttaaaaatga 6540
   agttttaaat caatctaaag tatatatgag taaacttggt ctgacagtta ccaatgctta 6600
   atcagtgagg cacctatctc agcgatctgt ctatttcgtt catccatagt tgcctgactc 6660
   cccgtcgtgt agataactac gatacgggag ggcttaccat ctggccccag tgctgcaatg 6720
   ataccgcgag acccacgctc accggctcca gatttatcag caataaacca gccagccgga 6780
   agggccgagc gcagaagtgg tcctgcaact ttatccgcct ccatccagtc tattaattgt 6840
   tgccgggaag ctagagtaag tagttcgcca gttaatagtt tgcgcaacgt tgttgccatt 6900
   gccgatgata agctgtcaaa catggcctgt cgcttgcggt attcggaatc ttgcacgccc 6960
   tcgctcaagc cttcgtcact ggtcccgcca ccaaacgttt cggcgagaag caggccatta 7020
   tcgccggcat ggcggccgac gcgcggggag aggcggtttg cgtattgggc gccagggtgg 7080
   tttttctttt caccagtgag acgggcaaca gctgattgcc cttcaccgcc tggccctgag 7140
   agagttgcag caagcggtcc acgctggttt gccccagcag gcgaaaatcc tgtttgatgg 7200
   tggttaacgg cgggatataa catgagctgt cttcggtatc gtcgtatccc actaccgaga 7260
   tatccgcacc aacgcgcagc ccggactcgg taatggcgcg cattgcgccc agcgccatct 7320
   gatcgttggc aaccagcatc gcagtgggaa cgatgccctc attcagcatt tgcatggttt 7380
   gttgaaaacc ggacatggca ctccagtcgc cttcccgttc cgctatcggc tgaatttgat 7440
   tgcgagtgag atatttatgc cagccagcca gacgcagacg cgccgagaca gaacttaatg 7500
   ggcccgctaa cagcgcgatt tgctggtgac ccaatgcgac cagatgctcc acgcccagtc 7560
   gcgtaccgtc ttcatgggag aaaataatac tgttgatggg tgtctggtca gagacatcaa 7620
   gaaataacgc cggaacatta gtgcaggcag cttccacagc aatggcatcc tggtcatcca 7680
   gcggatagtt aatgatcagc ccactgacgc gttgcgcgag aagattgtgc accgccgctt 7740
   tacaggcttc gacgccgctt cgttctacca tcgacaccac cacgctggca cccagttgat 7800
   cggcgcgaga tttaatcgcc gcgacaattt gcgacggcgc gtgcagggcc agactggagg 7860
   tggcaacgcc aatcagcaac gactgtttgc ccgccagttg ttgtgccacg cggttgggaa 7920
   tgtaattcag ctccgccatc gccgcttcca ctttttcccg cgttttcgca gaaacgtggc 7980
   tggcctggtt caccacgcgg gaaacggtct gataagagac accggcatac tctgcgacat 8040
   cgtataacgt tactggtttc acattcacca ccctgaattg actctcttcc gggcgctatc 8100
   atgccatacc gcgaaaggtt ttgcaccatt cgatggtgtc aacgtaaatg catgccgctt 8160
   cgccttcgcg cgcgaattgc aagctgatcc gggcttatcg actgcacggt gcaccaatgc 8220
   ttctggcgtc aggcagccat cggaagctgt ggtatggctg tgcaggtcgt aaatcactgc 8280
   ataattcgtg tcgctcaagg cgcactcccg ttctggataa tgttttttgc gccgacatca 8340
   taacggttct ggcaaatatt ctgaaatgag ctgttgacaa ttaatcatcg gctcgtataa 8400
   tgtgtggaat tgtgagcgga taacaatttc acacaggaaa cagaattaaa agatatgacc 8460
   atgattacgc c 8471
<210> 57
   <211> 12311
   <212> DNA
   <213> Artificial Sequence
<220>
   <223> vector
<400> 57
   aagcttgcat gcctgcaggt cgactctaga attaatgcag ctggcacgac aggtttcccg      60
   actggaaagc gggcagtgag cgcaacgcaa ttaatgtgag ttagctcact cattaggcac     120
   cccaggcttt acactttatg cttccggctc gtatgttgtg tggaattgtg agcggataac     180
   aatttcacac aggaaacagc tatgaccatg attacgccaa gcgcgcaatt aaccctcact     240
   aaagggaaca aaagctgggt accgggcccc ccctcgaggt cgacggtatc gataagcttg     300
   atatcgaatt cgcgtcatct gtctacgaca acaccttttg tccaattaga gccaaattat     360
   gattctagta acaggcggag ccggcttcat cggctcaaat ttcgtactgc aatggtgtgc     420
   gcacaatgag gaacccgtcc tcaacctcga cgccctgacc tacgcaggca acctggccaa     480
   cctgcagccg ctggaaggca accctcagca tcgctttgtg caaggcaata tttgcgatgc     540
   tgcgcttctg accaagctgt tcgcagagca ccgcccgcgc gccgtggttc acttcgcggc 600
   ggaatcccat gtagaccgct caatcaccgg ccccgaagcg tttgtcgaaa ccaacgtgat 660
   gggcacgttt cgcttgcttg aagccgcccg ggcgcattgg aatagtttgg aaggtgcaga 720
   gaaggaggcc ttccgtttcc tccatgtctc taccgacgaa gtctacggca cactagggcc 780
   aaacgacccg gcgttcaccg aaaccacgcc gtacgcgccg aacagcccat actccgccag 840
   caaggcagcc agcgaccatc tggtacgctc gtatttccat acctacggca tgccggtact 900
   cactaccaac tgctccaaca attacgggcc gctccacttc ccggaaaaac tgatcccgct 960
   gatgatcgtc aacgcactcg ccggtaaggc gctgcctgtc tatggcgacg gccagcaaat 1020
   ccgcgactgg ctgtatgtcg aagatcactg ctcgggcatc cgtcgcgtac tggaagccgg 1080
   tgcgttcggc gagacgtaca atattggcgg ctggaatgaa aaagccaaca ttgacattgt 1140
   gcgtacactc tgcagccttc tcgacgagat ggcacctgcg gcatcgcgcc aggtaatcaa 1200
   tcagaagacc ggcgagcctg tcgaacagta tgcagaactc atcgcctacg taaccgaccg 1260
   cccaggccat gaccgccgtt atgccatcga tgcacgcaag atcgagcggg agctcggctg 1320
   gaaacctgcc gaaaccttcg agacgggcat tcgaaagaca gtcgcttggt acttggccaa 1380
   ccagaaatgg gtaaaaggtg tcatggacgg cagctaccgt gactgggtgg cacaacaata 1440
   cggggcaaat aaagcgtgaa aatcctgctg ttggggaaaa acgggcaagt aggctgggag 1500
   ctacagcgcg ccttggcgcc gctgggtgag gtcattgcgc tggatcgtca gggggccgag 1560
   ggcttatgtg gcgacttgtc caacctggac ggcttggccg ctacgattcg tcagctggcg 1620
   ccggacgtga tcgtcaacgc tgctgcctac actgcagtgg ataaagctga gagcgatcag 1680
   gcactggctg caatgatcaa tgccgcggct cctgctgtat tagcacgtga aacagcagct 1740
   ttgggcgcct ggttgattca ctattccacc gattatgtat ttgacggcag cggcagtcag 1800
   cgctgggagg aaactgcgcc taccggcccc ctttcggtct acggccggac caagctggaa 1860
   ggcgagcatg ccattctcgc cagcggcgcc aaggccgtgg tactgcgcac cagctgggtg 1920
   tatgctgcgc gcgggcacaa ttttgccaag accatgctgc gcctggcggc ggagcgtgag 1980
   acgttgagcg tggtagcaga ccaatttggc gcacccacgg gcgctgacct gatcgccgac 2040
   gttactgcac acatcctgcg gcaaatcttc aatgggcaag acaaccgtca cctggcaggg 2100
   atttaccact tggctgcgtc cggtgaaacc tcttggcatg gttttgctca gttcgtgctg 2160
   gcgcatgctc aacgcactgg cgtagcgctg aaagtgacag ctgataaggt tgccgcaatc 2220
   agcaccgaag cttatccagt acctgcacca cgtccgcgca actcgcgcct ggcactgggc 2280
   aaactggaaa acacgttcaa tttcaaaatg ccgctttggg agcaaggcgt gcaacgtatg 2340
   ctggacgaaa tccagtaata gggactctca tggctcgtaa aggaattatt ctggccggcg 2400
   gttcgggtac acgcctgcat ccggccacac tttcggtttc gaagcagctg ctgccggtgt 2460
   atgacaaacc gatgatctac tacccgctga gcaccctgct gctcgctggt atccgggaca 2520
   tcctgatcat ttccaccccg caggacaccc cgcgcttcga acagctgctg ggcgatggca 2580
   gccagtgggg cctgaacctg tcatacgcaa tacaaccaag cccggatggc ttggcgcaag 2640
   cgttcaccat cggcgctgac ttcatcggta acgacccttc tgcgttggtt ctcggtgaca 2700
   atattttcta cggccatgac ttccaggcac tgctattgaa cgcagataaa cgtgaatccg 2760
   gtgcttcagt attcgcttat catgttcatg acccagaacg ctatggcgta gcggagtttg 2820
   acgatagcgg tcgcgtattg tcgctggaag aaaaaccggc agttccaaag tctagctatg 2880
   cggtcaccgg cctgtatttc tatgacaatc aggtagtcaa tctggctcgc gagctgaagc 2940
   cttccccacg tggcgagctg gaaatcaccg acctcaacaa cctttacttg cagcagcagc 3000
   agttgcaggt cgaaatcatg ggccgtggct atgcgtggct cgacaccggc acgcacgaca 3060
   gtctgctgga ggctagccag tacatcgcaa ccatggagcg ccgtcagggc ttgaaagtcg 3120
   cctgccctga ggaaatttgc taccgcgctg gctggatcaa cgctgagcaa ctcgagtgcc 3180
   tggctcaacc actgctgaaa aacggttatg gcaagtatct gcagaacttg ctgaaagaga 3240
   aggtgttctg atgcaagcca ttccgctgga tatccccgaa gtcgtgctgt ttacccccaa 3300
   ggtttttggc gacgaacgtg gtttcttcta cgagagcttc aacgcccgtg ttttcagcga 3360
   agtgaccggc ctgcagcccg acttcgtaca agacaaccac tcgcgctcgg taaaaggcgt 3420
   gctccgtggc ctgcactatc agctggcacc tcacgcccag ggcaagctgg tgcgtgtggt 3480
   gcaaggcgaa gtcttcgatg ttgcggtgga tatccgtcgc tcgtccacaa ccttcggtaa 3540
   atgggtaggt gcggtgttgt cggccgagaa caagaaccag ctgtggatcc cgccagggtt 3600
   cgcacacggg ttcgtcacgt tgagtgaaac cgcagagttc ctctacaaga ccaccgactt 3660
   ctactcgccg cagtgcgagc gctgcattgc ctggaatgat ccggcagtgg gtatcgaatg 3720
   gcccatcgac tccgtaccaa gcttgtctgg caaggaccag cttggggtcg cattggctga 3780
   cgccgaactg ttcgactaac ggttttagcg gagaagggct gcggtagcgc agccgaattc 3840
   ctgcagcccg ggggatccac tagttctaga ggatccccgg gtaccgagct cgaattcact 3900
   ggccgtcgtt ttacagccaa gcttggctgt tttggcggat gagagaagat tttcagcctg 3960
   atacagatta aatcagaacg cagaagcggt ctgataaaac agaatttgcc tggcggcagt 4020
   agcgcggtgg tcccacctga ccccatgccg aactcagaag tgaaacgccg tagcgccgat 4080
   ggtagtgtgg ggtctcccca tgcgagagta gggaactgcc aggcatcaaa taaaacgaaa 4140
   ggctcagtcg aaagactggg cctttcgttt tatctgttgt ttgtcggtga acgctctcct 4200
   gagtaggaca aatccgccgg gagcggattt gaacgttgcg aagcaacggc ccggagggtg 4260
   gcgggcagga cgcccgccat aaactgccag gcatcaaatt aagcagaagg ccatcctgac 4320
   ggatggcctt tttgcgtttc tacaaactct tttgtttatt tttctaaata cattcaaata 4380
   tgtatccgct catgagacaa taaccctgat aaatgcttca ataatattga aaaaggaaga 4440
   gtatgagtat tcaacatttc cgtgtcgccc ttattccctt ttttgcggca ttttgccttc 4500
   ctgtttttgc tcacccagaa acgctggtga aagtaaaaga tgctgaagat cagttgggtg 4560
   cacgagtggg ttacatcgaa ctggatctca acagcggtaa gatccttgag agttttcgcc 4620
   ccgaagaacg ttttccaatg atgagcactt ttgatccccc tgcggcgtcg ctgatcgccc 4680
   tcgcgacgtt gtgcgggtgg cttgtccctg agggcgctgc gacagatagc taaaaatctg 4740
   cgtcaggatc gccgtagagc gcgcgtcgcg tcgattggag gcttcccctt tggttgacgg 4800
   tcttcaatcg ctctacggcg atcctgacgc ttttttgttg cgtaccgtcg atcgttttat 4860
   ttctgtcgat cccgaaaaag tttttgcctt ttgtaaaaaa cttctcggtc gccccgcaaa 4920
   ttttcgattc cagatttttt aaaaaccaag ccagaaatac gacacaccgt ttgcagataa 4980
   tctgtctttc ggaaaaatca agtgcgatac aaaattttta gcacccctga gctgcgcaaa 5040
   gtcccgcttc gtgaaaattt tcgtgccgcg tgattttccg ccaaaaactt taacgaacgt 5100
   tcgttataat ggtgtcatga ccttcacgac gaagtaccaa aattggcccg aatcatcagc 5160
   tatggatctc tctgatgtcg cgctggagtc cgacgcgctc gatgctgccg tcgatttaaa 5220
   aacggtgatc ggatttttcc gagctctcga tacgacggac gcgccagcat cacgagactg 5280
   ggccagtgcc gcgagcgacc tagaaactct cgtggcggat cttgaggagc tggctgacga 5340
   gctgcgtgct cggcagcgcc aggaggacgc acagtagtgg aggatcgaat cagttgcgcc 5400
   tactgcggtg gcctgattcc tccccggcct gacccgcgag gacggcgcgc aaaatattgc 5460
   tcagatgcgt gtcgtgccgc agccagccgc gagcgcgcca acaaacgcca cgccgaggag 5520
   ctggaggcgg ctaggtcgca aatggcgctg gaagtgcgtc ccccgagcga aattttggcc 5580
   atggtcgtca cagagctgga agcggcagcg agaattatcc gcgatcgtgg cgcggtgccc 5640
   gcaggcatga caaacatcgt aaatgccgcg tttcgtgtgg ccgtggccgc ccaggacgtg 5700
   tcagcgccgc caccacctgc accgaatcgg cagcagcgtc gcgcgtcgaa aaagcgcaca 5760
   ggcggcaaga agcgataagc tgcacgaata cctgaaaaat gttgaacgcc ccgtgagcgg 5820
   taactcacag ggcgtcggct aacccccagt ccaaacctgg gagaaagcgc tcaaaaatga 5880
   ctctagcgga ttcacgagac attgacacac cggcctggaa attttccgct gatctgttcg 5940
   acacccatcc cgagctcgcg ctgcgatcac gtggctggac gagcgaagac cgccgcgaat 6000
   tcctcgctca cctgggcaga gaaaatttcc agggcagcaa gacccgcgac ttcgccagcg 6060
   cttggatcaa agacccggac acgggagaaa cacagccgaa gttataccga gttggttcaa 6120
   aatcgcttgc ccggtgccag tatgttgctc tgacgcacgc gcagcacgca gccgtgcttg 6180
   tcctggacat tgatgtgccg agccaccagg ccggcgggaa aatcgagcac gtaaaccccg 6240
   aggtctacgc gattttggag cgctgggcac gcctggaaaa agcgccagct tggatcggcg 6300
   tgaatccact gagcgggaaa tgccagctca tctggctcat tgatccggtg tatgccgcag 6360
   caggcatgag cagcccgaat atgcgcctgc tggctgcaac gaccgaggaa atgacccgcg 6420
   ttttcggcgc tgaccaggct ttttcacata ggctgagccg gtggccactg cacgtctccg 6480
   acgatcccac cgcgtaccgc tggcatgccc agcacaatcg cgtggatcgc ctagctgatc 6540
   ttatggaggt tgctcgcatg atctcaggca cagaaaaacc taaaaaacgc tatgagcagg 6600
   agttttctag cggacgggca cgtatcgaag cggcaagaaa agccactgcg gaagcaaaag 6660
   cacttgccac gcttgaagca agcctgccga gcgccgctga agcgtctgga gagctgatcg 6720
   acggcgtccg tgtcctctgg actgctccag ggcgtgccgc ccgtgatgag acggcttttc 6780
   gccacgcttt gactgtggga taccagttaa aagcggctgg tgagcgccta aaagacacca 6840
   agatcatcga cgcctacgag cgtgcctaca ccgtcgctca ggcggtcgga gcagacggcc 6900
   gtgagcctga tctgccgccg atgcgtgacc gccagacgat ggcgcgacgt gtgcgcggct 6960
   acgtcgctaa aggccagcca gtcgtccctg ctcgtcagac agagacgcag agcagccgag 7020
   ggcgaaaagc tctggccact atgggaagac gtggcggtaa aaaggccgca gaacgctgga 7080
   aagacccaaa cagtgagtac gcccgagcac agcgagaaaa actagctaag tccagtcaac 7140
   gacaagctag gaaagctaaa ggaaatcgct tgaccattgc aggttggttt atgactgttg 7200
   agggagagac tggctcgtgg ccgacaatca atgaagctat gtctgaattt agcgtgtcac 7260
   gtcagaccgt gaatagagca cttaagtctg cgggcattga acttccacga ggacgccgta 7320
   aagcttccca gtaaatgtgc catctcgtag gcagaaaacg gttccccccg taggggtctc 7380
   tctcttggcc tcctttctag gtcgggctga ttgctcttga agctctctag gggggctcac 7440
   accataggca gataacggtt ccccaccggc tcacctcgta agcgcacaag gactgctccc 7500
   aaagatcttc aaagccactg ccgcgactcc gcttcgcgaa gccttgcccc gcggaaattt 7560
   cctccaccga gttcgtgcac acccctatgc caagcttctt tcaccctaaa ttcgagagat 7620
   tggattctta ccgtggaaat tcttcgcaaa aatcgtcccc tgatcgccct tgcgacgttg 7680
   ctcgcggcgg tgccgctggt tgcgcttggc ttgaccgact tgatcctccg gcgttcagcc 7740
   tgtgccacag ccgacaggat ggtgaccacc atttgcccca tatcaccgtc ggtactgatc 7800
   ccgtcgtcaa taaaccgaac cgctacaccc tgagcatcaa actcttttat cagttggatc 7860
   atgtcggcgg tgtcgcggcc aagacggtcg agcttcttca ccagaatgac atcaccttcc 7920
   tccaccttca tcctcagcaa atccagccct tcccgatctg ttgaactgcc ggatgccttg 7980
   tcggtaaaga tgcggttagc ttttacccct gcatctttga gcgctgaggt ctgcctcgtg 8040
   aagaaggtgt tgctgactca taccaggcct gaatcgcccc atcatccagc cagaaagtga 8100
   gggagccacg gttgatgaga gctttgttgt aggtggacca gttggtgatt ttgaactttt 8160
   gctttgccac ggaacggtct gcgttgtcgg gaagatgcgt gatctgatcc ttcaactcag 8220
   caaaagttcg atttattcaa caaagccgcc gtcccgtcaa gtcagcgtaa tgctctgcca 8280
   gtgttacaac caattaacca attctgatta gaaaaactca tcgagcatca aatgaaactg 8340
   caatttattc atatcaggat tatcaatacc atatttttga aaaagccgtt tctgtaatga 8400
   aggagaaaac tcaccgaggc agttccatag gatggcaaga tcctggtatc ggtctgcgat 8460
   tccgactcgt ccaacatcaa tacaacctat taatttcccc tcgtcaaaaa taaggttatc 8520
   aagtgagaaa tcaccatgag tgacgactga atccggtgag aatggcaaaa gcttatgcat 8580
   ttctttccag acttgttcaa caggccagcc attacgctcg tcatcaaaat cactcgcatc 8640
   aaccaaaccg ttattcattc gtgattgcgc ctgagcgaga cgaaatacgc gatcgctgtt 8700
   aaaaggacaa ttacaaacag gaatcgaatg caaccggcgc aggaacactg ccagcgcatc 8760
   aacaatattt tcacctgaat caggatattc ttctaatacc tggaatgctg ttttcccggg 8820
   gatcgcagtg gtgagtaacc atgcatcatc aggagtacgg ataaaatgct tgatggtcgg 8880
   aagaggcata aattccgtca gccagtttag tctgaccatc tcatctgtaa catcattggc 8940
   aacgctacct ttgccatgtt tcagaaacaa ctctggcgca tcgggcttcc catacaatcg 9000
   atagattgtc gcacctgatt gcccgacatt atcgcgagcc catttatacc catataaatc 9060
   agcatccatg ttggaattta atcgcggcct cgagcaagac gtttcccgtt gaatatggct 9120
   cataacaccc cttgtattac tgtttatgta agcagacagt tttattgttc atgatgatat 9180
   atttttatct tgtgcaatgt aacatcagag attttgagac acaacgtggc tttgttgaat 9240
   aaatcgaact tttgctgagt tgaaggatca gatcacgcat cttcccgaca acgcagaccg 9300
   ttccgtggca aagcaaaagt tcaaaatcac caactggtcc acctacaaca aagctctcat 9360
   caaccgtggc tccctcactt tctggctgga tgatggggcg attcaggcct ggtatgagtc 9420
   agcaacacct tcttcacgag gcagacctca gcgctagcgg agtgtatact ggcttactat 9480
   gttggcactg atgagggtgt cagtgaagtg cttcatgtgg caggagaaaa aaggctgcac 9540
   cggtgcgtca gcagaatatg tgatacagga tatattccgc ttcctcgctc actgactcgc 9600
   tacgctcggt cgttcgactg cggcgagcgg aaatggctta cgaacggggc ggagatttcc 9660
   tggaagatgc caggaagata cttaacaggg aagtgagagg gccgcggcaa agccgttttt 9720
   ccataggctc cgcccccctg acaagcatca cgaaatctga cgctcaaatc agtggtggcg 9780
   aaacccgaca ggactataaa gataccaggc gtttccccct ggcggctccc tcgtgcgctc 9840
   tcctgttcct gcctttcggt ttaccggtgt cattccgctg ttatggccgc gtttgtctca 9900
   ttccacgcct gacactcagt tccgggtagg cagttcgctc caagctggac tgtatgcacg 9960
   aaccccccgt tcagtccgac cgctgcgcct tatccggtaa ctatcgtctt gagtccaacc 10020
   cggaaagaca tgcaaaagca ccactggcag cagccactgg taattgattt agaggagtta 10080
   gtcttgaagt catgcgccgg ttaaggctaa actgaaagga caagttttgg tgactgcgct 10140
   cctccaagcc agttacctcg gttcaaagag ttggtagctc agagaacctt cgaaaaaccg 10200
   ccctgcaagg cggttttttc gttttcagag caagagatta cgcgcagacc aaaacgatct 10260
   caagaagatc atcttattaa ggggtctgac gctcagtgga acgaaaactc acgttaaggg 10320
   attttggtca tgagattatc aaaaaggatc ttcacctaga tccttttaaa ttaaaaatga 10380
   agttttaaat caatctaaag tatatatgag taaacttggt ctgacagtta ccaatgctta 10440
   atcagtgagg cacctatctc agcgatctgt ctatttcgtt catccatagt tgcctgactc 10500
   cccgtcgtgt agataactac gatacgggag ggcttaccat ctggccccag tgctgcaatg 10560
   ataccgcgag acccacgctc accggctcca gatttatcag caataaacca gccagccgga 10620
   agggccgagc gcagaagtgg tcctgcaact ttatccgcct ccatccagtc tattaattgt 10680
   tgccgggaag ctagagtaag tagttcgcca gttaatagtt tgcgcaacgt tgttgccatt 10740
   gccgatgata agctgtcaaa catggcctgt cgcttgcggt attcggaatc ttgcacgccc 10800
   tcgctcaagc cttcgtcact ggtcccgcca ccaaacgttt cggcgagaag caggccatta 10860
   tcgccggcat ggcggccgac gcgcggggag aggcggtttg cgtattgggc gccagggtgg 10920
   tttttctttt caccagtgag acgggcaaca gctgattgcc cttcaccgcc tggccctgag 10980
   agagttgcag caagcggtcc acgctggttt gccccagcag gcgaaaatcc tgtttgatgg 11040
   tggttaacgg cgggatataa catgagctgt cttcggtatc gtcgtatccc actaccgaga 11100
   tatccgcacc aacgcgcagc ccggactcgg taatggcgcg cattgcgccc agcgccatct 11160
   gatcgttggc aaccagcatc gcagtgggaa cgatgccctc attcagcatt tgcatggttt 11220
   gttgaaaacc ggacatggca ctccagtcgc cttcccgttc cgctatcggc tgaatttgat 11280
   tgcgagtgag atatttatgc cagccagcca gacgcagacg cgccgagaca gaacttaatg 11340
   ggcccgctaa cagcgcgatt tgctggtgac ccaatgcgac cagatgctcc acgcccagtc 11400
   gcgtaccgtc ttcatgggag aaaataatac tgttgatggg tgtctggtca gagacatcaa 11460
   gaaataacgc cggaacatta gtgcaggcag cttccacagc aatggcatcc tggtcatcca 11520
   gcggatagtt aatgatcagc ccactgacgc gttgcgcgag aagattgtgc accgccgctt 11580
   tacaggcttc gacgccgctt cgttctacca tcgacaccac cacgctggca cccagttgat 11640
   cggcgcgaga tttaatcgcc gcgacaattt gcgacggcgc gtgcagggcc agactggagg 11700
   tggcaacgcc aatcagcaac gactgtttgc ccgccagttg ttgtgccacg cggttgggaa 11760
   tgtaattcag ctccgccatc gccgcttcca ctttttcccg cgttttcgca gaaacgtggc 11820
   tggcctggtt caccacgcgg gaaacggtct gataagagac accggcatac tctgcgacat 11880
   cgtataacgt tactggtttc acattcacca ccctgaattg actctcttcc gggcgctatc 11940
   atgccatacc gcgaaaggtt ttgcaccatt cgatggtgtc aacgtaaatg catgccgctt 12000
   cgccttcgcg cgcgaattgc aagctgatcc gggcttatcg actgcacggt gcaccaatgc 12060
   ttctggcgtc aggcagccat cggaagctgt ggtatggctg tgcaggtcgt aaatcactgc 12120
   ataattcgtg tcgctcaagg cgcactcccg ttctggataa tgttttttgc gccgacatca 12180
   taacggttct ggcaaatatt ctgaaatgag ctgttgacaa ttaatcatcg gctcgtataa 12240
   tgtgtggaat tgtgagcgga taacaatttc acacaggaaa cagaattaaa agatatgacc 12300
   atgattacgc c 12311
<210> 58
   <211> 9892
   <212> DNA
   <213> Artificial Sequence
<220>
   <223> vector
<400> 58
   ctcgggccgt ctcttgggct tgatcggcct tcttgcgcat ctcacgcgct cctgcggcgg 60
   cctgtagggc aggctcatac ccctgccgaa ccgcttttgt cagccggtcg gccacggctt 120
   ccggcgtctc aacgcgcttt gagattccca gcttttcggc caatccctgc ggtgcatagg 180
   cgcgtggctc gaccgcttgc gggctgatgg tgacgtggcc cactggtggc cgctccaggg 240
   cctcgtagaa cgcctgaatg cgcgtgtgac gtgccttgct gccctcgatg ccccgttgca 300
   gccctagatc ggccacagcg gccgcaaacg tggtctggtc gcgggtcatc tgcgctttgt 360
   tgccgatgaa ctccttggcc gacagcctgc cgtcctgcgt cagcggcacc acgaacgcgg 420
   tcatgtgcgg gctggtttcg tcacggtgga tgctggccgt cacgatgcga tccgccccgt 480
   acttgtccgc cagccacttg tgcgccttct cgaagaacgc cgcctgctgt tcttggctgg 540
   ccgacttcca ccattccggg ctggccgtca tgacgtactc gaccgccaac acagcgtcct 600
   tgcgccgctt ctctggcagc aactcgcgca gtcggcccat cgcttcatcg gtgctgctgg 660
   ccgcccagtg ctcgttctct ggcgtcctgc tggcgtcagc gttgggcgtc tcgcgctcgc 720
   ggtaggcgtg cttgagactg gccgccacgt tgcccatttt cgccagcttc ttgcatcgca 780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtaaa 2760
   ccagcaatag acataagcgg ctatttaacg accctgccct gaaccgacga ccgggtcgaa 2820
   tttgctttcg aatttctgcc attcatccgc ttattatcac ttattcaggc gtagcaccag 2880
   gcgtttaagg gcaccaataa ctgccttaaa aaaattacgc cccgccctgc cactcatcgc 2940
   agtcggccta ttggttaaaa aatgagctga tttaacaaaa atttaacgcg aattttaaca 3000
   aaatattaac gcttacaatt tccattcgcc attcaggctg cgcaactgtt gggaagggcg 3060
   atcggtgcgg gcctcttcgc tattacgcca gctggcgaaa gggggatgtg ctgcaaggcg 3120
   attaagttgg gtaacgccag ggttttccca gtcacgacgt tgtaaaacga cggccagtga 3180
   gcgcgcgtaa tacgactcac tatagggcga attggagctc caccgcggtg gcggccgctc 3240
   tagacgccag gattgaacag taccaccagc acgcccatgt ctatccggtc catgatcgtt 3300
   cttctcccgt aggtcgaagt tgccaggcca ggaccagccc ggccagaacg agaagggcgc 3360
   cggcgaggaa tggcgcgccg gccaggggca gcggcgcgag cggaccgctg ccccagtgga 3420
   acaggccgct catcagcggc ggaccgacga tcgcggcgag gctcatcagg ctgctcagca 3480
   cgccctgcaa ctcgccctgg cggtcgaccg gcacgcgggc cgagagcagc ccctgcatgg 3540
   ccggggtggc gaggctgccg agcgcgaagg gcagcagcgc gcagaccagc cagaatgacg 3600
   agtcgaccag ggcgaacagc agcaggccgc agccttgcag ggcgaggccc aggcgcagca 3660
   ggcgggcgtc gtccaggcgc cgcttgcaga ggttcacgcc gagggtctgg gcgagcaccg 3720
   cgagcacgcc gtagagggcc agcgagtagc cgatccaggc gctgctccag tgaaacttct 3780
   cgatcacgaa gaacggccag accaccatca ccgcctgcaa gccgaggaat accagggcaa 3840
   gcaccgccag caggcgtccg acccccggtt gccgagccag gccgctgatc gagcgcaagg 3900
   cattcatccg cctcgggtcc aggcggcggc gtcgcgtcgg gggcagggtt tcctcgagga 3960
   acaggccggc gagcagggcg ttgagcaggc acaggccggc ggccagcaac agcggcagcg 4020
   tcgtgccgtg caccgccagc agcccaccga gggcggggcc gaggatcatg cccagggcga 4080
   ggccggcgta cagccagccg aagtgccggg tgcgctgccc gtgcgtgccg aggtcagccg 4140
   cgcaggccat cgcggtggcc acgctggcgc cggtgagccc ggccagcgcg cgaccgagga 4200
   acagcatcca gaggctgtcg gccagcgcca gcagcagata gctgagggcg aagccgagca 4260
   tcgccaggac caggacgggg cggcgtccga agcggtcgct gaggctgccg aggaccggcg 4320
   aaaagaacaa ttgcagcagc gcgaaggtca tcaccagggc ggcgccccag gtggccgcgt 4380
   cgcggaccgc cagcggcgcc acgctgccga tcagcgtcgg cagcaggggc acgatcaggc 4440
   cgacgccagc ggcatccagc aggcaggtga ggaacagcag aggcaggacg cgtttcgcgc 4500
   cgggaccgtg ttcccgcgtg gcggaggggc agaggctggt cgtggacacg ccaggatcct 4560
   cccggcgaac acaggaaatc ctgatcttgg gacgccagcg agggcaaggg aaactaccgg 4620
   aattcacagg ttcgacgaac ggtcgcggca acgccggcgg gcggcgtgat gtcataatga 4680
   tgcgacccgt gcgccagtcg ggtatccttg ccggcctctc atctagaggg aatgcgtttc 4740
   gccgactagg ccttggcctt gccggaagct acggacgcca cggccgggcc ggcgaggcgc 4800
   ttcagcaggc gcgggcggtt ggtctccagc gcgccgccgc gtccccgcag gccgtcccac 4860
   aggccccagc ccaggcagcg cagcttgagc agcttgtcgc gttcgagcag gagcaccgcg 4920
   aggccctggg tcagggtcgg caggttcgcc agcagggcca gcggcgagga ccgggcgtag 4980
   cggcgcagga ccagcaggcc gttgcgcgcc aggtagtagc ggcgcagcgg ggcgtggttc 5040
   atcgcgctga ggctgagacc gccgaggcgg cgggtcttgc gcgtgccgat gcggtgctcg 5100
   aggaccagcc gcgggtcgac gtacaggggc acgtccagcg cctgggcgcg caggctgtat 5160
   tcggtgtcca cgtggtcgat gaacagttcc tcgtcgaagt ggccgaggcg ctggtaggcc 5220
   tcgcgggtca gcaggcagcc ggaggagatc aggaacgagg tgcgctgcgg ggtcgtcagg 5280
   ccgtccagag acaattgcct gagcgtcagt ccgtcgagat ggatggccgg caggaagcgc 5340
   cggtcacccc ggtcgaagat ccgtgggccg agcaggcagg cctgaccgtt gcgcgcctgc 5400
   aggttgcgcc actgggcggc gaggaaggcg ccgccgggac gggagtcctg gtcgagcagc 5460
   agcacaccct gcacgccacg ccggaatagc gcgtcgagtc cctggttgaa ggcgccggcg 5520
   atgccctgcc ggttgccgtg gtgcagcacg gcgatgcctt gcccgcgcag ccgggcattg 5580
   cgctgcggat cgctgtgcgg tgagttgtcg acggcaagga agcgcagttg cggaaacgcc 5640
   gccgccagtt cgccaaggtg ttccaggtcg tcgtcgccag gattgaacag taccaccagc 5700
   acgcccatgt ctatccggtc catgatcgtt cttctcccgt aggtcaggac gcagccttca 5760
   gccatcgcgc atccccctcc ctatgacaac gttcgaccac ctgggccgct ttaccgcaag 5820
   cgatactgtg cggttgtgac aattccatga aacgccgaca ggccgccgcc atggccgggt 5880
   cctcgagcaa gcgccacagc gccccgcgca actcctgctc gcgcaatggc acgcccaggc 5940
   gcatcccgca gccgagccgg accagccgtt cggcattgtc gaactggtcg tgggcgcagg 6000
   gcagcagcac ctgcggcacc cccgccgcca aggccaggct catggcgccg ataccgcccg 6060
   gatggaccag cccggcgcac gatggcagca aggctcccag tggcgcgtag gcgcgctgca 6120
   gcacgtggtt cggcaagccg cgcagcggtt cctggccggc gccggtgagg aagatcccac 6180
   gcgcgccgag gcgttccagc gcgcgcaggg ccatggcgta gaagtcgccc tgcaggtgtt 6240
   cggtcgagcc ctgggtgaac accagcggcc ggctgccctg atcgagaaag cgttgcagtt 6300
   cgtcgtcgag cggggtcccc gggatactgc cgtcgaacag cgggaagccg gtcatgtgca 6360
   ggggttgcgg ccaatcctgc tggggcggcg cgaaccaggc cgggaacagg cagaccacgc 6420
   cctgcggcga atgcatccat tgggtgaaga tgcgcttcac cggcgtctcc aggccgacct 6480
   tgcgccgcac cgcgttgata tccggcgcgc aggtgcgatc cagcttgaag cgctcgatgc 6540
   agcgccagag cagcttgcgc atcgccagcg gcatctgctc gggcacgttg aacttggggt 6600
   gtaccggcgg caggtgcgcc gacaacaagg tcgatggcga gacctgcgcg gacaggtagg 6660
   gaatcccgta cttctcgtga gcgatgcgtg cgcccagcgc ccagagcgag ccgaccacca 6720
   cgatgtcgtc atggcgctgc gccgagacgt actcgtagac cggctcgatc atcccggcga 6780
   tggtttgcca gagcacgccg aaggacgtct tggggtccca caggcgcgga tcgcccatgg 6840
   tccggcggta ggtcagttcg tcgctcagcg ggacgaacgc gatgccgtgc tgctccaccg 6900
   cgtcgcgaaa caccgggatg gtgcagaggc tcacgcggtg cccgcgcaat ttcagggtcc 6960
   gggccaggcc gatgaaggga aatacgtcgc cggccgagcc gatggcgatg aggatggcgt 7020
   gcatggtgct actccgtgcg ttatgcaacc gcaaagcccg gccaggccgg gtcttcgcag 7080
   gtcaagggtt caggcgtagc cgatggccat ctcgtggaat cccgccgcgc gttccgcccg 7140
   ctgcggctcc ggttgcttca gcaggtgctc gagcagggcg cggtgcacgc gtaccgcggc 7200
   cagcttggac tccaggtcga ggaaatgccc ggtgccctcc acccgcgaga aactgcagtg 7260
   cggcaggtag tcgcggaact ggcgggcgtc ctcggcggtg gtgtattcgt cccagctgcc 7320
   gttgatgaaa tgcacgtggc tctggatccg ctccaggcaa gccaggtagc cccgatcgtt 7380
   gagcgccagc acctggtcga tgtgaaagcg cgcctgctcg tattcgccgg tggccagcga 7440
   agccatgtgc tgatggttgc tggctttcag gcgcggcggc aggtatttgc cgacggtctc 7500
   gttgagcaga tggccgatcg ccgacttgtc gtccagctcg atcagcgcct gcgcccgccc 7560
   gacgtagtcg agcatcgcct ggttcagtcc aggggcgaat gccatcacca ccgagctgcg 7620
   gatgccgcgc ggattgcgcg acagcgccag cagcgtggag ataccgcccc aggacgcgga 7680
   gaccaggtga ttgacctcga agcgctcgat cagcgccagg aggatttcca cctcgtcgtc 7740
   cttggtgatc aacccccgct gcgggttgtg ctgacgcgac tgcccggcga agggcaggtc 7800
   gaacagcacc acgttgaaat gttcggccag gcacttgcag gtccgggcga acgaggcggt 7860
   ggtcgccatc gcgccgttga ccagcatcac cgtgctgcgc ccgggatcct gcccaacgcg 7920
   ctcgacatgt acccgcaggc ccttgcaaac cgataccaac agactttcgc gccgcatttc 7980
   acacctccca aaaatgccag atcccccggg ctgcaggaat tcgatatcaa gcttatcgat 8040
   accgtcgacc tcgagggggg gcccggtacc cagcttttgt tccctttagt gagggttaat 8100
   tgcgcgcttg gcgtaatcat ggtcatagct gtttcctgtg tgaaattgtt atccgctcac 8160
   aattccacac aacatacgag ccggaagcat aaagtgtaaa gcctggggtg cctaatgagt 8220
   gagctaactc acattaattg cgttgcgctc actgcccgct ttccagtcgg gaaacctgtc 8280
   gtgccagctg cattaatgaa tcggccaacg cgcggggaga ggcggtttgc gtattgggcg 8340
   catgcataaa aactgttgta attcattaag cattctgccg acatggaagc catcacaaac 8400
   ggcatgatga acctgaatcg ccagcggcat cagcaccttg tcgccttgcg tataatattt 8460
   gcccatgggg gtgggcgaag aactccagca tgagatcccc gcgctggagg atcatccagc 8520
   cggcgtcccg gaaaacgatt ccgaagccca acctttcata gaaggcggcg gtggaatcga 8580
   aatctcgtga tggcaggttg ggcgtcgctt ggtcggtcat ttcgaacccc agagtcccgc 8640
   tcagaagaac tcgtcaagaa ggcgatagaa ggcgatgcgc tgcgaatcgg gagcggcgat 8700
   accgtaaagc acgaggaagc ggtcagccca ttcgccgcca agctcttcag caatatcacg 8760
   ggtagccaac gctatgtcct gatagcggtc cgccacaccc agccggccac agtcgatgaa 8820
   tccagaaaag cggccatttt ccaccatgat attcggcaag caggcatcgc catgggtcac 8880
   gacgagatcc tcgccgtcgg gcatgcgcgc cttgagcctg gcgaacagtt cggctggcgc 8940
   gagcccctga tgctcttcgt ccagatcatc ctgatcgaca agaccggctt ccatccgagt 9000
   acgtgctcgc tcgatgcgat gtttcgcttg gtggtcgaat gggcaggtag ccggatcaag 9060
   cgtatgcagc cgccgcattg catcagccat gatggatact ttctcggcag gagcaaggtg 9120
   agatgacagg agatcctgcc ccggcacttc gcccaatagc agccagtccc ttcccgcttc 9180
   agtgacaacg tcgagcacag ctgcgcaagg aacgcccgtc gtggccagcc acgatagccg 9240
   cgctgcctcg tcctgcagtt cattcagggc accggacagg tcggtcttga caaaaagaac 9300
   cgggcgcccc tgcgctgaca gccggaacac ggcggcatca gagcagccga ttgtctgttg 9360
   tgcccagtca tagccgaata gcctctccac ccaagcggcc ggagaacctg cgtgcaatcc 9420
   atcttgttca atcatgcgaa acgatcctca tcctgtctct tgatcagatc ttgatcccct 9480
   gcgccatcag atccttggcg gcaagaaagc catccagttt actttgcagg gcttcccaac 9540
   cttaccagag ggcgccccag ctggcaattc cggttcgctt gctgtccata aaaccgccca 9600
   gtctagctat cgccatgtaa gcccactgca agctacctgc tttctctttg cgcttgcgtt 9660
   ttcccttgtc cagatagccc agtagctgac attcatccca ggtggcactt ttcggggaaa 9720
   tgtgcgcgcc cgcgttcctg ctggcgctgg gcctgtttct ggcgctggac ttcccgctgt 9780
   tccgtcagca gcttttcgcc cacggccttg atgatcgcgg cggccttggc ctgcatatcc 9840
   cgattcaacg gccccagggc gtccagaacg ggcttcaggc gctcccgaag gt 9892
<210> 59
   <211> 1477
   <212> DNA
   <213> Pseudomonas aeruginosa
<400> 59
   tgagaggccg gcaaggatac ccgactggcg cacgggtcgc atcattatga catcacgccg      60
   cccgccggcg ttgccgcgac cgttcgtcga acctgtgaat tccggtagtt tcccttgccc     120
   tcgctggcgt cccaagatca ggatttcctg tgttcgccgg gaggatcctg gcgtgtccac     180
   gaccagcctc tgcccctccg ccacgcggga acacggtccc ggcgcgaaac gcgtcctgcc     240
   tctgctgttc ctcacctgcc tgctggatgc cgctggcgtc ggcctgatcg tgcccctgct     300
   gccgacgctg atcggcagcg tggcgccgct ggcggtccgc gacgcggcca cctggggcgc     360
   cgccctggtg atgaccttcg cgctgctgca attgttcttt tcgccggtcc tcggcagcct     420
   cagcgaccgc ttcggacgcc gccccgtcct ggtcctggcg atgctcggct tcgccctcag     480
   ctatctgctg ctggcgctgg ccgacagcct ctggatgctg ttcctcggtc gcgcgctggc     540
   cgggctcacc ggcgccagcg tggccaccgc gatggcctgc gcggctgacc tcggcacgca     600
   cgggcagcgc acccggcact tcggctggct gtacgccggc ctcgccctgg gcatgatcct     660
   cggccccgcc ctcggtgggc tgctggcggt gcacggcacg acgctgccgc tgttgctggc     720
   cgccggcctg tgcctgctca acgccctgct cgccggcctg ttcctcgagg aaaccctgcc     780
   cccgacgcga cgccgccgcc tggacccgag gcggatgaat gccttgcgct cgatcagcgg     840
   cctggctcgg caaccggggg tcggacgcct gctggcggtg cttgccctgg tattcctcgg     900
   cttgcaggcg gtgatggtgg tctggccgtt cttcgtgatc gagaagtttc actggagcag     960
   cgcctggatc ggctactcgc tggccctcta cggcgtgctc gcggtgctcg cccagaccct    1020
   cggcgtgaac ctctgcaagc ggcgcctgga cgacgcccgc ctgctgcgcc tgggcctcgc    1080
   cctgcaaggc tgcggcctgc tgctgttcgc cctggtcgac tcgtcattct ggctggtctg    1140
   cgcgctgctg cccttcgcgc tcggcagcct cgccaccccg gccatgcagg ggctgctctc    1200
   ggcccgcgtg ccggtcgacc gccagggcga gttgcagggc gtgctgagca gcctgatgag    1260
   cctcgccgcg atcgtcggtc cgccgctgat gagcggcctg ttccactggg gcagcggtcc    1320
   gctcgcgccg ctgcccctgg ccggcgcgcc attcctcgcc ggcgcccttc tcgttctggc    1380
   cgggctggtc ctggcctggc aacttcgacc tacgggagaa gaacgatcat ggaccggata    1440
   gacatgggcg tgctggtggt actgttcaat cctggcg                             1477
<210> 60
   <211> 32
   <212> DNA
   <213> Artificial Sequence
<220>
   <223> primer
<400> 60
   aggaaatcta gatgagaggc cggcaaggat ac 32
<210> 61
   <211> 32
   <212> DNA
   <213> Artificial Sequence
<220>
   <223> primer
<400> 61
   ccaggttcta gacgccagga ttgaacagta cc 32
<210> 62
   <211> 7332
   <212> DNA
   <213> Artificial Sequence
<220>
   <223> vector
<400> 62
   cgaaaatttt tgggaggtgt gaaatgcggc gcgaaagtct gttggtatcg gtttgcaagg 60
   gcctgcgggt acatgtcgag cgcgttgggc aggatcccgg gcgcagcacg gtgatgctgg 120
   tcaacggcgc gatggcgacc accgcctcgt tcgcccggac ctgcaagtgc ctggccgaac 180
   atttcaacgt ggtgctgttc gacctgccct tcgccgggca gtcgcgtcag cacaacccgc 240
   agcgcgggtt gatcaccaag gacgacgagg tggaaatcct cctggcgctg atcgagcgct 300
   tcgaggtcaa tcacctggtc tccgcgtcct ggggcggtat ctccacgctg ctggcgctgt 360
   cgcgcaatcc gcgcggcatc cgcagctcgg tggtgatggc attcgcccct ggactgaacc 420
   aggcgatgct cgactacgtc gggcgggcgc aggcgctgat cgagctggac gacaagtcgg 480
   cgatcggcca tctgctcaac gagaccgtcg gcaaatacct gccgcagcgc ctgaaagcca 540
   gcaaccatca gcacatggct tcgctggcca ccggcgaata cgagcaggcg cgctttcaca 600
   tcgaccaggt gctggcgctc aacgatcggg gctacttggc ttgcctggag cggatccaga 660
   gccacgtgca tttcatcaac ggcagctggg acgaatacac caccgccgag gacgcccgcc 720
   agttccgcga ctacctgccg cactgcagtt tctcgcgggt ggagggcacc gggcatttcc 780
   tcgacctgga gtccaagctg gcagcggtac gcgtgcaccg cgccctgctc gagcacctgc 840
   tgaagcaacc ggagccgcag cgggcggaac gcgcggcggg attccacgag atggccatcg 900
   gctacgcctg aacccttgac ctgcgaagac ccggcctggc cgggctttgc ggttgcataa 960
   cgcacggagt agccccatgc acgccatcct catcgccatc ggctcggccg gcgacgtatt 1020
   tcccttcatc ggcctggccc ggaccctgaa actgcgcggg caccgcgtga gcctctgcac 1080
   catcccggtg tttcgcgacg cggtggagca gcacggcatc gcgttcgtcc cgctgagcga 1140
   cgaactgacc taccgccgga ccatgggcga tccgcgcctg tgggacccca agacgtcctt 1200
   cggcgtgctc tggcaagcca tcgccgggat gatcgagccg gtctacgagt acgtctcggc 1260
   gcagcgccat gacgacatcg tggtggtcgg ctcgctatgg gcgctgggcg cacgcatcgc 1320
   tcacgagaag tacgggattc cctacctgtc cgcgcaggtc tcgccatcga ccctgttgtc 1380
   ggcgcacctg ccgccggtac accccaagtt caacgtgccc gagcagatgc cgctggcgat 1440
   gcgcaagctg ctctggcgct gcatcgagcg cttcaagctg gatcgcacct gcgcgccgga 1500
   gatcaacgcg gtgcgccgca aggtcggcct ggaaacgccg gtgaagcgca tcttcaccca 1560
   atggatgcat tcgccgcagg gcgtggtctg cctgttcccg gcctggttcg cgccgcccca 1620
   gcaggattgg ccgcaacccc tgcacatgac cggcttcccg ctgttcgacg gcagtatccc 1680
   ggggaccccg ctcgacgacg aactgcaacg ctttctcgat cagggcagcc ggccgctggt 1740
   gttcacccag ggctcgaccg aacacctgca gggcgacttc tacgccatgg ccctgcgcgc 1800
   gctggaacgc ctcggcgcgc gtgggatctt cctcaccggc gccggccagg aaccgctgcg 1860
   cggcttgccg aaccacgtgc tgcagcgcgc ctacgcgcca ctgggagcct tgctgccatc 1920
   gtgcgccggg ctggtccatc cgggcggtat cggcgccatg agcctagcct tggcggcggg 1980
   ggtgccgcag gtgctgctgc cctgtgccca cgaccagttc gacaatgccg aacggctggt 2040
   ccggctcggc tgcgggatgc gcctgggcgt gccgttgcgc gagcaggagt tgcgcggggc 2100
   gctgtggcgc ttgctcgagg acccggccat ggcggcggcc tgtcggcgtt tcatggaatt 2160
   gtcacaaccg cacagtatcg cttgcggtaa agcggcccag gtggtcgaac gttgtcatag 2220
   ggagggggat gctcgatggc tgaaggctgc gtcctgaacg gtctagagcg gccgccaccg 2280
   cggtggagct ccaattcgcc ctatagtgag tcgtattacg cgcgctcact ggccgtcgtt 2340
   ttacaacgtc gtgactggga aaaccctggc gttacccaac ttaatcgcct tgcagcacat 2400
   ccccctttcg ccagctggcg taatagcgaa gaggcccgca ccgatcgccc ttcccaacag 2460
   ttgcgcagcc tgaatggcga atggaaattg taagcgttaa tattttgtta aaattcgcgt 2520
   taaatttttg ttaaatcagc tcatttttta accaataggc cgactgcgat gagtggcagg 2580
   gcggggcgta atttttttaa ggcagttatt ggtgccctta aacgcctggt gctacgcctg 2640
   aataagtgat aataagcgga tgaatggcag aaattcgaaa gcaaattcga cccggtcgtc 2700
   ggttcagggc agggtcgtta aatagccgct tatgtctatt gctggtttac cggtttattg 2760
   actaccggaa gcagtgtgac cgtgtgcttc tcaaatgcct gaggccagtt tgctcaggct 2820
   ctccccgtgg aggtaataat tgacgatatg atcatttatt ctgcctccca gagcctgata 2880
   aaaacggtga atccgttagc gaggtgccgc cggcttccat tcaggtcgag gtggcccggc 2940
   tccatgcacc gcgacgcaac gcggggaggc agacaaggta tagggcggcg aggcggctac 3000
   agccgatagt ctggaacagc gcacttacgg gttgctgcgc aacccaagtg ctaccggcgc 3060
   ggcagcgtga cccgtgtcgg cggctccaac ggctcgccat cgtccagaaa acacggctca 3120
   tcgggcatcg gcaggcgctg ctgcccgcgc cgttcccatt cctccgtttc ggtcaaggct 3180
   ggcaggtctg gttccatgcc cggaatgccg ggctggctgg gcggctcctc gccggggccg 3240
   gtcggtagtt gctgctcgcc cggatacagg gtcgggatgc ggcgcaggtc gccatgcccc 3300
   aacagcgatt cgtcctggtc gtcgtgatca accaccacgg cggcactgaa caccgacagg 3360
   cgcaactggt cgcggggctg gccccacgcc acgcggtcat tgaccacgta ggccgacacg 3420
   gtgccggggc cgttgagctt cacgacggag atccagcgct cggccaccaa gtccttgact 3480
   gcgtattgga ccgtccgcaa agaacgtccg atgagcttgg aaagtgtctt ctggctgacc 3540
   accacggcgt tctggtggcc catctgcgcc acgaggtgat gcagcagcat tgccgccgtg 3600
   ggtttcctcg caataagccc ggcccacgcc tcatgcgctt tgcgttccgt ttgcacccag 3660
   tgaccgggct tgttcttggc ttgaatgccg atttctctgg actgcgtggc catgcttatc 3720
   tccatgcggt agggtgccgc acggttgcgg caccatgcgc aatcagctgc aacttttcgg 3780
   cagcgcgaca acaattatgc gttgcgtaaa agtggcagtc aattacagat tttctttaac 3840
   ctacgcaatg agctattgcg gggggtgccg caatgagctg ttgcgtaccc ccctttttta 3900
   agttgttgat ttttaagtct ttcgcatttc gccctatatc tagttctttg gtgcccaaag 3960
   aagggcaccc ctgcggggtt cccccacgcc ttcggcgcgg ctccccctcc ggcaaaaagt 4020
   ggcccctccg gggcttgttg atcgactgcg cggccttcgg ccttgcccaa ggtggcgctg 4080
   cccccttgga acccccgcac tcgccgccgt gaggctcggg gggcaggcgg gcgggcttcg 4140
   ccttcgactg cccccactcg cataggcttg ggtcgttcca ggcgcgtcaa ggccaagccg 4200
   ctgcgcggtc gctgcgcgag ccttgacccg ccttccactt ggtgtccaac cggcaagcga 4260
   agcgcgcagg ccgcaggccg gaggcttttc cccagagaaa attaaaaaaa ttgatggggc 4320
   aaggccgcag gccgcgcagt tggagccggt gggtatgtgg tcgaaggctg ggtagccggt 4380
   gggcaatccc tgtggtcaag ctcgtgggca ggcgcagcct gtccatcagc ttgtccagca 4440
   gggttgtcca cgggccgagc gaagcgagcc agccggtggc cgctcgcggc catcgtccac 4500
   atatccacgg gctggcaagg gagcgcagcg accgcgcagg gcgaagcccg gagagcaagc 4560
   ccgtagggcg ccgcagccgc cgtaggcggt cacgactttg cgaagcaaag tctagtgagt 4620
   atactcaagc attgagtggc ccgccggagg caccgccttg cgctgccccc gtcgagccgg 4680
   ttggacacca aaagggaggg gcaggcatgg cggcatacgc gatcatgcga tgcaagaagc 4740
   tggcgaaaat gggcaacgtg gcggccagtc tcaagcacgc ctaccgcgag cgcgagacgc 4800
   ccaacgctga cgccagcagg acgccagaga acgagcactg ggcggccagc agcaccgatg 4860
   aagcgatggg ccgactgcgc gagttgctgc cagagaagcg gcgcaaggac gctgtgttgg 4920
   cggtcgagta cgtcatgacg gccagcccgg aatggtggaa gtcggccagc caagaacagc 4980
   aggcggcgtt cttcgagaag gcgcacaagt ggctggcgga caagtacggg gcggatcgca 5040
   tcgtgacggc cagcatccac cgtgacgaaa ccagcccgca catgaccgcg ttcgtggtgc 5100
   cgctgacgca ggacggcagg ctgtcggcca aggagttcat cggcaacaaa gcgcagatga 5160
   cccgcgacca gaccacgttt gcggccgctg tggccgatct agggctgcaa cggggcatcg 5220
   agggcagcaa ggcacgtcac acgcgcattc aggcgttcta cgaggccctg gagcggccac 5280
   cagtgggcca cgtcaccatc agcccgcaag cggtcgagcc acgcgcctat gcaccgcagg 5340
   gattggccga aaagctggga atctcaaagc gcgttgagac gccggaagcc gtggccgacc 5400
   ggctgacaaa agcggttcgg caggggtatg agcctgccct acaggccgcc gcaggagcgc 5460
   gtgagatgcg caagaaggcc gatcaagccc aagagacggc ccgagacctt cgggagcgcc 5520
   tgaagcccgt tctggacgcc ctggggccgt tgaatcggga tatgcaggcc aaggccgccg 5580
   cgatcatcaa ggccgtgggc gaaaagctgc tgacggaaca gcgggaagtc cagcgccaga 5640
   aacaggccca gcgccagcag gaacgcgggc gcgcacattt ccccgaaaag tgccacctgg 5700
   gatgaatgtc agctactggg ctatctggac aagggaaaac gcaagcgcaa agagaaagca 5760
   ggtagcttgc agtgggctta catggcgata gctagactgg gcggttttat ggacagcaag 5820
   cgaaccggaa ttgccagctg gggcgccctc tggtaaggtt gggaagccct gcaaagtaaa 5880
   ctggatggct ttcttgccgc caaggatctg atggcgcagg ggatcaagat ctgatcaaga 5940
   gacaggatga ggatcgtttc gcatgattga acaagatgga ttgcacgcag gttctccggc 6000
   cgcttgggtg gagaggctat tcggctatga ctgggcacaa cagacaatcg gctgctctga 6060
   tgccgccgtg ttccggctgt cagcgcaggg gcgcccggtt ctttttgtca agaccgacct 6120
   gtccggtgcc ctgaatgaac tgcaggacga ggcagcgcgg ctatcgtggc tggccacgac 6180
   gggcgttcct tgcgcagctg tgctcgacgt tgtcactgaa gcgggaaggg actggctgct 6240
   attgggcgaa gtgccggggc aggatctcct gtcatctcac cttgctcctg ccgagaaagt 6300
   atccatcatg gctgatgcaa tgcggcggct gcatacgctt gatccggcta cctgcccatt 6360
   cgaccaccaa gcgaaacatc gcatcgagcg agcacgtact cggatggaag ccggtcttgt 6420
   cgatcaggat gatctggacg aagagcatca ggggctcgcg ccagccgaac tgttcgccag 6480
   gctcaaggcg cgcatgcccg acggcgagga tctcgtcgtg acccatggcg atgcctgctt      6540
   gccgaatatc atggtggaaa atggccgctt ttctggattc atcgactgtg gccggctggg      6600
   tgtggcggac cgctatcagg acatagcgtt ggctacccgt gatattgctg aagagcttgg      6660
   cggcgaatgg gctgaccgct tcctcgtgct ttacggtatc gccgctcccg attcgcagcg      6720
   catcgccttc tatcgccttc ttgacgagtt cttctgagcg ggactctggg gttcgaaatg      6780
   accgaccaag cgacgcccaa cctgccatca cgagatttcg attccaccgc cgccttctat      6840
   gaaaggttgg gcttcggaat cgttttccgg gacgccggct ggatgatcct ccagcgcggg      6900
   gatctcatgc tggagttctt cgcccacccc catgggcaaa tattatacgc aaggcgacaa      6960
   ggtgctgatg ccgctggcga ttcaggttca tcatgccgtt tgtgatggct tccatgtcgg      7020
   cagaatgctt aatgaattac aacagttttt atgcatgcgc ccaatacgca aaccgcctct      7080
   ccccgcgcgt tggccgattc attaatgcag ctggcacgac aggtttcccg actggaaagc      7140
   gggcagtgag cgcaacgcaa ttaatgtgag ttagctcact cattaggcac cccaggcttt      7200
   acactttatg cttccggctc gtatgttgtg tggaattgtg agcggataac aatttcacac      7260
   aggaaacagc tatgaccatg attacgccaa gcgcgcaatt aaccctcact aaagggaaca      7320
   aaagctgggt ac                                                          7332
<210> 63
   <211> 7354
   <212> DNA
   <213> Artificial Sequence
<220>
   <223> vector
<400> 63
   cgaattcaaa actttttggg aggtgtgaga tgcggcgcga aagtctgttg gtaacggtat        60
   gcaagggcct gcgggtacat gtcgagcgcg tggggcagga tcccgggcgc gacacggtga       120
   tgctggtcaa cggcgcgatg gcgaccaccg cctcgttcgc ccggacctgc aagtgcctgg       180
   ccgaacattt caacgtggtg ctgttcgacc tgcccttcgc cgggcagtcg cggcagcaca       240
   atccgcagcg cgggttgatc accaaggacg acgaggtgga gattctcctg gcgctgatcg       300
   agcgcttcgc tgtcaaccac ctggtctcgg cctcctgggg cggcatctcc acgctgctgg       360
   cgctgtcgcg caacccgcgc ggggtccgca gctcggtggt gatggcgttc gcgccggggc       420
   tgaaccaggc gatgctcgat tatgtcgggc gggcccagga actgatcgaa ctggacgaca       480
   agtcggcgat cggccacctg ctcaacgaga ccgtcggcaa gtacctgccg ccgcggctga       540
   aggccagcaa ccatcagcac atggcctccc tggccactgg cgagtacgag caggcgcgtt       600
   tccacatcga ccaggtgctg gcgctcaatg accgtggcta cctgagctgc ctggggcaga       660
   tccagagtca cgtgcatttc atcaacggca gctgggacga gtacaccacc gccgaggacg       720
   cccgccagtt ccgcgattac ctgccgcatt gcagtttttc gcgggtggaa ggcaccgggc 780
   acttcctcga cctggagtcc aagctggcgg cggcgcgtgt gcaccgggcg ttgctcgagc 840
   acctgctggc gcaaccggaa ccgtggcgct ccgagcaggc ggcgggattc cacgagatgg 900
   ccatcggcta cgcctgaccc gtcgggatct gcgaaggccc ggcatggccg ggccttgccg 960
   ttgcacaacg caaggagtag ccccatgcac gccattctca tcgccatcgg ttcggccggc 1020
   gacgtgttcc ccttcatcgg cctggcccgc accctgaagt tgcgcggcca ccgcgtcagc 1080
   ctgtgcacca ttccggtgtt tcgcgccgcg gtggagcagc acggcatcga gttcgtcccg 1140
   ctcagcgacg aactgaccta ccgccggacc atgggcgacc cgcgcctgtg ggatccgaag 1200
   acctcgttcg gagtgctctg gcaggccatc gccgggatga tcgagccggt ctacgagtac 1260
   gtctgcgcac agcgccacga cgacatcgtg gtggtcggtt cgctgtgggc cctgggcgcg 1320
   cggatcgccc atgagaaata cgggattccc tacctgtcgg tgcaggtctc gccgtcgacc 1380
   ctgctgtcgg cgcacctgcc gccggtccac cccaggttca acgtgcccga gcaggtcccg 1440
   ctggcgatgc gcaagttgct ctggcgctgc atcgaacgct tcaagctgga ccgcacctgc 1500
   gccccggaga tcaacgcggt gcgccgcaag gtcggcctgg tcggcccggc gaagcgcatc 1560
   ttcacccagt ggatgcattc gccacaggga gtgctctgcc tgttcccggc ctggttcgca 1620
   ccgccccagc aggactggcc gcaaccgctg cacatgaccg gcttcccgct gttcgacggc 1680
   agcgtcccgg ggacccgcct cgacgacgag ttgcagcgct tcctcgagca gggcagtcgg 1740
   ccgctggtgt tcacccaggg ttcgaccgag cacctgcagg gagacttcta tgccatggcc 1800
   ttgcgcgcgc tggagcgtct cggcgcccgc ggcatcttcc tcaccggcgc cggccaggag 1860
   ccgctgcgtg gcttgccgag ccacgtgctg caacgctcgt acgtgccgtt gggggccttg 1920
   ctgccggcgt gcgccgggct ggtccacccg gccggcatcg gcgccatgag cctggcgctg 1980
   gcggcggggg tgccgcaggt gctgctgcct tgcgcccacg accagttcga caacgccgaa 2040
   cgcctggtcc gcctcggctg cggtatccgc ctgggcctgc cgctacgcga gcaggcgctg 2100
   cgcgagtcgc tctggcggct gctcgaggac ccggcgctgg cggcggcctg tcggcgtttc 2160
   atggaattgt cacaaccgca cagtatcgct tgcggtaaag cggcccaagt ggtcgaacgt 2220
   tgtcataggg agggggatgt gcgatggctg aaagccgcgt cctgagccgt gctggcagaa 2280
   ttctctagag cggccgccac cgcggtggag ctccaattcg ccctatagtg agtcgtatta 2340
   cgcgcgctca ctggccgtcg ttttacaacg tcgtgactgg gaaaaccctg gcgttaccca 2400
   acttaatcgc cttgcagcac atcccccttt cgccagctgg cgtaatagcg aagaggcccg 2460
   caccgatcgc ccttcccaac agttgcgcag cctgaatggc gaatggaaat tgtaagcgtt 2520
   aatattttgt taaaattcgc gttaaatttt tgttaaatca gctcattttt taaccaatag 2580
   gccgactgcg atgagtggca gggcggggcg taattttttt aaggcagtta ttggtgccct 2640
   taaacgcctg gtgctacgcc tgaataagtg ataataagcg gatgaatggc agaaattcga 2700
   aagcaaattc gacccggtcg tcggttcagg gcagggtcgt taaatagccg cttatgtcta 2760
   ttgctggttt accggtttat tgactaccgg aagcagtgtg accgtgtgct tctcaaatgc 2820
   ctgaggccag tttgctcagg ctctccccgt ggaggtaata attgacgata tgatcattta 2880
   ttctgcctcc cagagcctga taaaaacggt gaatccgtta gcgaggtgcc gccggcttcc 2940
   attcaggtcg aggtggcccg gctccatgca ccgcgacgca acgcggggag gcagacaagg 3000
   tatagggcgg cgaggcggct acagccgata gtctggaaca gcgcacttac gggttgctgc 3060
   gcaacccaag tgctaccggc gcggcagcgt gacccgtgtc ggcggctcca acggctcgcc 3120
   atcgtccaga aaacacggct catcgggcat cggcaggcgc tgctgcccgc gccgttccca 3180
   ttcctccgtt tcggtcaagg ctggcaggtc tggttccatg cccggaatgc cgggctggct 3240
   gggcggctcc tcgccggggc cggtcggtag ttgctgctcg cccggataca gggtcgggat 3300
   gcggcgcagg tcgccatgcc ccaacagcga ttcgtcctgg tcgtcgtgat caaccaccac 3360
   ggcggcactg aacaccgaca ggcgcaactg gtcgcggggc tggccccacg ccacgcggtc 3420
   attgaccacg taggccgaca cggtgccggg gccgttgagc ttcacgacgg agatccagcg 3480
   ctcggccacc aagtccttga ctgcgtattg gaccgtccgc aaagaacgtc cgatgagctt 3540
   ggaaagtgtc ttctggctga ccaccacggc gttctggtgg cccatctgcg ccacgaggtg 3600
   atgcagcagc attgccgccg tgggtttcct cgcaataagc ccggcccacg cctcatgcgc 3660
   tttgcgttcc gtttgcaccc agtgaccggg cttgttcttg gcttgaatgc cgatttctct 3720
   ggactgcgtg gccatgctta tctccatgcg gtagggtgcc gcacggttgc ggcaccatgc 3780
   gcaatcagct gcaacttttc ggcagcgcga caacaattat gcgttgcgta aaagtggcag 3840
   tcaattacag attttcttta acctacgcaa tgagctattg cggggggtgc cgcaatgagc 3900
   tgttgcgtac cccccttttt taagttgttg atttttaagt ctttcgcatt tcgccctata 3960
   tctagttctt tggtgcccaa agaagggcac ccctgcgggg ttcccccacg ccttcggcgc 4020
   ggctccccct ccggcaaaaa gtggcccctc cggggcttgt tgatcgactg cgcggccttc 4080
   ggccttgccc aaggtggcgc tgcccccttg gaacccccgc actcgccgcc gtgaggctcg 4140
   gggggcaggc gggcgggctt cgccttcgac tgcccccact cgcataggct tgggtcgttc 4200
   caggcgcgtc aaggccaagc cgctgcgcgg tcgctgcgcg agccttgacc cgccttccac 4260
   ttggtgtcca accggcaagc gaagcgcgca ggccgcaggc cggaggcttt tccccagaga 4320
   aaattaaaaa aattgatggg gcaaggccgc aggccgcgca gttggagccg gtgggtatgt 4380
   ggtcgaaggc tgggtagccg gtgggcaatc cctgtggtca agctcgtggg caggcgcagc 4440
   ctgtccatca gcttgtccag cagggttgtc cacgggccga gcgaagcgag ccagccggtg 4500
   gccgctcgcg gccatcgtcc acatatccac gggctggcaa gggagcgcag cgaccgcgca 4560
   gggcgaagcc cggagagcaa gcccgtaggg cgccgcagcc gccgtaggcg gtcacgactt 4620
   tgcgaagcaa agtctagtga gtatactcaa gcattgagtg gcccgccgga ggcaccgcct 4680
   tgcgctgccc ccgtcgagcc ggttggacac caaaagggag gggcaggcat ggcggcatac 4740
   gcgatcatgc gatgcaagaa gctggcgaaa atgggcaacg tggcggccag tctcaagcac 4800
   gcctaccgcg agcgcgagac gcccaacgct gacgccagca ggacgccaga gaacgagcac 4860
   tgggcggcca gcagcaccga tgaagcgatg ggccgactgc gcgagttgct gccagagaag 4920
   cggcgcaagg acgctgtgtt ggcggtcgag tacgtcatga cggccagccc ggaatggtgg 4980
   aagtcggcca gccaagaaca gcaggcggcg ttcttcgaga aggcgcacaa gtggctggcg 5040
   gacaagtacg gggcggatcg catcgtgacg gccagcatcc accgtgacga aaccagcccg 5100
   cacatgaccg cgttcgtggt gccgctgacg caggacggca ggctgtcggc caaggagttc 5160
   atcggcaaca aagcgcagat gacccgcgac cagaccacgt ttgcggccgc tgtggccgat 5220
   ctagggctgc aacggggcat cgagggcagc aaggcacgtc acacgcgcat tcaggcgttc 5280
   tacgaggccc tggagcggcc accagtgggc cacgtcacca tcagcccgca agcggtcgag 5340
   ccacgcgcct atgcaccgca gggattggcc gaaaagctgg gaatctcaaa gcgcgttgag 5400
   acgccggaag ccgtggccga ccggctgaca aaagcggttc ggcaggggta tgagcctgcc 5460
   ctacaggccg ccgcaggagc gcgtgagatg cgcaagaagg ccgatcaagc ccaagagacg 5520
   gcccgagacc ttcgggagcg cctgaagccc gttctggacg ccctggggcc gttgaatcgg 5580
   gatatgcagg ccaaggccgc cgcgatcatc aaggccgtgg gcgaaaagct gctgacggaa 5640
   cagcgggaag tccagcgcca gaaacaggcc cagcgccagc aggaacgcgg gcgcgcacat 5700
   ttccccgaaa agtgccacct gggatgaatg tcagctactg ggctatctgg acaagggaaa 5760
   acgcaagcgc aaagagaaag caggtagctt gcagtgggct tacatggcga tagctagact 5820
   gggcggtttt atggacagca agcgaaccgg aattgccagc tggggcgccc tctggtaagg 5880
   ttgggaagcc ctgcaaagta aactggatgg ctttcttgcc gccaaggatc tgatggcgca 5940
   ggggatcaag atctgatcaa gagacaggat gaggatcgtt tcgcatgatt gaacaagatg 6000
   gattgcacgc aggttctccg gccgcttggg tggagaggct attcggctat gactgggcac 6060
   aacagacaat cggctgctct gatgccgccg tgttccggct gtcagcgcag gggcgcccgg 6120
   ttctttttgt caagaccgac ctgtccggtg ccctgaatga actgcaggac gaggcagcgc 6180
   ggctatcgtg gctggccacg acgggcgttc cttgcgcagc tgtgctcgac gttgtcactg 6240
   aagcgggaag ggactggctg ctattgggcg aagtgccggg gcaggatctc ctgtcatctc 6300
   accttgctcc tgccgagaaa gtatccatca tggctgatgc aatgcggcgg ctgcatacgc 6360
   ttgatccggc tacctgccca ttcgaccacc aagcgaaaca tcgcatcgag cgagcacgta 6420
   ctcggatgga agccggtctt gtcgatcagg atgatctgga cgaagagcat caggggctcg 6480
   cgccagccga actgttcgcc aggctcaagg cgcgcatgcc cgacggcgag gatctcgtcg 6540
   tgacccatgg cgatgcctgc ttgccgaata tcatggtgga aaatggccgc ttttctggat 6600
   tcatcgactg tggccggctg ggtgtggcgg accgctatca ggacatagcg ttggctaccc      6660
   gtgatattgc tgaagagctt ggcggcgaat gggctgaccg cttcctcgtg ctttacggta      6720
   tcgccgctcc cgattcgcag cgcatcgcct tctatcgcct tcttgacgag ttcttctgag      6780
   cgggactctg gggttcgaaa tgaccgacca agcgacgccc aacctgccat cacgagattt      6840
   cgattccacc gccgccttct atgaaaggtt gggcttcgga atcgttttcc gggacgccgg      6900
   ctggatgatc ctccagcgcg gggatctcat gctggagttc ttcgcccacc cccatgggca      6960
   aatattatac gcaaggcgac aaggtgctga tgccgctggc gattcaggtt catcatgccg      7020
   tttgtgatgg cttccatgtc ggcagaatgc ttaatgaatt acaacagttt ttatgcatgc      7080
   gcccaatacg caaaccgcct ctccccgcgc gttggccgat tcattaatgc agctggcacg      7140
   acaggtttcc cgactggaaa gcgggcagtg agcgcaacgc aattaatgtg agttagctca      7200
   ctcattaggc accccaggct ttacacttta tgcttccggc tcgtatgttg tgtggaattg      7260
   tgagcggata acaatttcac acaggaaaca gctatgacca tgattacgcc aagcgcgcaa      7320
   ttaaccctca ctaaagggaa caaaagctgg gtac                                  7354
<210> 64
   <211> 2298
   <212> DNA
   <213> Artificial Sequence
<220>
   <223> syn operon
<400> 64
   aagcttgaat tcggtaccga aaatttttgg gaggtgtgaa atgcggcgcg aaagtctgtt      60
   ggtatcggtt tgcaagggcc tgcgggtaca tgtcgagcgc gttgggcagg atcccgggcg     120
   cagcacggtg atgctggtca acggcgcgat ggcgaccacc gcctcgttcg cccggacctg     180
   caagtgcctg gccgaacatt tcaacgtggt gctgttcgac ctgcccttcg ccgggcagtc     240
   gcgtcagcac aacccgcagc gcgggttgat caccaaggac gacgaggtgg aaatcctcct     300
   ggcgctgatc gagcgcttcg aggtcaatca cctggtctcc gcgtcctggg gcggtatctc     360
   cacgctgctg gcgctgtcgc gcaatccgcg cggcatccgc agctcggtgg tgatggcatt     420
   cgcccctgga ctgaaccagg cgatgctcga ctacgtcggg cgggcgcagg cgctgatcga     480
   gctggacgac aagtcggcga tcggccatct gctcaacgag accgtcggca aatacctgcc     540
   gcagcgcctg aaagccagca accatcagca catggcttcg ctggccaccg gcgaatacga     600
   gcaggcgcgc tttcacatcg accaggtgct ggcgctcaac gatcggggct acttggcttg     660
   cctggagcgg atccagagcc acgtgcattt catcaacggc agctgggacg aatacaccac     720
   cgccgaggac gcccgccagt tccgcgacta cctgccgcac tgcagtttct cgcgggtgga     780
   gggcaccggg catttcctcg acctggagtc caagctggca gcggtacgcg tgcaccgcgc     840
   cctgctcgag cacctgctga agcaaccgga gccgcagcgg gcggaacgcg cggcgggatt 900
   ccacgagatg gccatcggct acgcctgaac ccttgacctg cgaagacccg gcctggccgg 960
   gctttgcggt tgcataacgc acggagtagc cccatgcacg ccatcctcat cgccatcggc 1020
   tcggccggcg acgtatttcc cttcatcggc ctggcccgga ccctgaaact gcgcgggcac 1080
   cgcgtgagcc tctgcaccat cccggtgttt cgcgacgcgg tggagcagca cggcatcgcg 1140
   ttcgtcccgc tgagcgacga actgacctac cgccggacca tgggcgatcc gcgcctgtgg 1200
   gaccccaaga cgtccttcgg cgtgctctgg caagccatcg ccgggatgat cgagccggtc 1260
   tacgagtacg tctcggcgca gcgccatgac gacatcgtgg tggtcggctc gctatgggcg 1320
   ctgggcgcac gcatcgctca cgagaagtac gggattccct acctgtccgc gcaggtctcg 1380
   ccatcgaccc tgttgtcggc gcacctgccg ccggtacacc ccaagttcaa cgtgcccgag 1440
   cagatgccgc tggcgatgcg caagctgctc tggcgctgca tcgagcgctt caagctggat 1500
   cgcacctgcg cgccggagat caacgcggtg cgccgcaagg tcggcctgga aacgccggtg 1560
   aagcgcatct tcacccaatg gatgcattcg ccgcagggcg tggtctgcct gttcccggcc 1620
   tggttcgcgc cgccccagca ggattggccg caacccctgc acatgaccgg cttcccgctg 1680
   ttcgacggca gtatcccggg gaccccgctc gacgacgaac tgcaacgctt tctcgatcag 1740
   ggcagccggc cgctggtgtt cacccagggc tcgaccgaac acctgcaggg cgacttctac 1800
   gccatggccc tgcgcgcgct ggaacgcctc ggcgcgcgtg ggatcttcct caccggcgcc 1860
   ggccaggaac cgctgcgcgg cttgccgaac cacgtgctgc agcgcgccta cgcgccactg 1920
   ggagccttgc tgccatcgtg cgccgggctg gtccatccgg gcggtatcgg cgccatgagc 1980
   ctagccttgg cggcgggggt gccgcaggtg ctgctgccct gtgcccacga ccagttcgac 2040
   aatgccgaac ggctggtccg gctcggctgc gggatgcgcc tgggcgtgcc gttgcgcgag 2100
   caggagttgc gcggggcgct gtggcgcttg ctcgaggacc cggccatggc ggcggcctgt 2160
   cggcgtttca tggaattgtc acaaccgcac agtatcgctt gcggtaaagc ggcccaggtg 2220
   gtcgaacgtt gtcataggga gggggatgct cgatggctga aggctgcgtc ctgaacggtc 2280
   tagagaattc ggcgcgcc 2298
<210> 65
   <211> 2302
   <212> DNA
   <213> Artificial Sequence
<220>
   <223> syn operon
<400> 65
   ggtaccgaat tcaaaacttt ttgggaggtg tgagatgcgg cgcgaaagtc tgttggtaac      60
   ggtatgcaag ggcctgcggg tacatgtcga gcgcgtgggg caggatcccg ggcgcgacac     120
   ggtgatgctg gtcaacggcg cgatggcgac caccgcctcg ttcgcccgga cctgcaagtg     180
   cctggccgaa catttcaacg tggtgctgtt cgacctgccc ttcgccgggc agtcgcggca 240
   gcacaatccg cagcgcgggt tgatcaccaa ggacgacgag gtggagattc tcctggcgct 300
   gatcgagcgc ttcgctgtca accacctggt ctcggcctcc tggggcggca tctccacgct 360
   gctggcgctg tcgcgcaacc cgcgcggggt ccgcagctcg gtggtgatgg cgttcgcgcc 420
   ggggctgaac caggcgatgc tcgattatgt cgggcgggcc caggaactga tcgaactgga 480
   cgacaagtcg gcgatcggcc acctgctcaa cgagaccgtc ggcaagtacc tgccgccgcg 540
   gctgaaggcc agcaaccatc agcacatggc ctccctggcc actggcgagt acgagcaggc 600
   gcgtttccac atcgaccagg tgctggcgct caatgaccgt ggctacctga gctgcctggg 660
   gcagatccag agtcacgtgc atttcatcaa cggcagctgg gacgagtaca ccaccgccga 720
   ggacgcccgc cagttccgcg attacctgcc gcattgcagt ttttcgcggg tggaaggcac 780
   cgggcacttc ctcgacctgg agtccaagct ggcggcggcg cgtgtgcacc gggcgttgct 840
   cgagcacctg ctggcgcaac cggaaccgtg gcgctccgag caggcggcgg gattccacga 900
   gatggccatc ggctacgcct gacccgtcgg gatctgcgaa ggcccggcat ggccgggcct 960
   tgccgttgca caacgcaagg agtagcccca tgcacgccat tctcatcgcc atcggttcgg 1020
   ccggcgacgt gttccccttc atcggcctgg cccgcaccct gaagttgcgc ggccaccgcg 1080
   tcagcctgtg caccattccg gtgtttcgcg ccgcggtgga gcagcacggc atcgagttcg 1140
   tcccgctcag cgacgaactg acctaccgcc ggaccatggg cgacccgcgc ctgtgggatc 1200
   cgaagacctc gttcggagtg ctctggcagg ccatcgccgg gatgatcgag ccggtctacg 1260
   agtacgtctg cgcacagcgc cacgacgaca tcgtggtggt cggttcgctg tgggccctgg 1320
   gcgcgcggat cgcccatgag aaatacggga ttccctacct gtcggtgcag gtctcgccgt 1380
   cgaccctgct gtcggcgcac ctgccgccgg tccaccccag gttcaacgtg cccgagcagg 1440
   tcccgctggc gatgcgcaag ttgctctggc gctgcatcga acgcttcaag ctggaccgca 1500
   cctgcgcccc ggagatcaac gcggtgcgcc gcaaggtcgg cctggtcggc ccggcgaagc 1560
   gcatcttcac ccagtggatg cattcgccac agggagtgct ctgcctgttc ccggcctggt 1620
   tcgcaccgcc ccagcaggac tggccgcaac cgctgcacat gaccggcttc ccgctgttcg 1680
   acggcagcgt cccggggacc cgcctcgacg acgagttgca gcgcttcctc gagcagggca 1740
   gtcggccgct ggtgttcacc cagggttcga ccgagcacct gcagggagac ttctatgcca 1800
   tggccttgcg cgcgctggag cgtctcggcg cccgcggcat cttcctcacc ggcgccggcc 1860
   aggagccgct gcgtggcttg ccgagccacg tgctgcaacg ctcgtacgtg ccgttggggg 1920
   ccttgctgcc ggcgtgcgcc gggctggtcc acccggccgg catcggcgcc atgagcctgg 1980
   cgctggcggc gggggtgccg caggtgctgc tgccttgcgc ccacgaccag ttcgacaacg 2040
   ccgaacgcct ggtccgcctc ggctgcggta tccgcctggg cctgccgcta cgcgagcagg 2100
   cgctgcgcga gtcgctctgg cggctgctcg aggacccggc gctggcggcg gcctgtcggc 2160
   gtttcatgga attgtcacaa ccgcacagta tcgcttgcgg taaagcggcc caagtggtcg 2220
   aacgttgtca tagggagggg gatgtgcgat ggctgaaagc cgcgtcctga gccgtgctgg 2280
   cagaattctc tagaggcgcg cc 2302
<210> 66
   <211> 8325
   <212> DNA
   <213> Artificial Sequence
<220>
   <223> vector
<400> 66
   ctagatacgg gagaagaacg atcatggacc ggatagacat gggcgtgctg gtggtgctgt      60
   tcaatcctgg cgacgacgac ctggaacacc ttggcgaact ggcggcggcc tttccgcaac     120
   tgcgcttcct cgccgtcgac aactcgccgc acagcgatcc gcagcgcaac gcccggctgc     180
   gcgggcaagg catcgccgtg ctctaccacg gcaaccggca gggcatcgcc ggcgccttca     240
   accaggggct cgacacgctg ttccggcgcg gcctgcaggg tgtgctgctg ctcgaccagg     300
   actcccgtcc cggcggcgcc ttcctcgccg cccagtggcg caacctgcag gcatgcaacg     360
   gccaggcctg cctgctcggc ccacggatct tcgaccgggg cgaccggcgc ttcctgccgg     420
   ccatccacct cgacgggctg gcgctcaggc aactgtccct ggacggcctg acgaccccac     480
   agcgcacctc gttcctgatc tcctccggct gcctgctgac ccgcgaggcc taccagcgcc     540
   tcggccactt cgacgaggaa ctgttcatcg accacgtgga caccgagtac agcctgcgcg     600
   cccaggcgct ggacgtgccc ctgtacgtcg acccgcggct ggtcctcgag caccgcatcg     660
   gcacgcgcaa gacccgccgc ctcggcggtc tcagcctcag cgcgatgaac cacgccccac     720
   tgcgccgcta ctacctggcg cgcaacggcc tgctggtcct gcgccgctac gcccggtcct     780
   cgccgctggc cctgctggcg aacctgccga ccctgaccca gggcctcgcg gtgctcctgc     840
   tcgaacgcga caagctgctc aagctgcgct gcctgggctg gggcctgtgg gacggcctgc     900
   gggggcgcgg cggcgcgctg gagcgcaacc gcccgcgcct gctgaagcgc ctcgccggtc     960
   cggcggtggc gcccacagtt cccggcaagg ccaaggccta gtcggcgaaa cgcattgagc    1020
   tccaattcgc cctatagtga gtcgtattac gcgcgctcac tggccgtcgt tttacaacgt    1080
   cgtgactggg aaaaccctgg cgttacccaa cttaatcgcc ttgcagcaca tccccctttc    1140
   gccagctggc gtaatagcga agaggcccgc accgatcgcc cttcccaaca gttgcgcagc    1200
   ctgaatggcg aatggaaatt gtaagcgtta atattttgtt aaaattcgcg ttaaattttt    1260
   gttaaatcag ctcatttttt aaccaatagg ccgactgcga tgagtggcag ggcggggcgt    1320
   aattttttta aggcagttat tggtgccctt aaacgcctgg tgctacgcct gaataagtga    1380
   taataagcgg atgaatggca gaaattcgaa agcaaattcg acccggtcgt cggttcaggg    1440
   cagggtcgtt aaatagccgc ttatgtctat tgctggttta ccggtttatt gactaccgga     1500
   agcagtgtga ccgtgtgctt ctcaaatgcc tgaggccagt ttgctcaggc tctccccgtg     1560
   gaggtaataa ttgacgatat gatcatttat tctgcctccc agagcctgat aaaaacggtg     1620
   aatccgttag cgaggtgccg ccggcttcca ttcaggtcga ggtggcccgg ctccatgcac     1680
   cgcgacgcaa cgcggggagg cagacaaggt atagggcggc gaggcggcta cagccgatag     1740
   tctggaacag cgcacttacg ggttgctgcg caacccaagt gctaccggcg cggcagcgtg     1800
   acccgtgtcg gcggctccaa cggctcgcca tcgtccagaa aacacggctc atcgggcatc     1860
   ggcaggcgct gctgcccgcg ccgttcccat tcctccgttt cggtcaaggc tggcaggtct     1920
   ggttccatgc ccggaatgcc gggctggctg ggcggctcct cgccggggcc ggtcggtagt     1980
   tgctgctcgc ccggatacag ggtcgggatg cggcgcaggt cgccatgccc caacagcgat     2040
   tcgtcctggt cgtcgtgatc aaccaccacg gcggcactga acaccgacag gcgcaactgg     2100
   tcgcggggct ggccccacgc cacgcggtca ttgaccacgt aggccgacac ggtgccgggg     2160
   ccgttgagct tcacgacgga gatccagcgc tcggccacca agtccttgac tgcgtattgg     2220
   accgtccgca aagaacgtcc gatgagcttg gaaagtgtct tctggctgac caccacggcg     2280
   ttctggtggc ccatctgcgc cacgaggtga tgcagcagca ttgccgccgt gggtttcctc     2340
   gcaataagcc cggcccacgc ctcatgcgct ttgcgttccg tttgcaccca gtgaccgggc     2400
   ttgttcttgg cttgaatgcc gatttctctg gactgcgtgg ccatgcttat ctccatgcgg     2460
   tagggtgccg cacggttgcg gcaccatgcg caatcagctg caacttttcg gcagcgcgac     2520
   aacaattatg cgttgcgtaa aagtggcagt caattacaga ttttctttaa cctacgcaat     2580
   gagctattgc ggggggtgcc gcaatgagct gttgcgtacc cccctttttt aagttgttga     2640
   tttttaagtc tttcgcattt cgccctatat ctagttcttt ggtgcccaaa gaagggcacc     2700
   cctgcggggt tcccccacgc cttcggcgcg gctccccctc cggcaaaaag tggcccctcc    2760
   ggggcttgtt gatcgactgc gcggccttcg gccttgccca aggtggcgct gcccccttgg    2820
   aacccccgca ctcgccgccg tgaggctcgg ggggcaggcg ggcgggcttc gccttcgact     2880
   gcccccactc gcataggctt gggtcgttcc aggcgcgtca aggccaagcc gctgcgcggt     2940
   cgctgcgcga gccttgaccc gccttccact tggtgtccaa ccggcaagcg aagcgcgcag     3000
   gccgcaggcc ggaggctttt ccccagagaa aattaaaaaa attgatgggg caaggccgca     3060
   ggccgcgcag ttggagccgg tgggtatgtg gtcgaaggct gggtagccgg tgggcaatcc     3120
   ctgtggtcaa gctcgtgggc aggcgcagcc tgtccatcag cttgtccagc agggttgtcc     3180
   acgggccgag cgaagcgagc cagccggtgg ccgctcgcgg ccatcgtcca catatccacg     3240
   ggctggcaag ggagcgcagc gaccgcgcag ggcgaagccc ggagagcaag cccgtagggc     3300
   gccgcagccg ccgtaggcgg tcacgacttt gcgaagcaaa gtctagtgag tatactcaag     3360
   cattgagtgg cccgccggag gcaccgcctt gcgctgcccc cgtcgagccg gttggacacc 3420
   aaaagggagg ggcaggcatg gcggcatacg cgatcatgcg atgcaagaag ctggcgaaaa 3480
   tgggcaacgt ggcggccagt ctcaagcacg cctaccgcga gcgcgagacg cccaacgctg 3540
   acgccagcag gacgccagag aacgagcact gggcggccag cagcaccgat gaagcgatgg 3600
   gccgactgcg cgagttgctg ccagagaagc ggcgcaagga cgctgtgttg gcggtcgagt 3660
   acgtcatgac ggccagcccg gaatggtgga agtcggccag ccaagaacag caggcggcgt 3720
   tcttcgagaa ggcgcacaag tggctggcgg acaagtacgg ggcggatcgc atcgtgacgg 3780
   ccagcatcca ccgtgacgaa accagcccgc acatgaccgc gttcgtggtg ccgctgacgc 3840
   aggacggcag gctgtcggcc aaggagttca tcggcaacaa agcgcagatg acccgcgacc 3900
   agaccacgtt tgcggccgct gtggccgatc tagggctgca acggggcatc gagggcagca 3960
   aggcacgtca cacgcgcatt caggcgttct acgaggccct ggagcggcca ccagtgggcc 4020
   acgtcaccat cagcccgcaa gcggtcgagc cacgcgccta tgcaccgcag ggattggccg 4080
   aaaagctggg aatctcaaag cgcgttgaga cgccggaagc cgtggccgac cggctgacaa 4140
   aagcggttcg gcaggggtat gagcctgccc tacaggccgc cgcaggagcg cgtgagatgc 4200
   gcaagaaggc cgatcaagcc caagagacgg cccgagacct tcgggagcgc ctgaagcccg 4260
   ttctggacgc cctggggccg ttgaatcggg atatgcaggc caaggccgcc gcgatcatca 4320
   aggccgtggg cgaaaagctg ctgacggaac agcgggaagt ccagcgccag aaacaggccc 4380
   agcgccagca ggaacgcggg cgcgcacatt tccccgaaaa gtgccacctg ggatgaatgt 4440
   cagctactgg gctatctgga caagggaaaa cgcaagcgca aagagaaagc aggtagcttg 4500
   cagtgggctt acatggcgat agctagactg ggcggtttta tggacagcaa gcgaaccgga 4560
   attgccagct ggggcgccct ctggtaaggt tgggaagccc tgcaaagtaa actggatggc 4620
   tttcttgccg ccaaggatct gatggcgcag gggatcaaga tctgatcaag agacaggatg 4680
   aggatcgttt cgcatgattg aacaagatgg attgcacgca ggttctccgg ccgcttgggt 4740
   ggagaggcta ttcggctatg actgggcaca acagacaatc ggctgctctg atgccgccgt 4800
   gttccggctg tcagcgcagg ggcgcccggt tctttttgtc aagaccgacc tgtccggtgc 4860
   cctgaatgaa ctgcaggacg aggcagcgcg gctatcgtgg ctggccacga cgggcgttcc 4920
   ttgcgcagct gtgctcgacg ttgtcactga agcgggaagg gactggctgc tattgggcga 4980
   agtgccgggg caggatctcc tgtcatctca ccttgctcct gccgagaaag tatccatcat 5040
   ggctgatgca atgcggcggc tgcatacgct tgatccggct acctgcccat tcgaccacca 5100
   agcgaaacat cgcatcgagc gagcacgtac tcggatggaa gccggtcttg tcgatcagga 5160
   tgatctggac gaagagcatc aggggctcgc gccagccgaa ctgttcgcca ggctcaaggc 5220
   gcgcatgccc gacggcgagg atctcgtcgt gacccatggc gatgcctgct tgccgaatat 5280
   catggtggaa aatggccgct tttctggatt catcgactgt ggccggctgg gtgtggcgga 5340
   ccgctatcag gacatagcgt tggctacccg tgatattgct gaagagcttg gcggcgaatg 5400
   ggctgaccgc ttcctcgtgc tttacggtat cgccgctccc gattcgcagc gcatcgcctt 5460
   ctatcgcctt cttgacgagt tcttctgagc gggactctgg ggttcgaaat gaccgaccaa 5520
   gcgacgccca acctgccatc acgagatttc gattccaccg ccgccttcta tgaaaggttg 5580
   ggcttcggaa tcgttttccg ggacgccggc tggatgatcc tccagcgcgg ggatctcatg 5640
   ctggagttct tcgcccaccc ccatgggcaa atattatacg caaggcgaca aggtgctgat 5700
   gccgctggcg attcaggttc atcatgccgt ttgtgatggc ttccatgtcg gcagaatgct 5760
   taatgaatta caacagtttt tatgcatgcg cccaatacgc aaaccgcctc tccccgcgcg 5820
   ttggccgatt cattaatgca gctggcacga caggtttccc gactggaaag cgggcagtga 5880
   gcgcaacgca attaatgtga gttagctcac tcattaggca ccccaggctt tacactttat 5940
   gcttccggct cgtatgttgt gtggaattgt gagcggataa caatttcaca caggaaacag 6000
   ctatgaccat gattacgcca agcgcgcaat taaccctcac taaagggaac aaaagctggg 6060
   taccgaaaat ttttgggagg tgtgaaatgc ggcgcgaaag tctgttggta tcggtttgca 6120
   agggcctgcg ggtacatgtc gagcgcgttg ggcaggatcc cgggcgcagc acggtgatgc 6180
   tggtcaacgg cgcgatggcg accaccgcct cgttcgcccg gacctgcaag tgcctggccg 6240
   aacatttcaa cgtggtgctg ttcgacctgc ccttcgccgg gcagtcgcgt cagcacaacc 6300
   cgcagcgcgg gttgatcacc aaggacgacg aggtggaaat cctcctggcg ctgatcgagc 6360
   gcttcgaggt caatcacctg gtctccgcgt cctggggcgg tatctccacg ctgctggcgc 6420
   tgtcgcgcaa tccgcgcggc atccgcagct cggtggtgat ggcattcgcc cctggactga 6480
   accaggcgat gctcgactac gtcgggcggg cgcaggcgct gatcgagctg gacgacaagt 6540
   cggcgatcgg ccatctgctc aacgagaccg tcggcaaata cctgccgcag cgcctgaaag 6600
   ccagcaacca tcagcacatg gcttcgctgg ccaccggcga atacgagcag gcgcgctttc 6660
   acatcgacca ggtgctggcg ctcaacgatc ggggctactt ggcttgcctg gagcggatcc 6720
   agagccacgt gcatttcatc aacggcagct gggacgaata caccaccgcc gaggacgccc 6780
   gccagttccg cgactacctg ccgcactgca gtttctcgcg ggtggagggc accgggcatt 6840
   tcctcgacct ggagtccaag ctggcagcgg tacgcgtgca ccgcgccctg ctcgagcacc 6900
   tgctgaagca accggagccg cagcgggcgg aacgcgcggc gggattccac gagatggcca 6960
   tcggctacgc ctgaaccctt gacctgcgaa gacccggcct ggccgggctt tgcggttgca 7020
   taacgcacgg agtagcccca tgcacgccat cctcatcgcc atcggctcgg ccggcgacgt 7080
   atttcccttc atcggcctgg cccggaccct gaaactgcgc gggcaccgcg tgagcctctg 7140
   caccatcccg gtgtttcgcg acgcggtgga gcagcacggc atcgcgttcg tcccgctgag 7200
   cgacgaactg acctaccgcc ggaccatggg cgatccgcgc ctgtgggacc ccaagacgtc 7260
   cttcggcgtg ctctggcaag ccatcgccgg gatgatcgag ccggtctacg agtacgtctc 7320
   ggcgcagcgc catgacgaca tcgtggtggt cggctcgcta tgggcgctgg gcgcacgcat 7380
   cgctcacgag aagtacggga ttccctacct gtccgcgcag gtctcgccat cgaccctgtt 7440
   gtcggcgcac ctgccgccgg tacaccccaa gttcaacgtg cccgagcaga tgccgctggc 7500
   gatgcgcaag ctgctctggc gctgcatcga gcgcttcaag ctggatcgca cctgcgcgcc 7560
   ggagatcaac gcggtgcgcc gcaaggtcgg cctggaaacg ccggtgaagc gcatcttcac 7620
   ccaatggatg cattcgccgc agggcgtggt ctgcctgttc ccggcctggt tcgcgccgcc 7680
   ccagcaggat tggccgcaac ccctgcacat gaccggcttc ccgctgttcg acggcagtat 7740
   cccggggacc ccgctcgacg acgaactgca acgctttctc gatcagggca gccggccgct 7800
   ggtgttcacc cagggctcga ccgaacacct gcagggcgac ttctacgcca tggccctgcg 7860
   cgcgctggaa cgcctcggcg cgcgtgggat cttcctcacc ggcgccggcc aggaaccgct 7920
   gcgcggcttg ccgaaccacg tgctgcagcg cgcctacgcg ccactgggag ccttgctgcc 7980
   atcgtgcgcc gggctggtcc atccgggcgg tatcggcgcc atgagcctag ccttggcggc 8040
   gggggtgccg caggtgctgc tgccctgtgc ccacgaccag ttcgacaatg ccgaacggct 8100
   ggtccggctc ggctgcggga tgcgcctggg cgtgccgttg cgcgagcagg agttgcgcgg 8160
   ggcgctgtgg cgcttgctcg aggacccggc catggcggcg gcctgtcggc gtttcatgga 8220
   attgtcacaa ccgcacagta tcgcttgcgg taaagcggcc caggtggtcg aacgttgtca 8280
   tagggagggg gatgctcgat ggctgaaggc tgcgtcctga acggt 8325
<210> 67
   <211> 8335
   <212> DNA
   <213> Artificial Sequence
<220>
   <223> vector
<400> 67
   ctagatacgg gagaagaacg atcatgacga tcctgggggc gctggtgatt ctgtacgacc      60
   cgacggacga gcagttgtcg gggctggagg cgctcgcgcg cgacagcgac gcgctcgtgg     120
   tcgtggacaa cacgccgcac gagcacgcgg cggcgcgcga gcgggtgcgt gcgctgtcgg     180
   cgcggacgaa cacggtgtgg cgacaccacg gcaaccgggg cggggtcgcg ggcgggtaca     240
   acgcggggct gtcggtgctg ttcgcgcagg gcgtcgaggc ggtcgcgctg ttcgaccagg     300
   actcgacggt gccggccggg tacttcgagc ggatgcgcga ggcgtgcgcg caactgggtg     360
   agcaaccggg cgcgcacgcg ggcgcgttca tcgcgggccc gcggatctac gacgcgaacg     420
   agcagcgctt cctgccggag ctgatgacga gcggggtgac ggtgcgccgc gtgcgggtgg     480
   agggcgagac ggcgccgcag cgctgcgcgt tcctgatctc gtcgggcagc gtgatttcgc     540
   gggccgcgta cgcgcggctc ggtcgattcg acgaggcgct gttcatcgat cacgtcgaca     600
   ccgagtattg cctgcgcgcg ctcgcgcaca acgtgccgct gtacgtggtg ccgccgctcg 660
   tgctgacgca ccggatcggc gcgcggcgcc ggcacaaggt ggggccgttc gagctgacgg 720
   cgatgcatca cgggtggttg cgccgatact acggcgcgcg caacgcgatg caactggggc 780
   tgcagtacgg cttgcggttt ccggtggcgc tggtgccgaa tctgctgacg atatggcagg 840
   tgatccaggt ggtgctgtgc gagcgggaga agggcgcgaa gctgcgcggg atcgcgctgg 900
   gcgtgctcga cggcctgttc gggcggctgg gatcgttcga cgatgcgcgc gcgggcgcgg 960
   cggcgcgcga gccggtgcgg caggaatgat cggcgaaacg cattgagctc caattcgccc 1020
   tatagtgagt cgtattacgc gcgctcactg gccgtcgttt tacaacgtcg tgactgggaa 1080
   aaccctggcg ttacccaact taatcgcctt gcagcacatc cccctttcgc cagctggcgt 1140
   aatagcgaag aggcccgcac cgatcgccct tcccaacagt tgcgcagcct gaatggcgaa 1200
   tggaaattgt aagcgttaat attttgttaa aattcgcgtt aaatttttgt taaatcagct 1260
   cattttttaa ccaataggcc gactgcgatg agtggcaggg cggggcgtaa tttttttaag 1320
   gcagttattg gtgcccttaa acgcctggtg ctacgcctga ataagtgata ataagcggat 1380
   gaatggcaga aattcgaaag caaattcgac ccggtcgtcg gttcagggca gggtcgttaa 1440
   atagccgctt atgtctattg ctggtttacc ggtttattga ctaccggaag cagtgtgacc 1500
   gtgtgcttct caaatgcctg aggccagttt gctcaggctc tccccgtgga ggtaataatt 1560
   gacgatatga tcatttattc tgcctcccag agcctgataa aaacggtgaa tccgttagcg 1620
   aggtgccgcc ggcttccatt caggtcgagg tggcccggct ccatgcaccg cgacgcaacg 1680
   cggggaggca gacaaggtat agggcggcga ggcggctaca gccgatagtc tggaacagcg 1740
   cacttacggg ttgctgcgca acccaagtgc taccggcgcg gcagcgtgac ccgtgtcggc 1800
   ggctccaacg gctcgccatc gtccagaaaa cacggctcat cgggcatcgg caggcgctgc 1860
   tgcccgcgcc gttcccattc ctccgtttcg gtcaaggctg gcaggtctgg ttccatgccc 1920
   ggaatgccgg gctggctggg cggctcctcg ccggggccgg tcggtagttg ctgctcgccc 1980
   ggatacaggg tcgggatgcg gcgcaggtcg ccatgcccca acagcgattc gtcctggtcg 2040
   tcgtgatcaa ccaccacggc ggcactgaac accgacaggc gcaactggtc gcggggctgg 2100
   ccccacgcca cgcggtcatt gaccacgtag gccgacacgg tgccggggcc gttgagcttc 2160
   acgacggaga tccagcgctc ggccaccaag tccttgactg cgtattggac cgtccgcaaa 2220
   gaacgtccga tgagcttgga aagtgtcttc tggctgacca ccacggcgtt ctggtggccc 2280
   atctgcgcca cgaggtgatg cagcagcatt gccgccgtgg gtttcctcgc aataagcccg 2340
   gcccacgcct catgcgcttt gcgttccgtt tgcacccagt gaccgggctt gttcttggct 2400
   tgaatgccga tttctctgga ctgcgtggcc atgcttatct ccatgcggta gggtgccgca 2460
   cggttgcggc accatgcgca atcagctgca acttttcggc agcgcgacaa caattatgcg 2520
   ttgcgtaaaa gtggcagtca attacagatt ttctttaacc tacgcaatga gctattgcgg 2580
   ggggtgccgc aatgagctgt tgcgtacccc ccttttttaa gttgttgatt tttaagtctt 2640
   tcgcatttcg ccctatatct agttctttgg tgcccaaaga agggcacccc tgcggggttc 2700
   ccccacgcct tcggcgcggc tccccctccg gcaaaaagtg gcccctccgg ggcttgttga 2760
   tcgactgcgc ggccttcggc cttgcccaag gtggcgctgc ccccttggaa cccccgcact 2820
   cgccgccgtg aggctcgggg ggcaggcggg cgggcttcgc cttcgactgc ccccactcgc 2880
   ataggcttgg gtcgttccag gcgcgtcaag gccaagccgc tgcgcggtcg ctgcgcgagc 2940
   cttgacccgc cttccacttg gtgtccaacc ggcaagcgaa gcgcgcaggc cgcaggccgg 3000
   aggcttttcc ccagagaaaa ttaaaaaaat tgatggggca aggccgcagg ccgcgcagtt 3060
   ggagccggtg ggtatgtggt cgaaggctgg gtagccggtg ggcaatccct gtggtcaagc 3120
   tcgtgggcag gcgcagcctg tccatcagct tgtccagcag ggttgtccac gggccgagcg 3180
   aagcgagcca gccggtggcc gctcgcggcc atcgtccaca tatccacggg ctggcaaggg 3240
   agcgcagcga ccgcgcaggg cgaagcccgg agagcaagcc cgtagggcgc cgcagccgcc 3300
   gtaggcggtc acgactttgc gaagcaaagt ctagtgagta tactcaagca ttgagtggcc 3360
   cgccggaggc accgccttgc gctgcccccg tcgagccggt tggacaccaa aagggagggg 3420
   caggcatggc ggcatacgcg atcatgcgat gcaagaagct ggcgaaaatg ggcaacgtgg 3480
   cggccagtct caagcacgcc taccgcgagc gcgagacgcc caacgctgac gccagcagga 3540
   cgccagagaa cgagcactgg gcggccagca gcaccgatga agcgatgggc cgactgcgcg 3600
   agttgctgcc agagaagcgg cgcaaggacg ctgtgttggc ggtcgagtac gtcatgacgg 3660
   ccagcccgga atggtggaag tcggccagcc aagaacagca ggcggcgttc ttcgagaagg 3720
   cgcacaagtg gctggcggac aagtacgggg cggatcgcat cgtgacggcc agcatccacc 3780
   gtgacgaaac cagcccgcac atgaccgcgt tcgtggtgcc gctgacgcag gacggcaggc 3840
   tgtcggccaa ggagttcatc ggcaacaaag cgcagatgac ccgcgaccag accacgtttg 3900
   cggccgctgt ggccgatcta gggctgcaac ggggcatcga gggcagcaag gcacgtcaca 3960
   cgcgcattca ggcgttctac gaggccctgg agcggccacc agtgggccac gtcaccatca 4020
   gcccgcaagc ggtcgagcca cgcgcctatg caccgcaggg attggccgaa aagctgggaa 4080
   tctcaaagcg cgttgagacg ccggaagccg tggccgaccg gctgacaaaa gcggttcggc 4140
   aggggtatga gcctgcccta caggccgccg caggagcgcg tgagatgcgc aagaaggccg 4200
   atcaagccca agagacggcc cgagaccttc gggagcgcct gaagcccgtt ctggacgccc 4260
   tggggccgtt gaatcgggat atgcaggcca aggccgccgc gatcatcaag gccgtgggcg 4320
   aaaagctgct gacggaacag cgggaagtcc agcgccagaa acaggcccag cgccagcagg 4380
   aacgcgggcg cgcacatttc cccgaaaagt gccacctggg atgaatgtca gctactgggc 4440
   tatctggaca agggaaaacg caagcgcaaa gagaaagcag gtagcttgca gtgggcttac 4500
   atggcgatag ctagactggg cggttttatg gacagcaagc gaaccggaat tgccagctgg 4560
   ggcgccctct ggtaaggttg ggaagccctg caaagtaaac tggatggctt tcttgccgcc 4620
   aaggatctga tggcgcaggg gatcaagatc tgatcaagag acaggatgag gatcgtttcg 4680
   catgattgaa caagatggat tgcacgcagg ttctccggcc gcttgggtgg agaggctatt 4740
   cggctatgac tgggcacaac agacaatcgg ctgctctgat gccgccgtgt tccggctgtc 4800
   agcgcagggg cgcccggttc tttttgtcaa gaccgacctg tccggtgccc tgaatgaact 4860
   gcaggacgag gcagcgcggc tatcgtggct ggccacgacg ggcgttcctt gcgcagctgt 4920
   gctcgacgtt gtcactgaag cgggaaggga ctggctgcta ttgggcgaag tgccggggca 4980
   ggatctcctg tcatctcacc ttgctcctgc cgagaaagta tccatcatgg ctgatgcaat 5040
   gcggcggctg catacgcttg atccggctac ctgcccattc gaccaccaag cgaaacatcg 5100
   catcgagcga gcacgtactc ggatggaagc cggtcttgtc gatcaggatg atctggacga 5160
   agagcatcag gggctcgcgc cagccgaact gttcgccagg ctcaaggcgc gcatgcccga 5220
   cggcgaggat ctcgtcgtga cccatggcga tgcctgcttg ccgaatatca tggtggaaaa 5280
   tggccgcttt tctggattca tcgactgtgg ccggctgggt gtggcggacc gctatcagga 5340
   catagcgttg gctacccgtg atattgctga agagcttggc ggcgaatggg ctgaccgctt 5400
   cctcgtgctt tacggtatcg ccgctcccga ttcgcagcgc atcgccttct atcgccttct 5460
   tgacgagttc ttctgagcgg gactctgggg ttcgaaatga ccgaccaagc gacgcccaac 5520
   ctgccatcac gagatttcga ttccaccgcc gccttctatg aaaggttggg cttcggaatc 5580
   gttttccggg acgccggctg gatgatcctc cagcgcgggg atctcatgct ggagttcttc 5640
   gcccaccccc atgggcaaat attatacgca aggcgacaag gtgctgatgc cgctggcgat 5700
   tcaggttcat catgccgttt gtgatggctt ccatgtcggc agaatgctta atgaattaca 5760
   acagttttta tgcatgcgcc caatacgcaa accgcctctc cccgcgcgtt ggccgattca 5820
   ttaatgcagc tggcacgaca ggtttcccga ctggaaagcg ggcagtgagc gcaacgcaat 5880
   taatgtgagt tagctcactc attaggcacc ccaggcttta cactttatgc ttccggctcg 5940
   tatgttgtgt ggaattgtga gcggataaca atttcacaca ggaaacagct atgaccatga 6000
   ttacgccaag cgcgcaatta accctcacta aagggaacaa aagctgggta ccgaattcaa 6060
   aactttttgg gaggtgtgag atgcggcgcg aaagtctgtt ggtaacggta tgcaagggcc 6120
   tgcgggtaca tgtcgagcgc gtggggcagg atcccgggcg cgacacggtg atgctggtca 6180
   acggcgcgat ggcgaccacc gcctcgttcg cccggacctg caagtgcctg gccgaacatt 6240
   tcaacgtggt gctgttcgac ctgcccttcg ccgggcagtc gcggcagcac aatccgcagc 6300
   gcgggttgat caccaaggac gacgaggtgg agattctcct ggcgctgatc gagcgcttcg 6360
   ctgtcaacca cctggtctcg gcctcctggg gcggcatctc cacgctgctg gcgctgtcgc 6420
   gcaacccgcg cggggtccgc agctcggtgg tgatggcgtt cgcgccgggg ctgaaccagg 6480
   cgatgctcga ttatgtcggg cgggcccagg aactgatcga actggacgac aagtcggcga 6540
   tcggccacct gctcaacgag accgtcggca agtacctgcc gccgcggctg aaggccagca 6600
   accatcagca catggcctcc ctggccactg gcgagtacga gcaggcgcgt ttccacatcg 6660
   accaggtgct ggcgctcaat gaccgtggct acctgagctg cctggggcag atccagagtc 6720
   acgtgcattt catcaacggc agctgggacg agtacaccac cgccgaggac gcccgccagt 6780
   tccgcgatta cctgccgcat tgcagttttt cgcgggtgga aggcaccggg cacttcctcg 6840
   acctggagtc caagctggcg gcggcgcgtg tgcaccgggc gttgctcgag cacctgctgg 6900
   cgcaaccgga accgtggcgc tccgagcagg cggcgggatt ccacgagatg gccatcggct 6960
   acgcctgacc cgtcgggatc tgcgaaggcc cggcatggcc gggccttgcc gttgcacaac 7020
   gcaaggagta gccccatgca cgccattctc atcgccatcg gttcggccgg cgacgtgttc 7080
   cccttcatcg gcctggcccg caccctgaag ttgcgcggcc accgcgtcag cctgtgcacc 7140
   attccggtgt ttcgcgccgc ggtggagcag cacggcatcg agttcgtccc gctcagcgac 7200
   gaactgacct accgccggac catgggcgac ccgcgcctgt gggatccgaa gacctcgttc 7260
   ggagtgctct ggcaggccat cgccgggatg atcgagccgg tctacgagta cgtctgcgca 7320
   cagcgccacg acgacatcgt ggtggtcggt tcgctgtggg ccctgggcgc gcggatcgcc 7380
   catgagaaat acgggattcc ctacctgtcg gtgcaggtct cgccgtcgac cctgctgtcg 7440
   gcgcacctgc cgccggtcca ccccaggttc aacgtgcccg agcaggtccc gctggcgatg 7500
   cgcaagttgc tctggcgctg catcgaacgc ttcaagctgg accgcacctg cgccccggag 7560
   atcaacgcgg tgcgccgcaa ggtcggcctg gtcggcccgg cgaagcgcat cttcacccag 7620
   tggatgcatt cgccacaggg agtgctctgc ctgttcccgg cctggttcgc accgccccag 7680
   caggactggc cgcaaccgct gcacatgacc ggcttcccgc tgttcgacgg cagcgtcccg 7740
   gggacccgcc tcgacgacga gttgcagcgc ttcctcgagc agggcagtcg gccgctggtg 7800
   ttcacccagg gttcgaccga gcacctgcag ggagacttct atgccatggc cttgcgcgcg 7860
   ctggagcgtc tcggcgcccg cggcatcttc ctcaccggcg ccggccagga gccgctgcgt 7920
   ggcttgccga gccacgtgct gcaacgctcg tacgtgccgt tgggggcctt gctgccggcg 7980
   tgcgccgggc tggtccaccc ggccggcatc ggcgccatga gcctggcgct ggcggcgggg 8040
   gtgccgcagg tgctgctgcc ttgcgcccac gaccagttcg acaacgccga acgcctggtc 8100
   cgcctcggct gcggtatccg cctgggcctg ccgctacgcg agcaggcgct gcgcgagtcg 8160
   ctctggcggc tgctcgagga cccggcgctg gcggcggcct gtcggcgttt catggaattg 8220
   tcacaaccgc acagtatcgc ttgcggtaaa gcggcccaag tggtcgaacg ttgtcatagg 8280
   gagggggatg tgcgatggct gaaagccgcg tcctgagccg tgctggcaga attct 8335
<210> 68
   <211> 1023
   <212> DNA
   <213> Pseudomonas aeruginosa
<400> 68
   tctagatacg ggagaagaac gatcatggac cggatagaca tgggcgtgct ggtggtgctg      60
   ttcaatcctg gcgacgacga cctggaacac cttggcgaac tggcggcggc ctttccgcaa     120
   ctgcgcttcc tcgccgtcga caactcgccg cacagcgatc cgcagcgcaa cgcccggctg     180
   cgcgggcaag gcatcgccgt gctctaccac ggcaaccggc agggcatcgc cggcgccttc     240
   aaccaggggc tcgacacgct gttccggcgc ggcctgcagg gtgtgctgct gctcgaccag     300
   gactcccgtc ccggcggcgc cttcctcgcc gcccagtggc gcaacctgca ggcatgcaac     360
   ggccaggcct gcctgctcgg cccacggatc ttcgaccggg gcgaccggcg cttcctgccg     420
   gccatccacc tcgacgggct ggcgctcagg caactgtccc tggacggcct gacgacccca     480
   cagcgcacct cgttcctgat ctcctccggc tgcctgctga cccgcgaggc ctaccagcgc     540
   ctcggccact tcgacgagga actgttcatc gaccacgtgg acaccgagta cagcctgcgc     600
   gcccaggcgc tggacgtgcc cctgtacgtc gacccgcggc tggtcctcga gcaccgcatc     660
   ggcacgcgca agacccgccg cctcggcggt ctcagcctca gcgcgatgaa ccacgcccca     720
   ctgcgccgct actacctggc gcgcaacggc ctgctggtcc tgcgccgcta cgcccggtcc     780
   tcgccgctgg ccctgctggc gaacctgccg accctgaccc agggcctcgc ggtgctcctg     840
   ctcgaacgcg acaagctgct caagctgcgc tgcctgggct ggggcctgtg ggacggcctg     900
   cgggggcgcg gcggcgcgct ggagcgcaac cgcccgcgcc tgctgaagcg cctcgccggt     960
   ccggcggtgg cgcccacagt tcccggcaag gccaaggcct agtcggcgaa acgcattgag    1020
   ctc                                                                   1023
<210> 69
   <211> 13768
   <212> DNA
   <213> Artificial Sequence
<220>
   <223> vector
<400> 69
   ctagaggtgg acggccgcac gtatgagcac gcggtgacgc aggtgctgca ggccacgggg      60
   gtgcggggga ttctgctcgc gccggatgcg ccggatgcgc cggcggcatc ggacggggcg     120
   gcgctgctca agcgccgcta cgtgccgctc gcggcgttgc tgccgcgctg ccgggcgctg     180
   gtgcaccacg gggggatcgg gacggcgtcg ctcgcgtacg cggcgggggt gccgcaggtg     240
   gtgacgccgt tcgcgcacga ccagttcgac aacgcgcagc gggtggcggc gagcggctgc     300
   ggggtgcggc tggacgcgcc ggtgcgcggc gagccgctcg cgcgggcgct ggcgcaggtg     360
   ctgggcgacg cggcgatggc ggcgcgctgc gcgcaggtgc gcgcgcggat ggcggcggag     420
   ccgaacggct gcgacgcggc ggcgcgcttc atcgagcgct tcgcgccggg cgtcgcggcg 480
   cggcgggcgc agccggcatg agcgcgcagg cgatgtcggc ggatcaggcg ggcgttgcgc 540
   cgccggcggc cgccccgctg cgcggcgcga agctcgcgct gctgacgttc gcgctgtcgc 600
   tcgcgacgtt catcgaagtg ctggattcga cggtggcgaa cgtggcggtg ccggcgatct 660
   cgggcagcct cggggtgtcg aacagccagg gcacgtgggt gatcagctcg tactcggtgg 720
   ccgcggcgat cgcggtgccg ctgacggggt ggcttgcgcg gcgcgtgggc gagctgaggc 780
   tgttcgtggc gtcggtgatc ctgttcacgc tgacgtcgct gctgtgcggg ctcgcgcggg 840
   acctggaggt gctggttgcg tgccgggcgc tgcaggggct gttctcgggg ccgatggtgc 900
   cgctgtcgca gacgatcctg atgcgcgcgt tcccgccggc gcggcgcacg ctggcgctgg 960
   cgctgtgggg gatgacggtg ctgctcgcgc cgatcttcgg gccggtggtg ggcggctggc 1020
   tgatcgacaa cttctcgtgg ccgtggatct tcctgatcaa cctgccgatc gggctgttct 1080
   cgttcgcggt gtgcacgctg atgctgcgcc cgcaggcgca gcgcggcgag gcgagcccga 1140
   tcgacgcgcc ggggatcgtg ctgctggtga tcggggtggg ctcgctgcag gcgatgctgg 1200
   acctggggca cgaccggggc tggttcgatt cgccgctgat cacggcgctg gcgatcgcgg 1260
   cgggggtgtc gctcgtgtcg ctgctgatct gggagctggg cgaggcgcat ccggtggtgg 1320
   atctgagcct gttccgggag cggaccttca cgttctgcgt ggtgatcatc tcgctgggga 1380
   tgatgagctt ctcggtggtg ggggtggtgt ttccgctgtg gctgcaggcg gtgatgggat 1440
   acacggcgta ccaggcgggg ctggcgacgg cgtcgatggg ggtgctggcg ctggtgttct 1500
   cgatcctggt ggggctgtac gcgagccggg tggacgcgcg ggtgctggtg acgttcgggt 1560
   tcggggtgtt tgcggcggtg atgtggtgga gcacgcactt cacgctgtcg atgacgttcg 1620
   cgcaggtggt gacgccgcgg ctgattcagg ggatggggct gccgtgcttc ttcataccgc 1680
   tgacggcggc gacgctgtcg cgggtgccgg acgagaagct ggcggcggcg tcgagcctgt 1740
   cgaacttcct gcggacgctg tcggcggcgt tcggcacggc gctgagcgtg acgtggtggg 1800
   acaaccgggc gacgtaccac tacgcggtgg tgtcgcaatc ggtgacgcgc gcctcggaga 1860
   acacgcagcg gtacgtggac gcgctgcacg cgatggggct gcacggcgcg cgggagctga 1920
   gctcgctgca ccaggtggtg cggcagcagg cgtacatgat ggcgacgaac gacatgttct 1980
   acatggcgag cgcgacgtgc ctgctgctgg cggggctgat gtggctgacg cggccgaagc 2040
   ggggcgcggc ggcggcgctc gggcactgag gcgaggcatg tcgcgccccg catgacgaag 2100
   gcgaaggaga agggcgatgc gccgaagtcc tggggacgcg gcgcgtcgat gcggcaacga 2160
   agcgggcatt tcggcattcc gaaccaccaa agggaagagc gatgacgatc ctgggggcgc 2220
   tggtgttcgg gcggctggga tcgttcgacg atgcgcgcgc gggcgcggcg gcgcgcgagc 2280
   cggtgcggca ggaatgaacg gaacgggccg cagcgggata ccggaaagca agaaggacgc 2340
   atcatacgaa tgacgcagac agcaacgcaa gcagccactc gcgcgatgat cgcgacagga 2400
   agccgcgcgg cgcgccggct cgcggcagcc gcgctcgcgt gggcgctcgc cggctgcgtg 2460
   ccgtcgggct tcgagccggc gctcgcgccg cgcacgccgg gcgacgacgc gctcgcgcac 2520
   acggcggggg gcgccgcgca cggcgcatgg ccgagccccg actgggtccg gcagctcggc 2580
   gatccgcaac tcgacgcgct cgtcgacgag gcgctgcggc agaacccgac gctgcaggcc 2640
   gcgcaggcgc gcatcggcgt cgcgcagtcg cagctgcagc agttcgaatc gctgacgggg 2700
   ctcaccgcga cggcgggcgc gtcgctctcc aaggcgcacg tgccgcgctc gggcggcacc 2760
   atcaatacga cgttcaacgg cttgccggtg tcggtgccgc tcgtcggcga atcggtggtg 2820
   tcgtcgtcgt cgctgttcgt cgggctgaac tatcagctgg acctgtgggg caagaacgcg 2880
   gcggccacgc gcgggctgct gtcgatgcgc gatgcggcgc gcgtggaggc cgagcaggcg 2940
   cggctcgcgc tgtcggtggc gatcgtgacg ctgtacggcg agctggaccg cgcgtatgcg 3000
   ctgcgcgagc tgctgcagca gaagcgccgc gcgagcgagc aggtggagac ggtgctgcgc 3060
   gagcgcgcgg cgcgcgggat cgacaacggc tacgatgcgg acgacgcggc gctcaagcgg 3120
   ggcaagctgc tcgagcagct cgcgctgacc gacgagcaga tccagttgca gaagctgcaa 3180
   ctgggggtgc tgagcgggcg ggggccggag cgcgggctgt cgctcgcgcg gccgaagctc 3240
   gcgccgctcg cggacgcgcc gctgccggcg cggctgccgg ccgggctgct ggggcggcgg 3300
   ccggacatcg tcgcggcgcg gctgcgggtg gaggcggcgt acgcggcgat cgacggcacg 3360
   cgcgcgtcgt tctacccgga cgtgaacctg gcggcgctgg gcgggctgtt cgcgctcacg 3420
   ccggcgtcgc tgttcaagca cgatgcgctg gggggctcga tcggtccggc gctgtcgctg 3480
   ccgatcttcg atcgcggccg gctgaaggcg aagctggggg gcgacgtggc gaacgcggac 3540
   gtggcgctgg cgctgtacaa ccagacggtg gatgcggcgc tgggcgaggt ggcgcggcag 3600
   ttgacgtcgc tgtcgacggt ggatgcgctg ctcgaggcgc agcagcaggc ggtgcgctcg 3660
   gcgcagcgga tggtggcgct ggcgcaggac cggcaccggc gggggatggg gatgcgcaag 3720
   gacgtgaacg tggcgaagct gacgctgctg gacgagcgtg cgcacgtgat cgagctgcag 3780
   gcgcggcggc ggacgctgcg ggtggggctg atcggggcgc tgggcggcgg cttcgacgcg 3840
   cggccggcgg gcggcgcgcc gctcgcgcag ggcaagccgt tcgcggcggc gagcgacagg 3900
   ccgcccgatt gagcggcacg cacgcatgcg gcccgaagcc accgacaccc gaagacaccg 3960
   acaccaacgc caccttcacc gtgtacacga gcgattcaac cgacaccgcc cccgagcatc 4020
   gaagcccgtc gggccgatcc gcgacggctt gcgggccggc ccggccgttg ccggccggcg 4080
   ccaccgacat cacgcacgcg aagaccttga acgataccgc caccgatacc ccgcgcgcga 4140
   aggcgcccac cgatccggcc gccctcgacg gcgcgcacgc gcagcccgtg ccggcgcacg 4200
   agcgcggatc gcctccgccg ccggaagccg cggcgacgct cgccgcgcgc cgcgcgacgc 4260
   gccgccggcg cttcgcgctg ttcttcgggc tgctggcgct ggccgcgctg accgcggggc 4320
   tctactggtt cgtcgccggg cgcttcagcg aggagacgga cgacgcgtac gtggccggca 4380
   acgtggtgca gatcgccgcg cagatccagg ggacggtgac cgacgtgctg gtggcggaca 4440
   cgcagcaggt gaaggcgggg caggcgctgg tgaagctcga cgacgcggac gcgtcggcgg 4500
   cgttcgcgca ggcgcgggcg cagctcgcgc aggcggtgcg gcaggtggcg aacacgcggc 4560
   tctcgatggg gatgtacgag gagacggtga aggcgcgcga ggcggacctg aagcttgcgc 4620
   agcaggcgta tccggaggaa ctggcgcggc gaaagtcgtc gctggcgaac gcgcaggcgg 4680
   cgctggcggg ggcgcaggcg cagctggagg cggcgcgcgc gctgggcagc gagcggccgg 4740
   tcgagcagaa cccggcggtg cagcaggcgg ccgcgcagtt caagctggcg taccggaacc 4800
   tgaggcgcac gacgatcgtg tcgccggtgg acggcacggt cggtcagcgg tcggtgcaga 4860
   tcggtcagca ggtggggccg ggggtgccgc tgatgtcggt ggtgcagttg cggcaggtgt 4920
   gggtggaggc gaacttcaag gaagggcaga tccggcacat gcgggtgggc cagccggtgc 4980
   ggctcgaatc ggacctgtac ggcgcgcggg tgacgtacca cggccgggtg gagggggtct 5040
   cggcgggcac gggcagcgcg ttctcgatgc tgccgtcgca gaacgcggcg gggaactgga 5100
   tcaaggtggt gcagcgcctg ccggtggtga tctcgctgga gccgtcggag ctggcggcgc 5160
   acccgctgcg ggtggggctg tcgatgcgcg cgacggtgga gacgaaggtg cgtggcggcc 5220
   gcctgctcga cggcgacgcg ccgctgccgg ggctgcgcac gcgggtgcac gaagcgcagg 5280
   cgggcgaggc cgaggccgcg gcttcggcag tgattcggga gaatgacggc cgcaggtgac 5340
   gggcggttgc gggatcgctc tagagcggcc gccaccgcgg tggagctcca attcgcccta 5400
   tagtgagtcg tattacgcgc gctcactggc cgtcgtttta caacgtcgtg actgggaaaa 5460
   ccctggcgtt acccaactta atcgccttgc agcacatccc cctttcgcca gctggcgtaa 5520
   tagcgaagag gcccgcaccg atcgcccttc ccaacagttg cgcagcctga atggcgaatg 5580
   gaaattgtaa gcgttaatat tttgttaaaa ttcgcgttaa atttttgtta aatcagctca 5640
   ttttttaacc aataggccga ctgcgatgag tggcagggcg gggcgtaatt tttttaaggc 5700
   agttattggt gcccttaaac gcctggtgct acgcctgaat aagtgataat aagcggatga 5760
   atggcagaaa ttcgaaagca aattcgaccc ggtcgtcggt tcagggcagg gtcgttaaat 5820
   agccgcttat gtctattgct ggtttaccgg tttattgact accggaagca gtgtgaccgt 5880
   gtgcttctca aatgcctgag gccagtttgc tcaggctctc cccgtggagg taataattga 5940
   cgatatgatc atttattctg cctcccagag cctgataaaa acggtgaatc cgttagcgag 6000
   gtgccgccgg cttccattca ggtcgaggtg gcccggctcc atgcaccgcg acgcaacgcg 6060
   gggaggcaga caaggtatag ggcggcgagg cggctacagc cgatagtctg gaacagcgca 6120
   cttacgggtt gctgcgcaac ccaagtgcta ccggcgcggc agcgtgaccc gtgtcggcgg 6180
   ctccaacggc tcgccatcgt ccagaaaaca cggctcatcg ggcatcggca ggcgctgctg 6240
   cccgcgccgt tcccattcct ccgtttcggt caaggctggc aggtctggtt ccatgcccgg 6300
   aatgccgggc tggctgggcg gctcctcgcc ggggccggtc ggtagttgct gctcgcccgg 6360
   atacagggtc gggatgcggc gcaggtcgcc atgccccaac agcgattcgt cctggtcgtc 6420
   gtgatcaacc accacggcgg cactgaacac cgacaggcgc aactggtcgc ggggctggcc 6480
   ccacgccacg cggtcattga ccacgtaggc cgacacggtg ccggggccgt tgagcttcac 6540
   gacggagatc cagcgctcgg ccaccaagtc cttgactgcg tattggaccg tccgcaaaga 6600
   acgtccgatg agcttggaaa gtgtcttctg gctgaccacc acggcgttct ggtggcccat 6660
   ctgcgccacg aggtgatgca gcagcattgc cgccgtgggt ttcctcgcaa taagcccggc 6720
   ccacgcctca tgcgctttgc gttccgtttg cacccagtga ccgggcttgt tcttggcttg 6780
   aatgccgatt tctctggact gcgtggccat gcttatctcc atgcggtagg gtgccgcacg 6840
   gttgcggcac catgcgcaat cagctgcaac ttttcggcag cgcgacaaca attatgcgtt 6900
   gcgtaaaagt ggcagtcaat tacagatttt ctttaaccta cgcaatgagc tattgcgggg 6960
   ggtgccgcaa tgagctgttg cgtacccccc ttttttaagt tgttgatttt taagtctttc 7020
   gcatttcgcc ctatatctag ttctttggtg cccaaagaag ggcacccctg cggggttccc 7080
   ccacgccttc ggcgcggctc cccctccggc aaaaagtggc ccctccgggg cttgttgatc 7140
   gactgcgcgg ccttcggcct tgcccaaggt ggcgctgccc ccttggaacc cccgcactcg 7200
   ccgccgtgag gctcgggggg caggcgggcg ggcttcgcct tcgactgccc ccactcgcat 7260
   aggcttgggt cgttccaggc gcgtcaaggc caagccgctg cgcggtcgct gcgcgagcct 7320
   tgacccgcct tccacttggt gtccaaccgg caagcgaagc gcgcaggccg caggccggag 7380
   gcttttcccc agagaaaatt aaaaaaattg atggggcaag gccgcaggcc gcgcagttgg 7440
   agccggtggg tatgtggtcg aaggctgggt agccggtggg caatccctgt ggtcaagctc 7500
   gtgggcaggc gcagcctgtc catcagcttg tccagcaggg ttgtccacgg gccgagcgaa 7560
   gcgagccagc cggtggccgc tcgcggccat cgtccacata tccacgggct ggcaagggag 7620
   cgcagcgacc gcgcagggcg aagcccggag agcaagcccg tagggcgccg cagccgccgt 7680
   aggcggtcac gactttgcga agcaaagtct agtgagtata ctcaagcatt gagtggcccg 7740
   ccggaggcac cgccttgcgc tgcccccgtc gagccggttg gacaccaaaa gggaggggca 7800
   ggcatggcgg catacgcgat catgcgatgc aagaagctgg cgaaaatggg caacgtggcg 7860
   gccagtctca agcacgccta ccgcgagcgc gagacgccca acgctgacgc cagcaggacg 7920
   ccagagaacg agcactgggc ggccagcagc accgatgaag cgatgggccg actgcgcgag 7980
   ttgctgccag agaagcggcg caaggacgct gtgttggcgg tcgagtacgt catgacggcc 8040
   agcccggaat ggtggaagtc ggccagccaa gaacagcagg cggcgttctt cgagaaggcg 8100
   cacaagtggc tggcggacaa gtacggggcg gatcgcatcg tgacggccag catccaccgt 8160
   gacgaaacca gcccgcacat gaccgcgttc gtggtgccgc tgacgcagga cggcaggctg 8220
   tcggccaagg agttcatcgg caacaaagcg cagatgaccc gcgaccagac cacgtttgcg 8280
   gccgctgtgg ccgatctagg gctgcaacgg ggcatcgagg gcagcaaggc acgtcacacg 8340
   cgcattcagg cgttctacga ggccctggag cggccaccag tgggccacgt caccatcagc 8400
   ccgcaagcgg tcgagccacg cgcctatgca ccgcagggat tggccgaaaa gctgggaatc 8460
   tcaaagcgcg ttgagacgcc ggaagccgtg gccgaccggc tgacaaaagc ggttcggcag 8520
   gggtatgagc ctgccctaca ggccgccgca ggagcgcgtg agatgcgcaa gaaggccgat 8580
   caagcccaag agacggcccg agaccttcgg gagcgcctga agcccgttct ggacgccctg 8640
   gggccgttga atcgggatat gcaggccaag gccgccgcga tcatcaaggc cgtgggcgaa 8700
   aagctgctga cggaacagcg ggaagtccag cgccagaaac aggcccagcg ccagcaggaa 8760
   cgcgggcgcg cacatttccc cgaaaagtgc cacctgggat gaatgtcagc tactgggcta 8820
   tctggacaag ggaaaacgca agcgcaaaga gaaagcaggt agcttgcagt gggcttacat 8880
   ggcgatagct agactgggcg gttttatgga cagcaagcga accggaattg ccagctgggg 8940
   cgccctctgg taaggttggg aagccctgca aagtaaactg gatggctttc ttgccgccaa 9000
   ggatctgatg gcgcagggga tcaagatctg atcaagagac aggatgagga tcgtttcgca 9060
   tgattgaaca agatggattg cacgcaggtt ctccggccgc ttgggtggag aggctattcg 9120
   gctatgactg ggcacaacag acaatcggct gctctgatgc cgccgtgttc cggctgtcag 9180
   cgcaggggcg cccggttctt tttgtcaaga ccgacctgtc cggtgccctg aatgaactgc 9240
   aggacgaggc agcgcggcta tcgtggctgg ccacgacggg cgttccttgc gcagctgtgc 9300
   tcgacgttgt cactgaagcg ggaagggact ggctgctatt gggcgaagtg ccggggcagg 9360
   atctcctgtc atctcacctt gctcctgccg agaaagtatc catcatggct gatgcaatgc 9420
   ggcggctgca tacgcttgat ccggctacct gcccattcga ccaccaagcg aaacatcgca 9480
   tcgagcgagc acgtactcgg atggaagccg gtcttgtcga tcaggatgat ctggacgaag 9540
   agcatcaggg gctcgcgcca gccgaactgt tcgccaggct caaggcgcgc atgcccgacg 9600
   gcgaggatct cgtcgtgacc catggcgatg cctgcttgcc gaatatcatg gtggaaaatg 9660
   gccgcttttc tggattcatc gactgtggcc ggctgggtgt ggcggaccgc tatcaggaca 9720
   tagcgttggc tacccgtgat attgctgaag agcttggcgg cgaatgggct gaccgcttcc 9780
   tcgtgcttta cggtatcgcc gctcccgatt cgcagcgcat cgccttctat cgccttcttg 9840
   acgagttctt ctgagcggga ctctggggtt cgaaatgacc gaccaagcga cgcccaacct 9900
   gccatcacga gatttcgatt ccaccgccgc cttctatgaa aggttgggct tcggaatcgt 9960
   tttccgggac gccggctgga tgatcctcca gcgcggggat ctcatgctgg agttcttcgc 10020
   ccacccccat gggcaaatat tatacgcaag gcgacaaggt gctgatgccg ctggcgattc 10080
   aggttcatca tgccgtttgt gatggcttcc atgtcggcag aatgcttaat gaattacaac 10140
   agtttttatg catgcgccca atacgcaaac cgcctctccc cgcgcgttgg ccgattcatt 10200
   aatgcagctg gcacgacagg tttcccgact ggaaagcggg cagtgagcgc aacgcaatta 10260
   atgtgagtta gctcactcat taggcacccc aggctttaca ctttatgctt ccggctcgta 10320
   tgttgtgtgg aattgtgagc ggataacaat ttcacacagg aaacagctat gaccatgatt 10380
   acgccaagcg cgcaattaac cctcactaaa gggaacaaaa gctgggtacc gggccccccc 10440
   tcgaggtcga cggtatcgat aagcttgata tcgaattcct gcagcccggg ggatctggca 10500
   tttttgggag gtgtgaaatg cggcgcgaaa gtctgttggt atcggtttgc aagggcctgc 10560
   gggtacatgt cgagcgcgtt gggcaggatc ccgggcgcag cacggtgatg ctggtcaacg 10620
   gcgcgatggc gaccaccgcc tcgttcgccc ggacctgcaa gtgcctggcc gaacatttca 10680
   acgtggtgct gttcgacctg cccttcgccg ggcagtcgcg tcagcacaac ccgcagcggg 10740
   ggttgatcac caaggacgac gaggtggaaa tcctcctggc gctgatcgag cgcttcgagg 10800
   tcaatcacct ggtctccgcg tcctggggcg gtatctccac gctgctggcg ctgtcgcgca 10860
   atccgcgcgg catccgcagc tcggtggtga tggcattcgc ccctggactg aaccaggcga 10920
   tgctcgacta cgtcgggcgg gcgcaggcgc tgatcgagct ggacgacaag tcggcgatcg 10980
   gccatctgct caacgagacc gtcggcaaat acctgccgcc gcgcctgaaa gccagcaacc 11040
   atcagcacat ggcttcgctg gccaccggcg aatacgagca ggcgcgcttt cacatcgacc 11100
   aggtgctggc gctcaacgat cggggctacc tggcttgcct ggagcggatc cagagccacg 11160
   tgcatttcat caacggcagc tgggacgaat acaccaccgc cgaggacgcc cgccagttcc 11220
   gcgactacct gccgcactgc agtttctcgc gggtggaggg caccgggcat ttcctcgacc 11280
   tggagtccaa gctggccgcg gtacgcgtgc accgcgccct gctcgagcac ctgctgaagc 11340
   aaccggagcc gcagcgggcg gaacgcgcgg cgggattcca cgagatggcc atcggctacg 11400
   cctgaaccct tgacctgcga agacccggcc tggccgggct ttgcggttgc ataacgcacg 11460
   gagtagcacc atgcacgcca tcctcatcgc catcggctcg gccggcgacg tatttccctt 11520
   catcggcctg gcccggaccc tgaaattgcg cgggcaccgc gtgagcctct gcaccatccc 11580
   ggtgtttcgc gacgcggtgg agcagcacgg catcgcgttc gtcccgctga gcgacgaact 11640
   gacctaccgc cggaccatgg gcgatccgcg cctgtgggac cccaagacgt ccttcggcgt 11700
   gctctggcaa accatcgccg ggatgatcga gccggtctac gagtacgtct cggcgcagcg 11760
   ccatgacgac atcgtggtgg tcggctcgct ctgggcgctg ggcgcacgca tcgctcacga 11820
   gaagtacggg attccctacc tgtccgcgca ggtctcgcca tcgaccttgt tgtcggcgca 11880
   cctgccgccg gtacacccca agttcaacgt gcccgagcag atgccgctgg cgatgcgcaa 11940
   gctgctctgg cgctgcatcg agcgcttcaa gctggatcgc acctgcgcgc cggatatcaa 12000
   cgcggtgcgg cgcaaggtcg gcctggagac gccggtgaag cgcatcttca cccaatggat 12060
   gcattcgccg cagggcgtgg tctgcctgtt cccggcctgg ttcgcgccgc cccagcagga 12120
   ttggccgcaa cccctgcaca tgaccggctt cccgctgttc gacggcagta tcccggggac 12180
   cccgctcgac gacgaactgc aacgctttct cgatcagggc agccggccgc tggtgttcac 12240
   ccagggctcg accgaacacc tgcagggcga cttctacgcc atggccctgc gcgcgctgga 12300
   acgcctcggc gcgcgtggga tcttcctcac cggcgccggc caggaaccgc tgcgcggctt 12360
   gccgaaccac gtgctgcagc gcgcctacgc gccactggga gccttgctgc catcgtgcgc 12420
   cgggctggtc catccgggcg gtatcggcgc catgagcctg gccttggcgg cgggggtgcc 12480
   gcaggtgctg ctgccctgcg cccacgacca gttcgacaat gccgaacggc tggtccggct 12540
   cggctgcggg atgcgcctgg gcgtgccatt gcgcgagcag gagttgcgcg gggcgctgtg 12600
   gcgcttgctc gaggacccgg ccatggcggc ggcctgtcgg cgtttcatgg aattgtcaca 12660
   accgcacagt atcgcttgcg gtaaagcggc ccaggtggtc gaacgttgtc atagggaggg 12720
   ggatgcgcga tggctgaagg ctgcgtcctg acctacggga gaagaacgat catggaccgg 12780
   atagacatgg gcgtgctggt ggtactgttc aatcctggcg acgacgacct ggaacacctt 12840
   ggcgaactgg cggcggcgtt tccgcaactg cgcttccttg ccgtcgacaa ctcaccgcac 12900
   agcgatccgc agcgcaatgc ccggctgcgc gggcaaggca tcgccgtgct gcaccacggc 12960
   aaccggcagg gcatcgccgg cgccttcaac cagggactcg acgcgctatt ccggcgtggc 13020
   gtgcagggtg tgctgctgct cgaccaggac tcccgtcccg gcggcgcctt cctcgccgcc 13080
   cagtggcgca acctgcaggc gcgcaacggt caggcctgcc tgctcggccc acggatcttc 13140
   gaccggggtg accggcgctt cctgccggcc atccatctcg acggactgac gctcaggcaa 13200
   ttgtctctgg acggcctgac gaccccgcag cgcacctcgt tcctgatctc ctccggctgc 13260
   ctgctgaccc gcgaggccta ccagcgcctc ggccacttcg acgaggaact gttcatcgac 13320
   cacgtggaca ccgaatacag cctgcgcgcc caggcgctgg acgtgcccct gtacgtcgac 13380
   ccgcggctgg tcctcgagca ccgcatcggc acgcgcaaga cccgccgcct cggcggtctc 13440
   agcctcagcg cgatgaacca cgccccgctg cgccgctact acctggcgcg caacggcctg 13500
   ctggtcctgc gccgctacgc ccggtcctcg ccgctggccc tgctggcgaa cctgccgacc 13560
   ctgacccagg gcctcgcggt gctcctgctc gaacgcgaca agctgctcaa gctgcgctgc 13620
   ctgggctggg gcctgtggga cggcctgcgg ggacgcggcg gcgcgctgga gaccaaccgc 13680
   ccgcgcctgc tgaagcgcct cgccggcccg gccgtggcgt ccgtagcttc cggcaaggcc 13740
   aaggcctagt cggcgaaacg cattccct 13768
<210> 70
   <211> 5365
   <212> DNA
   <213> Artificial Sequence
<220>
   <223> synth operon
<400> 70
   tctagaggtg gacggccgca cgtatgagca cgcggtgacg caggtgctgc aggccacggg 60
   ggtgcggggg attctgctcg cgccggatgc gccggatgcg ccggcggcat cggacggggc 120
   ggcgctgctc aagcgccgct acgtgccgct cgcggcgttg ctgccgcgct gccgggcgct 180
   ggtgcaccac ggggggatcg ggacggcgtc gctcgcgtac gcggcggggg tgccgcaggt 240
   ggtgacgccg ttcgcgcacg accagttcga caacgcgcag cgggtggcgg cgagcggctg 300
   cggggtgcgg ctggacgcgc cggtgcgcgg cgagccgctc gcgcgggcgc tggcgcaggt 360
   gctgggcgac gcggcgatgg cggcgcgctg cgcgcaggtg cgcgcgcgga tggcggcgga 420
   gccgaacggc tgcgacgcgg cggcgcgctt catcgagcgc ttcgcgccgg gcgtcgcggc 480
   gcggcgggcg cagccggcat gagcgcgcag gcgatgtcgg cggatcaggc gggcgttgcg 540
   ccgccggcgg ccgccccgct gcgcggcgcg aagctcgcgc tgctgacgtt cgcgctgtcg 600
   ctcgcgacgt tcatcgaagt gctggattcg acggtggcga acgtggcggt gccggcgatc 660
   tcgggcagcc tcggggtgtc gaacagccag ggcacgtggg tgatcagctc gtactcggtg 720
   gccgcggcga tcgcggtgcc gctgacgggg tggcttgcgc ggcgcgtggg cgagctgagg 780
   ctgttcgtgg cgtcggtgat cctgttcacg ctgacgtcgc tgctgtgcgg gctcgcgcgg 840
   gacctggagg tgctggttgc gtgccgggcg ctgcaggggc tgttctcggg gccgatggtg 900
   ccgctgtcgc agacgatcct gatgcgcgcg ttcccgccgg cgcggcgcac gctggcgctg 960
   gcgctgtggg ggatgacggt gctgctcgcg ccgatcttcg ggccggtggt gggcggctgg 1020
   ctgatcgaca acttctcgtg gccgtggatc ttcctgatca acctgccgat cgggctgttc 1080
   tcgttcgcgg tgtgcacgct gatgctgcgc ccgcaggcgc agcgcggcga ggcgagcccg 1140
   atcgacgcgc cggggatcgt gctgctggtg atcggggtgg gctcgctgca ggcgatgctg 1200
   gacctggggc acgaccgggg ctggttcgat tcgccgctga tcacggcgct ggcgatcgcg 1260
   gcgggggtgt cgctcgtgtc gctgctgatc tgggagctgg gcgaggcgca tccggtggtg 1320
   gatctgagcc tgttccggga gcggaccttc acgttctgcg tggtgatcat ctcgctgggg 1380
   atgatgagct tctcggtggt gggggtggtg tttccgctgt ggctgcaggc ggtgatggga 1440
   tacacggcgt accaggcggg gctggcgacg gcgtcgatgg gggtgctggc gctggtgttc 1500
   tcgatcctgg tggggctgta cgcgagccgg gtggacgcgc gggtgctggt gacgttcggg 1560
   ttcggggtgt ttgcggcggt gatgtggtgg agcacgcact tcacgctgtc gatgacgttc 1620
   gcgcaggtgg tgacgccgcg gctgattcag gggatggggc tgccgtgctt cttcataccg 1680
   ctgacggcgg cgacgctgtc gcgggtgccg gacgagaagc tggcggcggc gtcgagcctg 1740
   tcgaacttcc tgcggacgct gtcggcggcg ttcggcacgg cgctgagcgt gacgtggtgg 1800
   gacaaccggg cgacgtacca ctacgcggtg gtgtcgcaat cggtgacgcg cgcctcggag 1860
   aacacgcagc ggtacgtgga cgcgctgcac gcgatggggc tgcacggcgc gcgggagctg 1920
   agctcgctgc accaggtggt gcggcagcag gcgtacatga tggcgacgaa cgacatgttc 1980
   tacatggcga gcgcgacgtg cctgctgctg gcggggctga tgtggctgac gcggccgaag 2040
   cggggcgcgg cggcggcgct cgggcactga ggcgaggcat gtcgcgcccc gcatgacgaa 2100
   ggcgaaggag aagggcgatg cgccgaagtc ctggggacgc ggcgcgtcga tgcggcaacg 2160
   aagcgggcat ttcggcattc cgaaccacca aagggaagag cgatgacgat cctgggggcg 2220
   ctggtgttcg ggcggctggg atcgttcgac gatgcgcgcg cgggcgcggc ggcgcgcgag 2280
   ccggtgcggc aggaatgaac ggaacgggcc gcagcgggat accggaaagc aagaaggacg 2340
   catcatacga atgacgcaga cagcaacgca agcagccact cgcgcgatga tcgcgacagg 2400
   aagccgcgcg gcgcgccggc tcgcggcagc cgcgctcgcg tgggcgctcg ccggctgcgt 2460
   gccgtcgggc ttcgagccgg cgctcgcgcc gcgcacgccg ggcgacgacg cgctcgcgca 2520
   cacggcgggg ggcgccgcgc acggcgcatg gccgagcccc gactgggtcc ggcagctcgg 2580
   cgatccgcaa ctcgacgcgc tcgtcgacga ggcgctgcgg cagaacccga cgctgcaggc 2640
   cgcgcaggcg cgcatcggcg tcgcgcagtc gcagctgcag cagttcgaat cgctgacggg 2700
   gctcaccgcg acggcgggcg cgtcgctctc caaggcgcac gtgccgcgct cgggcggcac 2760
   catcaatacg acgttcaacg gcttgccggt gtcggtgccg ctcgtcggcg aatcggtggt 2820
   gtcgtcgtcg tcgctgttcg tcgggctgaa ctatcagctg gacctgtggg gcaagaacgc 2880
   ggcggccacg cgcgggctgc tgtcgatgcg cgatgcggcg cgcgtggagg ccgagcaggc 2940
   gcggctcgcg ctgtcggtgg cgatcgtgac gctgtacggc gagctggacc gcgcgtatgc 3000
   gctgcgcgag ctgctgcagc agaagcgccg cgcgagcgag caggtggaga cggtgctgcg 3060
   cgagcgcgcg gcgcgcggga tcgacaacgg ctacgatgcg gacgacgcgg cgctcaagcg 3120
   gggcaagctg ctcgagcagc tcgcgctgac cgacgagcag atccagttgc agaagctgca 3180
   actgggggtg ctgagcgggc gggggccgga gcgcgggctg tcgctcgcgc ggccgaagct 3240
   cgcgccgctc gcggacgcgc cgctgccggc gcggctgccg gccgggctgc tggggcggcg 3300
   gccggacatc gtcgcggcgc ggctgcgggt ggaggcggcg tacgcggcga tcgacggcac 3360
   gcgcgcgtcg ttctacccgg acgtgaacct ggcggcgctg ggcgggctgt tcgcgctcac 3420
   gccggcgtcg ctgttcaagc acgatgcgct ggggggctcg atcggtccgg cgctgtcgct 3480
   gccgatcttc gatcgcggcc ggctgaaggc gaagctgggg ggcgacgtgg cgaacgcgga 3540
   cgtggcgctg gcgctgtaca accagacggt ggatgcggcg ctgggcgagg tggcgcggca 3600
   gttgacgtcg ctgtcgacgg tggatgcgct gctcgaggcg cagcagcagg cggtgcgctc 3660
   ggcgcagcgg atggtggcgc tggcgcagga ccggcaccgg cgggggatgg ggatgcgcaa 3720
   ggacgtgaac gtggcgaagc tgacgctgct ggacgagcgt gcgcacgtga tcgagctgca 3780
   ggcgcggcgg cggacgctgc gggtggggct gatcggggcg ctgggcggcg gcttcgacgc 3840
   gcggccggcg ggcggcgcgc cgctcgcgca gggcaagccg ttcgcggcgg cgagcgacag 3900
   gccgcccgat tgagcggcac gcacgcatgc ggcccgaagc caccgacacc cgaagacacc      3960
   gacaccaacg ccaccttcac cgtgtacacg agcgattcaa ccgacaccgc ccccgagcat      4020
   cgaagcccgt cgggccgatc cgcgacggct tgcgggccgg cccggccgtt gccggccggc 4080
   gccaccgaca tcacgcacgc gaagaccttg aacgataccg ccaccgatac cccgcgcgcg 4140
   aaggcgccca ccgatccggc cgccctcgac ggcgcgcacg cgcagcccgt gccggcgcac 4200
   gagcgcggat cgcctccgcc gccggaagcc gcggcgacgc tcgccgcgcg ccgcgcgacg 4260
   cgccgccggc gcttcgcgct gttcttcggg ctgctggcgc tggccgcgct gaccgcgggg 4320
   ctctactggt tcgtcgccgg gcgcttcagc gaggagacgg acgacgcgta cgtggccggc 4380
   aacgtggtgc agatcgccgc gcagatccag gggacggtga ccgacgtgct ggtggcggac 4440
   acgcagcagg tgaaggcggg gcaggcgctg gtgaagctcg acgacgcgga cgcgtcggcg 4500
   gcgttcgcgc aggcgcgggc gcagctcgcg caggcggtgc ggcaggtggc gaacacgcgg 4560
   ctctcgatgg ggatgtacga ggagacggtg aaggcgcgcg aggcggacct gaagcttgcg 4620
   cagcaggcgt atccggagga actggcgcgg cgaaagtcgt cgctggcgaa cgcgcaggcg 4680
   gcgctggcgg gggcgcaggc gcagctggag gcggcgcgcg cgctgggcag cgagcggccg 4740
   gtcgagcaga acccggcggt gcagcaggcg gccgcgcagt tcaagctggc gtaccggaac 4800
   ctgaggcgca cgacgatcgt gtcgccggtg gacggcacgg tcggtcagcg gtcggtgcag 4860
   atcggtcagc aggtggggcc gggggtgccg ctgatgtcgg tggtgcagtt gcggcaggtg 4920
   tgggtggagg cgaacttcaa ggaagggcag atccggcaca tgcgggtggg ccagccggtg 4980
   cggctcgaat cggacctgta cggcgcgcgg gtgacgtacc acggccgggt ggagggggtc 5040
   tcggcgggca cgggcagcgc gttctcgatg ctgccgtcgc agaacgcggc ggggaactgg 5100
   atcaaggtgg tgcagcgcct gccggtggtg atctcgctgg agccgtcgga gctggcggcg 5160
   cacccgctgc gggtggggct gtcgatgcgc gcgacggtgg agacgaaggt gcgtggcggc 5220
   cgcctgctcg acggcgacgc gccgctgccg gggctgcgca cgcgggtgca cgaagcgcag 5280
   gcgggcgagg ccgaggccgc ggcttcggca gtgattcggg agaatgacgg ccgcaggtga 5340
   cgggcggttg cgggatcgct ctaga 5365
<210> 71
   <211> 34
   <212> DNA
   <213> Artificial Sequence
<220>
   <223> primer
<400> 71
   tatatataac cggtattaat gcagctggca cgac 34
<210> 72
   <211> 20
   <212> DNA
   <213> Artificial Sequence
<220>
   <223> primer
<400> 72
   ggccgaccgg tactagtgga 20
<210> 73
   <211> 11960
   <212> DNA
   <213> Artificial Sequence
<220>
   <223> vector
<400> 73
   accttcggga gcgcctgaag cccgttctgg acgccctggg gccgttgaat cgggatatgc      60
   aggccaaggc cgccgcgatc atcaaggccg tgggcgaaaa gctgctgacg gaacagcggg     120
   aagtccagcg ccagaaacag gcccagcgcc agcaggaacg cgggcgcgca catttccccg     180
   aaaagtgcca cctgggatga atgtcagcta ctgggctatc tggacaaggg aaaacgcaag     240
   cgcaaagaga aagcaggtag cttgcagtgg gcttacatgg cgatagctag actgggcggt     300
   tttatggaca gcaagcgaac cggaattgcc agctggggcg ccctctggta aggttgggaa     360
   gccctgcaaa gtaaactgga tggctttctt gccgccaagg atctgatggc gcaggggatc     420
   aagatctgat caagagacag gatgaggatc gtttcgcatg attgaacaag atggattgca     480
   cgcaggttct ccggccgctt gggtggagag gctattcggc tatgactggg cacaacagac     540
   aatcggctgc tctgatgccg ccgtgttccg gctgtcagcg caggggcgcc cggttctttt     600
   tgtcaagacc gacctgtccg gtgccctgaa tgaactgcag gacgaggcag cgcggctatc     660
   gtggctggcc acgacgggcg ttccttgcgc agctgtgctc gacgttgtca ctgaagcggg     720
   aagggactgg ctgctattgg gcgaagtgcc ggggcaggat ctcctgtcat ctcaccttgc     780
   tcctgccgag aaagtatcca tcatggctga tgcaatgcgg cggctgcata cgcttgatcc     840
   ggctacctgc ccattcgacc accaagcgaa acatcgcatc gagcgagcac gtactcggat     900
   ggaagccggt cttgtcgatc aggatgatct ggacgaagag catcaggggc tcgcgccagc     960
   cgaactgttc gccaggctca aggcgcgcat gcccgacggc gaggatctcg tcgtgaccca    1020
   tggcgatgcc tgcttgccga atatcatggt ggaaaatggc cgcttttctg gattcatcga    1080
   ctgtggccgg ctgggtgtgg cggaccgcta tcaggacata gcgttggcta cccgtgatat    1140
   tgctgaagag cttggcggcg aatgggctga ccgcttcctc gtgctttacg gtatcgccgc    1200
   tcccgattcg cagcgcatcg ccttctatcg ccttcttgac gagttcttct gagcgggact    1260
   ctggggttcg aaatgaccga ccaagcgacg cccaacctgc catcacgaga tttcgattcc    1320
   accgccgcct tctatgaaag gttgggcttc ggaatcgttt tccgggacgc cggctggatg 1380
   atcctccagc gcggggatct catgctggag ttcttcgccc acccccatgg gcaaatatta 1440
   tacgcaaggc gacaaggtgc tgatgccgct ggcgattcag gttcatcatg ccgtttgtga 1500
   tggcttccat gtcggcagaa tgcttaatga attacaacag tttttatgca tgcgcccaat 1560
   acgcaaaccg cctctccccg cgcgttggcc gattcattaa tgcagctggc acgacaggtt 1620
   tcccgactgg aaagcgggca gtgagcgcaa cgcaattaat gtgagttagc tcactcatta 1680
   ggcaccccag gctttacact ttatgcttcc ggctcgtatg ttgtgtggaa ttgtgagcgg 1740
   ataacaattt cacacaggaa acagctatga ccatgattac gccaagcgcg caattaaccc 1800
   tcactaaagg gaacaaaagc tgggtaccgg gccccccctc gaggtcgacg gtatcgataa 1860
   gctttgggag gtgtgaaatg cggcgcgaaa gtctgttggt atcggtttgc aagggcctgc 1920
   gggtacatgt cgagcgcgtt gggcaggatc ccgggcgcag cacggtgatg ctggtcaacg 1980
   gcgcgatggc gaccaccgcc tcgttcgccc ggacctgcaa gtgcctggcc gaacatttca 2040
   acgtggtgct gttcgacctg cccttcgccg ggcagtcgcg tcagcacaac ccgcagcgcg 2100
   ggttgatcac caaggacgac gaggtggaaa tcctcctggc gctgatcgag cgcttcgagg 2160
   tcaatcacct ggtctccgcg tcctggggcg gtatctccac gctgctggcg ctgtcgcgca 2220
   atccgcgcgg catccgcagc tcggtggtga tggcattcgc ccctggactg aaccaggcga 2280
   tgctcgacta cgtcgggcgg gcgcaggcgc tgatcgagct ggacgacaag tcggcgatcg 2340
   gccatctgct caacgagacc gtcggcaaat acctgccgca gcgcctgaaa gccagcaacc 2400
   atcagcacat ggcttcgctg gccaccggcg aatacgagca ggcgcgcttt cacatcgacc 2460
   aggtgctggc gctcaacgat cggggctact tggcttgcct ggagcggatc cagagccacg 2520
   tgcatttcat caacggcagc tgggacgaat acaccaccgc cgaggacgcc cgccagttcc 2580
   gcgactacct gccgcactgc agtttctcgc gggtggaggg caccgggcat ttcctcgacc 2640
   tggagtccaa gctggcagcg gtacgcgtgc accgcgccct gctcgagcac ctgctgaagc 2700
   aaccggagcc gcagcgggcg gaacgcgcgg cgggattcca cgagatggcc atcggctacg 2760
   cctgaaccct tgacctgcga agacccggcc tggccgggct ttgcggttgc ataacgcacg 2820
   gagtagcccc atgcacgcca tcctcatcgc catcggctcg gccggcgacg tatttccctt 2880
   catcggcctg gcccggaccc tgaaactgcg cgggcaccgc gtgagcctct gcaccatccc 2940
   ggtgtttcgc gacgcggtgg agcagcacgg catcgcgttc gtcccgctga gcgacgaact 3000
   gacctaccgc cggaccatgg gcgatccgcg cctgtgggac cccaagacgt ccttcggcgt 3060
   gctctggcaa gccatcgccg ggatgatcga gccggtctac gagtacgtct cggcgcagcg 3120
   ccatgacgac atcgtggtgg tcggctcgct atgggcgctg ggcgcacgca tcgctcacga 3180
   gaagtacggg attccctacc tgtccgcgca ggtctcgcca tcgaccctgt tgtcggcgca 3240
   cctgccgccg gtacacccca agttcaacgt gcccgagcag atgccgctgg cgatgcgcaa 3300
   gctgctctgg cgctgcatcg agcgcttcaa gctggatcgc acctgcgcgc cggagatcaa 3360
   cgcggtgcgc cgcaaggtcg gcctggaaac gccggtgaag cgcatcttca cccaatggat 3420
   gcattcgccg cagggcgtgg tctgcctgtt cccggcctgg ttcgcgccgc cccagcagga 3480
   ttggccgcaa cccctgcaca tgaccggctt cccgctgttc gacggcagta tcccggggac 3540
   cccgctcgac gacgaactgc aacgctttct cgatcagggc agccggccgc tggtgttcac 3600
   ccagggctcg accgaacacc tgcagggcga cttctacgcc atggccctgc gcgcgctgga 3660
   acgcctcggc gcgcgtggga tcttcctcac cggcgccggc caggaaccgc tgcgcggctt 3720
   gccgaaccac gtgctgcagc gcgcctacgc gccactggga gccttgctgc catcgtgcgc 3780
   cgggctggtc catccgggcg gtatcggcgc catgagccta gccttggcgg cgggggtgcc 3840
   gcaggtgctg ctgccctgtg cccacgacca gttcgacaat gccgaacggc tggtccggct 3900
   cggctgcggg atgcgcctgg gcgtgccgtt gcgcgagcag gagttgcgcg gggcgctgtg 3960
   gcgcttgctc gaggacccgg ccatggcggc ggcctgtcgg cgtttcatgg aattgtcaca 4020
   accgcacagt atcgcttgcg gtaaagcggc ccaggtggtc gaacgttgtc atagggaggg 4080
   ggatgctcga tggctgaagg ctgcgtcctg aacggtgctg gcataacagt ctagagcggc 4140
   cgccaccgcg gtggagctcc aattcgccct atagtgagtc gtattacgcg cgctcactgg 4200
   ccgtcgtttt acaacgtcgt gactgggaaa accctggcgt tacccaactt aatcgccttg 4260
   cagcacatcc ccctttcgcc agctggcgta atagcgaaga ggcccgcacc gatcgccctt 4320
   cccaacagtt gcgcagcctg aatggcgaat ggaaattgta agcgttaata ttttgttaaa 4380
   attcgcgtta aatttttgtt aaatcagctc attttttaac caataggccg actgcgatga 4440
   gtggcagggc ggggcgtaat ttttttaagg cagttattgg tgcccttaaa cgcctggtgc 4500
   tacgcctgaa taagtgataa taagcggatg aatggcagaa attcgaaagc aaattcgacc 4560
   cggtcgtcgg ttcagggcag ggtcgttaaa tagccgctta tgtctattgc tggtttaccg 4620
   gtaggtcaac tttcgcaaca tccggcttga ccataacggc gctgtcggcc gcattgagaa 4680
   ctgcttcagc gataagctcc gcatcaccac tttcgtgcga agccgaggca taacggcctt 4740
   cttgatgacc gaacgcatgc caatgctgtt cgagcgcatc gtccgtgagc gaagataggt 4800
   ctgggtatcg attccgataa taagccagat ccagcacaaa tcgccttgag ccagtctttt 4860
   tctgggggac gggcactgaa tttcccttaa catgcatgct cggctgcaca tctacgctcg 4920
   gcaagccttc cttaacagac atttttcact ttcctatgaa tattcaagag cgccagaccg 4980
   ctgaaacatg aagaatgacg tctaacctgc cgagccccga ggctattata atttttgttg 5040
   ggttgttcaa catgaatgca agaactgcca tcactctaat tcctatttca ggaatactga 5100
   caaataaaaa tgacactttc caacccccgg aaacgaaccc gacttccaga tgcgcacccc 5160
   gcgctgccat gcccagcgaa ctagagtccc aggatagcgg tagcttaagg ccctctagac 5220
   ttgtcagcac ccagatttgt ctccgtgagt tgcatcgctc aaacaactgt ttcttgcatg 5280
   gacacctgac gactccctgt tgtgtctagg caaccatgag gtcaccttcc accgctgcaa 5340
   cggctcctcc tcttccagcg tgagcagatc ggcacggtca gtaccggcac ccagcagtgt 5400
   atttttcgac atcagggtgg tctaattccg gcagcgctag cagctcgcct tgcgttgccg 5460
   gggcgaagtt tctcagcgtc atctgtctac gacaacacct tttgtccaat tagagccaaa 5520
   ttatgattct agtaacaggc ggagccggct tcatcggctc aaatttcgta ctgcaatggt 5580
   gtgcgcacaa tgaggaaccc gtcctcaacc tcgacgccct gacctacgca ggcaacctgg 5640
   ccaacctgca gccgctggaa ggcaaccctc agcatcgctt tgtgcaaggc aatatttgcg 5700
   atgctgcgct tctgaccaag ctgttcgcag agcaccgccc gcgcgccgtg gttcacttcg 5760
   cggcggaatc ccatgtagac cgctcaatca ccggccccga agcgtttgtc gaaaccaacg 5820
   tgatgggcac gtttcgcttg cttgaagccg cccgggcgca ttggaatagt ttggaaggtg 5880
   cagagaagga ggccttccgt ttcctccatg tctctaccga cgaagtctac ggcacactag 5940
   ggccaaacga cccggcgttc accgaaacca cgccgtacgc gccgaacagc ccatactccg 6000
   ccagcaaggc agccagcgac catctggtac gctcgtattt ccatacctac ggcatgccgg 6060
   tactcactac caactgctcc aacaattacg ggccgctcca cttcccggaa aaactgatcc 6120
   cgctgatgat cgtcaacgca ctcgccggta aggcgctgcc tgtctatggc gacggccagc 6180
   aaatccgcga ctggctgtat gtcgaagatc actgctcggg catccgtcgc gtactggaag 6240
   ccggtgcgtt cggcgagacg tacaatattg gcggctggaa tgaaaaagcc aacattgaca 6300
   ttgtgcgtac actctgcagc cttctcgacg agatggcacc tgcggcatcg cgccaggtaa 6360
   tcaatcagaa gaccggcgag cctgtcgaac agtatgcaga actcatcgcc tacgtaaccg 6420
   accgcccagg ccatgaccgc cgttatgcca tcgatgcacg caagatcgag cgggagctcg 6480
   gctggaaacc tgccgaaacc ttcgagacgg gcattcgaaa gacagtcgct tggtacttgg 6540
   ccaaccagaa atgggtaaaa ggtgtcatgg acggcagcta ccgtgactgg gtggcacaac 6600
   aatacggggc aaataaagcg tgaaaatcct gctgttgggg aaaaacgggc aagtaggctg 6660
   ggagctacag cgcgccttgg cgccgctggg tgaggtcatt gcgctggatc gtcagggggc 6720
   cgagggctta tgtggcgact tgtccaacct ggacggcttg gccgctacga ttcgtcagct 6780
   ggcgccggac gtgatcgtca acgctgctgc ctacactgca gtggataaag ctgagagcga 6840
   tcaggcactg gctgcaatga tcaatgccgc ggctcctgct gtattagcac gtgaaacagc 6900
   agctttgggc gcctggttga ttcactattc caccgattat gtatttgacg gcagcggcag 6960
   tcagcgctgg gaggaaactg cgcctaccgg ccccctttcg gtctacggcc ggaccaagct 7020
   ggaaggcgag catgccattc tcgccagcgg cgccaaggcc gtggtactgc gcaccagctg 7080
   ggtgtatgct gcgcgcgggc acaattttgc caagaccatg ctgcgcctgg cggcggagcg 7140
   tgagacgttg agcgtggtag cagaccaatt tggcgcaccc acgggcgctg acctgatcgc 7200
   cgacgttact gcacacatcc tgcggcaaat cttcaatggg caagacaacc gtcacctggc 7260
   agggatttac cacttggctg cgtccggtga aacctcttgg catggttttg ctcagttcgt 7320
   gctggcgcat gctcaacgca ctggcgtagc gctgaaagtg acagctgata aggttgccgc 7380
   aatcagcacc gaagcttatc cagtacctgc accacgtccg cgcaactcgc gcctggcact 7440
   gggcaaactg gaaaacacgt tcaatttcaa aatgccgctt tgggagcaag gcgtgcaacg 7500
   tatgctggac gaaatccagt aatagggact ctcatggctc gtaaaggaat tattctggcc 7560
   ggcggttcgg gtacacgcct gcatccggcc acactttcgg tttcgaagca gctgctgccg 7620
   gtgtatgaca aaccgatgat ctactacccg ctgagcaccc tgctgctcgc tggtatccgg 7680
   gacatcctga tcatttccac cccgcaggac accccgcgct tcgaacagct gctgggcgat 7740
   ggcagccagt ggggcctgaa cctgtcatac gcaatacaac caagcccgga tggcttggcg 7800
   caagcgttca ccatcggcgc tgacttcatc ggtaacgacc cttctgcgtt ggttctcggt 7860
   gacaatattt tctacggcca tgacttccag gcactgctat tgaacgcaga taaacgtgaa 7920
   tccggtgctt cagtattcgc ttatcatgtt catgacccag aacgctatgg cgtagcggag 7980
   tttgacgata gcggtcgcgt attgtcgctg gaagaaaaac cggcagttcc aaagtctagc 8040
   tatgcggtca ccggcctgta tttctatgac aatcaggtag tcaatctggc tcgcgagctg 8100
   aagccttccc cacgtggcga gctggaaatc accgacctca acaaccttta cttgcagcag 8160
   cagcagttgc aggtcgaaat catgggccgt ggctatgcgt ggctcgacac cggcacgcac 8220
   gacagtctgc tggaggctag ccagtacatc gcaaccatgg agcgccgtca gggcttgaaa 8280
   gtcgcctgcc ctgaggaaat ttgctaccgc gctggctgga tcaacgctga gcaactcgag 8340
   tgcctggctc aaccactgct gaaaaacggt tatggcaagt atctgcagaa cttgctgaaa 8400
   gagaaggtgt tctgatgcaa gccattccgc tggatatccc cgaagtcgtg ctgtttaccc 8460
   ccaaggtttt tggcgacgaa cgtggtttct tctacgagag cttcaacgcc cgtgttttca 8520
   gcgaagtgac cggcctgcag cccgacttcg tacaagacaa ccactcgcgc tcggtaaaag 8580
   gcgtgctccg tggcctgcac tatcagctgg cacctcacgc ccagggcaag ctggtgcgtg 8640
   tggtgcaagg cgaagtcttc gatgttgcgg tggatatccg tcgctcgtcc acaaccttcg 8700
   gtaaatgggt aggtgcggtg ttgtcggccg agaacaagaa ccagctgtgg atcccgccag 8760
   ggttcgcaca cgggttcgtc acgttgagtg aaaccgcaga gttcctctac aagaccaccg 8820
   acttctactc gccgcagtgc gagcgctgca ttgcctggaa tgatccggca gtgggtatcg 8880
   aatggcccat cgactccgta ccaagcttgt ctggcaagga ccagcttggg gtcgcattgg 8940
   ctgacgccga actgttcgac taacggtttt agcggagaag ggctgcggta gcgcagcctt 9000
   gtctctgaac acatgccata ccgggtcttg ccgatagtgg cgtttttcac acgccactaa 9060
   gaagcaaccg ctgcatggcc tggcaaataa tcagaatttg ccccttcctt gtaggccatt 9120
   tcccaaagat acccctgcgc ctgttttcca ttgcacacgt taaacgtgag acttagtctc 9180
   gacccgtcgc tgccaaatca gtgaccggtt tattgactac cggaagcagt gtgaccgtgt 9240
   gcttctcaaa tgcctgaggc cagtttgctc aggctctccc cgtggaggta ataattgacg 9300
   atatgatcat ttattctgcc tcccagagcc tgataaaaac ggtgaatccg ttagcgaggt 9360
   gccgccggct tccattcagg tcgaggtggc ccggctccat gcaccgcgac gcaacgcggg 9420
   gaggcagaca aggtataggg cggcgaggcg gctacagccg atagtctgga acagcgcact 9480
   tacgggttgc tgcgcaaccc aagtgctacc ggcgcggcag cgtgacccgt gtcggcggct 9540
   ccaacggctc gccatcgtcc agaaaacacg gctcatcggg catcggcagg cgctgctgcc 9600
   cgcgccgttc ccattcctcc gtttcggtca aggctggcag gtctggttcc atgcccggaa 9660
   tgccgggctg gctgggcggc tcctcgccgg ggccggtcgg tagttgctgc tcgcccggat 9720
   acagggtcgg gatgcggcgc aggtcgccat gccccaacag cgattcgtcc tggtcgtcgt 9780
   gatcaaccac cacggcggca ctgaacaccg acaggcgcaa ctggtcgcgg ggctggcccc 9840
   acgccacgcg gtcattgacc acgtaggccg acacggtgcc ggggccgttg agcttcacga 9900
   cggagatcca gcgctcggcc accaagtcct tgactgcgta ttggaccgtc cgcaaagaac 9960
   gtccgatgag cttggaaagt gtcttctggc tgaccaccac ggcgttctgg tggcccatct 10020
   gcgccacgag gtgatgcagc agcattgccg ccgtgggttt cctcgcaata agcccggccc 10080
   acgcctcatg cgctttgcgt tccgtttgca cccagtgacc gggcttgttc ttggcttgaa 10140
   tgccgatttc tctggactgc gtggccatgc ttatctccat gcggtagggt gccgcacggt 10200
   tgcggcacca tgcgcaatca gctgcaactt ttcggcagcg cgacaacaat tatgcgttgc 10260
   gtaaaagtgg cagtcaatta cagattttct ttaacctacg caatgagcta ttgcgggggg 10320
   tgccgcaatg agctgttgcg tacccccctt ttttaagttg ttgattttta agtctttcgc 10380
   atttcgccct atatctagtt ctttggtgcc caaagaaggg cacccctgcg gggttccccc 10440
   acgccttcgg cgcggctccc cctccggcaa aaagtggccc ctccggggct tgttgatcga 10500
   ctgcgcggcc ttcggccttg cccaaggtgg cgctgccccc ttggaacccc cgcactcgcc 10560
   gccgtgaggc tcggggggca ggcgggcggg cttcgccttc gactgccccc actcgcatag 10620
   gcttgggtcg ttccaggcgc gtcaaggcca agccgctgcg cggtcgctgc gcgagccttg 10680
   acccgccttc cacttggtgt ccaaccggca agcgaagcgc gcaggccgca ggccggaggc 10740
   ttttccccag agaaaattaa aaaaattgat ggggcaaggc cgcaggccgc gcagttggag 10800
   ccggtgggta tgtggtcgaa ggctgggtag ccggtgggca atccctgtgg tcaagctcgt 10860
   gggcaggcgc agcctgtcca tcagcttgtc cagcagggtt gtccacgggc cgagcgaagc 10920
   gagccagccg gtggccgctc gcggccatcg tccacatatc cacgggctgg caagggagcg 10980
   cagcgaccgc gcagggcgaa gcccggagag caagcccgta gggcgccgca gccgccgtag 11040
   gcggtcacga ctttgcgaag caaagtctag tgagtatact caagcattga gtggcccgcc 11100
   ggaggcaccg ccttgcgctg cccccgtcga gccggttgga caccaaaagg gaggggcagg 11160
   catggcggca tacgcgatca tgcgatgcaa gaagctggcg aaaatgggca acgtggcggc 11220
   cagtctcaag cacgcctacc gcgagcgcga gacgcccaac gctgacgcca gcaggacgcc 11280
   agagaacgag cactgggcgg ccagcagcac cgatgaagcg atgggccgac tgcgcgagtt 11340
   gctgccagag aagcggcgca aggacgctgt gttggcggtc gagtacgtca tgacggccag 11400
   cccggaatgg tggaagtcgg ccagccaaga acagcaggcg gcgttcttcg agaaggcgca 11460
   caagtggctg gcggacaagt acggggcgga tcgcatcgtg acggccagca tccaccgtga 11520
   cgaaaccagc ccgcacatga ccgcgttcgt ggtgccgctg acgcaggacg gcaggctgtc 11580
   ggccaaggag ttcatcggca acaaagcgca gatgacccgc gaccagacca cgtttgcggc 11640
   cgctgtggcc gatctagggc tgcaacgggg catcgagggc agcaaggcac gtcacacgcg 11700
   cattcaggcg ttctacgagg ccctggagcg gccaccagtg ggccacgtca ccatcagccc 11760
   gcaagcggtc gagccacgcg cctatgcacc gcagggattg gccgaaaagc tgggaatctc 11820
   aaagcgcgtt gagacgccgg aagccgtggc cgaccggctg acaaaagcgg ttcggcaggg 11880
   gtatgagcct gccctacagg ccgccgcagg agcgcgtgag atgcgcaaga aggccgatca 11940
   agcccaagag acggcccgag 11960
<210> 74
   <211> 13289
   <212> DNA
   <213> Artificial Sequence
<220>
   <223> vector
<400> 74
   ccggtcactg atttggcagc gacgggtcga gactaagtct cacgtttaac gtgtgcaatg        60
   gaaaacaggc gcaggggtat ctttgggaaa tggcctacaa ggaaggggca aattctgatt       120
   atttgccagg ccatgcagcg gttgcttctt agtggcgtgt gaaaaacgcc actatcggca       180
   agacccggta tggcatgtgt tcagagacaa ggctgcgcta ccgcagccct tctccgctaa       240
   aaccgttagt cgaacagttc ggcgtcagcc aatgcgaccc caagctggtc cttgccagac       300
   aagcttggta cggagtcgat gggccattcg atacccactg ccggatcatt ccaggcaatg       360
   cagcgctcgc actgcggcga gtagaagtcg gtggtcttgt agaggaactc tgcggtttca       420
   ctcaacgtga cgaacccgtg tgcgaaccct ggcgggatcc acagctggtt cttgttctcg       480
   gccgacaaca ccgcacctac ccatttaccg aaggttgtgg acgagcgacg gatatccacc       540
   gcaacatcga agacttcgcc ttgcaccaca cgcaccagct tgccctgggc gtgaggtgcc       600
   agctgatagt gcaggccacg gagcacgcct tttaccgagc gcgagtggtt gtcttgtacg       660
   aagtcgggct gcaggccggt cacttcgctg aaaacacggg cgttgaagct ctcgtagaag       720
   aaaccacgtt cgtcgccaaa aaccttgggg gtaaacagca cgacttcggg gatatccagc 780
   ggaatggctt gcatcagaac accttctctt tcagcaagtt ctgcagatac ttgccataac 840
   cgtttttcag cagtggttga gccaggcact cgagttgctc agcgttgatc cagccagcgc 900
   ggtagcaaat ttcctcaggg caggcgactt tcaagccctg acggcgctcc atggttgcga 960
   tgtactggct agcctccagc agactgtcgt gcgtgccggt gtcgagccac gcatagccac 1020
   ggcccatgat ttcgacctgc aactgctgct gctgcaagta aaggttgttg aggtcggtga 1080
   tttccagctc gccacgtggg gaaggcttca gctcgcgagc cagattgact acctgattgt 1140
   catagaaata caggccggtg accgcatagc tagactttgg aactgccggt ttttcttcca 1200
   gcgacaatac gcgaccgcta tcgtcaaact ccgctacgcc atagcgttct gggtcatgaa 1260
   catgataagc gaatactgaa gcaccggatt cacgtttatc tgcgttcaat agcagtgcct 1320
   ggaagtcatg gccgtagaaa atattgtcac cgagaaccaa cgcagaaggg tcgttaccga 1380
   tgaagtcagc gccgatggtg aacgcttgcg ccaagccatc cgggcttggt tgtattgcgt 1440
   atgacaggtt caggccccac tggctgccat cgcccagcag ctgttcgaag cgcggggtgt 1500
   cctgcggggt ggaaatgatc aggatgtccc ggataccagc gagcagcagg gtgctcagcg 1560
   ggtagtagat catcggtttg tcatacaccg gcagcagctg cttcgaaacc gaaagtgtgg 1620
   ccggatgcag gcgtgtaccc gaaccgccgg ccagaataat tcctttacga gccatgagag 1680
   tccctattac tggatttcgt ccagcatacg ttgcacgcct tgctcccaaa gcggcatttt 1740
   gaaattgaac gtgttttcca gtttgcccag tgccaggcgc gagttgcgcg gacgtggtgc 1800
   aggtactgga taagcttcgg tgctgattgc ggcaacctta tcagctgtca ctttcagcgc 1860
   tacgccagtg cgttgagcat gcgccagcac gaactgagca aaaccatgcc aagaggtttc 1920
   accggacgca gccaagtggt aaatccctgc caggtgacgg ttgtcttgcc cattgaagat 1980
   ttgccgcagg atgtgtgcag taacgtcggc gatcaggtca gcgcccgtgg gtgcgccaaa 2040
   ttggtctgct accacgctca acgtctcacg ctccgccgcc aggcgcagca tggtcttggc 2100
   aaaattgtgc ccgcgcgcag catacaccca gctggtgcgc agtaccacgg ccttggcgcc 2160
   gctggcgaga atggcatgct cgccttccag cttggtccgg ccgtagaccg aaagggggcc 2220
   ggtaggcgca gtttcctccc agcgctgact gccgctgccg tcaaatacat aatcggtgga 2280
   atagtgaatc aaccaggcgc ccaaagctgc tgtttcacgt gctaatacag caggagccgc 2340
   ggcattgatc attgcagcca gtgcctgatc gctctcagct ttatccactg cagtgtaggc 2400
   agcagcgttg acgatcacgt ccggcgccag ctgacgaatc gtagcggcca agccgtccag 2460
   gttggacaag tcgccacata agccctcggc cccctgacga tccagcgcaa tgacctcacc 2520
   cagcggcgcc aaggcgcgct gtagctccca gcctacttgc ccgtttttcc ccaacagcag 2580
   gattttcacg ctttatttgc cccgtattgt tgtgccaccc agtcacggta gctgccgtcc 2640
   atgacacctt ttacccattt ctggttggcc aagtaccaag cgactgtctt tcgaatgccc 2700
   gtctcgaagg tttcggcagg tttccagccg agctcccgct cgatcttgcg tgcatcgatg 2760
   gcataacggc ggtcatggcc tgggcggtcg gttacgtagg cgatgagttc tgcatactgt 2820
   tcgacaggct cgccggtctt ctgattgatt acctggcgcg atgccgcagg tgccatctcg 2880
   tcgagaaggc tgcagagtgt acgcacaatg tcaatgttgg ctttttcatt ccagccgcca 2940
   atattgtacg tctcgccgaa cgcaccggct tccagtacgc gacggatgcc cgagcagtga 3000
   tcttcgacat acagccagtc gcggatttgc tggccgtcgc catagacagg cagcgcctta 3060
   ccggcgagtg cgttgacgat catcagcggg atcagttttt ccgggaagtg gagcggcccg 3120
   taattgttgg agcagttggt agtgagtacc ggcatgccgt aggtatggaa atacgagcgt 3180
   accagatggt cgctggctgc cttgctggcg gagtatgggc tgttcggcgc gtacggcgtg 3240
   gtttcggtga acgccgggtc gtttggccct agtgtgccgt agacttcgtc ggtagagaca 3300
   tggaggaaac ggaaggcctc cttctctgca ccttccaaac tattccaatg cgcccgggcg 3360
   gcttcaagca agcgaaacgt gcccatcacg ttggtttcga caaacgcttc ggggccggtg 3420
   attgagcggt ctacatggga ttccgccgcg aagtgaacca cggcgcgcgg gcggtgctct 3480
   gcgaacagct tggtcagaag cgcagcatcg caaatattgc cttgcacaaa gcgatgctga 3540
   gggttgcctt ccagcggctg caggttggcc aggttgcctg cgtaggtcag ggcgtcgagg 3600
   ttgaggacgg gttcctcatt gtgcgcacac cattgcagta cgaaatttga gccgatgaag 3660
   ccggctccgc ctgttactag aatcataatt tggctctaat tggacaaaag gtgttgtcgt 3720
   agacagatga cgctgagaaa cttcgccccg gcaacgcaag gcgagctgct agcgctgccg 3780
   gaattagacc accctgatgt cgaaaaatac actgctgggt gccggtactg accgtgccga 3840
   tctgctcacg ctggaagagg aggagccgtt gcagcggtgg aaggtgacct catggttgcc 3900
   tagacacaac agggagtcgt caggtgtcca tgcaagaaac agttgtttga gcgatgcaac 3960
   tcacggagac aaatctgggt gctgacaagt ctagagggcc ttaagctacc gctatcctgg 4020
   gactctagtt cgctgggcat ggcagcgcgg ggtgcgcatc tggaagtcgg gttcgtttcc 4080
   gggggttgga aagtgtcatt tttatttgtc agtattcctg aaataggaat tagagtgatg 4140
   gcagttcttg cattcatgtt gaacaaccca acaaaaatta taatagcctc ggggctcggc 4200
   aggttagacg tcattcttca tgtttcagcg gtctggcgct cttgaatatt cataggaaag 4260
   tgaaaaatgt ctgttaagga aggcttgccg agcgtagatg tgcagccgag catgcatgtt 4320
   aagggaaatt cagtgcccgt cccccagaaa aagactggct caaggcgatt tgtgctggat 4380
   ctggcttatt atcggaatcg atacccagac ctatcttcgc tcacggacga tgcgctcgaa 4440
   cagcattggc atgcgttcgg tcatcaagaa ggccgttatg cctcggcttc gcacgaaagt 4500
   ggtgatgcgg agcttatcgc tgaagcagtt ctcaatgcgg ccgacagcgc cgttatggtc 4560
   aagccggatg ttgcgaaagt tgacctaccg gtaaaccagc aatagacata agcggctatt 4620
   taacgaccct gccctgaacc gacgaccggg tcgaatttgc tttcgaattt ctgccattca 4680
   tccgcttatt atcacttatt caggcgtagc accaggcgtt taagggcacc aataactgcc 4740
   ttaaaaaaat tacgccccgc cctgccactc atcgcagtcg gcctattggt taaaaaatga 4800
   gctgatttaa caaaaattta acgcgaattt taacaaaata ttaacgctta caatttccat 4860
   tcgccattca ggctgcgcaa ctgttgggaa gggcgatcgg tgcgggcctc ttcgctatta 4920
   cgccagctgg cgaaaggggg atgtgctgca aggcgattaa gttgggtaac gccagggttt 4980
   tcccagtcac gacgttgtaa aacgacggcc agtgagcgcg cgtaatacga ctcactatag 5040
   ggcgaattgg agctccaccg cggtggcggc cgctctagaa gtaccaccag cacgcccatg 5100
   tctatccggt ccatgatcgt tcttctcccg taggtcgaag ttgccaggcc aggaccagcc 5160
   cggccagaac gagaagggcg ccggcgagga atggcgcgcc ggccaggggc agcggcgcga 5220
   gcggaccgct gccccagtgg aacaggccgc tcatcagcgg cggaccgacg atcgcggcga 5280
   ggctcatcag gctgctcagc acgccctgca actcgccctg gcggtcgacc ggcacgcggg 5340
   ccgagagcag cccctgcatg gccggggtgg cgaggctgcc gagcgcgaag ggcagcagcg 5400
   cgcagaccag ccagaatgac gagtcgacca gggcgaacag cagcaggccg cagccttgca 5460
   gggcgaggcc caggcgcagc aggcgggcgt cgtccaggcg ccgcttgcag aggttcacgc 5520
   cgagggtctg ggcgagcacc gcgagcacgc cgtagagggc cagcgagtag ccgatccagg 5580
   cgctgctcca gtgaaacttc tcgatcacga agaacggcca gaccaccatc accgcctgca 5640
   agccgaggaa taccagggca agcaccgcca gcaggcgtcc gacccccggt tgccgagcca 5700
   ggccgctgat cgagcgcaag gcattcatcc gcctcgggtc caggcggcgg cgtcgcgtcg 5760
   ggggcagggt ttcctcgagg aacaggccgg cgagcagggc gttgagcagg cacaggccgg 5820
   cggccagcaa cagcggcagc gtcgtgccgt gcaccgccag cagcccaccg agggcggggc 5880
   cgaggatcat gcccagggcg aggccggcgt acagccagcc gaagtgccgg gtgcgctgcc 5940
   cgtgcgtgcc gaggtcagcc gcgcaggcca tcgcggtggc cacgctggcg ccggtgagcc 6000
   cggccagcgc gcgaccgagg aacagcatcc agaggctgtc ggccagcgcc agcagcagat 6060
   agctgagggc gaagccgagc atcgccagga ccaggacggg gcggcgtccg aagcggtcgc 6120
   tgaggctgcc gaggaccggc gaaaagaaca attgcagcag cgcgaaggtc atcaccaggg 6180
   cggcgcccca ggtggccgcg tcgcggaccg ccagcggcgc cacgctgccg atcagcgtcg 6240
   gcagcagggg cacgatcagg ccgacgccag cggcatccag caggcaggtg aggaacagca 6300
   gaggcaggac gcgtttcgcg ccgggaccgt gttcccgcgt ggcggagggg cagaggctgg 6360
   tcgtggacac gccaggatcc tcccggcgtc aggacgcagc cttcagccat cgcgcatccc 6420
   cctccctatg acaacgttcg accacctggg ccgctttacc gcaagcgata ctgtgcggtt 6480
   gtgacaattc catgaaacgc cgacaggccg ccgccatggc cgggtcctcg agcaagcgcc 6540
   acagcgcccc gcgcaactcc tgctcgcgca atggcacgcc caggcgcatc ccgcagccga 6600
   gccggaccag ccgttcggca ttgtcgaact ggtcgtgggc gcagggcagc agcacctgcg 6660
   gcacccccgc cgccaaggcc aggctcatgg cgccgatacc gcccggatgg accagcccgg 6720
   cgcacgatgg cagcaaggct cccagtggcg cgtaggcgcg ctgcagcacg tggttcggca 6780
   agccgcgcag cggttcctgg ccggcgccgg tgaggaagat cccacgcgcg ccgaggcgtt 6840
   ccagcgcgcg cagggccatg gcgtagaagt cgccctgcag gtgttcggtc gagccctggg 6900
   tgaacaccag cggccggctg ccctgatcga gaaagcgttg cagttcgtcg tcgagcgggg 6960
   tccccgggat actgccgtcg aacagcggga agccggtcat gtgcaggggt tgcggccaat 7020
   cctgctgggg cggcgcgaac caggccggga acaggcagac cacgccctgc ggcgaatgca 7080
   tccattgggt gaagatgcgc ttcaccggcg tctccaggcc gaccttgcgc cgcaccgcgt 7140
   tgatatccgg cgcgcaggtg cgatccagct tgaagcgctc gatgcagcgc cagagcagct 7200
   tgcgcatcgc cagcggcatc tgctcgggca cgttgaactt ggggtgtacc ggcggcaggt 7260
   gcgccgacaa caaggtcgat ggcgagacct gcgcggacag gtagggaatc ccgtacttct 7320
   cgtgagcgat gcgtgcgccc agcgcccaga gcgagccgac caccacgatg tcgtcatggc 7380
   gctgcgccga gacgtactcg tagaccggct cgatcatccc ggcgatggtt tgccagagca 7440
   cgccgaagga cgtcttgggg tcccacaggc gcggatcgcc catggtccgg cggtaggtca 7500
   gttcgtcgct cagcgggacg aacgcgatgc cgtgctgctc caccgcgtcg cgaaacaccg 7560
   ggatggtgca gaggctcacg cggtgcccgc gcaatttcag ggtccgggcc aggccgatga 7620
   agggaaatac gtcgccggcc gagccgatgg cgatgaggat ggcgtgcatg gtgctactcc 7680
   gtgcgttatg caaccgcaaa gcccggccag gccgggtctt cgcaggtcaa gggttcaggc 7740
   gtagccgatg gccatctcgt ggaatcccgc cgcgcgttcc gcccgctgcg gctccggttg 7800
   cttcagcagg tgctcgagca gggcgcggtg cacgcgtacc gcggccagct tggactccag 7860
   gtcgaggaaa tgcccggtgc cctccacccg cgagaaactg cagtgcggca ggtagtcgcg 7920
   gaactggcgg gcgtcctcgg cggtggtgta ttcgtcccag ctgccgttga tgaaatgcac 7980
   gtggctctgg atccgctcca ggcaagccag gtagccccga tcgttgagcg ccagcacctg 8040
   gtcgatgtga aagcgcgcct gctcgtattc gccggtggcc agcgaagcca tgtgctgatg 8100
   gttgctggct ttcaggcgcg gcggcaggta tttgccgacg gtctcgttga gcagatggcc 8160
   gatcgccgac ttgtcgtcca gctcgatcag cgcctgcgcc cgcccgacgt agtcgagcat 8220
   cgcctggttc agtccagggg cgaatgccat caccaccgag ctgcggatgc cgcgcggatt 8280
   gcgcgacagc gccagcagcg tggagatacc gccccaggac gcggagacca ggtgattgac 8340
   ctcgaagcgc tcgatcagcg ccaggaggat ttccacctcg tcgtccttgg tgatcaaccc 8400
   ccgctgcggg ttgtgctgac gcgactgccc ggcgaagggc aggtcgaaca gcaccacgtt 8460
   gaaatgttcg gccaggcact tgcaggtccg ggcgaacgag gcggtggtcg ccatcgcgcc 8520
   gttgaccagc atcaccgtgc tgcgcccggg atcctgccca acgcgctcga catgtacccg 8580
   caggcccttg caaaccgata ccaacagact ttcgcgccgc atttcacacc tcccaaaaat 8640
   gccagatccc ccgggctgca ggaattcgat atcaagctta tcgataccgt cgacctcgag 8700
   ggggggcccg gtacccagct tttgttccct ttagtgaggg ttaattgcgc gcttggcgta 8760
   atcatggtca tagctgtttc ctgtgtgaaa ttgttatccg ctcacaattc cacacaacat 8820
   acgagccgga agcataaagt gtaaagcctg gggtgcctaa tgagtgagct aactcacatt 8880
   aattgcgttg cgctcactgc ccgctttcca gtcgggaaac ctgtcgtgcc agctgcatta 8940
   atgaatcggc caacgcgcgg ggagaggcgg tttgcgtatt gggcgcatgc ataaaaactg 9000
   ttgtaattca ttaagcattc tgccgacatg gaagccatca caaacggcat gatgaacctg 9060
   aatcgccagc ggcatcagca ccttgtcgcc ttgcgtataa tatttgccca tgggggtggg 9120
   cgaagaactc cagcatgaga tccccgcgct ggaggatcat ccagccggcg tcccggaaaa 9180
   cgattccgaa gcccaacctt tcatagaagg cggcggtgga atcgaaatct cgtgatggca 9240
   ggttgggcgt cgcttggtcg gtcatttcga accccagagt cccgctcaga agaactcgtc 9300
   aagaaggcga tagaaggcga tgcgctgcga atcgggagcg gcgataccgt aaagcacgag 9360
   gaagcggtca gcccattcgc cgccaagctc ttcagcaata tcacgggtag ccaacgctat 9420
   gtcctgatag cggtccgcca cacccagccg gccacagtcg atgaatccag aaaagcggcc 9480
   attttccacc atgatattcg gcaagcaggc atcgccatgg gtcacgacga gatcctcgcc 9540
   gtcgggcatg cgcgccttga gcctggcgaa cagttcggct ggcgcgagcc cctgatgctc 9600
   ttcgtccaga tcatcctgat cgacaagacc ggcttccatc cgagtacgtg ctcgctcgat 9660
   gcgatgtttc gcttggtggt cgaatgggca ggtagccgga tcaagcgtat gcagccgccg 9720
   cattgcatca gccatgatgg atactttctc ggcaggagca aggtgagatg acaggagatc 9780
   ctgccccggc acttcgccca atagcagcca gtcccttccc gcttcagtga caacgtcgag 9840
   cacagctgcg caaggaacgc ccgtcgtggc cagccacgat agccgcgctg cctcgtcctg 9900
   cagttcattc agggcaccgg acaggtcggt cttgacaaaa agaaccgggc gcccctgcgc 9960
   tgacagccgg aacacggcgg catcagagca gccgattgtc tgttgtgccc agtcatagcc 10020
   gaatagcctc tccacccaag cggccggaga acctgcgtgc aatccatctt gttcaatcat 10080
   gcgaaacgat cctcatcctg tctcttgatc agatcttgat cccctgcgcc atcagatcct 10140
   tggcggcaag aaagccatcc agtttacttt gcagggcttc ccaaccttac cagagggcgc 10200
   cccagctggc aattccggtt cgcttgctgt ccataaaacc gcccagtcta gctatcgcca 10260
   tgtaagccca ctgcaagcta cctgctttct ctttgcgctt gcgttttccc ttgtccagat 10320
   agcccagtag ctgacattca tcccaggtgg cacttttcgg ggaaatgtgc gcgcccgcgt 10380
   tcctgctggc gctgggcctg tttctggcgc tggacttccc gctgttccgt cagcagcttt 10440
   tcgcccacgg ccttgatgat cgcggcggcc ttggcctgca tatcccgatt caacggcccc 10500
   agggcgtcca gaacgggctt caggcgctcc cgaaggtctc gggccgtctc ttgggcttga 10560
   tcggccttct tgcgcatctc acgcgctcct gcggcggcct gtagggcagg ctcatacccc 10620
   tgccgaaccg cttttgtcag ccggtcggcc acggcttccg gcgtctcaac gcgctttgag 10680
   attcccagct tttcggccaa tccctgcggt gcataggcgc gtggctcgac cgcttgcggg 10740
   ctgatggtga cgtggcccac tggtggccgc tccagggcct cgtagaacgc ctgaatgcgc 10800
   gtgtgacgtg ccttgctgcc ctcgatgccc cgttgcagcc ctagatcggc cacagcggcc 10860
   gcaaacgtgg tctggtcgcg ggtcatctgc gctttgttgc cgatgaactc cttggccgac 10920
   agcctgccgt cctgcgtcag cggcaccacg aacgcggtca tgtgcgggct ggtttcgtca 10980
   cggtggatgc tggccgtcac gatgcgatcc gccccgtact tgtccgccag ccacttgtgc 11040
   gccttctcga agaacgccgc ctgctgttct tggctggccg acttccacca ttccgggctg 11100
   gccgtcatga cgtactcgac cgccaacaca gcgtccttgc gccgcttctc tggcagcaac 11160
   tcgcgcagtc ggcccatcgc ttcatcggtg ctgctggccg cccagtgctc gttctctggc 11220
   gtcctgctgg cgtcagcgtt gggcgtctcg cgctcgcggt aggcgtgctt gagactggcc 11280
   gccacgttgc ccattttcgc cagcttcttg catcgcatga tcgcgtatgc cgccatgcct 11340
   gcccctccct tttggtgtcc aaccggctcg acgggggcag cgcaaggcgg tgcctccggc 11400
   gggccactca atgcttgagt atactcacta gactttgctt cgcaaagtcg tgaccgccta 11460
   cggcggctgc ggcgccctac gggcttgctc tccgggcttc gccctgcgcg gtcgctgcgc 11520
   tcccttgcca gcccgtggat atgtggacga tggccgcgag cggccaccgg ctggctcgct 11580
   tcgctcggcc cgtggacaac cctgctggac aagctgatgg acaggctgcg cctgcccacg 11640
   agcttgacca cagggattgc ccaccggcta cccagccttc gaccacatac ccaccggctc 11700
   caactgcgcg gcctgcggcc ttgccccatc aattttttta attttctctg gggaaaagcc 11760
   tccggcctgc ggcctgcgcg cttcgcttgc cggttggaca ccaagtggaa ggcgggtcaa 11820
   ggctcgcgca gcgaccgcgc agcggcttgg ccttgacgcg cctggaacga cccaagccta 11880
   tgcgagtggg ggcagtcgaa ggcgaagccc gcccgcctgc cccccgagcc tcacggcggc 11940
   gagtgcgggg gttccaaggg ggcagcgcca ccttgggcaa ggccgaaggc cgcgcagtcg 12000
   atcaacaagc cccggagggg ccactttttg ccggaggggg agccgcgccg aaggcgtggg 12060
   ggaaccccgc aggggtgccc ttctttgggc accaaagaac tagatatagg gcgaaatgcg 12120
   aaagacttaa aaatcaacaa cttaaaaaag gggggtacgc aacagctcat tgcggcaccc 12180
   cccgcaatag ctcattgcgt aggttaaaga aaatctgtaa ttgactgcca cttttacgca 12240
   acgcataatt gttgtcgcgc tgccgaaaag ttgcagctga ttgcgcatgg tgccgcaacc 12300
   gtgcggcacc ctaccgcatg gagataagca tggccacgca gtccagagaa atcggcattc 12360
   aagccaagaa caagcccggt cactgggtgc aaacggaacg caaagcgcat gaggcgtggg 12420
   ccgggcttat tgcgaggaaa cccacggcgg caatgctgct gcatcacctc gtggcgcaga 12480
   tgggccacca gaacgccgtg gtggtcagcc agaagacact ttccaagctc atcggacgtt 12540
   ctttgcggac ggtccaatac gcagtcaagg acttggtggc cgagcgctgg atctccgtcg 12600
   tgaagctcaa cggccccggc accgtgtcgg cctacgtggt caatgaccgc gtggcgtggg 12660
   gccagccccg cgaccagttg cgcctgtcgg tgttcagtgc cgccgtggtg gttgatcacg 12720
   acgaccagga cgaatcgctg ttggggcatg gcgacctgcg ccgcatcccg accctgtatc 12780
   cgggcgagca gcaactaccg accggccccg gcgaggagcc gcccagccag cccggcattc 12840
   cgggcatgga accagacctg ccagccttga ccgaaacgga ggaatgggaa cggcgcgggc 12900
   agcagcgcct gccgatgccc gatgagccgt gttttctgga cgatggcgag ccgttggagc 12960
   cgccgacacg ggtcacgctg ccgcgccggt agcacttggg ttgcgcagca acccgtaagt 13020
   gcgctgttcc agactatcgg ctgtagccgc ctcgccgccc tataccttgt ctgcctcccc 13080
   gcgttgcgtc gcggtgcatg gagccgggcc acctcgacct gaatggaagc cggcggcacc 13140
   tcgctaacgg attcaccgtt tttatcaggc tctgggaggc agaataaatg atcatatcgt 13200
   caattattac ctccacgggg agagcctgag caaactggcc tcaggcattt gagaagcaca 13260
   cggtcacact gcttccggta gtcaataaa 13289
<210> 75
   <211> 14250
   <212> DNA
   <213> Artificial Sequence
<220>
   <223> vector
<400> 75
   ctcgggccgt ctcttgggct tgatcggcct tcttgcgcat ctcacgcgct cctgcggcgg      60
   cctgtagggc aggctcatac ccctgccgaa ccgcttttgt cagccggtcg gccacggctt     120
   ccggcgtctc aacgcgcttt gagattccca gcttttcggc caatccctgc ggtgcatagg     180
   cgcgtggctc gaccgcttgc gggctgatgg tgacgtggcc cactggtggc cgctccaggg     240
   cctcgtagaa cgcctgaatg cgcgtgtgac gtgccttgct gccctcgatg ccccgttgca     300
   gccctagatc ggccacagcg gccgcaaacg tggtctggtc gcgggtcatc tgcgctttgt     360
   tgccgatgaa ctccttggcc gacagcctgc cgtcctgcgt cagcggcacc acgaacgcgg     420
   tcatgtgcgg gctggtttcg tcacggtgga tgctggccgt cacgatgcga tccgccccgt     480
   acttgtccgc cagccacttg tgcgccttct cgaagaacgc cgcctgctgt tcttggctgg     540
   ccgacttcca ccattccggg ctggccgtca tgacgtactc gaccgccaac acagcgtcct     600
   tgcgccgctt ctctggcagc aactcgcgca gtcggcccat cgcttcatcg gtgctgctgg     660
   ccgcccagtg ctcgttctct ggcgtcctgc tggcgtcagc gttgggcgtc tcgcgctcgc     720
   ggtaggcgtg cttgagactg gccgccacgt tgcccatttt cgccagcttc ttgcatcgca     780
   tgatcgcgta tgccgccatg cctgcccctc ccttttggtg tccaaccggc tcgacggggg 840
   cagcgcaagg cggtgcctcc ggcgggccac tcaatgcttg agtatactca ctagactttg 900
   cttcgcaaag tcgtgaccgc ctacggcggc tgcggcgccc tacgggcttg ctctccgggc 960
   ttcgccctgc gcggtcgctg cgctcccttg ccagcccgtg gatatgtgga cgatggccgc 1020
   gagcggccac cggctggctc gcttcgctcg gcccgtggac aaccctgctg gacaagctga 1080
   tggacaggct gcgcctgccc acgagcttga ccacagggat tgcccaccgg ctacccagcc 1140
   ttcgaccaca tacccaccgg ctccaactgc gcggcctgcg gccttgcccc atcaattttt 1200
   ttaattttct ctggggaaaa gcctccggcc tgcggcctgc gcgcttcgct tgccggttgg 1260
   acaccaagtg gaaggcgggt caaggctcgc gcagcgaccg cgcagcggct tggccttgac 1320
   gcgcctggaa cgacccaagc ctatgcgagt gggggcagtc gaaggcgaag cccgcccgcc 1380
   tgccccccga gcctcacggc ggcgagtgcg ggggttccaa gggggcagcg ccaccttggg 1440
   caaggccgaa ggccgcgcag tcgatcaaca agccccggag gggccacttt ttgccggagg 1500
   gggagccgcg ccgaaggcgt gggggaaccc cgcaggggtg cccttctttg ggcaccaaag 1560
   aactagatat agggcgaaat gcgaaagact taaaaatcaa caacttaaaa aaggggggta 1620
   cgcaacagct cattgcggca ccccccgcaa tagctcattg cgtaggttaa agaaaatctg 1680
   taattgactg ccacttttac gcaacgcata attgttgtcg cgctgccgaa aagttgcagc 1740
   tgattgcgca tggtgccgca accgtgcggc accctaccgc atggagataa gcatggccac 1800
   gcagtccaga gaaatcggca ttcaagccaa gaacaagccc ggtcactggg tgcaaacgga 1860
   acgcaaagcg catgaggcgt gggccgggct tattgcgagg aaacccacgg cggcaatgct 1920
   gctgcatcac ctcgtggcgc agatgggcca ccagaacgcc gtggtggtca gccagaagac 1980
   actttccaag ctcatcggac gttctttgcg gacggtccaa tacgcagtca aggacttggt 2040
   ggccgagcgc tggatctccg tcgtgaagct caacggcccc ggcaccgtgt cggcctacgt 2100
   ggtcaatgac cgcgtggcgt ggggccagcc ccgcgaccag ttgcgcctgt cggtgttcag 2160
   tgccgccgtg gtggttgatc acgacgacca ggacgaatcg ctgttggggc atggcgacct 2220
   gcgccgcatc ccgaccctgt atccgggcga gcagcaacta ccgaccggcc ccggcgagga 2280
   gccgcccagc cagcccggca ttccgggcat ggaaccagac ctgccagcct tgaccgaaac 2340
   ggaggaatgg gaacggcgcg ggcagcagcg cctgccgatg cccgatgagc cgtgttttct 2400
   ggacgatggc gagccgttgg agccgccgac acgggtcacg ctgccgcgcc ggtagcactt 2460
   gggttgcgca gcaacccgta agtgcgctgt tccagactat cggctgtagc cgcctcgccg 2520
   ccctatacct tgtctgcctc cccgcgttgc gtcgcggtgc atggagccgg gccacctcga 2580
   cctgaatgga agccggcggc acctcgctaa cggattcacc gtttttatca ggctctggga 2640
   ggcagaataa atgatcatat cgtcaattat tacctccacg gggagagcct gagcaaactg 2700
   gcctcaggca tttgagaagc acacggtcac actgcttccg gtagtcaata aaccggtcac 2760
   tgatttggca gcgacgggtc gagactaagt ctcacgttta acgtgtgcaa tggaaaacag 2820
   gcgcaggggt atctttggga aatggcctac aaggaagggg caaattctga ttatttgcca 2880
   ggccatgcag cggttgcttc ttagtggcgt gtgaaaaacg ccactatcgg caagacccgg 2940
   tatggcatgt gttcagagac aaggctgcgc taccgcagcc cttctccgct aaaaccgtta 3000
   gtcgaacagt tcggcgtcag ccaatgcgac cccaagctgg tccttgccag acaagcttgg 3060
   tacggagtcg atgggccatt cgatacccac tgccggatca ttccaggcaa tgcagcgctc 3120
   gcactgcggc gagtagaagt cggtggtctt gtagaggaac tctgcggttt cactcaacgt 3180
   gacgaacccg tgtgcgaacc ctggcgggat ccacagctgg ttcttgttct cggccgacaa 3240
   caccgcacct acccatttac cgaaggttgt ggacgagcga cggatatcca ccgcaacatc 3300
   gaagacttcg ccttgcacca cacgcaccag cttgccctgg gcgtgaggtg ccagctgata 3360
   gtgcaggcca cggagcacgc cttttaccga gcgcgagtgg ttgtcttgta cgaagtcggg 3420
   ctgcaggccg gtcacttcgc tgaaaacacg ggcgttgaag ctctcgtaga agaaaccacg 3480
   ttcgtcgcca aaaaccttgg gggtaaacag cacgacttcg gggatatcca gcggaatggc 3540
   ttgcatcaga acaccttctc tttcagcaag ttctgcagat acttgccata accgtttttc 3600
   agcagtggtt gagccaggca ctcgagttgc tcagcgttga tccagccagc gcggtagcaa 3660
   atttcctcag ggcaggcgac tttcaagccc tgacggcgct ccatggttgc gatgtactgg 3720
   ctagcctcca gcagactgtc gtgcgtgccg gtgtcgagcc acgcatagcc acggcccatg 3780
   atttcgacct gcaactgctg ctgctgcaag taaaggttgt tgaggtcggt gatttccagc 3840
   tcgccacgtg gggaaggctt cagctcgcga gccagattga ctacctgatt gtcatagaaa 3900
   tacaggccgg tgaccgcata gctagacttt ggaactgccg gtttttcttc cagcgacaat 3960
   acgcgaccgc tatcgtcaaa ctccgctacg ccatagcgtt ctgggtcatg aacatgataa 4020
   gcgaatactg aagcaccgga ttcacgttta tctgcgttca atagcagtgc ctggaagtca 4080
   tggccgtaga aaatattgtc accgagaacc aacgcagaag ggtcgttacc gatgaagtca 4140
   gcgccgatgg tgaacgcttg cgccaagcca tccgggcttg gttgtattgc gtatgacagg 4200
   ttcaggcccc actggctgcc atcgcccagc agctgttcga agcgcggggt gtcctgcggg 4260
   gtggaaatga tcaggatgtc ccggatacca gcgagcagca gggtgctcag cgggtagtag 4320
   atcatcggtt tgtcatacac cggcagcagc tgcttcgaaa ccgaaagtgt ggccggatgc 4380
   aggcgtgtac ccgaaccgcc ggccagaata attcctttac gagccatgag agtccctatt 4440
   actggatttc gtccagcata cgttgcacgc cttgctccca aagcggcatt ttgaaattga 4500
   acgtgttttc cagtttgccc agtgccaggc gcgagttgcg cggacgtggt gcaggtactg 4560
   gataagcttc ggtgctgatt gcggcaacct tatcagctgt cactttcagc gctacgccag 4620
   tgcgttgagc atgcgccagc acgaactgag caaaaccatg ccaagaggtt tcaccggacg 4680
   cagccaagtg gtaaatccct gccaggtgac ggttgtcttg cccattgaag atttgccgca 4740
   ggatgtgtgc agtaacgtcg gcgatcaggt cagcgcccgt gggtgcgcca aattggtctg 4800
   ctaccacgct caacgtctca cgctccgccg ccaggcgcag catggtcttg gcaaaattgt 4860
   gcccgcgcgc agcatacacc cagctggtgc gcagtaccac ggccttggcg ccgctggcga 4920
   gaatggcatg ctcgccttcc agcttggtcc ggccgtagac cgaaaggggg ccggtaggcg 4980
   cagtttcctc ccagcgctga ctgccgctgc cgtcaaatac ataatcggtg gaatagtgaa 5040
   tcaaccaggc gcccaaagct gctgtttcac gtgctaatac agcaggagcc gcggcattga 5100
   tcattgcagc cagtgcctga tcgctctcag ctttatccac tgcagtgtag gcagcagcgt 5160
   tgacgatcac gtccggcgcc agctgacgaa tcgtagcggc caagccgtcc aggttggaca 5220
   agtcgccaca taagccctcg gccccctgac gatccagcgc aatgacctca cccagcggcg 5280
   ccaaggcgcg ctgtagctcc cagcctactt gcccgttttt ccccaacagc aggattttca 5340
   cgctttattt gccccgtatt gttgtgccac ccagtcacgg tagctgccgt ccatgacacc 5400
   ttttacccat ttctggttgg ccaagtacca agcgactgtc tttcgaatgc ccgtctcgaa 5460
   ggtttcggca ggtttccagc cgagctcccg ctcgatcttg cgtgcatcga tggcataacg 5520
   gcggtcatgg cctgggcggt cggttacgta ggcgatgagt tctgcatact gttcgacagg 5580
   ctcgccggtc ttctgattga ttacctggcg cgatgccgca ggtgccatct cgtcgagaag 5640
   gctgcagagt gtacgcacaa tgtcaatgtt ggctttttca ttccagccgc caatattgta 5700
   cgtctcgccg aacgcaccgg cttccagtac gcgacggatg cccgagcagt gatcttcgac 5760
   atacagccag tcgcggattt gctggccgtc gccatagaca ggcagcgcct taccggcgag 5820
   tgcgttgacg atcatcagcg ggatcagttt ttccgggaag tggagcggcc cgtaattgtt 5880
   ggagcagttg gtagtgagta ccggcatgcc gtaggtatgg aaatacgagc gtaccagatg 5940
   gtcgctggct gccttgctgg cggagtatgg gctgttcggc gcgtacggcg tggtttcggt 6000
   gaacgccggg tcgtttggcc ctagtgtgcc gtagacttcg tcggtagaga catggaggaa 6060
   acggaaggcc tccttctctg caccttccaa actattccaa tgcgcccggg cggcttcaag 6120
   caagcgaaac gtgcccatca cgttggtttc gacaaacgct tcggggccgg tgattgagcg 6180
   gtctacatgg gattccgccg cgaagtgaac cacggcgcgc gggcggtgct ctgcgaacag 6240
   cttggtcaga agcgcagcat cgcaaatatt gccttgcaca aagcgatgct gagggttgcc 6300
   ttccagcggc tgcaggttgg ccaggttgcc tgcgtaggtc agggcgtcga ggttgaggac 6360
   gggttcctca ttgtgcgcac accattgcag tacgaaattt gagccgatga agccggctcc 6420
   gcctgttact agaatcataa tttggctcta attggacaaa aggtgttgtc gtagacagat 6480
   gacgctgaga aacttcgccc cggcaacgca aggcgagctg ctagcgctgc cggaattaga 6540
   ccaccctgat gtcgaaaaat acactgctgg gtgccggtac tgaccgtgcc gatctgctca 6600
   cgctggaaga ggaggagccg ttgcagcggt ggaaggtgac ctcatggttg cctagacaca 6660
   acagggagtc gtcaggtgtc catgcaagaa acagttgttt gagcgatgca actcacggag 6720
   acaaatctgg gtgctgacaa gtctagaggg ccttaagcta ccgctatcct gggactctag 6780
   ttcgctgggc atggcagcgc ggggtgcgca tctggaagtc gggttcgttt ccgggggttg 6840
   gaaagtgtca tttttatttg tcagtattcc tgaaatagga attagagtga tggcagttct 6900
   tgcattcatg ttgaacaacc caacaaaaat tataatagcc tcggggctcg gcaggttaga 6960
   cgtcattctt catgtttcag cggtctggcg ctcttgaata ttcataggaa agtgaaaaat 7020
   gtctgttaag gaaggcttgc cgagcgtaga tgtgcagccg agcatgcatg ttaagggaaa 7080
   ttcagtgccc gtcccccaga aaaagactgg ctcaaggcga tttgtgctgg atctggctta 7140
   ttatcggaat cgatacccag acctatcttc gctcacggac gatgcgctcg aacagcattg 7200
   gcatgcgttc ggtcatcaag aaggccgtta tgcctcggct tcgcacgaaa gtggtgatgc 7260
   ggagcttatc gctgaagcag ttctcaatgc ggccgacagc gccgttatgg tcaagccgga 7320
   tgttgcgaaa gttgacctac cggtaaacca gcaatagaca taagcggcta tttaacgacc 7380
   ctgccctgaa ccgacgaccg ggtcgaattt gctttcgaat ttctgccatt catccgctta 7440
   ttatcactta ttcaggcgta gcaccaggcg tttaagggca ccaataactg ccttaaaaaa 7500
   attacgcccc gccctgccac tcatcgcagt cggcctattg gttaaaaaat gagctgattt 7560
   aacaaaaatt taacgcgaat tttaacaaaa tattaacgct tacaatttcc attcgccatt 7620
   caggctgcgc aactgttggg aagggcgatc ggtgcgggcc tcttcgctat tacgccagct 7680
   ggcgaaaggg ggatgtgctg caaggcgatt aagttgggta acgccagggt tttcccagtc 7740
   acgacgttgt aaaacgacgg ccagtgagcg cgcgtaatac gactcactat agggcgaatt 7800
   ggagctccac cgcggtggcg gccgctctag agggaatgcg tttcgccgac taggccttgg 7860
   ccttgccgga agctacggac gccacggccg ggccggcgag gcgcttcagc aggcgcgggc 7920
   ggttggtctc cagcgcgccg ccgcgtcccc gcaggccgtc ccacaggccc cagcccaggc 7980
   agcgcagctt gagcagcttg tcgcgttcga gcaggagcac cgcgaggccc tgggtcaggg 8040
   tcggcaggtt cgccagcagg gccagcggcg aggaccgggc gtagcggcgc aggaccagca 8100
   ggccgttgcg cgccaggtag tagcggcgca gcggggcgtg gttcatcgcg ctgaggctga 8160
   gaccgccgag gcggcgggtc ttgcgcgtgc cgatgcggtg ctcgaggacc agccgcgggt 8220
   cgacgtacag gggcacgtcc agcgcctggg cgcgcaggct gtattcggtg tccacgtggt 8280
   cgatgaacag ttcctcgtcg aagtggccga ggcgctggta ggcctcgcgg gtcagcaggc 8340
   agccggagga gatcaggaac gaggtgcgct gcggggtcgt caggccgtcc agagacaatt 8400
   gcctgagcgt cagtccgtcg agatggatgg ccggcaggaa gcgccggtca ccccggtcga 8460
   agatccgtgg gccgagcagg caggcctgac cgttgcgcgc ctgcaggttg cgccactggg 8520
   cggcgaggaa ggcgccgccg ggacgggagt cctggtcgag cagcagcaca ccctgcacgc 8580
   cacgccggaa tagcgcgtcg agtccctggt tgaaggcgcc ggcgatgccc tgccggttgc 8640
   cgtggtgcag cacggcgatg ccttgcccgc gcagccgggc attgcgctgc ggatcgctgt 8700
   gcggtgagtt gtcgacggca aggaagcgca gttgcggaaa cgccgccgcc agttcgccaa 8760
   ggtgttccag gtcgtcgtcg ccaggattga acagtaccac cagcacgccc atgtctatcc 8820
   ggtccatgat cgttcttctc ccgtaggtcg aagttgccag gccaggacca gcccggccag 8880
   aacgagaagg gcgccggcga ggaatggcgc gccggccagg ggcagcggcg cgagcggacc 8940
   gctgccccag tggaacaggc cgctcatcag cggcggaccg acgatcgcgg cgaggctcat 9000
   caggctgctc agcacgccct gcaactcgcc ctggcggtcg accggcacgc gggccgagag 9060
   cagcccctgc atggccgggg tggcgaggct gccgagcgcg aagggcagca gcgcgcagac 9120
   cagccagaat gacgagtcga ccagggcgaa cagcagcagg ccgcagcctt gcagggcgag 9180
   gcccaggcgc agcaggcggg cgtcgtccag gcgccgcttg cagaggttca cgccgagggt 9240
   ctgggcgagc accgcgagca cgccgtagag ggccagcgag tagccgatcc aggcgctgct 9300
   ccagtgaaac ttctcgatca cgaagaacgg ccagaccacc atcaccgcct gcaagccgag 9360
   gaataccagg gcaagcaccg ccagcaggcg tccgaccccc ggttgccgag ccaggccgct 9420
   gatcgagcgc aaggcattca tccgcctcgg gtccaggcgg cggcgtcgcg tcgggggcag 9480
   ggtttcctcg aggaacaggc cggcgagcag ggcgttgagc aggcacaggc cggcggccag 9540
   caacagcggc agcgtcgtgc cgtgcaccgc cagcagccca ccgagggcgg ggccgaggat 9600
   catgcccagg gcgaggccgg cgtacagcca gccgaagtgc cgggtgcgct gcccgtgcgt 9660
   gccgaggtca gccgcgcagg ccatcgcggt ggccacgctg gcgccggtga gcccggccag 9720
   cgcgcgaccg aggaacagca tccagaggct gtcggccagc gccagcagca gatagctgag 9780
   ggcgaagccg agcatcgcca ggaccaggac ggggcggcgt ccgaagcggt cgctgaggct 9840
   gccgaggacc ggcgaaaaga acaattgcag cagcgcgaag gtcatcacca gggcggcgcc 9900
   ccaggtggcc gcgtcgcgga ccgccagcgg cgccacgctg ccgatcagcg tcggcagcag 9960
   gggcacgatc aggccgacgc cagcggcatc cagcaggcag gtgaggaaca gcagaggcag 10020
   gacgcgtttc gcgccgggac cgtgttcccg cgtggcggag gggcagaggc tggtcgtgga 10080
   cacgccagga tcctcccggc gtcaggacgc agccttcagc catcgcgcat ccccctccct 10140
   atgacaacgt tcgaccacct gggccgcttt accgcaagcg atactgtgcg gttgtgacaa 10200
   ttccatgaaa cgccgacagg ccgccgccat ggccgggtcc tcgagcaagc gccacagcgc 10260
   cccgcgcaac tcctgctcgc gcaatggcac gcccaggcgc atcccgcagc cgagccggac 10320
   cagccgttcg gcattgtcga actggtcgtg ggcgcagggc agcagcacct gcggcacccc 10380
   cgccgccaag gccaggctca tggcgccgat accgcccgga tggaccagcc cggcgcacga 10440
   tggcagcaag gctcccagtg gcgcgtaggc gcgctgcagc acgtggttcg gcaagccgcg 10500
   cagcggttcc tggccggcgc cggtgaggaa gatcccacgc gcgccgaggc gttccagcgc 10560
   gcgcagggcc atggcgtaga agtcgccctg caggtgttcg gtcgagccct gggtgaacac 10620
   cagcggccgg ctgccctgat cgagaaagcg ttgcagttcg tcgtcgagcg gggtccccgg 10680
   gatactgccg tcgaacagcg ggaagccggt catgtgcagg ggttgcggcc aatcctgctg 10740
   gggcggcgcg aaccaggccg ggaacaggca gaccacgccc tgcggcgaat gcatccattg 10800
   ggtgaagatg cgcttcaccg gcgtctccag gccgaccttg cgccgcaccg cgttgatatc 10860
   cggcgcgcag gtgcgatcca gcttgaagcg ctcgatgcag cgccagagca gcttgcgcat 10920
   cgccagcggc atctgctcgg gcacgttgaa cttggggtgt accggcggca ggtgcgccga 10980
   caacaaggtc gatggcgaga cctgcgcgga caggtaggga atcccgtact tctcgtgagc 11040
   gatgcgtgcg cccagcgccc agagcgagcc gaccaccacg atgtcgtcat ggcgctgcgc 11100
   cgagacgtac tcgtagaccg gctcgatcat cccggcgatg gtttgccaga gcacgccgaa 11160
   ggacgtcttg gggtcccaca ggcgcggatc gcccatggtc cggcggtagg tcagttcgtc 11220
   gctcagcggg acgaacgcga tgccgtgctg ctccaccgcg tcgcgaaaca ccgggatggt 11280
   gcagaggctc acgcggtgcc cgcgcaattt cagggtccgg gccaggccga tgaagggaaa 11340
   tacgtcgccg gccgagccga tggcgatgag gatggcgtgc atggtgctac tccgtgcgtt 11400
   atgcaaccgc aaagcccggc caggccgggt cttcgcaggt caagggttca ggcgtagccg 11460
   atggccatct cgtggaatcc cgccgcgcgt tccgcccgct gcggctccgg ttgcttcagc 11520
   aggtgctcga gcagggcgcg gtgcacgcgt accgcggcca gcttggactc caggtcgagg 11580
   aaatgcccgg tgccctccac ccgcgagaaa ctgcagtgcg gcaggtagtc gcggaactgg 11640
   cgggcgtcct cggcggtggt gtattcgtcc cagctgccgt tgatgaaatg cacgtggctc 11700
   tggatccgct ccaggcaagc caggtagccc cgatcgttga gcgccagcac ctggtcgatg 11760
   tgaaagcgcg cctgctcgta ttcgccggtg gccagcgaag ccatgtgctg atggttgctg 11820
   gctttcaggc gcggcggcag gtatttgccg acggtctcgt tgagcagatg gccgatcgcc 11880
   gacttgtcgt ccagctcgat cagcgcctgc gcccgcccga cgtagtcgag catcgcctgg 11940
   ttcagtccag gggcgaatgc catcaccacc gagctgcgga tgccgcgcgg attgcgcgac 12000
   agcgccagca gcgtggagat accgccccag gacgcggaga ccaggtgatt gacctcgaag 12060
   cgctcgatca gcgccaggag gatttccacc tcgtcgtcct tggtgatcaa cccccgctgc 12120
   gggttgtgct gacgcgactg cccggcgaag ggcaggtcga acagcaccac gttgaaatgt 12180
   tcggccaggc acttgcaggt ccgggcgaac gaggcggtgg tcgccatcgc gccgttgacc 12240
   agcatcaccg tgctgcgccc gggatcctgc ccaacgcgct cgacatgtac ccgcaggccc 12300
   ttgcaaaccg ataccaacag actttcgcgc cgcatttcac acctcccaaa aatgccagat 12360
   cccccgggct gcaggaattc gatatcaagc ttatcgatac cgtcgacctc gagggggggc 12420
   ccggtaccca gcttttgttc cctttagtga gggttaattg cgcgcttggc gtaatcatgg 12480
   tcatagctgt ttcctgtgtg aaattgttat ccgctcacaa ttccacacaa catacgagcc 12540
   ggaagcataa agtgtaaagc ctggggtgcc taatgagtga gctaactcac attaattgcg 12600
   ttgcgctcac tgcccgcttt ccagtcggga aacctgtcgt gccagctgca ttaatgaatc 12660
   ggccaacgcg cggggagagg cggtttgcgt attgggcgca tgcataaaaa ctgttgtaat 12720
   tcattaagca ttctgccgac atggaagcca tcacaaacgg catgatgaac ctgaatcgcc 12780
   agcggcatca gcaccttgtc gccttgcgta taatatttgc ccatgggggt gggcgaagaa 12840
   ctccagcatg agatccccgc gctggaggat catccagccg gcgtcccgga aaacgattcc 12900
   gaagcccaac ctttcataga aggcggcggt ggaatcgaaa tctcgtgatg gcaggttggg 12960
   cgtcgcttgg tcggtcattt cgaaccccag agtcccgctc agaagaactc gtcaagaagg 13020
   cgatagaagg cgatgcgctg cgaatcggga gcggcgatac cgtaaagcac gaggaagcgg 13080
   tcagcccatt cgccgccaag ctcttcagca atatcacggg tagccaacgc tatgtcctga 13140
   tagcggtccg ccacacccag ccggccacag tcgatgaatc cagaaaagcg gccattttcc 13200
   accatgatat tcggcaagca ggcatcgcca tgggtcacga cgagatcctc gccgtcgggc 13260
   atgcgcgcct tgagcctggc gaacagttcg gctggcgcga gcccctgatg ctcttcgtcc 13320
   agatcatcct gatcgacaag accggcttcc atccgagtac gtgctcgctc gatgcgatgt 13380
   ttcgcttggt ggtcgaatgg gcaggtagcc ggatcaagcg tatgcagccg ccgcattgca 13440
   tcagccatga tggatacttt ctcggcagga gcaaggtgag atgacaggag atcctgcccc 13500
   ggcacttcgc ccaatagcag ccagtccctt cccgcttcag tgacaacgtc gagcacagct 13560
   gcgcaaggaa cgcccgtcgt ggccagccac gatagccgcg ctgcctcgtc ctgcagttca 13620
   ttcagggcac cggacaggtc ggtcttgaca aaaagaaccg ggcgcccctg cgctgacagc 13680
   cggaacacgg cggcatcaga gcagccgatt gtctgttgtg cccagtcata gccgaatagc 13740
   ctctccaccc aagcggccgg agaacctgcg tgcaatccat cttgttcaat catgcgaaac 13800
   gatcctcatc ctgtctcttg atcagatctt gatcccctgc gccatcagat ccttggcggc 13860
   aagaaagcca tccagtttac tttgcagggc ttcccaacct taccagaggg cgccccagct 13920
   ggcaattccg gttcgcttgc tgtccataaa accgcccagt ctagctatcg ccatgtaagc 13980
   ccactgcaag ctacctgctt tctctttgcg cttgcgtttt cccttgtcca gatagcccag 14040
   tagctgacat tcatcccagg tggcactttt cggggaaatg tgcgcgcccg cgttcctgct 14100
   ggcgctgggc ctgtttctgg cgctggactt cccgctgttc cgtcagcagc ttttcgccca 14160
   cggccttgat gatcgcggcg gccttggcct gcatatcccg attcaacggc cccagggcgt 14220
   ccagaacggg cttcaggcgc tcccgaaggt 14250
<210> 76
   <211> 1011
   <212> DNA
   <213> Burkholderia thailandensis
<400> 76
   tctagatacg ggagaagaac gatcatgacg atcctggggg cgctggtgat tctgtacgac      60
   ccgacggacg agcagttgtc ggggctggag gcgctcgcgc gcgacagcga cgcgctcgtg     120
   gtcgtggaca acacgccgca cgagcacgcg gcggcgcgcg agcgggtgcg tgcgctgtcg     180
   gcgcggacga acacggtgtg gcgacaccac ggcaaccggg gcggggtcgc gggcgggtac     240
   aacgcggggc tgtcggtgct gttcgcgcag ggcgtcgagg cggtcgcgct gttcgaccag     300
   gactcgacgg tgccggccgg gtacttcgag cggatgcgcg aggcgtgcgc gcaactgggt     360
   gagcaaccgg gcgcgcacgc gggcgcgttc atcgcgggcc cgcggatcta cgacgcgaac     420
   gagcagcgct tcctgccgga gctgatgacg agcggggtga cggtgcgccg cgtgcgggtg     480
   gagggcgaga cggcgccgca gcgctgcgcg ttcctgatct cgtcgggcag cgtgatttcg     540
   cgggccgcgt acgcgcggct cggtcgattc gacgaggcgc tgttcatcga tcacgtcgac     600
   accgagtatt gcctgcgcgc gctcgcgcac aacgtgccgc tgtacgtggt gccgccgctc     660
   gtgctgacgc accggatcgg cgcgcggcgc cggcacaagg tggggccgtt cgagctgacg     720
   gcgatgcatc acgggtggtt gcgccgatac tacggcgcgc gcaacgcgat gcaactgggg     780
   ctgcagtacg gcttgcggtt tccggtggcg ctggtgccga atctgctgac gatatggcag     840
   gtgatccagg tggtgctgtg cgagcgggag aagggcgcga agctgcgcgg gatcgcgctg     900
   ggcgtgctcg acggcctgtt cgggcggctg ggatcgttcg acgatgcgcg cgcgggcgcg     960
   gcggcgcgcg agccggtgcg gcaggaatga tcggcgaaac gcattgagct c             1011
<210> 77
   <211> 888
   <212> DNA
   <213> Pseudomonas aeruginosa
<220>
   <221> CDS
   <222> (1)..(888)
<400> 77
   atg cgg cgc gaa agt ctg ttg gta tcg gtt tgc aag ggc ctg cgg gta        48
   Met Arg Arg Glu Ser Leu Leu Val Ser Val Cys Lys Gly Leu Arg Val
   1               5                   10                  15
   cat gtc gag cgc gtt ggg cag gat ccc ggg cgc agc acg gtg atg ctg        96
   His Val Glu Arg Val Gly Gln Asp Pro Gly Arg Ser Thr Val Met Leu
               20                  25                  30
   gtc aac ggc gcg atg gcg acc acc gcc tcg ttc gcc cgg acc tgc aag        144
   Val Asn Gly Ala Met Ala Thr Thr Ala Ser Phe Ala Arg Thr Cys Lys
           35                  40                  45
   tgc ctg gcc gaa cat ttc aac gtg gtg ctg ttc gac ctg ccc ttc gcc        192
   Cys Leu Ala Glu His Phe Asn Val Val Leu Phe Asp Leu Pro Phe Ala
       50                  55                  60
   ggg cag tcg cgt cag cac aac ccg cag cgc ggg ttg atc acc aag gac        240
   Gly Gln Ser Arg Gln His Asn Pro Gln Arg Gly Leu Ile Thr Lys Asp
   65                  70                  75                  80
   gac gag gtg gaa atc ctc ctg gcg ctg atc gag cgc ttc gag gtc aat        288
   Asp Glu Val Glu Ile Leu Leu Ala Leu Ile Glu Arg Phe Glu Val Asn
                   85                  90                  95
   cac ctg gtc tcc gcg tcg tgg ggc ggt atc tcc acg ctg ctg gcg ctg        336
   His Leu Val Ser Ala Ser Trp Gly Gly Ile Ser Thr Leu Leu Ala Leu
               100                 105                 110
   tcg cgc aat ccg cgc ggc atc cgc agc tcg gtg gtg atg gca ttc gcc        384
   Ser Arg Asn Pro Arg Gly Ile Arg Ser Ser Val Val Met Ala Phe Ala
           115                 120                 125
   cct gga ctg aac cag gcg atg ctc gac tac gtc ggg cgg gcg cag gcg        432
   Pro Gly Leu Asn Gln Ala Met Leu Asp Tyr Val Gly Arg Ala Gln Ala
       130                 135                 140
   ctg atc gag ctg gac gac aag tcg gcg atc ggc cat ctg ctc aac gag        480
   Leu Ile Glu Leu Asp Asp Lys Ser Ala Ile Gly His Leu Leu Asn Glu
   145                 150                 155                 160
   acc gtc ggc aaa tac ctg ccg ccg cgc ctg aaa gcc agc aac cat cag        528
   Thr Val Gly Lys Tyr Leu Pro Pro Arg Leu Lys Ala Ser Asn His Gln
                   165                 170                 175
   cac atg gct tcg ctg gcc acc ggc gaa tac gag cag gcg cgc ttt cac        576
   His Met Ala Ser Leu Ala Thr Gly Glu Tyr Glu Gln Ala Arg Phe His
               180                 185                 190
   atc gac cag gtg ctg gcg ctc aac gat cgg ggc tac ctg gct tgc ctg        624
   Ile Asp Gln Val Leu Ala Leu Asn Asp Arg Gly Tyr Leu Ala Cys Leu
           195                 200                 205
   gag cgg atc cag agc cac gtg cat ttc atc aac ggc agc tgg gac gaa        672
   Glu Arg Ile Gln Ser His Val His Phe Ile Asn Gly Ser Trp Asp Glu
       210                 215                 220
   tac acc acc gcc gag gac gcc cgc cag ttc cgc gac tac ctg ccg cac        720
   Tyr Thr Thr Ala Glu Asp Ala Arg Gln Phe Arg Asp Tyr Leu Pro His
   225                 230                 235                 240
   tgc agt ttc tcg cgg gtg gag ggc acc ggg cat ttc ctc gac ctg gag        768
   Cys Ser Phe Ser Arg Val Glu Gly Thr Gly His Phe Leu Asp Leu Glu
                   245                 250                 255
   tcc aag ctg gcc gcg gta cgc gtg cac cgc gcc ctg ctc gag cac ctg        816
   Ser Lys Leu Ala Ala Val Arg Val His Arg Ala Leu Leu Glu His Leu
               260                 265                 270
   ctg aag caa ccg gag ccg cag cgg gcg gaa cgc gcg gcg gga ttc cac        864
   Leu Lys Gln Pro Glu Pro Gln Arg Ala Glu Arg Ala Ala Gly Phe His
           275                 280                 285
   gag atg gcc atc ggc tac gcc tga                                        888
   Glu Met Ala Ile Gly Tyr Ala
       290                 295
<210> 78
   <211> 295
   <212> PRT
   <213> Pseudomonas aeruginosa
<400> 78
   Met Arg Arg Glu Ser Leu Leu Val Ser Val Cys Lys Gly Leu Arg Val
   1               5                   10                  15
   His Val Glu Arg Val Gly Gln Asp Pro Gly Arg Ser Thr Val Met Leu
               20                  25                  30
   Val Asn Gly Ala Met Ala Thr Thr Ala Ser Phe Ala Arg Thr Cys Lys
           35                  40                  45
   Cys Leu Ala Glu His Phe Asn Val Val Leu Phe Asp Leu Pro Phe Ala
       50                  55                  60
   Gly Gln Ser Arg Gln His Asn Pro Gln Arg Gly Leu Ile Thr Lys Asp
   65                  70                  75                  80
   Asp Glu Val Glu Ile Leu Leu Ala Leu Ile Glu Arg Phe Glu Val Asn
                   85                  90                  95
   His Leu Val Ser Ala Ser Trp Gly Gly Ile Ser Thr Leu Leu Ala Leu
               100                 105                 110
   Ser Arg Asn Pro Arg Gly Ile Arg Ser Ser Val Val Met Ala Phe Ala
           115                 120                 125
   Pro Gly Leu Asn Gln Ala Met Leu Asp Tyr Val Gly Arg Ala Gln Ala
       130                 135                 140
   Leu Ile Glu Leu Asp Asp Lys Ser Ala Ile Gly His Leu Leu Asn Glu
   145                 150                 155                 160
   Thr Val Gly Lys Tyr Leu Pro Pro Arg Leu Lys Ala Ser Asn His Gln
                   165                 170                 175
   His Met Ala Ser Leu Ala Thr Gly Glu Tyr Glu Gln Ala Arg Phe His
               180                 185                 190
   Ile Asp Gln Val Leu Ala Leu Asn Asp Arg Gly Tyr Leu Ala Cys Leu
           195                 200                 205
   Glu Arg Ile Gln Ser His Val His Phe Ile Asn Gly Ser Trp Asp Glu
       210                 215                 220
   Tyr Thr Thr Ala Glu Asp Ala Arg Gln Phe Arg Asp Tyr Leu Pro His
   225                 230                 235                 240
   Cys Ser Phe Ser Arg Val Glu Gly Thr Gly His Phe Leu Asp Leu Glu
                   245                 250                 255
   Ser Lys Leu Ala Ala Val Arg Val His Arg Ala Leu Leu Glu His Leu
   260 265 270
   Leu Lys Gln Pro Glu Pro Gln Arg Ala Glu Arg Ala Ala Gly Phe His
   275 280 285
   Glu Met Ala Ile Gly Tyr Ala
   290 295
<210> 79
   <211> 888
   <212> DNA
   <213> Pseudomonas aeruginosa
<220>
   <221> CDS
   <222> (1)..(888)
<400> 79
   atg cgg cgc gaa agt ctg ttg gta tcg gtt tgc aag ggc ctg cgg gta          48
   Met Arg Arg Glu Ser Leu Leu Val Ser Val Cys Lys Gly Leu Arg Val
   1               5                   10                  15
   cat gtc gag cgc gtt ggg cag gat ccc ggg cgc agc acg gtg atg ctg          96
   His Val Glu Arg Val Gly Gln Asp Pro Gly Arg Ser Thr Val Met Leu
               20                  25                  30
   gtc aac ggc gcg atg gcg acc acc gcc tcg ttc gcc cgg acc tgc aag         144
   Val Asn Gly Ala Met Ala Thr Thr Ala Ser Phe Ala Arg Thr Cys Lys
           35                  40                  45
   tgc ctg gcc gaa cat ttc aac gtg gtg ctg ttc gac ctg ccc ttc gcc         192
   Cys Leu Ala Glu His Phe Asn Val Val Leu Phe Asp Leu Pro Phe Ala
       50                  55                  60
   ggg cag tcg cgt cag cac aac ccg cag cgc ggg ttg atc acc aag gac         240
   Gly Gln Ser Arg Gln His Asn Pro Gln Arg Gly Leu Ile Thr Lys Asp
   65                  70                  75                  80
   gac gag gtg gaa atc ctc ctg gcg ctg atc gag cgc ttc gag gtc aat         288
   Asp Glu Val Glu Ile Leu Leu Ala Leu Ile Glu Arg Phe Glu Val Asn
                   85                  90                  95
   cac ctg gtc tcc gcg tcc tgg ggc ggt atc tcc acg ctg ctg gcg ctg         336
   His Leu Val Ser Ala Ser Trp Gly Gly Ile Ser Thr Leu Leu Ala Leu
               100                 105                 110
   tcg cgc aat ccg cgc ggc atc cgc agc tcg gtg gtg atg gca ttc gcc         384
   Ser Arg Asn Pro Arg Gly Ile Arg Ser Ser Val Val Met Ala Phe Ala
           115                 120                 125
   cct gga ctg aac cag gcg atg ctc gac tac gtc ggg cgg gcg cag gcg         432
   Pro Gly Leu Asn Gln Ala Met Leu Asp Tyr Val Gly Arg Ala Gln Ala
       130                 135                 140
   ctg atc gag ctg gac gac aag tcg gcg atc ggc cat ctg ctc aac gag         480
   Leu Ile Glu Leu Asp Asp Lys Ser Ala Ile Gly His Leu Leu Asn Glu
   145                 150                 155                 160
   acc gtc ggc aaa tac ctg ccg cag cgc ctg aaa gcc agc aac cat cag         528
   Thr Val Gly Lys Tyr Leu Pro Gln Arg Leu Lys Ala Ser Asn His Gln
                   165                 170                 175
   cac atg gct tcg ctg gcc acc ggc gaa tac gag cag gcg cgc ttt cac         576
   His Met Ala Ser Leu Ala Thr Gly Glu Tyr Glu Gln Ala Arg Phe His
               180                 185                 190
   atc gac cag gtg ctg gcg ctc aac gat cgg ggc tac ttg gct tgc ctg         624
   Ile Asp Gln Val Leu Ala Leu Asn Asp Arg Gly Tyr Leu Ala Cys Leu
           195                 200                 205
   gag cgg atc cag agc cac gtg cat ttc atc aac ggc agc tgg gac gaa         672
   Glu Arg Ile Gln Ser His Val His Phe Ile Asn Gly Ser Trp Asp Glu
       210                 215                 220
   tac acc acc gcc gag gac gcc cgc cag ttc cgc gac tac ctg ccg cac         720
   Tyr Thr Thr Ala Glu Asp Ala Arg Gln Phe Arg Asp Tyr Leu Pro His
   225                 230                 235                 240
   tgc agt ttc tcg cgg gtg gag ggc acc ggg cat ttc ctc gac ctg gag         768
   Cys Ser Phe Ser Arg Val Glu Gly Thr Gly His Phe Leu Asp Leu Glu
                   245                 250                 255
   tcc aag ctg gca gcg gta cgc gtg cac cgc gcc ctg ctc gag cac ctg         816
   Ser Lys Leu Ala Ala Val Arg Val His Arg Ala Leu Leu Glu His Leu
               260                 265                 270
   ctg aag caa ccg gag ccg cag cgg gcg gaa cgc gcg gcg gga ttc cac         864
   Leu Lys Gln Pro Glu Pro Gln Arg Ala Glu Arg Ala Ala Gly Phe His
           275                 280                 285
   gag atg gcc atc ggc tac gcc tga                                         888
   Glu Met Ala Ile Gly Tyr Ala
       290                 295
<210> 80
   <211> 295
   <212> PRT
   <213> Pseudomonas aeruginosa
<400> 80
   Met Arg Arg Glu Ser Leu Leu Val Ser Val Cys Lys Gly Leu Arg Val
   1               5                   10                  15
   His Val Glu Arg Val Gly Gln Asp Pro Gly Arg Ser Thr Val Met Leu
               20                  25                  30
   Val Asn Gly Ala Met Ala Thr Thr Ala Ser Phe Ala Arg Thr Cys Lys
           35                  40                  45
   Cys Leu Ala Glu His Phe Asn Val Val Leu Phe Asp Leu Pro Phe Ala
       50                  55                  60
   Gly Gln Ser Arg Gln His Asn Pro Gln Arg Gly Leu Ile Thr Lys Asp
   65                  70                  75                  80
   Asp Glu Val Glu Ile Leu Leu Ala Leu Ile Glu Arg Phe Glu Val Asn
                   85                  90                  95
   His Leu Val Ser Ala Ser Trp Gly Gly Ile Ser Thr Leu Leu Ala Leu
               100                 105                 110
   Ser Arg Asn Pro Arg Gly Ile Arg Ser Ser Val Val Met Ala Phe Ala
           115                 120                 125
   Pro Gly Leu Asn Gln Ala Met Leu Asp Tyr Val Gly Arg Ala Gln Ala
       130                 135                 140
   Leu Ile Glu Leu Asp Asp Lys Ser Ala Ile Gly His Leu Leu Asn Glu
   145                 150                 155                 160
   Thr Val Gly Lys Tyr Leu Pro Gln Arg Leu Lys Ala Ser Asn His Gln
                   165                 170                 175
   His Met Ala Ser Leu Ala Thr Gly Glu Tyr Glu Gln Ala Arg Phe His
               180                 185                 190
   Ile Asp Gln Val Leu Ala Leu Asn Asp Arg Gly Tyr Leu Ala Cys Leu
           195                 200                 205
   Glu Arg Ile Gln Ser His Val His Phe Ile Asn Gly Ser Trp Asp Glu
       210                 215                 220
   Tyr Thr Thr Ala Glu Asp Ala Arg Gln Phe Arg Asp Tyr Leu Pro His
   225                 230                 235                 240
   Cys Ser Phe Ser Arg Val Glu Gly Thr Gly His Phe Leu Asp Leu Glu
                   245                 250                 255
   Ser Lys Leu Ala Ala Val Arg Val His Arg Ala Leu Leu Glu His Leu
               260                 265                 270
   Leu Lys Gln Pro Glu Pro Gln Arg Ala Glu Arg Ala Ala Gly Phe His
           275                 280                 285
   Glu Met Ala Ile Gly Tyr Ala
       290                 295
<210> 81
   <211> 888
   <212> DNA
   <213> Pseudomonas aeruginosa
<220>
   <221> CDS
   <222> (1)..(888)
<400> 81
   atg cgg cgc gaa agt ctg ttg gta acg gta tgc aag ggc ctg cgg gta      48
   Met Arg Arg Glu Ser Leu Leu Val Thr Val Cys Lys Gly Leu Arg Val
   1               5                   10                  15
   cat gtc gag cgc gtg ggg cag gat ccc ggg cgc gac acg gtg atg ctg      96
   His Val Glu Arg Val Gly Gln Asp Pro Gly Arg Asp Thr Val Met Leu
               20                  25                  30
   gtc aac ggc gcg atg gcg acc acc gcc tcg ttc gcc cgg acc tgc aag     144
   Val Asn Gly Ala Met Ala Thr Thr Ala Ser Phe Ala Arg Thr Cys Lys
           35                  40                  45
   tgc ctg gcc gaa cat ttc aac gtg gtg ctg ttc gac ctg ccc ttc gcc     192
   Cys Leu Ala Glu His Phe Asn Val Val Leu Phe Asp Leu Pro Phe Ala
       50                  55                  60
   ggg cag tcg cgg cag cac aat ccg cag cgc ggg ttg atc acc aag gac     240
   Gly Gln Ser Arg Gln His Asn Pro Gln Arg Gly Leu Ile Thr Lys Asp
   65                  70                  75                  80
   gac gag gtg gag att ctc ctg gcg ctg atc gag cgc ttc gct gtc aac     288
   Asp Glu Val Glu Ile Leu Leu Ala Leu Ile Glu Arg Phe Ala Val Asn
                   85                  90                  95
   cac ctg gtc tcg gcc tcc tgg ggc ggc atc tcc acg ctg ctg gcg ctg     336
   His Leu Val Ser Ala Ser Trp Gly Gly Ile Ser Thr Leu Leu Ala Leu
               100                 105                 110
   tcg cgc aac ccg cgc ggg gtc cgc agc tcg gtg gtg atg gcg ttc gcg     384
   Ser Arg Asn Pro Arg Gly Val Arg Ser Ser Val Val Met Ala Phe Ala
           115                 120                 125
   ccg ggg ctg aac cag gcg atg ctc gat tat gtc ggg cgg gcc cag gaa     432
   Pro Gly Leu Asn Gln Ala Met Leu Asp Tyr Val Gly Arg Ala Gln Glu
       130                 135                 140
   ctg atc gaa ctg gac gac aag tcg gcg atc ggc cac ctg ctc aac gag     480
   Leu Ile Glu Leu Asp Asp Lys Ser Ala Ile Gly His Leu Leu Asn Glu
   145                 150                 155                 160
   acc gtc ggc aag tac ctg ccg ccg cgg ctg aag gcc agc aac cat cag     528
   Thr Val Gly Lys Tyr Leu Pro Pro Arg Leu Lys Ala Ser Asn His Gln
                   165                 170                 175
   cac atg gcc tcc ctg gcc act ggc gag tac gag cag gcg cgt ttc cac     576
   His Met Ala Ser Leu Ala Thr Gly Glu Tyr Glu Gln Ala Arg Phe His
               180                 185                 190
   atc gac cag gtg ctg gcg ctc aat gac cgt ggc tac ctg agc tgc ctg     624
   Ile Asp Gln Val Leu Ala Leu Asn Asp Arg Gly Tyr Leu Ser Cys Leu
           195                 200                 205
   ggg cag atc cag agt cac gtg cat ttc atc aac ggc agc tgg gac gag     672
   Gly Gln Ile Gln Ser His Val His Phe Ile Asn Gly Ser Trp Asp Glu
       210                 215                 220
   tac acc acc gcc gag gac gcc cgc cag ttc cgc gat tac ctg ccg cat     720
   Tyr Thr Thr Ala Glu Asp Ala Arg Gln Phe Arg Asp Tyr Leu Pro His
   225                 230                 235                 240
   tgc agt ttt tcg cgg gtg gaa ggc acc ggg cac ttc ctc gac ctg gag     768
   Cys Ser Phe Ser Arg Val Glu Gly Thr Gly His Phe Leu Asp Leu Glu
                   245                 250                 255
   tcc aag ctg gcg gcg gcg cgt gtg cac cgg gcg ttg ctc gag cac ctg        816
   Ser Lys Leu Ala Ala Ala Arg Val His Arg Ala Leu Leu Glu His Leu
               260                 265                 270
   ctg gcg caa ccg gaa ccg tgg cgc tcc gag cag gcg gcg gga ttc cac        864
   Leu Ala Gln Pro Glu Pro Trp Arg Ser Glu Gln Ala Ala Gly Phe His
           275                 280                 285
   gag atg gcc atc ggc tac gcc tga                                        888
   Glu Met Ala Ile Gly Tyr Ala
       290                 295
<210> 82
   <211> 295
   <212> PRT
   <213> Pseudomonas aeruginosa
<400> 82
   Met Arg Arg Glu Ser Leu Leu Val Thr Val Cys Lys Gly Leu Arg Val
   1               5                   10                  15
   His Val Glu Arg Val Gly Gln Asp Pro Gly Arg Asp Thr Val Met Leu
               20                  25                  30
   Val Asn Gly Ala Met Ala Thr Thr Ala Ser Phe Ala Arg Thr Cys Lys
           35                  40                  45
   Cys Leu Ala Glu His Phe Asn Val Val Leu Phe Asp Leu Pro Phe Ala
       50                  55                  60
   Gly Gln Ser Arg Gln His Asn Pro Gln Arg Gly Leu Ile Thr Lys Asp
   65                  70                  75                  80
   Asp Glu Val Glu Ile Leu Leu Ala Leu Ile Glu Arg Phe Ala Val Asn
                   85                  90                  95
   His Leu Val Ser Ala Ser Trp Gly Gly Ile Ser Thr Leu Leu Ala Leu
               100                 105                 110
   Ser Arg Asn Pro Arg Gly Val Arg Ser Ser Val Val Met Ala Phe Ala
           115                 120                 125
   Pro Gly Leu Asn Gln Ala Met Leu Asp Tyr Val Gly Arg Ala Gln Glu
       130                 135                 140
   Leu Ile Glu Leu Asp Asp Lys Ser Ala Ile Gly His Leu Leu Asn Glu
   145                 150                 155                 160
   Thr Val Gly Lys Tyr Leu Pro Pro Arg Leu Lys Ala Ser Asn His Gln
                   165                 170                 175
   His Met Ala Ser Leu Ala Thr Gly Glu Tyr Glu Gln Ala Arg Phe His
               180                 185                 190
   Ile Asp Gln Val Leu Ala Leu Asn Asp Arg Gly Tyr Leu Ser Cys Leu
           195                 200                 205
   Gly Gln Ile Gln Ser His Val His Phe Ile Asn Gly Ser Trp Asp Glu
       210                 215                 220
   Tyr Thr Thr Ala Glu Asp Ala Arg Gln Phe Arg Asp Tyr Leu Pro His
   225                 230                 235                 240
   Cys Ser Phe Ser Arg Val Glu Gly Thr Gly His Phe Leu Asp Leu Glu
                   245                 250                 255
   Ser Lys Leu Ala Ala Ala Arg Val His Arg Ala Leu Leu Glu His Leu
               260                 265                 270
   Leu Ala Gln Pro Glu Pro Trp Arg Ser Glu Gln Ala Ala Gly Phe His
           275                 280                 285
   Glu Met Ala Ile Gly Tyr Ala
       290                 295
<210> 83
   <211> 1281
   <212> DNA
   <213> Pseudomonas aeruginosa
<220>
   <221> CDS
   <222> (1)..(1281)
<400> 83
   atg cac gcc att ctc atc gcc atc ggt tcg gcc ggc gac gtg ttc ccc         48
   Met His Ala Ile Leu Ile Ala Ile Gly Ser Ala Gly Asp Val Phe Pro
   1               5                   10                  15
   ttc atc ggc ctg gcc cgc acc ctg aag ttg cgc ggc cac cgc gtc agc         96
   Phe Ile Gly Leu Ala Arg Thr Leu Lys Leu Arg Gly His Arg Val Ser
               20                  25                  30
   ctg tgc acc att ccg gtg ttt cgc gcc gcg gtg gag cag cac ggc atc        144
   Leu Cys Thr Ile Pro Val Phe Arg Ala Ala Val Glu Gln His Gly Ile
           35                  40                  45
   gag ttc gtc ccg ctc agc gac gaa ctg acc tac cgc cgg acc atg ggc        192
   Glu Phe Val Pro Leu Ser Asp Glu Leu Thr Tyr Arg Arg Thr Met Gly
       50                  55                  60
   gac ccg cgc ctg tgg gat ccg aag acc tcg ttc gga gtg ctc tgg cag        240
   Asp Pro Arg Leu Trp Asp Pro Lys Thr Ser Phe Gly Val Leu Trp Gln
   65                  70                  75                  80
   gcc atc gcc ggg atg atc gag ccg gtc tac gag tac gtc tgc gca cag        288
   Ala Ile Ala Gly Met Ile Glu Pro Val Tyr Glu Tyr Val Cys Ala Gln
                   85                  90                  95
   cgc cac gac gac atc gtg gtg gtc ggt tcg ctg tgg gcc ctg ggc gcg          336
   Arg His Asp Asp Ile Val Val Val Gly Ser Leu Trp Ala Leu Gly Ala
               100                 105                 110
   cgg atc gcc cat gag aaa tac ggg att ccc tac ctg tcg gtg cag gtc          384
   Arg Ile Ala His Glu Lys Tyr Gly Ile Pro Tyr Leu Ser Val Gln Val
           115                 120                 125
   tcg ccg tcg acc ctg ctg tcg gcg cac ctg ccg ccg gtc cac ccc agg          432
   Ser Pro Ser Thr Leu Leu Ser Ala His Leu Pro Pro Val His Pro Arg
       130                 135                 140
   ttc aac gtg ccc gag cag gtc ccg ctg gcg atg cgc aag ttg ctc tgg          480
   Phe Asn Val Pro Glu Gln Val Pro Leu Ala Met Arg Lys Leu Leu Trp
   145                 150                 155                 160
   cgc tgc atc gaa cgc ttc aag ctg gac cgc acc tgc gcc ccg gag atc          528
   Arg Cys Ile Glu Arg Phe Lys Leu Asp Arg Thr Cys Ala Pro Glu Ile
                   165                 170                 175
   aac gcg gtg cgc cgc aag gtc ggc ctg gtc ggc ccg gcg aag cgc atc          576
   Asn Ala Val Arg Arg Lys Val Gly Leu Val Gly Pro Ala Lys Arg Ile
               180                 185                 190
   ttc acc cag tgg atg cat tcg cca cag gga gtg ctc tgc ctg ttc ccg          624
   Phe Thr Gln Trp Met His Ser Pro Gln Gly Val Leu Cys Leu Phe Pro
           195                 200                 205
   gcc tgg ttc gca ccg ccc cag cag gac tgg ccg caa ccg ctg cac atg          672
   Ala Trp Phe Ala Pro Pro Gln Gln Asp Trp Pro Gln Pro Leu His Met
       210                 215                 220
   acc ggc ttc ccg ctg ttc gac ggc agc gtc ccg ggg acc cgc ctc gac          720
   Thr Gly Phe Pro Leu Phe Asp Gly Ser Val Pro Gly Thr Arg Leu Asp
   225                 230                 235                 240
   gac gag ttg cag cgc ttc ctc gag cag ggc agt cgg ccg ctg gtg ttc          768
   Asp Glu Leu Gln Arg Phe Leu Glu Gln Gly Ser Arg Pro Leu Val Phe
                   245                 250                 255
   acc cag ggt tcg acc gag cac ctg cag gga gac ttc tat gcc atg gcc          816
   Thr Gln Gly Ser Thr Glu His Leu Gln Gly Asp Phe Tyr Ala Met Ala
               260                 265                 270
   ttg cgc gcg ctg gag cgt ctc ggc gcc cgc ggc atc ttc ctc acc ggc          864
   Leu Arg Ala Leu Glu Arg Leu Gly Ala Arg Gly Ile Phe Leu Thr Gly
           275                 280                 285
   gcc ggc cag gag ccg ctg cgt ggc ttg ccg agc cac gtg ctg caa cgc          912
   Ala Gly Gln Glu Pro Leu Arg Gly Leu Pro Ser His Val Leu Gln Arg
       290                 295                 300
   tcg tac gtg ccg ttg ggg gcc ttg ctg ccg gcg tgc gcc ggg ctg gtc          960
   Ser Tyr Val Pro Leu Gly Ala Leu Leu Pro Ala Cys Ala Gly Leu Val
   305                 310                 315                 320
   cac ccg gcc ggc atc ggc gcc atg agc ctg gcg ctg gcg gcg ggg gtg         1008
   His Pro Ala Gly Ile Gly Ala Met Ser Leu Ala Leu Ala Ala Gly Val
                   325                 330                 335
   ccg cag gtg ctg ctg cct tgc gcc cac gac cag ttc gac aac gcc gaa         1056
   Pro Gln Val Leu Leu Pro Cys Ala His Asp Gln Phe Asp Asn Ala Glu
               340                 345                 350
   cgc ctg gtc cgc ctc ggc tgc ggt atc cgc ctg ggc ctg ccg cta cgc      1104
   Arg Leu Val Arg Leu Gly Cys Gly Ile Arg Leu Gly Leu Pro Leu Arg
           355                 360                 365
   gag cag gcg ctg cgc gag tcg ctc tgg cgg ctg ctc gag gac ccg gcg      1152
   Glu Gln Ala Leu Arg Glu Ser Leu Trp Arg Leu Leu Glu Asp Pro Ala
       370                 375                 380
   ctg gcg gcg gcc tgt cgg cgt ttc atg gaa ttg tca caa ccg cac agt      1200
   Leu Ala Ala Ala Cys Arg Arg Phe Met Glu Leu Ser Gln Pro His Ser
   385                 390                 395                 400
   atc gct tgc ggt aaa gcg gcc caa gtg gtc gaa cgt tgt cat agg gag      1248
   Ile Ala Cys Gly Lys Ala Ala Gln Val Val Glu Arg Cys His Arg Glu
                   405                 410                 415
   ggg gat gtg cga tgg ctg aaa gcc gcg tcc tga                          1281
   Gly Asp Val Arg Trp Leu Lys Ala Ala Ser
               420                 425
<210> 84
   <211> 426
   <212> PRT
   <213> Pseudomonas aeruginosa
<400> 84
   Met His Ala Ile Leu Ile Ala Ile Gly Ser Ala Gly Asp Val Phe Pro
   1               5                   10                  15
   Phe Ile Gly Leu Ala Arg Thr Leu Lys Leu Arg Gly His Arg Val Ser
               20                  25                  30
   Leu Cys Thr Ile Pro Val Phe Arg Ala Ala Val Glu Gln His Gly Ile
           35                  40                  45
   Glu Phe Val Pro Leu Ser Asp Glu Leu Thr Tyr Arg Arg Thr Met Gly
       50                  55                  60
   Asp Pro Arg Leu Trp Asp Pro Lys Thr Ser Phe Gly Val Leu Trp Gln
   65                  70                  75                  80
   Ala Ile Ala Gly Met Ile Glu Pro Val Tyr Glu Tyr Val Cys Ala Gln
                   85                  90                  95
   Arg His Asp Asp Ile Val Val Val Gly Ser Leu Trp Ala Leu Gly Ala
               100                 105                 110
   Arg Ile Ala His Glu Lys Tyr Gly Ile Pro Tyr Leu Ser Val Gln Val
           115                 120                 125
   Ser Pro Ser Thr Leu Leu Ser Ala His Leu Pro Pro Val His Pro Arg
       130                 135                 140
   Phe Asn Val Pro Glu Gln Val Pro Leu Ala Met Arg Lys Leu Leu Trp
   145 150 155 160
   Arg Cys Ile Glu Arg Phe Lys Leu Asp Arg Thr Cys Ala Pro Glu Ile
   165 170 175
   Asn Ala Val Arg Arg Lys Val Gly Leu Val Gly Pro Ala Lys Arg Ile
   180 185 190
   Phe Thr Gln Trp Met His Ser Pro Gln Gly Val Leu Cys Leu Phe Pro
   195 200 205
   Ala Trp Phe Ala Pro Pro Gln Gln Asp Trp Pro Gln Pro Leu His Met
   210 215 220
   Thr Gly Phe Pro Leu Phe Asp Gly Ser Val Pro Gly Thr Arg Leu Asp
   225 230 235 240
   Asp Glu Leu Gln Arg Phe Leu Glu Gln Gly Ser Arg Pro Leu Val Phe
   245 250 255
   Thr Gln Gly Ser Thr Glu His Leu Gln Gly Asp Phe Tyr Ala Met Ala
   260 265 270
   Leu Arg Ala Leu Glu Arg Leu Gly Ala Arg Gly Ile Phe Leu Thr Gly
   275 280 285
   Ala Gly Gln Glu Pro Leu Arg Gly Leu Pro Ser His Val Leu Gln Arg
   290 295 300
   Ser Tyr Val Pro Leu Gly Ala Leu Leu Pro Ala Cys Ala Gly Leu Val
   305 310 315 320
   His Pro Ala Gly Ile Gly Ala Met Ser Leu Ala Leu Ala Ala Gly Val
   325 330 335
   Pro Gln Val Leu Leu Pro Cys Ala His Asp Gln Phe Asp Asn Ala Glu
   340 345 350
   Arg Leu Val Arg Leu Gly Cys Gly Ile Arg Leu Gly Leu Pro Leu Arg
   355 360 365
   Glu Gln Ala Leu Arg Glu Ser Leu Trp Arg Leu Leu Glu Asp Pro Ala
   370 375 380
   Leu Ala Ala Ala Cys Arg Arg Phe Met Glu Leu Ser Gln Pro His Ser
   385 390 395 400
   Ile Ala Cys Gly Lys Ala Ala Gln Val Val Glu Arg Cys His Arg Glu
   405 410 415
   Gly Asp Val Arg Trp Leu Lys Ala Ala Ser
               420                     425
<210> 85
   <211> 1281
   <212> DNA
   <213> Pseudomonas aeruginosa
<220>
   <221> CDS
   <222> (1)..(1281)
<400> 85
   atg cac gcc atc ctc atc gcc atc ggc tcg gcc ggc gac gta ttt ccc        48
   Met His Ala Ile Leu Ile Ala Ile Gly Ser Ala Gly Asp Val Phe Pro
   1               5                   10                  15
   ttc atc ggc ctg gcc cgg acc ctg aaa ctg cgc ggg cac cgc gtg agc        96
   Phe Ile Gly Leu Ala Arg Thr Leu Lys Leu Arg Gly His Arg Val Ser
               20                  25                  30
   ctc tgc acc atc ccg gtg ttt cgc gac gcg gtg gag cag cac ggc atc       144
   Leu Cys Thr Ile Pro Val Phe Arg Asp Ala Val Glu Gln His Gly Ile
           35                  40                  45
   gcg ttc gtc ccg ctg agc gac gaa ctg acc tac cgc cgg acc atg ggc       192
   Ala Phe Val Pro Leu Ser Asp Glu Leu Thr Tyr Arg Arg Thr Met Gly
       50                  55                  60
   gat ccg cgc ctg tgg gac ccc aag acg tcc ttc ggc gtg ctc tgg caa       240
   Asp Pro Arg Leu Trp Asp Pro Lys Thr Ser Phe Gly Val Leu Trp Gln
   65                  70                  75                  80
   gcc atc gcc ggg atg atc gag ccg gtc tac gag tac gtc tcg gcg cag       288
   Ala Ile Ala Gly Met Ile Glu Pro Val Tyr Glu Tyr Val Ser Ala Gln
                   85                  90                  95
   cgc cat gac gac atc gtg gtg gtc ggc tcg cta tgg gcg ctg ggc gca       336
   Arg His Asp Asp Ile Val Val Val Gly Ser Leu Trp Ala Leu Gly Ala
               100                 105                 110
   cgc atc gct cac gag aag tac ggg att ccc tac ctg tcc gcg cag gtc       384
   Arg Ile Ala His Glu Lys Tyr Gly Ile Pro Tyr Leu Ser Ala Gln Val
           115                 120                 125
   tcg cca tcg acc ctg ttg tcg gcg cac ctg ccg ccg gta cac ccc aag       432
   Ser Pro Ser Thr Leu Leu Ser Ala His Leu Pro Pro Val His Pro Lys
       130                 135                 140
   ttc aac gtg ccc gag cag atg ccg ctg gcg atg cgc aag ctg ctc tgg       480
   Phe Asn Val Pro Glu Gln Met Pro Leu Ala Met Arg Lys Leu Leu Trp
   145                 150                 155                 160
   cgc tgc atc gag cgc ttc aag ctg gat cgc acc tgc gcg ccg gag atc       528
   Arg Cys Ile Glu Arg Phe Lys Leu Asp Arg Thr Cys Ala Pro Glu Ile
                   165                 170                 175
   aac gcg gtg cgc cgc aag gtc ggc ctg gaa acg ccg gtg aag cgc atc       576
   Asn Ala Val Arg Arg Lys Val Gly Leu Glu Thr Pro Val Lys Arg Ile
               180                 185                 190
   ttc acc caa tgg atg cat tcg ccg cag ggc gtg gtc tgc ctg ttc ccg      624
   Phe Thr Gln Trp Met His Ser Pro Gln Gly Val Val Cys Leu Phe Pro
           195                 200                 205
   gcc tgg ttc gcg ccg ccc cag cag gat tgg ccg caa ccc ctg cac atg      672
   Ala Trp Phe Ala Pro Pro Gln Gln Asp Trp Pro Gln Pro Leu His Met
       210                 215                 220
   acc ggc ttc ccg ctg ttc gac ggc agt atc ccg ggg acc ccg ctc gac      720
   Thr Gly Phe Pro Leu Phe Asp Gly Ser Ile Pro Gly Thr Pro Leu Asp
   225                 230                 235                 240
   gac gaa ctg caa cgc ttt ctc gat cag ggc agc cgg ccg ctg gtg ttc      768
   Asp Glu Leu Gln Arg Phe Leu Asp Gln Gly Ser Arg Pro Leu Val Phe
                   245                 250                 255
   acc cag ggc tcg acc gaa cac ctg cag ggc gac ttc tac gcc atg gcc      816
   Thr Gln Gly Ser Thr Glu His Leu Gln Gly Asp Phe Tyr Ala Met Ala
               260                 265                 270
   ctg cgc gcg ctg gaa cgc ctc ggc gcg cgt ggg atc ttc ctc acc ggc      864
   Leu Arg Ala Leu Glu Arg Leu Gly Ala Arg Gly Ile Phe Leu Thr Gly
           275                 280                 285
   gcc ggc cag gaa ccg ctg cgc ggc ttg ccg aac cac gtg ctg cag cgc      912
   Ala Gly Gln Glu Pro Leu Arg Gly Leu Pro Asn His Val Leu Gln Arg
       290                 295                 300
   gcc tac gcg cca ctg gga gcc ttg ctg cca tcg tgc gcc ggg ctg gtc      960
   Ala Tyr Ala Pro Leu Gly Ala Leu Leu Pro Ser Cys Ala Gly Leu Val
   305                 310                 315                 320
   cat ccg ggc ggt atc ggc gcc atg agc cta gcc ttg gcg gcg ggg gtg     1008
   His Pro Gly Gly Ile Gly Ala Met Ser Leu Ala Leu Ala Ala Gly Val
                   325                 330                 335
   ccg cag gtg ctg ctg ccc tgt gcc cac gac cag ttc gac aat gcc gaa     1056
   Pro Gln Val Leu Leu Pro Cys Ala His Asp Gln Phe Asp Asn Ala Glu
               340                 345                 350
   cgg ctg gtc cgg ctc ggc tgc ggg atg cgc ctg ggc gtg ccg ttg cgc     1104
   Arg Leu Val Arg Leu Gly Cys Gly Met Arg Leu Gly Val Pro Leu Arg
           355                 360                 365
   gag cag gag ttg cgc ggg gcg ctg tgg cgc ttg ctc gag gac ccg gcc     1152
   Glu Gln Glu Leu Arg Gly Ala Leu Trp Arg Leu Leu Glu Asp Pro Ala
       370                 375                 380
   atg gcg gcg gcc tgt cgg cgt ttc atg gaa ttg tca caa ccg cac agt     1200
   Met Ala Ala Ala Cys Arg Arg Phe Met Glu Leu Ser Gln Pro His Ser
   385                 390                 395                 400
   atc gct tgc ggt aaa gcg gcc cag gtg gtc gaa cgt tgt cat agg gag     1248
   Ile Ala Cys Gly Lys Ala Ala Gln Val Val Glu Arg Cys His Arg Glu
                   405                 410                 415
   ggg gat gct cga tgg ctg aag gct gcg tcc tga                         1281
   Gly Asp Ala Arg Trp Leu Lys Ala Ala Ser
               420                 425
<210> 86
   <211> 426
   <212> PRT
   <213> Pseudomonas aeruginosa
<400> 86
   Met His Ala Ile Leu Ile Ala Ile Gly Ser Ala Gly Asp Val Phe Pro
   1 5 10 15
   Phe Ile Gly Leu Ala Arg Thr Leu Lys Leu Arg Gly His Arg Val Ser
   20 25 30
   Leu Cys Thr Ile Pro Val Phe Arg Asp Ala Val Glu Gln His Gly Ile
   35 40 45
   Ala Phe Val Pro Leu Ser Asp Glu Leu Thr Tyr Arg Arg Thr Met Gly
   50 55 60
   Asp Pro Arg Leu Trp Asp Pro Lys Thr Ser Phe Gly Val Leu Trp Gln
   65 70 75 80
   Ala Ile Ala Gly Met Ile Glu Pro Val Tyr Glu Tyr Val Ser Ala Gln
   85 90 95
   Arg His Asp Asp Ile Val Val Val Gly Ser Leu Trp Ala Leu Gly Ala
   100 105 110
   Arg Ile Ala His Glu Lys Tyr Gly Ile Pro Tyr Leu Ser Ala Gln Val
   115 120 125
   Ser Pro Ser Thr Leu Leu Ser Ala His Leu Pro Pro Val His Pro Lys
   130 135 140
   Phe Asn Val Pro Glu Gln Met Pro Leu Ala Met Arg Lys Leu Leu Trp
   145 150 155 160
   Arg Cys Ile Glu Arg Phe Lys Leu Asp Arg Thr Cys Ala Pro Glu Ile
   165 170 175
   Asn Ala Val Arg Arg Lys Val Gly Leu Glu Thr Pro Val Lys Arg Ile
   180 185 190
   Phe Thr Gln Trp Met His Ser Pro Gln Gly Val Val Cys Leu Phe Pro
   195 200 205
   Ala Trp Phe Ala Pro Pro Gln Gln Asp Trp Pro Gln Pro Leu His Met
   210 215 220
   Thr Gly Phe Pro Leu Phe Asp Gly Ser Ile Pro Gly Thr Pro Leu Asp
   225 230 235 240
   Asp Glu Leu Gln Arg Phe Leu Asp Gln Gly Ser Arg Pro Leu Val Phe
   245 250 255
   Thr Gln Gly Ser Thr Glu His Leu Gln Gly Asp Phe Tyr Ala Met Ala
               260                 265                 270
   Leu Arg Ala Leu Glu Arg Leu Gly Ala Arg Gly Ile Phe Leu Thr Gly
           275                 280                 285
   Ala Gly Gln Glu Pro Leu Arg Gly Leu Pro Asn His Val Leu Gln Arg
       290                 295                 300
   Ala Tyr Ala Pro Leu Gly Ala Leu Leu Pro Ser Cys Ala Gly Leu Val
   305                 310                 315                 320
   His Pro Gly Gly Ile Gly Ala Met Ser Leu Ala Leu Ala Ala Gly Val
                   325                 330                 335
   Pro Gln Val Leu Leu Pro Cys Ala His Asp Gln Phe Asp Asn Ala Glu
               340                 345                 350
   Arg Leu Val Arg Leu Gly Cys Gly Met Arg Leu Gly Val Pro Leu Arg
           355                 360                 365
   Glu Gln Glu Leu Arg Gly Ala Leu Trp Arg Leu Leu Glu Asp Pro Ala
       370                 375                 380
   Met Ala Ala Ala Cys Arg Arg Phe Met Glu Leu Ser Gln Pro His Ser
   385                 390                 395                 400
   Ile Ala Cys Gly Lys Ala Ala Gln Val Val Glu Arg Cys His Arg Glu
                   405                 410                 415
   Gly Asp Ala Arg Trp Leu Lys Ala Ala Ser
               420                 425
<210> 87
   <211> 1281
   <212> DNA
   <213> Pseudomonas aeruginosa
<220>
   <221> CDS
   <222> (1)..(1281)
<400> 87
   atg cac gcc atc ctc atc gcc atc ggc tcg gcc ggc gac gta ttt ccc          48
   Met His Ala Ile Leu Ile Ala Ile Gly Ser Ala Gly Asp Val Phe Pro
   1               5                   10                  15
   ttc atc ggc ttg gcc cgg acc ctg aaa ttg cgc ggg cac cgc gtg agc          96
   Phe Ile Gly Leu Ala Arg Thr Leu Lys Leu Arg Gly His Arg Val Ser
               20                  25                  30
   ctc tgc acc atc ccg gtg ttt cgc gac gcg gtg gag cag cac ggc atc        144
   Leu Cys Thr Ile Pro Val Phe Arg Asp Ala Val Glu Gln His Gly Ile
           35                  40                  45
   gcg ttc gtc ccg ctg agc gac gaa ctg acc tac cgc cgg acc atg ggc        192
   Ala Phe Val Pro Leu Ser Asp Glu Leu Thr Tyr Arg Arg Thr Met Gly
       50                  55                  60
   gat ccg cgc ctg tgg gac ccc aag acg tcc ttc ggc gtg ctc tgg caa        240
   Asp Pro Arg Leu Trp Asp Pro Lys Thr Ser Phe Gly Val Leu Trp Gln
   65                  70                  75                  80
   gcc atc gcc ggg atg atc gag ccg gtc tac gag tac gtc tcg gcg cag        288
   Ala Ile Ala Gly Met Ile Glu Pro Val Tyr Glu Tyr Val Ser Ala Gln
                   85                  90                  95
   cgc cat gac gac atc gtg gtg gtc ggc tcg ctc tgg gcg ctg ggc gca        336
   Arg His Asp Asp Ile Val Val Val Gly Ser Leu Trp Ala Leu Gly Ala
               100                 105                 110
   cgc atc gct cac gag aag tac ggg att ccc tac ctg tcc gcg cag gtc        384
   Arg Ile Ala His Glu Lys Tyr Gly Ile Pro Tyr Leu Ser Ala Gln Val
           115                 120                 125
   tcg cca tcg acc ttg ttg tcg gcg cac ctg ccg ccg gta cac ccc aag        432
   Ser Pro Ser Thr Leu Leu Ser Ala His Leu Pro Pro Val His Pro Lys
       130                 135                 140
   ttc aac gtg ccc gag cag atg ccg ctg gcg atg cgc aag ctg ctc tgg        480
   Phe Asn Val Pro Glu Gln Met Pro Leu Ala Met Arg Lys Leu Leu Trp
   145                 150                 155                 160
   cgc tgc atc gag cgc ttc aag ctg gat cgc acc tgc gcg ccg gag atc        528
   Arg Cys Ile Glu Arg Phe Lys Leu Asp Arg Thr Cys Ala Pro Glu Ile
                   165                 170                 175
   aac gcg gtg cgc cgc aag gtc ggc ctg gag acg ccg gtg aag cgc atc        576
   Asn Ala Val Arg Arg Lys Val Gly Leu Glu Thr Pro Val Lys Arg Ile
               180                 185                 190
   ttc acc caa tgg atg cat tcg ccg cag ggc gtg gtc tgc ctg ttc ccg        624
   Phe Thr Gln Trp Met His Ser Pro Gln Gly Val Val Cys Leu Phe Pro
           195                 200                 205
   gcc tgg ttc gcg ccg ccc cag cag gat tgg ccg caa ccc ctg cac atg        672
   Ala Trp Phe Ala Pro Pro Gln Gln Asp Trp Pro Gln Pro Leu His Met
       210                 215                 220
   acc ggc ttc ccg ctg ttc gac ggc agt atc ccg ggg acc ccg ctc gac        720
   Thr Gly Phe Pro Leu Phe Asp Gly Ser Ile Pro Gly Thr Pro Leu Asp
   225                 230                 235                 240
   gac gaa ctg caa cgc ttt ctc gat cag ggc agc cgg ccg ctg gtg ttc        768
   Asp Glu Leu Gln Arg Phe Leu Asp Gln Gly Ser Arg Pro Leu Val Phe
                   245                 250                 255
   acc cag ggc tcg acc gaa cac ctg cag ggc gac ttc tac gcc atg gcc        816
   Thr Gln Gly Ser Thr Glu His Leu Gln Gly Asp Phe Tyr Ala Met Ala
               260                 265                 270
   ctg cgc gcg ctg gaa cgc ctc ggc gcg cgt ggg atc ttc ctc acc ggc        864
   Leu Arg Ala Leu Glu Arg Leu Gly Ala Arg Gly Ile Phe Leu Thr Gly
           275                 280                 285
   gcc ggc cag gaa ccg ctg cgc ggc ttg ccg aat cac gtg ctg cag cgc        912
   Ala Gly Gln Glu Pro Leu Arg Gly Leu Pro Asn His Val Leu Gln Arg
       290                 295                 300
   gcc tac gcg cca ctg gga gcc ttg ctg cca tcg tgc gcc ggg ctg gtc      960
   Ala Tyr Ala Pro Leu Gly Ala Leu Leu Pro Ser Cys Ala Gly Leu Val
   305                 310                 315                 320
   cat ccg ggc ggt atc ggc gcc atg agc ctg gcc ttg gcg gcg ggg gtg     1008
   His Pro Gly Gly Ile Gly Ala Met Ser Leu Ala Leu Ala Ala Gly Val
                   325                 330                 335
   ccg cag gtg ctg ctg ccc tgc gcc cac gac cag ttc gac aat gcc gaa     1056
   Pro Gln Val Leu Leu Pro Cys Ala His Asp Gln Phe Asp Asn Ala Glu
               340                 345                 350
   cgg ctg gtc cgg ctc ggc tgc ggg atg cgc ctg ggc gtg ccg ttg cgc     1104
   Arg Leu Val Arg Leu Gly Cys Gly Met Arg Leu Gly Val Pro Leu Arg
           355                 360                 365
   gag cag gag ttg cgc ggg gcg ctg tgg cgc ttg ctc gag gac ccg gcc     1152
   Glu Gln Glu Leu Arg Gly Ala Leu Trp Arg Leu Leu Glu Asp Pro Ala
       370                 375                 380
   atg gcg gcg gcc tgt cgg cgt ttc atg gaa ttg tca caa ccg cac agt     1200
   Met Ala Ala Ala Cys Arg Arg Phe Met Glu Leu Ser Gln Pro His Ser
   385                 390                 395                 400
   atc gct tgc ggt aaa gcg gcc cac gtg gtc gaa cgt tgt cat agg gag     1248
   Ile Ala Cys Gly Lys Ala Ala His Val Val Glu Arg Cys His Arg Glu
                   405                 410                 415
   ggg gat gcg cga tgg ctg aag gct gcg tcc tga                         1281
   Gly Asp Ala Arg Trp Leu Lys Ala Ala Ser
               420                 425
<210> 88
   <211> 426
   <212> PRT
   <213> Pseudomonas aeruginosa
<400> 88
   Met His Ala Ile Leu Ile Ala Ile Gly Ser Ala Gly Asp Val Phe Pro
   1               5                   10                  15
   Phe Ile Gly Leu Ala Arg Thr Leu Lys Leu Arg Gly His Arg Val Ser
               20                  25                  30
   Leu Cys Thr Ile Pro Val Phe Arg Asp Ala Val Glu Gln His Gly Ile
           35                  40                  45
   Ala Phe Val Pro Leu Ser Asp Glu Leu Thr Tyr Arg Arg Thr Met Gly
       50                  55                  60
   Asp Pro Arg Leu Trp Asp Pro Lys Thr Ser Phe Gly Val Leu Trp Gln
   65                  70                  75                  80
   Ala Ile Ala Gly Met Ile Glu Pro Val Tyr Glu Tyr Val Ser Ala Gln
                   85                  90                  95
   Arg His Asp Asp Ile Val Val Val Gly Ser Leu Trp Ala Leu Gly Ala
               100                 105                 110
   Arg Ile Ala His Glu Lys Tyr Gly Ile Pro Tyr Leu Ser Ala Gln Val
           115                 120                 125
   Ser Pro Ser Thr Leu Leu Ser Ala His Leu Pro Pro Val His Pro Lys
       130                 135                 140
   Phe Asn Val Pro Glu Gln Met Pro Leu Ala Met Arg Lys Leu Leu Trp
   145                 150                 155                 160
   Arg Cys Ile Glu Arg Phe Lys Leu Asp Arg Thr Cys Ala Pro Glu Ile
                   165                 170                 175
   Asn Ala Val Arg Arg Lys Val Gly Leu Glu Thr Pro Val Lys Arg Ile
               180                 185                 190
   Phe Thr Gln Trp Met His Ser Pro Gln Gly Val Val Cys Leu Phe Pro
           195                 200                 205
   Ala Trp Phe Ala Pro Pro Gln Gln Asp Trp Pro Gln Pro Leu His Met
       210                 215                 220
   Thr Gly Phe Pro Leu Phe Asp Gly Ser Ile Pro Gly Thr Pro Leu Asp
   225                 230                 235                 240
   Asp Glu Leu Gln Arg Phe Leu Asp Gln Gly Ser Arg Pro Leu Val Phe
                   245                 250                 255
   Thr Gln Gly Ser Thr Glu His Leu Gln Gly Asp Phe Tyr Ala Met Ala
               260                 265                 270
   Leu Arg Ala Leu Glu Arg Leu Gly Ala Arg Gly Ile Phe Leu Thr Gly
           275                 280                 285
   Ala Gly Gln Glu Pro Leu Arg Gly Leu Pro Asn His Val Leu Gln Arg
       290                 295                 300
   Ala Tyr Ala Pro Leu Gly Ala Leu Leu Pro Ser Cys Ala Gly Leu Val
   305                 310                 315                 320
   His Pro Gly Gly Ile Gly Ala Met Ser Leu Ala Leu Ala Ala Gly Val
                   325                 330                 335
   Pro Gln Val Leu Leu Pro Cys Ala His Asp Gln Phe Asp Asn Ala Glu
               340                 345                 350
   Arg Leu Val Arg Leu Gly Cys Gly Met Arg Leu Gly Val Pro Leu Arg
           355                 360                 365
   Glu Gln Glu Leu Arg Gly Ala Leu Trp Arg Leu Leu Glu Asp Pro Ala
       370                 375                 380
   Met Ala Ala Ala Cys Arg Arg Phe Met Glu Leu Ser Gln Pro His Ser
   385                 390                 395                 400
   Ile Ala Cys Gly Lys Ala Ala His Val Val Glu Arg Cys His Arg Glu
                   405                 410                 415
   Gly Asp Ala Arg Trp Leu Lys Ala Ala Ser
               420                 425
<210> 89
   <211> 978
   <212> DNA
   <213> Pseudomonas aeruginosa
<220>
   <221> CDS
   <222> (1)..(978)
<400> 89
   atg gac cgg ata gac atg ggc gtg ctg gtg gtg ctg ttc aat cct ggc        48
   Met Asp Arg Ile Asp Met Gly Val Leu Val Val Leu Phe Asn Pro Gly
   1               5                   10                  15
   gac gac gac ctg gaa cac ctt ggc gaa ctg gcg gcg gcc ttt ccg caa        96
   Asp Asp Asp Leu Glu His Leu Gly Glu Leu Ala Ala Ala Phe Pro Gln
               20                  25                  30
   ctg cgc ttc ctc gcc gtc gac aac tcg ccg cac agc gat ccg cag cgc       144
   Leu Arg Phe Leu Ala Val Asp Asn Ser Pro His Ser Asp Pro Gln Arg
           35                  40                  45
   aac gcc cgg ctg cgc ggg caa ggc atc gcc gtg ctc tac cac ggc aac       192
   Asn Ala Arg Leu Arg Gly Gln Gly Ile Ala Val Leu Tyr His Gly Asn
       50                  55                  60
   cgg cag ggc atc gcc ggc gcc ttc aac cag ggg ctc gac acg ctg ttc       240
   Arg Gln Gly Ile Ala Gly Ala Phe Asn Gln Gly Leu Asp Thr Leu Phe
   65                  70                  75                  80
   cgg cgc ggc ctg cag ggt gtg ctg ctg ctc gac cag gac tcc cgt ccc       288
   Arg Arg Gly Leu Gln Gly Val Leu Leu Leu Asp Gln Asp Ser Arg Pro
                   85                  90                  95
   ggc ggc gcc ttc ctc gcc gcc cag tgg cgc aac ctg cag gca tgc aac       336
   Gly Gly Ala Phe Leu Ala Ala Gln Trp Arg Asn Leu Gln Ala Cys Asn
               100                 105                 110
   ggc cag gcc tgc ctg ctc ggc cca cgg atc ttc gac cgg ggc gac cgg       384
   Gly Gln Ala Cys Leu Leu Gly Pro Arg Ile Phe Asp Arg Gly Asp Arg
           115                 120                 125
   cgc ttc ctg ccg gcc atc cac ctc gac ggg ctg gcg ctc agg caa ctg       432
   Arg Phe Leu Pro Ala Ile His Leu Asp Gly Leu Ala Leu Arg Gln Leu
       130                 135                 140
   tcc ctg gac ggc ctg acg acc cca cag cgc acc tcg ttc ctg atc tcc        480
   Ser Leu Asp Gly Leu Thr Thr Pro Gln Arg Thr Ser Phe Leu Ile Ser
   145                 150                 155                 160
   tcc ggc tgc ctg ctg acc cgc gag gcc tac cag cgc ctc ggc cac ttc        528
   Ser Gly Cys Leu Leu Thr Arg Glu Ala Tyr Gln Arg Leu Gly His Phe
                   165                 170                 175
   gac gag gaa ctg ttc atc gac cac gtg gac acc gag tac agc ctg cgc        576
   Asp Glu Glu Leu Phe Ile Asp His Val Asp Thr Glu Tyr Ser Leu Arg
               180                 185                 190
   gcc cag gcg ctg gac gtg ccc ctg tac gtc gac ccg cgg ctg gtc ctc        624
   Ala Gln Ala Leu Asp Val Pro Leu Tyr Val Asp Pro Arg Leu Val Leu
           195                 200                 205
   gag cac cgc atc ggc acg cgc aag acc cgc cgc ctc ggc ggt ctc agc        672
   Glu His Arg Ile Gly Thr Arg Lys Thr Arg Arg Leu Gly Gly Leu Ser
       210                 215                 220
   ctc agc gcg atg aac cac gcc cca ctg cgc cgc tac tac ctg gcg cgc        720
   Leu Ser Ala Met Asn His Ala Pro Leu Arg Arg Tyr Tyr Leu Ala Arg
   225                 230                 235                 240
   aac ggc ctg ctg gtc ctg cgc cgc tac gcc cgg tcc tcg ccg ctg gcc        768
   Asn Gly Leu Leu Val Leu Arg Arg Tyr Ala Arg Ser Ser Pro Leu Ala
                   245                 250                 255
   ctg ctg gcg aac ctg ccg acc ctg acc cag ggc ctc gcg gtg ctc ctg        816
   Leu Leu Ala Asn Leu Pro Thr Leu Thr Gln Gly Leu Ala Val Leu Leu
               260                 265                 270
   ctc gaa cgc gac aag ctg ctc aag ctg cgc tgc ctg ggc tgg ggc ctg        864
   Leu Glu Arg Asp Lys Leu Leu Lys Leu Arg Cys Leu Gly Trp Gly Leu
           275                 280                 285
   tgg gac ggc ctg cgg ggg cgc ggc ggc gcg ctg gag cgc aac cgc ccg        912
   Trp Asp Gly Leu Arg Gly Arg Gly Gly Ala Leu Glu Arg Asn Arg Pro
       290                 295                 300
   cgc ctg ctg aag cgc ctc gcc ggt ccg gcg gtg gcg ccc aca gtt ccc        960
   Arg Leu Leu Lys Arg Leu Ala Gly Pro Ala Val Ala Pro Thr Val Pro
   305                 310                 315                 320
   ggc aag gcc aag gcc tag                                                978
   Gly Lys Ala Lys Ala
                   325
<210> 90
   <211> 325
   <212> PRT
   <213> Pseudomonas aeruginosa
<400> 90
   Met Asp Arg Ile Asp Met Gly Val Leu Val Val Leu Phe Asn Pro Gly
   1               5                   10                  15
   Asp Asp Asp Leu Glu His Leu Gly Glu Leu Ala Ala Ala Phe Pro Gln
               20                  25                  30
   Leu Arg Phe Leu Ala Val Asp Asn Ser Pro His Ser Asp Pro Gln Arg
           35                  40                  45
   Asn Ala Arg Leu Arg Gly Gln Gly Ile Ala Val Leu Tyr His Gly Asn
       50                  55                  60
   Arg Gln Gly Ile Ala Gly Ala Phe Asn Gln Gly Leu Asp Thr Leu Phe
   65                  70                  75                  80
   Arg Arg Gly Leu Gln Gly Val Leu Leu Leu Asp Gln Asp Ser Arg Pro
                   85                  90                  95
   Gly Gly Ala Phe Leu Ala Ala Gln Trp Arg Asn Leu Gln Ala Cys Asn
               100                 105                 110
   Gly Gln Ala Cys Leu Leu Gly Pro Arg Ile Phe Asp Arg Gly Asp Arg
           115                 120                 125
   Arg Phe Leu Pro Ala Ile His Leu Asp Gly Leu Ala Leu Arg Gln Leu
       130                 135                 140
   Ser Leu Asp Gly Leu Thr Thr Pro Gln Arg Thr Ser Phe Leu Ile Ser
   145                 150                 155                 160
   Ser Gly Cys Leu Leu Thr Arg Glu Ala Tyr Gln Arg Leu Gly His Phe
                   165                 170                 175
   Asp Glu Glu Leu Phe Ile Asp His Val Asp Thr Glu Tyr Ser Leu Arg
               180                 185                 190
   Ala Gln Ala Leu Asp Val Pro Leu Tyr Val Asp Pro Arg Leu Val Leu
           195                 200                 205
   Glu His Arg Ile Gly Thr Arg Lys Thr Arg Arg Leu Gly Gly Leu Ser
       210                 215                 220
   Leu Ser Ala Met Asn His Ala Pro Leu Arg Arg Tyr Tyr Leu Ala Arg
   225                 230                 235                 240
   Asn Gly Leu Leu Val Leu Arg Arg Tyr Ala Arg Ser Ser Pro Leu Ala
                   245                 250                 255
   Leu Leu Ala Asn Leu Pro Thr Leu Thr Gln Gly Leu Ala Val Leu Leu
               260                 265                 270
   Leu Glu Arg Asp Lys Leu Leu Lys Leu Arg Cys Leu Gly Trp Gly Leu
           275                 280                 285
   Trp Asp Gly Leu Arg Gly Arg Gly Gly Ala Leu Glu Arg Asn Arg Pro
       290                 295                 300
   Arg Leu Leu Lys Arg Leu Ala Gly Pro Ala Val Ala Pro Thr Val Pro
   305                 310                 315                 320
   Gly Lys Ala Lys Ala
                   325
<210> 91
   <211> 978
   <212> DNA
   <213> Pseudomonas aeruginosa
<220>
   <221> CDS
   <222> (1)..(978)
<400> 91
   atg gac cgg ata gac atg ggc gtg ctg gtg gta ctg ttc aat cct ggc      48
   Met Asp Arg Ile Asp Met Gly Val Leu Val Val Leu Phe Asn Pro Gly
   1               5                   10                  15
   gac gac gac ctg gaa cac ctt ggc gaa ctg gcg gcg gcg ttt ccg caa      96
   Asp Asp Asp Leu Glu His Leu Gly Glu Leu Ala Ala Ala Phe Pro Gln
               20                  25                  30
   ctg cgc ttc ctc gcc gtc gac aac tca ccg cac agc gat ccg cag cgc     144
   Leu Arg Phe Leu Ala Val Asp Asn Ser Pro His Ser Asp Pro Gln Arg
           35                  40                  45
   aat gcc cgg ctg cgc ggg caa ggc atc gcc gtg ctg cac cac ggc aac     192
   Asn Ala Arg Leu Arg Gly Gln Gly Ile Ala Val Leu His His Gly Asn
       50                  55                  60
   cgg cag ggc atc gcc ggc gcc ttc aac cag ggg ctc gac gcg ctg ttc     240
   Arg Gln Gly Ile Ala Gly Ala Phe Asn Gln Gly Leu Asp Ala Leu Phe
   65                  70                  75                  80
   cgg cgt ggc gtg cag ggt gtg ctg ctg ctc gac cag gac tcc cgt ccc     288
   Arg Arg Gly Val Gln Gly Val Leu Leu Leu Asp Gln Asp Ser Arg Pro
                   85                  90                  95
   ggc ggc gcc ttc ctc gcc gcc cag tgg cgc aac ctg cag gcg cgc aac     336
   Gly Gly Ala Phe Leu Ala Ala Gln Trp Arg Asn Leu Gln Ala Arg Asn
               100                 105                 110
   ggt cag gcc tgc ctg ctc ggc cca cgg atc ttc gac cgg ggt gac cgg     384
   Gly Gln Ala Cys Leu Leu Gly Pro Arg Ile Phe Asp Arg Gly Asp Arg
           115                 120                 125
   cgc ttc ctg ccg gcc atc cat ctc gac gga ctg acg ctc agg caa ttg     432
   Arg Phe Leu Pro Ala Ile His Leu Asp Gly Leu Thr Leu Arg Gln Leu
       130                 135                 140
   tct ctg gac ggc ctg acg acc ccg cag cgc acc tcg ttc ctg atc tcc     480
   Ser Leu Asp Gly Leu Thr Thr Pro Gln Arg Thr Ser Phe Leu Ile Ser
   145                 150                 155                 160
   tcc ggc tgc ctg ctg acc cgc gag gcc tac cag cgc ctc ggc cac ttc     528
   Ser Gly Cys Leu Leu Thr Arg Glu Ala Tyr Gln Arg Leu Gly His Phe
                   165                 170                 175
   gac gag gaa ctg ttc atc gac cac gtg gac acc gaa tac agc ctg cgc        576
   Asp Glu Glu Leu Phe Ile Asp His Val Asp Thr Glu Tyr Ser Leu Arg
               180                 185                 190
   gcc cag gcg ctg gac gtg ccc ctg tac gtc gac ccg cgg ctg gtc ctc        624
   Ala Gln Ala Leu Asp Val Pro Leu Tyr Val Asp Pro Arg Leu Val Leu
           195                 200                 205
   gag cac cgc atc ggc acg cgc aag acc cgc cgc ctc ggc ggt ctc agc        672
   Glu His Arg Ile Gly Thr Arg Lys Thr Arg Arg Leu Gly Gly Leu Ser
       210                 215                 220
   ctc agc gcg atg aac cac gcc ccg ctg cgc cgc tac tac ctg gcg cgc        720
   Leu Ser Ala Met Asn His Ala Pro Leu Arg Arg Tyr Tyr Leu Ala Arg
   225                 230                 235                 240
   aac ggc ctg ctg gtc ctg cgc cgc tac gcc cgg tcc tcg ccg ctg gcc        768
   Asn Gly Leu Leu Val Leu Arg Arg Tyr Ala Arg Ser Ser Pro Leu Ala
                   245                 250                 255
   ctg ctg gcg aac ctg ccg acc ctg acc cag ggc ctc gcg gtg ctc ctg        816
   Leu Leu Ala Asn Leu Pro Thr Leu Thr Gln Gly Leu Ala Val Leu Leu
               260                 265                 270
   ctc gaa cgc gac aag ctg ctc aag ctg cgc tgc ctg ggc tgg ggc ctg        864
   Leu Glu Arg Asp Lys Leu Leu Lys Leu Arg Cys Leu Gly Trp Gly Leu
           275                 280                 285
   tgg gac ggc ctg cgg gga cgc ggc ggc gcg ctg gag cgc aac cgc ccg        912
   Trp Asp Gly Leu Arg Gly Arg Gly Gly Ala Leu Glu Arg Asn Arg Pro
       290                 295                 300
   cgc ctg ctg aag cgc ctc gcc ggc ccg gcc gtg gcg tcc gta gct tcc        960
   Arg Leu Leu Lys Arg Leu Ala Gly Pro Ala Val Ala Ser Val Ala Ser
   305                 310                 315                 320
   ggc aag gcc aag gcc tag                                                978
   Gly Lys Ala Lys Ala
                   325
<210> 92
   <211> 325
   <212> PRT
   <213> Pseudomonas aeruginosa
<400> 92
   Met Asp Arg Ile Asp Met Gly Val Leu Val Val Leu Phe Asn Pro Gly
   1               5                   10                  15
   Asp Asp Asp Leu Glu His Leu Gly Glu Leu Ala Ala Ala Phe Pro Gln
               20                  25                  30
   Leu Arg Phe Leu Ala Val Asp Asn Ser Pro His Ser Asp Pro Gln Arg
           35                  40                  45
   Asn Ala Arg Leu Arg Gly Gln Gly Ile Ala Val Leu His His Gly Asn
       50                  55                  60
   Arg Gln Gly Ile Ala Gly Ala Phe Asn Gln Gly Leu Asp Ala Leu Phe
   65 70 75 80
   Arg Arg Gly Val Gln Gly Val Leu Leu Leu Asp Gln Asp Ser Arg Pro
   85 90 95
   Gly Gly Ala Phe Leu Ala Ala Gln Trp Arg Asn Leu Gln Ala Arg Asn
   100 105 110
   Gly Gln Ala Cys Leu Leu Gly Pro Arg Ile Phe Asp Arg Gly Asp Arg
   115 120 125
   Arg Phe Leu Pro Ala Ile His Leu Asp Gly Leu Thr Leu Arg Gln Leu
   130 135 140
   Ser Leu Asp Gly Leu Thr Thr Pro Gln Arg Thr Ser Phe Leu Ile Ser
   145 150 155 160
   Ser Gly Cys Leu Leu Thr Arg Glu Ala Tyr Gln Arg Leu Gly His Phe
   165 170 175
   Asp Glu Glu Leu Phe Ile Asp His Val Asp Thr Glu Tyr Ser Leu Arg
   180 185 190
   Ala Gln Ala Leu Asp Val Pro Leu Tyr Val Asp Pro Arg Leu Val Leu
   195 200 205
   Glu His Arg Ile Gly Thr Arg Lys Thr Arg Arg Leu Gly Gly Leu Ser
   210 215 220
   Leu Ser Ala Met Asn His Ala Pro Leu Arg Arg Tyr Tyr Leu Ala Arg
   225 230 235 240
   Asn Gly Leu Leu Val Leu Arg Arg Tyr Ala Arg Ser Ser Pro Leu Ala
   245 250 255
   Leu Leu Ala Asn Leu Pro Thr Leu Thr Gln Gly Leu Ala Val Leu Leu
   260 265 270
   Leu Glu Arg Asp Lys Leu Leu Lys Leu Arg Cys Leu Gly Trp Gly Leu
   275 280 285
   Trp Asp Gly Leu Arg Gly Arg Gly Gly Ala Leu Glu Arg Asn Arg Pro
   290 295 300
   Arg Leu Leu Lys Arg Leu Ala Gly Pro Ala Val Ala Ser Val Ala Ser
   305 310 315 320
   Gly Lys Ala Lys Ala
                   325

## Beschreibung

### Gebiet der Erfindung

Gegenstand der Erfindung sind Zellen und Verfahren zur Herstellung von Rhamnolipiden.

### Stand der Technik

Surfactants werden heutzutage im Wesentlichen basierend auf Basis petrochemischer Rohstoffe hergestellt. Die Nutzung von Surfactants auf Basis nachwachsender Rohstoffe ist aufgrund der absehbaren Verknappung petrochemischer Rohstoffe und zunehmender Nachfrage nach Produkten, die auf nachwachsenden Rohstoffen basieren bzw. biologisch abbaubar sind, eine entsprechende Alternative.

Rhamnolipide bestehen aus ein (Monorhamnosyllipide) oder zwei Rhamnoseresten (Dirhamnosyllipide) und ein oder zwei 3-Hydroxyfettsäureresten (siehe Handbook of Hydrocarbon and Lipid Microbiology, 2010, Seiten 3037-51). Sie besitzen oberflächenaktive Eigenschaften, die man zum Einsatz als Surfactant in verschiedensten Anwendungen benötigt (siehe Leitermann *et al.,* 2009).

Diese Lipide werden heutzutage mit Wildtyp-Isolaten verschiedener human- und tierpathogener Bakterien, insbesondere Vertreter der Gattungen *Pseudomonas* und *Burkholderia,* hergestellt (siehe Handbook of Hydrocarbon and Lipid Microbiology, 2010, Seiten 3037-51). Die Tatsache, dass diese Produktionsorganismen in der Lage sind Krankheiten auszulösen, reduziert die Kundenakzeptanz für die konventionell hergestellten Rhamnolipide ganz erheblich. Zudem wirken sich höhere Sicherheitsanforderungen durch ein erhöhtes Investitionsvolumen und ggf. zusätzliche Aufarbeitungsschritte auch auf die Herstellkosten aus.

Obwohl mit Hilfe dieser Produktionsorganismen z.T. hohe Produkttiter, sowie Raum-Zeit- bzw. Kohlenstoffausbeuten erzielt werden können, setzt dies den Einsatz von pflanzlichen Ölen als alleiniges oder Co-Substrat voraus (siehe Handbook of Hydrocarbon and Lipid Microbiology, 2010, Seiten 3037-51). Pflanzliche Öle sind jedoch im Vergleich zu anderen Kohlenstoffquellen, wie etwa Glukose, Saccharose oder Polysacchariden wie z.B. Stärke, Zellulose und Hemizellulose, Glyzerin, CO, CO₂ oder CH₄ vergleichsweise teure Rohstoffe. Zudem zeichnen sich Rhamnolipide aufgrund ihres Tensidcharakters dadurch aus, dass sie in Fermentationsprozessen zu starkem Schäumen neigen. Dies ist insbesondere dann der Fall, wenn lipophile Substrate eingesetzt werden. Diese Problematik wird bei Verwendung wasserlöslicher Substrate wie etwa Glukose, Saccharose, Polysaccharide (Stärke, Zellulose, Hemizellulose) oder Glyzerin deutlich reduziert.

Schließlich sind die Eigenschaften der von den Wildtyp-Isolaten produzierten Rhamnolipide nur eingeschränkt beeinflussbar. Dies findet bisher ausschließlich über die Optimierung der Prozessführung (pH-Wert, Sauerstoffversorgung, Medienzusammensetzung, *feeding-*Strategien, Stickstoffversorgung, Temperatur, Substratwahl, etc.) statt. Jedoch wäre eine sehr gezielte Beeinflussung bestimmter Produkteigenschaften, wie etwa das Verhältnis der verschiedenen Rhamnolipidspezies (Anzahl von Rhamnose- und 3-Hydroxyfettsäure-Resten) oder Kettenlänge und Sättigungsgrad der 3-Hydroxyfettsäureresten wünschenswert, um die für die Anwendung relevanten Produkteigenschaften modulieren zu können.

Rhamnolipide werden, sollen sie im großen Maße als Surfactants in Haushalt-, Reinigungs-, Kosmetik-, Nahrungsmittelprozessierungs-, Pharma-, Pflanzenschutz- und anderen Anwendungen eingesetzt werden, in den Wettbewerb mit den heutzutage eingesetzten Surfactants treten müssen. Diese sind großvolumige Chemikalien, die zu sehr niedrigen Kosten, ohne offensichtliche gesundheitliche Risiken für den Kunden und mit klar definierten und modulierbaren Produktspezifikationen hergestellt werden können. Daher müssen auch Rhamnolipide zu möglichst niedrigen Kosten, ohne gesundheitliche Risiken für den Kunden und mit möglichst definierten Eigenschaften hergestellt werden können.

Zwar sind Rhamnolipide bereits in GRAS-Organismen (*generally regarded as save*) basierend auf günstigen Kohlenstoff-Quellen, wie etwa Glukose oder Glyzerin hergestellt worden, jedoch handelt es sich dabei ausschließlich um Monorhamnosyllipide (Ochsner et al. Appl. Environ. Microbiol. 1995. 61(9):3503-3506).

Cha et al. in Bioresour Technol. 2008. 99(7):2192-9 beschreiben andererseits die Herstellung von Monorhamnosyllipiden aus Soyaöl in *P. putida* durch Einbringen der Gene *rhlA* und *rhlB* aus *Pseudomonas aeruginosa.*

WO2004050882 offenbart ein Verfahren zur Phytoremediation einer Umgebung, die mit mindestens einem Schwermetall oder Ölkohlenwasserstoff kontaminiert ist, wobei das Verfahren umfasst: (a) Bereitstellen einer transgenen Pflanze, wobei die Pflanze mindestens eine heterologe Nukleinsäure exprimiert, die ein Enzym mit Rhamnosyltransferase-Aktivität codiert, (b) Pflanzen oder Lokalisieren der transgenen Pflanze in der Umgebung.

US2007020624 betrifft isolierte Nukleinsäuren und Polypeptide, die von Pseudomonas aeruginosa abgeleitet sind, die als molekulare Ziele für die Diagnose, Prophylaxe und Behandlung von pathologischen Zuständen sowie Materialien und Verfahren zur Diagnose, Vorbeugung und Linderung von pathologischen Zuständen, die aus bakteriellen Infektionen resultieren, nützlich sind.

WO2004083385 offenbart ein Verfahren zum Identifizieren eines Modulators der Quorumsensing-Signalgebung in Bakterien, wobei das Verfahren umfasst: Bereitstellen einer Zelle, die ein Quorumsensing-gesteuertes Gen umfasst, wobei die Zelle auf ein Quorum-Sensing-Signalmolekül anspricht, so dass ein detektierbares Signal erzeugt wird: in Kontakt bringen der Zelle mit einem Quorum-Sensino-Signalmolekül in Gegenwart und Abwesenheit einer Testverbindung; und Detektieren einer Änderung in dem detektierbaren Signal, um dadurch die Testverbindung als einen Modulator der Quorum Sensing Signalisierung in Bakterien zu identifizieren.

Ochsner et al. "Production of Pseudomonas aeruginosa rhamnolipid biosurfactants in heterologous hosts", Applied and Environmental Micorbiology, vol. 61, no. 9, page 3503, offenbart den Pseudomonas aeruginosa Stamm PG201 mit erhöhter Rhamnosyltransferase-Aktivität.

Der abschließende Projekt-Bericht Bio-Engineering High Performance Microbial Strains for MEOR by Directed Protein Evolution Technology", 2008, Oil & Natural Gas Technology Projects, offenbart Pseudomonas aeruginosa, Pseudomonas fluorescens und *E. coli* Stämme enthaltend die Rhamnosyltransferase Gene rhlAB und aufweisend erhöhte Rhamnolipidproduktionsraten.

"Pseudomonas aeruginosa rhamnosyl transferase genes and regulatory protein gene, complete cds.", EMBL, Database accession no. L28170, offenbart die Nukleinsäure- und Aminosäuresequenzen von als Rhamnosyltransferasen annotierten Proteinen aus Pseudomonas aeruginosa.

Es besteht daher ein steigender Bedarf an der kostengünstigen und aus gesundheitlicher Sicht sicheren Herstellung von Mono- und Dirhamnosyllipiden mit definierten sowie modulierbaren Eigenschaften.
Diese Modulation kann beispielsweise durch eine ausgewogene Bereitstellung der einzelnen Enzymaktivitäten erfolgen, welche die Anreicherung von Monorhamnosyllipiden reduziert. Diese Modulation kann aber auch beispielsweise erfolgen durch die Verwendung von Enzymen mit bestimmten Eigenschaften z.B. bzgl. Substratspezifität und somit etwa der Kettenlänge der in Rhamnolipide eingebauten Hydroxyfettsäuren.

Der vorliegenden Erfindung lag daher die Aufgabe zugrunde, eine Möglichkeit bereitzustellen, Rhamnolipide mit sicheren Produktionswirten aus gut zugänglichen Kohlenstoffquellen herzustellen.

### Beschreibung der Erfindung

Überraschenderweise wurde gefunden, dass die im Folgenden beschriebenen Zellen sowie Verfahren, in denen diese Zellen eingesetzt werden, einen Beitrag leisten, die der Erfindung gestellte Aufgabe zu lösen.

Gegenstand der vorliegenden Erfindung sind daher Zellen, welche Rhamnolipide zu bilden vermögen und verglichen zu ihrem Wildtyp mindestens eine gesteigerte Aktivität eines Genproduktes von Homologen der Genprodukte *rhlA, rhlB* und *rhlC* sowie weiteren wie in Anspruch 1 beschriebenen aufweisen.
Ein weiterer Gegenstand der Erfindung ist ein Verfahren zur Herstellung von Rhamnolipiden unter Einsatz der vorgenannten Zellen als Biokatalysator und einfacher Kohlenstoffquellen.

Ein Vorteil der vorliegenden Erfindung ist es, dass Organismen eingesetzt werden können, die nicht pathogen und einfach zu kultivieren sind.
Ein weiterer Vorteil ist es, dass ein Einsatz von Ölen als alleiniges oder Co-Substrat nicht notwendig ist.
Noch ein Vorteil ist es, dass sich mit Hilfe der Erfindung Rhamnolipide mit definierten sowie modulierbaren Eigenschaften herstellen lassen.
Noch ein Vorteil der vorliegenden Erfindung ist es, dass sich Dirhamnosyllipide darstellen lassen.
Ein weiterer Vorteil ist, dass sich Rhamnolipide mit höheren Raum-Zeit- und Kohlenstoffausbeuten als mit Zellen ohne Verstärkung dieser Aktivitäten herstellen lassen.

Einen Beitrag zur Lösung der eingangs genannten Aufgabe leistet eine Zelle, ausgewählt aus Mikroorganismen, bevorzugt eine isolierte Zelle, welche mindestens ein Rhamnolipid der allgemeinen Formel (I) oder dessen Salz zu bilden vermag, 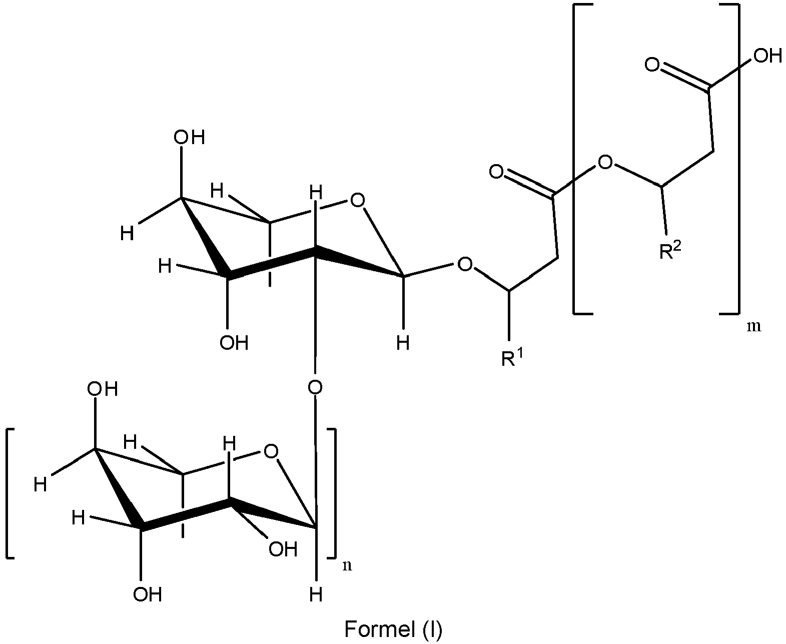 wobei
m = 2, 1 oder 0, insbesondere 1 oder 0,
n = 1 oder 0, insbesondere 1,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12, dadurch gekennzeichnet, dass sie derart gentechnisch verändert wurde, dass sie verglichen zu ihrem Wildtyp eine gesteigerte Aktivität mindestens eines der Enzyme E₁, E₂ und E₃ aufweist, wobei das Enzym E₁ die Umsetzung von 3-Hydroxyalkanoyl-ACP über 3-Hydroxyalkanoyl-3-Hydroxyalkansäure-ACP zu Hydroxyalkanoyl-3-Hydroxyalkansäure zu katalysieren vermag, das Enzym E₂ eine Rhamnosyltransferase I ist und die Umsetzung von dTDP-Rhamnose und 3-Hydroxyalkanoyl-3-Hydroxyalkanoat zu α-L-Rhamnopyranosyl-3-Hydroxyalkanoyl-3-Hydroxyalkanoat zu katalysieren vermag und das Enzym E₃ eine Rhamnosyltransferase II ist und die Umsetzung von dTDP-Rhamnose und α-L-Rhamnopyranosyl-3-Hydroxyalkanoyl-3-Hydroxyalkanoat zu α-L-rhamnopyranosyl-(1-2)-α-L-rhamnopyranosyl-3-Hydroxyalkanoyl-3-Hydroxyalkanoat zu katalysieren vermag, und dass sie verglichen zu ihrem Wildtyp eine gesteigerte, wie jeweils im Folgenden spezifizierte Aktivität mindestens eines der Enzyme ausgewählt aus der Gruppe bestehend aus
ein Enzym E₄, eine dTTP:α-D-Glukose-1-Phosphat Thymidylyltransferase, EC 2.7.7.24, insbesondere eine mit Polypeptidsequenz Seq ID Nr. 10 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 10 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 10 besitzt, wobei unter enzymatischer Aktivität für ein Enzym E₄ die Fähigkeit verstanden wird, α-D-Glukose-1-Phosphat und dTTP zu dTDP-Glukose umzusetzen,
ein Enzym E₅, eine dTTP-Glukose-4,6-Hydrolyase, EC 4.2.1.46, insbesondere eine mit Polypeptidsequenz Seq ID Nr. 12 oder mit einer Polypeptidsequenz bei der bis zu 25%, bevorzugt bis zu 20%, besonders bevorzugt bis zu 15% insbesondere bis zu 10. 9. 8, 7. 6, 5, 4, 3, 2, 1% der Aminosäurereste gegenüber der Bezugssequenz Seq ID Nr. 12 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 12 besitzt, wobei unter enzymatischer Aktivität für ein Enzym E₅ die Fähigkeit verstanden wird, dTDP-Glukose zu dTDP-4-Dehydro-6-Deoxy-D-Glukose umzusetzen,
ein Enzym E₆, eine dTDP-4-Dehydrorhamnose-3,5-Epimerase, EC 5.1.3.13, insbesondere eine mit Polypeptidsequenz Seq ID Nr. 14 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 14 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 14 besitzt, wobei unter enzymatischer Aktivität für ein Enzym E₆ die Fähigkeit verstanden wird, dTDP-4-Dehydro-6-Deoxy-D-Glukose zu dTDP-4-Dehydro-6-Deoxy-L-Mannose umzusetzen und
ein Enzym E₇, eine dTDP-4-Dehydrorhamnose-Reduktase, EC 1.1.1.133, insbesondere eine mit Polypeptidsequenz Seq ID Nr. 16 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 16 durch Deletion, Insertion. Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 16 besitzt, wobei unter enzymatischer Aktivität für ein Enzym E₇ die Fähigkeit verstanden wird, dTDP-4-Dehydro-6-Deoxy-L-Mannose zu dTDP-6-Deoxy-L-Mannose umzusetzen,
   aufweist,
wobei diese Enzyme E₁, E₂ und E₃ bevorzugt
ausgewählt werden aus der Gruppe bestehend aus, ein Enzym E₁ ausgewählt aus
ein Enzym E₁ₐ mit Polypeptidsequenz Seq ID Nr. 2 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 2 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 2 besitzt, wobei unter enzymatischer Aktivität für ein Enzym E₁ₐ die Fähigkeit verstanden wird, bevorzugt 3-Hydroxydekanoyl-ACP über 3-Hydroxydekanoyl-3-Hydroxydekansäure-ACP zu Hydroxydekanoyl-3-Hydroxydekansäure umzusetzen,
ein Enzym E_{1b} mit Polypeptidsequenz Seq ID Nr. 18 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 18 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 18 besitzt, wobei unter enzymatischer Aktivität für ein Enzym E_{1b} die Fähigkeit verstanden wird, bevorzugt 3-Hydroxytetradekanoyl-ACP über 3-Hydroxytetradekanoyl-3-Hydroxytetradekansäure-ACP zu Hydroxytetradekanoyl-3-Hydroxytetradekansäure umzusetzen,
ein Enzym E_{1c} mit Polypeptidsequenz Seq ID Nr. 78 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 78 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 78 besitzt, wobei unter enzymatischer Aktivität für ein Enzym E_{1c} die Fähigkeit verstanden wird, bevorzugt 3-Hydroxytetradekanoyl-ACP über 3-Hydroxytetradekanoyl-3-Hydroxytetradekansäure-ACP zu Hydroxytetradekanoyl-3-Hydroxytetradekansäure umzusetzen,
ein Enzym E_{1d} mit Polypeptidsequenz Seq ID Nr. 80 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 80 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 80 besitzt, wobei unter enzymatischer Aktivität für ein Enzym E_{1d} die Fähigkeit verstanden wird, bevorzugt 3-Hydroxytetradekanoyl-ACP über 3-Hydroxytetradekanoyl-3-Hydroxytetradekansäure-ACP zu Hydroxytetradekanoyl-3-Hydroxytetradekansäure umzusetzen, und
ein Enzym E₁ₑ mit Polypeptidsequenz Seq ID Nr. 82 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 82 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 82 besitzt, wobei unter enzymatischer Aktivität für ein Enzym E₁ₑ die Fähigkeit verstanden wird, bevorzugt 3-Hydroxytetradekanoyl-ACP über 3-Hydroxytetradekanoyl-3-Hydroxytetradekansäure-ACP zu Hydroxytetradekanoyl-3-Hydroxytetradekansäure umzusetzen,
ein Enzym E₂ mit Polypeptidsequenz ausgewählt aus ein Enzym E₂ₐ mit Polypeptidsequenz Seq ID Nr. 4 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 4 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 4 besitzt, wobei unter enzymatischer Aktivität für ein Enzym E₂ₐ die Fähigkeit verstanden wird, bevorzugt dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
ein Enzym E_{2b} mit Polypeptidsequenz Seq ID Nr. 20 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 20 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 20 besitzt, wobei unter enzymatischer Aktivität für ein Enzym E_{2b} die Fähigkeit verstanden wird, bevorzugt dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
ein Enzym E_{2c} mit Polypeptidsequenz Seq ID Nr. 84 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 84 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 84 besitzt, wobei unter enzymatischer Aktivität für ein Enzym E_{2c} die Fähigkeit verstanden wird, bevorzugt dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
ein Enzym E_{2d} mit Polypeptidsequenz Seq ID Nr. 86 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 86 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 86 besitzt, wobei unter enzymatischer Aktivität für ein Enzym E_{2d} die Fähigkeit verstanden wird, bevorzugt dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen, und
ein Enzym E₂ₑ mit Polypeptidsequenz Seq ID Nr. 88 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 88 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 88 besitzt, wobei unter enzymatischer Aktivität für ein Enzym E₂ₑ die Fähigkeit verstanden wird, bevorzugt dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen, und
ein Enzym E₃ ausgewählt aus ein Enzym E₃ₐ mit Polypeptidsequenz Seq ID Nr. 6 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 6 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 6 besitzt, wobei unter enzymatischer Aktivität für ein Enzym E₃ₐ die Fähigkeit verstanden wird, bevorzugt dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
ein Enzym E_{3b} mit Polypeptidsequenz Seq ID Nr. 22 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 22 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 22 besitzt, wobei unter enzymatischer Aktivität für ein Enzym E_{3b} die Fähigkeit verstanden wird, bevorzugt dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
ein Enzym E_{3c} mit Polypeptidsequenz Seq ID Nr. 90 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 90 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der Bezugssequenz Seq ID Nr. 90 besitzt, wobei unter enzymatischer Aktivität für ein Enzym E_{3c} die Fähigkeit verstanden wird, bevorzugt dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen, und
ein Enzym E_{3d} mit Polypeptidsequenz Seq ID Nr. 92 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der Bezugssequenz Seq ID Nr. 92 durch Deletion, Insertion, Substitution oder eine Kombination davon verändert sind und die noch mindestens 10%, bevorzugt 50%, besonders bevorzugt 80%, insbesondere mehr als 92% der enzymatischen Aktivität des Enzyms mit der Bezugssequenz Seq ID Nr. 92 besitzt, wobei unter enzymatischer Aktivität für ein Enzym E_{3d} die Fähigkeit verstanden wird, bevorzugt dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen.

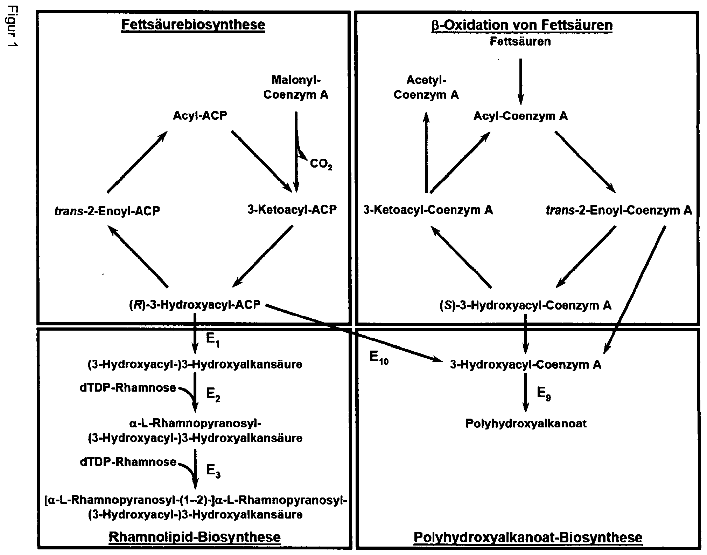

Zur Übersicht vergleiche Figur 1.

Mit "Wildtyp" einer Zelle wird hierin eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind. Unter dem Begriff "Rhamnolipid" wird im Zusammenhang mit der vorliegenden Erfindung eine Verbindung der allgemeinen Formel (I) oder dessen Salz verstanden.
Es ist offenbar, dass die oben konkret angegebenen Aktivitäten für die Enzyme E₁ₐ bis E_{3b} nur eine spezielle beispielhafte Auswahl eines breiteren Aktivitätsspektrum der vorgenannten Enzyme darstellt; die jeweilig genannte Aktivität ist diejenige, für die ein verlässliches Messverfahren bei gegebenem Enzym vorhanden ist. So ist es offensichtlich, dass ein Enzym, welches ein Substrat mit einem unverzweigten, gesättigten C₁₀-Alkylrest ebenfalls - wenn auch gegebenenfalls mit verringerter Aktivität - solche Substrate umsetzen wird, die einen C₆- oder C₁₆-Alkylrest aufweisen, der gegebenenfalls auch verzweigt oder ungesättigt sein kann.

Der Begriff "gesteigerte Aktivität eines Enzyms" ist vorzugsweise als gesteigerte intrazelluläre Aktivität zu verstehen.
Die nun folgenden Ausführungen zur Erhöhung der Enzymaktivität in Zellen gelten sowohl für die Erhöhung der Aktivität des Enzyms E₁ bis E₃ als auch für alle nachfolgend genannten Enzyme, deren Aktivität gegebenenfalls erhöht werden kann.
Grundsätzlich lässt sich eine Steigerung der enzymatischen Aktivität dadurch erzielen, dass man die Kopiezahl der Gensequenz bzw. der Gensequenzen erhöht, welche für das Enzym kodieren, einen starken Promotor oder eine verbesserte Ribosomenbindungsstelle verwendet, eine negative Regulation der Genexpression, beispielsweise durch Transkriptionsregulatoren, abschwächt, oder eine positive Regulation der Genexpression, beispielsweise durch Transkriptionsregulatoren, verstärkt, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder eine Kombination dieser Methoden mit einem Vektor erzeugt, der das gewünschte Gen, ein Allel dieses Gens oder Teile davon und gegebenenfalls einen die Expression des Gens ermöglichenden Promotor enthält. Die heterologe Expression wird insbesondere durch Integration des Gens oder der Allele in das Chromosom der Zelle oder einen extrachromosomal replizierenden Vektor erzielt.
Einen Überblick über die Möglichkeiten zur Erhöhung der Enzym-Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.
Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschliessende optische Auswertung mit entsprechender Software zur Konzentrationsbestimmung (Lohaus und Meyer (1989) Biospektrum, 5: 32-39; Lottspeich (1999) Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.
Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-, produkt- oder substratinhibierbar sind.
Wird die Erhöhung der Enzymaktivität durch Erhöhung der Synthese eines Enzyms bewerkstelligt, so erhöht man beispielsweise die Kopiezahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopiezahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, in US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993)), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.
Zur Erhöhung der Expression der jeweiligen Gene werden zum Beispiel episomale Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985) DNA cloning: a practical approach, Vol. I-III, IRL Press Ltd. , Oxford; Rodriguez, R.L. und Denhardt, D. T (eds) (1988) Vectors : a survey of molecular cloning vectors and their uses, 179-204, Butterworth, Stoneham; Goeddel, D. V. (1990) Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York.
Der Plasmidvektor, der das zu amplifizierende Gen enthält, wird anschließend durch Konjugation oder Transformation in den gewünschten Stamm überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Let-ters 123: 343-347 (1994) beschrieben. Nach homologer Rekombination mittels eines "cross-over"-Ereignisses enthält der resultierende Stamm mindestens zwei Kopien des betreffenden Gens.
Unter der vorstehend und in den nachfolgenden Ausführungen verwendeten Formulierung "eine im Vergleich zu ihrem Wildtyp gesteigerte Aktivität eines Enzyms Eₓ" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche "eine gegenüber ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Synthese des Enzyms Eₓ induziert wird.

Änderungen von Aminosäureresten einer gegebenen Polypeptidsequenz, die zu keinen wesentlichen Änderungen der Eigenschaften und Funktion des gegebenen Polypeptides führen, sind dem Fachmann bekannt. So können beispielsweise sogenannte konservierte Aminosäuren gegeneinander ausgetauscht werden; Beispiele für solche geeigneten Aminosäuresubstitutionen sind: Ala gegen Ser; Arg gegen Lys; Asn gegen Gln oder His; Asp gegen Glu; Cys gegen Ser; Gln gegen Asn; Glu gegen Asp; Gly gegen Pro; His gegen Asn oder Gln; Ile gegen Leu oder Val; Leu gegen Met oder Val; Lys gegen Arg oder Gln oder Glu; Met gegen Leu oder Ile; Phe gegen Met oder Leu oder Tyr; Ser gegen Thr; Thr gegen Ser; Trp gegen Tyr; Tyr gegen Trp oder Phe; Val gegen Ile oder Leu. Ebenso ist bekannt, dass Änderungen besonders am N- oder C-Terminus eines Polypeptides in Form von beispielsweise Aminosäureinsertionen oder -deletionen oft keinen wesentlichen Einfluss auf die Funktion des Polypeptides ausüben.

Die Aktivität eines Enzyms kann bestimmt werden, indem Zellen, welche diese Aktivität enthalten, auf dem Fachmann bekannte Art und Weise aufgeschlossen werden, beispielsweise mit Hilfe einer Kugelmühle, einer French Press oder eines Ultraschall-Desintegrators und anschließend intakte Zellen, Zellbruchstücke und Aufschluss-Hilfsmittel, wie etwa Glaskugeln durch 10 Minuten Zentrifugation bei 13.000 rpm und 4°C abgetrennt werden. Mit dem resultierenden zellfreien Rohextrakt können dann Enzymassays mit anschließender LC-ESI-MS Detektion der Produkte durchgeführt werden. Alternativ kann das Enzym in dem Fachmann bekannter Art und Weise durch chromatographische Methoden (wie Nickel-Nitrilotriessigsäure-Affinitäts-Chromatographie, Streptavidin-Affinitäts-Chromatographie, Gelfiltrations-Chromatographie oder lonenaustausch-Chromatographie) angereichert oder auch bis zu Homogenität gereinigt werden.

Die Aktivität des Enzyms E₁ wird dann mit den wie oben beschrieben gewonnenen Proben folgendermaßen bestimmt: Ein Standardassay enthält 100 µM *E. coli* ACP, 1 mM β-Mercaptoethanol, 200 µM Malonyl-Coenzym A, 40 µM Octanoyl-Coenzym A (für E₁ₐ) oder Dodekanoyl-Coenzym A (für E_{1b}), 100 µM NADPH, 2 µg *E. coli* FabD, 2 µg *Mycobacterium tuberculosis* FabH, 1 µg *E. coli* FabG, 0,1 M Natriumphosphat-Puffer, pH 7,0, and 5 µg Enzym E₁ in einem finalen Volumen von 120 µL. ACP, β-Mercaptoethanol und Natriumphosphat-Puffer werden 30 min bei 37°C vorinkubiert, um das ACP vollständig zu reduzieren. Die Reaktion wird gestartet durch Zugabe von Enzym E₁. Die Reaktionen werden abgestoppt mit 2 ml Wasser, welches mit HCl auf pH 2,0,angesäuert worden ist und anschließend zweimal mit 2 ml Chloroform/Methanol (2:1 (v:v)) extrahiert. Es erfolgt Phasentrennung durch Zentrifugation (16.100 g, 5 min, RT). Die untere organische Phase wird abgenommen, in der Vakuumzentrifuge vollständig verdampft und das Sediment in 50 µl Methanol aufgenommen. Ungelöste Bestandteile werden durch Zentrifugation (16.100 *g*, 5 min, RT) sedimentiert und die Probe mittels LC-ESI-MS analysiert. Die Identifizierung der Produkte erfolgt durch Analyse der entsprechenden Massenspuren und der MS²-Spektren.

Die Aktivität des Enzyms E₂ wird dann mit den wie oben beschrieben gewonnenen Proben folgendermaßen bestimmt: ein Standardassay kann aus 185 µl 10 mM Tris-HCl (pH 7,5), 10 µl 125 mM dTDP-Rhamnose und 50 µl Proteinrohextrakt (ca. 1 mg Gesamtprotein) oder gereinigtem Protein in Lösung (5 µg gereinigtes Protein) bestehen. Die Reaktion wird durch die Zugabe von 10 µl 10 mM ethanolischer Lösung von 3-Hydroxydekanoyl-3-Hydroxydekansäure (für E₂ₐ) oder 3-Hydroxytetradekanoyl-3-Hydroxytetradekansäure (für E_{2b}) gestartet und 1 h bei 30°C unter Schütteln (600 rpm) inkubiert. Anschließend wird die Reaktion mit 1 ml Aceton versetzt. Ungelöste Bestandteile werden durch Zentrifugation (16.100 g, 5 min, RT) sedimentiert und die Probe mittels LC-ESI-MS analysiert. Die Identifizierung der Produkte erfolgt durch Analyse der entsprechenden Massenspuren und der MS²-Spektren.

Die Aktivität des Enzyms E₃ wird dann mit den wie oben beschrieben gewonnenen Proben folgendermaßen bestimmt: ein Standardassay kann aus 185 µl 10 mM Tris-HCl (pH 7,5), 10 µl 125 mM dTDP-Rhamnose und 50 µl Proteinrohextrakt (ca. 1 mg Gesamtprotein) oder gereinigtem Protein in Lösung (5 µg gereinigtes Protein) bestehen. Die Reaktion wird durch die Zugabe von 10 µl 10 mM ethanolischer Lösung von α-L-Rhamnopyranosyl-3-Hydroxydekanoyl-3-Hydroxydekansäure (für E₃ₐ) oder α-L-Rhamnopyranosyl-3-Hydroxytetradekanoyl-3-Hydroxytetradekansäure (für E_{3b}) gestartet und 1 h bei 30°C unter Schütteln (600 rpm) inkubiert. Anschließend wird die Reaktion mit 1 ml Aceton versetzt. Ungelöste Bestandteile werden durch Zentrifugation (16.100 *g*, 5 min, RT) sedimentiert und die Probe mittels LC-ESI-MS analysiert. Die Identifizierung der Produkte erfolgt durch Analyse der entsprechenden Massenspuren und der MS²-Spektren.

Es werden erfindungsgemäß Zellen bevorzugt, die gesteigerte Aktivitäten folgender Enzymkombinationen aufweisen:
E₁, E₂, E₃, E₁E₂, E₁E₃, E₂E₃ und E₁E₂E₃,
   wovon die Kombination
E₂, E₂E₃ und E₁E₂E₃, insbesondere E₁E₂E₃
   besonders bevorzugt ist.

In einer bevorzugten Ausgestaltungsform der erfindungsgemäßen Zelle, die eine gesteigerte Aktivität der Enzymkombination E₁E₂E₃ aufweist, ist n bevorzugt =1.

Die erfindungsgemäßen Zellen sind Mikroorganismen wie Hefen, Pilze oder Bakterien, wobei Mikroorganismen besonders bevorzugt und Bakterien und Hefen am meisten bevorzugt sind.
Als Bakterien, Hefen oder Pilze sind insbesondere diejenigen Bakterien, Hefen oder Pilze geeignet, die bei der Deutschen Sammlung von Mikroorganismen und Zellkulturen GmbH (DSMZ), Braunschweig, Deutschland, als Bakterien-, Hefe- oder Pilz-Stämme hinterlegt sind. Erfindungsgemäß geeignete Bakterien gehören zu den Gattungen, die unter
http://www.dsmz.de/species/bacteria.htm
aufgeführt sind, erfindungsgemäß geeignete Hefen gehören zu denjenigen Gattungen, die unter
http://www.dsmz.de/species/yeasts.htm
aufgeführt sind und erfindungsgemäß geeignete Pilze sind diejenigen, die unter
http://www.dsmz.de/species/fungi.htm
aufgeführt sind.

Erfindungsgemäß bevorzugte Zellen sind diejenigen der Gattungen *Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium* und *Cupriavidus,* wobei *Aspergillus nidulans, Aspergillus niger, Alcaligenes latus, Bacillus megaterium, Bacillus subtilis, Brevibacterium flavum, Brevibacterium lactofermentum, Burkholderia andropogonis, B. brasilensis, B. caledonica, B. caribensis, B. caryophylli, B. fungorum, B. gladioli, B. glathei, B. glumae, B. graminis, B. hospita, B. kururiensis, B. phenazinium, B. phymatum, B. phytofirmans, B. plantarii, B. sacchari, B. singaporensis, B. sordidicola, B. terricola, B. tropica, B. tuberum, B. ubonensis, B. unamae, B. xenovorans, B. anthina, B. pyrrocinia, B. thailandensis, Candida blankii, Candida rugosa, Corynebacterium glutamicum, Corynebacterium efficiens, Escherichia coli, Hansenula polymorpha, Kluveromyces lactis, Methylobacterium extorquens, Paracoccus versutus, Pseudomonas argentinensis, P. borbori, P. citronellolis, P. flavescens, P. mendocina, P. nitroreducens, P. oleovorans, P. pseudoalcaligenes, P. resinovorans, P. straminea, P. aurantiaca, P. aureofaciens, P. chlororaphis, P. fragi, P. lundensis, P. taetrolens, P. antarctica, P. azotoformans, 'P. blatchfordae', P. brassicacearum, P. brenneri, P. cedrina, P. corrugata, P. fluorescens, P. gessardii, P. libanensis, P. mandelii, P. marginalis, P. mediterranea, P. meridiana, P. migulae, P. mucidolens, P. orientalis, P. panacis, P. proteolytica, P. rhodesiae, P. synxantha, P. thivervalensis, P. tolaasii, P. veronii, P. denitrificans, P. pertucinogena, P. cremoricolorata, P. fulva, P. monteilii, P. mosselii, P. parafulva, P. putida, P. balearica, P. stutzeri, P. amygdali, P. avellanae, P. caricapapayae, P. cichorii, P. coronafaciens, P. ficuserectae, 'P. helianthi', P. meliae, P. savastanoi, P. syringae, P. tomato, P. viridiflava, P. abietaniphila, P. acidophila, P. agarici, P. alcaliphila, P. alkanolytica, P. amyloderamosa, P. asplenii, P. azotifigens, P. cannabina, P. coenobios, P. congelans, P. costantinii, P. cruciviae, P. delhiensis, P. excibis, P. extremorientalis, P. frederiksbergensis, P. fuscovaginae, P. gelidicola, P. grimontii, P. indica, P. jessenii, P. jinjuensis, P. kilonensis, P. knackmussii, P. koreensis, P. lini, P. lutea, P. moraviensis, P. otitidis, P. pachastrellae, P. palleroniana, P. papaveris, P. peli, P. perolens, P. poae, P. pohangensis, P. psychrophila, P. psychrotolerans, P. rathonis, P. reptilivora, P. resiniphila, P. rhizosphaerae, P. rubescens, P. salomonii, P. segitis, P. septica, P. simiae, P. suis, P. thermotolerans, P. aeruginosa, P. tremae, P. trivialis, P. turbinellae, P. tuticorinensis, P. umsongensis, P. vancouverensis, P. vranovensis, P. xanthomarina, Ralstonia eutropha, Rhodospirillum rubrum, Rhodobacter sphaeroides, Saccharomyces cerevisiae, Yarrowia lipolytica* und *Zymomonas mobilis,*
insbesondere *Pseudomonas putida, Escherichia coli* und *Burkholderia thailandensis* besonders bevorzugt sind.

Erfindungsgemäß bevorzugte Zellen vermögen als Wildtyp keine oder keine nachweisbaren Mengen an Rhamnolipiden zu bilden und weisen darüber hinaus als Wildtyp bevorzugt keine oder keine nachweisbare Aktivität der Enzyme E₁,E₂ und E₃ auf.

Es ist erfindungsgemäß vorteilhaft, wenn die erfindungsgemäße Zelle eine Zelle ist, welche als Wildtyp Polyhydroxyalkanoate mit Kettenlängen des Mono-Alkanoates von C₆ bis C₁₆ zu bilden vermag. Solche Zellen sind beispielsweise *Burkholderia* sp., *Burkholderia thailandensis, Pseudomonas* sp., *Pseudomonas putida, Pseudomonas aeruginosa, Pseudomonas oleovorans, Pseudomonas stutzeri, Pseudomonas fluorescens, Pseudomonas citronellolis, Pseudomonas resinovorans, Comamonas testosteroni, Aeromonas hydrophila, Cupriavidus necator, Alcaligenes latus* und *Ralstonia eutropha.* In diesem Zusammenhang sind bevorzugte erfindungsgemäße Zellen derart gentechnisch verändert, dass sie verglichen zu ihrem Wildtyp weniger Polyhydroxyalkanoate zu bilden vermögen.
Solche Zellen sind beispielsweise beschrieben in De Eugenio et al., Environ Microbiol. 2010. 12(1):207-21 und Rehm et al., Appl Environ Microbiol. 2001. 67(7):3102-9. Solch eine, verglichen zu ihrem Wildtyp weniger Polyhydroxyalkanoate zu bilden vermögende Zelle ist insbesondere dadurch gekennzeichnet, dass sie verglichen zu ihrem Wildtyp eine verminderte Aktivität mindestens eines Enzymes E₉ oder E₁₀ aufweist,
wobei E₉ eine Polyhydroxyalkanoat-Synthase, EC:2.3.1.-, insbesondere mit Polypeptidsequenz Seq ID Nr. 30 oder Seq ID Nr. 32 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der jeweiligen Bezugssequenz Seq ID Nr. 30 oder Seq ID Nr. 32 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der jeweiligen Bezugssequenz Seq ID Nr. 30 oder Seq ID Nr. 32 besitzt, darstellt, wobei unter enzymatischer Aktivität für ein Enzym E₉ die Fähigkeit verstanden wird, 3-Hydroxyalkanoyl-Coenzym A zu Poly-3-Hydroxyalkansäure, insbesondere 3-Hydroxytetradekanoyl-Coenzym A zu Poly-3-Hydroxytetradekansäure, umzusetzen, und
E₁₀ eine 3-Hydroxyalkanoyl-ACP:Coenzym A-Transferase, insbesondere mit Polypeptidsequenz Seq ID Nr. 34 oder Seq ID Nr. 36 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der jeweiligen Bezugssequenz Seq ID Nr. 34 oder Seq ID Nr. 36 durch Deletion, Insertion, Substitution oder eine Kombination davon verändert sind und die noch mindestens 10%, bevorzugt 50%, besonders bevorzugt 80%, insbesondere mehr als 90% der enzymatischen Aktivität des Enzyms mit der jeweiligen Bezugssequenz Seq ID Nr. 34 oder Seq ID Nr. 36 besitzt, darstellt, wobei unter enzymatischer Aktivität für ein Enzym E₁₀ die Fähigkeit verstanden wird, 3-Hydroxyalkanoyl-ACP zu 3-Hydroxyalkananoyl-Coenzym A, insbesondere 3-Hydroxyalkananoyl-ACP zu 3-Hydroxytetradekanoyl-Coenzym A, umzusetzen.

Zur Übersicht vergleiche Figur 1.

Die Aktivität des Enzyms E₉ wird dann mit den wie oben für die Enzyme E₁ bis E₃ beschrieben gewonnenen Proben bestimmt, indem zunächst 560 µl 100 mM Tris/HCl, pH 7,5, 20 µl 35 mM DTNB in DMSO und 20 µl 41 mM 3-Hydroxydekanoyl-Coenzym A gemischt werden. Anschließend werden 5 µg gereinigtes Enzym E₉ in 100 µl Tris/HCl, pH 7,5, zugesetzt und anschließend für 1 min kontinuierlich im Spektrophotometer die Zunahme der Extinktion bei 412 nm (verursacht durch Anlagerung von 5,5'-Dithiobis(2-Nitrobenzoat) (DTNB) an freie SH-Gruppen) über die Zeit aufgenommen (ΔE/min).

Die Aktivität des Enzyms E₁₀ wird dann mit den wie oben für die Enzyme E₁ bis E₃ beschrieben gewonnenen Proben bestimmt. Der Standard-Assay enthält 3 mM MgCl₂, 40 µM Hydroxydekanoyl-Coenzym A und 20 µM E. coli ACP in 50 mM Tris-HCl, pH 7,5, in einem Gesamtvolumen von 200 µl. Die Reaktion wird gestartet durch Zugabe von 5 µg gereinigtem Enzym E₁₀ in 50 µl Tris/HCl, pH 7,5 und für 1 h bei 30°C inkubiert. Die Reaktion wird abgestoppt durch Zugabe von 50% (w/v) Trichloressigsäure und 10 mg/ml BSA (30 µl). Freigesetztes Coenzym A wird spektrophotometrisch bestimmt, indem die Zunahme der Extinktion bei 412 nm, verursacht durch Anlagerung von 5,5'-Dithiobis(2-Nitrobenzoat) (DTNB) an freie SH-Gruppen, über die Zeit aufgenommen wird.

Unter der verwendeten Formulierung "verminderte Aktivität eines Enzyms Eₓ" wird dementsprechend vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verminderte Aktivität verstanden. Die Formulierung "verminderte Aktivität" beinhaltet auch keine detektierbare Aktivität ("Aktivität von null"). Die Verminderung der Aktivität eines bestimmten Enzyms kann beispielsweise durch gezielte Mutation oder durch andere, dem Fachmann bekannte Maßnahmen zur Verminderung der Aktivität eines bestimmten Enzyms erfolgen. Verfahren zur Verminderung von enzymatischen Aktivitäten in Mikroorganismen sind dem Fachmann bekannt.
Insbesondere molekularbiologische Techniken bieten sich hier an. Anleitung zur Modifikation und Verminderung von Proteinexpressionen und damit einhergehender Enzymaktivitätsverringerung speziell für *Pseudomonas* und *Burkholderia,* insbesondere zum Unterbrechen spezieller Gene findet der Fachmann beispielsweise in Dubeau et al. 2009. BMC Microbiology 9:263; Singh & Röhm. Microbiology. 2008. 154:797-809 oder Lee et al. FEMS Microbiol Lett. 2009. 297(1):38-48.
Erfindungsgemäß bevorzugte Zellen sind dadurch gekennzeichnet, dass die Verminderung der enzymatischen Aktivität erreicht wird durch Modifikation eines Genes umfassend eine der genannten Nukleinsäuresequenzen, wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz, RNA-Interferenz (siRNA), *antisense*-RNA oder Modifikation (Insertion, Deletion oder Punktmutationen) von regulatorischen Sequenzen, wie etwa Promotoren und Terminatoren oder von Ribosomenbindestellen, welche das Gen flankieren.
Unter Fremd-DNA ist in diesem Zusammenhang jegliche DNA-Sequenz zu verstehen, die dem Gen (und nicht dem Organismus) "fremd" ist, d.h. auch endogene DNA-Sequenzen können in diesem Zusammenhang als "Fremd-DNA" fungieren.
In diesem Zusammenhang ist es insbesondere bevorzugt, dass das Gen durch Insertion eines Selektionsmarkergens unterbrochen wird, somit die Fremd-DNA ein Selektionsmarkergen ist, wobei bevorzugt die Insertion durch homologe Rekombination in den Genlocus erfolgte.

In einer bevorzugten Ausführungsform der erfindungsgemäßen Zelle handelt es sich bei der Zelle um *Pseudomonas putida* Zellen, welche eine verminderte Polyhydroxyalkanoat-Synthese verglichen zu ihrem Wildtyp aufweisen. Solche Zellen werden beispielsweise in Ren et al., Journal Applied Microbiology and Biotechnology 1998 Jun, 49(6):743-50 als GPp121, GPp122, GPp123 und GPp124, in Huisman et al., J Biol Chem. 1991 Feb 5;266(4):2191-8 als GPp104 sowie in De Eugenio et al., Environ Microbiol. 2010. 12(1):207-21 als KT42C1 und in Ouyang et al. Macromol Biosci. 2007. 7(2):227-33 als KTOY01 und KTOY02 beschrieben.

Für den Fall, dass die erfindungsgemäße Zelle ein Rhamnolipid mit m=1 zu bilden vermag, ist es bevorzugt, dass sich der über R¹ und R² bestimmte Rest 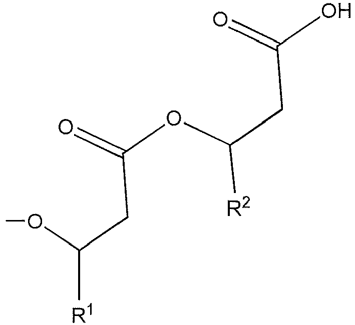 ableitet von 3-Hydroxyoctanoyl-3-Hydroxyoctansäure, 3-Hydroxyoctanoyl-3-Hydroxydekansäure, 3-Hydroxydekanoyl-3-Hydroxyoctansäure, 3-Hydroxyoctanoyl-3-Hydroxydecensäure, 3-Hydroxydecenoyl-3-Hydroxyoctansäure, 3-Hydroxyoctanoyl-3-Hydroxydodekansäure, 3-Hydroxydodekanoyl-3-Hydroxyoctansäure, 3-Hydroxyoctanoyl-3-Hydroxydodecensäure, 3-Hydroxydodecenoyl-3-Hydroxyoctansäure, 3-Hydroxydekanoyl-3-Hydroxydekansäure, 3-Hydroxydekanoyl-3-Hydroxydecensäure, 3-Hydroxydecenoyl-3-Hydroxydekansäure, 3-Hydroxydecenoyl-3-Hydroxydecensäure, 3-Hydroxydekanoyl-3-Hydroxydodekansäure, 3-Hydroxydodekanoyl-3-Hydroxydekansäure, 3-Hydroxydekanoyl-3-Hydroxydodecensäure, 3-Hydroxydekanoyl-3-Hydroxytetradecensäure, 3-Hydroxytetradekanoyl-3-Hydroxydecensäure, 3-Hydroxydodecenoyl-3-Hydroxydekansäure, 3-Hydroxydekanoyl-3-Hydroxytetradekansäure, 3-Hydroxytetradekanoyl-3-Hydroxydekansäure, 3-Hydroxydekanoyl-3-Hydroxytetradecensäure, 3-Hydroxytetradecenoyl-3-Hydroxydekansäure, 3-Hydroxydodekanoyl-3-Hydroxydodekansäure, 3-Hydroxydodecenoyl-3-Hydroxydodekansäure, 3-Hydroxydodekanoyl-3-Hydroxydodecensäure, 3-Hydroxydodekanoyl-3-Hydroxytetradekansäure, 3-Hydroxytetradekanoyl-3-Hydroxydodekansäure, 3-Hydroxytetradekanoyl-3-Hydroxytetradekansäure, 3-Hydroxyhexadekanoyl-3-Hydroxytetradekansäure, 3-Hydroxytetradekanoyl-3-Hydroxyhexadekansäure oder 3-Hydroxyhexadekanoyl-3-Hydroxyhexadekansäure.

Es ist dem Fachmann ersichtlich, dass eine erfindungsgemäße Zelle auch Mischungen verschiedener Rhamnolipide der allgemeinen Formel (I) zu bilden vermag.
In diesem Zusammenhang ist es bevorzugt, dass die erfindungsgemäßen Zellen Mischungen von Rhamnolipiden der allgemeinen Formel (I) zu bilden vermögen, die dadurch gekennzeichnet sind, dass in mehr als 80 Gew.-%, bevorzugt mehr als 90 Gew.-%, besonders bevorzugt mehr als 95 Gew.-% der gebildeten Rhamnolipide n=1 ist und sich der über R¹ und R² bestimmte Rest bei weniger als 10 Gew.-%, bevorzugt weniger als 5 Gew.-%, besonders bevorzugt weniger als 2 Gew.-% der gebildeten Rhamnolipide von 3-Hydroxydekanoyl-3-Hydroxyoctansäure oder 3-Hydroxyoctanoyl-3-Hydroxydekansäure ableitet,
wobei sich die angegebenen Gew.-% auf die Summe aller gebildeten Rhamnolipide der allgemeinen Formel (I) bezieht.

Die Aktivität des Enzyms E₄ wird mit den wie oben für die Enzyme E₁ bis E₃ beschrieben gewonnenen Proben bestimmt, indem α-D-Glukose-1-Phosphat (1,3 mM) mit dTTP (5 mM) und 5 µg gereinigtem Enzym E₄ in 50 µl Natriumphosphat-Puffer, pH 8,5, inkubiert und nach 5, 10 und 20 min Inkubation bei 30°C die Reaktion durch Zugabe von 20 µl Chloroform abgestoppt wird. Die Mixtur wird dann gevortexed und 5 min bei 16.000 *g* und Raumtemperatur zentrifugiert. Die wässrige Phase wird in ein neues Reaktionsgefäß überführt und die organische Phase erneut mit 80 µl Wasser extrahiert. Beide wässrige Phasen werden vereinigt und mittels HPLC analysiert. Dabei kommt eine Phenosphere-ODS2-Säule (250 x 4,6 mm; Phenomenex, Torrance, USA) oder eine Spheresorb-ODS2-Säule (250 x 4,6 mm; Waters, Milford, USA) zum Einsatz. Die Elution der Analyten erfolgt mit einer Flussrate von 1 ml min⁻¹ mit 0,5 M KH₂PO₄ (Eluent A) für 15 min, gefolgt von einem linearen Gradienten bis zu 80% Eluent A und 20% Methanol über einen Zeitraum von 14 min bei einer Flußrate von 0,7 ml min⁻¹. Analyten, welche von den ODS2-Säulen eluieren, werden dann in eine Phenosphere-SAX Ionenaustauscher-Säule (250 x 4,6 mm; Phenomenex, Torrance, USA) injiziert und die Analyten mit einer Flußrate von 1 ml min⁻¹ und einem linearen Ammoniumformiat-Gradienten (2 bis 600 mM über 25 min) eluiert. Die Quantifizierung von dTDP-Glukose erfolgt dann über ihre UV-Absorption mit einem Photodiodenarray-Detektor (DAD). Das Absorptionsmaximum von Thymidin liegt bei 267 nm. Die Kalibration erfolgt mittels authentischer Nukleotidzucker (Sigma-Aldrich, München, USA).

Die Aktivität des Enzyms E₅ wird dann mit den wie oben für die Enzyme E₁ bis E₃ beschrieben gewonnenen Proben bestimmt, indem dTDP-α-D-Glukose (1,3 mM) mit 5 µg gereinigtem Enzym E₅ in 50 µl Natriumphosphat-Puffer, pH 8,5, inkubiert und nach 5, 10 und 20 min Inkubation bei 30°C die Reaktion durch Zugabe von 20 µl Chloroform abgestoppt wird. Die Mixtur wird dann gevortexed und 5 min bei 16.000 *g* und Raumtemperatur zentrifugiert. Die wässrige Phase wird in ein neues Reaktionsgefäß überführt und die organische Phase erneut mit 80 µl Wasser extrahiert. Beide wässrige Phasen werden vereinigt und mittels HPLC analysiert. Dabei kommt eine Phenosphere-ODS2-Säule (250 x 4,6 mm; Phenomenex, Torrance, USA) oder eine Spheresorb-ODS2-Säule (250 x 4,6 mm; Waters, Milford, USA) zum Einsatz. Die Elution der Analyten erfolgt mit einer Flußrate von 1 ml min⁻¹ mit 0,5 M KH₂PO₄ (Eluent A) für 15 min, gefolgt von einem linearen Gradienten bis zu 80% Eluent A und 20% Methanol über einen Zeitraum von 14 min bei einer Flußrate von 0,7 ml min⁻¹. Analyten, welche von den ODS2-Säulen eluieren, werden dann in eine Phenosphere-SAX Ionenaustauscher-Säule (250 x 4,6 mm; Phenomenex, Torrance, USA) injiziert und die Analyten mit einer Flußrate von 1 ml min⁻¹ und einem linearen Ammoniumformiat-Gradienten (2 bis 600 mM über 25 min) eluiert. Die Quantifizierung von dTDP-Glukose und dTDP-4-Dehydro-6-Deoxy-D-Glukose erfolgt dann über ihre UV-Absorption mit einem Photodiodenarray-Detektor (DAD). Das Absorptionsmaximum von Thymidin liegt bei 267 nm. Die Kalibration erfolgt mittels authentischer Nukleotidzucker (Sigma-Aldrich, München, USA).

Die Aktivität des Enzyms E₆ wird dann mit den wie oben für die Enzyme E₁ bis E₃ beschrieben gewonnenen Proben bestimmt, indem zunächst dTDP-α-D- Glukose (1,3 mM) mit 5 µg gereinigtem Enzym E₅ in 50 µl Natriumphosphat-Puffer, pH 8,5, für 10 min bei 30°C inkubiert werden. Anschließend werden 0,5 µg gereinigtes Enzym E₆ zugesetzt, und nach 5, 10 und 20 min Inkubation bei 30°C die Reaktion durch Zugabe von 20 µl Chloroform abgestoppt. Die Mixtur wird dann gevortexed und 5 min bei 16.000 *g* und Raumtemperatur zentrifugiert. Die wässrige Phase wird in ein neues Reaktionsgefäß überführt und die organische Phase erneut mit 80 µl Wasser extrahiert. Beide wässrige Phasen werden vereinigt und mittels HPLC analysiert. Dabei kommt eine Phenosphere-ODS2-Säule (250 x 4,6 mm; Phenomenex, Torrance, USA) oder eine Spheresorb-ODS2-Säule (250 x 4,6 mm; Waters, Milford, USA) zum Einsatz. Die Elution der Analyten erfolgt mit einer Flußrate von 1 ml min⁻¹ mit 0,5 M KH₂PO₄ (Eluent A) für 15 min, gefolgt von einem linearen Gradienten bis zu 80% Eluent A und 20% Methanol über einen Zeitraum von 14 min bei einer Flußrate von 0,7 ml min⁻¹. Analyten, welche von den ODS2-Säulen eluieren, werden dann in eine Phenosphere-SAX Ionenaustauscher-Säule (250 x 4,6 mm; Phenomenex, Torrance, USA) injiziert und die Analyten mit einer Flußrate von 1 ml min⁻¹ und einem linearen Ammoniumformiat-Gradienten (2 bis 600 mM über 25 min) eluiert. Die Quantifizierung von dTDP-Glukose, dTDP-4-Dehydro-6-Deoxy-D-Glukose und dTDP-6-Deoxy-L-Mannose erfolgt dann über ihre UV-Absorption mit einem Photodiodenarray-Detektor (DAD). Das Absorptionsmaximum von Thymidin liegt bei 267 nm. Die Kalibration erfolgt mittels authentischer Nukleotidzucker (Sigma-Aldrich, München, USA).

Die Aktivität des Enzyms E₇ wird dann mit den wie oben für die Enzyme E₁ bis E₃ beschrieben gewonnenen Proben bestimmt, indem zunächst dTDP-α-D-Glukose (1,3 mM) mit 5 µg gereinigtem Enzym E₅ in 50 µl Natriumphosphat-Puffer, pH 8,5, für 10 min bei 30°C inkubiert werden. Anschließend werden 5 µg gereinigtes Enzym E₆ und 0,5 µg gereinigtes Enzym E₇ sowie NADPH (10 mM) zugesetzt, und nach 5, 10 und 20 min Inkubation bei 30°C die Reaktion durch Zugabe von 20 µl Chloroform abgestoppt. Die Mixtur wird dann gevortexed und 5 min bei 16.000 *g* und Raumtemperatur zentrifugiert. Die wässrige Phase wird in ein neues Reaktionsgefäß überführt und die organische Phase erneut mit 80 µl Wasser extrahiert. Beide wässrige Phasen werden vereinigt und mittels HPLC analysiert. Dabei kommt eine Phenosphere-ODS2-Säule (250 x 4,6 mm; Phenomenex, Torrance, USA) oder eine Spheresorb-ODS2-Säule (250 x 4,6 mm; Waters, Milford, USA) zum Einsatz. Die Elution der Analyten erfolgt mit einer Flußrate von 1 ml min⁻¹ mit 0,5 M KH₂PO₄ (Eluent A) für 15 min, gefolgt von einem linearen Gradienten bis zu 80% Eluent A and 20% Methanol über einen Zeitraum von 14 min bei einer Flußrate von 0,7 ml min⁻¹. Analyten, welche von den ODS2-Säulen eluieren, werden dann in eine Phenosphere-SAX Ionenaustauscher-Säule (250 x 4,6 mm; Phenomenex, Torrance, USA) injiziert und die Analyten mit einer Flußrate von 1 ml min⁻¹ und einem linearen Ammoniumformiat-Gradienten (2 bis 600 mM über 25 min) eluiert. Die Quantifizierung von dTDP-Glukose, dTDP-4-Dehydro-6-Deoxy-D-Glukose, dTDP-6-Deoxy-L-Mannose und dTDP-4-Dehydro-6-Deoxy-L-Mannose erfolgt dann über ihre UV-Absorption mit einem Photodiodenarray-Detektor (DAD). Das Absorptionsmaximum von Thymidin liegt bei 267 nm. Die Kalibration erfolgt mittels authentischer Nukleotidzucker (Sigma-Aldrich, München, USA).

Es werden erfindungsgemäß Zellen bevorzugt, die gesteigerte Aktivitäten folgender Enzymkombinationen aufweisen:
E₄E₅, E₄E₆, E₄E₇, E₅E₆, E₅E₇, E₆E₇, E₄E₅E₆, E₄E₅E₇, E₅E₆E₇, E₄E₆E₇, E₄E₅E₆E₇,
   wovon die Kombination
E₄E₅E₆E₇
   besonders bevorzugt ist.

Es kann erfindungsgemäß vorteilhaft sein, wenn die erfindungsgemäße Zelle in der Fettsäure-Biosynthese derart gentechnisch verändert wurde, dass die enzymatischen Reaktionen, die zur Umsetzung von Acyl-ACP und Malonyl-Coenzym A zu 3-Ketoacyl-ACP und/oder zur Umsetzung von 3-Ketoacyl-ACP zu (*R*)-3-Hydroxyalkanoyl-ACP führen, verstärkt werden. Zusätzlich oder alternativ kann es erfindungsgemäß vorteilhaft sein, wenn die erfindungsgemäße Zelle in der Fettsäure-Biosynthese derart gentechnisch verändert wurde, dass die enzymatischen Reaktionen, die zur Umsetzung von (*R*)-3-Hydroxyalkanoyl-ACP zu *trans*-2-Enoyl-ACP und/oder zur Umsetzung von *trans*-2-Enoyl-ACP zu Acyl-ACP führen, abgeschwächt werden.
Genauso vorteilhaft kann es sein, wenn die erfindungsgemäße Zelle in der β-Oxidation von Fettsäuren derart gentechnisch verändert wurde, dass die enzymatischen Reaktionen, die zur Umsetzung von Acyl-Coenzym A zu *trans*-2-Enoyl-Coenzym A und/oder zur Umsetzung von *trans*-2-Enoyl-Coenzym A zu (*S*)-3-Hydroxyalkanoyl-Coenzym A führen, verstärkt werden. Zusätzlich oder alternativ kann es erfindungsgemäß vorteilhaft sein, wenn die erfindungsgemäße Zelle in der β-Oxidation von Fettsäuren derart gentechnisch verändert wurde, dass die enzymatischen Reaktionen, die zur Umsetzung von (*S*)-3-Hydroxyalkanoyl-Coenzym A zu 3-Ketoacyl-Coenzym A und/oder zur Umsetzung von 3-Ketoacyl-Coenzym A zu Acyl-Coenzym A und Acetyl-Coenzym A führen, abgeschwächt werden.
Zur Übersicht vergleiche Figur 1.

Da sich die erfindungsgemäße Zellen vorteilhaft zur Herstellung von Rhamnolipiden verwenden lassen und da diese Lipide anschließend gegebenenfalls aufgereinigt werden, ist es vorteilhaft, wenn die erfindungsgemäßen Zellen eine gegenüber ihrem Wildtyp erhöhte Aktivität mindestens eines Enzyms E₈ aufweisen, welches den Export eines Rhamnolipids der allgemeinen Formel (I) aus der Zelle in das umgebende Medium katalysiert.

Bevorzugt werden in diesem Zusammenhang Proteine E₈ ausgewählt aus der Gruppe bestehend aus
ein Enzym E₈ mit Polypeptidsequenz Seq ID Nr. 8, Seq ID Nr. 24, Seq ID Nr. 26 oder Seq ID Nr. 28 oder mit einer Polypeptidsequenz bei der bis zu 25%, bevorzugt bis zu 20%, besonders bevorzugt bis zu 15% insbesondere bis zu 10, 9, 8, 7, 6, 5, 4, 3, 2, 1% der Aminosäurereste gegenüber der jeweiligen Bezugssequenz Seq ID Nr. 8, Seq ID Nr. 24, Seq ID Nr. 26 oder Seq ID Nr. 28 durch Deletion, Insertion, Substitution oder eine Kombination davon verändert sind und die noch mindestens 50%, bevorzugt 65%, besonders bevorzugt 80%, insbesondere mehr als 90% der enzymatischen Aktivität des Enzyms mit der jeweiligen Bezugssequenz Seq ID Nr. 8, Seq ID Nr. 24, Seq ID Nr. 26 oder Seq ID Nr. 28 besitzt, wobei unter enzymatischer Aktivität für ein Enzym E₈ die Fähigkeit verstanden wird, ein Rhamnolipid der allgemeinen Formel (I) aus der Zelle in das umgebende Medium zu exportieren.
Eine weitere, bevorzugte Ausführungsform erfindungsgemäßer Zellen ist dadurch gekennzeichnet, dass sie mindestens eine der unten genannten erfindungsgemäßen Nukleinsäuren oder Vektoren aufweist.

Erfindungsgemäße Zellen lassen sich vorteilhaft zur Herstellung von Rhamnolipiden verwenden.
Somit wird die Verwendung erfindungsgemäßer Zellen zur Herstellung von Verbindungen der allgemeinen Formel (I) offenbart.

Ein weiterer Gegenstand der vorliegenden Erfindung ist ein Verfahren zur Herstellung von Rhamnolipiden der allgemeinen Formel (I),
wobei
m = 2, 1 oder 0, insbesondere 1 oder 0,
n = 1 oder 0, insbesondere 1,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl-, Heptenyl, Nonenyl, Undekenyl und Tridekenyl und (CH₂)ₒ-CH₃ mit o = 1 bis 23, bevorzugt 4 bis 12, umfassend die Verfahrensschritte
I) in Kontakt Bringen der erfindungsgemäßen Zelle mit einem Medium beinhaltend eine Kohlenstoffquelle
II) Kultivieren der Zelle unter Bedingungen, die es der Zelle ermöglichen, aus der Kohlenstoffquelle Rhamnolipid zu bilden und
III) gegebenenfalls Isolierung der gebildeten Rhamnolipide.

Die erfindungsgemäßen, gentechnisch veränderten Zellen können kontinuierlich oder diskontinuierlich im batch-Verfahren (Satzkultivierung) oder im fed-batch-Verfahren (Zulaufverfahren) oder repeated-fed-batch-Verfahren (repetitives Zulaufverfahren) zum Zwecke der Produktion der vorstehend genannten Produkte mit dem Nährmedium in Kontakt gebracht und somit kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.
Das zu verwendende Kulturmedium muss in geeigneter Weise den Ansprüchen der jeweiligen Stämme genügen. Beschreibungen von Kulturmedien verschiedener Hefestämme sind beispielsweise in "Nonconventional yeast in biotechnology" (Hrsg. Klaus Wolf, Springer-Verlag Berlin, 1996) enthalten.
Als Kohlenstoffquelle können Kohlehydrate wie z. B. Glukose, Saccharose, Arabinose, Xylose, Laktose, Fructose, Maltose, Melasse, Stärke, Cellulose und Hemicellulose, pflanzliche und tierische Öle und Fette wie z. B. Sojaöl, Distelöl, Erdnussöl, Hanföl, Jatrophaöl, Kokosnussfett, Kürbiskernöl, Leinöl, Maisöl, Mohnöl, Nachtkerzenöl, Olivenöl, Palmkernöl, Palmöl, Rapsöl, Sesamöl, Sonnenblumenöl, Traubenkernöl, Walnussöl, Weizenkeimöl und Kokosfett, Fettsäuren, wie z. B. Caprylsäure, Caprinsäure, Laurinsäure, Myristinsäure, Palmitinsäure, Palmitolensäure, Stearinsäure, Arachidonsäure, Behensäure, Ölsäure, Linolsäure, Linolensäure, Gamma-Linolensäure und deren Methyl- oder Ethylester sowie Fettsäuregemische, Mono-, Di- und Triglyceride mit den gerade erwähnten Fettsäuren, Alkohole wie z. B. Glyzerin, Ethanol und Methanol, Kohlenwasserstoffe wie Methan, kohlenstoffhaltige Gase und Gasgemische, wie CO, CO₂, Synthese- oder Rauchgas, Aminosäuren wie L-Glutamat oder L-Valin oder organische Säuren wie z. B. Essigsäure verwendet werden. Diese Stoffe können einzeln oder als Mischung verwendet werden. Besonders bevorzugt ist der Einsatz von Kohlehydraten, insbesondere von Monosacchariden, Oligosacchariden oder Polysacchariden, als Kohlenstoffquelle wie dies in US 6,01,494 und US 6,136,576 beschrieben ist sowie von Kohlenwasserstoffen, insbesondere von Alkanen, Alkenen und Alkinen sowie den daraus abgeleiteten Monocarbonsäuren und den aus diesen Monocarbonsäuren abgeleiteten Mono-, Di- und Triglyceriden, sowie von Glyzerin und Acetat. Ganz besonders bevorzugt sind Mono-, Di- und Triglyceride, enthaltend die Veresterungsprodukte von Glyzerin mit Caprylsäure, Caprinsäure, Laurinsäure, Myristinsäure, Palmitinsäure, Palmitolensäure, Stearinsäure, Arachidonsäure, Behensäure, Ölsäure, Linolsäure, Linolensäure und/oder Gamma-Linolensäure.
Ein großer Vorteil der vorliegenden Erfindung ist es, dass die erfindungsgemäßen Zellen Rhamnolipide von einfachsten Kohlenstoffquellen wie beispielsweise Glukose, Saccharose oder Glyzerin zu bilden vermögen, so dass eine Bereitstellung von längerkettigen C-Quellen im Medium während des erfindungsgemäßen Verfahrens nicht notwendig ist. Somit ist es bei mangelnder Verfügbarkeit vorteilhaft, dass das Medium in Schritt I) des erfindungsgemäßen Verfahren keine oder keine nachweisbaren Mengen an Carbonsäuren mit einer Kettenlänge von größer sechs Kohlenstoffatomen oder von diesen ableitbare Ester oder Glyceride enthält.

Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat, Ammoniak, Ammoniumhydroxid oder Ammoniakwasser verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.
Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder sauerstoffhaltige Gasmischungen wie z. B. Luft in die Kultur eingetragen.
Die Temperatur der Kultur liegt normalerweise bei mehr als 20°C, vorzugsweise bei mehr als 25°C, sie kann auch mehr als 40°C betragen, wobei vorteilhafterweise eine Kultivierungstemperatur von 95°C, besonders bevorzugt 90°C und am meisten bevorzugt 80°C nicht überschritten wird.
Im Schritt III) des erfindungsgemäßen Verfahrens können die durch die Zellen gebildeten Rhamnolipide gegebenenfalls aus den Zellen und/oder dem Nährmedium isoliert werden, wobei zur Isolierung alle dem Fachmann bekannten Methoden zur Isolierung von niedermolekularen Substanzen aus komplexen Zusammensetzungen in Betracht kommen wie beispielsweise Filtration, Extraktion, Adsorption (Chromatographie) oder Kristallisation.
Darüber hinaus enthält die Produktphase Reste an Biomasse und verschiedene Verunreinigungen, wie Öle, Fettsäuren und andere Nährmedienbestandteile. Die Abtrennung der Verunreinigungen erfolgt bevorzugt in einem lösungsmittelfreien Prozess. So kann zum Beispiel die Produktphase mit Wasser verdünnt werden, um die Einstellung des pH-Werts zu erleichtern. Produkt- und wässrige Phase können dann homogenisiert werden, indem die Rhamnolipide durch Senken oder Anheben des pH-Werts durch Säuren oder Laugen in eine wasserlösliche Form überführt werden. Potentiell kann die Solubilisierung der Rhamnolipide in der wässrigen Phase durch Inkubation bei höheren Temperaturen, z.B. bei 60 bis 90 °C, und konstantes Mischen unterstützt werden. Durch anschließendes Anheben oder Senken des pH-Werts durch Laugen oder Säuren können dann die Rhamnolipide wieder in eine wasserunlösliche Form überführt werden, so dass sie leicht von der wässrigen Phase getrennt werden können. Die Produktphase kann dann noch ein- oder mehrmals mit Wasser gewaschen werden, um wasserlösliche Verunreinigungen zu entfernen.
Ölreste können beispielsweise durch Extraktion mittels geeigneter Lösungsmittel vorteilhafterweise mittels organischer Lösungsmittel abgetrennt werden. Bevorzugt wird als Lösungsmittel ein Alkan wie z.B. n-Hexan.
Die Abtrennung des Produkts von der wässrigen Phase kann alternativ zu dem oben beschriebenen lösungsmittelfreien Prozess mit einem geeigneten Lösungsmittel, z.B. einem Ester wie beispielsweise Ethylacetat oder Butylacetat erfolgen. Die genannten Extraktionsschritte können in beliebiger Reihenfolge durchgeführt werden.
Hierbei werden vorzugsweise Lösungsmittel eingesetzt, insbesondere organische Lösungsmittel. Bevorzugt wird als Lösungsmittel n-Pentanol. Zur Entfernung des Lösungsmittels erfolgt beispielsweise eine Destillation. Anschließend kann das lyophilisierte Produkt, beispielsweise mittels chromatographischer Methoden, weiter aufgereinigt werden. Beispielhaft seien an dieser Stelle die Fällung mittels geeigneter Lösungsmittel, die Extraktion mittels geeigneter Lösungsmittel, die Komplexierung, beispielsweise mittels Cyclodextrinen oder Cyclodextrin-Derivaten, die Kristallisation, die Aufreinigung bzw. Isolierung mittels chromatographischer Methoden oder die Überführung der Rhamnolipide in leicht abtrennbare Derivate genannt.

Die mit dem erfindungsgemäßen Verfahren herstellbaren Rhamnolipide werden offenbart, insbesondere auch die oben beschriebenen, mit erfindungsgemäßem Verfahren herstellbaren Rhamnolipid-Mischungen.
Die mit dem erfindungsgemäßen Verfahren herstellbaren Rhamnolipide und Mischungen lassen sich vorteilhaft in Reinigungsmitteln, in kosmetischen oder pharmazeutischen Formulierungen sowie in Pflanzenschutz-Formulierungen einsetzen.
Somit wird die Verwendung der mit dem erfindungsgemäßen Verfahren erhaltenen Rhamnolipide zur Herstellung von kosmetischen, dermatologischen oder pharmazeutischen Formulierungen, von Pflanzenschutz-Formulierungen sowie von Pflege- und Reinigungsmitteln und Tensidkonzentraten offenbart.

Unter dem Begriff "Pflegemittel" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt.
Unter "Pflanzenschutz-Formulierungen" sind solche Formulierungen zu verstehen, die von der Art ihrer Herrichtung nach augenfällig zum Pflanzenschutz verwendet werden; dies ist insbesondere dann der Fall, wenn in der Formulierung mindestens eine Verbindung aus den Klassen der Herbizide, Fungizide, Insektizide, Akarizide, Nematizide, Schutzstoffe gegen Vogelfraß, Pflanzennährstoffe und Bodenstrukturverbesserungsmittel enthalten ist. Die mit dem erfindungsgemäßen Verfahren hergestellten Rhamnolipide können in Pflege- und Reinigungsmitteln für Haushalt, Industrie, insbesondere für harte Oberflächen, Leder oder Textilien verwendet werden.

Einen Beitrag zur Lösung der Aufgabe leistet eine erfindunsgemäße Zelle enthaltend eine isolierte Nukleinsäure, welche mindestens jeweils eine Sequenz ausgewählt aus den drei Gruppen [A1 bis G1], [A2 bis G2] und [A3 bis G3] aufweist,
wobei
die Gruppe [A1 bis G1] aus den folgenden Sequenzen besteht:
A1a) eine Sequenz gemäß Seq ID Nr. 1, wobei diese Sequenz für ein Protein kodiert, welches in der Lage ist,
   3-Hydroxydekanoyl-ACP über 3-Hydroxydekanoyl-3-Hydroxydekanoyl-ACP zu 3-Hydroxydekanoyl-3-Hydroxydekansäure umzusetzen,
B1a) eine intronfreie Sequenz, die von einer Sequenz nach A1a) abgeleitet ist und die das gleiche Protein oder Peptid kodiert wie die Sequenz nach Seq ID Nr. 1,
C1a) eine Sequenz, die ein Protein oder Peptid kodiert, das die Aminosäuresequenz gemäß Seq ID Nr. 2 umfasst, und das bevorzugt in der Lage ist,
   3-Hydroxydekanoyl-ACP über 3-Hydroxydekanoyl-3-Hydroxydekanoyl-ACP zu 3-Hydroxydekanoyl-3-Hydroxydekansäure umzusetzen,
D1a) eine Sequenz, die mit einer Sequenz nach einer der Gruppen A1a) bis C1a), besonders bevorzugt nach Gruppe A1a), zu mindestens 70%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   3-Hydroxydekanoyl-ACP über 3-Hydroxydekanoyl-3-Hydroxydekanoyl-ACP zu 3-Hydroxydekanoyl-3-Hydroxydekansäure umzusetzen,
E1a) eine Sequenz, die mit dem Gegenstrang einer Sequenz nach einer der Gruppen A1a) bis D1a), besonders bevorzugt nach Gruppe A1a), hybridisiert oder unter Berücksichtigung der Degeneration des genetischen Codes hybridisieren würde, wobei diese Sequenz für ein Protein oder Peptid kodiert, welches in der Lage ist,
   3-Hydroxydekanoyl-ACP über 3-Hydroxydekanoyl-3-Hydroxydekanoyl-ACP zu 3-Hydroxydekanoyl-3-Hydroxydekansäure umzusetzen,
F1a) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A1a) bis E1a), besonders bevorzugt nach Gruppe A1a), wobei dieses Derivat vorzugsweise für ein Protein oder Peptid kodiert, welches in der Lage ist,
   3-Hydroxydekanoyl-ACP über 3-Hydroxydekanoyl-3-Hydroxydekanoyl-ACP zu 3-Hydroxydekanoyl-3-Hydroxydekansäure umzusetzen,
G1a) eine komplementäre Sequenz zu einer Sequenz nach einer der Gruppen A1a) bis F1a), besonders bevorzugt nach Gruppe A1a),
A1b) eine Sequenz gemäß Seq ID Nr. 17, wobei diese Sequenz für ein Protein kodiert, welches in der Lage ist,
   3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen,
B1b) eine intronfreie Sequenz, die von einer Sequenz nach A1b) abgeleitet ist und die das gleiche Protein oder Peptid kodiert wie die Sequenz nach Seq ID Nr. 17,
C1b) eine Sequenz, die ein Protein oder Peptid kodiert, das die Aminosäuresequenz gemäß Seq ID Nr. 18 umfasst, und das bevorzugt in der Lage ist,
   3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen,
D1b) eine Sequenz, die mit einer Sequenz nach einer der Gruppen A1b) bis C1b), besonders bevorzugt nach Gruppe A1b), zu mindestens 70%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen,
E1b) eine Sequenz, die mit dem Gegenstrang einer Sequenz nach einer der Gruppen A1b) bis D1b), besonders bevorzugt nach Gruppe A1b), hybridisiert oder unter Berücksichtigung der Degeneration des genetischen Codes hybridisieren würde, wobei diese Sequenz für ein Protein oder Peptid kodiert, welches in der Lage ist,
   3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen,
F1b) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A1b) bis E1b), besonders bevorzugt nach Gruppe A1b), wobei dieses Derivat vorzugsweise für ein Protein oder Peptid kodiert, welches in der Lage ist,
   3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen, und
G1b) eine komplementäre Sequenz zu einer Sequenz nach einer der Gruppen A1b) bis F1b), besonders bevorzugt nach Gruppe A1b), und
die Gruppe [A2 bis G2] aus den folgenden Sequenzen besteht:
A2a) eine Sequenz gemäß Seq ID Nr. 3, wobei diese Sequenz für ein Protein kodiert, welches in der Lage ist,
   dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
B2a) eine intronfreie Sequenz, die von einer Sequenz nach A2a) abgeleitet ist und die das gleiche Protein oder Peptid kodiert wie die Sequenz nach Seq ID Nr. 3,
C2a) eine Sequenz, die ein Protein oder Peptid kodiert, das die Aminosäuresequenz gemäß Seq ID Nr. 4 umfasst, und welches bevorzugt in der Lage ist,
   dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
D2a) eine Sequenz, die mit einer Sequenz nach einer der Gruppen A2a) bis C2a), besonders bevorzugt nach Gruppe A2a), zu mindestens 80%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
E2a) eine Sequenz, die mit dem Gegenstrang einer Sequenz nach einer der Gruppen A2a) bis D2a), besonders bevorzugt nach Gruppe A2a), hybridisiert oder unter Berücksichtigung der Degeneration des genetischen Codes hybridisieren würde, wobei diese Sequenz für ein Protein oder Peptid kodiert, welches in der Lage ist,
   dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
F2a) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A2a) bis E2a), besonders bevorzugt nach Gruppe A2a), wobei dieses Derivat vorzugsweise für ein Protein oder Peptid kodiert, welches in der Lage ist,
   dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
G2a) eine komplementäre Sequenz zu einer Sequenz nach einer der Gruppen A2a) bis F2a), besonders bevorzugt nach Gruppe A2a),
A2b) eine Sequenz gemäß Seq ID Nr. 19, wobei diese Sequenz für ein Protein kodiert, welches in der Lage ist,
   dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
B2b) eine intronfreie Sequenz, die von einer Sequenz nach A2b) abgeleitet ist und die das gleiche Protein oder Peptid kodiert wie die Sequenz nach Seq ID Nr. 19,
C2b) eine Sequenz, die ein Protein oder Peptid kodiert, das die Aminosäuresequenz gemäß Seq ID Nr. 20 umfasst, und welches bevorzugt in der Lage ist,
   dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
D2b) eine Sequenz, die mit einer Sequenz nach einer der Gruppen A2b) bis C2b), besonders bevorzugt nach Gruppe A2b), zu mindestens 70%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
E2b) eine Sequenz, die mit dem Gegenstrang einer Sequenz nach einer der Gruppen A2b) bis D2b), besonders bevorzugt nach Gruppe A2b), hybridisiert oder unter Berücksichtigung der Degeneration des genetischen Codes hybridisieren würde, wobei diese Sequenz für ein Protein oder Peptid kodiert, welches in der Lage ist,
   dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
F2b) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A2b) bis E2b), besonders bevorzugt nach Gruppe A2b), wobei dieses Derivat vorzugsweise für ein Protein oder Peptid kodiert, welches in der Lage ist,
   dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen, und
G2b) eine komplementäre Sequenz zu einer Sequenz nach einer der Gruppen A2b) bis F2b), besonders bevorzugt nach Gruppe A2b),
und
die Gruppe [A3 bis G3] aus den folgenden Sequenzen besteht:
A3a) eine Sequenz gemäß Seq ID Nr. 5, wobei diese Sequenz für ein Protein kodiert, welches in der Lage ist,
   dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
B3a) eine intronfreie Sequenz, die von einer Sequenz nach A3a) abgeleitet ist und die das gleiche Protein oder Peptid kodiert wie die Sequenz nach Seq ID Nr. 5,
C3a) eine Sequenz, die ein Protein oder Peptid kodiert, das die Aminosäuresequenz gemäß Seq ID Nr. 6 umfasst, und welches bevorzugt in der Lage ist,
   dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
D3a) eine Sequenz, die mit einer Sequenz nach einer der Gruppen A3a) bis C3a), besonders bevorzugt nach Gruppe A3a), zu mindestens 80%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
E3a) eine Sequenz, die mit dem Gegenstrang einer Sequenz nach einer der Gruppen A3a) bis D3a), besonders bevorzugt nach Gruppe A3a), hybridisiert oder unter Berücksichtigung der Degeneration des genetischen Codes hybridisieren würde, wobei diese Sequenz für ein Protein oder Peptid kodiert, welches in der Lage ist,
   dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
F3a) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A3a) bis E3a), besonders bevorzugt nach Gruppe A3a), wobei dieses Derivat vorzugsweise für ein Protein oder Peptid kodiert,
   welches in der Lage ist,
   dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
G3a) eine komplementäre Sequenz zu einer Sequenz nach einer der Gruppen A3a) bis F3a), besonders bevorzugt nach Gruppe A3a),
A3b) eine Sequenz gemäß Seq ID Nr. 21, wobei diese Sequenz für ein Protein kodiert, welches in der Lage ist,
   dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
B3b) eine intronfreie Sequenz, die von einer Sequenz nach A3b) abgeleitet ist und die das gleiche Protein oder Peptid kodiert wie die Sequenz nach Seq ID Nr. 21,
C3b) eine Sequenz, die ein Protein oder Peptid kodiert, das die Aminosäuresequenz gemäß Seq ID Nr. 22 umfasst, und welches bevorzugt in der Lage ist,
   dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
D3b) eine Sequenz, die mit einer Sequenz nach einer der Gruppen A3b) bis C3b), besonders bevorzugt nach Gruppe A3b), zu mindestens 60%, besonders bevorzugt zu mindestens 90%, darüber hinaus bevorzugt zu mindestens 95% und am meisten bevorzugt zu mindestens 99% identisch ist, wobei diese Sequenz vorzugsweise für ein Protein oder Peptid kodiert, welches in der Lage ist,
   dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
E3b) eine Sequenz, die mit dem Gegenstrang einer Sequenz nach einer der Gruppen A3b) bis D3b), besonders bevorzugt nach Gruppe A3b), hybridisiert oder unter Berücksichtigung der Degeneration des genetischen Codes hybridisieren würde, wobei diese Sequenz für ein Protein oder Peptid kodiert, welches in der Lage ist,
   dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
F3b) ein durch Substitution, Addition, Inversion und/oder Deletion mindestens einer Base, vorzugsweise von mindestens 2 Basen, darüber hinaus bevorzugt von mindestens 5 Basen und am meisten bevorzugt mindestens 10 Basen, jedoch von nicht mehr als 100 Basen, besonders bevorzugt von nicht mehr als 50 Basen und am meisten bevorzugt von nicht mehr als 25 Basen erhaltenes Derivat einer Sequenz nach einer der Gruppen A3b) bis E3b), besonders bevorzugt nach Gruppe A3b), wobei dieses Derivat vorzugsweise für ein Protein oder Peptid kodiert,
   welches in der Lage ist,
   dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen, und
G3b) eine komplementäre Sequenz zu einer Sequenz nach einer der Gruppen A3b) bis F3b), besonders bevorzugt nach Gruppe A3b).

Die "Nukleotid-Identität" oder "Aminosäure-Identität" wird hier mit Hilfe bekannter Verfahren bestimmt. Generell werden besondere Computerprogramme mit Algorithmen unter Berücksichtigung spezieller Erfordernisse verwendet.
Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
GAP (Deveroy, J. et al., Nucleic Acid Research 12 (1984), Seite 387, Genetics Computer Group University of Wisconsin, Medicine (Wi), und BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. *et al.,* vorstehend).
Der bekannte Smith-Waterman Algorithmus kann ebenso zur Bestimmung der Nukleotid-Identität verwendet werden.

Bevorzugte Parameter für die Bestimmung der "Nukleotid-Identität" sind bei Verwendung des BLASTN-Programms (Altschul, S. et al., Journal of Molecular Biology 215 (1990), Seiten 403-410:

| | |
|---|---|
| Expect Threshold: | 10 |
| Word size: | 28 |
| Match Score: | 1 |
| Mismatch Score: | -2 |
| Gap costs: | Linear |

Die vorstehenden Parameter sind die default-Parameter im Nukleotidsequenzvergleich.
Das GAP-Programm ist ebenso zur Verwendung mit den vorstehenden Parametern geeignet.

Bevorzugte Parameter für die Bestimmung der "Aminosäure-Identität" sind bei Verwendung des BLASTP-Programms (Altschul, S. et al., Journal of Molecular Biology 215 (1990), Seiten 403-410:

| | |
|---|---|
| Expect Threshold: | 10 |
| Word size: | 3 |
| Matrix: | BLOSUM62 |
| Gap costs: | Existence: 11; Extension: 1 |
| Compositional adjustments: | Conditional compositional score matrix adjustment |

Die vorstehenden Parameter sind die default-Parameter im Aminosäuresequenzvergleich. Das GAP-Programm ist ebenso zur Verwendung mit den vorstehenden Parametern geeignet.

Eine Identität von 60% gemäß dem vorstehenden Algorithmus bedeutet im Zusammenhang mit der vorliegenden Erfindung 60% Identität. Das gleiche gilt für höhere Identitäten.

Das Merkmal "Sequenz, die mit dem Gegenstrang einer Sequenz hybridisiert oder unter Berücksichtigung der Degeneration des genetischen Codes hybridisieren würde" weist auf eine Sequenz hin, die unter vorzugsweise stringenten Bedingungen mit dem Gegenstrang einer Bezugssequenz hybridisiert oder unter Berücksichtigung der Degeneration des genetischen Codes hybridisieren würde. Beispielsweise können die Hybridisierungen bei 68°C in 2 x SSC oder nach dem Protokoll des Digoxigenin-Labelling-Kits der Firma Boehringer (Mannheim) durchgeführt werden. Bevorzugte Hybridisierungsbedingungen sind z. B. Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS.
Zu den Derivaten der erfindungsgemäßen isolierten DNA, welche gemäß Alternative F1), F2) oder F3) durch Substitution, Addition, Inversion und/oder Deletion einer oder mehrerer Basen einer Sequenz nach einer der Gruppen A1) bis E1), A2) bis E2) und A3) bis E3) erhalten werden können, gehören insbesondere solche Sequenzen, welche in dem Protein, welches sie kodieren, zu konservativen Aminosäureaustauschen, wie z. B. dem Austausch von Glycin gegen Alanin oder von Asparaginsäure gegen Glutaminsäure führen. Solche funktionsneutralen Mutationen werden als Sinnmutationen (sense mutations) bezeichnet und führen zu keiner grundsätzlichen Veränderung der Aktivität des Polypeptids. Weiterhin ist bekannt, dass Änderungen am N- und/oder C-Terminus eines Polypeptids dessen Funktion nicht wesentlich beeinträchtigen oder dieses sogar stabilisieren können, so dass dementsprechend auch DNA-Sequenzen, bei denen am 3'-Ende oder am 5'-Ende der Sequenz mit den erfindungsgemäßen Nukleinsäuren Basen angefügt sind, von der vorliegenden Erfindung umfasst sind. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Die erfindungsgemäß enthaltene Nukleinsäure ist bevorzugt ein Vektor, insbesondere ein Expressionsvektor oder eine Genüberexpressionskassette. Als Vektoren kommen alle dem Fachmann bekannten Vektoren in Betracht, die üblicherweise zum Einschleusen von DNA in eine Wirtzelle eingesetzt werden. Diese Vektoren können sowohl autonom replizieren, da sie Replikationsursprünge, wie etwa jenen des 2µ-Plasmids oder ARS (autonom replizierende Sequenzen) besitzen oder in die Chromosomen integrieren (nicht-replikative Plasmide). Unter Vektoren werden auch lineare DNA-Fragmente verstanden, die keinerlei Replikationsursprünge besitzen, wie etwa Geninsertions- oder Genüberexpressionskassetten. Genüberexpressionskassetten bestehen üblicherweise aus einem Marker, den überzuexprimierenden Genen sowie für die Expression der Gene relevanten regulatorischen Bereichen, wie etwa Promotoren und Terminatoren. Bevorzugte Vektoren sind ausgewählt aus der Gruppe umfassend Plasmide und Kassetten, wie etwa *E*. *coli*-Hefe-Shuttle-Plasmide, besonders bevorzugt sind Expressionsvektoren, Geninsertions- oder Genüberexpressionskassetten, insbesondere die unten beschriebenen Vektoren Seq ID Nr. 38, Seq ID Nr. 40, Seq ID Nr. 42, Seq ID Nr. 45 und Seq ID Nr. 47.

Gemäß einer bevorzugten Ausführungsform des erfindungsgemäß enthaltenen Vektors liegen die Sequenzen der Gruppen [A1 bis G1], [A2 bis G2] und [A3 bis G3] unter der Kontrolle mindestens eines konstitutiven oder regulierbaren Promotors, welcher zur Expression des von diesen DNA-Sequenzen kodierten Polypeptids in der Zelle eines Mikroorganismus, vorzugsweise einer Bakterien-, Hefe- oder Pilzzelle, wobei *Aspergillus nidulans, Aspergillus niger, Alcaligenes latus, Bacillus megaterium, Bacillus subtilis, Brevibacterium flavum, Brevibacterium lactofermentum, Burkholderia andropogonis, B. brasilensis, B. caledonica, B. caribensis, B. caryophylli, B. fungorum, B. gladioli, B. glathei, B. glumae, B. graminis, B. hospita, B. kururiensis, B. phenazinium, B. phymatum, B. phytofirmans, B. plantarii, B. sacchari, B. singaporensis, B. sordidicola, B. terricola, B. tropica, B. tuberum, B. ubonensis, B. unamae, B. xenovorans, B. anthina, B. pyrrocinia, B. thailandensis, Candida blankii, Candida rugosa, Corynebacterium glutamicum, Corynebacterium efficiens, Escherichia coli, Hansenula polymorpha, Kluveromyces lactis, Methylobacterium extorquens, Paracoccus versutus, Pseudomonas argentinensis, P. borbori, P. citronellolis, P. flavescens, P. mendocina, P. nitroreducens, P. oleovorans, P. pseudoalcaligenes, P. resinovorans, P. straminea, P. aurantiaca, P. aureofaciens, P. chlororaphis, P. fragi, P. lundensis, P. taetrolens, P. antarctica, P. azotoformans, 'P. blatchfordae', P. brassicacearum, P. brenneri, P. cedrina, P. corrugata, P. fluorescens, P. gessardii, P. libanensis, P. mandelii, P. marginalis, P. mediterranea, P. meridiana, P. migulae, P. mucidolens, P. orientalis, P. panacis, P. proteolytica, P. rhodesiae, P. synxantha, P. thivervalensis, P. tolaasii, P. veronii, P. denitrificans, P. pertucinogena, P. cremoricolorata, P. fulva, P. monteilii, P. mosselii, P. parafulva, P. putida, P. balearica, P. stutzeri, P. amygdali, P. avellanae, P. caricapapayae, P. cichorii, P. coronafaciens, P. ficuserectae, 'P. helianthi', P. meliae, P. savastanoi, P. syringae, P. tomato, P. viridiflava, P. abietaniphila, P. acidophila, P. agarici, P. alcaliphila, P. alkanolytica, P. amyloderamosa, P. asplenii, P. azotifigens, P. cannabina, P. coenobios, P. congelans, P. costantinii, P. cruciviae, P. delhiensis, P. excibis, P. extremorientalis, P. frederiksbergensis, P. fuscovaginae, P. gelidicola, P. grimontii, P. indica, P. jessenii, P. jinjuensis, P. kilonensis, P. knackmussii, P. koreensis, P. lini, P. lutea, P. moraviensis, P. otitidis, P. pachastrellae, P. palleroniana, P. papaveris, P. peli, P. perolens, P. poae, P. pohangensis, P. psychrophila, P. psychrotolerans, P. rathonis, P. reptilivora, P. resiniphila, P. rhizosphaerae, P. rubescens, P. salomonii, P. segitis, P. septica, P. simiae, P. suis, P. thermotolerans, P. aeruginosa, P. tremae, P. trivialis, P. turbinellae, P. tuticorinensis, P. umsongensis, P. vancouverensis, P. vranovensis, P. xanthomarina, Ralstonia eutropha, Rhodospirillum rubrum, Rhodobacter sphaeroides, Saccharomyces cerevisiae, Yarrowia lipolytica, Zymomonas mobilis,*
insbesondere *Pseudomonas putida, Escherichia coli und Burkholderia thailandensis* besonders bevorzugt sind, geeignet ist. Beispiele für konstitutive Promotoren sind *lac, lacUV5, tac, trc* (jeweils in Abwesenheit des LacI-Repressors in den erfindungsgemäßen Zellen), Ltet-O1 (in Abwesenheit des TetR-Repressors in den erfindungsgemäßen Zellen), T5 und *gap.* Beispiele für induzierbare Promotoren sind *lac, lacUV5, tac, trc* (jeweils in Gegenwart des Lacl-Repressors in den erfindungsgemäßen Zellen), Ltet-O1 (in Gegenwart des TetR-Repressors in den erfindungsgemäßen Zellen), T5 (in Kombination mit einem *lac*-Operator und der Gegenwart des Lacl-Repressors in den erfindungsgemäßen Zellen), SP6 und T7 (in Gegenwart des das die kognate RNA-Polymerase kodierenden Genes, dessen Expression seinerseits reguliert ist). Der erfindungsgemäß enthaltene Vektor sollte neben einem Promotor vorzugsweise eine Ribosomenbindungsstelle sowie einen Terminator umfassen. Dabei ist es besonders bevorzugt, dass die erfindungsgemäß enthaltene Nukleinsäure in eine Expressionskassette des Vektors umfassend den Promotor, die Ribosomenbindungsstelle und den Terminator eingebaut ist. Neben den vorstehend genannten strukturellen Elementen kann der Vektor des Weiteren dem Fachmann bekannte Selektionsgene umfassen.

Alle angegebenen Prozent (%) sind wenn nicht anders angegeben Massenprozent. In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Kurze Beschreibung der Figuren:

Figur 1: Fettsäure-Biosynthese, β-Oxidation von Fettsäuren und Verknüpfung dieser Stoffwechselwege mit der Biosynthese von Rhamnolipiden (Enzyme E₁, E₂ und E₃) und Polyhydroxyalkanoaten (Enzyme E₉ und E₁₀). Dargestellt sind die Kohlenstoffflüsse in Fettsäure-Biosynthese, β-Oxidation von Fettsäuren, Rhamnolipid-Biosynthese und Polyhydroxyalkanoat-Biosynthese. Verbrauch und Bildung von Coenzymen, Redoxäquivalenten sowie Nukleotiden sind nicht gezeigt.

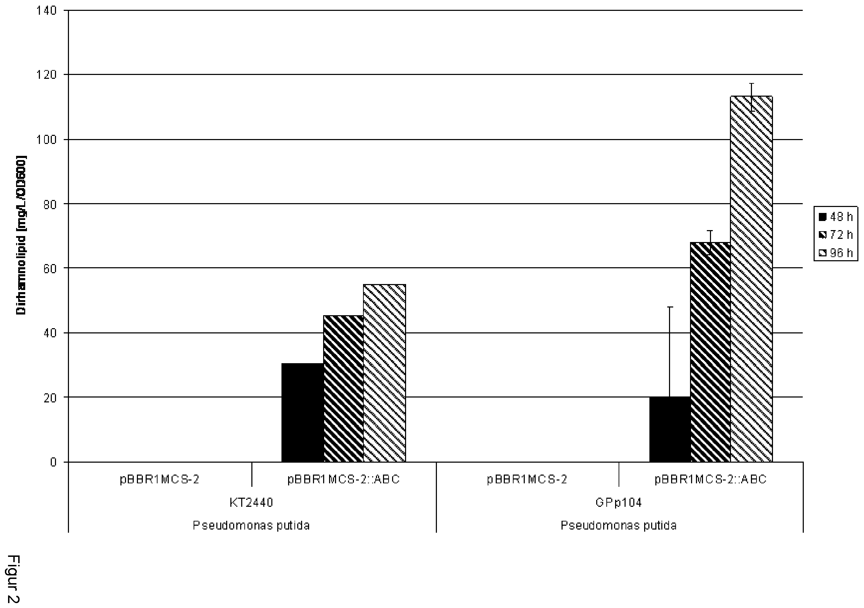

Figur 2: Dirhamnosyllipidbildung (mg/l/OD 600 nm) der rekombinanten Stämme *P. putida* KT2440 pBBR1MCS-2 und pBBR1MCS-2::ABC sowie GPp104 pBBR1 MCS-2 und pBBR1MCS-2::ABC nach 48 h, 72 h und 96 h Kultivierung in CMP-Medium. Die Analyse der Rhamnolipidkonzentration erfolgte mittels HPLC.

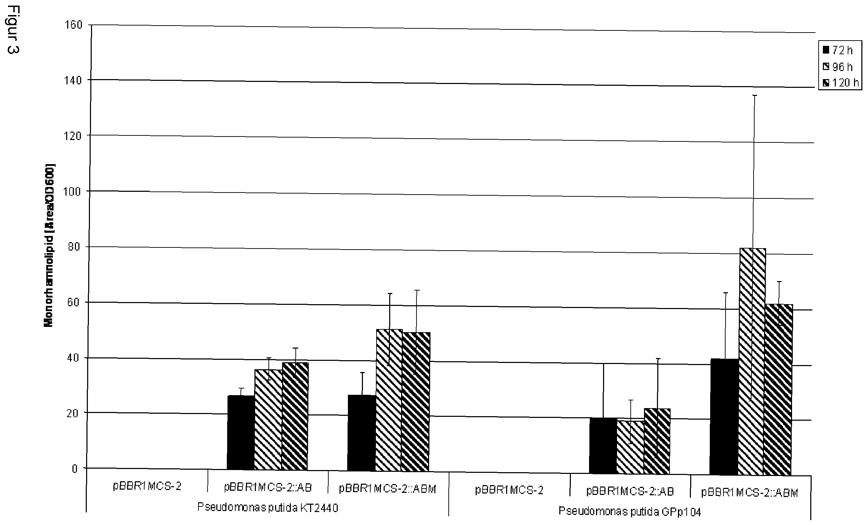

Figur 3: Monorhamnosyllipidbildung (Peaklfläche/OD 600 nm) der rekombinanten Stämme *P. putida* KT2440 pBBR1MCS-2, pBBR1MCS-2::AB und pBBR1MCS-2::ABM sowie GPp104 pBBR1MCS-2, pBBR1MCS-2::AB und pBBR1MCS-2::ABM nach 48 h, 72 h und 96 h Kultivierung in CMP-Medium. Die Analyse der Rhamnolipidkonzentration erfolgte mittels HPLC.

### Beispiele:

### 1. Konstruktion eines Vektors pBBR1MCS-2::AB zur heterologen Expression der Pseudomonas aeruginosa 1707 Gene rhlA und rhlB in Pseudomonas putida

Zur heterologen Expression der *Pseudomonas aeruginosa* DSM1707 Gene *rhlA* und *rhlB* wurde das Plasmid pBBR1MCS-2::AB (Seq ID Nr. 38) konstruiert. Dazu wurde das synthetische Operon *rhlAB* (Seq ID Nr. 37) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::AB wurde das synthetische Operon mittels *Bgl*II und *Xba*I aus dem Vektor geschnitten und anschließend in den mit *Bam*HI und *Xba*I geschnittenen Expressionsvektor pBBR1MCS-2 (Seq ID Nr. 49) (beschrieben bei Kovach et al., 1995: Four new derivatives of the broad-host-range cloning vector pBBR1MCS carrying different antibiotic-resistance cassettes. Gene, 166:175-176) ligiert. Das resultierende Plasmid pBBR1MCS-2::AB (Seq ID Nr. 38) ist 7422 Basenpaare groß. Die Ligation sowie die Transformation chemisch kompetenter *E. coli* DH5α-Zellen (Gibco-BRL, Karlsruhe) erfolgte in dem Fachmann bekannter Art und Weise. Die Authentizität des *Inserts* wurde durch DNA-Sequenzanalyse überprüft.
Die Transformation von *Pseudomonas putida* KT2440 und GPp104 mit den Vektoren pBBR1MCS-2 (Seq ID Nr. 49) und pBBR1MCS-2::AB erfolgte wie vorbeschrieben (Iwasaki et al. Biosci. Biotech. Biochem. 1994. 58(5):851-854). Die Plasmid-DNA von 10 Klonen wurde isoliert und analysiert. Die erhaltenen, die Plasmide tragenden Stämme wurden *P. putida* KT2440 pBBR1MCS-2, *P. putida* GPp104 pBBR1MCS-2, *P. putida* KT2440 pBBR1MCS-2::AB bzw. *P. putida* GPp104 pBBR1MCS-2::AB genannt.

### 2. Konstruktion eines Vektors pBBR1MCS-2::ABC zur heterologen Expression der Pseudomonas aeruginosa DSM1707 Gene rhlA, rhlB und rhlC in Pseudomonas putida

Zur heterologen Expression der *Pseudomonas aeruginosa* DSM1707 Gene *rhlA, rhlB* und *rhlC* wurde das Plasmid pBBR1MCS-2::ABC (Seq ID Nr. 40) konstruiert. Dazu wurde das synthetische Operon *rhlABC* (Seq ID Nr. 39) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::ABC wurde das synthetische Operon mittels *Bgl*II und *Xba*I aus dem Vektor geschnitten und anschließend in den mit *Bam*HI und *Xba*I geschnittenen Expressionsvektor pBBR1 MCS-2 (Seq ID Nr. 49) (Kovach et al., 1995: Four new derivatives of the broad-host-range cloning vector pBBR1MCS carrying different antibiotic-resistance cassettes. Gene, 166:175-176) ligiert. Das resultierende Plasmid pBBR1MCS-2::ABC (Seq ID Nr. 40) ist 8409 Basenpaare groß. Die Ligation sowie die Transformation chemisch kompetenter *E. coli* DH5α-Zellen (Gibco-BRL, Karlsruhe) erfolgte in dem Fachmann bekannter Art und Weise. Die Authentizität des *Inserts* wurde durch DNA-Sequenzanalyse überprüft.
Die Transformation von *Pseudomonas putida* KT2440 und GPp104 mit dem Vektor pBBR1MCS-2::ABC erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme wurden *P. putida* KT2440 pBBR1MCS-2::ABC bzw. *P. putida* GPp104 pBBR1MCS-2::ABC genannt.

### 3. Konstruktion eines Vektors pBBR1MCS-2::ABM zur heterologen Expression der Pseudomonas aeruginosa DSM1707 Gene rhlA, rhlB und pa1131 in Pseudomonas putida

Zur heterologen Expression der *Pseudomonas aeruginosa* DSM1707 Gene *rhlA, rhlB* und *pa1131* wurde das Plasmid pBBR1MCS-2::ABM (Seq ID Nr. 42) konstruiert. Dazu wurde das synthetische Operon *rhlAB-pa1131* (Seq ID Nr. 41) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::ABM wurde das synthetische Operon mittels *Bg*/II und *Xba*I aus dem Vektor geschnitten und anschließend in dem mit *Bam*HI und *Xba*I geschnittenen Expressionsvektor pBBR1MCS-2 (Seq ID Nr. 49) (Kovach et al., 1995: Four new derivatives of the broad-host-range cloning vector pBBR1MCS carrying different antibiotic-resistance cassettes. Gene, 166:175-176) ligiert. Das resultierende Plasmid pBBR1MCS-2::ABM (Seq ID Nr. 42) ist 8702 Basenpaare groß. Die Ligation sowie die Transformation chemisch kompetenter *E. coli* DH5α-Zellen (Gibco-BRL, Karlsruhe) erfolgte in dem Fachmann bekannter Art und Weise. Die Authentizität des *Inserts* wurde durch DNA-Sequenzanalyse überprüft.
Die Transformation von *Pseudomonas putida* KT2440 und GPp104 mit dem Vektor pBBR1MCS-2::ABM erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme wurden *P. putida* KT2440 pBBR1 MCS-2::ABM bzw. *P. putida* GPp104 pBBR1MCS-2::ABM genannt.

### 4. Quantifizierung der Rhamnolipidproduktion durch rekombinante P. putida-Stämme

Die rekombinanten Stämme *P*. *putida* KT2440 pBBR1MCS-2; *P. putida* KT2440 pBBRIMCS-2::AB; *P. putida* KT2440 pBBR1MCS-2::ABC; *P. putida* KT2440 pBBR1MCS-2::ABM; *P. putida* GPp104 pBBR1MCS-2; *P. putida* GPp104 pBBR1MCS-2::AB, *P. putida* GPp104 pBBR1MCS-2::ABC und *P*. *putida* GPp104 pBBR1MCS-2::ABM wurden auf LB-Agar-Kanamycin (50 µg/ml)-Platten kultiviert.

Zur Produktion der Rhamnolipide wurde das im Folgenden als CMP-Medium bezeichnete Medium verwendet. Dieses besteht aus 2% (w/v) Glukose, 0,007% (w/v) KH₂PO₄, 0,11% Na₂HPO₄ x 2 H₂O, 0,2% (w/v) NaNO₃, 0,04% (w/v) MgSO₄ x H₂O, 0,01% (w/v) CaCl₂ x 2 H₂O und 0,2% (v/v) einer Spurenelementlösung. Diese besteht aus 0,2% (w/v) FeSO₄ x 7 H₂O, 0,15% (w/v) MnSO₄ x H₂O und 0,06% (w/v) (NH₄)MO₇O₂₄ x 4 H₂O. Der pH-Wert des Mediums wurde mit NaOH auf 6,7 eingestellt und das Medium folgend mittels Autoklav (121 °C, 20 min) sterilisiert. Ein Einstellen des pH-Wertes während der Kultivierung war nicht nötig.

Zur Untersuchung der Rhamnolipidproduktion im Schüttelkolben wurde zunächst eine Vorkultur angesetzt. Hierzu wurde eine Impföse eines frisch auf LB-Agar-Platte ausgestrichenen Stammes verwendet und 10 ml LB-Medium in einem 100 ml Erlenmeyerkolben beimpft. Alle rekombinanten *P. putida* Stämme wurden im LB-Medium, dem 50 µg/ml Kanamycin zugesetzt wurde. Die Kultivierung der Stämme erfolgte bei 30 °C und 200 rpm über Nacht.

Die Vorkulturen wurden verwendet, um 50 ml CMP-Medium im 250 ml Erlenmeyerkolben zu beimpfen (Start-OD₆₀₀ 0,1). Die Kulturen wurden bei 200 rpm und 30 °C für maximal 120 h kultiviert. Im Abstand von 24 h wurde eine Probe von 1 ml Brühe dem Kulturkolben entnommen. Die Probenvorbereitung für die nachfolgenden chromatographischen Analysen erfolgte folgendermaßen:
Mit einer Verdrängerpipette (Combitip) wurde in einem 2 ml-Reaktionsgefäß 1 ml Aceton vorgelegt und das Reaktionsgefäß zur Minimierung von Verdunstung unmittelbar verschlossen. Es folgte die Zugabe von 1 ml Brühe. Nach Vortexen des Brühe/Acetongemisches wurde dieses für 3 min bei 13.000 rpm abzentrifugiert, und 800 µl des Überstands in ein HPLC-Gefäß überführt.

Zum Nachweis und zur Quantifizierung von Rhamnolipiden wurde ein Evaporative Light Scattering Detektor (Sedex LT-ELSD Model 85LT) benutzt. Die eigentliche Messung wurde mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen betrug 5 µl und die Laufzeit der Methode lag bei 20 min. Als mobile Phase wurde wässrige 0,1% TFA (Trifluoressigsäure, Lösung A) und Methanol (Lösung B) verwendet. Die Säulentemperatur betrug 40 °C. Als Detektoren dienten der ELSD (Detektortemperatur 60 °C) und der DAD (Diodenarray, 210 nm). Der in der Methode verwendete Gradient war:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

Während *P. putida* KT2440 pBBR1MCS-2 und GPp104 pBBR1MCS-2 keine Rhamnolipide produzierten, war in den rekombinanten Stämmen *P. putida* KT2440 pBBR1MCS-2::AB, *P. putida* KT2440 pBBR1MCS-2::ABC, *P. putida* KT2440 pBBR1MCS-2::ABM, *P. putida* GPp104 pBBR1MCS-2::AB, *P. putida* GPp104 pBBR1MCS-2::ABC und *P. putida* GPp104 pBBR1MCS-2::ABM die Bildung von unterschiedlichen Rhamnolipidspezies feststellbar (Fig. 2 und 3).

Durch die Einbringung des pBBR1MCS-2::AB bzw. pBBR1MCS-2::ABM in *P. putida* konnten Monorhamnosyllipide generiert werden (Fig. 3). Da kein Referenzmaterial für
Monorhamnosyllipide vorhanden war, erfolgte die Identifizierung der Produkte durch Analyse der entsprechenden Massenspuren und der MS²-Spektren bei LC-MS.
Wurde zusätzlich *rhlC* (pBBR1MCS-2::ABC) in die Stämme eingebracht, so wurden Mono- und Dirhamnosyllipide produziert (Fig. 2).

Der direkte vergleich der Rhamnolipidbildung durch *P. putida* pBBR1MCS-2::AB bzw. *P. putida* pBBR1MCS-2::ABM zeigt, dass die Coexpression von *P. aeruginosa p3111* mit *P. aeruginosa rhlAB* zu einer Verbesserung der Rhamnolipidbiosynthese führt (Fig. 3). Während die Stämme *P. putida* KT2440 pBBR1MCS-2::AB und *P. putida* GPp104 pBBR1MCS-2::AB etwa 39 (*P. putida* KT2440 pBBR1MCS-2::AB) bzw. 23 Peakflächen Rhamnolipide/OD 600 nm (*P. putida* GPp104 pBBR1MCS-2::AB) nach 120 h produziert hatten, bildeten die Stämme *P. putida* KT2440 pBBR1MCS-2::ABM und *P. putida* GPp104 pBBR1MCS-2::ABM etwa 50 (*P. putida* KT2440 pBBR1MCS-2::ABM) bzw. 62 Peakflächen Rhamnolipide/OD 600 nm (*P. putida* GPp104 pBBR1MCS-2::ABM) nach 120 h.

Wurde die Monorhamnosyllipidsynthese der Stämme *P. putida* KT2440 pBBR1MCS-2::ABM und *P. putida* GPp104 pBBR1MCS-2::ABM verglichen, so konnten in der PHA-negativen Mutante P. *putida* GPp104 pBBR1MCS-2::ABM 62 Peakflächen/OD 600 nm (120 h Kultivierung) und mit *P. putida* KT2440 pBBR1MCS-2::ABM 50 Area/OD 600 nm Monorhamnosyllipide detektiert werden (Fig. 3).
Eine vergleichende Analyse der Dirhamnosyllipidbildung (mg/l/OD 600 nm) in den Stämmen *P. putida* KT2440 und GPp104 zeigte ebenfalls eine stärkere Bildung der Dirhamnosyllipide im PHA-negativen Stammhintergrund des *P. putida* GPp104. *P. putida* GPp104 pBBR1MCS-2::ABC bildete durchschnittlich 113 mg/l/OD 600 nm Dirhamnosyllipide (96 h), wohingegen mit *P. putida* KT2440 pBBR1MCS-2::ABC nur 55 mg/L/OD 600 nm Dirhamnosyllipide nach 96 h nachzuweisen waren (Figur 2).
Somit konnte gezeigt werden, dass die Verwendung eines bezüglich PHA-Synthese abgeschwächten Stammhintergrunds zu einer Verbesserung der Rhamnolipidbiosynthese führt.

### 5. Konstruktion eines Vektors pBBR1MCS-2::ABMC zur heterologen Expression der Pseudomonas aeruginosa DSM1707 Gene rhlA, rhlB, pa1131 und rhlC in Pseudomonas putida

Zur heterologen Expression der *Pseudomonas aeruginosa* DSM1707 Gene *rhlA, rhlB, pa1131* und *rhlC* wurde das Plasmid pBBR1MCS-2::ABMC (Seq ID Nr. 51) konstruiert. Dazu wurde das synthetische Operon *rhlAB-pa1131-rhlC* (Seq ID Nr. 50) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::ABMC wurde das synthetische Operon mittels *Bg*/II und *Xba*I aus dem Vektor geschnitten und anschließend in dem mit *Bam*HI und *Xba*I geschnittenen Expressionsvektor pBBR1MCS-2 (Seq ID Nr. 49) (Kovach et al., 1995: Four new derivatives of the broad-host-range cloning vector pBBR1MCS carrying different antibiotic-resistance cassettes. Gene, 166:175-176) ligiert. Das resultierende Plasmid pBBR1MCS-2::ABMC (Seq ID Nr. 51) ist 9663 Basenpaare groß. Die Ligation sowie die Transformation chemisch kompetenter *E. coli* DH5α-Zellen (Gibco-BRL, Karlsruhe) erfolgte in dem Fachmann bekannter Art und Weise. Die Authentizität des *Inserts* wurde durch DNA-Sequenzanalyse überprüft.
Die Transformation von *Pseudomonas putida* KT2440 und GPp104 mit dem Vektor pBBR1 MCS-2::ABMC erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme wurden *P. putida* KT2440 pBBR1 MCS-2::ABMC bzw. *P. putida* GPp104 pBBR1MCS-2::ABMC genannt.

### 6. Qualitativer Vergleich der Rhamnolipidproduktion durch rekombinante P. putida-Stämme und P. aeruginosa-Stämme

Die rekombinanten Stämme *P. putida* GPp104 pBBR1MCS-2 und *P. putida* GPp104 pBBR1 MCS-2::ABMC sowie *P. aeruginosa* DSM 19880 wurden auf LB-Agar-Kanamycin (50 µg/ml; *P. putida*)- bzw. LB-Agar-Platten (*P. aeruginosa*) kultiviert.

Zur Produktion der Rhamnolipide wurde das im Folgenden als CMP-Medium bezeichnete Medium verwendet. Dieses besteht aus 2% (w/v) Glukose, 0,007% (w/v) KH₂PO₄, 0,11% Na₂HPO₄ x 2 H₂O, 0,2% (w/v) NaNO₃, 0,04% (w/v) MgSO₄ x H₂O, 0,01% (w/v) CaCl₂ x 2 H₂O und 0,2% (v/v) einer Spurenelementlösung. Diese besteht aus 0,2% (w/v) FeSO₄ x 7 H₂O, 0,15% (w/v) MnSO₄ x H₂O und 0,06% (w/v) (NH₄)MO₇O₂₄ x 4 H₂O. Der pH-Wert des Mediums wurde mit NaOH auf 6,7 eingestellt und das Medium folgend mittels Autoklav (121 °C, 20 min) sterilisiert. Ein Einstellen des pH-Wertes während der Kultivierung war nicht nötig.

Zur Untersuchung der Rhamnolipidproduktion im Schüttelkolben wurde zunächst eine Vorkultur angesetzt. Hierzu wurde eine Impföse eines frisch auf LB-Agar-Platte ausgestrichenen Stammes verwendet und 10 ml LB-Medium in einem 100 ml Erlenmeyerkolben beimpft. Die rekombinanten *P. putida* Stämme wurden im LB-Medium, dem 50 µg/ml Kanamycin zugesetzt wurde, kultiviert. *P. aeruginosa* wurde im LB-Medium kultiviert. Die Kultivierung der Stämme erfolgte bei 30 °C und 200 rpm über Nacht.
Die Vorkulturen wurden verwendet, um 50 ml CMP-Medium im 250 ml Erlenmeyerkolben zu beimpfen (Start-OD₆₀₀ 0,1). Die Kulturen wurden bei 200 rpm und 30 °C für maximal 120 h kultiviert. Im Abstand von 24 h wurde eine Probe von 1 ml Brühe dem Kulturkolben entnommen. Die Probenvorbereitung für die nachfolgenden chromatographischen Analysen erfolgte folgendermaßen:

Mit einer Verdrängerpipette (Combitip) wurde in einem 2 ml-Reaktionsgefäß 1 ml Aceton vorgelegt und das Reaktionsgefäß zur Minimierung von Verdunstung unmittelbar verschlossen. Es folgte die Zugabe von 1 ml Brühe. Nach Vortexen des Brühe/Acetongemisches wurde dieses für 3 min bei 13.000 rpm abzentrifugiert, und 800 µl des Überstands in ein HPLC-Gefäß überführt.

Für die Identifizierung der entstandenen Produkte wurden 5 µl in eine UPLC-Anlage Accela (Thermo Scientific, Dreieich) injiziert. Die zu untersuchenden Substanzen wurden mit einer semi UPLC-Säule "PursuitXRs ULTRA (C8, 2,8 µm, 2,1 x 100 mm) (Varian, Darmstadt) analysiert. Die Trennung erfolgte innerhalb von 25 min mittels eines Gradienten bestehend aus der mobilen Phase A1 (H₂O, 0,1% (v/v) TFA) und der mobilen Phase B1 (Methanol, 0,1% (v/v) TFA) mit einer Flussrate von 0,3 ml/min bei 40 °C. Der zeitliche Verlauf des Gradienten war der folgende:

| Zeit [min] | Mobile Phase A1 [%] | Mobile Phase B1 [%] |
|---|---|---|
| 0 | 30 | 70 |
| 15 | 0 | 100 |
| 25 | 0 | 100 |
| 25,01 | 30 | 70 |
| 32 | 30 | 70 |

Die Detektion erfolgte mittels DAD-Detektor im Wellenlängenbereich von 200 - 600 nm sowie massenselektiv mit einem hochauflösenden FT-ICR Massenspektrometer LTQ-FT (Thermo Scientific, Dreieich) im Scanbereich m/z 100 - 1000. Die Ionisierung erfolgte mittels ESI (electrospray ionization). Exakte Massen und chemische Summenformeln wurden mit Hilfe des FT-ICR Massenanalysators, mit einer Auflösung von R = 100000 und einer Massengenauigkeit von ≤ 2 ppm ermittelt. Die Identifizierung der Produkte erfolgt durch Analyse der entsprechenden Massenspuren und der MS²-Spektren. Um die Stämme vergleichen zu können, wurden die Peakflächen der entsprechenden Substanzen gegenübergestellt.
Wie in Figur 4 dargestellt bildete der Stamm *P. putida* GPp104 pBBR1 MCS-2 keinerlei Rhamnolipide. *P. putida* GPp104 pBBR1MCS-2::ABMC sowie *P. aeruginosa* DSM 19880 bildeten Rhamnolipide, wobei das Verhältnis zwischen gebildeten Di- und Monorhamnosyllipiden bei *P. putida* GPp104 pBBR1MCS-2::ABMC etwa bei 4:1, bei *P. aeruginosa* DSM 19880 etwa bei 2:1 lag. Außerdem bildete der Stamm *P. putida* GPp104 pBBR1MCS-2::ABMC im Gegensatz zu *P. aeruginosa* DSM 19880 keine oder nur sehr wenige Rhamnolipide mit einem über R¹ und R² bestimmten Rest abgeleitet von 3-Hydroxyoctanoyl-3-Hydroxydekansäure oder 3-Hydroxydekanoyl-3-Hydroxyoctansäure.

### 7. Konstruktion eines Vektors pBBR1MCS-2::rfbBDAC und pBBR1MCS-2::ABC_rfbBDAC zur heterologen Expression in Pseudomonas putida

Bei der Firma Trenzyme GmbH (Konstanz) wurde ausgehend von chromosomaler DNA des *Pseudomonas putida* KT2440 das Rhamnose-Biosyntheseoperon *rfbBDAC* amplifiziert. Dazu wurden folgende Primer verwendet:
RL1: 5'-TATATATAGAATTCGCGTCATCTGTCTACGACAACAC-3' (Seq ID Nr. 48)
RL2: 5'-TATATATAGAATTCGGCTGCGCTACCGCAGCCCTTC-3' (Seq ID Nr. 43)

Das erhaltende PCR Produkt wurde in Trenzyme's Alligator Cloning System zwischenkloniert und in *E. coli* DH5α (New England Biolabs; Frankfurt) transformiert. Vektoren unterschiedlicher Kandidaten wurden analysiert und sequenziert. Nach erfolgreicher und fehlerfreier DNA-Sequenzierung, wurde der Vektor mittels *Eco*RI geschnitten und das Zielfragment *rfbBDAC* isoliert. Für eine weitere Zwischenklonierung wurde der Vektor pBBR1 MCS-2 (Kovach et al., 1995: Four new derivatives of the broad-host-range cloning vector pBBR1MCS carrying different antibiotic-resistance cassettes. Gene, 166:175-176) auf die gleiche Art und Weise geschnitten. Das geschnittene Zielfragment (*rfbBDAC*) und der geschnittene Vektor (pBBR1MCS-2) wurden durch konventionelle Ligation zusammengeführt. Der entstandene Vektor pBBR1MCS-2::rfbBDAC (Seq ID Nr. 45) wurde ebenfalls in *E. coli* DH5α (New England Biolabs; Frankfurt) transformiert. Einige Kandidaten der Transformanten wurden hinsichtlich der erfolgreichen Aufnahme des Plasmids untersucht.
Der Vektor pBBR1 MCS-2::rfbBDAC diente als Matrize für eine PCR. Es wurden folgende Oligonukleotide verwendet:
RL_XbaI-fw: 5'-TATATATATCTAGAATTAATGCAGCTGGCACGAC-3' (Seq ID Nr. 44)
RL_Xba_rev: 5'-GGCCGCTCTAGAACTAGTGGA-3' (Seq ID Nr. 46)

Die PCR wurde mit der Phusion™ High-Fidelity Master Mix von New England Biolabs (Frankfurt) Polymerase durchgeführt. Sie erfolgte in dem Fachmann bekannter Art und Weise. Die Zielsequenz (*lac*-Promotor und *rfbBDAC*) wurde ins Trenzyme Alligator Cloning System zwischenkloniert. *E. coli* DH5α (New England Biolabs; Frankfurt) Transformanten wurden selektioniert und die Plasmid-DNA verschiedener Kandidaten isoliert und sequenziert. Nachdem die Sequenz überprüft und auf die Richtigkeit untersucht worden ist, wurde der Vektor mit *Xba*I geschnitten. Das Zielfragment wurde in den ebenfalls mit *Xba*I geschnittenen pBBR1MCS-2::ABC (siehe oben) mittels herkömmlichen Ligierungsmethoden ligiert. Der erhaltende Zielvektor pBBR1MCS-2::ABC_rfbBDAC (Seq ID Nr. 47) hat eine Größe von 12249 Basenpaare. Das *Insert* des Vektors wurde sequenziert. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgröße, Reinigung der PCR-Produkte und DNA-Konzentrationsbestimmung erfolgte in dem Fachmann bekannter Art und Weise.
Die Transformation von *Pseudomonas putida* KT2440 und GPp104 mit dem Vektor pBBR1 MCS-2::ABC_rfbBDAC erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme werden *P. putida* KT2440 pBBR1MCS-2::ABC_rfbBDAC und *P. putida* GPp104 pBBR1MCS-2::ABC_rfbBDAC genannt.

### 8. Quantifizierung der Rhamnolipidproduktion durch rekombinante P. putida-Stämme mit und ohne Überexpression des rfbBDAC-Operons

Die rekombinanten Stämme *P. putida* KT2440 pBBR1MCS-2; *P. putida* KT2440 pBBR1MCS-2::ABC, *P. putida* KT2440 pBBR1MCS-2::ABC_rfbBDAC, *P. putida* GPp104 pBBR1MCS-2, *P. putida* GPp104 pBBR1MCS-2::ABC und *P. putida* GPp104 pBBR1MCS-2::ABC_rfbBDAC werden auf LB-Agar-Kanamycin (50 µg/ml)-Platten kultiviert.
Zur Produktion der Rhamnolipide wird das im Folgenden als CMP-Medium bezeichnete Medium verwendet. Dieses besteht aus 2% (w/v) Glukose, 0,007% (w/v) KH₂PO₄, 0,11% Na₂HPO₄ x 2 H₂O, 0,2% (w/v) NaNO₃, 0,04% (w/v) MgSO₄ x H₂O, 0,01% (w/v) CaCl₂ x 2 H₂O und 0,2% (v/v) einer Spurenelementlösung. Diese besteht aus 0,2% (w/v) FeSO₄ x 7 H₂O, 0,15% (w/v) MnSO₄ x H₂O und 0,06% (w/v) (NH₄)MO₇O₂₄ x 4 H₂O. Der pH-Wert des Mediums wird mit NaOH auf 6,7 eingestellt und das Medium folgend mittels Autoklav (121 °C, 20 min) sterilisiert. Ein Einstellen des pH-Wertes während der Kultivierung ist nicht nötig.

Zur Untersuchung der Rhamnolipidproduktion im Schüttelkolben wird zunächst eine Vorkultur angesetzt. Hierzu wird eine Impföse eines frisch auf LB-Agar-Platte ausgestrichenen Stammes verwendet und 10 ml LB-Medium in einem 100 ml Erlenmeyerkolben beimpft. Alle rekombinanten *P. putida* Stämme werden im LB-Medium, dem 50 µg/ml Kanamycin zugesetzt wird, kultiviert. Die Kultivierung der *P. putida* Stämme erfolge bei 30 °C und 200 rpm über Nacht.
Die Vorkulturen werden verwendet, um 50 ml CMP-Medium im 250 ml Erlenmeyerkolben zu beimpfen (Start-OD₆₀₀ 0,1). Die Kulturen werden bei 200 rpm und 30 °C für maximal 120 h kultiviert. Im Abstand von 24 h wird eine Probe von 1 ml Brühe dem Kulturkolben entnommen.

Die Probenvorbereitung für die nachfolgenden chromatographischen Analysen erfolgt folgendermaßen:
Mit einer Verdrängerpipette (Combitip) wird in einem 2 ml-Reaktionsgefäß 1 ml Aceton vorgelegt und das Reaktionsgefäß zur Minimierung von Verdunstung unmittelbar verschlossen. Es folgt die Zugabe von 1 ml Brühe. Nach Vortexen des Brühe/Acetongemisches wird dieses für 3 min bei 13.000 rpm abzentrifugiert, und 800 µl des Überstands in ein HPLC-Gefäß überführt.
Zum Nachweis und zur Quantifizierung von Rhamnolipiden wird ein Evaporative Light Scattering Detektor (Sedex LT-ELSD Model 85LT) benutzt. Die eigentliche Messung wird mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen beträg 5 µl und die Laufzeit der Methode ist 20 min. Als mobile Phase wird wässrige 0,1% TFA (Trifluor-Essigsäure, Lösung A) und Methanol (Lösung B) verwendet. Die Säulentemperatur beträgt 40 °C. Als Detektoren dienen der ELSD (Detektortemperatur 60 °C) und der DAD (Diodenarray, 210 nm). Der in der Methode verwendete Gradient ist:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

Während *P. putida* KT2440 pBBR1MCS-2 und GPp104 pBBR1MCS-2 keine Rhamnolipide produzieren, ist in den rekombinanten Stämmen *P. putida* KT2440 pBBR1MCS-2::ABC, *P. putida* KT2440 pBBR1MCS-2::ABC_rfbBDAC; *P. putida GPp104 pBBR1MCS-2::ABC und P. putida* GPp104 pBBR1MCS-2::ABC_rfbBDAC die Bildung von Rhamnolipiden feststellbar.

*P. putida* KT2440 pBBR1MCS-2::ABC_rfbBDAC zeigt im Vergleich zu *P. putida* KT2440 pBBR1MCS-2::ABC und *P. putida* GPp104 pBBR1MCS-2::ABC_rfbBDAC im Vergleich zu *P. putida* GPp104 pBBR1MCS-2::ABC eine verstärkte Bildung der Di- und Monorhamnosyllipide. Dies zeigt deutlich den positiven Einfluss der Verstärkung der Expression von *rfbBDAC* auf die Bildung von Mono- und Dirhamnosyllipiden.

Wird die Mono- und Dirhamnosyllipidbiosynthese der Stämme *P. putida* KT2440 pBBR1MCS-2::ABC_rfbBDAC und *P. putida* GPp104 pBBR1 MCS-2::ABC_rfbBDAC verglichen, so wird in der PHA-negativen Mutante P. *putida* GPp104 pBBR1MCS-2::ABC_rfbBDAC eine verstärkte Mono- und Dirhamnosyllipidsynthese detektiert.
Wie bereits oben beschrieben, ist bei der Verwendung eines in der PHA-Synthese inaktivierten Stammhintergrunds die Rhamnolipidbiosynthese verstärkt.

### 9. Generierung rekombinanter E. coli W3110 pBBR1MCS-2::ABC und E. coli W3110 pBBR1MCS-2::ABC_rfbBDAC

Die Transformation von *E. coli* W3110 erfolgte wie vorbeschrieben (Miller JH. A Short Course in Bacterial Genetics: A Laboratory Manual and Handbook for Escherichia coli and Related Bacteria. Plainview, NY: Cold Spring Harbor Lab. Press; 1992) mittels Elektroporation. Die Plasmid-DNA von je 10 Klonen wurde isoliert und analysiert. Die erhaltenen, die Plasmide tragenden Stämme wurden *E. coli* W3110 pBBR1MCS-2::ABC_rfbBDAC und *E. coli* W3110 pBBR1MCS-2::ABC_rfbBDAC genannt.

### 10. Quantifizierung der Rhamnolipidproduktion durch rekombinante E. coli-Stämme mit und ohne Überexpression des rfbBDAC-Operons

Die rekombinanten Stämme *E. coli* W3110 pBBR1MCS-2; *E. coli* W3110 pBBR1MCS-2::ABC und *E. coli* W3110 pBBR1MCS-2::ABC_rfbBDAC werden auf LB-Agar-Kanamycin (50 µg/ml)-Platten kultiviert.
Zur Produktion der Rhamnolipide wird das im Folgenden als CMP-Medium bezeichnete Medium verwendet. Dieses besteht aus 2 % (w/v) Glukose, 0,007 % (w/v) KH₂PO₄, 0,11 % Na₂HPO₄ x 2 H₂O, 0,2 % (w/v) NaNO₃, 0,04 % (w/v) MgSO₄ x H₂O, 0,01 % (w/v) CaCl₂ x 2 H₂O und 0,2 % (v/v) einer Spurenelementlösung. Diese besteht aus 0,2 % (w/v) FeSO₄ x 7 H₂O, 0,15 % (w/v) MnSO₄ x H₂O und 0,06 % (w/v) (NH₄)MO₇O₂₄ x 4 H₂O. Der pH-Wert des Mediums wird mit NaOH auf 6,7 eingestellt und das Medium folgend mittels Autoklav (121 °C, 20 min) sterilisiert. Ein Einstellen des pH-Wertes während der Kultivierung ist nicht nötig.

Zur Untersuchung der Rhamnolipidproduktion im Schüttelkolben wird zunächst eine Vorkultur angesetzt. Hierzu wird eine Impföse eines frisch auf LB-Agar-Platte ausgestrichenen Stammes verwendet und 10 ml LB-Medium in einem 100 ml Erlenmeyerkolben beimpft. Alle rekombinanten *E. coli* Stämme werden im LB-Medium, dem 50 µg/ml Kanamycin zugesetzt wird, kultiviert. Die Kultivierung der *E. coli* Stämme erfolge bei 37 °C und 200 rpm über Nacht. Die Vorkulturen werden verwendet, um 50 ml CMP-Medium im 250 ml Erlenmeyerkolben zu beimpfen (Start-OD₆₀₀ 0,1). Die Kulturen werden bei 200 rpm und 30 °C für maximal 120 h kultiviert. Im Abstand von 24 h wird eine Probe von 1 ml Brühe dem Kulturkolben entnommen. Die Probenvorbereitung für die nachfolgenden chromatographischen Analysen erfolgt folgendermaßen:
Mit einer Verdrängerpipette (Combitip) wird in einem 2 ml-Reaktionsgefäß 1 ml Aceton vorgelegt und das Reaktionsgefäß zur Minimierung von Verdunstung unmittelbar verschlossen. Es folgt die Zugabe von 1 ml Brühe. Nach Vortexen des Brühe/Acetongemisches wird dieses für 3 min bei 13.000 rpm abzentrifugiert, und 800 µl des Überstands in ein HPLC-Gefäß überführt.
Zum Nachweis und zur Quantifizierung von Rhamnolipiden wird ein Evaporative Light Scattering Detektor (Sedex LT-ELSD Model 85LT) benutzt. Die eigentliche Messung wird mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen beträg 5 µl und die Laufzeit der Methode ist 20 min. Als mobile Phase wird wässrige 0,1 % TFA (Trifluor-Essigsäure, Lösung A) und Methanol (Lösung B) verwendet. Die Säulentemperatur beträgt 40 °C. Als Detektoren dienen der ELSD (Detektortemperatur 60 °C) und der DAD (Diodenarray, 210 nm). Der in der Methode verwendete Gradient ist:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

Während *E. coli* W3110 pBBR1 MCS-2 keine Rhamnolipide produziert, ist in den rekombinanten Stämmen *E. coli* W3110 pBBR1MCS-2::ABC und *E. coli* W3110 pBBR1MCS-2::ABC_rfbBDAC die Bildung von Mono- und Dirhamnosyllipiden feststellbar, wobei *E. coli* W3110 pBBR1MCS-2::ABC_rfbBDAC signifikant mehr Mono- und Dirhamnosyllipide bildet als *E. coli* W3110 pBBR1MCS-2::ABC. Dies zeigt, dass die heterologe Expression von *rhlABC* aus *Pseudomonas aeruginosa* DSM1707 zur Bildung von Mono- und Dirhamnosyllipiden in *E. coli* führt. Dies zeigt weiterhin den positiven Einfluss der Verstärkung der Expression von *rfbBDAC* auf die Bildung von Mono- und Dirhamnosyllipiden.

### 11. Konstruktion eines Vektors pBBR1MCS-2::ABC-BTH_II1077- II1080-II1081 zur heterologen Expression der Pseudomonas aeruginosa DSM1707 Gene rhlA, rhlB und rhlC sowie der Burkholderia thailandensis E264-Gene BTH_II1077, BT_II1080 und BT_II1081 in Pseudomonas putida

Zur heterologen Expression der *Pseudomonas aeruginosa* DSM1707 Gene *rhlA, rhlB* und *rhlC* sowie der B. *thailandensis* E264-Gene *BTH_II1077, BT_II1080* und *BT_II1081* in *Pseudomonas putida* wird das Plasmid pBBR1MCS-2::ABC-BTH_II1077- II1080-II1081 (Seq ID Nr. 69) konstruiert. Dazu wird das synthetische Operon *BTH_II1077, BT_II1080* und *BT_II1081* (Seq ID Nr. 70) von der Firma DNA 2.0 (Menlo Park, CA, USA) synthetisiert und im kommerziellen Vektor pJ294 (DNA 2.0; Menlo Park, CA, USA) zwischenkloniert. Grundlage für die Synthese ist die genomische Sequenz des Stammes B. *thailandensis* E264. Ausgehend vom Vektor pJ294-BTH_II1077- II080-II081 wird das synthetische Operon mittels *Xbal* aus diesem Vektor geschnitten und anschließend in den ebenfalls mit *Xba*I geschnittenen Vektor pBBR1MCS-2::ABC (Seq ID Nr. 40) ligiert. Der erhaltene Zielvektor pBBR1MCS-2::ABC-BTH_II1077-II1080-II1081 (Seq ID Nr. 69) hat eine Größe von 13768 Basenpaaren. Das Insert des Vektors wird sequenziert. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgröße, Reinigung der PCR-Produkte und DNA-Konzentrationsbestimmung erfolgt in dem Fachmann bekannter Art und Weise.
Die Transformation von *Pseudomonas putida* KT2440 und GPp104 mit dem Vektor pBBR1MCS-2::ABC-BTH_II1077- II1080-II1081 (Seq ID Nr. 69) erfolgt wie vorbeschrieben (Iwasaki et al. Biosci. Biotech. Biochem. 1994. 58(5):851-854). Die Plasmid-DNA von je 10 Klonen wird isoliert und analysiert. Die erhaltenen, die Plasmide tragenden Stämme werden *P. putida* KT2440 pBBR1MCS-2::ABC-BTH_II1077- II1080-II1081 bzw. *P. putida* GPp104 pBBR1MCS-2::ABC-BTH_II1077- II1080-II1081 genannt.

### 12. Quantifizierung der Rhamnolipidproduktion durch rekombinante P. putida-Stämme mit und ohne Überexpression der B. thailandensis E264-Gene BTH_II1077, BT_II1080 und BT_II1081

Die in den Beispielen 1, 2 und 11 generierten rekombinanten Stämme *P. putida* -Stämme P. *putida* KT2440 pBBR1MCS-2::AB, *P. putida* KT2440 pBBR1MCS-2::AB-BTH_II1077- II1080-II1081, *P. putida* GPp104 pBBR1MCS-2::AB, *P. putida* GPp104 pBBR1MCS-2::AB-BTH_II1077- II1080-II1081, *P. putida* KT2440 pBBR1MCS-2::ABC, *P. putida* KT2440 pBBR1MCS-2::ABC-BTH_II1077- II1080-II1081 *P. putida* GPp104 pBBR1MCS-2::ABC und *P. putida* GPp104pBBR1MCS-2::ABC-BTH_II1077- II1080-II1081werden auf LB-Agar-Kanamycin (50 µg/ml)-Platten kultiviert.
Zur Produktion der Rhamnolipide wird das im Folgenden als M9-Medium bezeichnete Medium verwendet. Dieses Medium besteht aus 2 % (w/v) Glukose, 0,3 % (w/v) KH₂PO₄, 0,679 % Na₂HPO₄, 0,05 % (w/v) NaCl, 0,2 % (w/v) NH₄Cl, 0,049 % (w/v) MgSO₄ x 7 H₂O und 0,1 % (v/v) einer Spurenelementlösung. Diese besteht aus 1,78 % (w/v) FeSO₄ x 7 H₂O, 0,191 % (w/v) MnCl₂ x 7 H₂O, 3,65 % (w/v) HCl, 0,187 % (w/v) ZnSO₄ x 7 H₂O, 0,084 % (v/v) Na-EDTA x 2 H₂O, 0,03 % (v/v) H₃BO₃, 0,025 % (w/v) Na₂MoO₄ x 2 H₂O und 0,47 % (w/v) CaCl₂ x 2 H₂O. Der pH-Wert des Mediums wird mit NH₄OH auf 7,4 eingestellt und das Medium folgend mittels Autoklav (121 °C, 20 min) sterilisiert. Ein Einstellen des pH-Wertes während der Kultivierung ist nicht nötig.
Zur Untersuchung der Rhamnolipidproduktion im Schüttelkolben wird zunächst eine Vorkultur angesetzt. Hierzu wird eine Impföse eines frisch auf LB-Agar-Platte ausgestrichenen Stammes verwendet und 10 ml LB-Medium in einem 100 ml Erlenmeyerkolben beimpft. Alle rekombinanten *P. putida-Stämme* werden in LB-Medium, dem 50 µg/ml Kanamycin zugesetzt wurde, kultiviert. Die Kultivierung der *P. putida-Stämme* erfolgt bei 37 °C und 200 rpm über Nacht.
Die Vorkulturen werden verwendet, um 50 ml M9-Medium (+ 50 µg/ml Kanamycin) im 250 ml Erlenmeyerkolben zu beimpfen (Start-OD₆₀₀ 0,1). Die Kulturen werden bei 200 rpm und 30 °C kultiviert. Im Abstand von 24 h wird eine Probe von 1 ml Brühe dem Kulturkolben entnommen. Die Probenvorbereitung für die nachfolgenden chromatographischen Analysen und die chromatographischen Analysen selbst erfolgen wie in Beispiel 4 beschrieben.
Es wird gezeigt, dass die rekombinanten Stämme *P. putida* KT2440 pBBR1MCS-2::AB-BTH_II1077- II1080-II1081 und *P. putida* GPp104 pBBR1MCS-2::AB-BTH_II1077- II1080-II1081 wesentlich mehr Monorhamnosyllipide bilden als die Stämme *P. putida* KT2440 pBBR1MCS-2::AB und *P. putida* GPp104 pBBR1MCS-2::AB. Dies beweist, dass die Verstärkung von *BTH_II1077-II1080-II1081* aus *B. thailandensis* E264 die Bildung von Monorhamnosyllipiden in *P. putida-*Stämmen mit den *Pseudomonas aeruginosa* DSM1707 Genen *rhlAB* verstärkt.
Es wird weiterhin gezeigt, dass die rekombinanten Stämme *P. putida* KT2440 pBBR1MCS-2::ABC-BTH_II1077- II1080-II1081 und *P. putida* GPp104 pBBR1MCS-2::ABC-BTH_II1077-II1080-II1081 wesentlich mehr Mono- und Dirhamnosyllipide bilden als die Stämme *P. putida* KT2440 pBBR1MCS-2::ABC und *P. putida* GPp104 pBBR1MCS-2::ABC. Dies beweist, dass die Verstärkung von *BTH_II1077- II1080-II1081* aus *B. thailandensis* E264 die Bildung von Mono-und Dirhamnosyllipiden in *P. putida-*Stämmen mit den *Pseudomonas aeruginosa* DSM1707 Genen *rhlABC* verstärkt.
Es wird schliesslich gezeigt, dass die Abschwächung der Polyhydroxybutyratbildung im Stammhintergrund *P. putida* GPp104 gegenüber dem Stamm *P. putida* KT2440 zu einer gesteigerten Rhamnolipidbildung führt, da die Stämme *P. putida* KT2440 pBBR1 MCS-2::AB, *P. putida* KT2440 pBBR1MCS-2::ABC, *P. putida* KT2440 pBBR1MCS-2::AB-BTH_II1077- II1080-II1081 und *P. putida* KT2440 pBBR1MCS-2::ABC-BTH_II1077- II1080-II1081 wesentlich weniger Mono- () bzw. Mono- und Dirhamnosyllipide () zu bilden vermögen als die entsprechenden Kontrollstämme *P. putida* GPp104 pBBR1MCS-2::AB, *P. putida* GPp104 pBBR1MCS-2::ABC, *P. putida* GPp104 pBBR1MCS-2::AB-BTH-II1077- II1080-II1081 und *P. putida* GPp104 pBBR1MCS-2::ABC-BTH_II1077- II1080-II1081.

### 13. Konstruktion eines Vektors pBBR1MCS-2::ABCM zur heterologen Expression der Pseudomonas aeruginosa DSM1707 Gene rhlA, rhlB, pa1131 und rhlC in Pseudomonas putida

Zur heterologen Expression der *Pseudomonas aeruginosa* DSM1707 Gene *rhlA, rhlB, pa1131* und *rhlC* wurde das Plasmid pBBR1MCS-2::ABCM (Seq ID Nr. 58) konstruiert. Dazu wurde das Gen *pa1131* (Seq ID Nr. 59) ausgehend von genomischer DNA des Stammes *Pseudomonas aeruginosa* PAO1 (DSM 1707) mit den Oligonukleotiden
MFS2.0_xbaI_fw: 5'-AGGAAATCTAGATGAGAGGCCGGCAAGGATAC-3' (Seq ID Nr. 60)
MFS2.0_XbaI_rev:5'-CCAGGTTCTAGACGCCAGGATTGAACAGTACC-3' (Seq ID Nr. 61)
amplifiziert. Die Amplifikation des PCR-Produktes (1483 Basenpaare) wurde mit der Phusion™ High-Fidelity Master Mix von New England Biolabs (Frankfurt) Polymerase durchgeführt. Das PCR-Produkt wurde mit *Xba*I geschnitten und in den ebenfalls mit *Xba*I geschnittenen Vektor pBBR1MCS-2::ABC (Seq ID Nr. 40) mittels Fast Link Ligation Kit (Epicentre Technologies; Madison, WI, USA) ligiert. Der erhaltene Zielvektor pBBR1MCS-2::ABCM (Seq ID Nr. 58) hat eine Größe von 9892 Basenpaaren. Das Insert des Vektors wurde sequenziert. Die chromosomale DNA wurde mittels DNeasy Blood and Tissue Kit (Qiagen; Hilden) nach Herstellerangaben isoliert. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgröße, Reinigung der PCR-Produkte und DNA-Konzentrationsbestimmung erfolgte in dem Fachmann bekannter Art und Weise.
Die Transformation von *Pseudomonas putida* KT2440 und GPp104 mit dem Vektor pBBR1 MCS-2::ABCM erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme wurden *P. putida* KT2440 pBBR1 MCS-2::ABCM bzw. *P. putida* GPp104 pBBR1MCS-2::ABCM genannt.

### 14. Quantifizierung der Rhamnolipidproduktion durch rekombinante P. putida-Stämme mit und ohne Überexpression des Pseudomonas aeruginosa DSM1707 pa1131-Gens

Die in den Beispielen 2 und 13 generierten rekombinanten Stämme *P. putida* -Stämme *P. putida* KT2440 pBBR1MCS-2::ABC, *P. putida* KT2440 pBBR1MCS-2::ABCM, *P. putida* KT2440 pBBR1MCS-2::ABC und *P. putida* GPp104 pBBR1MCS-2::ABCM wurden auf LB-Agar-Kanamycin (50 µg/ml)-Platten kultiviert. Die anschliessende Kultivierung zur Produktion der Rhamnolipide erfolgte wie in Beispiel 12 beschrieben.
Die Probenvorbereitung für die nachfolgenden chromatographischen Analysen und die chromatographischen Analysen selbst erfolgten wie in Beispiel 4 beschrieben.
Die Ergebnisse sind in der folgenden Tabelle abgebildet.

Bildung von Di- und Monorhamnosyllipiden durch *P. putida-*Stämme mit und ohne Überexpression des *P. aeruginosa*-Gens *pa1131* nach 48 h Inkubation

| *P. putida*-Stämme | Dirhamnosyllipide [mg/L] | Mono-Rhamnosyllipide [Peakfläche] |
|---|---|---|
| KT2440 pBBR1MCS-2::ABC | 310 | 19 |
| KT2440 pBBR1MCS-2::ABCM | 1053 | 314 |
| GPp104 pBBR1MCS-2::ABC | 689 | 127 |
| GPp104 pBBR1MCS-2::ABCM | 960 | 1090 |

Die Ergebnisse zeigen, dass die Überexpression des *P. aeruginosa-*Gens *pa1131* in beiden Stammhintergründen (KT2440: Wildtyp bzw. GPp104 mit inaktivierter Polyhydroxybutyrat-Bildung) zu einer erhöhten Bildung von Di- und Monorhamnosyllipiden führt. Die Ergebnisse zeigen weiterhin, dass die Abschwächung der Polyhydroxybutyrat-Bildung in GPp104 generell zu einer verstärkten Bildung von Rhamnosyllipiden führt.

### 15. Konstruktion eines Vektors pEC-XT99A::AB zur heterologen Expression der Gene rhlA und rhlB aus Pseudomonas aeruginosa DSM1707 in Corynebacterium glutamicum

Zur heterologen Expression der Gene *rhlA* und *rhlB* aus *Pseudomonas aeruginosa* DSM1707 in *Corynebacterium glutamicum* wird das Plasmid pEC-XT99A::AB (Seq ID Nr. 52) konstruiert. Dazu wurde das synthetische Operon *rhlAB* (Seq ID Nr. 37) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::AB wird das synthetische Operon mittels *Bgl*II und *Xba*I aus dem Vektor geschnitten und anschließend in den mit *Bam*HI und *Xba*I geschnittenen Expressionsvektor pEC-XT99A (US-Patent 7118904) ligiert. Das resultierende Plasmid pEC-XT99A::AB (Seq ID Nr. 52) ist 9793 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Die Transformation von C. *glutamicum* ATCC13032 mit dem Vektor pEC-XT99A::AB erfolgt wie vorbeschrieben (Liebl et al., FEMS Microbiol. Lett. 53:299-303 (1989)). Die Selektion der Transformanten erfolgt auf LBHIS Agar-Platten (18,5 g/L Hirn-Herz-Infusion Boullion, 0.5 M Sorbitol, 5 g/L Bacto-Trypton, 2,5 g/L Bacto-Hefeextrakt, 5 g/L NaCl und 18 g/L Bacto-Agar, supplementiert mit 5 mg/L Tetracyclin). Die Platten wurden zwei Tage bei 33° C inkubiert. Der erhaltene, das Plasmid tragende Stamm, wird C. *glutamicum* pEC-XT99A::AB genannt.

### 16. Konstruktion eines Vektors pEC-XT99A::ABC zur heterologen Expression der Gene rhlA, rhlB und rhlC aus Pseudomonas aeruginosa DSM1707 in Corynebacterium glutamicum

Zur heterologen Expression der Gene *rhlA, rhlB* und *rhlC* aus *Pseudomonas aeruginosa* DSM1707 in *Corynebacterium glutamicum* wird das Plasmid pEC-XT99A::ABC (Seq ID Nr. 53) konstruiert. Dazu wurde das synthetische Operon *rhlABC* (Seq ID Nr. 39) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::ABC wird das synthetische Operon mittels *Bg*/II und *Xba*I aus dem Vektor geschnitten und anschließend in den mit *Bam*HI und *Xba*I geschnittenen Expressionsvektor pEC-XT99A (US-Patent 7118904) ligiert. Das resultierende Plasmid pEC-XT99A::ABC (Seq ID Nr. 53) ist 10780 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Die Transformation von *C. glutamicum* ATCC13032 mit dem Vektor pEC-XT99A::ABC erfolgt wie vorbeschrieben (Liebl et al., FEMS Microbiol. Lett. 53:299-303 (1989)). Die Selektion der Transformanten erfolgt auf LBHIS Agar-Platten (18,5 g/L Hirn-Herz-Infusion Boullion, 0.5 M Sorbitol, 5 g/L Bacto-Trypton, 2,5 g/L Bacto-Hefeextrakt, 5 g/L NaCl und 18 g/L Bacto-Agar, supplementiert mit 5 mg/L Tetracyclin). Die Platten wurden zwei Tage bei 33° C inkubiert. Der erhaltene, das Plasmid tragende Stamm, wird *C. glutamicum* pEC-XT99A::ABC genannt.

### 17. Konstruktion eines Vektors pEC-XT99A::ABM zur heterologen Expression der Gene rhlA, rhlB und pa1131 aus Pseudomonas aeruginosa DSM1707 in Corynebacterium glutamicum

Zur heterologen Expression der Gene *rhlA, rhlB* und *pa1131* aus *Pseudomonas aeruginosa* DSM1707 in *Corynebacterium glutamicum* wird das Plasmid pEC-XT99A::ABM (Seq ID Nr. 54) konstruiert. Dazu wurde das synthetische Operon *rhlABM* (Seq ID Nr. 41) von der Firma GeneArt AG (Regensburg) synthetisiert und im kommerziellen Vektor pMA (GeneArt AG) zwischenkloniert. Grundlage für die Synthese war die bereits bekannte genomische Sequenz des *Pseudomonas aeruginosa* DSM1707. Ausgehend vom Vektor pMA::ABM wird das synthetische Operon mittels *Bg*/II und *Xba*I aus dem Vektor geschnitten und anschließend in den mit *Bam*HI und *Xba*I geschnittenen Expressionsvektor pEC-XT99A (US-Patent 7118904) ligiert. Das resultierende Plasmid pEC-XT99A::ABM (Seq ID Nr. 54) ist 11073 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Die Transformation von *C. glutamicum* ATCC13032 mit dem Vektor pEC-XT99A::ABM erfolgt wie vorbeschrieben (Liebl et al., FEMS Microbiol. Lett. 53:299-303 (1989)). Die Selektion der Transformanten erfolgt auf LBHIS Agar-Platten (18,5 g/L Hirn-Herz-Infusion Boullion, 0.5 M Sorbitol, 5 g/L Bacto-Trypton, 2,5 g/L Bacto-Hefeextrakt, 5 g/L NaCl und 18 g/L Bacto-Agar, supplementiert mit 5 mg/L Tetracyclin). Die Platten wurden zwei Tage bei 33° C inkubiert. Der erhaltene, das Plasmid tragende Stamm, wird *C. glutamicum* pEC-XT99A::ABM genannt.

### 18. Konstruktion eines Vektors pEC-XT99A::ABCM zur heterologen Expression der Gene rhlA, rhlB, pa1131 und rhlC aus Pseudomonas aeruginosa DSM1707 in Corynebacterium glutamicum

Zur heterologen Expression der Gene *rhlA, rhlB, pa1131* und *rhlC* aus *Pseudomonas aeruginosa* DSM1707 in *Corynebacterium glutamicum* wird das Plasmid pEC-XT99A::ABCM (Seq ID Nr. 55) konstruiert. Dazu wurde das Gen *pa1131* (Seq ID Nr. 59) ausgehend von genomischer DNA des Stammes *Pseudomonas aeruginosa* PAO1 (DSM 1707) mit den Oligonukleotiden
MFS2.0_xbaI_fw: 5'-AGGAAATCTAGATGAGAGGCCGGCAAGGATAC-3' (Seq ID Nr. 60)
MFS2.0_XbaI_rev:5'-CCAGGTTCTAGACGCCAGGATTGAACAGTACC-3' (Seq ID Nr. 61)
amplifiziert. Die Amplifikation des PCR-Produktes (1483 Basenpaare) wurde mit der Phusion™ High-Fidelity Master Mix von New England Biolabs (Frankfurt) Polymerase durchgeführt. Das PCR-Produkt wurde mit *Xba*I geschnitten und in den ebenfalls mit *Xba*I geschnittenen Vektor pBBR1MCS-2::ABC (Seq ID Nr. 40) mittels Fast Link Ligation Kit (Epicentre Technologies; Madison, WI, USA) ligiert. Der erhaltene Zielvektor pEC-XT99A::ABCM (Seq ID Nr. 55) hat eine Größe von 12263 Basenpaaren. Das Insert des Vektors wurde sequenziert. Die chromosomale DNA wurde mittels DNeasy Blood and Tissue Kit (Qiagen; Hilden) nach Herstellerangaben isoliert. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgröße, Reinigung der PCR-Produkte und DNA-Konzentrationsbestimmung erfolgte in dem Fachmann bekannter Art und Weise.
Die Transformation von *C. glutamicum* ATCC13032 mit dem Vektor pEC-XT99A::ABCM erfolgt wie vorbeschrieben (Liebl et al., FEMS Microbiol. Lett. 53:299-303 (1989)). Die Selektion der Transformanten erfolgt auf LBHIS Agar-Platten (18,5 g/L Hirn-Herz-Infusion Boullion, 0.5 M Sorbitol, 5 g/L Bacto-Trypton, 2,5 g/L Bacto-Hefeextrakt, 5 g/L NaCl und 18 g/L Bacto-Agar, supplementiert mit 5 mg/L Tetracyclin). Die Platten wurden zwei Tage bei 33° C inkubiert. Der erhaltene, das Plasmid tragende Stamm, wird C. *glutamicum* pEC-XT99A::ABCM genannt.

### 19. Konstruktion eines Vektors pVWEX1::rfbBDAC zur heterologen Expression in C. glutamicum

Zur heterologen Expression der Gene *rfbBDAC* aus *P. putida* unter Kontrolle des lac-Promotors in C. *glutamicum* wird der Vektors pVWEX1::rfbBDAC (Seq ID Nr. 57) konstruiert. Dazu wird der Vektor pBBR1MCS-2::rfbBDAC (Seq ID Nr. 45) mitXbal verdaut und das die Gene *rfbBDAC* aus *P. putida* KT2440 und den *lac*-Promotor enthaltende Fragment (3840 bp)
in den mitXbal verdauten Vektor pVWEX1 (Seq ID Nr. 56) ligiert. Das resultierende Plasmid pVWEX1::rfbBDAC (Seq ID Nr. 57) ist 12311 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Die Transformation von *C. glutamicum* ATCC13032 pEC-XT99A, ATCC13032 pEC-XT99A::AB, ATCC13032 pEC-XT99A::ABM, ATCC13032 pEC-XT99A::ABC und ATCC13032 pEC-XT99A::ABCM mit dem Vektor pVWEX1::rfbBDAC erfolgt wie vorbeschrieben (Liebl et al., FEMS Microbiol. Lett. 53:299-303 (1989)). Die Selektion der Transformanten erfolgt auf LBHIS Agar-Platten (18,5 g/L Hirn-Herz-Infusion Boullion, 0.5 M Sorbitol, 5 g/L Bacto-Trypton, 2,5 g/L Bacto-Hefeextrakt, 5 g/L NaCl und 18 g/L Bacto-Agar, supplementiert mit 5 mg/L Tetracyclin und 25 mg/L Kanamycin). Die Platten wurden zwei Tage bei 33° C inkubiert. Die erhaltenen, die Plasmide tragenden Stämme werden C. *glutamicum* pEC-XT99A pVWEX1::rfbBDAC, C. *glutamicum* pEC-XT99A::AB pVWEX1::rfbBDAC, C. *glutamicum* pEC-XT99A::ABM pVWEX1::rfbBDAC, C. *glutamicum* pEC-XT99A::ABC pVWEX1::rfbBDAC und C. *glutamicum* pEC-XT99A::ABCM pVWEX1::rfbBDAC genannt.

### 20. Quantifizierung der Rhamnolipidproduktion durch rekombinante C. glutamicum-Stämme

Die in den Beispielen 15 bis 19 generierten rekombinanten Stämme C. *glutamicum* -Stämme *C. glutamicum* pEC-XT99A, C. *glutamicum* pEC-XT99A::AB, C. *glutamicum* pEC-XT99A::ABC, *C. glutamicum* pEC-XT99A::ABM, C. *glutamicum* pEC-XT99A::ABCM, C. *glutamicum* pEC-XT99A pVWEX1::rfbBDAC, C. *glutamicum* pEC-XT99A::AB pVWEX1::rfbBDAC, C. *glutamicum* pEC-XT99A::ABM pVWEX1::rfbBDAC, C. *glutamicum* pEC-XT99A::ABC pVWEX1::rfbBDAC und C. *glutamicum* pEC-XT99A::ABCM pVWEX1::rfbBDAC werden auf LBHIS Agar-Platten mit 5 mg/L Tetracyclin bzw. 5 mg/L Tetracyclin und 25 mg/L Kanamycin kultiviert. Zur Untersuchung der Rhamnolipidproduktion im Schüttelkolben werden zunächst Vorkulturen angesetzt. Hierzu wird eine Impföse eines frisch auf einer LBHIS Agar-Platte ausgestrichenen Stammes verwendet und 10 ml LBHIS-Medium (18,5 g/L Hirn-Herz-Infusion Boullion, 0.5 M Sorbitol, 5 g/L Bacto-Trypton, 2,5 g/L Bacto-Hefeextrakt und 5 g/L NaCl, supplementiert mit 5 mg/L Tetracyclin bzw. 5 mg/L Tetracyclin und 25 mg/L Kanamycin) in einem 100 ml Erlenmeyerkolben beimpft. Die Kultivierung der Stämme erfolgt bei 33 °C und 200 rpm über Nacht. Am nächsten Morgen werden 50 ml CGXII-Medium (mit 5 mg/L Tetracyclin bzw. 5 mg/L Tetracyclin und 25 mg/L Kanamycin) in einem 500 ml Erlenmeyerkolben mit Schikanen mit 1 ml der Vorkultur beimpft (Start-OD₆₀₀ 0,1).

### CGXII-Medium:

- 20 g/l (NH₄)₂SO₄ (Merck)
- 5 g/l Harnstoff (Merck)
- 1 g/l KH₂PO₄ (Merck)
- 1 g/l K₂HPO₄ (Merck)
- 0,25 g/l MgSO₄ • 7H₂O (Merck)
- 10 mg/l CaCl₂ (Merck)
- 42 g/l MOPS (Roth)
- 0,2 mg/l Biotin (Merck)
- 1 ml/l Spurensalzlösung
- pH 7 mit NaOH einstellen
- nach dem Autoklavieren 1 ml/l Protokatechusäure (30 g/l in verd. NaOH gelöst, sterilfiltriert) und 40 g/l Glucose (Merck) zugeben

### Spurensalzlösung:

- 10 g/l FeSO₄ • 7H₂O (Merck)
- 10 g/l MnSO₄ • H₂O (Merck)
- 1 g/l ZnSO₄ • 7H₂O (Merck)
- 0,2 g/l CuSO₄ • 5 H₂O (Merck)
- 20 mg/l NiCl₂ • 6 H₂O (Merck)
- zum Lösen mit HCl auf pH 1 ansäuern

Die Kulturen werden bei 200 rpm und 33 °C bis zu einer optischen Dichte (600 nm) von 0,4 - 0,6 kultiviert. Bei dieser optischen Dichte werden die Kulturen durch die Zugabe von IPTG (Isopropyl-β-D-thiogalactopyranosid; 1 mM Endkonzentration) induziert. Die sich anschließende Expression erfolgt ebenfalls bei 33 °C und 200 rpm für 72 h. Im Abstand von 24 h wird eine Probe von 1 ml Brühe dem Kulturkolben entnommen. Die Probenvorbereitung für die nachfolgenden chromatographischen Analysen und die chromatographischen Analysen selbst erfolgen wie in Beispiel 4 beschrieben.

Während C. *glutamicum* pEC-XT99A keine Rhamnolipide produziert, ist in den rekombinanten Stämmen C. *glutamicum* pEC-XT99A::AB, C. *glutamicum* pEC-XT99A::ABC, C. *glutamicum* pEC-XT99A::ABM und C. *glutamicum* pEC-XT99A::ABCM die Bildung von Rhamnolipiden nachweisbar. Anhand von Referenzmaterialien wird gezeigt, dass C. *glutamicum* pEC-XT99A::AB und C. *glutamicum* pEC-XT99A::ABM lediglich Monorhamnosyllipide bilden, während C. *glutamicum* pEC-XT99A::ABC, C. *glutamicum* pEC-XT99A::ABM und C. *glutamicum* pEC-XT99A::ABCM Dirhamnosyllipide und Monorhamnosyllipide zu bilden vermögen. Weiterhin wird gezeigt, dass C. *glutamicum* pEC-XT99A::ABM und C. *glutamicum* pEC-XT99A::ABCM mehr Monorhamnosyllipide bzw. Dirhamnosyllipide und Monorhamnosyllipide zu bilden vermögen als die jeweiligen Referenzstämme C. *glutamicum* pEC-XT99A::AB und C. *glutamicum* pEC-XT99A::ABC ohne Verstärkung des *pa1131*-Gens aus *Pseudomonas aeruginosa.*

Darüber hinaus wird gezeigt, dass die Stämme C. *glutamicum* pEC-XT99A::AB pVWEX1::rfbBDAC, C. *glutamicum* pEC-XT99A::ABM pVWEX1::rfbBDAC, C. *glutamicum* pEC-XT99A::ABC pVWEX1::rfbBDAC und C. *glutamicum* pEC-XT99A::ABCM pVWEX1::rfbBDAC wesentlich mehr Mono- (C. *glutamicum* pEC-XT99A::AB pVWEX1::rfbBDAC und C. *glutamicum* pEC-XT99A::ABM pVWEX1::rfbBDAC) bzw. Mono- und Dirhamnosyllipide (C. *glutamicum* pEC-XT99A::ABC pVWEX1::rfbBDAC und C. *glutamicum* pEC-XT99A::ABCM pVWEX1::rfbBDAC) bilden als die Stämme, C. *glutamicum* pEC-XT99A::ABM, C. *glutamicum* pEC-XT99A::ABC und *C. glutamicum* pEC-XT99A::ABCM ohne Verstärkung des der *rfbBDA-Gene* aus *P. putida.*

### 21. Konstruktion von Pseudomonas-Stämmen, die die Plasmide pBBR1MCS-2, pBBR1MCS-2::AB, pBBR1MCS-2::ABC, pBBR1MCS-2::ABM und pBBR1MCS-2::ABCM tragen

Die Plasmide pBBR1MCS-2, pBBR1MCS-2::AB, pBBR1MCS-2::ABC, pBBR1MCS-2::ABM und pBBR1MCS-2::ABCM werden in *Pseudomonas fluorescens* DSM 50090, *Pseudomonas fluorescens* DSM 9958, *Pseudomonas putida* DSM 6899, *Pseudomonas putida* DSM 50204, *Pseudomonas putida* 50194, *P. brassicacearum* DSM 13227, *P. stutzeri* DSM 10701, *Pseudomonas stutzeri* DSM 4166 und *Pseudomonas* fulva DSM 17717 per Elektroporation eingebracht. Die Transformation von *Pseudomonas-*Stämmen erfolgt wie vorbeschrieben (Iwasaki et al. Biosci. Biotech. Biochem. 1994. 58(5):851-854). Die Selektion der Transformanten erfolgt auf Nutrient-Agar-Platten (5 g/L Pepton; 3 g/L Fleischextrakt; 15 g/L Agar; pH 7; supplementiert mit 50 mg/L Kanamycin). Die Platten werden zwei Tage bei 30° C respektive 28 °C inkubiert. Die erhaltenen, die Plasmide tragenden Stämme, werden *Pseudomonas fluorescens* DSM 50090 pBBR1MCS-2, *Pseudomonas fluorescens* DSM 9958 pBBR1MCS-2, *Pseudomonas putida* DSM 6899 pBBR1MCS-2, *Pseudomonas putida* DSM 50204 pBBR1MCS-2, *Pseudomonas putida* 50194 pBBR1MCS-2, *P. brassicacearum* DSM 13227 pBBR1MCS-2, *P. stutzeri* DSM 10701 pBBR1MCS-2, *Pseudomonas stutzeri* DSM 4166 pBBR1MCS-2, *Pseudomonas* fulva DSM 17717 pBBR1MCS-2, *Pseudomonas fluorescens* DSM 50090 pBBR1MCS-2::AB, *Pseudomonas fluorescens* DSM 9958 pBBR1MCS-2::AB, *Pseudomonas putida* DSM 6899 pBBR1MCS-2::AB, *Pseudomonas putida* DSM 50204 pBBR1MCS-2::AB, *Pseudomonas putida* 50194 pBBR1MCS-2::AB, *P. brassicacearum* DSM 13227 pBBR1MCS-2::AB, *P. stutzeri* DSM 10701 pBBR1MCS-2::AB, *Pseudomonas stutzeri* DSM 4166 pBBR1MCS-2::AB, *Pseudomonas* fulva DSM 17717 pBBR1MCS-2::AB, *Pseudomonas fluorescens* DSM 50090 pBBR1MCS-2::ABC, *Pseudomonas fluorescens* DSM 9958 pBBR1MCS-2::ABC, *Pseudomonas putida* DSM 6899 pBBR1MCS-2::ABC, *Pseudomonas putida* DSM 50204 pBBR1MCS-2::ABC, *Pseudomonas putida* 50194 pBBR1MCS-2::ABC, *P. brassicacearum* DSM 13227 pBBR1MCS-2::ABC, *P. stutzeri* DSM 10701 pBBR1MCS-2::ABC, *Pseudomonas stutzeri* DSM 4166 pBBR1MCS-2::ABC, *Pseudomonas* fulva DSM 17717 pBBR1MCS-2::ABC, *Pseudomonas fluorescens* DSM 50090 pBBR1MCS-2::ABCM, *Pseudomonas fluorescens* DSM 9958 pBBR1MCS-2::ABCM, *Pseudomonas putida* DSM 6899 pBBR1MCS-2::ABCM, *Pseudomonas putida* DSM 50204 pBBR1MCS-2::ABCM, *Pseudomonas putida* 50194 pBBR1MCS-2::ABCM, *P. brassicacearum* DSM 13227 pBBR1MCS-2::ABCM, *P. stutzeri* DSM 10701 pBBR1MCS-2::ABCM, *Pseudomonas stutzeri* DSM 4166 pBBR1MCS-2::ABCM, *Pseudomonas* fulva DSM 17717 pBBR1MCS-2::ABCM, *Pseudomonas fluorescens* DSM 50090 pBBR1MCS-2::ABM, *Pseudomonas fluorescens* DSM 9958 pBBR1MCS-2::ABM, *Pseudomonas putida* DSM 6899 pBBR1MCS-2::ABM, *Pseudomonas putida* DSM 50204 pBBR1MCS-2::ABM, *Pseudomonas putida* 50194 pBBR1MCS-2::ABM, *P. brassicacearum* DSM 13227 pBBR1MCS-2::ABM, *P. stutzeri* DSM 10701 pBBR1MCS-2::ABM, *Pseudomonas stutzeri* DSM 4166 pBBR1MCS-2::ABM und *Pseudomonas* fulva DSM 17717 pBBR1MCS-2::ABM genannt.

### 22. Quantifizierung der Rhamnolipidproduktion durch rekombinante Pseudomonas-Stämme

Die in Beispiel 21 generierten rekombinanten Stämme *Pseudomonas-Stämme Pseudomonas fluorescens* DSM 50090, *Pseudomonas fluorescens* DSM 9958 pBBR1MCS-2, *Pseudomonas putida* DSM 6899 pBBR1MCS-2, *Pseudomonas putida* DSM 50204 pBBR1MCS-2, *Pseudomonas putida* 50194 pBBR1MCS-2, *P. brassicacearum* DSM 13227 pBBR1MCS-2, *P. stutzeri* DSM 10701 pBBR1MCS-2, *Pseudomonas stutzeri* DSM 4166 pBBR1MCS-2, *Pseudomonas* fulva DSM 17717 pBBR1MCS-2, *Pseudomonas fluorescens* DSM 50090 pBBR1MCS-2::AB, *Pseudomonas fluorescens* DSM 9958 pBBR1MCS-2::AB, *Pseudomonas putida* DSM 6899 pBBR1MCS-2::AB, *Pseudomonas putida* DSM 50204 pBBR1MCS-2::AB, *Pseudomonas putida* 50194 pBBR1MCS-2::AB, *P. brassicacearum* DSM 13227 pBBR1MCS-2::AB, *P. stutzeri* DSM 10701 pBBR1MCS-2::AB, *Pseudomonas stutzeri* DSM 4166 pBBR1MCS-2::AB, *Pseudomonas* fulva DSM 17717 pBBR1MCS-2::AB, *Pseudomonas fluorescens* DSM 50090 pBBR1MCS-2::ABC, *Pseudomonas fluorescens* DSM 9958 pBBR1MCS-2::ABC, *Pseudomonas putida* DSM 6899 pBBR1MCS-2::ABC, *Pseudomonas putida* DSM 50204 pBBR1MCS-2::ABC, *Pseudomonas putida* 50194 pBBR1MCS-2::ABC, *P. brassicacearum* DSM 13227 pBBR1MCS-2::ABC, *P. stutzeri* DSM 10701 pBBR1MCS-2::ABC, *Pseudomonas stutzeri* DSM 4166 pBBR1MCS-2::ABC, *Pseudomonas* fulva DSM 17717 pBBR1MCS-2::ABC, *Pseudomonas fluorescens* DSM 50090 pBBR1MCS-2::ABCM, *Pseudomonas fluorescens* DSM 9958 pBBR1MCS-2::ABCM, *Pseudomonas putida* DSM 6899 pBBR1MCS-2::ABCM, *Pseudomonas putida* DSM 50204 pBBR1MCS-2::ABCM, *Pseudomonas putida* 50194 pBBR1MCS-2::ABCM, *P. brassicacearum* DSM 13227 pBBR1MCS-2::ABCM, *P. stutzeri* DSM 10701 pBBR1MCS-2::ABCM, *Pseudomonas stutzeri* DSM 4166 pBBR1MCS-2::ABCM, *Pseudomonas* fulva DSM 17717 pBBR1MCS-2::ABCM, *Pseudomonas fluorescens* DSM 50090 pBBR1MCS-2::ABM, *Pseudomonas fluorescens* DSM 9958 pBBR1MCS-2::ABM, *Pseudomonas putida* DSM 6899 pBBR1MCS-2::ABM, *Pseudomonas putida* DSM 50204 pBBR1MCS-2::ABM, *Pseudomonas putida* 50194 pBBR1MCS-2::ABM, *P. brassicacearum* DSM 13227 pBBR1MCS-2::ABM, *P. stutzeri* DSM 10701 pBBR1MCS-2::ABM, *Pseudomonas stutzeri* DSM 4166 pBBR1MCS-2::ABM und *Pseudomonas* fulva DSM 17717 pBBR1MCS-2::ABM werden auf LB-Agar-Kanamycin (50 µg/ml)-Platten kultiviert. Die anschliessende Kultivierung zur Produktion der Rhamnolipide erfolgt wie in Beispiel 12 beschrieben. Die Probenvorbereitung für die nachfolgenden chromatographischen Analysen und die chromatographischen Analysen selbst erfolgen wie in Beispiel 4 beschrieben.

Während die *Pseudomonas-Stämme Pseudomonas fluorescens* DSM 50090, *Pseudomonas fluorescens* DSM 9958 pBBR1MCS-2, *Pseudomonas putida* DSM 6899 pBBR1MCS-2, *Pseudomonas putida* DSM 50204 pBBR1MCS-2, *Pseudomonas putida* 50194 pBBR1MCS-2, *P. brassicacearum* DSM 13227 pBBR1MCS-2, *P. stutzeri* DSM 10701 pBBR1MCS-2, *Pseudomonas stutzeri* DSM 4166 pBBR1MCS-2, *Pseudomonas* fulva DSM 17717 pBBR1MCS-2 keine Rhamnolipide produzieren, ist in den rekombinanten Stämmen *Pseudomonas fluorescens* DSM 50090 pBBR1MCS-2::AB, *Pseudomonas fluorescens* DSM 9958 pBBR1MCS-2::AB, *Pseudomonas putida* DSM 6899 pBBR1MCS-2::AB, *Pseudomonas putida* DSM 50204 pBBR1MCS-2::AB, *Pseudomonas putida* 50194 pBBR1MCS-2::AB, *P. brassicacearum* DSM 13227 pBBR1MCS-2::AB, *P. stutzeri* DSM 10701 pBBR1MCS-2::AB, *Pseudomonas stutzeri* DSM 4166 pBBR1MCS-2::AB, *Pseudomonas* fulva DSM 17717 pBBR1MCS-2::AB, *Pseudomonas fluorescens* DSM 50090 pBBR1MCS-2::ABM, *Pseudomonas fluorescens* DSM 9958 pBBR1MCS-2::ABM, *Pseudomonas putida* DSM 6899 pBBR1MCS-2::ABM, *Pseudomonas putida* DSM 50204 pBBR1MCS-2::ABM, *Pseudomonas putida* 50194 pBBR1MCS-2::ABM, *P. brassicacearum* DSM 13227 pBBR1MCS-2::ABM, *P. stutzeri* DSM 10701 pBBR1MCS-2::ABM, *Pseudomonas stutzeri* DSM 4166 pBBR1MCS-2::ABM und *Pseudomonas* fulva DSM 17717 pBBR1MCS-2::ABM die Bildung von Monorhamnosyllipiden sowie in den Stämmen *Pseudomonas fluorescens* DSM 50090 pBBR1MCS-2::ABC, *Pseudomonas fluorescens* DSM 9958 pBBR1MCS-2::ABC, *Pseudomonas putida* DSM 6899 pBBR1MCS-2::ABC, *Pseudomonas putida* DSM 50204 pBBR1MCS-2::ABC, *Pseudomonas putida* 50194 pBBR1MCS-2::ABC, *P. brassicacearum* DSM 13227 pBBR1MCS-2::ABC, *P. stutzeri* DSM 10701 pBBR1MCS-2::ABC, *Pseudomonas stutzeri* DSM 4166 pBBR1MCS-2::ABC, *Pseudomonas* fulva DSM 17717 pBBR1MCS-2::ABC, *Pseudomonas fluorescens* DSM 50090 pBBR1MCS-2::ABCM, *Pseudomonas fluorescens* DSM 9958 pBBR1MCS-2::ABCM, *Pseudomonas putida* DSM 6899 pBBR1MCS-2::ABCM, *Pseudomonas putida* DSM 50204 pBBR1MCS-2::ABCM, *Pseudomonas putida* 50194 pBBR1MCS-2::ABCM, *P. brassicacearum* DSM 13227 pBBR1MCS-2::ABCM, *P. stutzeri* DSM 10701 pBBR1MCS-2::ABCM, *Pseudomonas stutzeri* DSM 4166 pBBR1MCS-2::ABCM und *Pseudomonas* fulva DSM 17717 pBBR1MCS-2::ABCM die Bildung von Mono- und Dirhamnosyllipiden feststellbar.

Darüberhinaus werden durch die rekombinanten *Pseudomonas-*Stämme *Pseudomonas fluorescens* DSM 50090 pBBR1MCS-2::ABM, *Pseudomonas fluorescens* DSM 9958 pBBR1MCS-2::ABM, *Pseudomonas putida* DSM 6899 pBBR1MCS-2::ABM, *Pseudomonas putida* DSM 50204 pBBR1MCS-2::ABM, *Pseudomonas putida* 50194 pBBR1MCS-2::ABM, *P. brassicacearum* DSM 13227 pBBR1MCS-2::ABM, *P. stutzeri* DSM 10701 pBBR1MCS-2::ABM, *Pseudomonas stutzeri* DSM 4166 pBBR1MCS-2::ABM *Pseudomonas* fulva DSM 17717 pBBR1MCS-2::ABM weniger Mono-Rhamnosyllipide bzw. durch die rekombinanten *Pseudomonas-Stämme Pseudomonas fluorescens* DSM 50090 pBBR1MCS-2::ABCM, *Pseudomonas fluorescens* DSM 9958 pBBR1MCS-2::ABCM, *Pseudomonas putida* DSM 6899 pBBR1MCS-2::ABCM, *Pseudomonas putida* DSM 50204 pBBR1MCS-2::ABCM, *Pseudomonas putida* 50194 pBBR1MCS-2::ABCM, *P. brassicacearum* DSM 13227 pBBR1MCS-2::ABCM, *P. stutzeri* DSM 10701 pBBR1MCS-2::ABCM, *Pseudomonas stutzeri* DSM 4166 pBBR1MCS-2::ABCM und *Pseudomonas* fulva DSM 17717 pBBR1MCS-2::ABCM weniger Mono-und Dirhamnosyllipide gebildet als durch die jeweiligen Referenzstämme ohne das *P. aeruginosa-*Gen *pa1131 Pseudomonas fluorescens* DSM 50090 pBBR1MCS-2::AB, *Pseudomonas fluorescens* DSM 9958 pBBR1MCS-2::AB, *Pseudomonas putida* DSM 6899 pBBR1MCS-2::AB, *Pseudomonas putida* DSM 50204 pBBR1MCS-2::AB, *Pseudomonas putida* 50194 pBBR1MCS-2::AB, *P. brassicacearum* DSM 13227 pBBR1MCS-2::AB, *P. stutzeri* DSM 10701 pBBR1MCS-2::AB, *Pseudomonas stutzeri* DSM 4166 pBBR1MCS-2::AB und *Pseudomonas* fulva DSM 17717 pBBR1MCS-2::AB bzw. *Pseudomonas fluorescens* DSM 50090 pBBR1MCS-2::ABC, *Pseudomonas fluorescens* DSM 9958 pBBR1MCS-2::ABC, *Pseudomonas putida* DSM 6899 pBBR1MCS-2::ABC, *Pseudomonas putida* DSM 50204 pBBR1MCS-2::ABC, *Pseudomonas putida* 50194 pBBR1MCS-2::ABC, *P. brassicacearum* DSM 13227 pBBR1MCS-2::ABC, *P. stutzeri* DSM 10701 pBBR1MCS-2::ABC, *Pseudomonas stutzeri* DSM 4166 pBBR1MCS-2::ABC und *Pseudomonas* fulva DSM 17717 pBBR1MCS-2::ABC ohne Verstärkung des *pa1131*-Gens aus *Pseudomonas aeruginosa.*

### 23. Konstruktion der Vektoren pBBR1MCS-2::ABPAO1-C1 und pBBR1MCS-2::ABPA7-CE264 zur heterologen Expression alternativer rhlA-, rhlB- und rhlC-Gene aus Pseudomonas aeruginosa PAO1, Pseudomonas aeruginosa PA7, Pseudomonas aeruginosa 1 und Burkholderia thailandensis E264 in P. putida

Zur heterologen Expression der Gene *rhlA, rhlB* und *rhlC* aus *Pseudomonas aeruginosa* PAO1 bzw. *Pseudomonas aeruginosa* PA7 werden zunächst die Plasmide pBBR1MCS-2::ABPAO1 (Seq ID Nr. 62) und pBBR1MCS-2::ABPA7 (Seq ID Nr. 63) konstruiert. Dazu werden die synthetischen Operons *rhlABPAO1* (Seq ID Nr.64) und *rhlABPA7* (Seq ID Nr. 65) von der Firma DNA 2.0 (Menlo Park, CA, U.S.A) synthetisiert und im kommerziellen Vektor pJ294 (DNA 2.0) zwischenkloniert. Grundlage für die Synthese ist die bereits bekannte genomische Sequenz der Stämme *Pseudomonas aeruginosa* PAO1 und *Pseudomonas aeruginosa* PA7. Ausgehend von den Vektoren pJ294::ABPAO1 und pJ294::ABPA7 werden die synthetischen Operons mittels *Kpn*I und *Xba*I aus den Vektoren geschnitten und anschließend in den mit *Kpn*I und *Xba*I geschnittenen Expressionsvektor pBBR1MCS-2 (Seq ID Nr. 49) (Kovach et al., 1995: Four new derivatives of the broad-host-range cloning vector pBBR1MCS carrying different antibiotic-resistance cassettes. Gene, 166:175-176) ligiert. Die resultierenden Plasmide pBBR1MCS-2::ABPAO1 (Seq ID Nr. 62) und pBBR1MCS-2::ABPA7 (Seq ID Nr. 63) sind 7332 bzw. 7354 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Im zweiten Schritt werden die Plasmide pBBR1MCS-2::ABPAO1-C1 (Seq ID Nr. 66) und pBBR1MCS-2::ABPA7-CE264 (Seq ID Nr. 67) erzeugt. Dazu werden die *rhlC*-Gene aus *Pseudomonas aeruginosa* 1 (Seq ID Nr. 68) und *Burkholderia thailandensis* E264 (Seq ID Nr. 76) von der Firma DNA 2.0 (Menlo Park, CA, U.S.A) synthetisiert und im kommerziellen Vektor pJ294 (DNA 2.0) zwischenkloniert. Grundlage für die Synthese ist die bereits bekannte genomische Sequenz der Stämme *Pseudomonas aeruginosa* 1 und *Burkholderia thailandensis* E264. Ausgehend von den Vektoren pJ294::C1 und pJ294::CE264 werden die *rhlC*-Gene mittels *Xba* und *Sac*I aus den Vektoren geschnitten und anschließend in die ebenfalls mit *Xba* und Sacl geschnittenen Vektoren pBBR1MCS-2::ABPAO1 (Seq ID Nr. 62) bzw. pBBR1MCS-2::ABPA7 (Seq ID Nr. 63) ligiert. Die resultierenden Plasmide pBBR1MCS-2::ABPAO1-C1 (Seq ID Nr. 66) und pBBR1MCS-2::ABPA7-CE264 (Seq ID Nr. 67) sind 8325 bzw. 8335 Basenpaare groß. Die Ligation sowie die Transformation chemisch kompetenter *E. coli* DH5α-Zellen (Gibco-BRL, Karlsruhe) erfolgt in dem Fachmann bekannter Art und Weise. Die Authentizität des Inserts wird durch DNA-Sequenzanalyse überprüft.
Die Transformation von *Pseudomonas putida* KT2440 und GPp104 mit den Vektoren pBBR1MCS-2, pBBR1MCS-2::ABPAO1-C1 und pBBR1MCS-2::ABPA7-CE264 erfolgt wie vorbeschrieben (Iwasaki et al. Biosci. Biotech. Biochem. 1994. 58(5):851-854). Die Plasmid-DNA von je 10 Klonen werden isoliert und analysiert. Die erhaltenen, die Plasmide tragenden Stämme werden *P. putida* KT2440 pBBR1MCS-2, *P. putida* KT2440 pBBR1MCS-2::ABPAO1-C1, *P. putida* KT2440 pBBR1MCS-2::ABPA7-CE264, *P. putida* GPp104 pBBR1MCS-2, *P. putida* GPp104 pBBR1MCS-2::ABPAO1-C1 und *P. putida* GPp104 pBBR1MCS-2::ABPA7-CE264 genannt.

### 24. Quantifizierung der Rhamnolipidproduktion durch rekombinante P. putida-Stämme mit alternativen rhlA-, rhlB- und rhlC-Genen aus Pseudomonas aeruginosa PAO1, Pseudomonas aeruginosa PA7, Pseudomonas aeruginosa 1 und Burkholderia thailandensis E264

Die in Beispiel 23 generierten rekombinanten Stämme *P. putida* -Stämme werden auf LB-Agar-Kanamycin (50 µg/ml)-Platten kultiviert. Die anschliessende Kultivierung zur Produktion der Rhamnolipide erfolgt wie in Beispiel 12 beschrieben. Die Probenvorbereitung für die nachfolgenden chromatographischen Analysen und die chromatographischen Analysen selbst erfolgen wie in Beispiel 4 beschrieben.

Während die Stämme *P. putida* KT2440 pBBR1MCS-2 und *P. putida* GPp104 pBBR1MCS-2 nicht in der Lage sind Mono- und Dirhamnosyllipide zu produzieren, bilden die Stämme *P*. *putida* KT2440 pBBR1MCS-2::ABPAO1-C1, *P. putida* KT2440 pBBR1MCS-2::ABPA7-CE264, *P. putida* GPp104 pBBR1MCS-2::ABPAO1-C1 und *P. putida* GPp104 pBBR1MCS-2::ABPA7-CE264 sowohl Mono- als auch Dirhamnosyllipide. Es wird gezeigt, dass die Stämme mit Abschwächung der Polyhydroxybutyrat-Bildung *(P. putida* GPp104 pBBR1MCS-2::ABPAO1-C1 und *P. putida* GPp104 pBBR1MCS-2::ABPA7-CE264) mehr Mono- und Dirhamnosyllipide zu produzieren vermögen, als die Stämme ohne Abschwächung der Polyhydroxybutyrat-Bildung *(P. putida* KT2440 pBBR1MCS-2::ABPAO1-C1 und *P. putida* KT2440 pBBR1MCS-2::ABPA7-CE264).

### 25. Konstruktion der Vektoren pBBR1MCS-2::AB_rfbBDAC, pBBR1MCS-2::ABM_rfbBDAC und pBBR1MCS-2::ABMC_rfbBDAC zur Überexpression des P. putida rfbBDAC-Operons in P. putida und E. coli

Für die Konstruktion der Vektoren pBBR1MCS-2::AB_rfbBDAC, pBBR1MCS-2::ABM_rfbBDAC und pBBR1MCS-2::ABMC_rfbBDAC zur Überexpression des *P. putida* rfbBDAC-Operons in *P. putida* und *E. coli* wurde zunächst das *P. putida rfbBDAC-*Operon per PCR amplifiziert. Der Vektor pBBR1MCS-2::rfbBDAC (Seq ID Nr. 45) diente als Matrize für eine PCR. Es wurden folgende Oligonukleotide verwendet:
RL_Agel-fw: 5'-TATATATAACCGGTATTAATGCAGCTGGCACGAC-3' (Seq ID Nr. 71)
RL_Agel_rev: 5'-GGCCGACCGGTACTAGTGGA-3' (Seq ID Nr. 72)

Die PCR wurde mit der Phusion™ High-Fidelity Master Mix von New England Biolabs (Frankfurt) Polymerase durchgeführt. Sie erfolgte in dem Fachmann bekannter Art und Weise. Die Zielsequenz (*lac*-Promotor und *rfbBDAC*) wurde ins Trenzyme Alligator Cloning System zwischenkloniert. *E. coli* DH5α (New England Biolabs; Frankfurt) Transformanten wurden selektioniert und die Plasmid-DNA verschiedener Kandidaten isoliert und sequenziert. Nachdem die Sequenz überprüft und auf die Richtigkeit untersucht worden ist, wurde der Vektor mit *Age*I geschnitten. Das Zielfragment wurde in die ebenfalls mit *Age*I geschnittenen Vektoren pBBR1MCS-2::AB (Seq ID Nr. 38), pBBR1MCS-2::ABM (Seq ID Nr. 42) und pBBR1MCS-2::ABMC (Seq ID Nr. 51) mittels herkömmlichen Ligierungsmethoden ligiert. Die resultierenden Vektoren pBBR1MCS-2::AB_rfbBDAC (Seq ID Nr. 73), pBBR1MCS-2::ABM_rfbBDAC (Seq ID Nr. 74) und pBBR1MCS-2::ABMC_rfbBDAC (Seq ID Nr. 75) haben Größen von 11960, 13289 bzw. 14250 Basenpaare. Die Inserts der Vektoren wurden sequenziert. Die Durchführung der PCR, die Überprüfung der erfolgreichen Amplifikation der PCR mittels Agarosegel-Elektrophorese, Ethidiumbromidfärbung der DNA, Bestimmung der PCR-Fragmentgröße, Reinigung der PCR-Produkte und DNA-Konzentrationsbestimmung erfolgte in dem Fachmann bekannter Art und Weise.
Die Transformation von *Pseudomonas putida* KT2440 mit den Vektoren pBBR1MCS-2::AB_rfbBDAC, pBBR1MCS-2::ABM_rfbBDAC und pBBR1MCS-2::ABMC_rfbBDAC erfolgte wie vorbeschrieben (Iwasaki et al. Biosci. Biotech. Biochem. 1994. 58(5):851-854). Die Plasmid-DNA von je 10 Klonen wurde isoliert und analysiert. Die erhaltenen, die Plasmide tragenden Stämme werden *P. putida* KT2440 pBBR1MCS-2::AB_rfbBDAC, *P. putida* KT2440 pBBR1MCS-2::ABM_rfbBDAC und *P. putida* KT2440 pBBR1MCS-2::ABMC_rfbBDAC genannt.

### 26. Quantifizierung der Rhamnolipidproduktion durch rekombinante P. putida KT2440 pBBR1MCS-2::AB_rfbBDAC, P. putida KT2440 pBBR1MCS-2::ABM_rfbBDAC, P. putida KT2440 pBBR1MCS-2::ABC_rfbBDAC, P. putida KT2440 pBBR1MCS-2::-ABMC_rfbBDAC, P. putida KT2440 pBBR1MCS-2::AB, P. putida KT2440 pBBR1MCS-2::ABM, P. putida KT2440 pBBR1MCS-2::ABC und P. putida KT2440 pBBR1MCS-2::ABMC

Die in den Beispielen 2, 7 und 25 generierten rekombinanten Stämme *P. putida* -Stämme werden auf LB-Agar-Kanamycin (50 µg/ml)-Platten kultiviert. Die anschliessende Kultivierung zur Produktion der Rhamnolipide erfolgt wie in Beispiel 12 beschrieben. Die Probenvorbereitung für die nachfolgenden chromatographischen Analysen und die chromatographischen Analysen selbst erfolgen wie in Beispiel 4 beschrieben.

Es wird gezeigt, dass *P. putida* KT2440 pBBR1MCS-2::AB_rfbBDAC, *P. putida* KT2440 pBBR1MCS-2::ABM_rfbBDAC, *P. putida* KT2440 pBBR1MCS-2::AB und *P. putida* KT2440 pBBR1MCS-2::ABM in der Lage sind Monorhamnosyllipide zu bilden, während *P. putida* KT2440 pBBR1MCS-2::ABMC_rfbBDAC, *P. putida* KT2440 pBBR1MCS-2::ABC_rfbBDAC, *P. putida* KT2440 pBBR1MCS-2::ABC und *P. putida* KT2440 pBBR1MCS-2::ABMC in der Lage sind Mono- und Dirhamnosyllipide zu bilden.
Weiterhin wird gezeigt, dass *P. putida* KT2440 pBBR1MCS-2::ABM_rfbBDAC, *P. putida* KT2440 pBBR1MCS-2::ABM, KT2440 pBBR1MCS-2::ABMC_rfbBDAC und KT2440 pBBR1MCS-2::ABMC mehr Mono- und Dirhamnosyllipide zu bilden vermögen als die entsprechenden Kontrollstämme *P. putida* KT2440 pBBR1MCS-2::AB_rfbBDAC, *P. putida* KT2440 pBBR1MCS-2::AB, KT2440 pBBR1MCS-2::ABC_rfbBDAC und KT2440 pBBR1MCS-2::ABC ohne Verstärkung des *Pseudomonas aeruginosa-Gens pa1131.*

Schließlich wird gezeigt, dass *P. putida* KT2440 pBBR1MCS-2::AB_rfbBDAC, *P. putida* KT2440 pBBR1MCS-2::ABM_rfbBDAC, *P. putida* KT2440 pBBR1MCS-2::ABC_rfbBDAC, *P. putida* KT2440 pBBR1MCS-2::ABMC_rfbBDAC mehr Mono- *(P. putida* KT2440 pBBR1MCS-2::AB_rfbBDAC und *P. putida* KT2440 pBBR1MCS-2::ABM_rfbBDAC) bzw. Mono- und Dirhamnosyllipide *(P. putida* KT2440 pBBR1MCS-2::ABC_rfbBDAC und *P. putida* KT2440 pBBR1MCS-2::ABMC_rfbBDAC) zu bilden vermögen als die jeweiligen Kontrollstämme *P. putida* KT2440 pBBR1MCS-2::AB, *P. putida* KT2440 pBBR1MCS-2::ABM, *P. putida* KT2440 pBBR1MCS-2::ABC, *P. putida* KT2440 pBBR1MCS-2::ABMC ohne Verstärkung der *P. putida-*Gene *rfbBDAC.*

### 27. Generierung rekombinanter E. coli W3110 pBBR1MCS-2::AB, E. coli W3110 pBBR1MCS-2::ABM, E. coli W3110 pBBR1MCS-2::ABC, E. coli W3110 pBBR1MCS-2::ABCM, E. coli W3110 pBBR1MCS-2::AB_rfbBDAC, E. coli W3110 pBBR1MCS-2::ABM_rfbBDAC, E. coli W3110 pBBR1MCS-2::ABC_rfbBDAC und E. coli W3110 pBBR1MCS-2::ABCM_rfbBDAC

Die Transformation von *E. coli* W3110 erfolgte wie vorbeschrieben (Miller JH. A Short Course in Bacterial Genetics: A Laboratory Manual and Handbook for Escherichia coli and Related Bacteria. Plainview, NY: Cold Spring Harbor Lab. Press; 1992) mittels Elektroporation. Die Plasmid-DNA von je 10 Klonen wurde isoliert und analysiert. Die erhaltenen, die Plasmide tragenden Stämme wurden *E. coli* W3110 pBBR1MCS-2::AB, E. coli W3110 pBBR1MCS-2::ABM, *E. coli* W3110 pBBR1MCS-2::ABC, E. coli W3110 pBBR1MCS-2::ABCM, *E. coli* W3110 pBBR1MCS-2::AB_rfbBDAC, E. coli W3110 pBBR1MCS-2::ABM_rfbBDAC, *E. coli* W3110 pBBR1MCS-2::ABC_rfbBDAC *und* E*.* coli W3110 pBBR1MCS-2::ABCM_rfbBDAC genannt.

### 28. Quantifizierung der Rhamnolipidproduktion durch rekombinante E. coli W3110 pBBR1MCS-2::AB, E. coli W3110 pBBR1MCS-2::ABM, E. coli W3110 pBBR1MCS-2::ABC, E. coli W3110 pBBR1MCS-2::ABCM, E. coli W3110 pBBR1MCS-2::AB_rfbBDAC, E. coli W3110 pBBR1MCS-2::ABM_rfbBDAC, E. coli W3110 pBBR1MCS-2::ABC_rfbBDAC und E. coli W3110 pBBR1MCS-2::ABCM_rfbBDAC

Die in Beispiel 27 generierten rekombinanten *E. coli* -Stämme werden auf LB-Agar-Kanamycin (50 µg/ml)-Platten kultiviert. Die anschliessende Kultivierung zur Produktion der Rhamnolipide erfolgt wie in Beispiel 10 beschrieben. Die Probenvorbereitung für die nachfolgenden chromatographischen Analysen und die chromatographischen Analysen selbst erfolgen wie in Beispiel 4 beschrieben.

Es wird gezeigt, dass *E. coli* W3110 pBBR1MCS-2::AB, *E. coli* W3110 pBBR1MCS-2::ABM, *E*. *coli* W3110 pBBR1MCS-2::AB_rfbBDAC und E. coli W3110 pBBR1MCS-2::ABM_rfbBDAC in der Lage sind Monorhamnosyllipide zu bilden, während *E. coli W3110* pBBR1MCS-2::ABC, E. coli W3110 pBBR1MCS-2::ABCM, *E. coli* W3110 pBBR1MCS-2::ABC_rfbBDAC *und* E. coli W3110 pBBR1MCS-2::ABCM_rfbBDAC in der Lage sind Mono- und Dirhamnosyllipide zu bilden. Weiterhin wird gezeigt, dass *E. coli* W3110 pBBR1MCS-2::ABM und *E. coli W3110* pBBR1MCS-2::ABM_rfbBDAC mehr Monorhamnosyllipide bilden als *E. coli W3110* pBBR1MCS-2::AB und *E. coli* W3110 pBBR1MCS-2::AB_rfbBDAC ohne Verstärkung des *Pseudomonas aeruginosa-Gens pa1131.*
Weiterhin wird gezeigt, dass *E. coli* W3110 pBBR1MCS-2::ABCM und *E. coli* W3110 pBBR1MCS-2::ABCM_rfbBDAC mehr Mono- und Dirhamnosyllipide bilden als *E. coli* W3110 pBBR1MCS-2::ABC und *E. coli* W3110 pBBR1MCS-2::ABC_rfbBDAC ohne Verstärkung des *Pseudomonas aeruginosa-Gens pa1131.* Weiterhin wird gezeigt, dass *E. coli* W3110 pBBR1MCS-2::ABM und *E. coli* W3110 pBBR1MCS-2::ABM_rfbBDAC mehr Monorhamnosyllipide bilden als *E. coli* W3110 pBBR1MCS-2::AB und *E. coli W3110* pBBR1MCS-2::AB_rfbBDAC ohne Verstärkung des *Pseudomonas aeruginosa-Gens pa1131.* Schließlich wird gezeigt, dass *E. coli* W3110 pBBR1MCS-2::AB_rfbBDAC, *E. coli W3110* pBBR1MCS-2::ABM_rfbBDAC, *E. coli* W3110 pBBR1MCS-2::ABC_rfbBDAC und *E. coli* W3110 pBBR1MCS-2::ABCM_rfbBDAC mehr Mono- (*E. coli* W3110 *pBBR1MCS-2::AB_rfbBDAC, E*. *coli* W3110 pBBR1MCS-2::ABM_rfbBDAC) bzw. Mono- und Dirhamnosyllipide (*E. coli* W3110 pBBR1MCS-2::ABC_rfbBDAC und *E. coli* W3110 pBBR1MCS-2::ABCM_rfbBDAC) zu bilden vermögen als die jeweiligen Kontrollstämme *E. coli* W3110 pBBR1MCS-2::AB, *E. coli W3110* pBBR1MCS-2::ABM, *E. coli* W3110 pBBR1MCS-2::ABC und *E. coli* W3110 pBBR1MCS-2::ABCM ohne Verstärkung der *P. putida-Gene rfbBDAC.*

## Patentansprüche

1. Zelle, ausgewählt aus Mikroorganismen, welche mindestens ein Rhamnolipid der allgemeinen Formel (I) zu bilden vermag, 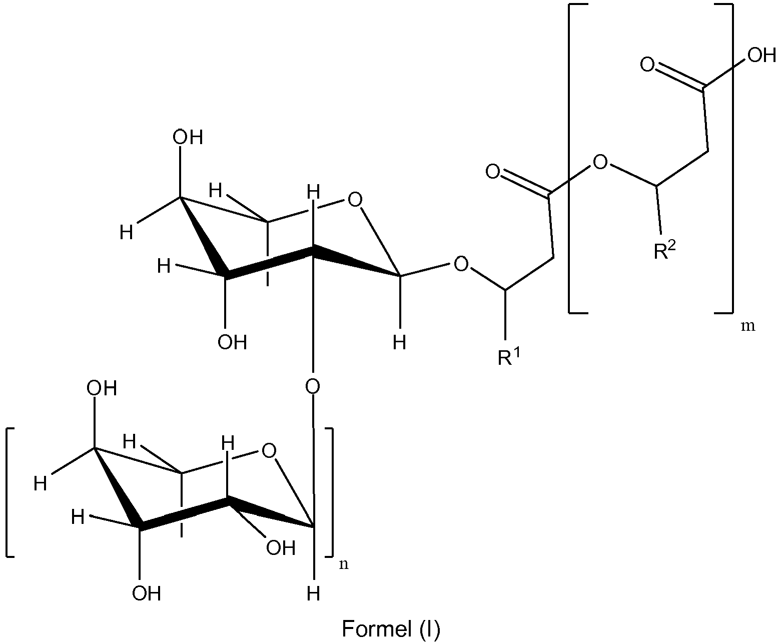 wobei
m = 2, 1 oder 0, insbesondere 1 oder 0,
n = 1 oder 0, insbesondere 1,
R¹ und R² = unabhängig voneinander gleicher oder verschiedener organischer Rest mit 2 bis 24 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest,
**dadurch gekennzeichnet, dass** sie derart gentechnisch verändert wurde, dass sie verglichen zu ihrem Wildtyp eine gesteigerte Aktivität mindestens eines der Enzyme E₁, E₂ und E₃ aufweist, wobei das Enzym E₁ die Umsetzung von 3-Hydroxyalkanoyl-ACP über 3-Hydroxyalkanoyl-3-Hydroxyalkansäure-ACP zu Hydroxyalkanoyl-3-Hydroxyalkansäure zu katalysieren vermag, das Enzym E₂ eine Rhamnosyltransferase I ist und die Umsetzung von dTDP-Rhamnose und 3-Hydroxyalkanoyl-3-Hydroxyalkanoat zu α-L-Rhamnopyranosyl-3-Hydroxyalkanoyl-3-Hydroxyalkanoat zu katalysieren vermag und das Enzym E₃ eine Rhamnosyltransferase II ist und die Umsetzung von dTDP-Rhamnose und α-L-Rhamnopyranosyl-3-Hydroxyalkanoyl-3-Hydroxyalkanoat zu α-L-rhamnopyranosyl-(1-2)-α-L-rhamnopyranosyl-3-Hydroxyalkanoyl-3-Hydroxyalkanoat zu katalysieren vermag und,
dass sie verglichen zu ihrem Wildtyp eine gesteigerte Aktivität mindestens eines der Enzyme ausgewählt aus der Gruppe bestehend aus
ein Enzym E₄, eine dTTP:α-D-Glukose-1-Phosphat Thymidylyltransferase, EC 2.7.7.24, insbesondere mit Polypeptidsequenz Seq ID Nr. 10 oder mit einer Polypeptidsequenz bei der bis zu 25% der Aminosäurereste gegenüber der Bezugssequenz Seq ID Nr. 10 durch Deletion, Insertion, Substitution oder eine Kombination davon verändert sind und die noch mindestens 10% der enzymatischen Aktivität des Enzyms mit der Bezugssequenz Seq ID Nr. 10 besitzt,
ein Enzym E₅, eine dTTP-Glukose-4,6-Hydrolyase, EC 4.2.1.46, insbesondere mit Polypeptidsequenz Seq ID Nr. 12 oder mit einer Polypeptidsequenz bei der bis zu 25% der Aminosäurereste gegenüber der Bezugssequenz Seq ID Nr. 12 durch Deletion, Insertion, Substitution oder eine Kombination davon verändert sind und die noch mindestens 10% der enzymatischen Aktivität des Enzyms mit der Bezugssequenz Seq ID Nr. 12 besitzt,
ein Enzym E₆, eine dTDP-4-Dehydrorhamnose-3,5-Epimerase, EC 5.1.3.13, insbesondere mit Polypeptidsequenz Seq ID Nr. 14 oder mit einer Polypeptidsequenz bei der bis zu 25% der Aminosäurereste gegenüber der Bezugssequenz Seq ID Nr. 14 durch Deletion, Insertion, Substitution oder eine Kombination davon verändert sind und die noch mindestens 10% der enzymatischen Aktivität des Enzyms mit der Bezugssequenz Seq ID Nr. 14 besitzt, und
ein Enzym E₇, eine dTDP-4-Dehydrorhamnose-Reduktase, EC 1.1.1.133, insbesondere mit Polypeptidsequenz Seq ID Nr. 16 oder mit einer Polypeptidsequenz bei der bis zu 25% der Aminosäurereste gegenüber der Bezugssequenz Seq ID Nr. 16 durch Deletion, Insertion, Substitution oder eine Kombination davon verändert sind und die noch mindestens 10% der enzymatischen Aktivität des Enzyms mit der Bezugssequenz Seq ID Nr. 16 besitzt,
aufweist.

2. Zelle gemäß Anspruch 1, **dadurch gekennzeichnet**, die Enzyme E₁, E₂ und E₃ ausgewählt werden aus der Gruppe bestehend aus,
ein Enzym E₁ ausgewählt aus
ein Enzym E₁ₐ mit Polypeptidsequenz Seq ID Nr. 2 oder mit einer Polypeptidsequenz bei der bis zu 25% der Aminosäurereste gegenüber der Bezugssequenz Seq ID Nr. 2 durch Deletion, Insertion, Substitution oder eine Kombination davon verändert sind und die noch mindestens 10% der enzymatischen Aktivität des Enzyms mit der Bezugssequenz Seq ID Nr. 2 besitzt, ein Enzym E_{1b} mit Polypeptidsequenz Seq ID Nr. 18 oder mit einer Polypeptidsequenz bei der bis zu 25% der Aminosäurereste gegenüber der Bezugssequenz Seq ID Nr. 18 durch Deletion, Insertion, Substitution oder eine Kombination davon verändert sind und die noch mindestens 10% der enzymatischen Aktivität des Enzyms mit der Bezugssequenz Seq ID Nr. 18 besitzt, ein Enzym E_{1c} mit Polypeptidsequenz Seq ID Nr. 78 oder mit einer Polypeptidsequenz bei der bis zu 25% der Aminosäurereste gegenüber der Bezugssequenz Seq ID Nr. 78 durch Deletion, Insertion, Substitution oder eine Kombination davon verändert sind und die noch mindestens 10% der enzymatischen Aktivität des Enzyms mit der Bezugssequenz Seq ID Nr. 78 besitzt, ein Enzym E_{1d} mit Polypeptidsequenz Seq ID Nr. 80 oder mit einer Polypeptidsequenz bei der bis zu 25% der Aminosäurereste gegenüber der Bezugssequenz Seq ID Nr. 80 durch Deletion, Insertion, Substitution oder eine Kombination davon verändert sind und die noch mindestens 10% der enzymatischen Aktivität des Enzyms mit der Bezugssequenz Seq ID Nr. 80 besitzt, und
ein Enzym E₁ₑ mit Polypeptidsequenz Seq ID Nr. 82 oder mit einer Polypeptidsequenz bei der bis zu 25% der Aminosäurereste gegenüber der Bezugssequenz Seq ID Nr. 82 durch Deletion, Insertion, Substitution oder eine Kombination davon verändert sind und die noch mindestens 10% der enzymatischen Aktivität des Enzyms mit der Bezugssequenz Seq ID Nr. 82 besitzt,
ein Enzym E₂ ausgewählt aus
ein Enzym E₂ₐ mit Polypeptidsequenz Seq ID Nr. 4 oder mit einer Polypeptidsequenz bei der bis zu 25% der Aminosäurereste gegenüber der Bezugssequenz Seq ID Nr. 4 durch Deletion, Insertion, Substitution oder eine Kombination davon verändert sind und die noch mindestens 10% der enzymatischen Aktivität des Enzyms mit der Bezugssequenz Seq ID Nr. 4 besitzt, ein Enzym E_{2b} mit Polypeptidsequenz Seq ID Nr. 20 oder mit einer Polypeptidsequenz bei der bis zu 25% der Aminosäurereste gegenüber der Bezugssequenz Seq ID Nr. 20 durch Deletion, Insertion, Substitution oder eine Kombination davon verändert sind und die noch mindestens 10% der enzymatischen Aktivität des Enzyms mit der Bezugssequenz Seq ID Nr. 20 besitzt, ein Enzym E_{2c} mit Polypeptidsequenz Seq ID Nr. 84 oder mit einer Polypeptidsequenz bei der bis zu 25% der Aminosäurereste gegenüber der Bezugssequenz Seq ID Nr. 84 durch Deletion, Insertion, Substitution oder eine Kombination davon verändert sind und die noch mindestens 10% der enzymatischen Aktivität des Enzyms mit der Bezugssequenz Seq ID Nr. 84 besitzt, ein Enzym E_{2d} mit Polypeptidsequenz Seq ID Nr. 86 oder mit einer Polypeptidsequenz bei der bis zu 25% der Aminosäurereste gegenüber der Bezugssequenz Seq ID Nr. 86 durch Deletion, Insertion, Substitution oder eine Kombination davon verändert sind und die noch mindestens 10% der enzymatischen Aktivität des Enzyms mit der Bezugssequenz Seq ID Nr. 86 besitzt, und
ein Enzym E₂ₑ mit Polypeptidsequenz Seq ID Nr. 88 oder mit einer Polypeptidsequenz bei der bis zu 25% der Aminosäurereste gegenüber der Bezugssequenz Seq ID Nr. 88 durch Deletion, Insertion, Substitution oder eine Kombination davon verändert sind und die noch mindestens 10% der enzymatischen Aktivität des Enzyms mit der Bezugssequenz Seq ID Nr. 88 besitzt, und
ein Enzym E₃ ausgewählt aus
ein Enzym E₃ₐ mit Polypeptidsequenz Seq ID Nr. 6 oder mit einer Polypeptidsequenz bei der bis zu 25% der Aminosäurereste gegenüber der Bezugssequenz Seq ID Nr. 6 durch Deletion, Insertion, Substitution oder eine Kombination davon verändert sind und die noch mindestens 10% der enzymatischen Aktivität des Enzyms mit der Bezugssequenz Seq ID Nr. 6 besitzt, ein Enzym E_{3b} mit Polypeptidsequenz Seq ID Nr. 22 oder mit einer Polypeptidsequenz bei der bis zu 25% der Aminosäurereste gegenüber der Bezugssequenz Seq ID Nr. 22 durch Deletion, Insertion, Substitution oder eine Kombination davon verändert sind und die noch mindestens 10% der enzymatischen Aktivität des Enzyms mit der Bezugssequenz Seq ID Nr. 22 besitzt, ein Enzym E_{3c} mit Polypeptidsequenz Seq ID Nr. 90 oder mit einer Polypeptidsequenz bei der bis zu 25% der Aminosäurereste gegenüber der Bezugssequenz Seq ID Nr. 90 durch Deletion, Insertion, Substitution oder eine Kombination davon verändert sind und die noch mindestens 10% der enzymatischen Aktivität des Enzyms mit der Bezugssequenz Seq ID Nr. 90 besitzt und
ein Enzym E_{3d} mit Polypeptidsequenz Seq ID Nr. 92 oder mit einer Polypeptidsequenz bei der bis zu 25% der Aminosäurereste gegenüber der Bezugssequenz Seq ID Nr. 92 durch Deletion, Insertion, Substitution oder eine Kombination davon verändert sind und die noch mindestens 10% der enzymatischen Aktivität des Enzyms mit der Bezugssequenz Seq ID Nr. 92 besitzt.

3. Zelle gemäß mindestens einem der vorherigen Ansprüche, **dadurch gekennzeichnet, dass** sie eine gesteigerte Aktivität der Enzymkombination
E₁E₂E₃ aufweist und
n = 1 ist.

4. Zelle gemäß mindestens einem der vorherigen Ansprüche, **dadurch gekennzeichnet, dass** sie ausgewählt ist aus einer Gattung aus der Gruppe bestehend aus *Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium und Cupriavidus.*

5. Zelle gemäß mindestens einem der vorherigen Ansprüche, **dadurch gekennzeichnet, dass** sie als Wildtyp Polyhydroxyalkanoate mit Kettenlängen von C₆ bis C₁₆ zu bilden vermag, insbesondere solche, die derart gentechnisch verändert wurde, dass sie verglichen zu ihrem Wildtyp weniger Polyhydroxyalkanoate zu bilden vermag.

6. Zelle gemäß Anspruch 5, **dadurch gekennzeichnet, dass** die Zelle verglichen zu ihrem Wildtyp eine verminderte Aktivität mindestens eines Enzymes Eg oder E₁₀ aufweist,
wobei Eg eine Polyhydroxyalkanoat-Synthase, EC:2.3.1.- mit der Fähigkeit, 3-Hydroxyalkanoyl-Coenzym A zu Poly-3-Hydroxyalkansäure umzusetzen, insbesondere mit Polypeptidsequenz Seq ID Nr. 30 oder Seq ID Nr. 32 oder mit einer Polypeptidsequenz bei der bis zu 25%,der Aminosäurereste gegenüber der jeweiligen Bezugssequenz Seq ID Nr. 30 oder Seq ID Nr. 32 durch Deletion, Insertion, Substitution oder eine Kombination davon verändert sind und die noch mindestens 10% der enzymatischen Aktivität des Enzyms mit der jeweiligen Bezugssequenz Seq ID Nr. 30 oder Seq ID Nr. 32 besitzt, und
E₁₀ eine 3-Hydroxyalkanoyl-ACP:Coenzym A-Transferase mit der Fähigkeit, 3-Hydroxyalkanoyl-ACP zu 3-Hydroxyalkananoyl-Coenzym A umzusetzen, insbesondere mit Polypeptidsequenz Seq ID Nr. 34 oder Seq ID Nr. 36 oder mit einer Polypeptidsequenz bei der bis zu 25% der Aminosäurereste gegenüber der jeweiligen Bezugssequenz Seq ID Nr. 34 oder Seq ID Nr. 36 durch Deletion, Insertion, Substitution oder eine Kombination davon verändert sind und die noch mindestens 10% der enzymatischen Aktivität des Enzyms mit der jeweiligen Bezugssequenz Seq ID Nr. 34 oder Seq ID Nr. 36 besitzt,
darstellt.

7. Zelle gemäß einem der vorherigen Ansprüche, **dadurch gekennzeichnet, dass** sie verglichen zu ihrem Wildtyp eine gesteigerte Aktivität mindestens eines Enzymes E₈, welches den Export eines Rhamnolipids der allgemeinen Formel (I) aus der Zelle in das umgebende Medium katalysiert, bevorzugt mit Polypeptidsequenz Seq ID Nr. 8, Seq ID Nr. 24, Seq ID Nr. 26 oder Seq ID Nr. 28 oder mit einer Polypeptidsequenz bei der bis zu 25% der Aminosäurereste gegenüber der jeweiligen Bezugssequenz Seq ID Nr. 8, Seq ID Nr. 24, Seq ID Nr. 26 oder Seq ID Nr. 28 durch Deletion, Insertion, Substitution oder eine Kombination davon verändert sind und die noch mindestens 10% der enzymatischen Aktivität des Enzyms mit der jeweiligen Bezugssequenz Seq ID Nr. 8, Seq ID Nr. 24, Seq ID Nr. 26 oder Seq ID Nr. 28 besitzt, aufweist.

8. Zelle gemäß einem der vorherigen Ansprüche, **dadurch gekennzeichnet, dass** sie mindestens eine isolierte Nukleinsäuren welche mindestens jeweils eine Sequenz ausgewählt aus den drei Gruppen [A1 bis G1], [A2 bis G2] und [A3 bis G3] aufweist, wobei die Gruppe [A1 bis G1] aus den folgenden Sequenzen besteht:
A1a) eine Sequenz gemäß Seq ID Nr. 1, wobei diese Sequenz für ein Protein kodiert, welches in der Lage ist, 3-Hydroxydekanoyl-ACP über 3-Hydroxydekanoyl-3-Hydroxydekanoyl-ACP zu 3-Hydroxydekanoyl-3-Hydroxydekansäure umzusetzen,
B1a) eine intronfreie Sequenz, die von einer Sequenz nach A1a) abgeleitet ist und die das gleiche Protein oder Peptid kodiert wie die Sequenz nach Seq ID Nr. 1,
C1a) eine Sequenz, die ein Protein oder Peptid kodiert, das die Aminosäuresequenz gemäß Seq ID Nr. 2 umfasst,
D1a) eine Sequenz, die mit einer Sequenz nach einer der Gruppen A1a) bis C1a) zu mindestens 70% identisch ist,
E1a) eine Sequenz, die mit dem Gegenstrang einer Sequenz nach einer der Gruppen A1a) bis D1a) hybridisiert oder unter Berücksichtigung der Degeneration des genetischen Codes hybridisieren würde, wobei diese Sequenz für ein Protein oder Peptid kodiert, welches in der Lage ist, 3-Hydroxydekanoyl-ACP über 3-Hydroxydekanoyl-3-Hydroxydekanoyl-ACP zu 3-Hydroxydekanoyl-3-Hydroxydekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F1a) ein durch Substitution, Addition, Inversion und/oder Deletion mindestens einer Base, jedoch von nicht mehr als 100 Basen, erhaltenes Derivat einer Sequenz nach einer der Gruppen A1a) bis E1a),
G1a) eine komplementäre Sequenz zu einer Sequenz nach einer der Gruppen A1a) bis F1a),
A1b) eine Sequenz gemäß Seq ID Nr. 17, wobei diese Sequenz für ein Protein kodiert, welches in der Lage ist, 3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen,
B1b) eine intronfreie Sequenz, die von einer Sequenz nach A1b) abgeleitet ist und die das gleiche Protein oder Peptid kodiert wie die Sequenz nach Seq ID Nr. 17,
C1b) eine Sequenz, die ein Protein oder Peptid kodiert, das die Aminosäuresequenz gemäß Seq ID Nr. 18 umfasst,
D1b) eine Sequenz, die mit einer Sequenz nach einer der Gruppen A1b) bis C1b) zu mindestens 70% identisch ist,
E1b) eine Sequenz, die mit dem Gegenstrang einer Sequenz nach einer der Gruppen A1b) bis D1b) hybridisiert oder unter Berücksichtigung der Degeneration des genetischen Codes hybridisieren würde, wobei diese Sequenz für ein Protein oder Peptid kodiert, welches in der Lage ist, 3-Hydroxytetradekanoyl-ACP über 3-Hydroxytetradekanoyl-3-Hydroxytetradekanoyl-ACP zu 3-Hydroxytetradekanoyl-3-Hydroxytetradekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F1b) ein durch Substitution, Addition, Inversion und/oder Deletion mindestens einer Base, jedoch von nicht mehr als 100 Basen, erhaltenes Derivat einer Sequenz nach einer der Gruppen A1b) bis E1b) und
G1b) eine komplementäre Sequenz zu einer Sequenz nach einer der Gruppen A1b) bis F1b) und
die Gruppe [A2 bis G2] aus den folgenden Sequenzen besteht:
A2a) eine Sequenz gemäß Seq ID Nr. 3, wobei diese Sequenz für ein Protein kodiert, welches in der Lage ist, dTDP-Rhamnose und 3-Hydroxydekanoyl-3-Hydroxydekansäure zu α-L-Rhamnopyranosyl-3-Hydroxydekanoyl-3-Hydroxydekansäure umzusetzen,
B2a) eine intronfreie Sequenz, die von einer Sequenz nach A2a) abgeleitet ist und die das gleiche Protein oder Peptid kodiert wie die Sequenz nach Seq ID Nr. 3,
C2a) eine Sequenz, die ein Protein oder Peptid kodiert, das die Aminosäuresequenz gemäß Seq ID Nr. 4 umfasst,
D2a) eine Sequenz, die mit einer Sequenz nach einer der Gruppen A2a) bis C2a) zu mindestens 80% identisch ist,
E2a) eine Sequenz, die mit dem Gegenstrang einer Sequenz nach einer der Gruppen A2a) bis D2a) hybridisiert oder unter Berücksichtigung der Degeneration des genetischen Codes hybridisieren würde, wobei diese Sequenz für ein Protein oder Peptid kodiert, welches in der Lage ist, dTDP-Rhamnose und 3-Hydroxydekanoyl-3-Hydroxydekansäure zu α-L-Rhamnopyranosyl-3-Hydroxydekanoyl-3-Hydroxydekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F2a) ein durch Substitution, Addition, Inversion und/oder Deletion mindestens einer Base, jedoch von nicht mehr als 100 Basen, erhaltenes Derivat einer Sequenz nach einer der Gruppen A2a) bis E2a),
G2a) eine komplementäre Sequenz zu einer Sequenz nach einer der Gruppen A2a) bis F2a),
A2b) eine Sequenz gemäß Seq ID Nr. 19, wobei diese Sequenz für ein Protein kodiert, welches in der Lage ist, dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen,
B2b) eine intronfreie Sequenz, die von einer Sequenz nach A2b) abgeleitet ist und die das gleiche Protein oder Peptid kodiert wie die Sequenz nach Seq ID Nr. 19,
C2b) eine Sequenz, die ein Protein oder Peptid kodiert, das die Aminosäuresequenz gemäß Seq ID Nr. 20 umfasst,
D2b) eine Sequenz, die mit einer Sequenz nach einer der Gruppen A2b) bis C2b) zu mindestens 70% identisch ist,
E2b) eine Sequenz, die mit dem Gegenstrang einer Sequenz nach einer der Gruppen A2b) bis D2b) hybridisiert oder unter Berücksichtigung der Degeneration des genetischen Codes hybridisieren würde, wobei diese Sequenz für ein Protein oder Peptid kodiert, welches in der Lage ist, dTDP-Rhamnose und 3-Hydroxytetradekanoyl-3-Hydroxytetradekansäure zu α-L-Rhamnopyranosyl-3-Hydroxytetradekanoyl-3-Hydroxytetradekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F2b) ein durch Substitution, Addition, Inversion und/oder Deletion mindestens einer Base, jedoch von nicht mehr als 100 Basen, erhaltenes Derivat einer Sequenz nach einer der Gruppen A2b) bis E2b) und
G2b) eine komplementäre Sequenz zu einer Sequenz nach einer der Gruppen A2b) bis F2b) und
die Gruppe [A3 bis G3] aus den folgenden Sequenzen besteht:
A3a) eine Sequenz gemäß Seq ID Nr. 5, wobei diese Sequenz für ein Protein kodiert, welches in der Lage ist, dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen,
B3a) eine intronfreie Sequenz, die von einer Sequenz nach A3a) abgeleitet ist und die das gleiche Protein oder Peptid kodiert wie die Sequenz nach Seq ID Nr. 5,
C3a) eine Sequenz, die ein Protein oder Peptid kodiert, das die Aminosäuresequenz gemäß Seq ID Nr. 6 umfasst,
D3a) eine Sequenz, die mit einer Sequenz nach einer der Gruppen A3a) bis C3a) zu mindestens 80% identisch ist,
E3a) eine Sequenz, die mit dem Gegenstrang einer Sequenz nach einer der Gruppen A3a) bis D3a) hybridisiert oder unter Berücksichtigung der Degeneration des genetischen Codes hybridisieren würde, wobei diese Sequenz für ein Protein oder Peptid kodiert, welches in der Lage ist, dTDP-Rhamnose und α-L-Rhamnopyranosyl-3-Hydroxydekanoyl-3-Hydroxydekansäure zu α-L-Rhamnopyranosyl-(1-2)-α-L-Rhamnopyranosyl-3-Hydroxydekanoyl-3-Hydroxydekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F3a) ein durch Substitution, Addition, Inversion und/oder Deletion mindestens einer Base, jedoch von nicht mehr als 100 Basen, erhaltenes Derivat einer Sequenz nach einer der Gruppen A3a) bis E3a),
G3a) eine komplementäre Sequenz zu einer Sequenz nach einer der Gruppen A3a) bis F3a),
A3b) eine Sequenz gemäß Seq ID Nr. 21, wobei diese Sequenz für ein Protein kodiert, welches in der Lage ist, dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen,
B3b) eine intronfreie Sequenz, die von einer Sequenz nach A3b) abgeleitet ist und die das gleiche Protein oder Peptid kodiert wie die Sequenz nach Seq ID Nr. 21,
C3b) eine Sequenz, die ein Protein oder Peptid kodiert, das die Aminosäuresequenz gemäß Seq ID Nr. 22 umfasst,
D3b) eine Sequenz, die mit einer Sequenz nach einer der Gruppen A3b) bis C3b) zu mindestens 60% identisch ist,
E3b) eine Sequenz, die mit dem Gegenstrang einer Sequenz nach einer der Gruppen A3b) bis D3b) hybridisiert oder unter Berücksichtigung der Degeneration des genetischen Codes hybridisieren würde, wobei diese Sequenz für ein Protein oder Peptid kodiert, welches in der Lage ist, dTDP-Rhamnose und α-L-Rhamnopyranosyl-3-Hydroxytetradekanoyl-3-Hydroxytetradekansäure zu α-L-Rhamnopyranosyl-(1-2)-α-L-Rhamnopyranosyl-3-Hydroxytetradekanoyl-3-Hydroxytetradekansäure umzusetzen, und die Hybridisierungsbedingungen Inkubation bei 65°C über Nacht in 7% SDS, 1% BSA, 1 mM EDTA, 250 mM Natriumphosphatpuffer (pH 7,2) und nachfolgendes Waschen bei 65°C mit 2 x SSC; 0,1% SDS sind,
F3b) ein durch Substitution, Addition, Inversion und/oder Deletion mindestens einer Base, jedoch von nicht mehr als 100 Basen, erhaltenes Derivat einer Sequenz nach einer der Gruppen A3b) bis E3b) und
G3b) eine komplementäre Sequenz zu einer Sequenz nach einer der Gruppen A3b) bis F3b),
oder mindestens einen Vektor, insbesondere einen Expressionsvektor oder eine Genüberexpressionskassette, umfassend mindestens eine Nukleinsäuresequenz ausgewählt aus Seq ID Nr. 38, Seq ID Nr. 40, Seq ID Nr. 42, Seq ID Nr. 45, Seq ID Nr. 47 und Nukleinsäuren ausgewählt aus den vorgenannten drei Gruppen [A1 bis G1], [A2 bis G2] und [A3 bis G3]
aufweist.

9. Verfahren zur Herstellung von Rhamnolipiden der allgemeinen Formel (I), umfassend die Verfahrensschritte
I) in Kontakt Bringen einer Zelle gemäß mindestens einem der Ansprüche 1 bis 8 mit einem Medium beinhaltend eine Kohlenstoffquelle
II) Kultivieren der Zelle unter Bedingungen, die es der Zelle ermöglichen, aus der Kohlenstoffquelle Rhamnolipid zu bilden und
III) gegebenenfalls Isolierung der gebildeten Rhamnolipide.

## Claims

1. Cell, selected from microorganisms, which is capable of forming at least one rhamnolipid of the general formula (I) 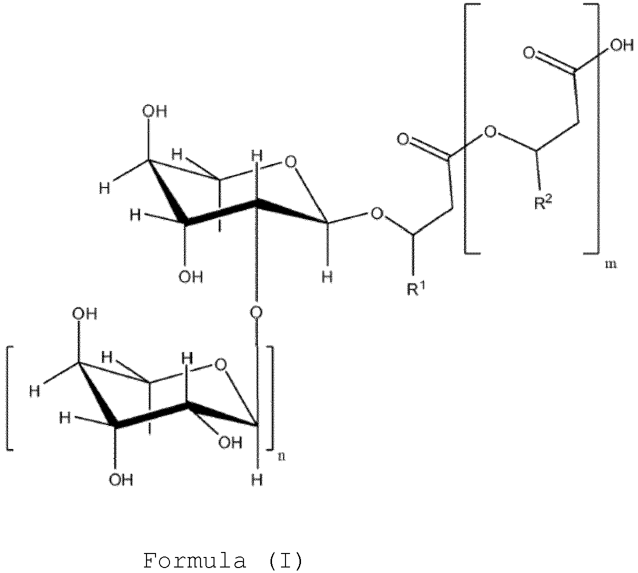 wherein
m = 2, 1 or 0, in particular 1 or 0,
n = 1 or 0, in particular 1,
R¹ and R² = mutually independently same or different organic residue with 2 to 24 carbon atoms, in particular optionally branched, optionally substituted, in particular hydroxy-substituted, optionally unsaturated, in particular optionally singly, doubly or triply unsaturated, alkyl residue, **characterized in that** it has been modified by genetic engineering in such a manner that compared to its wild type it has an increased activity of at least one of the enzymes E₁, E₂ and E₃, wherein the enzyme E₁ is capable of catalysing the conversion of 3-hydroxyalkanoyl-ACP to hydroxyalkanoyl-3-hydroxy-alkanoic acid via 3-hydroxyalkanoyl-3-hydroxy-alkanoic acid-ACP, the enzyme E₂ is a rhamnosyl transferase I and is capable of catalysing the conversion of dTDP-rhamnose and 3-hydroxyalkanoyl-3-hydroxyalkanoate to α-L-rhamnopyranosyl-3-hydroxyalkanoyl-3-hydroxyalkanoate and the enzyme E₃ is a rhamnosyl transferase II and is capable of catalysing the conversion of dTDP-rhamnose and α-L-rhamnopyranosyl-3-hydroxalkanoyl-3-hydroxyalkanoate to α-L-rhamnopyranosyl-(1-2)-α-L-rhamnopyranosyl-3-hydroxyalkanoyl-3-hydroxyalkanoate and that, compared to its wild type, it has an increased activity of at least one of the enzymes selected from the group consisting of
an enzyme E₄, a dTTP:α-D-glucose-1-phosphate thymidylyl transferase, EC 2.7.7.24, in particular with polypeptide sequence Seq ID No.10 or with a polypeptide sequence in which up to 25% of the amino acid residues have been changed compared to the reference sequence Seq ID No.10 by deletion, insertion, substitution or a combination thereof, and which still possesses at least 10% of the enzymatic activity of the enzyme with the reference sequence Seq ID No.10,
an enzyme E₅, a dTTP-glucose-4,6 hydrolase, EC 4.2.1.46, in particular with polypeptide sequence Seq ID No.12 or with a polypeptide sequence in which up to 25% of the amino acid residues have been changed compared to the reference sequence Seq ID No.12 by deletion, insertion, substitution or a combination thereof, and which still possesses at least 10% of the enzymatic activity of the enzyme with the reference sequence Seq ID No.12,
an enzyme E₆, a dTDP-4-dehydrorhamnose 3,5-epimerase, EC 5.1.3.13, in particular with polypeptide sequence Seq ID No.14 or with a polypeptide sequence in which up to 25% of the amino acid residues have been changed compared to the reference sequence Seq ID No.14 by deletion, insertion, substitution or a combination thereof, and which still possesses at least 10% of the enzymatic activity of the enzyme with the reference sequence Seq ID No.14, and
an enzyme E₇, a dTD-4-dehydrorhamnose reductase, EC 1.1.1.133, in particular with polypeptide sequence Seq ID No.16 or with a polypeptide sequence in which up to 25% of the amino acid residues have been changed compared to the reference sequence Seq ID No.16 by deletion, insertion, substitution or a combination thereof, and which still possesses at least 10% of the enzymatic activity of the enzyme with the reference sequence Seq ID No.16.

2. Cell according to Claim 1, **characterized in that** the enzymes E₁, E₂ and E₃ are selected from the group consisting of,
an enzyme E₁ selected from
an enzyme E₁ₐ with polypeptide sequence Seq ID No.2 or with a polypeptide sequence in which up to 25% of the amino acid residues have been changed compared to the reference sequence Seq ID No.2 by deletion, insertion, substitution or a combination thereof, and which still possesses at least 10% of the enzymatic activity of the enzyme with the reference sequence Seq ID No.2,
an enzyme E_{1b} with polypeptide sequence Seq ID No.18 or with a polypeptide sequence in which up to 25% of the amino acid residues have been changed compared to the reference sequence Seq ID No.18 by deletion, insertion, substitution or a combination thereof, and which still possesses at least 10% of the enzymatic activity of the enzyme with the reference sequence Seq ID No.18,
an enzyme E_{1c} with polypeptide sequence Seq ID No.78 or with a polypeptide sequence in which up to 25% of the amino acid residues have been changed compared to the reference sequence Seq ID No.78 by deletion, insertion, substitution or a combination thereof, and which still possesses at least 10% of the enzymatic activity of the enzyme with the reference sequence Seq ID No.78,
an enzyme E_{1d} with polypeptide sequence Seq ID No.80 or with a polypeptide sequence in which up to 25% of the amino acid residues have been changed compared to the reference sequence Seq ID No.80 by deletion, insertion, substitution or a combination thereof, and which still possesses at least 10% of the enzymatic activity of the enzyme with the reference sequence Seq ID No.80,
and
an enzyme E₁ₑ with polypeptide sequence Seq ID No.82 or with a polypeptide sequence in which up to 25% of the amino acid residues have been changed compared to the reference sequence Seq ID No.82 by deletion, insertion, substitution or a combination thereof, and which still possesses at least 10% of the enzymatic activity of the enzyme with the reference sequence Seq ID No.82,
an enzyme E₂ selected from
an enzyme E₂ₐ with polypeptide sequence Seq ID No.4 or with a polypeptide sequence in which up to 25% of the amino acid residues have been changed compared to the reference sequence Seq ID No.4 by deletion, insertion, substitution or a combination thereof, and which still possesses at least 10% of the enzymatic activity of the enzyme with the reference sequence Seq ID No.4,
an enzyme E_{2b} with polypeptide sequence Seq ID No.20 or with a polypeptide sequence in which up to 25% of the amino acid residues have been changed compared to the reference sequence Seq ID No.20 by deletion, insertion, substitution or a combination thereof, and which still possesses at least 10% of the enzymatic activity of the enzyme with the reference sequence Seq ID No.20,
an enzyme E_{2c} with polypeptide sequence Seq ID No.84 or with a polypeptide sequence in which up to 25% of the amino acid residues have been changed compared to the reference sequence Seq ID No.84 by deletion, insertion, substitution or a combination thereof, and which still possesses at least 10% of the enzymatic activity of the enzyme with the reference sequence Seq ID No.84,
an enzyme E_{2d} with polypeptide sequence Seq ID No.86 or with a polypeptide sequence in which up to 25% of the amino acid residues have been changed compared to the reference sequence Seq ID No.86 by deletion, insertion, substitution or a combination thereof, and which still possesses at least 10% of the enzymatic activity of the enzyme with the reference sequence Seq ID No.86,
and
an enzyme E₂ₑ with polypeptide sequence Seq ID No.88 or with a polypeptide sequence in which up to 25% of the amino acid residues have been changed compared to the reference sequence Seq ID No.88 by deletion, insertion, substitution or a combination thereof, and which still possesses at least 10% of the enzymatic activity of the enzyme with the reference sequence Seq ID No.88, and
an enzyme E₃ selected from
an enzyme E₃ₐ with polypeptide sequence Seq ID No.6 or with a polypeptide sequence in which up to 25% of the amino acid residues have been changed compared to the reference sequence Seq ID No.6 by deletion, insertion, substitution or a combination thereof, and which still possesses at least 10% of the enzymatic activity of the enzyme with the reference sequence Seq ID No.6,
an enzyme E_{3b} with polypeptide sequence Seq ID No.22 or with a polypeptide sequence in which up to 25% of the amino acid residues have been changed compared to the reference sequence Seq ID No.22 by deletion, insertion, substitution or a combination thereof, and which still possesses at least 10% of the enzymatic activity of the enzyme with the reference sequence Seq ID No.22,
an enzyme E_{3c} with polypeptide sequence Seq ID No.90 or with a polypeptide sequence in which up to 25% of the amino acid residues have been changed compared to the reference sequence Seq ID No.90 by deletion, insertion, substitution or a combination thereof, and which still possesses at least 10% of the enzymatic activity of the enzyme with the reference sequence Seq ID No.90,
and
an enzyme E_{3d} with polypeptide sequence Seq ID No.92 or with a polypeptide sequence in which up to 25% of the amino acid residues have been changed compared to the reference sequence Seq ID No.92 by deletion, insertion, substitution or a combination thereof, and which still possesses at least 10% of the enzymatic activity of the enzyme with the reference sequence Seq ID No.92.

3. Cell according to at least one of the previous claims, **characterized in that** it has an increased activity of the enzyme combination E₁E₂E₃ and
n = 1.

4. Cell according to at least one of the previous claims, **characterized in that** it is selected from a genus from the group consisting of *Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Burkholderia, Rhodobacter, Clostridium and Cupriavidus.*

5. Cell according to at least one of the previous claims, **characterized in that** as wild type it is capable of forming polyhydroxyalkanoates with chain lengths from C₆ to C₁₆, in particular one which has been modified by genetic engineering in such a manner that compared to its wild type it is capable of forming fewer polyhydroxyalkanoates.

6. Cell according to Claim 5, **characterized in that** compared to its wild type the cell has a decreased activity of at least one enzyme E₉ or E₁₀,
wherein E₉ is a polyhydroxyalkanoate synthase, EC:2.3.1 with the ability to convert 3-hydroxyalkanoyl-coenzyme A to poly-3-hydroxyalkanoic acid, in particular with polypeptide sequence Seq ID No.30 or Seq ID No.32 or with a polypeptide sequence in which up to 25% of the amino acid residues have been changed compared to the respective reference sequence Seq ID No.30 or Seq ID No.32 by deletion, insertion, substitution or a combination thereof, and which still possesses at least 10% of the enzymatic activity of the enzyme with the respective reference sequence Seq ID No.30 or Seq ID No.32, and E₁₀ is a 3-hydroxyalkanoyl-ACP:Coenzyme A transferase with the ability to convert 3-hydroxyalkanoyl-ACP to 3-hydroxyalkanoyl-coenzyme A, in particular with polypeptide sequence Seq ID No.34 or Seq ID No.36 or with a polypeptide sequence in which up to 25% of the amino acid residues have been changed compared to the respective reference sequence Seq ID No.34 or Seq ID No.36 by deletion, insertion, substitution or a combination thereof, and which still possesses at least 10% of the enzymatic activity of the enzyme with the respective reference sequence Seq ID No.34 or Seq ID No.36.

7. Cell according to one of the previous claims, **characterized in that** compared to its wild type it has an increased activity of at least one enzyme E₈, which catalyses the export of a rhamnolipid of the general formula (I) from the cell into the surrounding medium, preferably with polypeptide sequence Seq ID No.8, Seq ID No.24, Seq ID No.26 or Seq ID No.28 or with a polypeptide sequence in which up to 25% of the amino acid residues have been changed compared to the respective reference sequence Seq ID No.8, Seq ID No.24, Seq ID No.26 or Seq ID No.28 by deletion, insertion, substitution or a combination thereof, and which still possesses at least 10% of the enzymatic activity of the enzyme with the respective reference sequence Seq ID No.8, Seq ID No.24, Seq ID No.26 or Seq ID No.28.

8. Cell according to one of the previous claims, **characterized in that** it contains at least one isolated nucleic acids which has at least one sequence selected from the three groups [A1 to G1], [A2 to G2]and [A3 to G3] respectively
wherein
the group [A1 to G1] consists of the following sequences:
A1a) a sequence according to Seq ID No.1, wherein this sequence codes for a protein which is capable of converting 3-hydroxydecanoyl-ACP to 3-hydroxydecanoyl-3-hydroxydecanoic acid via 3-hydroxydecanoyl-3-hydroxydecanoyl-ACP,
B1a) an intron-free sequence which is derived from a sequence according to A1a) and which encodes the same protein or peptide as the sequence according to Seq ID No.1,
C1a) a sequence which encodes a protein or peptide which includes the amino acid sequence according to Seq ID No.2,
D1a) a sequence which is at least 70% identical with a sequence according to one of the groups A1a) to C1a),
E1a) a sequence which hybridizes with the reverse strand of a sequence according to one of the groups A1a) to D1a) or would hybridize taking account of the degeneracy of the genetic code, wherein this sequence codes for a protein or peptide which is capable of converting 3-hydroxydecanoyl-ACP into 3-hydroxydecanoyl-3-hydroxydecanoic acid via 3-hydroxydecanoyl-3-hydroxydecanoyl-ACP, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F1a) a derivative of a sequence according to one of the groups A1a) to E1a) obtained by substitution, addition, inversion and/or deletion of at least one base, but of not more than 100 bases,
G1a) a complementary sequence to a sequence according to one of the groups A1a) to F1a),
A1b) a sequence according to Seq ID No.17, wherein this sequence codes for a protein which is capable of converting 3-hydroxytetradecanoyl-ACP to 3-hydroxytetradecanoyl-3-hydroxytetradecanoic acid via 3-hydroxytetradecanoyl-3-hydroxytetradecanoyl-ACP,
B1b) an intron-free sequence which is derived from a sequence according to A1b) and which encodes the same protein or peptide as the sequence according to Seq ID No.17,
C1b) a sequence which encodes a protein or peptide which includes the amino acid sequence according to Seq ID No.18,
D1b) a sequence which is at least 70% identical with a sequence according to one of the groups A1b) to C1b),
E1b) a sequence which hybridizes with the reverse strand of a sequence according to one of the groups A1b) to C1b) or would hybridize taking account of the degeneracy of the genetic code, wherein this sequence codes for a protein or peptide which is capable of converting 3-hydroxytetradecanoyl-ACP into 3-hydroxytetradecanoyl-3-hydroxytetradecanoic acid via 3-hydroxytetradecanoyl-3-hydroxytetradecanoyl-ACP, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F1b) a derivative of a sequence according to one of the groups A1b) to E1b) obtained by substitution, addition, inversion and/or deletion of at least one base, but of not more than 100 bases, and
G1b) a complementary sequence to a sequence according to one of the groups A1b) to F1b), and
the group [A2 to G2] consists of the following sequences:
A2a) a sequence according to Seq ID No.3, wherein this sequence codes for a protein which is capable of converting dTDP-rhamnose and 3-hydroxydecanoyl-3-hydroxydecanoic acid to α-L-rhamnopyranosyl-3-hydroxydecanoyl-3-hydroxydecanoic acid,
B2a) an intron-free sequence which is derived from a sequence according to A2a) and which encodes the same protein or peptide as the sequence according to Seq ID No.3,
C2a) a sequence which encodes a protein or peptide which includes the amino acid sequence according to Seq ID No.4,
D2a) a sequence which is at least 80% identical with a sequence according to one of the groups A2a) to C2a),
E2a) a sequence which hybridizes with the reverse strand of a sequence according to one of the groups A2a) to D2a) or would hybridize taking account of the degeneracy of the genetic code, wherein this sequence codes for a protein or peptide which is capable of converting dTDP-rhamnose and 3-hydroxydecanoyl-3-hydroxydecanoic acid to α-L-rhamnopyranosyl-3-hydroxydecanoyl-3-hydroxydecanoic acid, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F2a) a derivative of a sequence according to one of the groups A2a) to E2a) obtained by substitution, addition, inversion and/or deletion of at least one base, but of not more than 100 bases,
G2a) a complementary sequence to a sequence according to one of the groups A2a) to F2a),
A2b) a sequence according to Seq ID No.19, wherein this sequence codes for a protein which is capable of converting dTDP-rhamnose and 3-hydroxytetradecanoyl-3-hydroxytetradecanoic acid to α-L-rhamnopyranosyl-3-hydroxytetradecanoyl-3-hydroxytetradecanoic acid
B2b) an intron-free sequence which is derived from a sequence according to A2b) and which encodes the same protein or peptide as the sequence according to Seq ID No.19,
C2b) a sequence which encodes a protein or peptide which includes the amino acid sequence according to Seq ID No.20,
D2b) a sequence which is at least 70% identical with a sequence according to one of the groups A2b) to C2b),
E2b) a sequence which hybridizes with the reverse strand of a sequence according to one of the groups A2b) to D2b) or would hybridize taking account of the degeneracy of the genetic code, wherein this sequence codes for a protein or peptide which is capable of converting dTDP-rhamnose and 3-hydroxytetradecanoyl-3-hydroxytetradecanoic acid into α-L-rhamnopyranosyl-3-hydroxytetradecanoyl-3-hydroxytetradecanoc acid, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F2b) a derivative of a sequence according to one of the groups A2b) to E2b) obtained by substitution, addition, inversion and/or deletion of at least one base, but of not more than 100 bases, and
G2b) a complementary sequence to a sequence according to one of the groups A2b) to F2b), and
the group [A3 to G3] consists of the following sequences:
A3a) a sequence according to Seq ID No.5, wherein this sequence codes for a protein which is capable of converting dTDP-rhamnose and α-L-rhamnopyranosyl-3-hydroxydecanoyl-3-hydroxydecanoic acid to α-L-rhamnopyranosyl-(1-2)-α-L-rhamnopyranosyl-3-hydroxydecanoyl-3-hydroxydecanoic acid,
B3a) an intron-free sequence which is derived from a sequence according to A3a) and which encodes the same protein or peptide as the sequence according to Seq ID No.5,
C3a) a sequence which encodes a protein or peptide which includes the amino acid sequence according to Seq ID No.6,
D3a) a sequence which is at least 80% identical with a sequence according to one of the groups A3a) to C3a),
E3a) a sequence which hybridizes with the reverse strand of a sequence according to one of the groups A3a) to D3a) or would hybridize taking account of the degeneracy of the genetic code, wherein this sequence codes for a protein or peptide which is capable of converting dTDP-rhamnose and α-L-rhamnopyranosyl-3-hydroxydecanoyl-3-hydroxydecanoic acid to α-L-rhamnopyranosyl-(1-2)-α-L-rhamnopyranosyl-3-hydroxydecanoyl-3-hydroxydecanoic acid, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F3a) a derivative of a sequence according to one of the groups A3a) to E3a) obtained by substitution, addition, inversion and/or deletion of at least one base, but of not more than 100 bases,
G3a) a complementary sequence to a sequence according to one of the groups A3a) to F3a),
A3b) a sequence according to Seq ID No.21, wherein this sequence codes for a protein which is capable of converting dTDP-rhamnose and α-L-rhamnopyranosyl-3-hydroxytetradecanoyl-3-hydroxytetradecanoic acid to α-L-rhamnopyranosyl-(1-2)-α-L-rhamnopyranosyl-3-hydroxytetradecanoyl-3-hydroxytetradecanoic acid
B3b) an intron-free sequence which is derived from a sequence according to A3b) and which encodes the same protein or peptide as the sequence according to Seq ID No.21,
C3b) a sequence which encodes a protein or peptide which includes the amino acid sequence according to Seq ID No.22,
D3b) a sequence which is at least 60% identical with a sequence according to one of the groups A3b) to C3b),
E3b) a sequence which hybridizes with the reverse strand of a sequence according to one of the groups A3b) to D3b) or would hybridize taking account of the degeneracy of the genetic code, wherein this sequence codes for a protein or peptide which is capable of converting dTDP-rhamnose and α-L-rhamnopyranosyl-3-hydroxytetradecanoyl-3-hydroxytetradecanoic acid to α-L-rhamnopyranosyl-(1-2)-α-L-rhamnopyranosyl-3-hydroxytetradecanoyl-3-hydroxytetradecanoic acid, and the hybridization conditions are incubation at 65°C overnight in 7% SDS, 1% BSA, 1 mM EDTA, 250 mM sodium phosphate buffer (pH 7.2) and subsequent washing at 65°C with 2 x SSC; 0.1% SDS,
F3b) a derivative of a sequence according to one of the groups A3b) to E3b) obtained by substitution, addition, inversion and/or deletion of at least one base, but of not more than 100 bases, and
G3b) a complementary sequence to a sequence according to one of the groups A3b) to F3b),
or at least one vector, in particular an expression vector or a gene overexpression cassette, comprising at least one nucleic acid sequence selected from Seq ID No.38, Seq ID No.40, Seq ID No.42, Seq ID No.45, Seq ID No.47 and nucleic acids selected from the aforementioned three groups [A1 to G1], [A2 to G2] and [A3 to G3].

9. Method for the production of rhamnolipids of the general formula (I), comprising the process steps
I) contacting a cell according to at least one of claims 1 to 8 with a medium containing a carbon source
II) culturing the cell under conditions which enable the cell to form rhamnolipid from the carbon source and
III) optionally isolating the rhamnolipids formed.

## Revendications

1. Cellule, choisie parmi des micro-organismes, qui peut former au moins un rhamnolipide de formule générale (I), 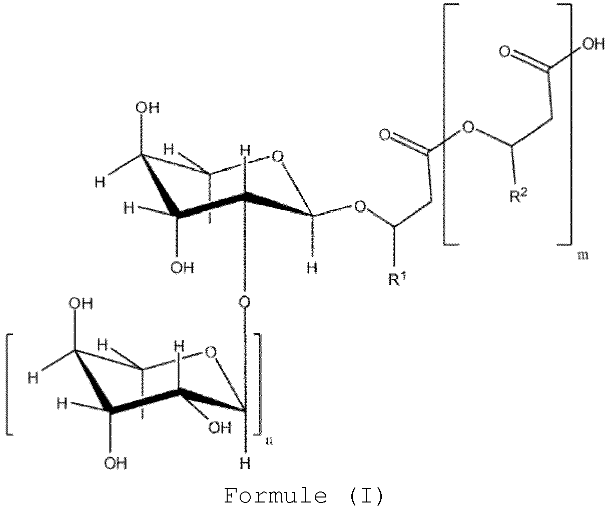 dans laquelle
m = 2, 1 ou 0, en particulier 1 ou 0,
n = 1 ou 0, en particulier 1,
R¹ et R² = indépendamment l'un de l'autre un radical organique, le même ou différent, ayant de 2 à 24 atomes de carbone, en particulier un radical alkyle éventuellement ramifié, éventuellement substitué, en particulier substitué par hydroxy, éventuellement insaturé, en particulier éventuellement une, deux ou trois fois insaturé,
**caractérisée en ce qu'**elle a été génétiquement modifiée de telle façon qu'elle présente en comparaison de son type sauvage une activité accrue d'au moins l'une des enzymes E₁, E₂ et E₃, l'enzyme E₁ étant capable de catalyser la conversion d'une 3-hydroxyalcanoyl-ACP via une acide 3-hydroxyalcanoyl-3-hydroxyalcanoïque-ACP en un acide hydroxyalcanoyl-3-hydroxyalcanoïque, l'enzyme E₂ étant une rhamnosyltransférase I et étant capable de catalyser la conversion de dTDP-rhamnose et 3-hydroxyalcanoyl-3-hydroxyalcanoate en α-L-rhamnopyranosyl-3-hydroxyalcanoyl-3-hydroxyalcanoate et l'enzyme E₃ étant une rhamnosyltransférase II et étant capable de catalyser la conversion de dTDP-rhamnose et α-L-rhamnopyranosyl-3-hydroxyalcanoyl-3-hydroxyalcanoate en α-L-rhamnopyranosyl-(1-2)-α-L-rhamnopyranosyl-3-hydroxyalcanoyl-3-hydroxyalcanoate et
**en ce qu'**elle présente en comparaison de son type sauvage une activité accrue d'au moins l'une des enzymes choisie dans le groupe constitué par
une enzyme E₄, une dTTP:α-D-glucose-1-phosphate thymidylyltransférase, EC 2.7.7.24, en particulier à séquence de polypeptide SEQ ID n° 10 ou ayant une séquence de polypeptide dans laquelle jusqu'à 25 % des résidus d'acides aminés sont changés par rapport à la séquence de référence SEQ ID n° 10 par délétion, insertion, substitution ou une combinaison de celles-ci et qui possède encore au moins 10 % de l'activité enzymatique de l'enzyme ayant la séquence de référence SEQ ID n° 10 ;
une enzyme E₅, une dTTP-glucose-4,6-hydrolase, EC 4.2.1.46, en particulier à séquence de polypeptide SEQ ID n° 12 ou ayant une séquence de polypeptide dans laquelle jusqu'à 25 % des résidus d'acides aminés sont changés par rapport à la séquence de référence SEQ ID n° 12 par délétion, insertion, substitution ou une combinaison de celles-ci et qui possède encore au moins 10 % de l'activité enzymatique de l'enzyme ayant la séquence de référence SEQ ID n° 12 ;
une enzyme E₆, une dTDP-4-déhydrorhamnose-3,5-épimérase, EC 5.1.3.13, en particulier à séquence de polypeptide SEQ ID n° 14 ou ayant une séquence de polypeptide dans laquelle jusqu'à 25 % des résidus d'acides aminés sont changés par rapport à la séquence de référence SEQ ID n° 14 par délétion, insertion, substitution ou une combinaison de celles-ci et qui possède encore au moins 10 % de l'activité enzymatique de l'enzyme ayant la séquence de référence SEQ ID n° 14 ; et
une enzyme E₇, une dTDP-4-déhydrorhamnose-réductase, EC 1.1.1.133, en particulier à séquence de polypeptide SEQ ID n° 16 ou ayant une séquence de polypeptide dans laquelle jusqu'à 25 % des résidus d'acides aminés sont changés par rapport à la séquence de référence SEQ ID n° 16 par délétion, insertion, substitution ou une combinaison de celles-ci et qui possède encore au moins 10 % de l'activité enzymatique de l'enzyme ayant la séquence de référence SEQ ID n° 16.

2. Cellule selon la revendication 1, **caractérisée en ce que** les enzymes E₁, E₂ et E₃ sont choisies dans le groupe constitué par
une enzyme E₁ choisie parmi
une enzyme E₁ₐ à séquence de polypeptide SEQ ID n° 2 ou ayant une séquence de polypeptide dans laquelle jusqu'à 25 % des résidus d'acides aminés sont changés par rapport à la séquence de référence SEQ ID n° 2 par délétion, insertion, substitution ou une combinaison de celles-ci et qui possède encore au moins 10 % de l'activité enzymatique de l'enzyme ayant la séquence de référence SEQ ID n° 2, une enzyme E_{1b} à séquence de polypeptide SEQ ID n° 18 ou ayant une séquence de polypeptide dans laquelle jusqu'à 25 % des résidus d'acides aminés sont changés par rapport à la séquence de référence SEQ ID n° 18 par délétion, insertion, substitution ou une combinaison de celles-ci et qui possède encore au moins 10 % de l'activité enzymatique de l'enzyme ayant la séquence de référence SEQ ID n° 18,
une enzyme E_{1c} à séquence de polypeptide SEQ ID n° 78 ou ayant une séquence de polypeptide dans laquelle jusqu'à 25 % des résidus d'acides aminés sont changés par rapport à la séquence de référence SEQ ID n° 78 par délétion, insertion, substitution ou une combinaison de celles-ci et qui possède encore au moins 10 % de l'activité enzymatique de l'enzyme ayant la séquence de référence SEQ ID n° 78,
une enzyme E_{1d} à séquence de polypeptide SEQ ID n° 80 ou ayant une séquence de polypeptide dans laquelle jusqu'à 25 % des résidus d'acides aminés sont changés par rapport à la séquence de référence SEQ ID n° 80 par délétion, insertion, substitution ou une combinaison de celles-ci et qui possède encore au moins 10 % de l'activité enzymatique de l'enzyme ayant la séquence de référence SEQ ID n° 80, et
une enzyme E₁ₑ à séquence de polypeptide SEQ ID n° 82 ou ayant une séquence de polypeptide dans laquelle jusqu'à 25 % des résidus d'acides aminés sont changés par rapport à la séquence de référence SEQ ID n° 82 par délétion, insertion, substitution ou une combinaison de celles-ci et qui possède encore au moins 10 % de l'activité enzymatique de l'enzyme ayant la séquence de référence SEQ ID n° 82,
une enzyme E₂ choisie parmi
une enzyme E₂ₐ à séquence de polypeptide SEQ ID n° 4 ou ayant une séquence de polypeptide dans laquelle jusqu'à 25 % des résidus d'acides aminés sont changés par rapport à la séquence de référence SEQ ID n° 4 par délétion, insertion, substitution ou une combinaison de celles-ci et qui possède encore au moins 10 % de l'activité enzymatique de l'enzyme ayant la séquence de référence SEQ ID n° 4, une enzyme E_{2b} à séquence de polypeptide SEQ ID n° 20 ou ayant une séquence de polypeptide dans laquelle jusqu'à 25 % des résidus d'acides aminés sont changés par rapport à la séquence de référence SEQ ID n° 20 par délétion, insertion, substitution ou une combinaison de celles-ci et qui possède encore au moins 10 % de l'activité enzymatique de l'enzyme ayant la séquence de référence SEQ ID n° 20,
une enzyme E_{2c} à séquence de polypeptide SEQ ID n° 84 ou ayant une séquence de polypeptide dans laquelle jusqu'à 25 % des résidus d'acides aminés sont changés par rapport à la séquence de référence SEQ ID n° 84 par délétion, insertion, substitution ou une combinaison de celles-ci et qui possède encore au moins 10 % de l'activité enzymatique de l'enzyme ayant la séquence de référence SEQ ID n° 84,
une enzyme E_{2d} à séquence de polypeptide SEQ ID n° 86 ou ayant une séquence de polypeptide dans laquelle jusqu'à 25 % des résidus d'acides aminés sont changés par rapport à la séquence de référence SEQ ID n° 86 par délétion, insertion, substitution ou une combinaison de celles-ci et qui possède encore au moins 10 % de l'activité enzymatique de l'enzyme ayant la séquence de référence SEQ ID n° 86, et
une enzyme E₂ₑ à séquence de polypeptide SEQ ID n° 88 ou ayant une séquence de polypeptide dans laquelle jusqu'à 25 % des résidus d'acides aminés sont changés par rapport à la séquence de référence SEQ ID n° 88 par délétion, insertion, substitution ou une combinaison de celles-ci et qui possède encore au moins 10 % de l'activité enzymatique de l'enzyme ayant la séquence de référence SEQ ID n° 88, et
une enzyme E₃ choisie parmi
une enzyme E₃ₐ à séquence de polypeptide SEQ ID n° 6 ou ayant une séquence de polypeptide dans laquelle jusqu'à 25 % des résidus d'acides aminés sont changés par rapport à la séquence de référence SEQ ID n° 6 par délétion, insertion, substitution ou une combinaison de celles-ci et qui possède encore au moins 10 % de l'activité enzymatique de l'enzyme ayant la séquence de référence SEQ ID n° 6, une enzyme E_{3b} à séquence de polypeptide SEQ ID n° 22 ou ayant une séquence de polypeptide dans laquelle jusqu'à 25 % des résidus d'acides aminés sont changés par rapport à la séquence de référence SEQ ID n° 22 par délétion, insertion, substitution ou une combinaison de celles-ci et qui possède encore au moins 10 % de l'activité enzymatique de l'enzyme ayant la séquence de référence SEQ ID n° 22,
une enzyme E_{3c} à séquence de polypeptide SEQ ID n° 90 ou ayant une séquence de polypeptide dans laquelle jusqu'à 25 % des résidus d'acides aminés sont changés par rapport à la séquence de référence SEQ ID n° 90 par délétion, insertion, substitution ou une combinaison de celles-ci et qui possède encore au moins 10 % de l'activité enzymatique de l'enzyme ayant la séquence de référence SEQ ID n° 90, et
une enzyme E_{3d} à séquence de polypeptide SEQ ID n° 92 ou ayant une séquence de polypeptide dans laquelle jusqu'à 25 % des résidus d'acides aminés sont changés par rapport à la séquence de référence SEQ ID n° 92 par délétion, insertion, substitution ou une combinaison de celles-ci et qui possède encore au moins 10 % de l'activité enzymatique de l'enzyme ayant la séquence de référence SEQ ID n° 92.

3. Cellule selon au moins l'une quelconque des revendications précédentes, **caractérisée en ce qu'**elle présente une activité accrue de l'association d'enzymes E₁E₂E₃ et
n = 1.

4. Cellule selon au moins l'une quelconque des revendications précédentes, **caractérisée en ce qu'**elle est choisie parmi un genre du groupe constitué par *Aspergillus, Corynebacterium, Brevibacterium, Bacillus, Acinetobacter, Alcaligenes, Lactobacillus, Paracoccus, Lactococcus, Candida, Pichia, Hansenula, Kluyveromyces, Saccharomyces, Escherichia, Zymomonas, Yarrowia, Methylobacterium, Ralstonia, Pseudomonas, Rhodospirillum, Rhodobacter, Burkholderia, Clostridium* et *Cupriavidus.*

5. Cellule selon au moins l'une quelconque des revendications précédentes, **caractérisée en ce qu'**en tant que type sauvage elle peut former des polyhydroxyalcanoates à longueurs de chaîne de C₆ à C₁₆, en particulier cellule qui a été génétiquement modifiée de telle sorte qu'en comparaison de son type sauvage elle est capable de former moins de polyhydroxyalcanoates.

6. Cellule selon la revendication 5, **caractérisée en ce que** la cellule présente en comparaison de son type sauvage une acticité réduite d'au moins une enzyme Eg ou E₁₀,
Eg représentant une polyhydroxyalcanoate-synthase, EC:2.3.1, ayant la capacité de convertir une 3-hydroxyalcanoyl-coenzyme A en un acide poly-3-hydroxyalcanoïque, en particulier à séquence de polypeptide SEQ ID n° 30 ou SEQ ID n° 32 ou ayant une séquence de polypeptide dans laquelle jusqu'à 25 % des résidus d'acides aminés sont changés par rapport à la séquence de référence respective SEQ ID n° 30 ou SEQ ID n° 32 par délétion, insertion, substitution ou une combinaison de celles-ci et qui possède encore au moins 10 % de l'activité enzymatique de l'enzyme ayant la séquence de référence respective SEQ ID n° 30 ou SEQ ID n° 32, et
E₁₀ représentant une 3-hydroxyalcanoyl-ACP:coenzyme A-transférase ayant la capacité de convertir une 3-hydroxyalcanoyl-ACP en une 3-hydroxyalcanoyl-coenzyme A, en particulier à séquence de polypeptide SEQ ID n° 34 ou SEQ ID n° 36 ou ayant une séquence de polypeptide dans laquelle jusqu'à 25 % des résidus d'acides aminés sont changés par rapport à la séquence de référence respective SEQ ID n° 34 ou SEQ ID n° 36 par délétion, insertion, substitution ou une combinaison de celles-ci et qui possède encore au moins 10 % de l'activité enzymatique de l'enzyme ayant la séquence de référence respective SEQ ID n° 34 ou SEQ ID n° 36.

7. Cellule selon l'une quelconque des revendications précédentes, **caractérisée en ce qu'**elle présente en comparaison de son type sauvage une activité accrue d'au moins une enzyme E₈ qui catalyse l'exportation dans le milieu environnant, à partir de la cellule, d'un rhamnolipide de formule générale (I), de préférence à séquence de polypeptide SEQ ID n° 8, SEQ ID n° 24, SEQ ID n° 26 ou SEQ ID n° 28 ou ayant une séquence de polypeptide dans laquelle jusqu'à 25 % des résidus d'acides aminés sont changés par rapport à la séquence de référence respective SEQ ID n° 8, SEQ ID n° 24, SEQ ID n° 26 ou SEQ ID n° 28 par délétion, insertion, substitution ou une combinaison de celles-ci et qui possède encore au moins 10 % de l'activité enzymatique de l'enzyme ayant la séquence de référence respective SEQ ID n° 8, SEQ ID n° 24, SEQ ID n° 26 ou SEQ ID n° 28.

8. Cellule selon au moins l'une quelconque des revendications précédentes, **caractérisée en ce qu'**elle comporte au moins un acide nucléique isolé qui présente au moins respectivement une séquence choisie dans les trois groupes [A1 à G1], [A2 à G2] et [A3 à G3],
où
le groupe [A1 à G1] consiste en les séquences suivantes :
A1a) une séquence selon SEQ ID n° 1, cette séquence codant pour une protéine qui est capable de convertir une 3-hydroxydécanoyl-ACP via une 3-hydroxydécanoyl-3-hydroxydécanoyl-ACP en acide 3-hydroxydécanoyl-3-hydroxydécanoïque,
B1a) une séquence exempte d'introns, qui est dérivée d'une séquence selon A1a) et qui code la même protéine ou le même peptide que la séquence selon SEQ ID n° 1,
C1a) une séquence qui code une protéine ou un peptide qui comprend la séquence d'acides aminés selon SEQ ID n° 2,
D1a) une séquence qui est identique à raison d'au moins 70 % à une séquence selon l'un des groupes A1a) à C1a),
E1a) une séquence qui s'hybride ou s'hybriderait, en tenant compte de la dégénérescence du code génétique, avec le brin opposé d'une séquence selon l'un des groupes A1a) à D1a), cette séquence codant pour une protéine ou un peptide qui est capable de convertir une 3-hydroxydécanoyl-ACP via une 3-hydroxydécanoyl-3-hydroxydécanoyl-ACP en acide 3-hydroxydécanoyl-3-hydroxydécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F1a) un dérivé d'une séquence selon l'un des groupes A1a) à E1a), obtenu par substitution, addition, inversion et/ou délétion d'au moins une base, mais d'au maximum 100 bases,
G1a) une séquence complémentaire d'une séquence selon l'un des groupes A1a) à F1a),
A1b) une séquence selon SEQ ID n° 17, cette séquence codant pour une protéine qui est capable de convertir une 3-hydroxytétradécanoyl-ACP via une 3-hydroxytétradécanoyl-3-hydroxytétradécanoyl-ACP en acide 3-hydroxytétradécanoyl-3-hydroxytétradécanoïque,
B1b) une séquence exempte d'introns, qui est dérivée d'une séquence selon A1b) et qui code la même protéine ou le même peptide que la séquence selon SEQ ID n° 17,
C1b) une séquence qui code une protéine ou un peptide qui comprend la séquence d'acides aminés selon SEQ ID n° 18,
D1b) une séquence qui est identique à raison d'au moins 70 % à une séquence selon l'un des groupes A1b) à C1b),
E1b) une séquence qui s'hybride ou s'hybriderait, en tenant compte de la dégénérescence du code génétique, avec le brin opposé d'une séquence selon l'un des groupes A1b) à D1b), cette séquence codant pour une protéine ou un peptide qui est capable de convertir une 3-hydroxytétradécanoyl-ACP via une 3-hydroxytétradécanoyl-3-hydroxytétradécanoyl-ACP en acide 3-hydroxytétradécanoyl-3-hydroxytétradécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F1b) un dérivé d'une séquence selon l'un des groupes A1b) à E1b), obtenu par substitution, addition, inversion et/ou délétion d'au moins une base, mais d'au maximum 100 bases et
G1b) une séquence complémentaire d'une séquence selon l'un des groupes A1b) à F1b) et
le groupe [A2 à G2] consiste en les séquences suivantes :
A2a) une séquence selon SEQ ID n° 3, cette séquence codant pour une protéine qui est capable de convertir du dTDP-rhamnose et de l'acide 3-hydroxydécanoyl-3-hydroxydécanoïque en acide α-L-rhamnopyranosyl-3-hydroxydécanoyl-3-hydroxydécanoïque,
B2a) une séquence exempte d'introns, qui est dérivée d'une séquence selon A2a) et qui code la même protéine ou le même peptide que la séquence selon SEQ ID n° 3,
C2a) une séquence qui code une protéine ou un peptide qui comprend la séquence d'acides aminés selon SEQ ID n° 4,
D2a) une séquence qui est identique à raison d'au moins 80 % à une séquence selon l'un des groupes A2a) à C2a),
E2a) une séquence qui s'hybride ou s'hybriderait, en tenant compte de la dégénérescence du code génétique, avec le brin opposé d'une séquence selon l'un des groupes A2a) à D2a), cette séquence codant pour une protéine ou un peptide qui est capable de convertir du dTDP-rhamnose et de l'acide 3-hydroxydécanoyl-3-hydroxydécanoïque en acide α-L-rhamnopyranosyl-3-hydroxydécanoyl-3-hydroxydécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F2a) un dérivé d'une séquence selon l'un des groupes A2a) à E2a), obtenu par substitution, addition, inversion et/ou délétion d'au moins une base, mais d'au maximum 100 bases,
G2a) une séquence complémentaire d'une séquence selon l'un des groupes A2a) à F2a),
A2b) une séquence selon SEQ ID n° 19, cette séquence codant pour une protéine qui est capable de convertir du dTDP-rhamnose et de l'acide 3-hydroxytétradécanoyl-3-hydroxytétradécanoïque en acide α-L-rhamnopyranosyl-3-hydroxytétradécanoyl-3-hydroxytétradécanoïque,
B2b) une séquence exempte d'introns, qui est dérivée d'une séquence selon A2b) et qui code la même protéine ou le même peptide que la séquence selon SEQ ID n° 19,
C2b) une séquence qui code une protéine ou un peptide qui comprend la séquence d'acides aminés selon SEQ ID n° 20,
D2b) une séquence qui est identique à raison d'au moins 70 % à une séquence selon l'un des groupes A2b) à C2b),
E2b) une séquence qui s'hybride ou s'hybriderait, en tenant compte de la dégénérescence du code génétique, avec le brin opposé d'une séquence selon l'un des groupes A2b) à D2b), cette séquence codant pour une protéine ou un peptide qui est capable de convertir du dTDP-rhamnose et de l'acide 3-hydroxytétradécanoyl-3-hydroxytétradécanoïque en acide α-L-rhamnopyranosyl-3-hydroxytétradécanoyl-3-hydroxytétradécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F2b) un dérivé d'une séquence selon l'un des groupes A2b) à E2b), obtenu par substitution, addition, inversion et/ou délétion d'au moins une base, mais d'au maximum 100 bases et
G2b) une séquence complémentaire d'une séquence selon l'un des groupes A2b) à F2b) et
le groupe [A3 à G3] consiste en les séquences suivantes :
A3a) une séquence selon SEQ ID n° 5, cette séquence codant pour une protéine qui est capable de convertir du dTDP-rhamnose et de l'acide α-L-rhamnopyranosyl-3-hydroxydécanoyl-3-hydroxydécanoïque en acide α-L-rhamnopyranosyl-(1-2)-α-L-rhamnopyranosyl-3-hydroxydécanoyl-3-hydroxydécanoïque,
B3a) une séquence exempte d'introns, qui est dérivée d'une séquence selon A3a) et qui code la même protéine ou le même peptide que la séquence selon SEQ ID n° 5,
C3a) une séquence qui code une protéine ou un peptide qui comprend la séquence d'acides aminés selon SEQ ID n° 6,
D3a) une séquence qui est identique à raison d'au moins 80 % à une séquence selon l'un des groupes A3a) à C3a),
E3a) une séquence qui s'hybride ou s'hybriderait, en tenant compte de la dégénérescence du code génétique, avec le brin opposé d'une séquence selon l'un des groupes A3a) à D3a), cette séquence codant pour une protéine ou un peptide qui est capable de convertir du dTDP-rhamnose et de l'acide α-L-rhamnopyranosyl-3-hydroxydécanoyl-3-hydroxydécanoïque en acide α-L-rhamnopyranosyl-(1-2)-α-L-rhamnopyranosyl-3-hydroxydécanoyl-3-hydroxydécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F3a) un dérivé d'une séquence selon l'un des groupes A3a) à E3a), obtenu par substitution, addition, inversion et/ou délétion d'au moins une base, mais d'au maximum 100 bases,
G3a) une séquence complémentaire d'une séquence selon l'un des groupes A3a) à F3a),
A3b) une séquence selon SEQ ID n° 21, cette séquence codant pour une protéine qui est capable de convertir du dTDP-rhamnose et de l'acide α-L-rhamnopyranosyl-3-hydroxytétradécanoyl-3-hydroxytétradécanoïque en acide α-L-rhamnopyranosyl-(1-2)-α-L-rhamnopyranosyl-3-hydroxytétradécanoyl-3-hydroxytétradécanoïque,
B3b) une séquence exempte d'introns, qui est dérivée d'une séquence selon A3b) et qui code la même protéine ou le même peptide que la séquence selon SEQ ID n° 21,
C3b) une séquence qui code une protéine ou un peptide qui comprend la séquence d'acides aminés selon SEQ ID n° 22,
D3b) une séquence qui est identique à raison d'au moins 60 % à une séquence selon l'un des groupes A3b) à C3b),
E3b) une séquence qui s'hybride ou s'hybriderait, en tenant compte de la dégénérescence du code génétique, avec le brin opposé d'une séquence selon l'un des groupes A3b) à D3b), cette séquence codant pour une protéine ou un peptide qui est capable de convertir du dTDP-rhamnose et de l'acide α-L-rhamnopyranosyl-3-hydroxytétradécanoyl-3-hydroxytétradécanoïque en acide α-L-rhamnopyranosyl-(1-2)-α-L-rhamnopyranosyl-3-hydroxytétradécanoyl-3-hydroxytétradécanoïque, et les conditions d'hybridation étant une incubation à 65 °C pendant une nuit dans SDS à 7 %, ASB à 1 %, EDTA 1 mM, tampon phosphate de sodium 250 mM (pH 7,2) et lavage subséquent à 65 °C avec 2 x SSC; SDS à 0,1 %,
F3b) un dérivé d'une séquence selon l'un des groupes A3b) à E3b), obtenu par substitution, addition, inversion et/ou délétion d'au moins une base, mais d'au maximum 100 bases et
G3b) une séquence complémentaire d'une séquence selon l'un des groupes A3b) à F3b),
ou au moins un vecteur, en particulier un vecteur d'expression ou une cassette de surexpression de gène, comprenant au moins une séquence d'acide nucléique choisie parmi SEQ ID n° 38, SEQ ID n° 40, SEQ ID n° 42, SEQ ID n° 45, SEQ ID n° 47 et des acides nucléiques choisis dans les trois groupes [A1 à G1], [A2 à G2] et [A3 à G3] précités.

9. Procédé pour la production de rhamnolipides de formule générale (I), comprenant les étapes de procédé
I) mise en contact d'une cellule selon au moins l'une quelconque des revendications 1 à 8 avec un milieu comportant une source de carbone
II) culture de la cellule dans des conditions qui permettent à la cellule de former un rhamnolipide partir de la source de carbone et
III) éventuellement isolement des rhamnolipides formés.
